(19) Europäisches Patentamt
European Patent Office
Office européen des brevets

(11) EP 3 768 674 B1

(12) # EUROPEAN PATENT SPECIFICATION

(45) Date of publication and mention of the grant of the patent:
**03.01.2024 Bulletin 2024/01**

(21) Application number: **19713022.2**

(22) Date of filing: **22.03.2019**

(51) International Patent Classification (IPC):
***C07D 403/12*** (2006.01) ***C07D 401/14*** (2006.01)
***C07D 401/12*** (2006.01) ***C07D 213/76*** (2006.01)
***C07D 239/42*** (2006.01) ***A61P 35/00*** (2006.01)
***A61K 31/506*** (2006.01)

(52) Cooperative Patent Classification (CPC):
**C07D 403/12; A61P 35/00; C07D 213/76; C07D 239/42; C07D 401/12; C07D 401/14**

(86) International application number:
**PCT/EP2019/057320**

(87) International publication number:
**WO 2019/180244 (26.09.2019 Gazette 2019/39)**

(54) **AMINOPYRIMIDINE DERIVATIVES AS CTPS1 INHIBITORS**

AMINOPYRIMIDIN-DERIVATE ALS CTPS1 INHIBITORS

DÉRIVÉS D' AMINOPYRIMIDINE COMME INHIBITEURS DE CTPS1

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.03.2018 EP 18163772**
**04.06.2018 EP 18175823**
**23.10.2018 EP 18202136**
**21.12.2018 PCT/EP2018/086617**

(43) Date of publication of application:
**27.01.2021 Bulletin 2021/04**

(73) Proprietor: **Step Pharma S.A.S.**
**75272 Paris, Cedex 06 (FR)**

(72) Inventors:
- **QUDDUS, Abdul**
  **Nottingham Nottinghamshire NG1 1GR (GB)**
- **NOVAK, Andrew**
  **Nottinghamshire NG1 1GR (GB)**
- **COUSIN, David**
  **Nottinghamshire NG1 1GR (GB)**
- **BLACKHAM, Emma**
  **Nottinghamshire NG1 1GR (GB)**
- **JONES, Geraint**
  **Nottinghamshire NG1 1GR (GB)**
- **WRIGGLESWORTH, Joseph**
  **Nottinghamshire NG1 1GR (GB)**
- **DUFFY, Lorna**
  **Nottinghamshire NG1 1GR (GB)**
- **BIRCH, Louise**
  **Nottinghamshire NG1 1GR (GB)**
- **GEORGE, Pascal**
  **75272 Paris, Cedex 06 (FR)**
- **AHMED, Saleh**
  **Nottinghamshire NG1 1GR (GB)**

(74) Representative: **Sagittarius IP**
**Marlow International**
**Parkway**
**Marlow, SL7 1YL (GB)**

(56) References cited:
**WO-A1-2009/075874** **WO-A1-2015/094119**
**WO-A2-2014/170435**

- **SAKAMOTO KOTARO ET AL: "Identification of cytidine-5-triphosphate synthase1-selective inhibitory peptide from random peptide library displayed on T7 phage", PEPTIDES, ELSEVIER, AMSTERDAM, NL, vol. 94, 1 July 2017 (2017-07-01), pages 56-63, XP085130857, ISSN: 0196-9781, DOI: 10.1016/J.PEPTIDES.2017.06.007**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- PONNAMBALANADAR ANANTHAKRISHNANADAR ET AL: "The effects of substituents on the rate of saponification of biphenyl-4-carboxylates", JOURNAL OF THE CHEMICAL SOCIETY, PERKIN TRANSACTIONS 2: PHYSICAL ORGANIC CHEMISTRY, vol. 11, no. 1, 1 January 1984 (1984-01-01), page 35, XP055559289, GB ISSN: 0300-9580, DOI: 10.1039/p29840000035

## Description

### Field of the invention

The invention relates to novel compounds, processes for the manufacture of such compounds, related intermediates, compositions comprising such compounds and the use of such compounds as cytidine triphosphate synthase 1 inhibitors, particularly in the treatment or prophylaxis of disorders associated with cell proliferation.

### Background of the invention

Nucleotides are a key building block for cellular metabolic processes such as deoxyribonucleic acid (DNA) and ribonucleic acid (RNA) synthesis. There are two classes of nucleotides, that contain either purine or pyrimidine bases, both of which are important for metabolic processes. Based on this, many therapies have been developed to target different aspects of nucleotide synthesis, with some inhibiting generation of purine nucleotides and some pyrimidine nucleotides or both.

The pyrimidine nucleotide cytidine 5' triphosphate (CTP) is a precursor required not just for the anabolism of DNA and RNA but also phospholipids and sialyation of proteins. CTP originates from two sources: a salvage pathway and a *de novo* synthesis pathway that depends on two enzymes, the CTP synthases (or synthetases) 1 and 2 (CTPS1 and CTPS2) (Evans and Guy 2004; Higgins, *et al.* 2007; Ostrander, *et al.* 1998).

CTPS1 and CTPS2 catalyse the conversion of uridine triphosphate (UTP) and glutamine into cytidine triphosphate (CTP) and L-glutamate:

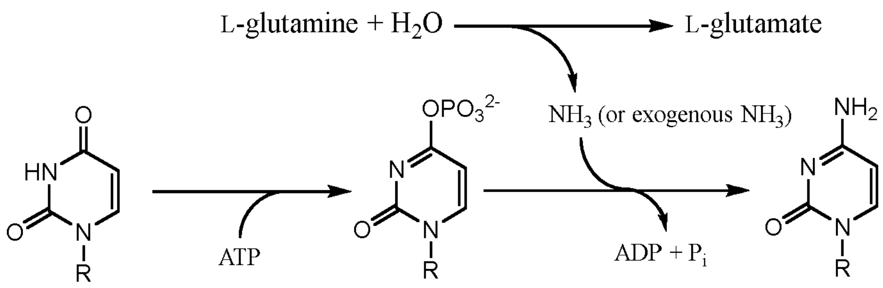

UTP 4-phospho-UTP CTP

Both enzymes have two domains, an N-terminal synthetase domain and a C-terminal glutaminase domain (Kursula, *et al.* 2006). The synthetase domain transfers a phosphate from adenosine triphosphate (ATP) to the 4-position of UTP to create an activated intermediate, 4-phospho-UTP. The glutaminase domain generates ammonia from glutamine, via a covalent thioester intermediate with a conserved active site cysteine, generating glutamate. This ammonium is transferred from the glutaminase domain to the synthetase domain *via* a tunnel or can be derived from external ammonium. This ammonium is then used by the synthetase domain to generate CTP from the 4-phospho-UTP (Lieberman, 1956).

Although CTPS exists as two isozymes in humans and other eukaryotic organisms, CTPS1 and CTPS2, functional differences between the two isozymes are not yet fully elucidated (van Kuilenburg, *et al.* 2000).

The immune system provides protection from infections and has therefore evolved to rapidly respond to the wide variety of pathogens that the individual may be exposed to. This response can take many forms, but the expansion and differentiation of immune populations is a critical element and is hence closely linked to rapid cell proliferation. Within this, CTP synthase activity appears to play an important role in DNA synthesis and the rapid expansion of lymphocytes following activation (Fairbanks, *et al.* 1995; van den Berg, *et al.* 1995).

Strong clinical validation that CTPS1 is the critical enzyme in human lymphocyte proliferation came with the identification of a loss-of-function homozygous mutation (rs145092287) in this enzyme that causes a distinct and life-threatening immunodeficiency, characterized by an impaired capacity of activated T- and B-cells to proliferate in response to antigen receptor-mediated activation. Activated CTPS1-deficient cells were shown to have decreased levels of CTP. Normal T-cell proliferation was restored in CTPS1-deficient cells by expressing wild-type CTPS1 or by addition of cytidine. CTPS1 expression was found to be low in resting lymphocytes, but rapidly upregulated following activation of these cells. Expression of CTPS1 in other tissues was generally low. CTPS2 seems to be ubiquitously expressed in a range of cells and tissues but at low levels, and the failure of CTPS2, which is still intact in the patients, to compensate for the mutated CTPS1, supports CTPS1 being the critical enzyme for the immune populations affected in the patients (Martin, *et al.* 2014).

Overall, these findings suggest that CTPS1 is a critical enzyme necessary to meet the demands for the supply of CTP required by several important immune cell populations.

Normally the immune response is tightly regulated to ensure protection from infection, whilst controlling any response targeting host tissues. In certain situations, the control of this process is not effective, leading to immune-mediated pathology. A wide range of human diseases are thought to be due to such inappropriate responses mediated by different elements of the immune system.

Given the role that cell populations, such as T and B lymphocytes, are thought to play in a wide range of autoimmune and other diseases, CTPS1 represents a target for a new class of immunosuppressive agents. Inhibition of CTPS1 therefore provides a novel approach to the inhibition of activated lymphocytes and selected other immune cell populations such as Natural Killer cells, Mucosal-Associated Invariant T (MAIT) and Invariant Natural Killer T cells, highlighted by the phenotype of the human mutation patients (Martin, *et al.* 2014).

Cancer can affect multiple cell types and tissues but the underlying cause is a breakdown in the control of cell division. This process is highly complex, requiring careful coordination of multiple pathways, many of which remain to be fully characterised. Cell division requires the effective replication of the cell's DNA and other constituents. Interfering with a cell's ability to replicate by targeting nucleic acid synthesis has been a core approach in cancer therapy for many years. Examples of therapies acting in this way are 6-thioguanine, 6-mecaptopurine, 5-fluorouracil, cytarabine, gemcitabine and pemetrexed.

As indicated above, pathways involved in providing the key building blocks for nucleic acid replication are the purine and pyrimidine synthesis pathways, and pyrimidine biosynthesis has been observed to be up-regulated in tumors and neoplastic cells.

CTPS activity is upregulated in a range of tumour types of both haematological and non-haematological origin, although heterogeneity is observed among patients. Linkages have also been made between high enzyme levels and resistance to chemotherapeutic agents.

Currently, the precise role that CTPS1 and CTPS2 may play in cancer is not completely clear. Several non-selective CTPS inhibitors have been developed for oncology indications up to phase I/II clinical trials, but were stopped due to toxicity and efficacy issues.

Most of the developed inhibitors are nucleoside-analogue prodrugs (3-deazauridine, CPEC, carbodine), which are converted to the active triphosphorylated metabolite by the kinases involved in pyrimidine biosynthesis: uridine/cytidine kinase, nucleoside monophosphate-kinase (NMP-kinase) and nucleoside diphosphatekinase (NDP-kinase). The remaining inhibitors (acivicin, DON) are reactive analogues of glutamine, which irreversibly inhibit the glutaminase domain of CTPS. Gemcitibine is also reported to have some inhibitory activity against CTPS (McClusky *et al.,* 2016).

CTPS therefore appears to be an important target in the cancer field. The nature of all of the above compounds is such that effects on other pathways are likely to contribute to the efficacy they show in inhibiting tumours.

Selective CTPS inhibitors therefore offer an attractive alternative approach for the treatment of tumours. Compounds with different potencies against CTPS1 and CTPS2 may offer important opportunities to target different tumours depending upon their relative dependence on these enzymes.

CTPS1 has also been suggested to play a role in vascular smooth muscle cell proliferation following vascular injury or surgery (Tang, *et al.* 2013).

As far as is known to date, no selective CTPS1 inhibitors have been developed. Recently, the CTPS1 selective inhibitory peptide CTpep-3 has been identified. The inhibitory effects of CTpep-3 however, were seen in cell free assays but not in the cellular context. This was not unexpected though, since the peptide is unlikely to enter the cell and hence is not easily developable as a therapeutic (Sakamoto, *et al.* 2017).

In summary, the available information and data strongly suggest that inhibitors of CTPS1 will reduce the proliferation of a number of immune and cancer cell populations, with the potential for an effect on other selected cell types such as vascular smooth muscle cells as well. Inhibitors of CTPS1 may therefore be expected to have utility for treatment or prophylaxis in a wide range of indications where the pathology is driven by these populations.

CTPS1 inhibitors represent a novel approach for inhibiting selected components of the immune system in various tissues, and the related pathologies or pathological conditions such as, in general terms, rejection of transplanted cells and tissues, Graft-related diseases or disorders, allergies and autoimmune diseases. In addition, CTPS1 inhibitors offer therapeutic potential in a range of cancer indications and in enhancing recovery from vascular injury or surgery and reducing morbidity and mortality associated with neointima and restenosis.

## Summary of the Invention

The invention provides a compound of formula (I):

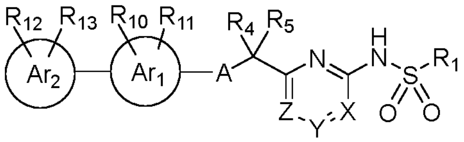

(I)

wherein

A is an amide linker having the following structure: -C(=O)NH- or -NHC(=O)-;

X is N or CH;

Y is N or $CR_2$;

Z is N or $CR_3$;
with the proviso that when at least one of X or Z is N, Y cannot be N;

$R_1$ is $C_{1-5}$alkyl, $CF_3$, $C_{0-2}$alkylene$C_{3-5}$cycloalkyl or $C_{0-2}$alkylene$C_{3-5}$cycloalkyl which cycloalkyl is substituted by $CH_3$;

$R_2$ is H, halo, $C_{1-2}$alkyl, $OC_{1-2}$alkyl, $C_{1-2}$haloalkyl or $OC_{1-2}$haloalkyl;

$R_3$ is H, halo, $CH_3$, $OCH_3$, $CF_3$ or $OCF_3$;
wherein at least one of $R_2$ and $R_3$ is H;

$R_4$ and $R_5$ are each independently H, $C_{1-6}$alkyl, $C_{1-6}$alkylOH, $C_{1-6}$haloalkyl, $C_{0-2}$alkylene$C_{3-6}$cycloalkyl, $C_{0-2}$alkylene$C_{3-6}$heterocycloalkyl, $C_{1-3}$alkylene$OC_{1-3}$alkyl, or $R_4$ and $R_5$ together with the carbon atom to which they are attached form a $C_{3-6}$cycloalkyl or $C_{3-6}$heterocycloalkyl; and
when A is -NHC(=O)-:
$R_4$ and $R_5$ may additionally be selected from halo, $OC_{1-6}$haloalkyl, $OC_{0-2}$alkylene$C_{3-6}$cycloalkyl, $OC_{0-2}$alkylene$C_{3-6}$heterocycloalkyl, $OC_{1-6}$alkyl and $NR_{21}R_{22}$;

Ar1 is a 6-membered aryl or heteroaryl;

Ar2 is a 6-membered aryl or heteroaryl and is attached to Ar1 in the para position relative to the amide;

$R_{10}$ is H, halo, $C_{1-3}$alkyl, $C_{1-2}$haloalkyl, $OC_{1-2}$alkyl, $OC_{1-2}$haloalkyl or CN;

$R_{11}$ is H, F, Cl, $C_{1-2}$alkyl, $CF_3$, $OCH_3$ or CN;

$R_{12}$ is attached to Ar2 in the ortho or meta position relative to Ar1 and $R_{12}$ is H, halo, $C_{1-4}$alkyl, $C_{2-4}$alkenyl, $C_{0-2}$alkylene$C_{3-5}$cycloalkyl, $OC_{1-4}$alkyl, $OC_{0-2}$alkylene$C_{3-5}$cycloalkyl, $C_{1-4}$haloalkyl, $OC_{1-4}$haloalkyl, hydroxy, $C_{1-4}$alkylOH, $SO_2C_{1-2}$alkyl, $C(O)N(C_{1-2}alkyl)_2$, $NHC(O)C_{1-3}$alkyl or $NR_{23}R_{24}$; and
when A is -NHC(=O)-:
$R_{12}$ may additionally be selected from CN, $OCH_2CH_2N(CH_3)_2$ and a $C_{3-6}$heterocycloalkyl comprising one nitrogen located at the point of attachment to Ar2, or $R_{12}$ together with a nitrogen atom to which it is attached forms an N-oxide ($N^+-O^-$);

$R_{13}$ is H or halo;

$R_{21}$ is H, $C_{1-5}$alkyl, $C(O)C_{1-5}$alkyl, $C(O)OC_{1-5}$alkyl;

$R_{22}$ is H or $CH_3$;

$R_{23}$ is H or $C_{1-2}$alkyl; and

$R_{24}$ is H or $C_{1-2}$alkyl.

Suitably, the invention provides a compound of formula (I):

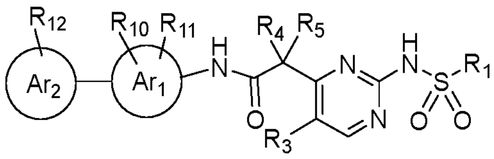

(I)

wherein

$R_1$ is $C_{1-5}$alkyl, $C_{0-2}$alkylene$C_{3-5}$cycloalkyl or $C_{0-2}$alkylene$C_{3-5}$cycloalkyl which cycloalkyl is substituted by $CH_3$;

$R_3$ is H, halo or $CH_3$;

$R_4$ and $R_5$ are each independently H, halo, $C_{1-6}$alkyl, $C_{1-3}$alkyleneO$C_{1-3}$alkyl, or $R_4$ and $R_5$ together with the carbon atom to which they are attached form a $C_{3-6}$cycloalkyl or $C_{3-6}$heterocycloalkyl;

Ar1 is a 6-membered aryl or heteroaryl;

Ar2 is a 6-membered aryl or heteroaryl and is attached to Ar1 in the para position relative to the amide;

$R_{10}$ is H, halo, $C_{1-2}$alkyl, O$C_{1-2}$alkyl, O$C_{1-2}$haloalkyl or CN;

$R_{11}$ is H, F, $CH_3$ or $OCH_3$; and

$R_{12}$ is attached to Ar2 in the ortho or meta position relative to Ar1 and $R_{12}$ is H, halo, $C_{1-4}$alkyl, O$C_{1-4}$alkyl, O$C_{0-2}$alkylene$C_{3-5}$cycloalkyl, CN, $C_{1-4}$haloalkyl, O$C_{1-4}$haloalkyl.

The invention also provides a compound of formula (I):

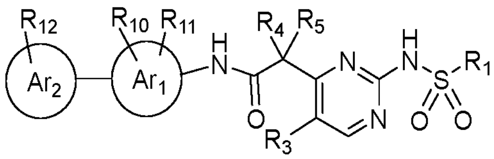

(I)

wherein

$R_1$ is $C_{1-5}$alkyl, $C_{0-2}$alkylene$C_{3-5}$cycloalkyl or $C_{0-2}$alkylene$C_{3-5}$cycloalkyl which cycloalkyl is substituted by $CH_3$;

$R_3$ is H, halo or $CH_3$;

$R_4$ and $R_5$ are each independently H, $C_{1-6}$alkyl, $C_{1-3}$alkyleneO$C_{1-3}$alkyl, or $R_4$ and $R_5$ together with the carbon atom to which they are attached form a $C_{3-6}$cycloalkyl;

Ar1 is a 6-membered aryl or heteroaryl;

Ar2 is a 6-membered aryl or heteroaryl and is attached to Ar1 in the para position relative to the amide;

$R_{10}$ is H, halo, $C_{1-2}$alkyl, O$C_{1-2}$alkyl, O$C_{1-2}$haloalkyl or CN;

$R_{11}$ is H, F, $CH_3$ or $OCH_3$; and

$R_{12}$ is attached to Ar2 in the ortho or meta position relative to Ar1 and $R_{12}$ is H, halo, $C_{1-4}$alkyl, O$C_{1-4}$alkyl, O$C_{0-2}$alkylene$C_{3-5}$cycloalkyl, CN, $C_{1-4}$haloalkyl, O$C_{1-4}$haloalkyl.

The invention also provides a compound of formula (I):

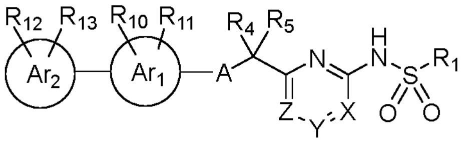
(I)

wherein

A is an amide linker having the following structure: -C(=O)NH- or -NHC(=O)-;

X is N or CH;

Y is N or $CR_2$;

Z is N or $CR_3$;
with the proviso that when at least one of X or Z is N, Y cannot be N;

$R_1$ is $C_{1-5}$alkyl, $C_{0-2}$alkylene$C_{3-5}$cycloalkyl or $C_{0-2}$alkylene$C_{3-5}$cycloalkyl which cycloalkyl is substituted by $CH_3$;

$R_2$ is H, $C_{1-2}$alkyl or $C_{1-2}$haloalkyl;

$R_3$ is H, halo or $CH_3$;
wherein at least one of $R_2$ and $R_3$ is H;

$R_4$ and $R_5$ are each independently H, $C_{1-6}$alkyl or $C_{1-3}$alkylene$OC_{1-3}$alkyl, or $R_4$ and $R_5$ together with the carbon atom to which they are attached form a $C_{3-6}$cycloalkyl or $C_{3-6}$heterocycloalkyl; and
when A is -NHC(=O)-:
$R_4$ and $R_5$ may additionally be selected from halo and $OC_{1-6}$alkyl;

Ar1 is a 6-membered aryl or heteroaryl;

Ar2 is a 6-membered aryl or heteroaryl and is attached to Ar1 in the para position relative to the amide;

$R_{10}$ is H, halo, $C_{1-3}$alkyl, $C_{1-2}$haloalkyl, $OC_{1-2}$alkyl, $OC_{1-2}$haloalkyl or CN;

$R_{11}$ is H, F, $CH_3$ or $OCH_3$;

$R_{12}$ is attached to Ar2 in the ortho or meta position relative to Ar1 and $R_{12}$ is H, halo, $C_{1-4}$alkyl, $OC_{1-4}$alkyl, $C_{1-4}$haloalkyl, $OC_{1-4}$haloalkyl, $C_{0-2}$alkylene$C_{3-5}$cycloalkyl, $OC_{0-2}$alkylene$C_{3-5}$cycloalkyl, CN or $C_{2-4}$alkenyl; and

$R_{13}$ is H.

A compound of formula (I) may be provided in the form of a salt and/or solvate thereof. Suitably, the compound of formula (I) may be provided in the form of a pharmaceutically acceptable salt and/or solvate thereof. In particular, the compound of formula (I) may be provided in the form of a pharmaceutically acceptable salt and/or solvate, such as a pharmaceutically acceptable salt.

Also provided is a compound of formula (I), or a pharmaceutically acceptable salt and/or solvate thereof, for use as a medicament, in particular for use in the inhibition of CTPS1 in a subject or the prophylaxis or treatment of associated diseases or disorders, such as those in which a reduction in T-cell and/or B-cell proliferation would be beneficial.

Suitably the disease or disorder is selected from: inflammatory skin diseases such as psoriasis or lichen planus; acute and/or chronic GVHD such as steroid resistant acute GVHD; acute lymphoproliferative syndrome (ALPS); systemic lupus erythematosus, lupus nephritis or cutaneous lupus; and transplantation. In addition, the disease or disorder may be selected from myasthenia gravis, multiple sclerosis, and scleroderma/systemic sclerosis.

Also provided is a compound of formula (I), or a pharmaceutically acceptable salt and/or solvate thereof, for use in the treatment of cancer.

Also provided is a compound of formula (I), or a pharmaceutically acceptable salt and/or solvate thereof, for use in enhancing recovery from vascular injury or surgery and reducing morbidity and mortality associated with neointima

and restenosis in a subject.

Also provided are pharmaceutical compositions containing a compound of formula (I), or a pharmaceutically acceptable salt and/or solvate thereof, and a pharmaceutically acceptable carrier or excipient.

Also provided are processes for preparing compounds of formula (I) and novel intermediates of use in the preparation of compounds of formula (I).

**Detailed description of the Invention**

The invention provides a compound of formula (I):

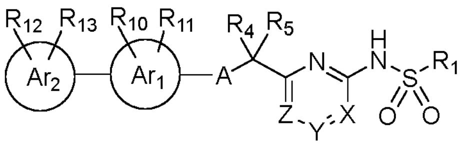

(I)

wherein

A is an amide linker having the following structure: -C(=O)NH- or -NHC(=O)-;

X is N or CH;

Y is N or $CR_2$;

Z is N or $CR_3$;
with the proviso that when at least one of X or Z is N, Y cannot be N;

$R_1$ is $C_{1-5}$alkyl, $CF_3$, $C_{0-2}$alkylene$C_{3-5}$cycloalkyl or $C_{0-2}$alkylene$C_{3-5}$cycloalkyl which cycloalkyl is substituted by $CH_3$;

$R_2$ is H, halo, $C_{1-2}$alkyl, $OC_{1-2}$alkyl, $C_{1-2}$haloalkyl or $OC_{1-2}$haloalkyl;

$R_3$ is H, halo, $CH_3$, $OCH_3$, $CF_3$ or $OCF_3$;
wherein at least one of $R_2$ and $R_3$ is H;

$R_4$ and $R_5$ are each independently H, $C_{1-6}$alkyl, $C_{1-6}$alkylOH, $C_{1-6}$haloalkyl, $C_{0-2}$alkylene$C_{3-6}$cycloalkyl, $C_{0-2}$alkylene$C_{3-6}$heterocycloalkyl, $C_{1-3}$alkylene$OC_{1-3}$alkyl, or $R_4$ and $R_5$ together with the carbon atom to which they are attached form a $C_{3-6}$cycloalkyl or $C_{3-6}$heterocycloalkyl; and
when A is -NHC(=O)-:
$R_4$ and $R_5$ may additionally be selected from halo, $OC_{1-6}$haloalkyl, $OC_{0-2}$alkylene$C_{3-6}$cycloalkyl, $OC_{0-2}$alkylene$C_{3-6}$heterocycloalkyl, $OC_{1-6}$alkyl and $NR_{21}R_{22}$;

Ar1 is a 6-membered aryl or heteroaryl;

Ar2 is a 6-membered aryl or heteroaryl and is attached to Ar1 in the para position relative to the amide;

$R_{10}$ is H, halo, $C_{1-3}$alkyl, $C_{1-2}$haloalkyl, $OC_{1-2}$alkyl, $OC_{1-2}$haloalkyl or CN;

$R_{11}$ is H, F, Cl, $C_{1-2}$alkyl, $CF_3$, $OCH_3$ or CN;

$R_{12}$ is attached to Ar2 in the ortho or meta position relative to Ar1 and $R_{12}$ is H, halo, $C_{1-4}$alkyl, $C_{2-4}$alkenyl, $C_{0-2}$alkylene$C_{3-5}$cycloalkyl, $OC_{1-4}$alkyl, $OC_{0-2}$alkylene$C_{3-5}$cycloalkyl, $C_{1-4}$haloalkyl, $OC_{1-4}$haloalkyl, hydroxy, $C_{1-4}$alkylOH, $SO_2C_{1-2}$alkyl, $C(O)N(C_{1-2}alkyl)_2$, $NHC(O)C_{1-3}$alkyl or $NR_{23}R_{24}$; and
when A is -NHC(=O)-:
$R_{12}$ may additionally be selected from CN, $OCH_2CH_2N(CH_3)_2$ and a $C_{3-6}$heterocycloalkyl comprising one nitrogen located at the point of attachment to Ar2, or $R_{12}$ together with a nitrogen atom to which it is attached forms an N-oxide ($N^+$-$O^-$);

$R_{13}$ is H or halo;

$R_{21}$ is H, $C_{1-5}$alkyl, C(O)$C_{1-5}$alkyl, C(O)O$C_{1-5}$alkyl;

$R_{22}$ is H or $CH_3$;

$R_{23}$ is H or $C_{1-2}$alkyl; and

$R_{24}$ is H or $C_{1-2}$alkyl;

or a salt and/or solvate thereof.

Suitably, the invention provides a compound of formula (I):

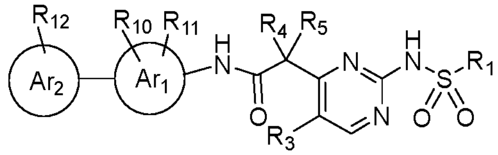

(I)

wherein

$R_1$ is $C_{1-5}$alkyl, $C_{0-2}$alkylene$C_{3-5}$cycloalkyl or $C_{0-2}$alkylene$C_{3-5}$cycloalkyl which cycloalkyl is substituted by $CH_3$;

$R_3$ is H, halo or $CH_3$;

$R_4$ and $R_5$ are each independently H, halo, $C_{1-6}$alkyl, $C_{1-3}$alkyleneO$C_{1-3}$alkyl, or $R_4$ and $R_5$ together with the carbon atom to which they are attached form a $C_{3-6}$cycloalkyl or $C_{3-6}$heterocycloalkyl;

Ar1 is a 6-membered aryl or heteroaryl;

Ar2 is a 6-membered aryl or heteroaryl and is attached to Ar1 in the para position relative to the amide;

$R_{10}$ is H, halo, $C_{1-2}$alkyl, O$C_{1-2}$alkyl, O$C_{1-2}$haloalkyl or CN;

$R_{11}$ is H, F, $CH_3$ or $OCH_3$; and

$R_{12}$ is attached to Ar2 in the ortho or meta position relative to Ar1 and $R_{12}$ is H, halo, $C_{1-4}$alkyl, O$C_{1-4}$alkyl, O$C_{0-2}$alkylene$C_{3-5}$cycloalkyl, CN, $C_{1-4}$haloalkyl, O$C_{1-4}$haloalkyl;

or a salt and/or solvate thereof.

The invention also provides a compound of formula (I):

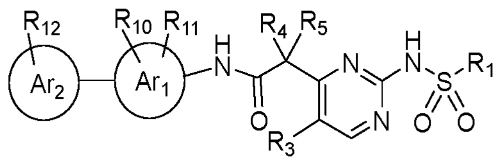

(I)

wherein

$R_1$ is $C_{1-5}$alkyl, $C_{0-2}$alkylene$C_{3-5}$cycloalkyl or $C_{0-2}$alkylene$C_{3-5}$cycloalkyl which cycloalkyl is substituted by $CH_3$;

$R_3$ is H, halo or $CH_3$;

$R_4$ and $R_5$ are each independently H, $C_{1-6}$alkyl, $C_{1-3}$alkyleneO$C_{1-3}$alkyl, or $R_4$ and $R_5$ together with the carbon atom to which they are attached form a $C_{3-6}$cycloalkyl;

Ar1 is a 6-membered aryl or heteroaryl;

Ar2 is a 6-membered aryl or heteroaryl and is attached to Ar1 in the para position relative to the amide;

$R_{10}$ is H, halo, $C_{1-2}$alkyl, $OC_{1-2}$alkyl, $OC_{1-2}$haloalkyl or CN;

$R_{11}$ is H, F, $CH_3$ or $OCH_3$; and

$R_{12}$ is attached to Ar2 in the ortho or meta position relative to Ar1 and $R_{12}$ is H, halo, $C_{1-4}$alkyl, $OC_{1-4}$alkyl, $OC_{0-2}$alkylene$C_{3-5}$cycloalkyl, CN, $C_{1-4}$haloalkyl, $OC_{1-4}$haloalkyl;

or a salt and/or solvate thereof.

The invention also provides a compound of formula (I):

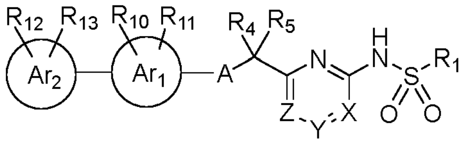

(I)

wherein

A is an amide linker having the following structure: -C(=O)NH- or -NHC(=O)-;

X is N or CH;

Y is N or $CR_2$;

Z is N or $CR_3$;
with the proviso that when at least one of X or Z is N, Y cannot be N;

$R_1$ is $C_{1-5}$alkyl, $C_{0-2}$alkylene$C_{3-5}$cycloalkyl or $C_{0-2}$alkylene$C_{3-5}$cycloalkyl which cycloalkyl is substituted by $CH_3$;

$R_2$ is H, $C_{1-2}$alkyl or $C_{1-2}$haloalkyl;

$R_3$ is H, halo or $CH_3$;
wherein at least one of $R_2$ and $R_3$ is H;

$R_4$ and $R_5$ are each independently H, $C_{1-6}$alkyl or $C_{1-3}$alkylene$OC_{1-3}$alkyl, or $R_4$ and $R_5$ together with the carbon atom to which they are attached form a $C_{3-6}$cycloalkyl or $C_{3-6}$heterocycloalkyl; and
when A is -NHC(=O)-:
$R_4$ and $R_5$ may additionally be selected from halo and $OC_{1-6}$alkyl;

Ar1 is a 6-membered aryl or heteroaryl;

Ar2 is a 6-membered aryl or heteroaryl and is attached to Ar1 in the para position relative to the amide;

$R_{10}$ is H, halo, $C_{1-3}$alkyl, $C_{1-2}$haloalkyl, $OC_{1-2}$alkyl, $OC_{1-2}$haloalkyl or CN;

$R_{11}$ is H, F, $CH_3$ or $OCH_3$;

$R_{12}$ is attached to Ar2 in the ortho or meta position relative to Ar1 and $R_{12}$ is H, halo, $C_{1-4}$alkyl, $OC_{1-4}$alkyl, $C_{1-4}$haloalkyl, $OC_{1-4}$haloalkyl, $C_{0-2}$alkylene$C_{3-5}$cycloalkyl, $OC_{0-2}$alkylene$C_{3-5}$cycloalkyl, CN or $C_{2-4}$alkenyl; and

$R_{13}$ is H;

or a salt and/or solvate thereof.

The term 'alkyl' as used herein, such as in $C_{1-3}$alkyl, $C_{1-4}$alkyl, $C_{1-5}$alkyl or $C_{1-6}$alkyl, whether alone or forming part of a larger group such as an Oalkyl group (e.g. $OC_{1-3}$alkyl, $OC_{1-4}$alkyl and $OC_{1-5}$alkyl), is a straight or a branched fully saturated hydrocarbon chain containing the specified number of carbon atoms. Examples of alkyl groups include the $C_{1-5}$alkyl groups methyl, ethyl, *n*-propyl, *iso*-propyl, *n*-butyl, *iso*-butyl, *sec*-butyl, *tert*-butyl and *n*-pentyl, sec-pentyl and 3-pentyl, in particular the $C_{1-3}$alkyl groups methyl, ethyl, *n*-propyl and *iso*-propyl. Reference to "propyl" includes *n*-propyl and *iso*-propyl, and reference to "butyl" includes *n*-butyl, isobutyl, *sec*-butyl and *tert*-butyl. Examples of Oalkyl groups include the $OC_{1-4}$alkyl groups methoxy, ethoxy, propoxy (which includes *n*-propoxy and *iso*-propoxy) and butoxy (which includes *n*-butoxy, *iso*-butoxy, *sec*-butoxy and *tert*-butoxy). $C_6$alkyl groups as used herein, whether alone or forming part of a larger group such as an $OC_6$alkyl group is a straight or a branched fully saturated hydrocarbon chain containing six carbon atoms. Examples of $C_6$alkyl groups include n-hexyl, 2-methylpentyl, 3-methylpentyl, 2,2-dimethylbutyl and 2,3-dimethylbutyl.

The term 'alkylene' as used herein, such as in $C_{0-2}$alkylene$C_{3-5}$cycloalkyl, $C_{1-2}$alkylene$OC_{1-2}$alkyl or $OC_{0-2}$alkylene$C_{3-5}$cycloalkyl is a bifunctional straight or a branched fully saturated hydrocarbon chain containing the specified number of carbon atoms. Examples of $C_{0-2}$alkylene groups are where the group is absent (i.e. $C_0$), methylene ($C_1$) and ethylene ($C_2$).

The term 'alkenyl' as used herein, such as in $C_{2-4}$alkenyl, is a straight or branched hydrocarbon chain containing the specified number of carbon atoms and a carbon-carbon double bond.

The term 'cycloalkyl' as used herein, such as in $C_{3-5}$cycloalkyl or $C_{3-6}$cycloalkyl, whether alone or forming part of a larger group such as $OC_{3-5}$cycloalkyl or $C_{0-2}$alkylene$C_{3-5}$cycloalkyl is a fully saturated hydrocarbon ring containing the specified number of carbon atoms. Examples of cycloalkyl groups include the $C_{3-6}$cycloalkyl groups cyclopropyl, cyclobutyl, cyclopentyl and cyclohexyl, in particular the $C_{3-5}$cycloalkyl groups cyclopropyl, cyclobutyl and cyclopentyl:

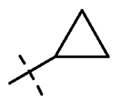 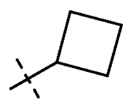 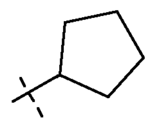

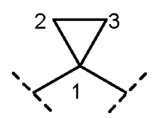
$C_3$ cycloalkyl

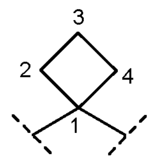
$C_4$ cycloalkyl

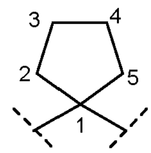
$C_5$ cycloalkyl

The term 'heterocycloalkyl' as used herein, such as in $C_{3-6}$heterocycloalkyl or $C_{0-2}$alkylene$C_{3-6}$heterocycloalkyl is a fully saturated hydrocarbon ring containing the specified number of carbon atoms and may include the carbon atom through which the cycloalkyl group is attached, wherein at least one of the carbon atoms in the ring is replaced by a heteroatom such as N, S or O. As required by valency, the nitrogen atom(s) may be connected to a hydrogen atom to form an NH group. Alternatively the nitrogen atom(s) may be substituted (such as one nitrogen atom is substituted), for example by $C_{1-4}$alkyl, C(O)H, C(O)$C_{1-4}$alkyl, C(O)$OC_{1-4}$alkyl, C(0)$OC_{1-4}$alkylaryl such as C(O)OBz, C(O)$NHC_{1-4}$alkyl, C(O)$NHC_{1-4}$alkylaryl such as C(O)NHBz, an Fmoc group, C(O)$C_{1-4}$haloalkyl, C(O)$OC_{1-4}$haloalkyl or C(0)$NHC_{1-4}$haloalkyl such as C(O)OtBu. Wherein a ring heteroatom is S, the term 'heterocycloalkyl' includes wherein the S atom(s) is substituted (such as one S atom is substituted) by one or two oxygen atoms (i.e. S(O) or $S(O)_2$). Alternatively, any sulphur atom(s) in the $C_{3-6}$heterocycloalkyl ring is not substituted.

Examples of $C_{3-6}$heterocycloalkyl groups include those comprising one heteroatom such as containing one heteroatom (e.g. oxygen) or containing two heteroatoms (e.g. two oxygen atoms or one oxygen atom and one nitrogen atom). Particular examples of $C_{3-6}$heterocycloalkyl comprising one oxygen atom include oxiranyl, oxetanyl, 3-dioxolanyl, morpholinyl, 1,4-oxathianyl, tetrahydropyranyl, 1,4-thioxanyl and 1,3,5-trioxanyl. Examples of $C_{3-6}$heterocycloalkyl include those comprising one oxygen atom such as containing one oxygen atom, or containing two oxygen atoms. Particular examples of $C_{3-6}$heterocycloalkyl comprising one oxygen atom include oxiranyl, oxetanyl, 3-dioxolanyl, morpholinyl, 1,4-oxathianyl, tetrahydropyranyl, 1,4-thioxanyl and 1,3,5-trioxanyl.

In one embodiment, the term 'heterocycloalkyl' as used herein, such as in $C_{3-6}$heterocycloalkyl is a fully saturated hydrocarbon ring containing the specified number of carbon atoms and may include the carbon atom through which the cycloalkyl group is attached, wherein at least one of the carbon atoms in the ring is replaced by a heteroatom such as N, S or O. Examples of $C_{3-6}$heterocycloalkyl groups include those comprising one heteroatom such as containing one heteroatom (e.g. oxygen) or containing two heteroatoms (e.g. two oxygen atoms or one oxygen atom and one nitrogen atom).

The heterocycloalkyl groups may have the following structures:

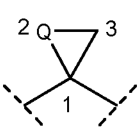
$C_3$ heterocycloalkyl

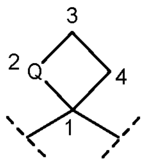
$C_4$ heterocycloalkyl

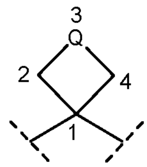
$C_4$ heterocycloalkyl

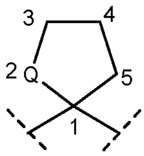
$C_5$ heterocycloalkyl

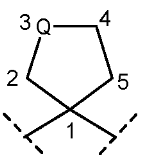
$C_5$ heterocycloalkyl

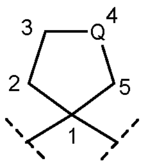
$C_5$ heterocycloalkyl

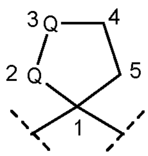
$C_5$ heterocycloalkyl

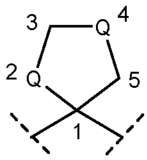
$C_5$ heterocycloalkyl

wherein each Q is a heteroatom independently selected from O, N or S. When Q is N, as required by valency, the nitrogen atom(s) may be connected to a hydrogen atom to form an NH group. Alternatively the nitrogen atom(s) may be substituted (such as one nitrogen atom is substituted), for example by $C_{1-4}$alkyl, C(O)H, C(O)$C_{1-4}$alkyl, C(O)O$C_{1-4}$alkyl, C(0)O$C_{1-4}$alkylaryl such as C(O)OBz, C(O)NH$C_{1-4}$alkyl, C(O)NH$C_{1-4}$alkylaryl such as C(O)NHBz, an Fmoc group, C(O)$C_{1-4}$haloalkyl, C(O)O$C_{1-4}$haloalkyl or C(0)NH$C_{1-4}$haloalkyl such as C(O)OtBu. When any Q is S, the S atoms can be substituted (such as one S atom is substituted) by one or two oxygen atoms (i.e. S(O) or $S(O)_2$). Alternatively, any sulphur atom(s) in the $C_{3-6}$heterocycloalkyl ring is not substituted.

The heterocycloalkyl groups may also have the following structures:

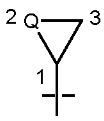
$C_3$ heterocycloalkyl

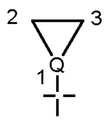
$C_3$ heterocycloalkyl

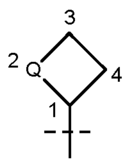
$C_4$ heterocycloalkyl

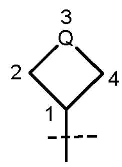
$C_4$ hetero-
cycloalkyl

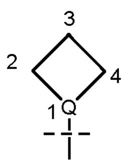
$C_4$ heterocycloalkyl

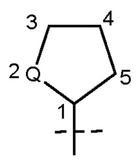
$C_5$ heterocycloalkyl

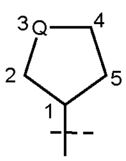
$C_5$ heterocycloalkyl

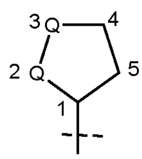
$C_5$ heterocycloalkyl

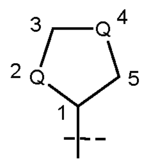
$C_5$ heterocycloalkyl

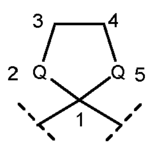
$C_5$ heterocycloalkyl

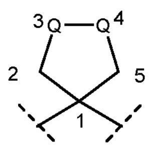
$C_5$ heterocycloalkyl

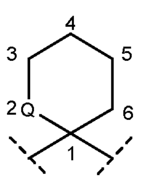
$C_6$ heterocycloalkyl

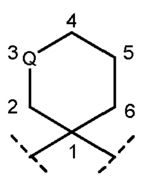
$C_6$ heterocycloalkyl

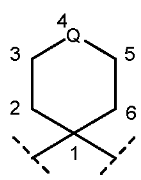
$C_6$ heterocycloalkyl

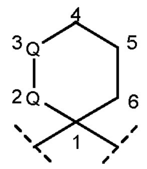
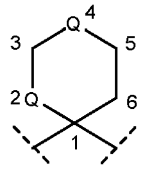
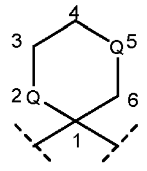
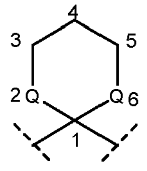
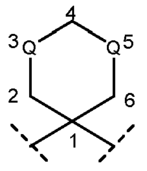

$C_6$ heterocycloalkyl $C_6$ heterocycloalkyl $C_6$ heterocycloalkyl $C_6$ heterocycloalkyl $C_6$ heterocycloalkyl

wherein each Q is independently selected from O, N or S, such as O or N. When Q is N, as required by valency, the nitrogen atom(s) may be connected to a hydrogen atom to form an NH group. Alternatively the nitrogen atom(s) may be substituted (such as one nitrogen atom is substituted), for example by $C_{1-4}$alkyl, C(O)H, C(O)$C_{1-4}$alkyl, C(O)O$C_{1-4}$alkyl, C(0)O$C_{1-4}$alkylaryl such as C(O)OBz, C(O)NH$C_{1-4}$alkyl, C(O)NH$C_{1-4}$alkylaryl such as C(O)NHBz, an Fmoc group, C(O)$C_{1-4}$haloalkyl, C(O)O$C_{1-4}$haloalkyl or C(0)NH$C_{1-4}$haloalkyl such as C(O)OtBu. When any Q is S, the S atoms can be substituted (such as one S atom is substituted) by one or two oxygen atoms (i.e. S(O) or $S(O)_2$). Alternatively, any sulphur atom(s) in the $C_{3-6}$heterocycloalkyl ring is not substituted.

When A is -C(=O)NH and $R_4$ and/or $R_5$ is Coalkylene$C_{3-6}$heterocycloalkyl, or when $R_4$ and $R_5$ together with the carbon atom to which they are attached form a $C_{3-6}$heterocycloalkyl, any heteroatom in the heterocycloalkyl may not be directly connected to the carbon to which $R_4$ and $R_5$ are connected.

Suitably, heterocycloalkyl is a fully saturated hydrocarbon ring containing the specified number of carbon atoms wherein at least one of the carbon atoms is replaced by a heteroatom such as N, S or O wherein as required by valency, any nitrogen atom is connected to a hydrogen atom, and wherein the S atom is not present as an oxide.

The term 'halo' or 'halogen' as used herein, refers to fluorine, chlorine, bromine or iodine. Particular examples of halo are fluorine and chlorine, especially fluorine.

The term 'haloalkyl' as used herein, such as in $C_{1-6}$haloalkyl, such as in $C_{1-4}$haloalkyl, whether alone or forming part of a larger group such as an Ohaloalkyl group, such as in O$C_{1-6}$haloalkyl,such as in O$C_{1-4}$haloalkyl, is a straight or a branched fully saturated hydrocarbon chain containing the specified number of carbon atoms and at least one halogen atom, such as fluoro or chloro, especially fluoro. An example of haloalkyl is $CF_3$. Further examples of haloalkyl are $CHF_2$ and $CH_2CF_3$. Examples of Ohaloalkyl include $OCF_3$, $OCHF_2$ and $OCH_2CF_3$.

The term '6-membered aryl' as used herein refers to a phenyl ring.

The term '6-membered heteroaryl' as used herein refers to 6-membered aromatic rings containing at least one heteroatom (e.g. nitrogen). Exemplary 6-membered heteroaryls include one nitrogen atom (pyridinyl), two nitrogen atoms (pyridazinyl, pyrimidinyl or pyrazinyl) and three nitrogen atoms (triazinyl).

The phrase 'in the para position relative to the amide' as used herein, such as in relation to the position of Ar2, means that compounds with the following substructure are formed:

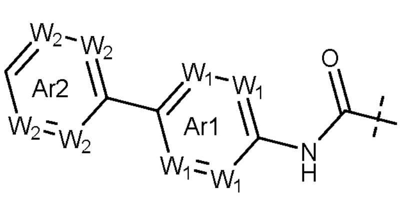

or

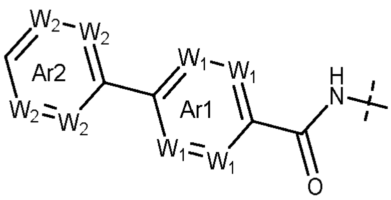

wherein $W_1$ may be N, CH, $CR_{10}$ or $CR_{11}$, and $W_2$ may be N, CH or $CR_{12}$ as allowed by the definitions provided for compounds of formula (I). $W_2$ may also be $CR_{13}$ as allowed by the definitions provided for compounds of formula (I).

The terms 'ortho' and 'meta' as used herein, such as when used in respect of defining the position of $R_{12}$ on Ar2 is with respect to Ar1, means that the following structures may form:

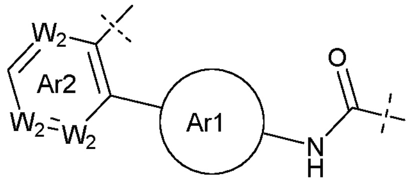

*ortho*

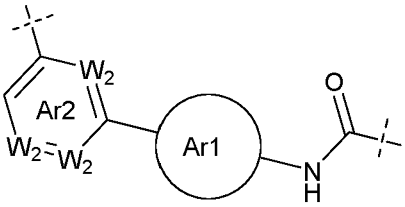

*meta*

The phrase 'A is an amide linker having the following structure: -C(=O)NH- or -NHC(=O)-' means the following structures form:

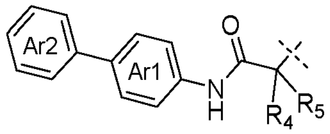

and

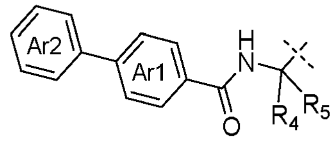

-NH(C=O)-    -(C=O)NH-

In one embodiment, A is -C(=O)NH-. In another embodiment, A is -NHC(=O)-.

In one embodiment X is N. In another embodiment, X is CH.

In one embodiment, Y is N. In another embodiment, Y is $CR_2$.

In one embodiment, Z is N. In another embodiment, Z is $CR_3$.

Suitably, X is N, Y is $CR_2$ and Z is $CR_3$. Alternatively, X is CH, Y is N and Z is $CR_3$. Alternatively, X is CH, Y is $CR_2$ and Z is $CR_3$. Alternatively, X is CH, Y is $CR_2$ and Z is N. Alternatively, X is N, Y is $CR_2$ and Z is N.

In one embodiment of the invention $R_1$ is $C_{1-5}$alkyl. When $R_1$ is $C_{1-5}$alkyl, $R_1$ may be methyl, ethyl, propyl (n-propyl or isopropyl), butyl (n-butyl, isobutyl, sec-butyl or tert-butyl) or pentyl (e.g. n-pentyl, sec-pentyl or 3-pentyl).

In a second embodiment of the invention $R_1$ is $C_{0-2}$alkylene$C_{3-5}$cycloalkyl which cycloalkyl is optionally substituted by $CH_3$. In some embodiments, $R_1$ is $C_{0-2}$alkylene$C_{3-5}$cycloalkyl. In other embodiments, $R_1$ is $C_{0-2}$alkylene$C_{3-5}$cycloalkyl which cycloalkyl is substituted by $CH_3$. $R_1$ may be $C_{3-5}$cycloalkyl, which cycloalkyl is optionally substituted by $CH_3$. $R_1$ may be $C_1$alkylene$C_{3-5}$cycloalkyl, which cycloalkyl is optionally substituted by $CH_3$. $R_1$ may be $C_2$alkylene$C_{3-5}$cycloalkyl, which cycloalkyl is optionally substituted by $CH_3$. $R_1$ may be $C_{0-2}$alkylene$C_3$cycloalkyl, which cycloalkyl is optionally substituted by $CH_3$. $R_1$ may be $C_{0-2}$alkylene$C_4$cycloalkyl, which cycloalkyl is optionally substituted by $CH_3$. $R_1$ may be $C_{0-2}$alkylene$C_5$cycloalkyl, which cycloalkyl is optionally substituted by $CH_3$. Suitably, where $C_{0-2}$alkylene$C_{3-5}$cycloalkyl is optionally substituted by $CH_3$, the $CH_3$ is at the point of attachment of the $C_{3-5}$cycloalkyl to the $C_{0-2}$alkylene.

In a third embodiment, $R_1$ is $CF_3$.

Suitably $R_1$ is cyclopropyl, cyclopropyl substituted by $CH_3$ at the point of attachment, cyclobutyl, $CH_3$ or $CH_2CH_3$. In particular $R_1$ is cyclopropyl, cyclobutyl, $CH_3$ or $CH_2CH_3$, especially cyclopropyl.

In one embodiment, $R_2$ is H. In a second embodiment, $R_2$ is halo such as F, Cl or Br, e.g. Cl or Br. In a third embodiment, $R_2$ is $C_{1-2}$alkyl. When $R_2$ is $C_{1-2}$alkyl, $R_2$ may be methyl or ethyl, such as methyl. In a fourth embodiment, $R_2$ is $OC_{1-2}$alkyl. When $R_2$ is $OC_{1-2}$alkyl, may be $OCH_3$ or OEt, such as $OCH_3$. In a fifth embodiment, $R_2$ is $C_{1-2}$haloalkyl. When $R_2$ is $C_{1-2}$haloalkyl, $R_2$ may be $CF_3$ or $CH_2CF_3$, such as $CF_3$. In a sixth embodiment, $R_2$ is $OC_{1-2}$haloalkyl. When $R_2$ is $OC_{1-2}$haloalkyl, $R_2$ may be $OCF_3$ or $OCH_2CF_3$, such as $OCF_3$.

Suitably, $R_2$ is H, $CH_3$ or $CF_3$, such as H or $CH_3$, in particular H.

In one embodiment $R_3$ is H. In a second embodiment $R_3$ is halo, in particular chloro or fluoro, especially fluoro. In a third embodiment, $R_3$ is $CH_3$. In a fourth embodiment, $R_3$ is $OCH_3$. In a fifth embodiment, $R_3$ is $CF_3$. In a sixth embodiment, $R_3$ is $OCF_3$.

Suitably, $R_3$ is H, halo in particular chloro or fluoro, especially fluoro, $CH_3$ or $CF_3$. More suitably, $R_3$ is H or F, such as H.

Suitably, at least one of $R_2$ and $R_3$ is H.

In one embodiment, $R_4$ and $R_5$ together with the carbon atom to which they are attached form a $C_{3-6}$cycloalkyl, such as cyclopropyl, cyclobutyl or cyclopentyl in particular cyclopropyl or cyclopentyl. In a second embodiment, $R_4$ and $R_5$ together with the carbon atom to which they are attached form a $C_{3-6}$heterocycloalkyl, such as a heterocyclohexyl, in particular a tetrahydropyranyl. Any nitrogen atom such as one nitrogen atom in the $C_{3-6}$heterocycloalkyl ring may be substituted, for example by $C_{1-4}$alkyl, C(O)H, C(O)$C_{1-4}$alkyl, C(O)O$C_{1-4}$alkyl, C(O)O$C_{1-4}$alkylaryl such as C(O)OBz, C(O)NH$C_{1-4}$alkyl, C(O)NH$C_{1-4}$alkylaryl such as C(O)NHBz, an Fmoc group, C(O)$C_{1-4}$haloalkyl, C(O)O$C_{1-4}$haloalkyl or C(0)NH$C_{1-4}$haloalkyl such as C(O)OtBu. Suitably, any nitrogen atom in the $C_{3-6}$heterocycloalkyl ring is not substituted. In a third embodiment, $R_4$ is $C_{1-6}$alkyl, in particular $C_{1-4}$alkyl such as methyl, ethyl, propyl (n-propyl or isopropyl) or butyl (n-butyl, isobutyl, sec-butyl or tert-butyl). In a fourth embodiment, $R_4$ is $C_{1-3}$alkyleneO$C_{1-3}$alkyl, in particular $C_{1-2}$alkyleneO$C_{1-2}$alkyl such as $C_1$alkyleneO$C_1$alkyl, $C_2$alkyleneO$C_1$alkyl, $C_1$alkyleneO$C_2$alkyl or $C_2$alkyleneO$C_2$alkyl. In a fifth embodiment, $R_4$ is H. In a sixth embodiment, $R_4$ is halo, such as chloro or fluoro, especially fluoro. In a seventh embodiment, $R_4$ is $C_{1-6}$haloalkyl, such as $CF_3$ or $CH_2CF_3$. In an eighth embodiment, $R_4$ is $C_{0-2}$alkylene$C_{3-6}$cycloalkyl such as $C_{3-6}$cycloalkyl, $C_1$alkylene$C_{3-6}$cycloalkyl, $C_2$alkylene$C_{3-6}$cycloalkyl, $C_{0-2}$alkylene$C_3$cycloalkyl, $C_{0-2}$alkylene$C_4$cycloalkyl, $C_{0-2}$alkylene$C_5$cycloalkyl or $C_{0-2}$alkylene$C_6$cycloalkyl. In a ninth embodiment, $R_4$ is $C_{0-2}$alkylene$C_{3-6}$heterocycloalkyl such as $C_{3-6}$heterocycloalkyl, $C_1$alkylene$C_{3-6}$heterocycloalkyl,

$C_2$alkylene$C_{3-6}$heterocycloalkyl, $C_{0-2}$alkylene$C_3$heterocycloalkyl, $C_{0-2}$alkylene$C_4$hetero-cycloalkyl, $C_{0-2}$alkylene$C_5$heterocycloalkyl or $C_{0-2}$alkylene$C_6$heterocycloalkyl. Suitably the heterocycloalkyl is a heterocyclopropyl, heterocyclobutyl, heterocyclopentyl or heterocyclohexyl ring such as a heterocyclohexyl ring. Suitably, the heterocyclopentyl ring is tetrahydrofuranyl or pyrrolidinyl. Suitably, the heterocyclohexyl ring is tetrahydropyranyl or piperidinyl. Any nitrogen atom such as one nitrogen atom in the $C_{3-6}$heterocycloalkyl ring may be substituted, for example by $C_{1-4}$alkyl, C(O)H, C(O)$C_{1-4}$alkyl, C(O)O$C_{1-4}$alkyl, C(0)O$C_{1-4}$alkylaryl such as C(O)OBz, C(O)NH$C_{1-4}$alkyl, C(O)NH$C_{1-4}$alkylaryl such as C(O)NHBz, an Fmoc group, C(O)$C_{1-4}$haloalkyl, C(O)O$C_{1-4}$haloalkyl or C(0)NH$C_{1-4}$haloalkyl such as C(O)OtBu. Suitably, any nitrogen atom in the $C_{3-6}$heterocycloalkyl ring is not substituted. In a tenth embodiment, $R_4$ is $C_{1-6}$alkylOH, such as $CH_2OH$ or $CH_2CH_2OH$. In an eleventh embodiment, $R_4$ is O$C_{1-6}$haloalkyl, such as O$C_{1-4}$haloalkyl, such as $OCF_3$ or $OCHF_2$. In a twelfth embodiment, $R_4$ is O$C_{0-2}$alkylene$C_{3-6}$cycloalkyl such as O$C_{3-6}$cycloalkyl, O$C_1$alkylene$C_{3-6}$cycloalkyl, O$C_2$alkylene$C_{3-6}$cycloalkyl, O$C_{0-2}$alkylene$C_3$cycloalkyl, O$C_{0-2}$alkylene$C_4$cycloalkyl, O$C_{0-2}$alkylene$C_5$cycloalkyl or O$C_{0-2}$alkylene$C_6$cycloalkyl. In a thirteenth embodiment, $R_4$ is O$C_{1-6}$alkyl, in particular O$C_{1-4}$alkyl such as methoxy, ethoxy, propoxy (n-propoxy or isopropoxy) or butoxy (n-butoxy, isobutoxy, sec-butoxy or tert-butoxy). In a fourteenth embodiment, $R_4$ is O$C_{0-2}$alkylene$C_{3-6}$heterocycloalkyl such as O$C_{3-6}$heterocycloalkyl, O$C_1$alkylene$C_{3-6}$heterocycloalkyl, O$C_2$alkylene$C_{3-6}$heterocycloalkyl, O$C_{0-2}$alkylene$C_3$heterocycloalkyl, O$C_{0-2}$alkylene$C_4$hetero-cycloalkyl, O$C_{0-2}$alkylene$C_5$heterocycloalkyl or O$C_{0-2}$alkylene$C_6$heterocycloalkyl. Suitably the heterocycloalkyl is a heterocyclopropyl, heterocyclobutyl, heterocyclopentyl or heterocyclohexyl ring such as a heterocyclohexyl ring. Suitably, the heterocyclopentyl ring is tetrahydrofuranyl or pyrrolidinyl. Suitably, the heterocyclohexyl ring is tetrahydropyranyl or piperidinyl. Any nitrogen atom such as one nitrogen atom in the $C_{3-6}$heterocycloalkyl ring may be substituted, for example by $C_{1-4}$alkyl, C(O)H, C(O)$C_{1-4}$alkyl, C(O)O$C_{1-4}$alkyl, C(0)O$C_{1-4}$alkylaryl such as C(O)OBz, C(O)NH$C_{1-4}$alkyl, C(O)NH$C_{1-4}$alkylaryl such as C(O)NHBz, an Fmoc group, C(O)$C_{1-4}$haloalkyl, C(O)O$C_{1-4}$haloalkyl or C(0)NH$C_{1-4}$haloalkyl such as C(O)OtBu. Suitably, any nitrogen atom in the $C_{3-6}$heterocycloalkyl ring is not substituted. In a fifteenth embodiment, $R_4$ is $NR_{21}R_{22}$.

When A is -NHC(=O)- or -C(=O)NH-, suitably, $R_4$ is H, $C_{1-6}$alkyl, $C_{1-6}$haloalkyl, $C_{1-6}$alkylOH, $C_{0-2}$alkylene$C_{3-6}$cycloalkyl, $C_{0-2}$alkylene$C_{3-6}$heterocycloalkyl, $C_{1-3}$alkyleneO$C_{1-3}$alkyl, or $R_4$ and $R_5$ together with the carbon atom to which they are attached form a $C_{3-6}$cycloalkyl or $C_{3-6}$heterocycloalkyl. When A is -NHC(=O)-, suitably $R_4$ may additionally be selected from halo, O$C_{1-6}$haloalkyl, O$C_{0-2}$alkylene$C_{3-6}$cycloalkyl, O$C_{0-2}$alkylene$C_{3-6}$heterocycloalkyl, O$C_{1-6}$alkyl or $NR_{21}R_{22}$.

Suitably $R_4$ is H, fluoro, $CH_3$, ethyl, $OCH_3$ or $CH_2CH_2OCH_3$, such as fluoro, ethyl, $OCH_3$ or $CH_2CH_2OCH_3$.

Suitably $R_4$ is H, $CH_3$, ethyl or $CH_2CH_2OCH_3$, in particular $CH_3$ or ethyl.

Suitably $R_4$ and $R_5$ together with the carbon atom to which they are attached form a cyclopropyl or cyclopentyl, in particular a cyclopentyl.

Suitably $R_4$ and $R_5$ together with the carbon atom to which they are attached form a heterocyclohexyl, such as tetrahydropyranyl or piperidinyl, especially tetrahydropyranyl. Any nitrogen atom such as one nitrogen atom in the $C_{3-6}$heterocycloalkyl ring may be substituted, for example by $C_{1-4}$alkyl, C(O)H, C(O)$C_{1-4}$alkyl, C(O)O$C_{1-4}$alkyl, C(0)O$C_{1-4}$alkylaryl such as C(O)OBz, C(O)NH$C_{1-4}$alkyl, C(O)NH$C_{1-4}$alkylaryl such as C(O)NHBz, an Fmoc group, C(O)$C_{1-4}$haloalkyl, C(O)O$C_{1-4}$haloalkyl or C(0)NH$C_{1-4}$haloalkyl such as C(O)OtBu. Suitably, any nitrogen atom in the $C_{3-6}$heterocycloalkyl ring is not substituted.

Suitably $R_4$ and $R_5$ together with the carbon atom to which they are attached form a heterocyclobutyl, such as azetidinyl. Any nitrogen atom such as one nitrogen atom in the $C_{3-6}$heterocycloalkyl ring may be substituted, for example by $C_{1-4}$alkyl, C(O)H, C(O)$C_{1-4}$alkyl, C(O)O$C_{1-4}$alkyl, C(0)O$C_{1-4}$alkylaryl such as C(O)OBz, C(O)NH$C_{1-4}$alkyl, C(O)NH$C_{1-4}$alkylaryl such as C(O)NHBz, an Fmoc group, C(O)$C_{1-4}$haloalkyl, C(O)O$C_{1-4}$haloalkyl or C(O)NH$C_{1-4}$haloalkyl such as C(O)OtBu. Suitably, any nitrogen atom in the $C_{3-6}$heterocycloalkyl ring is not substituted.

When $R_4$ is $NR_{21}R_{22}$, in one embodiment $R_{21}$ is H. In a second embodiment $R_{21}$ is $C_{1-5}$alkyl, such as methyl, ethyl or propyl, especially methyl. In a third embodiment $R_{21}$ is C(O)$C_{1-5}$alkyl, such as C(O)$CH_3$. In a fourth embodiment $R_{21}$ is C(O)O$C_{1-5}$alkyl, such as C(O)O$CH_3$ or C(O)Otert-butyl.

When $R_4$ is $NR_{21}R_{22}$, in one embodiment $R_{22}$ is H. In a second embodiment $R_{22}$ is methyl.

For example, $R_4$ is $NH_2$, $N(CH_3)_2$, NHC(O)$CH_3$, NHC(O)O$CH_3$, NHC(O)Otert-butyl and $CH_2CH_2OH$, especially, $N(CH_3)_2$, NHC(O)$CH_3$, NHC(O)O$CH_3$.

Suitably, $R_{21}$ is C(O)O$CH_3$ and $R_{22}$ is H. Suitably, $R_{21}$ is C(O)$CH_3$ and $R_{22}$ is H. Suitably, $R_{21}$ and $R_{22}$ are both $CH_3$. Suitably, $R_{21}$ and $R_{22}$ are both H.

In one embodiment $R_5$ is $C_{1-6}$alkyl, in particular $C_{1-4}$alkyl such as methyl, ethyl, propyl (n-propyl or isopropyl) or butyl (n-butyl, isobutyl, sec-butyl or tert-butyl). In a second embodiment $R_5$ is $C_{1-3}$alkyleneO$C_{1-3}$alkyl, in particular $C_{1-2}$alkyleneO$C_{1-2}$alkyl such as $C_1$alkyleneO$C_1$alkyl, $C_2$alkyleneO$C_1$alkyl, $C_1$alkyleneO$C_2$alkyl or $C_2$alkyleneO$C_2$alkyl. In a third embodiment $R_5$ is H. In a fourth embodiment, $R_5$ is halo, such as chloro or fluoro, especially fluoro. In a fifth embodiment, $R_5$ is $C_{1-6}$haloalkyl, such as $CF_3$ or $CH_2CF_3$. In a sixth embodiment, $R_5$ is $C_{0-2}$alkylene$C_{3-6}$cycloalkyl such as $C_{3-6}$cycloalkyl, $C_1$alkylene$C_{3-6}$cycloalkyl, $C_2$alkylene$C_{3-6}$cycloalkyl, $C_{0-2}$alkylene$C_3$cycloalkyl, $C_{0-2}$alkylene$C_4$cycloalkyl, $C_{0-2}$alkylene$C_5$cycloalkyl or $C_{0-2}$alkylene$C_6$cycloalkyl. In a seventh embodiment, $R_5$ is

$C_{0-2}$alkylene$C_{3-6}$heterocycloalkyl such as $C_{3-6}$heterocycloalkyl, $C_1$alkylene$C_{3-6}$heterocycloalkyl, $C_2$alkylene$C_{3-6}$heterocycloalkyl, $C_{0-2}$alkylene$C_3$heterocycloalkyl, $C_{0-2}$alkylene$C_4$hetero-cycloalkyl, $C_{0-2}$alkylene$C_5$heterocycloalkyl or $C_{0-2}$alkylene$C_6$heterocycloalkyl. Suitably the heterocycloalkyl is a heterocyclopropyl, heterocyclobutyl, heterocyclopentyl or heterocyclohexyl ring such as a heterocyclohexyl ring. Suitably, the heterocyclopentyl ring is tetrahydrofuranyl or pyrrolidinyl. Suitably, the heterocyclohexyl ring is tetrahydropyranyl or piperidinyl. Any nitrogen atom such as one nitrogen atom in the $C_{3-6}$heterocycloalkyl ring may be substituted, for example by $C_{1-4}$alkyl, C(O)H, C(O)$C_{1-4}$alkyl, C(O)O$C_{1-4}$alkyl, C(0)O$C_{1-4}$alkylaryl such as C(O)OBz, C(O)NH$C_{1-4}$alkyl, C(O)NH$C_{1-4}$alkylaryl such as C(O)NHBz, an Fmoc group, C(O)$C_{1-4}$haloalkyl, C(O)O$C_{1-4}$haloalkyl or C(0)NH$C_{1-4}$haloalkyl such as C(O)OtBu. Suitably, any nitrogen atom in the $C_{3-6}$heterocycloalkyl ring is not substituted. In an eighth embodiment, $R_5$ is $C_{1-6}$alkylOH, such as $CH_2OH$ or $CH_2CH_2OH$. In a ninth embodiment, $R_5$ is O$C_{1-6}$haloalkyl, such as O$C_{1-4}$haloalkyl, such as $OCF_3$ or $OCHF_2$. In a tenth embodiment, $R_5$ is O$C_{0-2}$alkylene$C_{3-6}$cycloalkyl such as O$C_{3-6}$cycloalkyl, O$C_1$alkylene$C_{3-6}$cycloalkyl, O$C_2$alkylene$C_{3-6}$cycloalkyl, O$C_{0-2}$alkylene$C_3$cycloalkyl, O$C_{0-2}$alkylene$C_4$cycloalkyl, O$C_{0-2}$alkylene$C_5$cycloalkyl or O$C_{0-2}$alkylene$C_6$cycloalkyl. In an eleventh embodiment, $R_5$ is O$C_{1-6}$alkyl, in particular O$C_{1-4}$alkyl such as methoxy, ethoxy, propoxy (n-propoxy or isopropoxy) or butoxy (n-butoxy, isobutoxy, sec-butoxy or tert-butoxy). In a twelfth embodiment, $R_5$ is O$C_{0-2}$alkylene$C_{3-6}$heterocycloalkyl such as O$C_{3-6}$heterocycloalkyl, O$C_1$alkylene$C_{3-6}$heterocycloalkyl, O$C_2$alkylene$C_{3-6}$heterocycloalkyl, O$C_{0-2}$alkylene$C_3$heterocycloalkyl, O$C_{0-2}$alkylene$C_4$heterocycloalkyl, O$C_{0-2}$alkylene$C_5$heterocycloalkyl or O$C_{0-2}$alkylene$C_6$heterocycloalkyl. Suitably the heterocycloalkyl is a heterocyclopropyl, heterocyclobutyl, heterocyclopentyl or heterocyclohexyl ring such as a heterocyclohexyl ring. Suitably, the heterocyclopentyl ring is tetrahydrofuranyl or pyrrolidinyl. Suitably, the heterocyclohexyl ring is tetrahydropyranyl or piperidinyl. Any nitrogen atom such as one nitrogen atom in the $C_{3-6}$heterocycloalkyl ring may be substituted, for example by $C_{1-4}$alkyl, C(O)H, C(O)$C_{1-4}$alkyl, C(O)O$C_{1-4}$alkyl, C(0)O$C_{1-4}$alkylaryl such as C(O)OBz, C(O)NH$C_{1-4}$alkyl, C(O)NH$C_{1-4}$alkylaryl such as C(O)NHBz, an Fmoc group, C(O)$C_{1-4}$haloalkyl, C(O)O$C_{1-4}$haloalkyl or C(0)NH$C_{1-4}$haloalkyl such as C(O)OtBu. Suitably, any nitrogen atom in the $C_{3-6}$heterocycloalkyl ring is not substituted. In a thirteenth embodiment, $R_5$ is $NR_{21}R_{22}$.

When A is -NHC(=O)- or -C(=O)NH-, suitably, $R_5$ is H, $C_{1-6}$alkyl, $C_{1-6}$haloalkyl, $C_{1-6}$alkylOH, $C_{0-2}$alkylene$C_{3-6}$cycloalkyl, $C_{0-2}$alkylene$C_{3-6}$heterocycloalkyl, $C_{1-3}$alkyleneO$C_{1-3}$alkyl, or $R_4$ and $R_5$ together with the carbon atom to which they are attached form a $C_{3-6}$cycloalkyl or $C_{3-6}$heterocycloalkyl. When A is -NHC(=O)-, suitably $R_5$ may additionally be selected from halo, O$C_{1-6}$haloalkyl, O$C_{0-2}$alkylene$C_{3-6}$cycloalkyl, O$C_{0-2}$alkylene$C_{3-6}$heterocycloalkyl, O$C_{1-6}$alkyl or $NR_{21}R_{22}$.

When $R_5$ is $NR_{21}R_{22}$, in one embodiment $R_{21}$ is H. In a second embodiment $R_{21}$ is $C_{1-5}$alkyl, such as methyl, ethyl or propyl, especially methyl. In a third embodiment $R_{21}$ is C(O)$C_{1-5}$alkyl, such as C(O)$CH_3$. In a fourth embodiment $R_{21}$ is C(O)O$C_{1-5}$alkyl, such as C(O)O$CH_3$ or C(O)Otert-butyl.

When $R_5$ is $NR_{21}R_{22}$, in one embodiment $R_{22}$ is H. In a second embodiment $R_{22}$ is methyl.

For example, $R_5$ is $NH_2$, $N(CH_3)_2$, NHC(O)$CH_3$, NHC(O)O$CH_3$, NHC(O)Otert-butyl and $CH_2CH_2OH$, especially, $N(CH_3)_2$, NHC(O)$CH_3$, NHC(O)O$CH_3$.

Suitably, $R_{21}$ is C(O)O$CH_3$ and $R_{22}$ is H. Suitably, $R_{21}$ is C(O)$CH_3$ and $R_{22}$ is H. Suitably, $R_{21}$ and $R_{22}$ are both $CH_3$. Suitably, $R_{21}$ and $R_{22}$ are both H.

Suitably $R_5$ is H, F, $CH_3$ or ethyl such as H, $CH_3$ or ethyl.

Suitably $R_4$ is H, $CH_3$, ethyl or $CH_2CH_2OCH_3$ and $R_5$ is H, $CH_3$ or ethyl, in particular $R_4$ is $CH_3$, or ethyl and $R_5$ is H, methyl or ethyl. For example, $R_4$ and $R_5$ are H, $R_4$ and $R_5$ are methyl, $R_4$ and $R_5$ are ethyl or $R_4$ is $CH_2CH_2OCH_3$ and $R_5$ is H.

Suitably, $R_4$ is F and $R_5$ is ethyl.

Suitably, $R_4$ is F and $R_5$ is F.

Suitably, $R_4$ is ethyl and $R_5$ is H.

Suitably $R_4$ and $R_5$ are arranged in the following configuration:

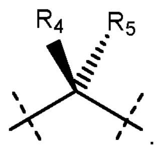

In one embodiment Ar1 is a 6-membered aryl, i.e. phenyl. In a second embodiment Ar1 is a 6-membered heteroaryl, in particular containing one nitrogen atom (pyridyl) or two nitrogen atoms (pyridazinyl, pyrimidinyl or pyrazinyl).

In particular Ar1 is phenyl, 2-pyridyl or 3-pyridyl, such as phenyl or 2-pyridyl. The position numbering for Ar1 is in respect of the amide, with the carbon at the point of attachment designated position 1 and other numbers providing the relative location of the nitrogen atoms, for example:

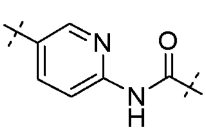
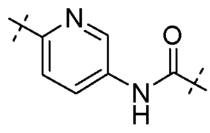

2-pyridyl ; 3-pyridyl .

In one embodiment $R_{10}$ is H. In a second embodiment $R_{10}$ is halo, for example fluoro or chloro. In a third embodiment $R_{10}$ is $C_{1-3}$alkyl such as $C_{1-2}$alkyl, such as $CH_3$ or ethyl. In a fourth embodiment $R_{10}$ is $OC_{1-2}$alkyl, such as $OCH_3$ or ethoxy. In a fifth embodiment $R_{10}$ is $OC_{1-2}$haloalkyl, such as $OCF_3$. In a sixth embodiment $R_{10}$ is CN. In a seventh embodiment, $R_{10}$ is $C_{1-2}$haloalkyl such as $CF_3$.

Suitably $R_{10}$ is H, fluoro, chloro, $CH_3$, $CF_3$, $OCH_3$, $OCF_3$ or CN, such as H, fluoro, chloro, $CH_3$, $OCH_3$, $OCF_3$ or CN, in particular H, fluoro, chloro, $OCH_3$, $OCF_3$ or CN especially H or fluoro.

Suitably, $R_{10}$ is H, For $CH_3$.

In one embodiment $R_{11}$ is H. In a second embodiment $R_{11}$ is F. In a third embodiment, $R_{11}$ is $C_{1-2}$alkyl such as $CH_3$ or Et, such as $CH_3$. In a fourth embodiment $R_{11}$ is $OCH_3$. In a fifth embodiment, $R_{11}$ is Cl. In a sixth embodiment, $R_{11}$ is Et. In a seventh embodiment, $R_{11}$ is $CF_3$. In an eighth embodiment, $R_{11}$ is CN.

Suitably, $R_{11}$ is H, F, $CH_3$ or $OCH_3$, such as H, F or $CH_3$, such as H or F, such as H.

In one embodiment, $R_{10}$ is in the ortho position with respect to the amide. In another embodiment, $R_{10}$ is in the meta position with respect to the amide. Suitably $R_{10}$ is in the ortho position with respect to the amide.

In one embodiment, $R_{11}$ is in the ortho position with respect to the amide. In another embodiment, $R_{11}$ is in the meta position with respect to the amide. Suitably $R_{11}$ is in the ortho position with respect to the amide.

In one embodiment Ar2 is a 6-membered aryl, i.e. phenyl. In a second embodiment Ar2 is a 6-membered heteroaryl, in particular containing one nitrogen atom (pyridyl) or two nitrogen atoms (pyridazinyl, pyrimidinyl or pyrazinyl).

The position numbering for Ar2 is in respect of the point of attachment to Ar1, for example:

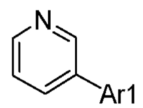
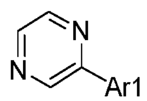

3-pyridyl 2,5-pyrazinyl .

In particular Ar2 is 3-pyridyl or 2,5-pyrazinyl, especially 2,5-pyrazinyl.

In one embodiment $R_{12}$ is H. In a second embodiment $R_{12}$ is halo, for example fluoro or chloro. In a third embodiment $R_{12}$ is $C_{1-4}$alkyl, such as methyl, ethyl, propyl (n-propyl or isopropyl) or butyl (n-butyl, isobutyl, sec-butyl or *tert*-butyl). In a fourth embodiment $R_{12}$ is $OC_{1-4}$alkyl, such as $OCH_3$, ethoxy, isopropoxy or n-propoxy. In a fifth embodiment $R_{12}$ is $OC_{0-2}$alkylene$C_{3-5}$cycloalkyl, such as $OC_{3-5}$cycloalkyl (e.g. cyclopropoxy or cyclobutoxy), $OC_1$alkylene$C_{3-5}$cycloalkyl or $OC_2$alkylene$C_{3-5}$cycloalkyl. In a sixth embodiment $R_{12}$ is CN. In a seventh embodiment $R_{12}$ is $C_{1-4}$haloalkyl, such as $CF_3$. In an eighth embodiment $R_{12}$ is $OC_{1-4}$haloalkyl, such as $OCF_3$, $OCHF_2$ or $OCH_2CF_3$. In a ninth embodiment, $R_{12}$ is $C_{2-4}$alkenyl such as $C(=CH_2)CH_3$. In a tenth embodiment, $R_{12}$ is $C_{0-2}$alkylene$C_{3-5}$cycloalkyl such as $C_{3-5}$cycloalkyl, $C_1$alkylene$C_{3-5}$cycloalkyl, $C_2$alkylene$C_{3-5}$cycloalkyl, $C_{0-2}$alkylene$C_3$cycloalkyl, $C_{0-2}$alkylene$C_4$cycloalkyl or $C_{0-2}$alkylene$C_5$cycloalkyl. In an eleventh embodiment, $R_{12}$ is hydroxy. In a twelfth embodiment, $R_{12}$ is $C_{1-4}$alkylOH such as $CH_2OH$. In a thirteenth embodiment, $R_{12}$ is $SO_2C_{1-2}$alkyl such as $SO_2CH_3$. In a fourteenth embodiment, $R_{12}$ is $C(O)N(C_{1-2}alkyl)_2$ such as $C(O)N(CH_3)_2$. In a fifteenth embodiment, $R_{12}$ is $NHC(O)C_{1-3}$alkyl. In a sixteenth embodiment, $R_{12}$ is $NR_{23}R_{24}$. In a seventeenth embodiment, $R_{12}$ is $OCH_2CH_2N(CH_3)_2$. In an eighteenth embodiment, $R_{12}$ is a $C_{3-6}$heterocycloalkyl comprising one nitrogen located at the point of attachment to Ar2. Suitably the heterocycloalkyl is a heterocyclopropyl, heterocyclobutyl, heterocyclopentyl or heterocyclohexyl ring such as a heterocyclohexyl ring. Suitably, the heterocyclopentyl ring is pyrrolidinyl. Suitably, the heterocyclohexyl ring is piperidinyl or piperazinyl. Any nitrogen atom such as one nitrogen atom in the $C_{3-6}$heterocycloalkyl ring may be substituted, for example by $C_{1-4}$alkyl, C(O)H, $C(O)C_{1-4}$alkyl, $C(O)OC_{1-4}$alkyl, $C(O)OC_{1-4}$alkylaryl such as C(O)OBz, $C(O)NHC_{1-4}$alkyl, $C(O)NHC_{1-4}$alkylaryl such as C(O)NHBz, an Fmoc group, $C(O)C_{1-4}$haloalkyl, $C(O)OC_{1-4}$haloalkyl or $C(O)NHC_{1-4}$haloalkyl such as C(O)OtBu. Suitably, any nitrogen atom in the $C_{3-6}$heterocycloalkyl ring is not substituted. In a nineteenth embodiment, $R_{12}$ together with a nitrogen atom to which it is attached forms an N-oxide ($N^+$-$O^-$).

When A is -NHC(=O)- or -C(=O)NH-, suitably, $R_{12}$ is attached to Ar2 in the ortho or meta position relative to Ar1 and $R_{12}$ is H, halo, $C_{1-4}$alkyl, $C_{2-4}$alkenyl, $C_{0-2}$alkylene$C_{3-5}$cycloalkyl, $OC_{1-4}$alkyl, $OC_{0-2}$alkylene$C_{3-5}$cycloalkyl, $C_{1-4}$haloalkyl, $OC_{1-4}$haloalkyl, hydroxy, $C_{1-4}$alkylOH, $SO_2C_{1-2}$alkyl, $C(O)N(C_{1-2}alkyl)_2$, $NHC(O)C_{1-3}$alkyl or $NR_{23}R_{24}$.

When A is -NHC(=O)-, suitably $R_{12}$ may additionally be selected from CN, $OCH_2CH_2N(CH_3)_2$ and a

$C_{3-6}$heterocycloalkyl comprising one nitrogen located at the point of attachment to Ar2, or $R_{12}$ together with a nitrogen atom to which it is attached forms an N-oxide ($N^+$-$O^-$).

The present invention provides N-oxides of the compound of formula (I). Suitably, when $R_{12}$ together with a nitrogen atom to which it is attached forms an N-oxide ($N^+$-$O^-$), the example following structures are formed:

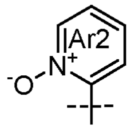
or
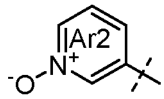
.

$R_{12}$ is suitably H, F, Cl, $CH_3$, $OCH_3$, OEt, O*i*Pr, OCyclopropyl, CN, $CF_3$, $OCHF_2$ or $OCH_2CF_3$. In particular, $R_{12}$ is Cl, CN, $CF_3$, $OCHF_2$, $OCH_2CF_3$, $OCH_3$, OEt, OiPr, OCyclopropyl, such as $CF_3$, $OCHF_2$, $OCH_2CF_3$, $OCH_3$, OEt, O*i*Pr, OCyclopropyl, e.g. OEt.

$R_{12}$ is suitably H, F, Cl, $CH_3$, iPr, $OCH_3$, OEt, O*i*Pr, OCyclopropyl, CN, $CF_3$, $OCHF_2$, $OCH_2CF_3$, $C_3$cycloalkyl or $C(=CH_2)CH_3$. In particular, $R_{12}$ is Cl, iPr, $OCH_3$, OEt, O*i*Pr, OCyclopropyl, CN, $CF_3$, $OCHF_2$, $OCH_2CF_3$, $C_3$cycloalkyl or $C(=CH_2)CH_3$, such as C!, $OCH_3$, OEt, O*i*Pr, OCyclopropyl, $CF_3$, $OCHF_2$, $OCH_2CF_3$ or $C_3$cycloalkyl, e.g. OEt.

When A is -C(=O)NH-, suitably $R_{12}$ is $CF_3$, OEt or OiPr, such as OEt or OiPr.

Suitably $R_{12}$ is in the meta position of Ar2. Alternatively, $R_{12}$ is in the ortho position of Ar2.

In one embodiment, $R_{13}$ is H. In another embodiment, $R_{13}$ is halo such as F or Cl, suitably F.

In one embodiment, $R_{13}$ is in the ortho position with respect to Ar1. In another embodiment, $R_{13}$ is in the para position with respect to Ar1. In another embodiment, $R_{13}$ is in the meta position with respect to Ar1.

In one embodiment, $R_{23}$ is H. In another embodiment, $R_{23}$ is $C_{1-2}$alkyl such as methyl.

In one embodiment, $R_{24}$ is H. In another embodiment $R_{24}$ is $C_{1-2}$alkyl such as methyl.

Suitably, $R_{23}$ is H and $R_{24}$ is ethyl. Suitably, $R_{23}$ is $CH_3$ and $R_{24}$ is $CH_3$.

Desirably, a compound of formula (I) does not include 2-(6-(methylsulfonamido)pyrazin-2-yl)-N-(4-(pyridin-3-yl)phenyl)acetamide.

In one embodiment, at least one of $R_{10}$, $R_{11}$, $R_{12}$ and $R_{13}$ is other than H.

Suitably, at least one of $R_4$, $R_5$, $R_{10}$, $R_{11}$, $R_{12}$ and $R_{13}$ is other than H.

More suitably, when $R_1$ is methyl, at least one of $R_4$, $R_5$, $R_{10}$, $R_{11}$, $R_{12}$ and $R_{13}$ is other than H.

Throughout the specification Ar1 and Ar2 may be depicted as follows:

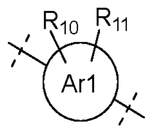
;
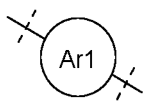
;
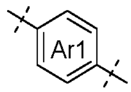
; Ar1

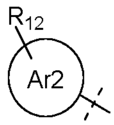
;
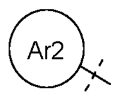
;
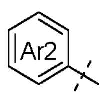
; Ar2

All depictions with respect to Ar1 are equivalent and all depictions with respect to Ar2 are equivalent, unless the context requires otherwise, depictions of Ar1 and Ar2 should not be taken to exclude the presence of heteroatoms or substitutions.

The present invention provides the compounds described in any one of Examples P1 to P111.

The present invention also provides the compounds described in any one of Examples P112 to P115.

The present invention also provides the compounds described in any one of Examples P116 to P225.

The present invention provides the following compounds:

N-(4-(5-chloropyridin-3-yl)phenyl)-2-(2-(cyclopropanesulfonamido)pyrimidin-4-yl)butanamide;

1-(2-(cyclopropanesulfonamido)pyrimidin-4-yl)-N-(4-(6-ethoxypyrazin-2-yl)phenyl)cyclopentanecarboxamide;

2-(2-(cyclopropanesulfonamido)pyrimidin-4-yl)-N-(4-(6-methoxypyrazin-2-yl)phenyl)-2-methylpropanamide;

2-(2-(cyclopropanesulfonamido)pyrimidin-4-yl)-2-methyl-N-(4-(5-(trifluoromethyl)pyridin-3-yl)phenyl)propanamide;

2-methyl-N-(2-methyl-4-(6-methylpyrazin-2-yl)phenyl)-2-(2-(methylsulfonamido)pyrimidin-4-yl)propanamide;

2-(2-(cyclopropanesulfonamido)pyrimidin-4-yl)-N-(2-fluoro-4-(pyrazin-2-yl)phenyl)butanamide;

2-(2-(cyclopropanesulfonamido)pyrimidin-4-yl)-N-(4-(5-(trifluoromethyl)pyridin-3-yl)phenyl)butanamide;

2-(2-(cyclopropanesulfonamido)pyrimidin-4-yl)-N-(4-(6-(trifluoromethyl)pyrazin-2-yl)phenyl)acetamide;

2-(2-(cyclopropanesulfonamido)pyrimidin-4-yl)-N-(5-(6-isopropoxypyrazin-2-yl)pyridin-2-yl)-2-methylpropanamide;

2-(2-(cyclopropanesulfonamido)pyrimidin-4-yl)-N-(4-(6-ethoxypyrazin-2-yl)phenyl)-2-ethylbutanamide;

2-(2-(cyclopropanesulfonamido)pyrimidin-4-yl)-N-(2-fluoro-4-(6-(trifluoromethyl)pyrazin-2-yl)phenyl)acetamide;

2-(2-(cyclopropanesulfonamido)pyrimidin-4-yl)-N-(2-fluoro-4-(6-isopropoxypyrazin-2-yl)phenyl)acetamide;

2-(2-(cyclopropanesulfonamido)pyrimidin-4-yl)-N-(4-(5-(trifluoromethyl)pyridin-3-yl)phenyl)acetamide;

2-(2-(cyclopropanesulfonamido)pyrimidin-4-yl)-N-(4-(5-(2,2,2-trifluoroethoxy)pyridin-3-yl)phenyl)acetamide;

2-(2-(cyclopropanesulfonamido)-5-fluoropyrimidin-4-yl)-N-(4-(pyridin-3-yl)phenyl)acetamide;

2-(2-(cyclopropanesulfonamido)pyrimidin-4-yl)-N-(4-(pyridin-3-yl)phenyl)acetamide;

N-([1,1'-biphenyl]-4-yl)-2-(2-(cyclopropanesulfonamido)pyrimidin-4-yl)acetamide;

2-(2-(cyclopropanesulfonamido)pyrimidin-4-yl)-N-(4-(6-ethoxypyrazin-2-yl)phenyl)acetamide;

2-(2-(cyclopropanesulfonamido)pyrimidin-4-yl)-N-(4-(6-methoxypyrazin-2-yl)phenyl)acetamide;

2-(2-(cyclopropanesulfonamido)pyrimidin-4-yl)-N-(4-(6-(2,2,2-trifluoroethoxy)pyrazin-2-yl)phenyl)acetamide;

2-(2-(cyclopropanesulfonamido)pyrimidin-4-yl)-N-(4-(6-isopropoxypyrazin-2-yl)phenyl)acetamide;

2-(2-(cyclobutanesulfonamido)pyrimidin-4-yl)-N-(5-(6-ethoxypyrazin-2-yl)pyridin-2-yl)-2-methylpropanamide;

2-(2-(cyclobutanesulfonamido)pyrimidin-4-yl)-N-(2-fluoro-4-(6-isopropoxypyrazin-2-yl)phenyl)-2-methylpropanamide;

2-(2-(cyclobutanesulfonamido)pyrimidin-4-yl)-N-(4-(6-ethoxypyrazin-2-yl)-2-methylphenyl)-2-methylpropanamide;

2-(2-(cyclobutanesulfonamido)pyrimidin-4-yl)-N-(4-(6-methoxypyrazin-2-yl)phenyl)-2-methylpropanamide;

2-(2-(cyclobutanesulfonamido)pyrimidin-4-yl)-N-(4-(6-ethoxypyrazin-2-yl)phenyl)-2-methylpropanamide;

2-(2-(cyclopropanesulfonamido)pyrimidin-4-yl)-N-(5-(6-ethoxypyrazin-2-yl)-3-fluoropyridin-2-yl)-2-methylpropanamide;

2-(2-(cyclopropanesulfonamido)pyrimidin-4-yl)-N-(5'-ethoxy-[3,3'-bipyridin]-6-yl)-2-methylpropanamide;

N-([3,3'-bipyridin]-6-yl)-2-(2-(cyclopropanesulfonamido)pyrimidin-4-yl)-2-methylpropanamide;

2-(2-(cyclopropanesulfonamido)pyrimidin-4-yl)-2-methyl-N-(5-(6-(trifluoromethyl)pyrazin-2-yl)pyridin-2-yl)propanamide;

2-(2-(cyclopropanesulfonamido)pyrimidin-4-yl)-N-(5-(6-ethoxypyrazin-2-yl)pyridin-2-yl)-2-methylpropanamide;

2-(2-(cyclopropanesulfonamido)pyrimidin-4-yl)-N-(5-(6-cyclopropoxypyrazin-2-yl)pyridin-2-yl)-2-methylpropanamide;

N-(2-chloro-4-(6-ethoxypyrazin-2-yl)phenyl)-2-(2-(cyclopropanesulfonamido)pyrimidin-4-yl)-2-methylpropanamide;

N-(2-cyano-4-(6-ethoxypyrazin-2-yl)phenyl)-2-(2-(cyclopropanesulfonamido)pyrimidin-4-yl)-2-methylpropanamide;

2-(2-(cyclopropanesulfonamido)pyrimidin-4-yl)-N-(2-fluoro-4-(5-isopropoxypyridin-3-yl)phenyl)-2-methylpropanamide;

2-(2-(cyclopropanesulfonamido)pyrimidin-4-yl)-N-(2-fluoro-4-(pyridin-3-yl)phenyl)-2-methylpropanamide;

2-(2-(cyclopropanesulfonamido)pyrimidin-4-yl)-N-(2-fluoro-4-(6-(trifluoromethyl)pyrazin-2-yl)phenyl)-2-methylpropanamide;

2-(2-(cyclopropanesulfonamido)pyrimidin-4-yl)-N-(4-(6-ethoxypyrazin-2-yl)-2-fluorophenyl)-2-methylpropanamide;

2-(2-(cyclopropanesulfonamido)pyrimidin-4-yl)-N-(2-fluoro-4-(6-isopropoxypyrazin-2-yl)phenyl)-2-methylpropanamide;

2-(2-(cyclopropanesulfonamido)pyrimidin-4-yl)-N-(4-(6-ethoxypyrazin-2-yl)-2-fluoro-5-methylphenyl)-2-methylpropanamide;

2-(2-(cyclopropanesulfonamido)pyrimidin-4-yl)-N-(4-(6-ethoxypyrazin-2-yl)-2,6-difluorophenyl)-2-methylpropanamide;

2-(2-(cyclopropanesulfonamido)pyrimidin-4-yl)-N-(2-fluoro-4-(pyrazin-2-yl)phenyl)-2-methylpropanamide;

2-(2-(cyclopropanesulfonamido)pyrimidin-4-yl)-2-methyl-N-(2-methyl-4-(6-(trifluoromethyl)pyrazin-2-yl)phenyl)propanamide;

2-(2-(cyclopropanesulfonamido)pyrimidin-4-yl)-N-(4-(6-ethoxypyrazin-2-yl)-2,3-dimethylphenyl)-2-methylpropanamide;

2-(2-(cyclopropanesulfonamido)pyrimidin-4-yl)-N-(4-(6-ethoxypyrazin-2-yl)-5-fluoro-2-methylphenyl)-2-methylpropanamide;

2-(2-(cyclopropanesulfonamido)pyrimidin-4-yl)-N-(4-(6-ethoxypyrazin-2-yl)-2,5-dimethylphenyl)-2-methylpropanamide;

2-(2-(cyclopropanesulfonamido)pyrimidin-4-yl)-N-(4-(6-ethoxypyrazin-2-yl)-2-(trifluoromethoxy)phenyl)-2-methylpropanamide;

2-(2-(cyclopropanesulfonamido)pyrimidin-4-yl)-N-(4-(6-ethoxypyrazin-2-yl)-5-fluoro-2-methoxyphenyl)-2-methylpropanamide;

2-(2-(cyclopropanesulfonamido)pyrimidin-4-yl)-N-(4-(6-ethoxypyrazin-2-yl)-2-methoxyphenyl)-2-methylpropanamide;

2-(2-(cyclopropanesulfonamido)pyrimidin-4-yl)-2-methyl-N-(4-(pyrimidin-5-yl)phenyl)propanamide;

N-(4-(5-chloropyridin-3-yl)phenyl)-2-(2-(cyclopropanesulfonamido)pyrimidin-4-yl)-2-methylpropanamide;

N-(4-(5-cyanopyridin-3-yl)phenyl)-2-(2-(cyclopropanesulfonamido)pyrimidin-4-yl)-2-methylpropanamide;

2-(2-(cyclopropanesulfonamido)pyrimidin-4-yl)-N-(4-(5-fluoropyridin-3-yl)phenyl)-2-methylpropanamide;

2-(2-(cyclopropanesulfonamido)pyrimidin-4-yl)-2-methyl-N-(4-(5-methylpyridin-3-yl)phenyl)propanamide;

2-(2-(cyclopropanesulfonamido)pyrimidin-4-yl)-N-(4-(5-(difluoromethoxy)pyridin-3-yl)phenyl)-2-methylpropanamide;

2-(2-(cyclopropanesulfonamido)pyrimidin-4-yl)-N-(4-(5-methoxypyridin-3-yl)phenyl)-2-methylpropanamide;

2-(2-(cyclopropanesulfonamido)pyrimidin-4-yl)-N-(4-(5-ethoxypyridin-3-yl)phenyl)-2-methylpropanamide;

2-(2-(cyclopropanesulfonamido)pyrimidin-4-yl)-N-(4-(5-isopropoxypyridin-3-yl)phenyl)-2-methylpropanamide;

2-(2-(cyclopropanesulfonamido)pyrimidin-4-yl)-2-methyl-N-(4-(pyridin-3-yl)phenyl)propanamide;

2-(2-(cyclopropanesulfonamido)pyrimidin-4-yl)-2-methyl-N-(3'-(trifluoromethyl)-[1,1'-biphenyl]-4-yl)propanamide;

N-(3'-chloro-[1,1'-biphenyl]-4-yl)-2-(2-(cyclopropanesulfonamido)pyrimidin-4-yl)-2-methylpropanamide;

N-(3'-cyano-[1,1'-biphenyl]-4-yl)-2-(2-(cyclopropanesulfonamido)pyrimidin-4-yl)-2-methylpropanamide;

2-(2-(cyclopropanesulfonamido)pyrimidin-4-yl)-N-(3'-ethoxy-[1,1'-biphenyl]-4-yl)-2-methylpropanamide;

2-(2-(cyclopropanesulfonamido)pyrimidin-4-yl)-2-methyl-N-(4-(6-(trifluoromethyl)pyrazin-2-yl)phenyl)propanamide;

2-(2-(cyclopropanesulfonamido)pyrimidin-4-yl)-N-(4-(6-ethoxypyrazin-2-yl)phenyl)-2-methylpropanamide;

2-(2-(cyclopropanesulfonamido)pyrimidin-4-yl)-N-(4-(6-cyclopropoxypyrazin-2-yl)phenyl)-2-methylpropanamide;

2-(2-(cyclopropanesulfonamido)pyrimidin-4-yl)-N-(4-(6-isopropoxypyrazin-2-yl)phenyl)-2-methylpropanamide;

2-(2-(cyclopropanesulfonamido)-5-fluoropyrimidin-4-yl)-N-(4-(6-ethoxypyrazin-2-yl)phenyl)-2-methylpropanamide;

N-(4-(6-ethoxypyrazin-2-yl)phenyl)-2-methyl-2-(2-((1-methylcyclopropane)-1-sulfonamido)pyrimidin-4-yl)propanamide;

2-(2-(cyclopropanesulfonamido)-5-methylpyrimidin-4-yl)-N-(4-(6-ethoxypyrazin-2-yl)phenyl)-2-methylpropanamide;

2-(2-(cyclopropanesulfonamido)pyrimidin-4-yl)-2-methyl-N-(4-(pyrazin-2-yl)phenyl)propanamide;

N-(4-(6-ethoxypyrazin-2-yl)-2-fluorophenyl)-2-(2-(ethylsulfonamido)pyrimidin-4-yl)-2-methylpropanamide;

2-(2-(ethylsulfonamido)pyrimidin-4-yl)-2-methyl-N-(4-(6-(trifluoromethyl)pyrazin-2-yl)phenyl)propanamide;

N-(4-(6-ethoxypyrazin-2-yl)phenyl)-2-(2-(ethylsulfonamido)pyrimidin-4-yl)-2-methylpropanamide;

N-(5-(6-ethoxypyrazin-2-yl)-3-fluoropyridin-2-yl)-2-methyl-2-(2-(methylsulfonamido)pyrimidin-4-yl)propanamide;

N-(5-(6-ethoxypyrazin-2-yl)pyridin-2-yl)-2-methyl-2-(2-(methylsulfonamido)pyrimidin-4-yl)propanamide;

N-(2-fluoro-4-(5-isopropoxypyridin-3-yl)phenyl)-2-methyl-2-(2-(methylsulfonamido)pyrimidin-4-yl)propanamide;

N-(2-fluoro-4-(6-isopropoxypyrazin-2-yl)phenyl)-2-methyl-2-(2-(methylsulfonamido)pyrimidin-4-yl)propanamide;

2-methyl-N-(2-methyl-4-(6-(trifluoromethyl)pyrazin-2-yl)phenyl)-2-(2-(methylsulfonamido)pyrimidin-4-yl)propanamide;

2-methyl-2-(2-(methylsulfonamido)pyrimidin-4-yl)-N-(4-(6-(trifluoromethyl)pyrazin-2-yl)phenyl)propanamide;

N-(4-(6-ethoxypyrazin-2-yl)phenyl)-2-methyl-2-(2-(methylsulfonamido)pyrimidin-4-yl)propanamide;

2-(2-((1,1-dimethylethyl)sulfonamido)pyrimidin-4-yl)-N-(4-(6-ethoxypyrazin-2-yl)phenyl)-2-methylpropanamide;

1-(2-(cyclopropanesulfonamido)pyrimidin-4-yl)-N-(4-(6-ethoxypyrazin-2-yl)phenyl)cyclopropanecarboxamide;

2-(2-(cyclopropanesulfonamido)pyrimidin-4-yl)-N-(5'-(trifluoromethyl)-[3,3'-bipyridin]-6-yl)butanamide;

2-(2-(cyclopropanesulfonamido)pyrimidin-4-yl)-N-(5'-(2,2,2-trifluoroethoxy)-[3,3'-bipyridin]-6-yl)butanamide;

N-([3,3'-bipyridin]-6-yl)-2-(2-(cyclopropanesulfonamido)pyrimidin-4-yl)butanamide;

2-(2-(cyclopropanesulfonamido)pyrimidin-4-yl)-N-(5-(6-(trifluoromethyl)pyrazin-2-yl)pyridin-2-yl)butanamide;

2-(2-(cyclopropanesulfonamido)pyrimidin-4-yl)-N-(5-(6-ethoxypyrazin-2-yl)pyridin-2-yl)butanamide;

2-(2-(cyclopropanesulfonamido)pyrimidin-4-yl)-N-(5-(6-isopropoxypyrazin-2-yl)pyridin-2-yl)butanamide;

N-(4-(5-chloropyridin-3-yl)-2-fluorophenyl)-2-(2-(cyclopropanesulfonamido)pyrimidin-4-yl)butanamide;

2-(2-(cyclopropanesulfonamido)pyrimidin-4-yl)-N-(2-fluoro-4-(5-(2,2,2-trifluoroethoxy)pyridin-3-yl)phenyl)butanamide;

2-(2-(cyclopropanesulfonamido)pyrimidin-4-yl)-N-(2-fluoro-4-(5-isopropoxypyridin-3-yl)phenyl)butanamide;

2-(2-(cyclopropanesulfonamido)pyrimidin-4-yl)-N-(2-fluoro-4-(pyridin-3-yl)phenyl)butanamide;

2-(2-(cyclopropanesulfonamido)pyrimidin-4-yl)-N-(2-fluoro-4-(6-(trifluoromethyl)pyrazin-2-yl)phenyl)butanamide;

2-(2-(cyclopropanesulfonamido)pyrimidin-4-yl)-N-(2-fluoro-4-(6-methoxypyrazin-2-yl)phenyl)butanamide;

2-(2-(cyclopropanesulfonamido)pyrimidin-4-yl)-N-(4-(6-ethoxypyrazin-2-yl)-2-fluorophenyl)butanamide;

2-(2-(cyclopropanesulfonamido)pyrimidin-4-yl)-N-(2-fluoro-4-(6-isopropoxypyrazin-2-yl)phenyl)butanamide;

2-(2-(cyclopropanesulfonamido)pyrimidin-4-yl)-N-(2-fluoro-4-(6-(2,2,2-trifluoroethoxy)pyrazin-2-yl)phenyl)butanamide;

N-(4-(5-cyanopyridin-3-yl)phenyl)-2-(2-(cyclopropanesulfonamido)pyrimidin-4-yl)butanamide;

2-(2-(cyclopropanesulfonamido)pyrimidin-4-yl)-N-(4-(5-(2,2,2-trifluoroethoxy)pyridin-3-yl)phenyl)butanamide;

2-(2-(cyclopropanesulfonamido)pyrimidin-4-yl)-N-(4-(5-isopropoxypyridin-3-yl)phenyl)butanamide;

2-(2-(cyclopropanesulfonamido)pyrimidin-4-yl)-N-(4-(pyridin-3-yl)phenyl)butanamide;

2-(2-(cyclopropanesulfonamido)pyrimidin-4-yl)-N-(4-(6-(trifluoromethyl)pyrazin-2-yl)phenyl)butanamide;

N-(4-(6-chloropyrazin-2-yl)phenyl)-2-(2-(cyclopropanesulfonamido)pyrimidin-4-yl)butanamide;

2-(2-(cyclopropanesulfonamido)pyrimidin-4-yl)-N-(4-(6-ethoxypyrazin-2-yl)phenyl)butanamide;

2-(2-(cyclopropanesulfonamido)pyrimidin-4-yl)-N-(4-(6-methoxypyrazin-2-yl)phenyl)butanamide;

2-(2-(cyclopropanesulfonamido)pyrimidin-4-yl)-N-(4-(6-isopropoxypyrazin-2-yl)phenyl)butanamide;

2-(2-(cyclopropanesulfonamido)pyrimidin-4-yl)-N-(4-(6-(2,2,2-trifluoroethoxy)pyrazin-2-yl)phenyl)butanamide;

2-(2-(cyclopropanesulfonamido)pyrimidin-4-yl)-N-(4-(pyrazin-2-yl)phenyl)butanamide;

2-(2-(cyclopropanesulfonamido)pyrimidin-4-yl)-N-(4-(6-ethoxypyrazin-2-yl)phenyl)-4-methoxybutanamide; and

2-(2-(cyclopropanesulfonamido)pyrimidin-4-yl)-N-(4-(pyridin-3-yl)phenyl)propanamide.

The present invention also provides the following compounds:

2-(2-(cyclopropanesulfonamido)pyrimidin-4-yl)-N-(5-(6-ethoxypyrazin-2-yl)pyridin-2-yl)-2-(R)-fluorobutanamide;

2-(2-(cyclopropanesulfonamido)pyrimidin-4-yl)-N-(5-(6-ethoxypyrazin-2-yl)pyridin-2-yl)-2-(S)-fluorobutanamide;

2-(2-(cyclopropanesulfonamido)pyrimidin-4-yl)-N-(5-(6-ethoxypyrazin-2-yl)pyridin-2-yl)-2-fluorobutanamide; and

4-(2-(cyclopropanesulfonamido)pyrimidin-4-yl)-N-(5-(6-ethoxypyrazin-2-yl)pyridin-2-yl)tetrahydro-2H-pyran-4-carboxamide.

The present invention also provides the following compounds:

2-(2-(cyclopropanesulfonamido)pyrimidin-4-yl)-N-(5-(6-isopropylpyrazin-2-yl)pyridin-2-yl)-2-methylpropanamide;

2-(2-(cyclopropanesulfonamido)pyrimidin-4-yl)-N-(4-(6-ethoxypyrazin-2-yl)-2-fluorophenyl)-2,2-difluoroacetamide;

N-((2-(cyclopropanesulfonamido)pyrimidin-4-yl)methyl)-4-(6-ethoxypyrazin-2-yl)benzamide;

2-(2-(cyclopropanesulfonamido)pyrimidin-4-yl)-2-methyl-N-(5-(6-(prop-1-en-2-yl)pyrazin-2-yl)pyridin-2-yl)propanamide;

2-(2-(cyclopropanesulfonamido)-6-methylpyrimidin-4-yl)-N-(5-(6-ethoxypyrazin-2-yl)pyridin-2-yl)-2-methylpropanamide;

2-(2-(cyclopropanesulfonamido)-6-(trifluoromethyl)pyrimidin-4-yl)-N-(5-(6-ethoxypyrazin-2-yl)pyridin-2-yl)-2-methylpropanamide;

2-(2-(cyclopropanesulfonamido)pyrimidin-4-yl)-N-(5-(6-cyclopropylpyrazin-2-yl)pyridin-2-yl)-2-methylpropanamide;

2-(2-(cyclopropanesulfonamido)pyrimidin-4-yl)-N-(6-(6-ethoxypyrazin-2-yl)pyridin-3-yl)-2-methylpropanamide;

2-(2-(cyclopropanesulfonamido)pyrimidin-4-yl)-N-(4-(6-cyclopropylpyrazin-2-yl)-2-fluorophenyl)-2-methylpropanamide;

2-(2-(cyclopropanesulfonamido)-6-methylpyrimidin-4-yl)-N-(4-(6-ethoxypyrazin-2-yl)-2-fluorophenyl)-2-methylpropanamide;

2-(2-(cyclopropanesulfonamido)-6-(trifluoromethyl)pyrimidin-4-yl)-N-(4-(6-ethoxypyrazin-2-yl)-2-fluorophenyl)-2-methylpropanamide;

2-(2-(cyclopropanesulfonamido)pyrimidin-4-yl)-2-methyl-N-(4-(6-(prop-1-en-2-yl)pyrazin-2-yl)phenyl)propanamide;

2-(2-(cyclopropanesulfonamido)pyrimidin-4-yl)-N-(4-(6-isopropylpyrazin-2-yl)phenyl)-2-methylpropanamide;

2-(2-(cyclopropanesulfonamido)pyrimidin-4-yl)-N-(4-(6-(dimethylamino)pyrazin-2-yl)phenyl)-2-methylpropanamide;

2-(2-(cyclopropanesulfonamido)-6-methylpyrimidin-4-yl)-N-(4-(6-ethoxypyrazin-2-yl)phenyl)-2-methylpropanamide;

2-(2-(cyclopropanesulfonamido)-6-(trifluoromethyl)pyrimidin-4-yl)-N-(4-(6-ethoxypyrazin-2-yl)phenyl)-2-methylpropanamide;

2-(2-(cyclopropanesulfonamido)-6-methoxypyrimidin-4-yl)-2-methyl-N-(4-(pyridin-3-yl)phenyl)propanamide;

1-(2-(cyclopropanesulfonamido)pyrimidin-4-yl)-N-(5-(6-ethoxypyrazin-2-yl)pyridin-2-yl)cyclopentane-1-carboxamide;

4-(2-(cyclopropanesulfonamido)pyrimidin-4-yl)-N-(4-(6-ethoxypyrazin-2-yl)phenyl)tetrahydro-2H-pyran-4-carboxamide;

N-(5-(6-ethoxypyrazin-2-yl)pyridin-2-yl)-4-(2-(methylsulfonamido)pyrimidin-4-yl)piperidine-4-carboxamide;

tert-butyl 4-(2-(cyclopropanesulfonamido)pyrimidin-4-yl)-4-((5-(6-ethoxypyrazin-2-yl)pyridin-2-yl)carbamoyl)piperidine-1-carboxylate;

4-(2-(cyclopropanesulfonamido)pyrimidin-4-yl)-N-(5-(6-ethoxypyrazin-2-yl)pyridin-2-yl)piperidine-4-carboxamide;

tert-butyl 3-(2-(cyclopropanesulfonamido)pyrimidin-4-yl)-3-((5-(6-ethoxypyrazin-2-yl)pyridin-2-yl)carbamoyl)azetidine-1-carboxylate;

tert-butyl 4-((5-(6-ethoxypyrazin-2-yl)pyridin-2-yl)carbamoyl)-4-(2-(methylsulfonamido) pyrimidin-4-yl)piperidine-1-carboxylate;

4-(2-(cyclopropanesulfonamido)pyrimidin-4-yl)-N-(4-(6-ethoxypyrazin-2-yl)-2-fluorophenyl)tetrahydro-2H-pyran-4-carboxamide;

2-(2-(cyclopropanesulfonamido)pyrimidin-4-yl)-N-(5-(6-ethoxypyrazin-2-yl)-3-fluoropyridin-2-yl)-4-methoxybutanamide;

2-(2-(cyclopropanesulfonamido)pyrimidin-4-yl)-N-(5-(6-ethoxypyrazin-2-yl)pyridin-2-yl)-4-methoxybutanamide;

2-(2-(cyclopropanesulfonamido)pyrimidin-4-yl)-N-(4-(6-ethoxypyrazin-2-yl)-2-fluorophenyl)-4-methoxybutanamide;

N-(5-(6-ethoxypyrazin-2-yl)pyridin-2-yl)-4-methoxy-2-methyl-2-(2-(methylsulfonamido) pyrimidin-4-yl)butanamide;

N-(5’-chloro-[3,3’-bipyridin]-6-yl)-2-(2-(cyclopropanesulfonamido)pyrimidin-4-yl)butanamide;

N-(5’-chloro-[3,3’-bipyridin]-6-yl)-2-(2-(cyclopropanesulfonamido)pyrimidin-4-yl)-2-fluorobutanamide;

2-(2-(cyclopropanesulfonamido)pyrimidin-4-yl)-N-(5-(6-cyclopropylpyrazin-2-yl)pyridin-2-yl)-2-fluorobutanamide;

N-(5-(6-ethoxypyrazin-2-yl)pyridin-2-yl)-2-fluoro-2-(2-(methylsulfonamido)pyrimidin-4-yl)butanamide;

2-(2-(cyclopropanesulfonamido)pyrimidin-4-yl)-N-(5-(6-ethoxypyrazin-2-yl)-3-methylpyridin-2-yl)butanamide;

2-(2-(cyclopropanesulfonamido)pyrimidin-4-yl)-N-(5-(6-cyclopropylpyrazin-2-yl)pyridin-2-yl)butanamide;

2-(2-(cyclopropanesulfonamido)pyrimidin-4-yl)-N-(5-(6-(2,2,2-trifluoroethoxy)pyrazin-2-yl)pyridin-2-yl)butanamide;

2-(2-(cyclopropanesulfonamido)pyrimidin-4-yl)-N-(3-fluoro-5-(6-methoxypyrazin-2-yl)pyridin-2-yl)butanamide;

2-(2-(cyclopropanesulfonamido)pyrimidin-4-yl)-N-(5-(6-methoxypyrazin-2-yl)pyridin-2-yl)butanamide;

2-(2-(cyclopropanesulfonamido)pyrimidin-4-yl)-N-(4-(6-cyclopropylpyrazin-2-yl)-2-fluorophenyl)butanamide;

2-(2-(cyclopropanesulfonamido)pyrimidin-4-yl)-N-(4-(6-ethoxypyrazin-2-yl)-2-methylphenyl)butanamide;

2-(2-(cyclopropanesulfonamido)pyrimidin-4-yl)-N-(5-(6-ethoxypyrazin-2-yl)-3-fluoropyridin-2-yl)butanamide;

2-(2-(cyclopropanesulfonamido)pyrimidin-4-yl)-N-(5-(6-ethoxypyrazin-2-yl)pyridin-2-yl)-2-methylbutanamide;

2-(2-(cyclopropanesulfonamido)pyrimidin-4-yl)-N-(5-(6-ethoxypyrazin-2-yl)pyridin-2-yl)-2-fluoro-3-methylbutanamide;

2-(2-(cyclopropanesulfonamido)pyrimidin-4-yl)-N-(4-(6-ethoxypyrazin-2-yl)-2-fluorophenyl)-3-methylbutanamide;

2-(2-(cyclopropanesulfonamido)pyrimidin-4-yl)-N-(5-(6-ethoxypyrazin-2-yl)pyridin-2-yl)-3-methylbutanamide;

2-(2-(cyclopropanesulfonamido)pyrimidin-4-yl)-N-(4-(6-ethoxypyrazin-2-yl)phenyl)-2-methoxyacetamide;

N-(5-(6-ethoxypyrazin-2-yl)pyridin-2-yl)-2-fluoro-2-(2-(methylsulfonamido)pyrimidin-4-yl)-(*R*)-butanamide;

N-(5-(6-ethoxypyrazin-2-yl)pyridin-2-yl)-2-fluoro-2-(2-(methylsulfonamido)pyrimidin-4-yl)-(*S*)-butanamide;

N-(4-(5-chloropyridin-3-yl)phenyl)-2-(6-(cyclopropanesulfonamido)pyridin-2-yl)acetamide;

N-(4-(5-cyanopyridin-3-yl)phenyl)-2-(6-(cyclopropanesulfonamido)pyridin-2-yl)acetamide;

2-(6-(cyclopropanesulfonamido)pyridin-2-yl)-N-(4-(5-fluoropyridin-3-yl)phenyl)acetamide;

2-(6-(cyclopropanesulfonamido)pyridin-2-yl)-N-(4-(5-methoxypyridin-3-yl)phenyl)acetamide;

2-(6-(cyclopropanesulfonamido)pyridin-2-yl)-N-(4-(pyridin-3-yl)phenyl)acetamide;

2-(6-(cyclopropanesulfonamido)pyridin-2-yl)-N-(4-(6-(trifluoromethyl)pyrazin-2-yl)phenyl)acetamide;

2-(6-(cyclopropanesulfonamido)pyridin-2-yl)-N-(4-(6-methoxypyrazin-2-yl)phenyl)acetamide;

2-(6-(cyclopropanesulfonamido)pyridin-2-yl)-N-(4-(pyrazin-2-yl)phenyl)acetamide;

N-([3,3'-bipyridin]-6-yl)-2-(6-(cyclopropanesulfonamido)pyridin-2-yl)-2-methylpropanamide;

N-(4-(5-chloropyridin-3-yl)phenyl)-2-(6-(cyclopropanesulfonamido)pyridin-2-yl)-2-methylpropanamide;

2-(6-(cyclopropanesulfonamido)pyridin-2-yl)-N-(4-(5-fluoropyridin-3-yl)phenyl)-2-methylpropanamide;

2-(6-(cyclopropanesulfonamido)pyridin-2-yl)-N-(4-(5-ethoxypyridin-3-yl)phenyl)-2-methylpropanamide;

2-(6-(cyclopropanesulfonamido)pyridin-2-yl)-2-methyl-N-(4-(pyridin-3-yl)phenyl)propanamide;

2-(6-(cyclopropanesulfonamido)pyridin-2-yl)-N-(2-fluoro-4-(pyrazin-2-yl)phenyl)-2-methylpropanamide;

2-(6-(cyclopropanesulfonamido)pyridin-2-yl)-2-methyl-N-(4-(6-(trifluoromethyl)pyrazin-2-yl)phenyl)propanamide;

N-(4-(6-chloropyrazin-2-yl)phenyl)-2-(6-(cyclopropanesulfonamido)pyridin-2-yl)-2-methylpropanamide;

2-(6-(cyclopropanesulfonamido)pyridin-2-yl)-N-(4-(6-ethoxypyrazin-2-yl)phenyl)-2-methylpropanamide;

2-(6-(cyclopropanesulfonamido)pyridin-2-yl)-N-(4-(6-methoxypyrazin-2-yl)phenyl)-2-methylpropanamide;

2-(6-(cyclopropanesulfonamido)pyridin-2-yl)-2-methyl-N-(4-(pyrazin-2-yl)phenyl)propanamide;

4-(6-(cyclopropanesulfonamido)pyridin-2-yl)-N-(5-(6-ethoxypyrazin-2-yl)pyridin-2-yl)tetrahydro-2H-pyran-4-carboxamide;

2-(6-(cyclopropanesulfonamido)pyridin-2-yl)-N-(5-(6-(trifluoromethyl)pyrazin-2-yl)pyridin-2-yl)butanamide;

2-(6-(cyclopropanesulfonamido)pyridin-2-yl)-N-(5-(6-ethoxypyrazin-2-yl)pyridin-2-yl)butanamide;

N-(4-(5-chloropyridin-3-yl)phenyl)-2-(6-(cyclopropanesulfonamido)pyridin-2-yl)butanamide;

2-(6-(cyclopropanesulfonamido)pyridin-2-yl)-N-(4-(6-ethoxypyrazin-2-yl)-2-fluorophenyl)butanamide;

2-(6-(cyclopropanesulfonamido)pyridin-2-yl)-N-(4-(6-ethoxypyrazin-2-yl)phenyl)butanamide;

2-(6-(cyclopropanesulfonamido)pyrazin-2-yl)-N-(4-(pyridin-3-yl)phenyl)acetamide;

2-(6-(ethylsulfonamido)pyrazin-2-yl)-N-(4-(pyridin-3-yl)phenyl)acetamide;

2-(6-(methylsulfonamido)pyrazin-2-yl)-N-(4-(pyridin-3-yl)phenyl)acetamide;

2-(6-(cyclopropanesulfonamido)pyrazin-2-yl)-N-(5-(6-ethoxypyrazin-2-yl)pyridin-2-yl)-2-methylpropanamide;

2-(6-(cyclopropanesulfonamido)pyrazin-2-yl)-N-(4-(6-ethoxypyrazin-2-yl)phenyl)-2-methylpropanamide;

4-(6-(cyclopropanesulfonamido)pyrazin-2-yl)-N-(5-(6-ethoxypyrazin-2-yl)pyridin-2-yl)tetrahydro-2H-pyran-4-carboxamide;

2-(6-(cyclopropanesulfonamido)pyrazin-2-yl)-N-(5-(6-ethoxypyrazin-2-yl)pyridin-2-yl)-4-methoxy-2-methylbutanamide;

N-(5-(6-ethoxypyrazin-2-yl)pyridin-2-yl)-4-methoxy-2-methyl-2-(6-(methylsulfonamido)pyrazin-2-yl)butanamide;

2-(6-(cyclopropanesulfonamido)pyrazin-2-yl)-N-(5-(6-ethoxypyrazin-2-yl)pyridin-2-yl)-2-fluorobutanamide;

2-(6-(cyclopropanesulfonamido)pyrazin-2-yl)-N-(5-(6-ethoxypyrazin-2-yl)pyridin-2-yl)butanamide;

2-(6-(cyclopropanesulfonamido)pyrazin-2-yl)-N-(4-(6-ethoxypyrazin-2-yl)phenyl)butanamide;

2-(6-(cyclopropanesulfonamido)pyrazin-2-yl)-N-(5-(6-ethoxypyrazin-2-yl)pyridin-2-yl)-2-methoxyacetamide;

2-(6-(cyclopropanesulfonamido)pyrazin-2-yl)-N-(4-(6-ethoxypyrazin-2-yl)phenyl)-2-methoxyacetamide;

2-(6-(cyclopropanesulfonamido)pyrazin-2-yl)-N-(5-(6-ethoxypyrazin-2-yl)pyridin-2-yl)-2-methoxypropanamide;

2-(6-(cyclopropanesulfonamido)pyrazin-2-yl)-N-(5-(6-ethoxypyrazin-2-yl)pyridin-2-yl)-2-(*R*)-fluorobutanamide;

2-(6-(cyclopropanesulfonamido)pyrazin-2-yl)-N-(5-(6-ethoxypyrazin-2-yl)pyridin-2-yl)-2-(S)-fluorobutanamide;

2-(4-(cyclopropanesulfonamido)pyrimidin-2-yl)-N-(5-(6-ethoxypyrazin-2-yl)pyridin-2-yl)butanamide;

N-(1-(2-(cyclopropanesulfonamido)pyrimidin-4-yl)cyclopropyl)-4-(6-ethoxypyrazin-2-yl)-2-fluorobenzamide;

N-(1-(2-(cyclopropanesulfonamido)pyrimidin-4-yl)propyl)-5-(6-ethoxypyrazin-2-yl)picolinamide;

N-(1-(2-(cyclopropanesulfonamido)pyrimidin-4-yl)propyl)-2-fluoro-4-(5-(trifluoromethyl)pyridin-3-yl)benzamide;

4-(5-chloropyridin-3-yl)-N-(1-(2-(cyclopropanesulfonamido)pyrimidin-4-yl)propyl)-2-fluorobenzamide;

N-(1-(2-(cyclopropanesulfonamido)pyrimidin-4-yl)propyl)-4-(5-(trifluoromethyl)pyridin-3-yl)benzamide;

4-(5-chloropyridin-3-yl)-N-(1-(2-(cyclopropanesulfonamido)pyrimidin-4-yl)propyl)benzamide;

N-(1-(2-(cyclopropanesulfonamido)pyrimidin-4-yl)propyl)-4-(6-ethoxypyrazin-2-yl)-2-(trifluoromethyl)benzamide;

N-(1-(2-(cyclopropanesulfonamido)pyrimidin-4-yl)propyl)-4-(6-ethoxypyrazin-2-yl)-2-fluorobenzamide;

N-(1-(2-(cyclopropanesulfonamido)pyrimidin-4-yl)propyl)-4-(6-(trifluoromethyl)pyrazin-2-yl)benzamide;

N-(1-(2-(cyclopropanesulfonamido)pyrimidin-4-yl)propyl)-4-(6-isopropoxypyrazin-2-yl)benzamide;

N-(1-(2-(cyclopropanesulfonamido)pyrimidin-4-yl)propyl)-4-(6-ethoxypyrazin-2-yl)benzamide;

N-(2-(2-(cyclopropanesulfonamido)pyrimidin-4-yl)butan-2-yl)-4-(6-ethoxypyrazin-2-yl)-2-fluorobenzamide;

N-(2-(6-(cyclopropanesulfonamido)pyrazin-2-yl)propan-2-yl)-2-fluoro-4-(6-isopropoxypyrazin-2-yl)benzamide;

N-(2-(6-(cyclopropanesulfonamido)pyrazin-2-yl)propan-2-yl)-4-(6-(trifluoromethyl)pyrazin-2-yl)benzamide;

N-(1-(6-(cyclopropanesulfonamido)pyrazin-2-yl)propyl)-4-(6-ethoxypyrazin-2-yl)-2-fluorobenzamide;

N-(1-(6-(cyclopropanesulfonamido)pyrazin-2-yl)propyl)-4-(6-ethoxypyrazin-2-yl)-2-(*R*)-fluorobenzamide; and

N-(1-(6-(cyclopropanesulfonamido)pyrazin-2-yl)propyl)-4-(6-ethoxypyrazin-2-yl)-2-(*S*)-fluorobenzamide.

The compounds of the invention may be provided in the form of a pharmaceutically acceptable salt and/or solvate thereof. In particular, the compound of formula (I) may be provided in the form of a pharmaceutically acceptable salt and/or solvate, such as a pharmaceutically acceptable salt.

Compounds of the invention of particular interest are those demonstrating an $IC_{50}$ of 1 uM or lower, especially 100nM or lower, in respect of CTPS1 enzyme, using the methods of the examples (or comparable methods).

Compounds of the invention of particular interest are those demonstrating a selectivity for CTPS1 over CTPS2 of 2-30 fold, suitably >30-60 fold or more suitably >60 fold, using the methods of the examples (or comparable methods). Desirably the selectivity is for human CTPS1 over human CTPS2.

It will be appreciated that for use in medicine the salts of the compounds of formula (I) should be pharmaceutically acceptable. Non-pharmaceutically acceptable salts of the compounds of formula (I) may be of use in other contexts such as during preparation of the compounds of formula (I). Suitable pharmaceutically acceptable salts will be apparent to those skilled in the art. Pharmaceutically acceptable salts include those described by Berge et al. (1977). Such pharmaceutically acceptable salts include acid and base addition salts. Pharmaceutically acceptable acid additional salts may be formed with inorganic acids e.g. hydrochloric, hydrobromic, sulphuric, nitric or phosphoric acid and organic acids e.g. succinic, maleic, acetic, fumaric, citric, tartaric, benzoic, p-toluenesulfonic, methanesulfonic or naphthalenesulfonic acid. Other salts e.g. oxalates or formates, may be used, for example in the isolation of compounds of formula (I) and are included within the scope of this invention.

Certain of the compounds of formula (I) may form acid or base addition salts with one or more equivalents of the acid or base. The present invention includes within its scope all possible stoichiometric and non-stoichiometric forms.

The compounds of formula (I) may be prepared in crystalline or non-crystalline form and, if crystalline, may optionally be solvated, e.g. as the hydrate. This invention includes within its scope stoichiometric solvates (e.g. hydrates) as well as compounds containing variable amounts of solvent (e.g. water).

It is to be understood that the present invention encompasses the following isomers of formula (I), namely all geometric, tautomeric and optical forms, and mixtures thereof (e.g. racemic mixtures). Where additional chiral centres are present in compounds of formula (I), the present invention includes within its scope all possible diastereoisomers, including mixtures thereof. The different isomeric forms may be separated or resolved one from the other by conventional methods, or any given isomer may be obtained by conventional synthetic methods or by stereospecific or asymmetric syntheses.

The present disclosure includes all isotopic forms of the compounds of the invention provided herein, whether in a form (i) wherein all atoms of a given atomic number have a mass number (or mixture of mass numbers) which predominates in nature (referred to herein as the "natural isotopic form") or (ii) wherein one or more atoms are replaced by atoms having the same atomic number, but a mass number different from the mass number of atoms which predominates in nature (referred to herein as an "unnatural variant isotopic form"). It is understood that an atom may naturally exist as a mixture of mass numbers. The term "unnatural variant isotopic form" also includes embodiments in which the proportion of an atom of given atomic number having a mass number found less commonly in nature (referred to herein as an "uncommon isotope") has been increased relative to that which is naturally occurring e.g. to the level of >20%,

>50%, >75%, >90%, >95% or >99% by number of the atoms of that atomic number (the latter embodiment referred to as an "isotopically enriched variant form"). The term "unnatural variant isotopic form" also includes embodiments in which the proportion of an uncommon isotope has been reduced relative to that which is naturally occurring. Isotopic forms may include radioactive forms (i.e. they incorporate radioisotopes) and non-radioactive forms. Radioactive forms will typically be isotopically enriched variant forms.

An unnatural variant isotopic form of a compound may thus contain one or more artificial or uncommon isotopes such as deuterium ($^{2}H$ or D), carbon-11 ($^{11}C$), carbon-13 ($^{13}C$), carbon-14 ($^{14}C$), nitrogen-13 ($^{13}N$), nitrogen-15 ($^{15}N$), oxygen-15 ($^{15}O$), oxygen-17 ($^{17}O$), oxygen-18 ($^{18}O$), phosphorus-32 ($^{32}P$), sulphur-35 ($^{35}S$), chlorine-36 ($^{36}Cl$), chlorine-37 ($^{31}Cl$), fluorine-18 ($^{18}F$) iodine-123 ($^{123}I$), iodine-125 ($^{125}I$) in one or more atoms or may contain an increased proportion of said isotopes as compared with the proportion that predominates in nature in one or more atoms.

Unnatural variant isotopic forms comprising radioisotopes may, for example, be used for drug and/or substrate tissue distribution studies. The radioactive isotopes tritium, i.e. $^{3}H$, and carbon-14, i.e. $^{14}C$, are particularly useful for this purpose in view of their ease of incorporation and ready means of detection. Unnatural variant isotopic forms which incorporate deuterium i.e. $^{2}H$ or D may afford certain therapeutic advantages resulting from greater metabolic stability, for example, increased *in vivo* half-life or reduced dosage requirements, and hence may be preferred in some circumstances. Further, unnatural variant isotopic forms may be prepared which incorporate positron emitting isotopes, such as $^{11}C$, $^{18}F$, $^{15}O$ and $^{13}N$, and would be useful in Positron Emission Topography (PET) studies for examining substrate receptor occupancy.

In one embodiment, the compounds of the invention are provided in a natural isotopic form.

In one embodiment, the compounds of the invention are provided in an unnatural variant isotopic form. In a specific embodiment, the unnatural variant isotopic form is a form in which deuterium (i.e. $^{2}H$ or D) is incorporated where hydrogen is specified in the chemical structure in one or more atoms of a compound of the invention. In one embodiment, the atoms of the compounds of the invention are in an isotopic form which is not radioactive. In one embodiment, one or more atoms of the compounds of the invention are in an isotopic form which is radioactive. Suitably radioactive isotopes are stable isotopes. Suitably the unnatural variant isotopic form is a pharmaceutically acceptable form.

In one embodiment, a compound of the invention is provided whereby a single atom of the compound exists in an unnatural variant isotopic form. In another embodiment, a compound of the invention is provided whereby two or more atoms exist in an unnatural variant isotopic form.

Unnatural isotopic variant forms can generally be prepared by conventional techniques known to those skilled in the art or by processes described herein e.g. processes analogous to those described in the accompanying Examples for preparing natural isotopic forms. Thus, unnatural isotopic variant forms could be prepared by using appropriate isotopically variant (or labelled) reagents in place of the normal reagents employed in the Examples. Since the compounds of formula (I) are intended for use in pharmaceutical compositions it will readily be understood that they are each preferably provided in substantially pure form, for example at least 60% pure, more suitably at least 75% pure and preferably at least 85%, especially at least 98% pure (% are on a weight for weight basis). Impure preparations of the compounds may be used for preparing the more pure forms used in the pharmaceutical compositions.

In general, the compounds of formula (I) may be made according to the organic synthesis techniques known to those skilled in this field, as well as by the representative methods set forth below, those in the Examples, and modifications thereof.

**General Routes:**

Generic routes by which compound examples of the invention may be conveniently prepared are summarised below.

## Scheme 1a

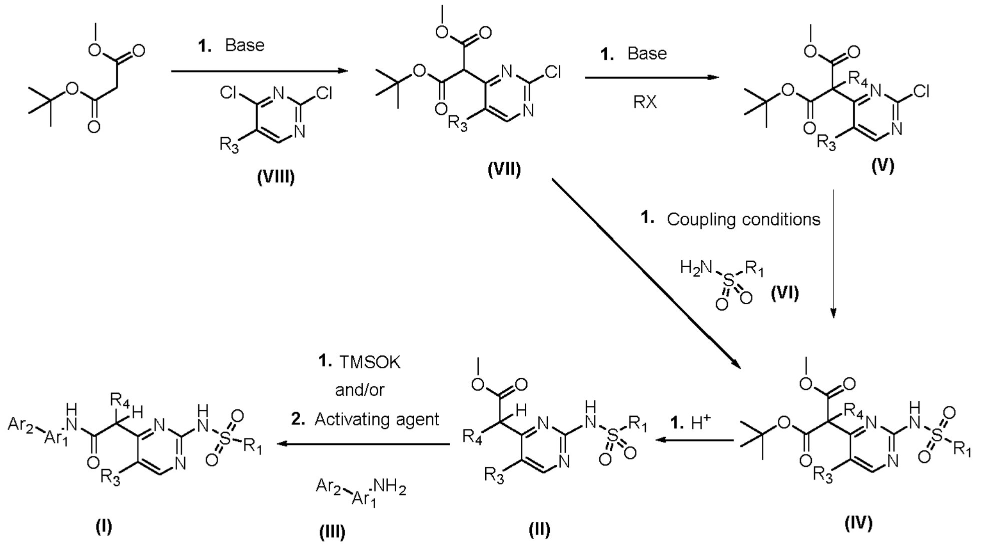


## Scheme 1b

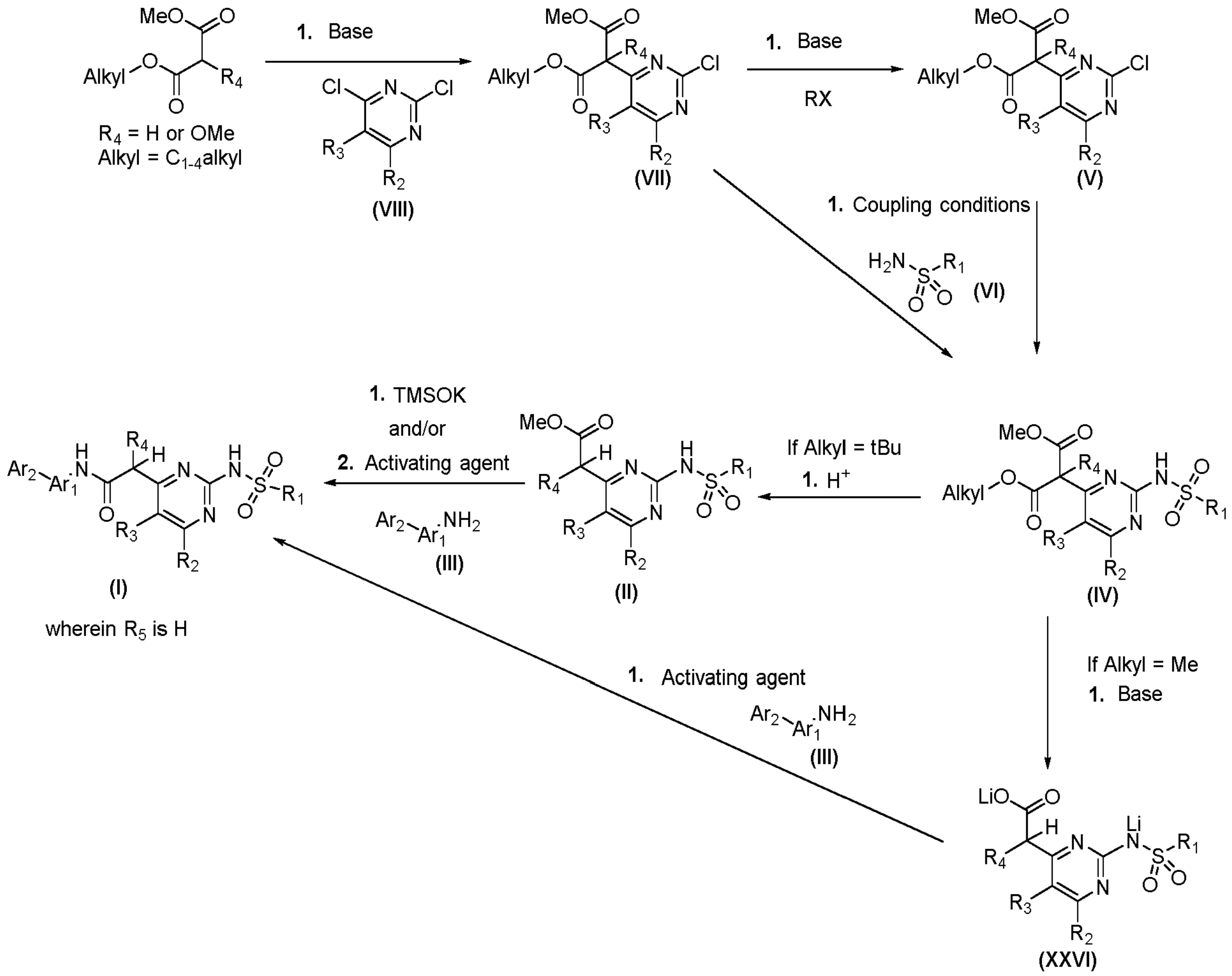


In general and as illustrated in Scheme 1a (wherein $R_4$ is H or Et) where $R_1$, $R_3$, Ar1 and Ar2 are defined above, or Scheme 1b (wherein $R_4$ is H or OMe) where $R_1$, $R_2$, $R_3$, Ar1 and Ar2 are defined above, the compounds of formula **(I)** may be prepared in four or five steps starting from a 2,4-dichloropyrimidine derivative of general formula **(VIII).** The derivative **(VIII)** can be reacted with an unsymmetrical malonate ester derivative to displace the more reactive chloride

and form intermediate compounds of formula **(VII).** Such reactions may be carried out in the presence of a strong base such as sodium hydride and in a polar solvent such as DMF. If mono alkylation is desired then treatment of intermediate **(VII)** with an inorganic base, such as sodium hydroxide, in the presence of an alkylating agent, such as iodoethane (EtI), yields compounds of the general formula **(V).** If a desmethyl ($R_4$ = H) linker is desired, compounds of general formula **(VII)** can be taken directly to compounds of general formula **(IV)** (see below).

Palladium catalysed sulfamination of 2-chloropyrimidine derivative **(VII)** and **(V)** can be undertaken using a catalyst such as [*t*-BuXPhos Pd(allyl)]OTf and substituted sulfonamide nucleophile **(VI),** in the presence of an inorganic base, for example potassium carbonate to form intermediate derivative **(IV).** This compound can then be deprotected *via* a decarboxylation, initiated by the use of a strong acid such as TFA to yield intermediate derivative **(II).** Such reactions are carried out in DCM at temperatures of 0 °C to room temperature.

Compounds of general formula **(I)** can be prepared by conversion of intermediate **(II)** by a one or two step process. Firstly, saponification using an agent such as TMSOK gives the intermediate carboxylic acid derivative followed by reaction with an activating agent, to generate a reactive, electrophilic carboxylic acid derivative, followed by subsequent reaction with an amine of formula **(III),** or a suitably protected derivative thereof. 2,4,6-Tripropyl-1,3,5,2,4,6-trioxatriphosphorinane-2,4,6-trioxide (T3P) is a reagent suitable for the activation of the carboxylate group. An alternative approach involves activation of the ester moiety directly using trimethylaluminium (usually a 2.0M solution in toluene or heptane) and addition of amine **(III).** These reactions are typically heated to 80 - 100 °C for a few hours in a solvent such as toluene.

If an alkoxy ($R_4$ = OMe) linker is required, compounds may be prepared in four steps starting from a 2,4-dichloropyrimidine derivative of general formula **(VIII)** (Scheme 1b). The derivative **(VIII)** can be reacted with a symmetrical malonate ester to form intermediate compounds of formula **(VII)** where $R_4$ = OMe. Compounds such as **(VII)** can then be coupled with a primary sulfonamide under conditions previously described. Compounds of formula **(IV)** where both alkyl groups are methyl can then be deprotected *via* a decarboxylation, initiated by the use of an alkali metal base to yield intermediate derivative **(XXVI).** The intermediate carboxylate derivative **(XXVI)** can undergo amide coupling as previously described to give final compounds of formula **(I).**

## Scheme 2a

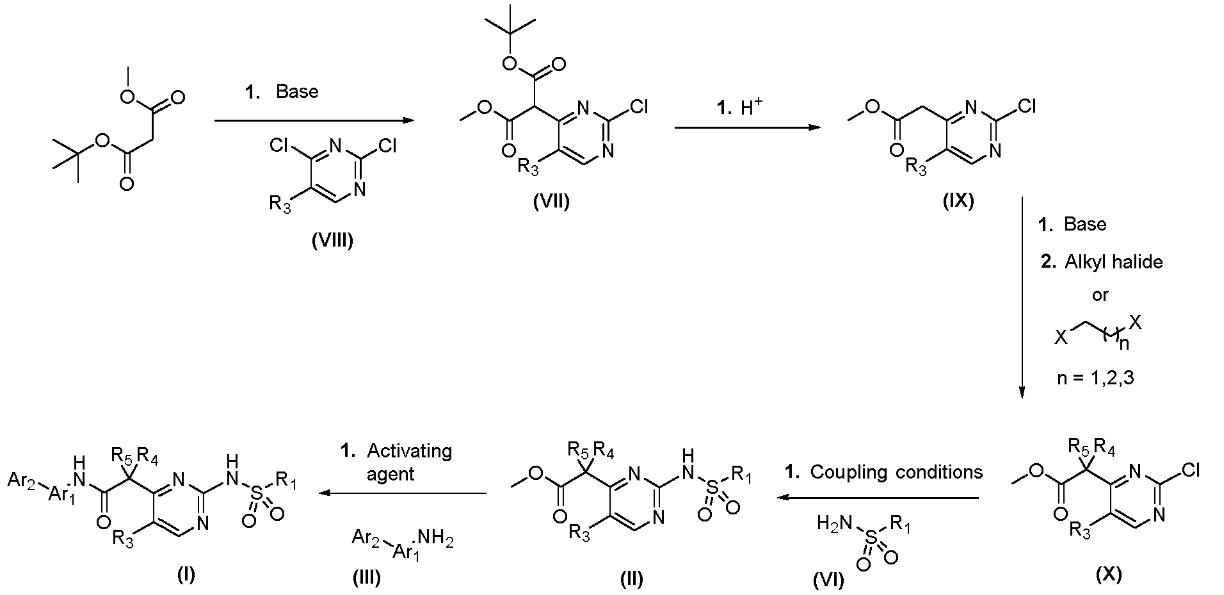

## Scheme 2b

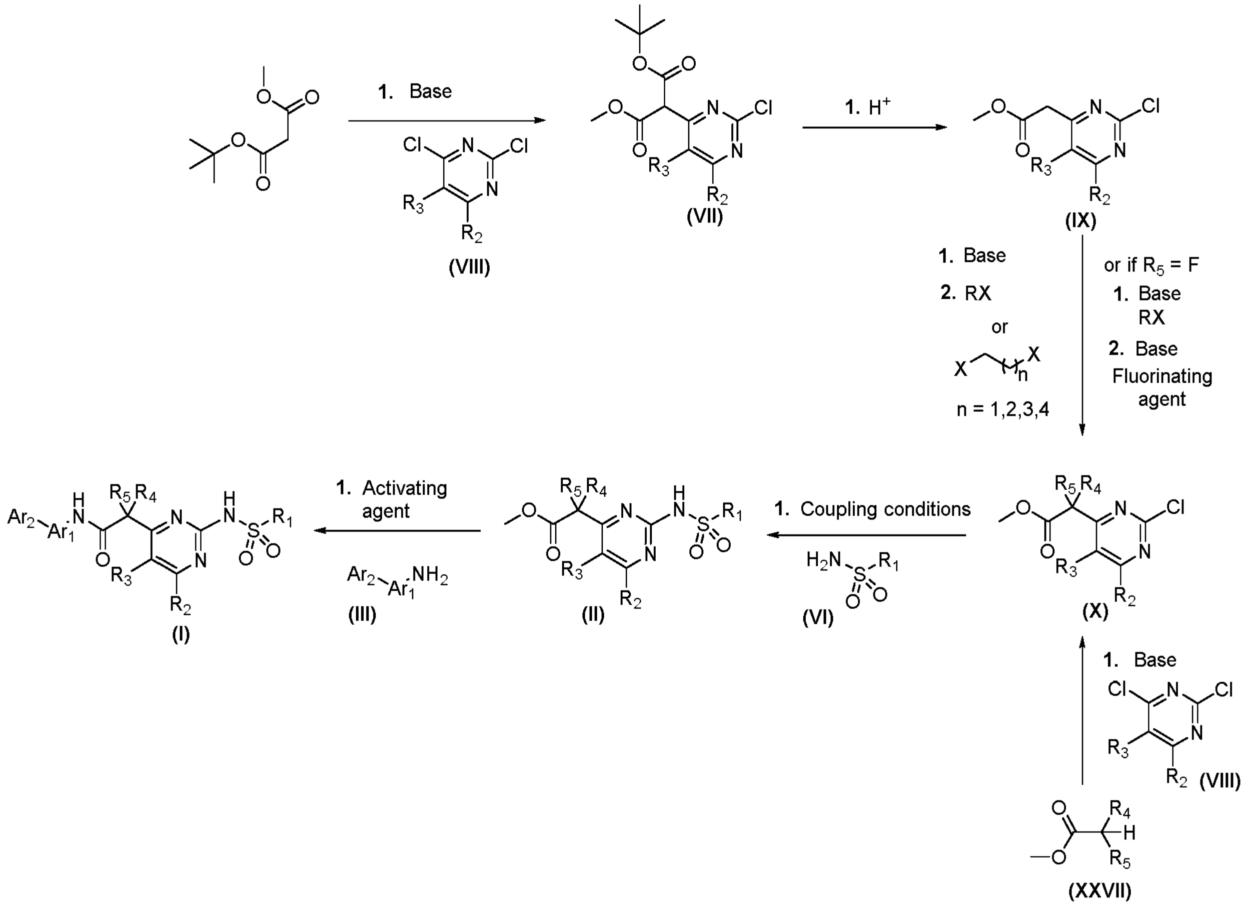


Suitably, $R_2$ is H, **(IX)** is converted to **(X)** using a base and alkyl halide or X-$CH_2$-$(CH_2)$n-X wherein n = 1,2,3 and the compounds of general formula **(I)** are obtained by a five step process.

In general and as illustrated in Schemes 2a and 2b, compounds of general formula **(I)** may be obtained by a five or six step process from a 2,4-dichloropyrimidine derivative of general formula **(VIII).** Firstly, the derivative **(VIII)** can be reacted with an unsymmetrical malonate ester as shown in Schemes 1a, 1b, 2a or 2b. For example, the unsymmetrical malonate ester can be treated with a base such as $Cs_2CO_3$ in the presence of di-chloropyrimidine **(VIII)** in a solvent such as DMF and heated to an elevated temperature such as 80 °C, followed by an aqueous work-up to obtain compounds of formula **(VII).** This intermediate compound can then be deprotected at this stage *via* a decarboxylation, initiated by the use of a strong acid such as TFA to yield intermediate derivative **(IX).** Certain intermediates such as **(IX)** where $R_3$ = H, are commercially available. Reaction of a methyl 2-(2-chloropyrimidin-4-yl)acetate derivative of general formula **(IX)** with an inorganic base such as potassium carbonate, in the presence of an alkylating agent leads to alkylation alpha to the ester. It will be understood by persons skilled in the art that both mono- and dialkylation may be achieved with careful control of the reaction conditions, but for a more reliable synthesis of the monoalkylated product, an alternative procedure should be considered (as in Scheme 1a). $R_4$ and $R_5$ can be connected to form a $C_{3-6}$cycloalkyl ring as defined above **((IX)** to **(X)).** Such compounds may be prepared by double alkylation with a dihaloalkane, such as 1,2-dibromoethane or 1,3-dibromobutane in the presence of an inorganic base such as sodium hydroxide. For compounds of general formula **(I)** wherein $R_4$ and $R_5$ together with the carbon to which they are attached form a $C_{3-6}$heterocycloalkyl, double alkylation of intermediates **(IX)** using a di-haloheteroalkane (such as $BrCH_2CH_2OCH_2CH_2Br$) in the presence of a base such as $Cs_2CO_3$ in a solvent such as MeCN at an elevated temperature such as 60 °C followed by direct column chromatography can be used to provide compounds of formula **(X).**

Palladium catalysed sulfamination of intermediate **(X)** may be achieved using a catalyst such as [*t*-BuXPhosPd(allyl)]OTf or *t*-BuXPhos-Pd-G3 and substituted sulfonamide nucleophile **(VI),** in the presence of an inorganic base, for example potassium carbonate to form intermediate derivative **(II).** Alternatively, sulfamination of intermediate **(X)** may be achieved using a substituted sulfonamide nucleophile **(VI),** in the presence of an inorganic base, for example $Cs_2CO_3$ and a solvent such as N-methyl pyrrolidinone to form intermediates **(II)** which may be obtained by precipitation following dilution in aqueous 4M HCl.

Final transformation to compounds of general formula **(I)** can be prepared by conversion of intermediate **(II)** by

activation of the ester moiety using trimethylaluminium (usually a 2.0 M solution in toluene or heptane) and addition of amine **(III)** (commercially available or prepared as in Schemes 6a, 6b, 7a or 7b). Alternatively, compounds of formula **(I)** may be obtained by a strong base-mediated amide formation between compounds **(II)** and **(III)** at room temperature using bases such as iPrMgCl, LiHMDS or KOtBu.

Compounds of the general formula **(VII)** where $R_2$ is O-alkyl may be accessed in two steps from commercial 2,4,6-trichloropyrimidine derivatives such as **(VIII)** where $R_2$ is Cl. Reaction of an unsymmetrical malonate ester can yield compounds such as **(VII)** which can then be treated with an alkoxide base such as sodium methoxide to displace the more reactive chloride to give compounds of general formula **(VII)** where $R_2$ = O-alkyl. Such compounds can then be progressed to final compounds of formula **(I)** following the steps previously described in Schemes 2a or 2b.

Compounds of general formula **(I)** where $R_1$, Ar1 and Ar2 are defined above and $R_4$ and $R_5$ together with the carbon to which they are attached form a $C_{3-6}$heterocycloalkyl, may be prepared in five steps starting from intermediate of general formula **(VIII).** Firstly, alkyl esters of general formula **(XXVII)** can be treated with a strong base such as LHMDS then reacted with 2,4-dichloropyrimidines such as derivative **(VIII).** Such compounds can then be converted to final compounds using the methods described in Scheme 2b. If any protecting groups remain after amide coupling, treatment with a strong acid such as TFA may yield final compounds of formula **(I).**

For compounds where $R_5$ is halo such as F and $R_4$ is $C_{1-6}$alkyl, a two-step procedure may be carried out to convert intermediates of formula **(IX)** to **(X),** see Scheme 2b. Firstly mono alkylation alpha to the ester may be achieved by treatment with an inorganic base such as potassium carbonate, in the presence of an alkylating agent. Reaction of these products with a strong base such as LHMDS followed by exposure to a fluorinating agent such as *N*-fluoro-*N*-(phenylsulfonyl)benzenesulfonamide may produce compounds of formula (X).

## Scheme 3

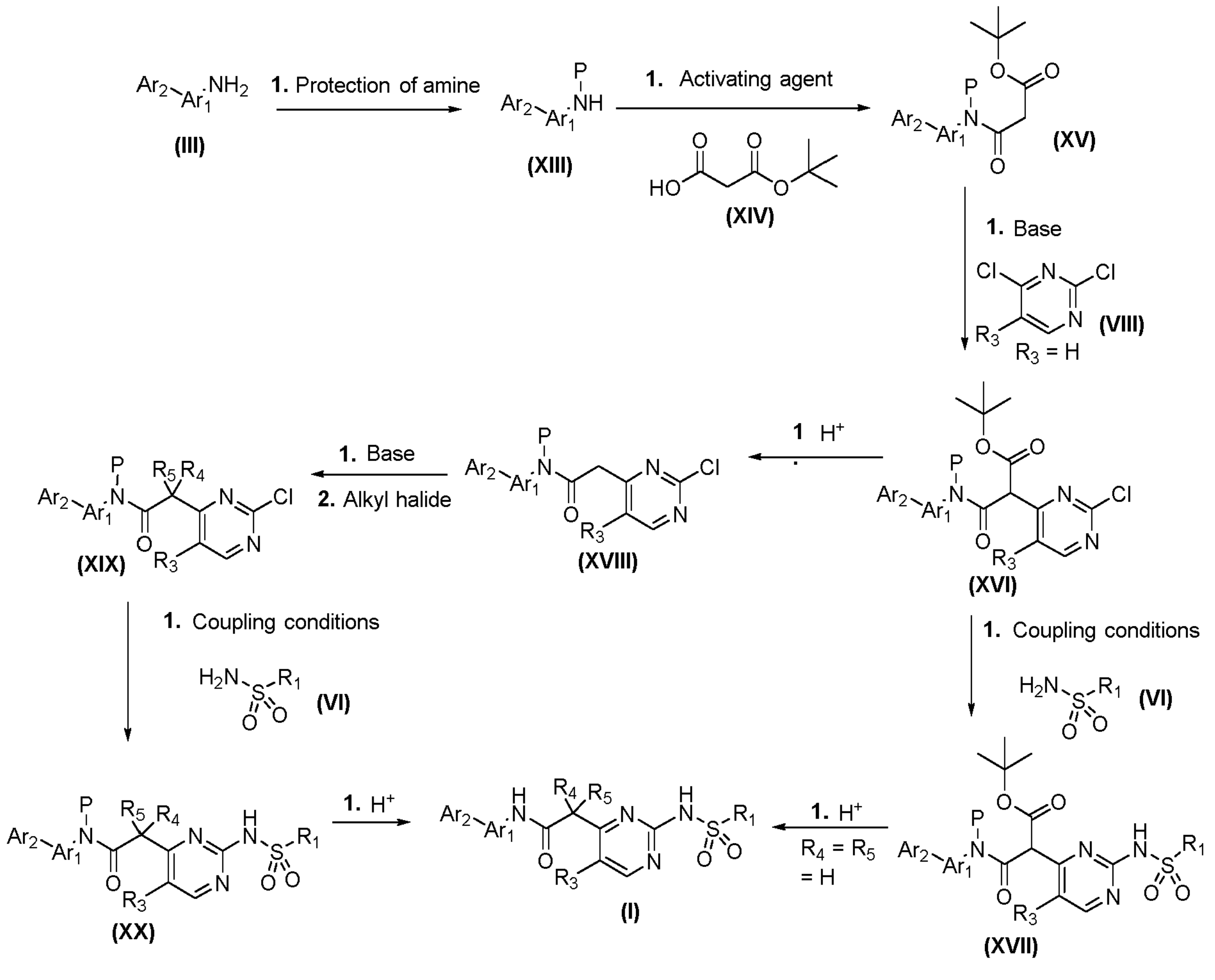

(III) (XIII) (XIV) (XV) (VIII) (XVI) (XVIII) (XIX) (VI) (XX) (I) (XVII)

In general and as illustrated in Scheme 3, compounds of general formula (I) wherein $R_3$ is H may be obtained by a seven step process when $R_4$ and/or $R_5$ = alkyl (or five step process when $R_4$ = $R_5$ = H) from anilines of formula **(III)** defined in Scheme 4 and 5. Firstly, aniline **(III)** can be protected with a suitable nitrogen protecting group such as a para-methoxybenzyl ether group by reacting aniline **(III)** with 4-methoxybenzaldehyde followed by reduction in situ with reducing agents such as sodium triacetoxyborohydride. Protected aniline of formula **(XIII)** can then be reacted with 3-(tert-butoxy)-3-oxopropanoic acid **(XIV)** in presence of a coupling reagent such as HATU to obtain intermediates **(XV).** Such intermediates **(XV)** may undergo $S_NAr$ with 2,4-dichloropyrimidine **(VIII)** ($R_3$ = H) in the presence of a strong base such as NaH to give pyrimidines of formula **(XVI).** The intermediate **(XVI)** may then undergo two transformations.

Firstly, decarboxylation with a strong acid such as TFA to obtain intermediates of formula **(XVIII)** followed by alkylation in the presence of a base such as $K_2CO_3$ results in the formation compounds of formula **(XIX).** Palladium catalysed sulfonamidation of intermediate **(XIX)** may be achieved using a catalytic system such as Pd-174 in the presence of a sulphonamide of the type **(VI)** to obtain compounds of the formula **(XX).** Compounds of formula **(I)** may be obtained by deprotection of the aniline nitrogen using a strong acidic system such as TFA/triflic acid.

Alternatively compounds of formula **(XVI)** may undergo sulfonamidation using sulphonamide of the type **(VI)** followed by double deprotection using a strong acidic system such as TFA/triflic acid to yield compounds of formula **(I).**

## Scheme 4a

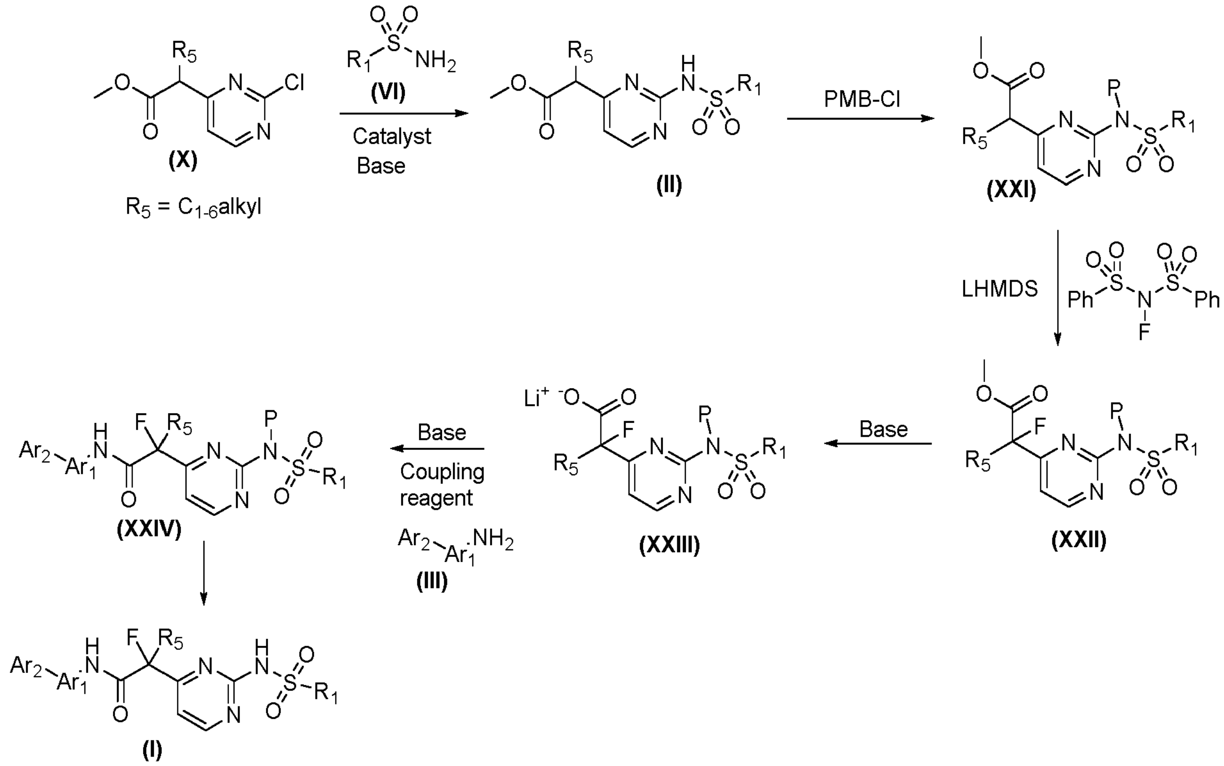

## Scheme 4b

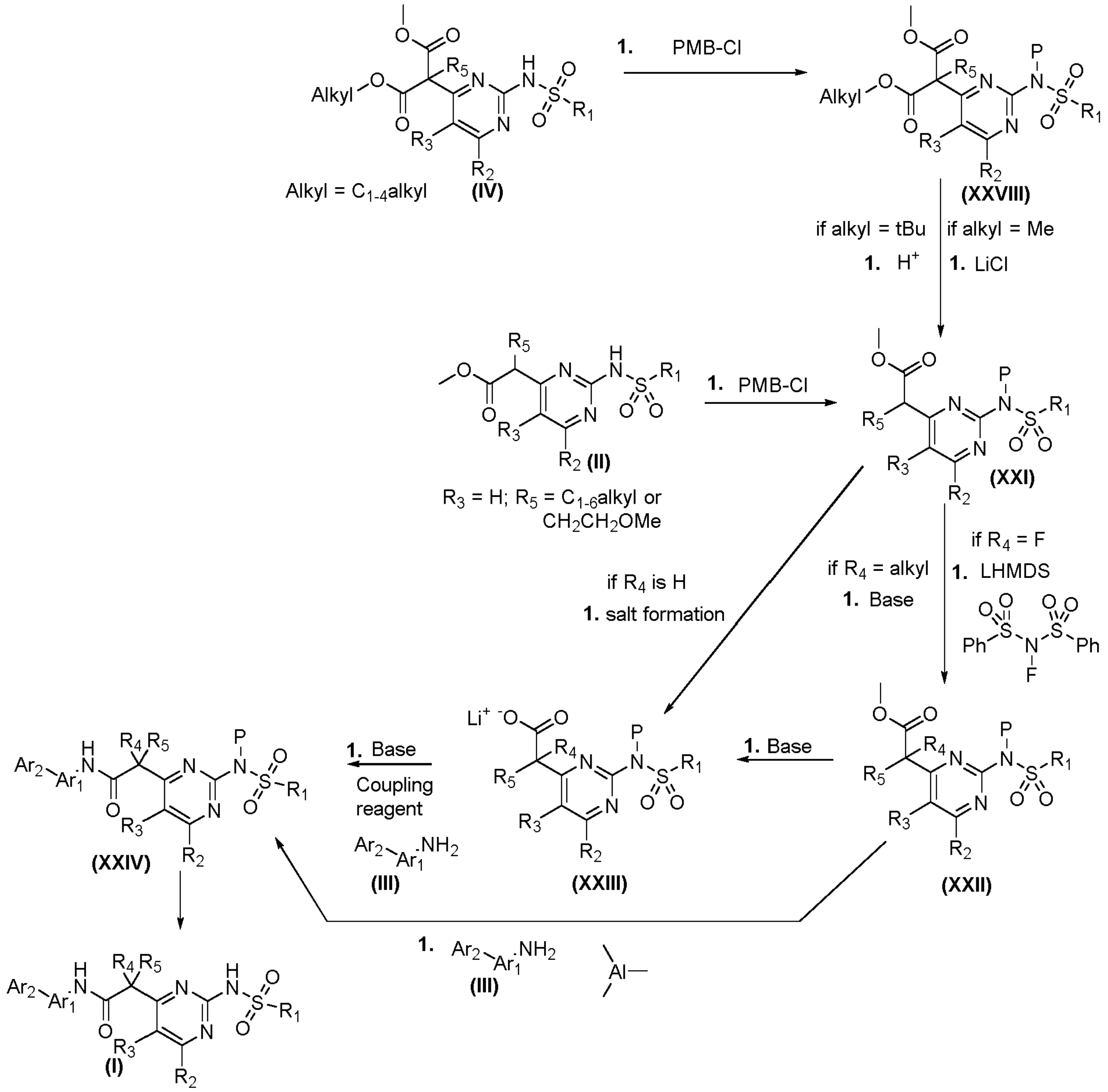


Suitably, $R_2$ is H, $R_3$ is H, $R_4$ is F and $R_5$ is $C_{1-6}$alkyl.

In general and as illustrated in Scheme 4a, compounds of general formula **(I)** where $R_1$, Ar1 and Ar2 are defined above, P is a nitrogen protecting group such as PMB, $R_4$ is halo such as F and $R_5$ = $C_{1-6}$alkyl may be prepared starting from the methyl ester **(II)** which may undergo protection such as with PMB-Cl to give intermediate **(XXI)** which can then undergo fluorination using a fluorinating agent such as *N*-fluoro-*N*-(phenylsulfonyl)benzenesulfonamide after being treated with an appropriate base such as LHMDS. Intermediate **(XXII)** can undergo salt formation using an inorganic base such as LiOH to yield intermediate **(XXIII)** which can then be activated with a coupling reagent such as T3P in presence of base and coupled with an aniline such as **(III)** to obtain the protected final compound **(XXIV).** To follow is the final deprotection step under strongly acidic conditions such as TFA in DCM to give the desired final compounds of general formula **(I).**

As shown in Scheme 4b, intermediates of formula **(XXI)** may also be prepared starting from pyrimidine **(IV)** which can undergo protection such as with PMB-Cl to give intermediate **(XXVIII).** Decarboxylation when the alkyl ester is tBu can be carried out with a strong acid such as TFA to yield derivatives of formula **(XXI).** Alternatively if the alkyl group is methyl, decarboxylation can be performed under Krapcho conditions employing a chloride ion source such as LiCl, in a polar aprotic solvent such as DMSO at elevated temperatures such as 140 °C to give derivatives of general formula **(XXI).**

For compounds where $R_4$ is $C_{1-6}$alkyl but where $R_4 \neq R_5$, derivatives of general formula **(XXI)** may be reacted

with an inorganic base such as potassium carbonate, in the presence of an alkylating agent to give compounds of formula **(XXII).** Such compounds can be converted to final compounds using methods previously described in Scheme 4a.

For compounds where $R_4$ = H is desired, compounds of formula **(XXI)** may be converted directly to carboxylate salts such as **(XXIII)** by treatment with a suitable agent such as TMSOK as previously described. Intermediates **(XXIII)** may be converted to compounds of formula **(I)** as described above, or in two steps by direct coupling of **(XXII)** with amines of formula **(III)** in the presence of an activating agent such as $AlMe_3$ followed by conversion of **(XXIV)** to compounds of formula **(I)** as described above.

## Scheme 5a

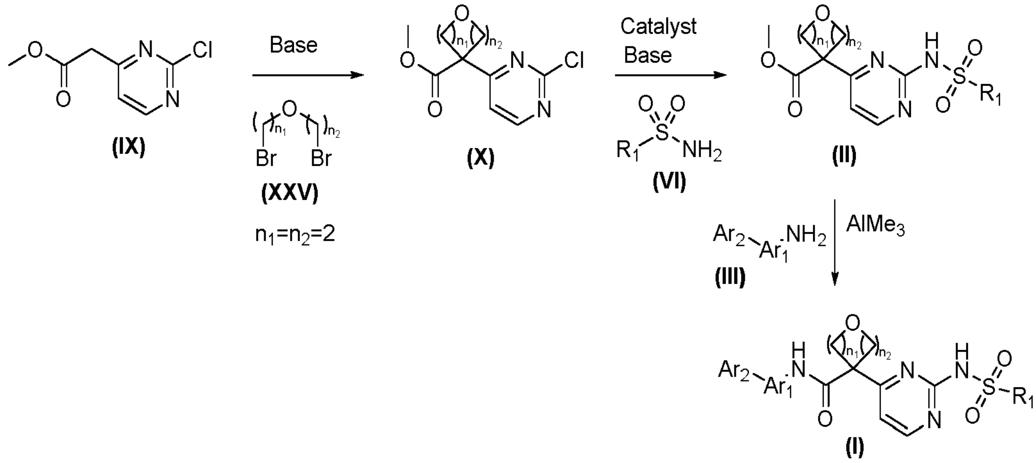


(IX) (XXV) (X) (VI) (II) (III) (I)

## Scheme 5b

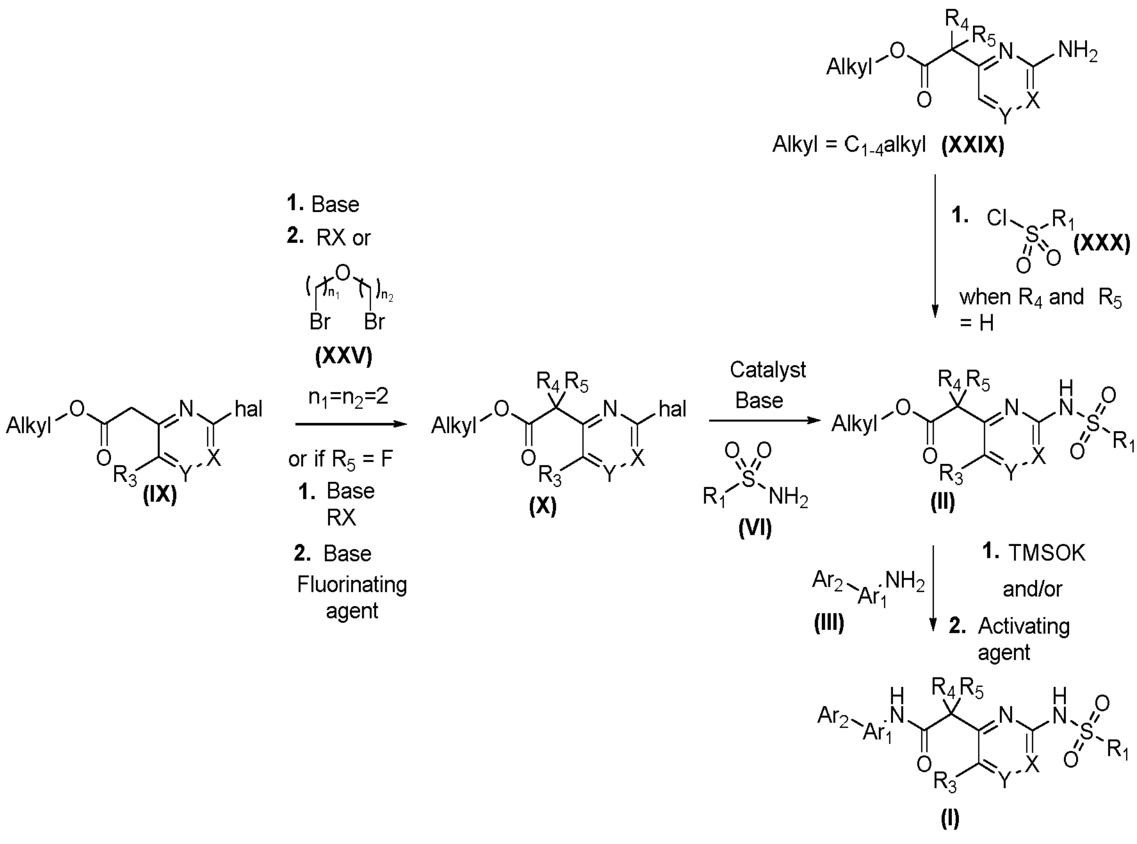


(XXIX) (XXX) (XXV) (IX) (X) (VI) (II) (III) (I)

Suitably, X is N, Y is CH, $R_3$ is H, **(IX)** is converted to **(X)** using a base and compounds of formula **(XXV)** wherein $n_1$=$n_2$=2, hal is Cl, alkyl is methyl, $R_4$ and $R_5$ together with the carbon atom to which they are attached form a tetrahydropyranyl ring, and compounds of formula **(II)** are converted to compounds of formula **(I)** using $AlMe_3$ and compounds of formula **(III).**

Compounds of general formula **(I)** where $R_1$, Ar1 and Ar2 are defined above and $R_4$ and $R_5$ together with the carbon to which they are attached form a $C_{3-6}$heterocycloalkyl, may be prepared in three steps starting from intermediate of general formula **(IX),** see Scheme 5a. Firstly, the derivative **(IX)** can be reacted with a symmetric di-bromoether of general formula **(XXV)** as shown in Scheme 5a to give an alpha-cyclic compound of formula **(X).** The intermediate thus obtained may be further reacted with sulfonamides of general formula **(VI)** to give compounds of formula **(II).** Finally, subjecting derivatives **(II)** to $AlMe_3$ in the presence of anilines of type **(III)** yields compounds of general formula **(I).** Alternative reaction conditions for converting compounds of formula **(IX)** to compounds of formula **(I)** are described above in respect of Schemes 2a and 2b.

Compounds of general formula **(I)** where $R_1$, $R_3$, Art and Ar2 are defined above, X = Y = CH or X = CH and Y = N, hal = Br or C!, $R_4$ is $C_{1-6}$alkyl and $R_5$ is H or $C_{1-6}$alkyl may be prepared in three or four steps starting from intermediate of general formula **(IX).** Reaction of a derivative of general formula **(IX)** with an inorganic base such as potassium carbonate, in the presence of an alkylating agent leads to alkylation alpha to the ester to give compounds of formula **(X).** It will be understood by persons skilled in the art that both mono- and dialkylation may be achieved with careful control of the reaction conditions. Compounds of formula **(X)** may then be progressed to final compounds of formula **(I)** following the steps described above in Scheme 5b.

Compounds of general formula **(I)** where $R_1$, $R_3$, Art and Ar2 are defined above, X = Y = CH or X = CH and Y = N and $R_4$ and $R_5$ together with the carbon to which they are attached form a $C_{3-6}$heterocycloalkyl, may be prepared in the same manner as described above for compounds when X = N and Y = CH.

Compounds of general formula **(II)** when $R_1$ and $R_3$ are as defined above, $R_4$ = $R_5$ = H and X and Y = CH may also be obtained by sulfonylation of commercial amines of formula **(XXIX)** with a suitable sulfonyl chloride **(XXX)** in pyridine. Intermediate **(II)** may then undergo hydrolysis and amide coupling using methods previously described.

Compounds of general formula **(I)** where $R_1$, $R_3$, Ar1 and Ar2 are defined above, X = CH and Y = N, hal = Br or C!, $R_4$ is $C_{1-6}$alkyl and $R_5$ is F may be prepared starting from intermediate of general formula **(IX).** Firstly mono alkylation alpha to the ester may be achieved by treatment with an inorganic base such as potassium carbonate, in the presence of an alkylating agent. Reaction of these products with a strong base such as LHMDS followed by exposure to a fluorinating agent such as *N*-fluoro-*N*-(phenylsulfonyl)benzenesulfonamide may produce compounds of formula **(X).** Compounds of formula **(X)** can then be progressed to compounds of formula **(I)** following the steps described in Scheme 5b.

## Scheme 6a

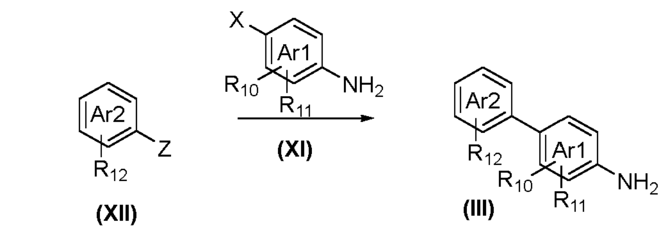

**(XII)** **(XI)** **(III)**

Z = $B(OH)_2$, $B(pin)_2$
X = Br, Cl

## Scheme 6b

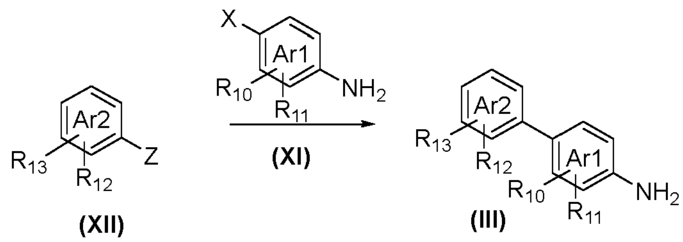

**(XII)** **(XI)** **(III)**

Z = $B(OH)_2$, $B(pin)_2$
X = Br, Cl

Intermediates of formula **(III)** wherein Ar1, $R_{10}$, $R_{11}$ and $R_{12}$ are defined above and Ar2 is an unsubstituted or substituted 3-pyridyl ring, may be synthesised by coupling under Suzuki conditions of a boronate of general formula (XII), wherein $R_{12}$ is defined above and Z represents a dihydroxyboryl or dialkyloxyboryl group, usually a 4,4,5,5-tetramethyl-1,3,3,2-dioxaborolan-2-yl group, to a substituted pyridine of formula **(XI)** where X denotes a halide. The couplings

according to the Suzuki method are performed, for example, by heating in the presence of a catalyst such as [1,1'-bis(diphenylphosphino)ferrocene]dichloropalladium(II) complex with dichloromethane and an inorganic base such as potassium carbonate in a solvent mixture of dioxane and water.

**Scheme 7a**

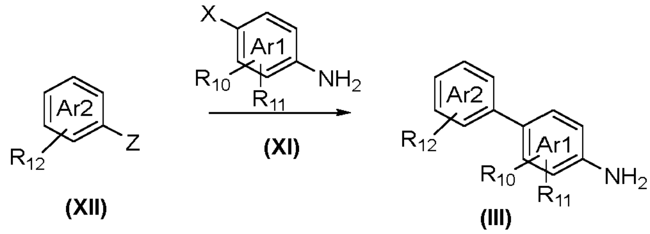

(XII) (XI) (III)

Z = Br, Cl
X = $B(OH)_2$, $B(pin)_2$

**Scheme 7b**

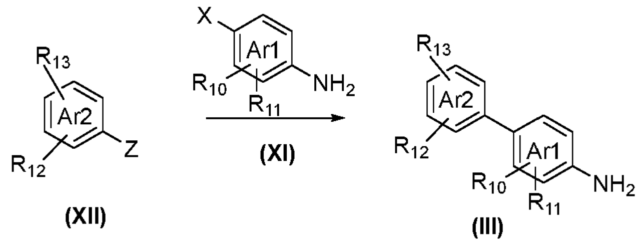

(XII) (XI) (III)

Z = Br, Cl
X = $B(OH)_2$, $B(pin)_2$

Intermediates of formula **(III)** wherein Ar1, $R_{10}$, $R_{11}$ and $R_{12}$ are defined above and Ar2 is an unsubstituted or substituted 2,5-pyrazinyl ring, may be synthesised by coupling under Suzuki conditions of an aromatic halide of general formula **(XII)** and Z represents a halide, to a boronate of general formula **(XI)** where X denotes a dihydroxyboryl or dialkyloxyboryl group, usually a 4,4,5,5-tetramethyl-1,3,3,2-dioxaborolan-2-yl group. The couplings according to the Suzuki method are performed, for example, by heating in the presence of a catalyst such as tetrakis(triphenylphosphine)palladium or [1,1'-bis(diphenylphosphino)ferrocene]dichloro palladium(II) and an inorganic base such as potassium carbonate in a solvent mixture of dioxane and water.

## Scheme 8

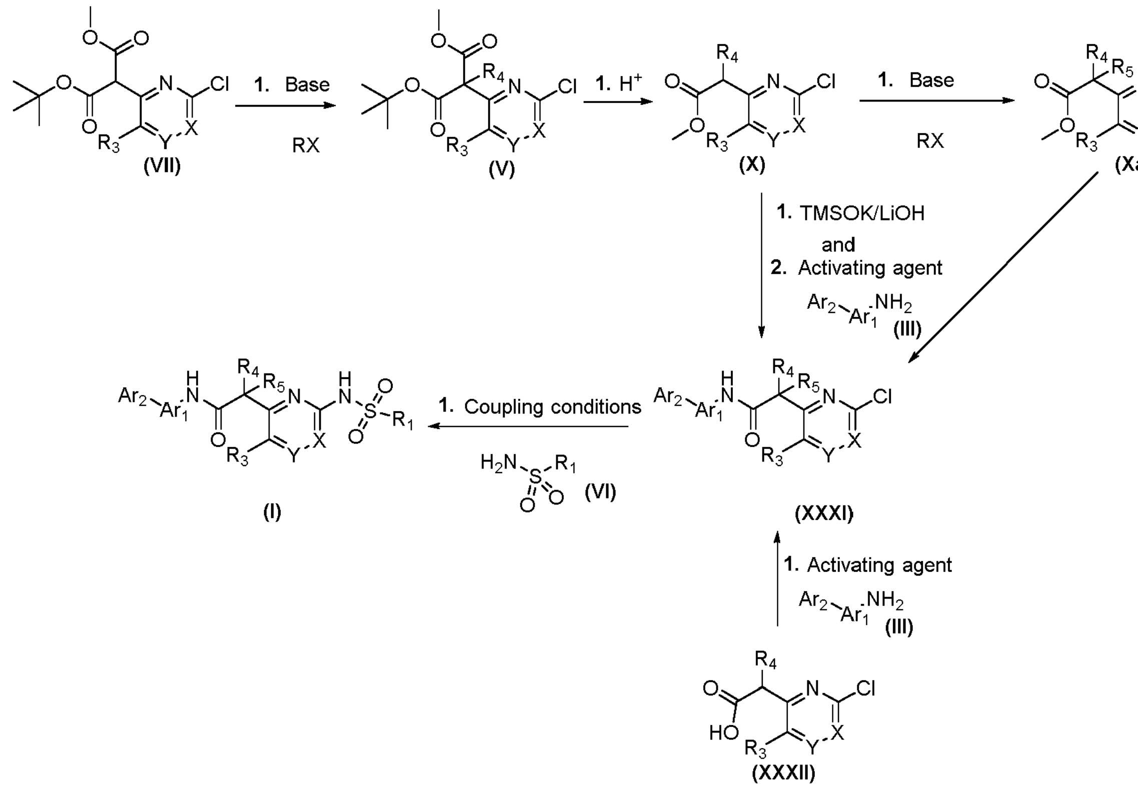


In general and as illustrated in Scheme 8, the compounds of formula **(I)** where $R_1$, $R_3$, Art and Ar2 are defined above, where X = N and Y = CH, where $R_4$ = H, $C_{1-6}$alkyl or $CH_2CH_2OMe$ and where $R_5$ = H may be prepared in four or five steps starting from an intermediate of general formula **(VII).** Alkylation can be achieved by treatment of intermediate **(VII)** with an inorganic base, such as sodium hydroxide, in the presence of an alkylating agent, such as iodoethane to yield compounds of the general formula **(V).** Decarboxylation can be initiated with a strong acid such as TFA to obtain intermediates of formula **(X).** Such intermediates may then undergo saponification and amide coupling according to methods described in Scheme 1 to give compounds of formula **(XXXI).** Final compounds of formula **(I)** can be accessed by coupling intermediates of formula **(XXXI)** with a primary sulfonamide as previously described in Scheme 1.

In general and as illustrated in Scheme 8, the compounds of formula **(I)** where $R_1$, $R_3$, Ar1 and Ar2 are defined above, where X = CH and Y = N, where $R_4$ = H or $CH_2CH_2OMe$ and where $R_5$ = H or Me, may be prepared in starting from an intermediate of general formula **(VIII)** following comparable methods to those described for when X = N and Y = CH in Scheme 8. If a linker where $R_5$ = Me is required alkylation of intermediates of formula **(X)** may be treated with an alkylating agent in the presence of a base to generate intermediates such as **(Xa).** Compounds of formula **(Xa)** can then be converted to final compounds via a three step procedure as described in Scheme 8.

Compounds of general formula **(XXXI)** when $R_4$ = $R_5$ = H and X = CH and Y = N may also be obtained by coupling commercial acids of formula **(XXXII)** with anilines of formula **(III)** under amide coupling conditions previously described. Compounds of this type can then be progressed to compounds of formula **(I)** using the previously described sulfamidation conditions.

## Scheme 9a

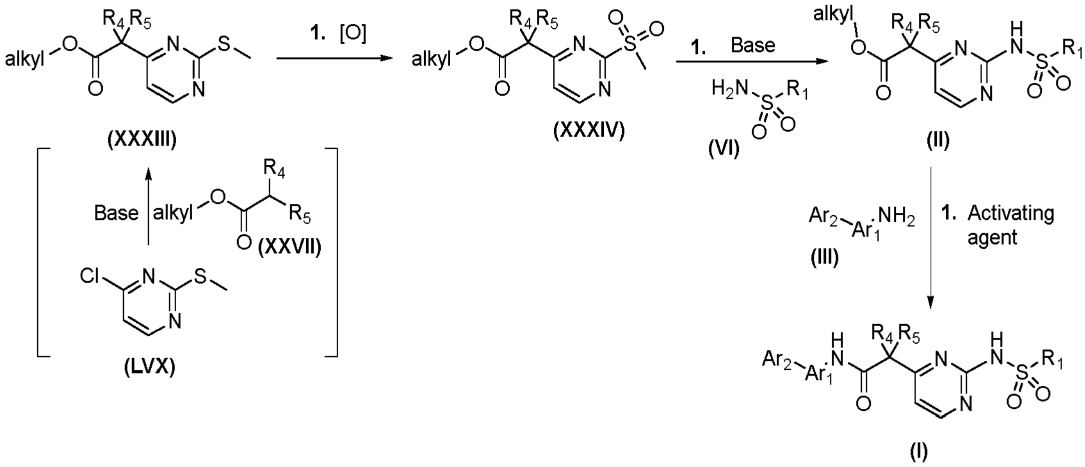


In general and as illustrated in Scheme 9a, compounds of formula **(I)** wherein $R_1$, Ar1 and Ar2 are as defined above, alkyl is $C_{1-4}$alkyl such as methyl or ethyl, e.g. methyl, and for example, $R_4$ and $R_5$ together with the carbon atom to which they are attached form a $C_{3-6}$heterocycloalkyl ring may be prepared in four steps from chloro-pyrimidine **(LVX).** Intermediates **(XXXVII)** are coupled to chloro-pyrimidine **(LVX)** in the presence of a base such as LHMDS to give intermediates **(XXXIII).** Thioethers of the general formula **(XXXIII)** may be transformed to sulfones **(XXXIV)** in the presence of an oxidising agent such as mCPBA. Displacement of the sulfone group with a primary sulphonamide **(VI)** in the presence of a base such as $Cs_2CO_3$ and a solvent such as N-methyl pyrrolidone gives compounds of formula **(II).** Compounds of formula **(I)** may be obtained by a strong base-mediated amide formation between compounds **(II)** and **(III)** at room temperature using bases such as iPrMgCl, LiHMDS or KOtBu.

## Scheme 9b

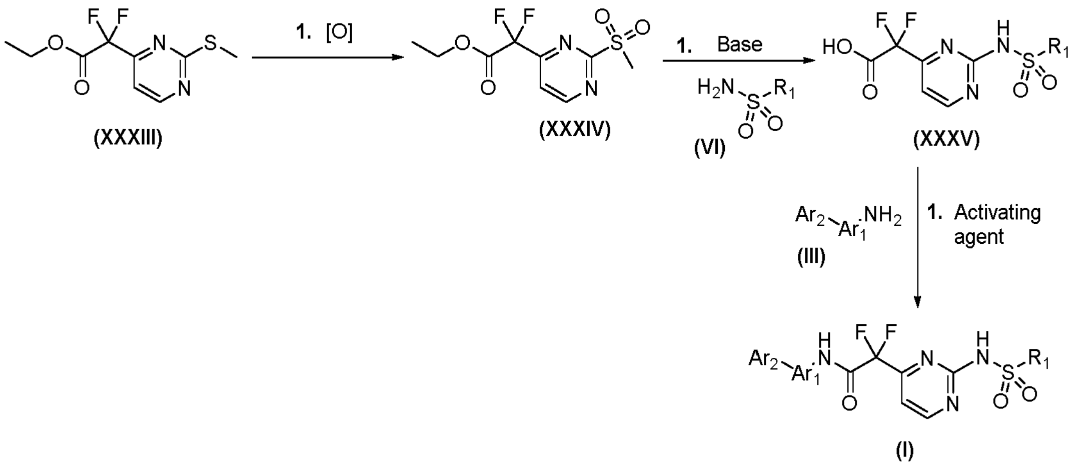


In general and as illustrated in Scheme 9b, compounds of general formula **(I)** wherein $R_4$ and $R_5$ are both F, $R_1$, Ar1 and Ar2 are defined above may be prepared in 3 steps from literature compound ethyl 2,2-difluoro-2-(2-(methylthio)pyrimidin-4-yl)acetate **(XXXIII)** i.e. $R_4 = R_5 = F$. Thioethers of the general formula **(XXXIII)** may be transformed to sulfones **(XXXIV)** in the presence of an oxidising agent such as Oxone® at room temperature in a polar protic solvent such as MeOH. Displacement of the sulfone group with a primary sulphonamide **(VI)** and subsequent ester hydrolysis to give acids of the general formula **(XXXV)** can be performed in a one pot procedure in the presence of a strong base such as NaH and in a polar aprotic solvent such as DMF. Acid derivative **(XXXV)** can then be activated with a coupling reagent such as HATU in the presence of a base and coupled with an aniline such as **(III)** to obtain the final compounds of formula **(I).**

## Scheme 10

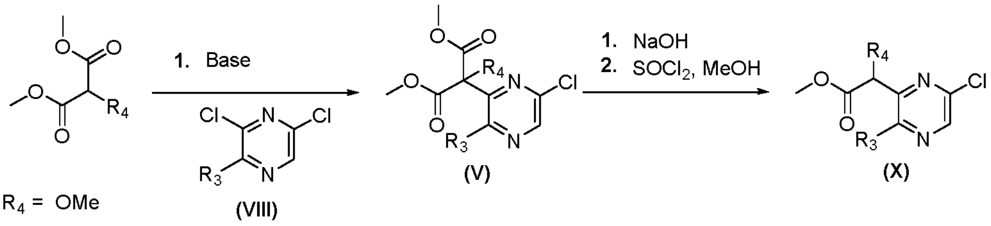


$R_4$ = OMe (VIII) (V) (X)

In general and as illustrated in Scheme 10, the compounds of general formula **(X)** where $R_1$, $R_3$, Ar1 and Ar2 are defined above and where $R_4$ = OMe may be prepared in four, five or six steps starting from a 2,4-dichloropyrazine derivative of general formula **(VIII).** Derivative **(VIII)** can be reacted with a symmetrical malonate ester when $R_4$ = OMe in the presence of a strong base such as sodium hydride and in a polar solvent such as DMF to form intermediate compounds of formula **(V).** A two-step procedure can then be carried out to access compounds of general structure **(X).** Firstly saponification using an alkali metal hydroxide such as NaOH can generate the biscarboxylic acid which once acidified may undergo spontaneous decarboxylation. The resulting carboxylic acid can then be converted to esters of general formula **(X)** by treatment with an activating agent such as thionyl chloride in the presence of an alcoholic solvent such as methanol. Derivatives of formula **(X)** can be converted to final compounds for formula **(I)** using methods previously described in Scheme 5.

## Scheme 11

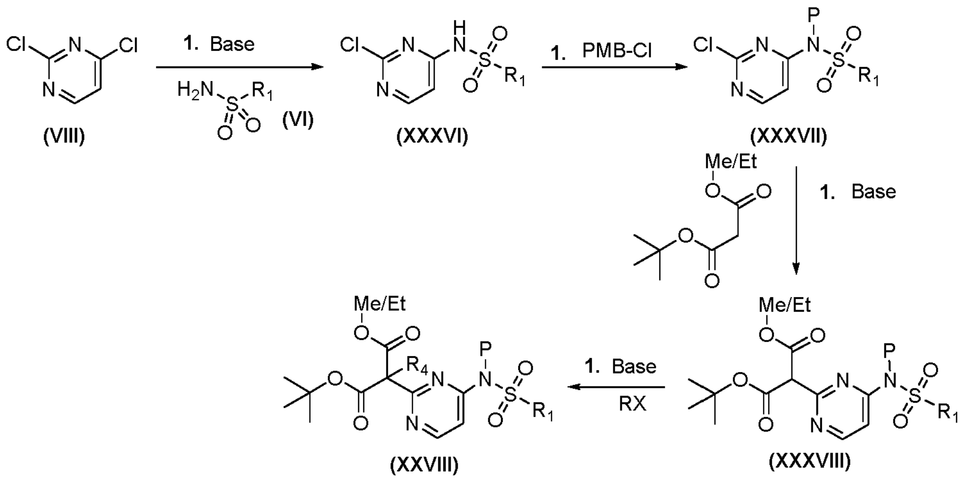


(VIII) (VI) (XXXVI) (XXXVII) (XXXVIII) (XXVIII)

In general and as illustrated in Scheme 11, the compounds of formula **(XXVIII)** where $R_1$ is defined above and where $R_4$ = H or Et, may be prepared in seven steps starting from a 2,4-dichloropyrimidine derivative of general formula **(VIII).** The derivative **(VIII)** can be reacted with sulfonamide of type **(VI)** in the presence of an inorganic base such as potassium carbonate to displace the more reactive chloride and form intermediate compounds of formula **(XXXVI).** Compounds of formula **(XXXVI)** may be protected e.g. using PMB-Cl to give compounds of formula **(XXXVII).**

This compound can then be converted to compounds of general formula **(XXXVIII)** by treatment with an unsymmetrical malonate in the presence of a base such as cesium carbonate in a solvent such as dimethoxyethane.

If mono alkylation is desired then treatment of intermediate **(XXXVIII)** with an inorganic base, such as potassium carbonate, in the presence of an alkylating agent, such as EtI, yields compounds of the general formula **(XXVIII).** This compound can then be converted to final compounds of formula **(I)** using methods previously described in Scheme 4.

Wherein $R_4$ = H, compounds of general formula **(XXXVIII)** can be taken directly to compounds of general formula **(I)** (such as described above).

**Benzamide pyrimidines**

## Scheme 12

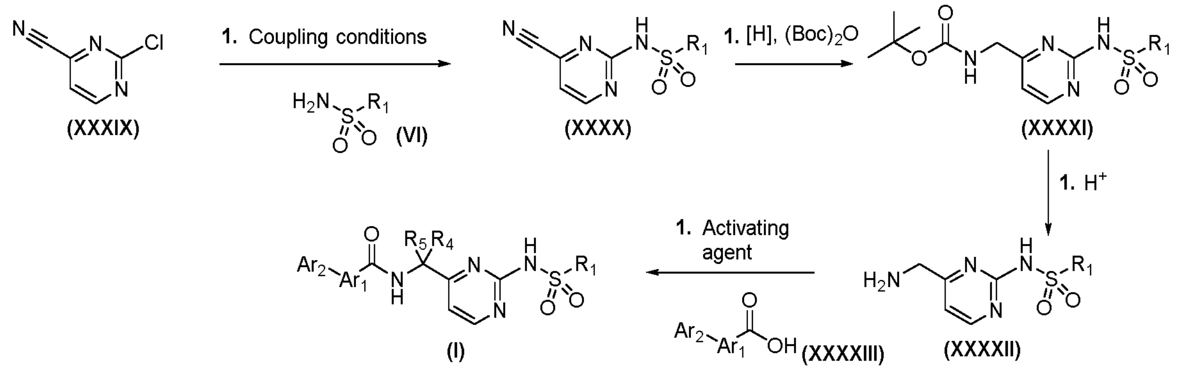


Compounds of general formula **(I)** may be obtained by a four step process, as shown in Scheme 12. 2-Chloropyrimidine-4-carbonitrile **(XXXIX)** can be converted to the corresponding sulfonamide **(XXXX)** using palladium catalysed sulfamination conditions previously reported in Scheme 1. Reduction of the nitrile group using sodium borohydride in the presence of nickel (II) chloride and di-*tert*-butyl dicarbonate may yield the protected benzylamine derivative of general formula **(XXXXI).** Deprotection can be carried out by acid hydrolysis using HCl in dioxane to yield benzylamine derivative of general formula **(XXXXII).** Amide coupling conditions may then be employed to convert the benzylamine derivative **(XXXXII)** to amides of general formula **(I)** by employing a coupling reagent together with a biaryl carboxylic acid **(XXXXIII)** (commercially available or prepared as in Scheme 19).

## Scheme 13

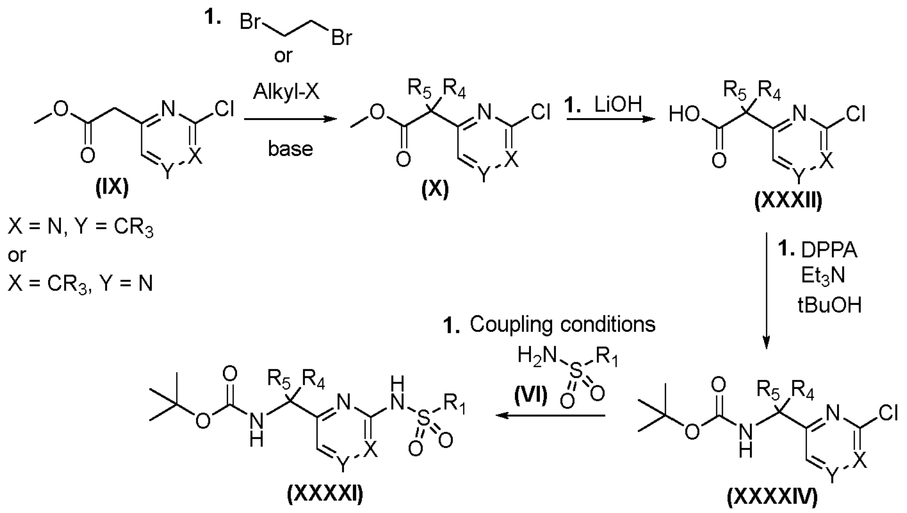


Compounds of general formula **(I)** where $R_1$, Ar, and $Ar_2$ are defined above, X = N and Y = CH, $R_3$ is H, $R_4$ is $C_{1-6}$alkyl and $R_5$ is H or $C_{1-6}$alkyl or $R_4$ and $R_5$ together with the carbon to which they are attached form a $C_{3-6}$cycloalkyl may be obtained by a six step process, as shown in Scheme 13 (and Scheme 12 for certain steps). Firstly, the derivative **(IX)** can be reacted with an alkyl halide to give compounds of general formula **(X)** where $R_4$ = alkyl and $R_5$ = H. Alternatively derivative **(IX)** can be reacted with an alkyl bis-halide to give compounds of general formula **(X)** where $R_4$ and $R_5$ can be connected to form a $C_{3-6}$heterocycloalkyl ring as defined above. Carboxylic acid **(XXXII)** can be obtained by hydrolysis of methyl ester **(X)** using an alkali metal base such as lithium hydroxide in a solvent mixture such as THF/MeOH. Curtius rearrangement can be carried out, for example, using diphenylphosphoryl azide in the presence of triethylamine and tert-butanol to yield carbamates such as **(XXXXIV).** The corresponding sulfonamide **(XXXXI)** may then be accessed by a palladium catalysed sulfamination employing conditions previously reported in Scheme 1. Carbamates of formula **(XXXXI)** can then be progressed to final compounds of formula **(I)** following Scheme 12.

Compounds of general formula **(I)** where $R_1$, Ar, and $Ar_2$ are defined above, X = CH and Y = N, $R_4$ is $C_{1-6}$alkyl and $R_5$ is H may be obtained by a four step process starting from a commercially available acid of formula **(XXXII)** following the subsequent steps described in Scheme 13.

## Scheme 14

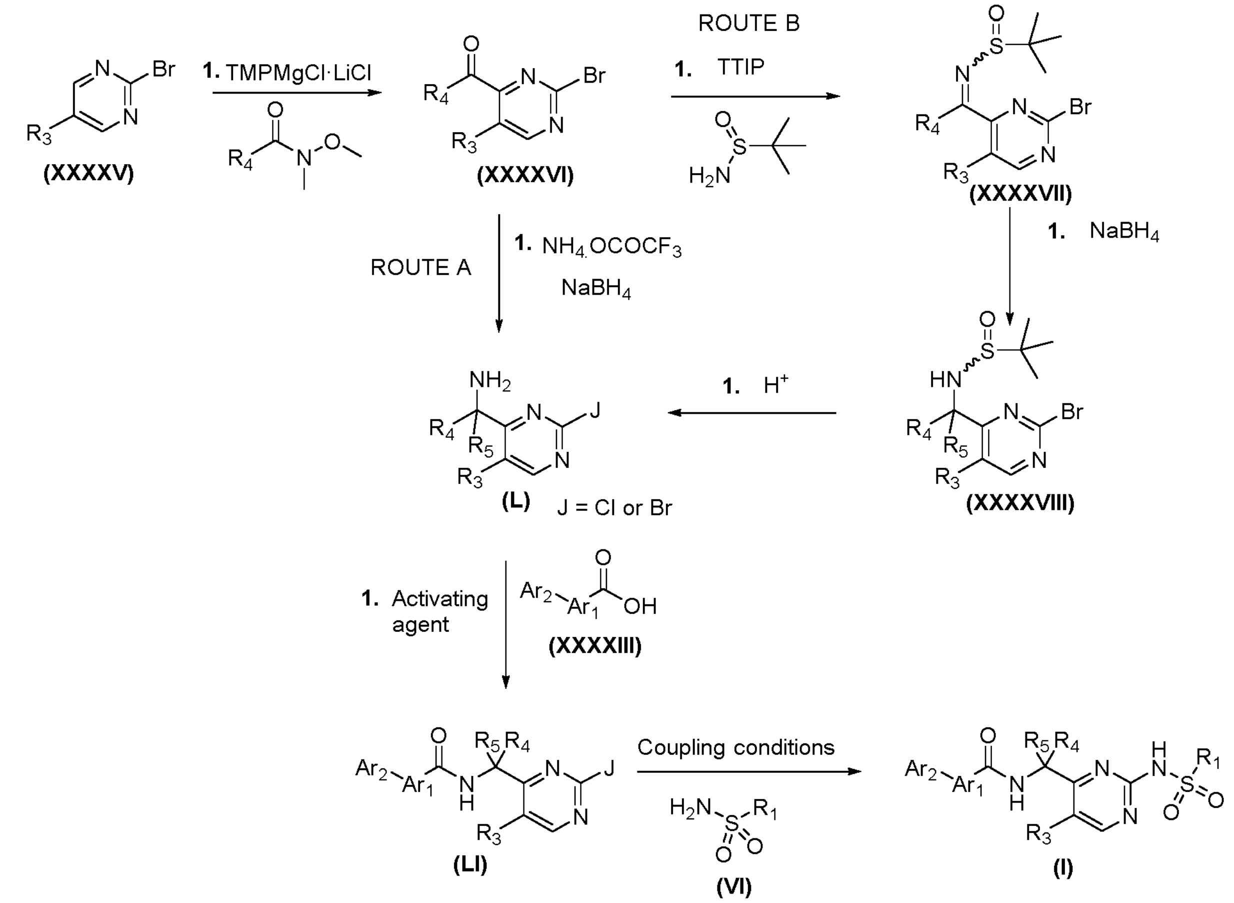


The pyrimidin-4-yl(propan-2-yl)benzamide derivatives of formula **(I)** in which $R_1$, $R_3$, Ar1 and Ar2 are defined above, $R_4$ = alkyl and $R_5$ = H may be prepared by two different routes as shown in Scheme 14. The two routes both begin by conversion of 2-bromopyrimidine to the corresponding ketone **(XXXXVI)** by treatment with a suitable base such as TMPMgCl LiCl followed by exposure to the Weinreb amide derivative. The two routes then converge at compounds of general formula **(L)** where they are then taken onto the final analogues by a two-step process.

ROUTE A: Treatment of ketone derivatives **(XXXXVI)** with ammonium trifluoroacetate followed by reduction using sodium borohydride may yield the benzylamine **(L).**

ROUTE B: Ketone of the general formula **(XXXXVI)** is converted to sulfinamide **(XXXXVII)** by treatment with a Lewis acid such as titanium isopropoxide followed by exposure to a sulfinamine such as 2-methylpropane-2-sulfinamide. Reduction using sodium borohydride may yield the sulfinamide **(XXXXVIII).** The intermediate of formula **(XXXXVIII)** may then be deprotected using a strong acid, such as HCl which may also lead to halogen exchange to give amines of general formula **(L)** where X = Cl.

Amide coupling conditions reported in Scheme 12 may then be employed to convert the benzylamine derivatives **(L)** to amides of general formula **(LI).** A palladium catalysed sulfamination as described in Scheme 12 may yield compounds of the general formula **(I).**

## Scheme 15

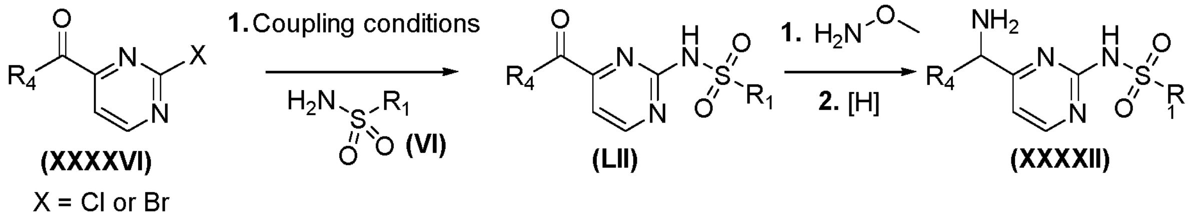


In general and as illustrated in Scheme 15, compounds of general formula **(XXXXII)** may be obtained by a three

step process from a ketone derivative of general formula **(XXXXVI).** Sulfamidation of derivative **(XXXXVI)** may be carried out using conditions described in Scheme 12 to give compounds of formula **(LII).** Oxime formation with methoxyamine can be followed by reduction in the presence of a suitable catalyst such as Pd/C under an atmosphere of $H_2$ gas in a polar protic solvent such as MeOH to afford amine derivatives of general formula **(XXXXII).** Amines of this type can be progressed to final compounds following Scheme 12.

## Scheme 16

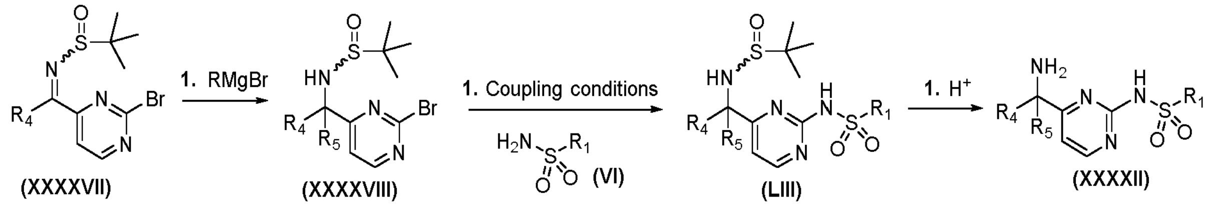

(XXXXVII) (XXXXVIII) (VI) (LIII) (XXXXII)

Alternatively, compounds of general formula **(XXXXII)** may be obtained by a three step process, as shown in Scheme 16. *N*-(2-(2-bromopyrimidin-4-yl)butan-2-yl)-2-methylpropane-2-sulfinamide **(XXXXVII)** can be synthesized as described above (Scheme 14). The imine can then be exposed to a nucleophile such as MeMgBr to yield intermediates such as **(XXXXVIII).** The corresponding sulfonamide **(LIII)** may then be accessed by a palladium catalysed sulfamination as described in Scheme 1. Deprotection can be carried out by acid hydrolysis using HCl to yield the benzylamine derivatives of general formula **(XXXXII)** which can then be converted to final compounds following Scheme 12.

## Scheme 17

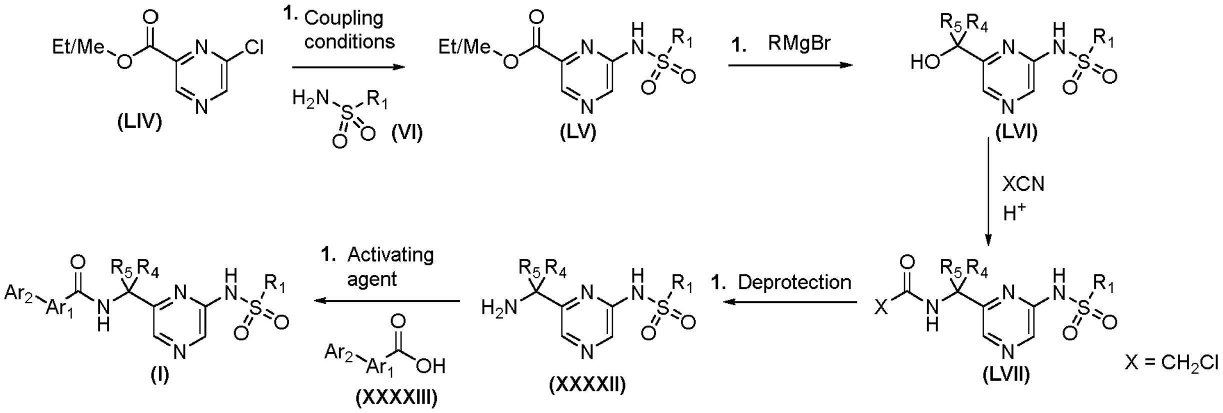

(LIV) (VI) (LV) (LVI) (LVII) (XXXXII) (XXXXIII) (I)

The benzamide derivatives of formula **(I)** in which $R_1$, $R_3$, Art and Ar2 are defined above and $R_4$ = $R_5$ = alkyl may be prepared in 5 steps as described in Scheme 17 by coupling a commercial aromatic chloride such as **(LIV)** with a primary sulfonamide using sulfamidation conditions described in Scheme 1. A double Grignard addition may then be carried out in an aprotic solvent such as THF to form intermediates of formula **(LVI).** A Ritter type reaction may then be undertaken using an alkylnitrile, such as 2-chloroacetonitrile in the presence of an acid such as $H_2SO_4$. The intermediate of formula **(LVII)** can be deprotected by reaction with thiourea in a protic solvent such as ethanol in the presence of acetic acid and heated under reflux to yield the benzylamine derivatives **(XXXXII).** Final compounds of formula **(I)** can be accessed using amide coupling conditions reported in Scheme 12.

## Scheme 18

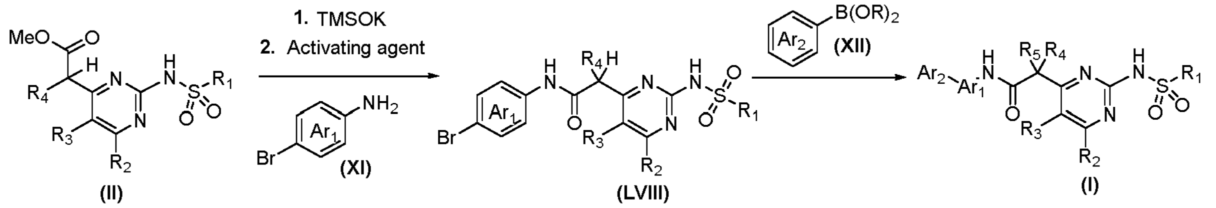

(II) (XI) (LVIII) (XII) (I)

In general and as illustrated in Scheme 18 compounds of general formula **(I)** can be prepared by conversion of intermediate **(II)** by a three step process. Firstly, saponification of **(II)** using an agent such as TMSOK gives the

intermediate carboxylic acid derivative, which may be followed by reaction with an activating agent such as T3P and a bromo-aniline of formula **(XI).** Intermediates of formula **(LVIII)** are then converted to compound of the invention of general formula **(I)** by coupling under Suzuki conditions with a boronate ester of general formula **(XII).** The boronate is usually a dihydroxyboryl or dialkyloxyboryl group, usually a 4,4,5,5-tetramethyl-1,3,3,2-dioxaborolan-2-y group. The couplings according to the Suzuki method are performed, for example, by heating in the presence of a catalyst such as [1,1'-bis(diphenylphosphino)ferrocene]dichloropalladium(II) and an inorganic base such as potassium carbonate in a solvent mixture of dioxane and water. It will be understood by persons skilled in the art that many catalysts and conditions can be employed for such couplings.

## Scheme 19

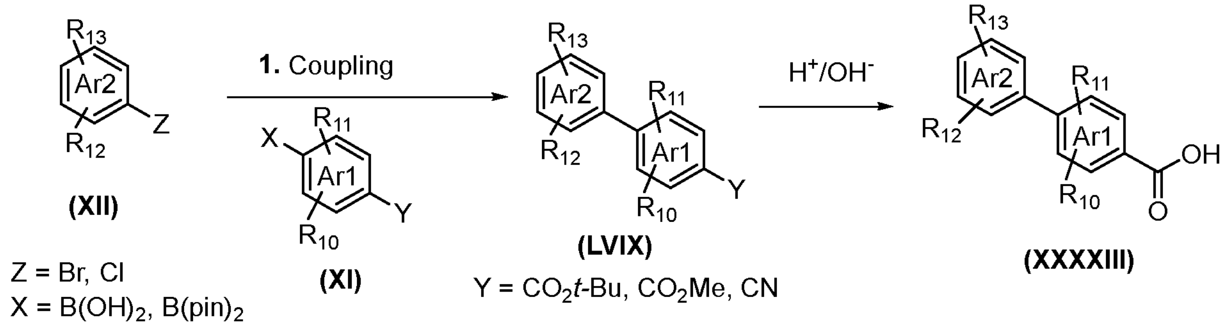


Intermediates of formula **(XXXXIII)** where $Ar_2$ is an unsubstituted or substituted 2-pyrazine ring or 3-pyridyl ring, may be synthesised as shown in Scheme 19 by coupling under Suzuki conditions of an aromatic halide of general formula **(XII),** of which $R_{12}$ and $R_{13}$ are defined above and Z represents Br or Cl, to a boronate of general formula **(XI)** wherein $R_{10}$ and $R_{11}$ are defined above, X denotes a dihydroxyboryl or dialkyloxyboryl group, such as a 4,4,5,5-tetramethyl-1,3,3,2-dioxaborolan-2-yl group. The couplings according to the Suzuki method are performed, for example, by heating in the presence of a catalyst such as [1,1'-bis(diphenylphosphino)ferrocene]dichloropalladium(II).$CH_2Cl_2$ adduct and an inorganic base such as cesium carbonate in a solvent mixture of dioxane and water under an inert atmosphere such as a nitrogen atmosphere to give compounds of formula **(LVIX).** The carboxylic acids of general formula **(XXXXIII)** are obtained by either deprotection of the t-butyl ester using a strong acid, such as TFA in a solvent of $CH_2Cl_2$, hydrolysis of the methyl ester using an alkali metal hydroxide such as NaOH in a solvent mixture such as THF/MeOH or hydrolysis of the nitrile using a strong acid such as concentrated HCl.

### Intermediates of the Invention

The present invention also relates to novel intermediates in the synthesis of compounds of formula (I). Particular intermediates of interest are those of the following general formulae, wherein unless otherwise stated, the variable groups and associated preferences are as defined previously for compounds of formula **(I):**

- a compound of formula **(II):**

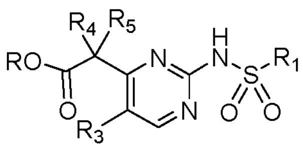

**(II)**

wherein R is H, $C_{1-6}$alkyl (e.g. methyl and ethyl) or benzyl;

- a compound of formula **(XX):**

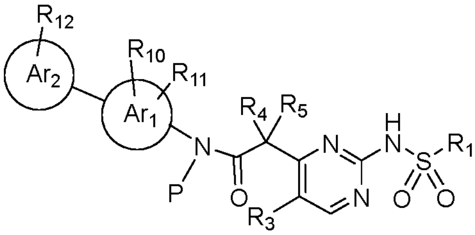

(XX);

wherein P is a nitrogen protecting group such as para-methoxybenzyl;

- a compound of formula **(XXIV):**

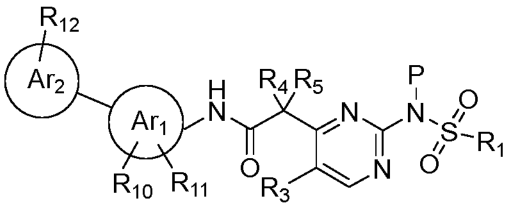

(XXIV);

wherein P is a nitrogen protecting group such as para-methoxybenzyl.

Also of interest are the following compounds wherein unless otherwise stated, variable groups and associated preferences are as defined previously for compounds of formula **(I):**

- a compound of formula **(II):**

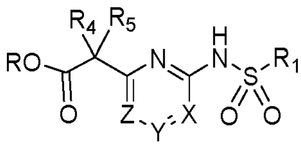

(II)

wherein R is H, $C_{1-6}$alkyl (e.g. methyl and ethyl) or benzyl;

- a compound of formula **(XX):**

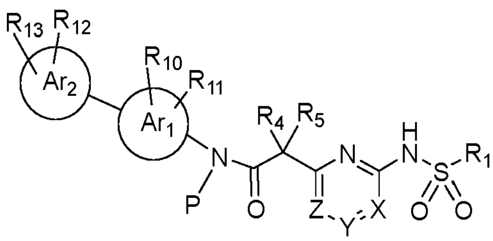

(XX);

wherein P is a nitrogen protecting group such as para-methoxybenzyl;

- a compound of formula **(XXIV):**

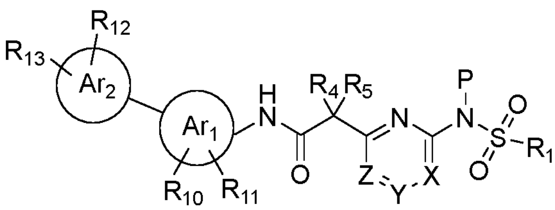

(XXIV);

wherein P is a nitrogen protecting group such as para-methoxybenzyl;

- a compound of formula **(XXXI):**

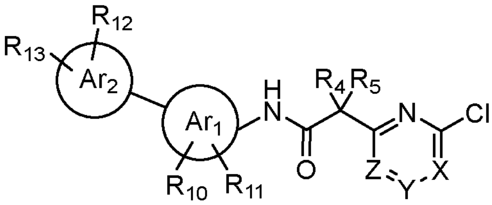

**(XXXI)**;

wherein $Ar_2$ is 3-pyridyl or 2,5-pyrazinyl;

- a compound of formula **(XXXXII):**

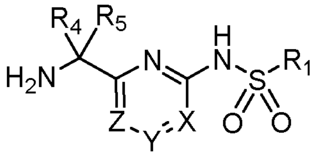

**(XXXXII)**;

- a compound of formula **(LI):**

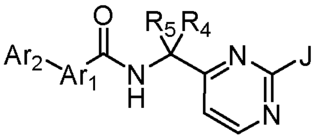

**(LI)**;

wherein J is Cl or Br;

- a compound of formula **(LVIII):**

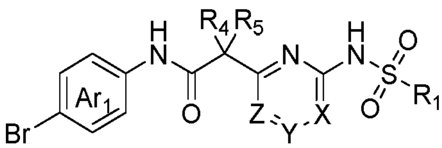

**(LVIII)**.

- Included as an aspect of the invention are all novel intermediates described in the examples, including:
- Intermediates INTC1 to INTC47; and
- Intermediates INTD1 to INTD51.

Also provided are intermediates INTC48 to INTC53.

Also provided are intermediates INTC54 to INTC177.

Also provided are intermediates INTC178 and INTC179.

Also provided are intermediates INTD52 to INTD86.

Included as an aspect of the invention are salts such as pharmaceutically acceptable salts of any one of the intermediates disclosed herein.

**Therapeutic Methods**

Compounds of formula (I) of the present invention have utility as inhibitors of CTPS1.

Therefore, the invention also provides a compound of formula (I), or a pharmaceutically acceptable salt and/or solvate (e.g. salt) thereof, for use as a medicament, in particular in the treatment or prophylaxis of a disease or disorder wherein an inhibitor of CTPS1 is beneficial, for example those diseases and disorders mentioned herein below.

More suitably, the disease or disorder wherein an inhibitor of CTPS1 is beneficial is a disease or disorder wherein a reduction in T-cell and/or B-cell proliferation would be beneficial.

The invention also provides a compound of formula (I), or a pharmaceutically acceptable salt and/or solvate (e.g. salt) thereof, for use in the inhibition of CTPS1 in a subject.

The invention also provides a compound of formula (I), or a pharmaceutically acceptable salt and/or solvate (e.g. salt) thereof, for use in the reduction of T-cell and/or B-cell proliferation in a subject.

More suitably, the disease or disorder wherein an inhibitor of CTPS1 is beneficial is a disease or disorder wherein a reduction in T-cell and/or B-cell proliferation would be beneficial.

The term 'treatment' or 'treating' as used herein includes the control, mitigation, reduction, or modulation of the disease state or its symptoms.

The term 'prophylaxis' or 'preventing' is used herein to mean preventing symptoms of a disease or disorder in a subject or preventing recurrence of symptoms of a disease or disorder in an afflicted subject and is not limited to complete prevention of an affliction.

Suitably, the disease or disorder is selected from rejection of transplanted cells and tissues, Graft-related diseases or disorders, allergies and autoimmune diseases.

In one embodiment the disease or disorder is the rejection of transplanted cells and tissues. The subject may have been transplanted with a graft selected from the group consisting of heart, kidney, lung, liver, pancreas, pancreatic islets, brain tissue, stomach, large intestine, small intestine, cornea, skin, trachea, bone, bone marrow (or any other source of hematopoietic precursor cells and stem cells including hematopoietic cells mobilized from bone marrow into peripheral blood or umbilical cord blood cells), muscle, or bladder. The compounds of the invention may be of use in preventing or suppressing an immune response associated with rejection of a donor tissue, cell, graft or organ transplant in a subject.

In a further embodiment the disease or disorder is a Graft-related disease or disorder. Graft-related diseases or disorders include graft versus host disease (GVHD), such as GVHD associated with bone marrow transplantation, and immune disorders resulting from or associated with rejection of organ, tissue, or cell graft transplantation (e.g., tissue or cell allografts or xenografts), including, e.g., grafts of skin, muscle, neurons, islets, organs, parenchymal cells of the liver, etc, and Host-Versus-Graft-Disease (HVGD). The compounds of the invention may be of use in preventing or suppressing acute rejection of such transplant in the recipient and/or for long-term maintenance therapy to prevent rejection of such transplant in the recipient (e.g., inhibiting rejection of insulin-producing islet cell transplant from a donor in the subject recipient suffering from diabetes). Thus the compounds of the invention have utility in preventing Host-Versus-Graft-Disease (HVGD) and Graft-Versus-Host-Disease (GVHD).

A CTPS1 inhibitor may be administered to the subject before, after transplantation and/or during transplantation. In some embodiments, the CTPS1 inhibitor may be administered to the subject on a periodic basis before and/or after transplantation.

In another embodiment, the disease or disorder is an allergy.

In additional embodiments the immune related disease or disorder is an autoimmune disease. As used herein, an "autoimmune disease" is a disease or disorder directed at a subject's own tissues. Examples of autoimmune diseases include, but are not limited to Addison's Disease, Adultonset Still's disease, Alopecia Areata, Alzheimer's disease, Anti-neutrophil Cytoplasmic Antibodies (ANCA)-Associated Vasculitis, Ankylosing Spondylitis, Anti-phospholipid Syndrome (Hughes' Syndrome), Aplastic Anemia, Arthritis, Asthma, Atherosclerosis, Atherosclerotic plaque, Atopic Dermatitis, Autoimmune Hemolytic Anemia, Autoimmune Hepatitis, Autoimmune Hypophysitis (Lymphocytic Hypophysitis), Autoimmune Inner Ear Disease, Autoimmune Lymphoproliferative Syndrome, Autoimmune Myocarditis, Autoimmune Neutropenia, Autoimmune Oophoritis, Autoimmune Orchitis, Auto-Inflammatory Diseases requiring an immunosuppressive treatment, Azoospermia, Bechet's Disease, Berger's Disease, Bullous Pemphigoid, Cardiomyopathy, Cardiovascular disease, Celiac disease including Refractory Celiac Disease (type I and type II), Chronic Fatigue Immune Dysfunction Syndrome (CFIDS), Chronic Idiopathic Polyneuritis, Chronic Inflammatory Demyelinating Polyneuropathy (CIPD), Chronic Relapsing Polyneuropathy (Guillain-Barré syndrome), Churg-Strauss Syndrome (CSS), Cicatricial Pemphigoid, Cold Agglutinin Disease (CAD), chronic obstructive pulmonary disease (COPD), CREST Syndrome, Cryoglobulin Syndromes, Cutaneous Lupus, Dermatitis Herpetiformis, Dermatomyositis, Eczema, Epidermolysis Bullosa Acquisita, Essential Mixed Cryoglobulinemia, Evan's Syndrome, Exophthalmos, Fibromyalgia, Goodpasture's Syndrome, Grave's disease, Hemophagocytic Lymphohistiocytosis (HLH) (including Type 1 Hemophagocytic Lymphohistiocytosis), Histiocytosis/Histiocytic Disorders, Hashimoto's Thyroiditis, Idiopathic Pulmonary Fibrosis, Idiopathic Thrombocytopenia Purpura (ITP), IgA Nephropathy, Immunoproliferative Diseases or Disorders, Inflammatory Bowel Disease (IBD), Interstitial Lung Disease, Juvenile Arthritis, Juvenile Idiopathic Arthritis (JIA), Kawasaki's Disease, Lambert-Eaton Myasthenic Syndrome, Lichen Planus, Localized Scleroderma, Lupus Nephritis, Ménière's Disease, Microangiopathic Hemoytic Anemia, Microscopic Polyangitis, Miller Fischer Syndrome/Acute Disseminated Encephalomyeloradiculopathy, Mixed Connective Tissue Disease, Multiple Sclerosis (MS), Muscular Rheumatism, Myalgic Encephalomyelitis (ME), Myasthenia Gravis, Ocular Inflammation, Pemphigus Foliaceus, Pemphigus Vulgaris, Pernicious Anemia, Polyarteritis Nodosa, Polychondritis, Polyglandular Syndromes (Whitaker's syndrome), Polymyalgia Rheumatica, Polymyositis, Primary Agammaglobulinemia, Primary Biliary Cirrhosis/Autoimmune Cholangiopathy, Primary Glomerulonephritis, Primary Sclerosing Cholangitis, Psoriasis, Psoriatic Arthritis, Pure Red Cell Anemia, Raynaud's Phenomenon, Reiter's Syndrome/Reactive Arthritis, Relapsing Polychondritis, Restenosis, Rheumatic Fever, Rheumatic Disease, Rheumatoid Arthritis, Sarcoidosis, Schmidt's Syndrome, Scleroderma/Systemic Sclerosis, Sjörgen's Syndrome, Stiff-Man Syndrome, The Sweet Syndrome (Febrile Neutrophilic Dermatosis), Systemic Lupus Erythematosus (SLE), Systemic Scleroderma, Takayasu Arteritis,

Temporal Arteritis/Giant Cell Arteritis, Thyroiditis, Type 1 diabetes, Type 2 diabetes, Uveitis, Vasculitis, Vitiligo, Wegener's Granulomatosis, and X-linked lymphoproliferative disease.

Of particular interest are diseases and disorders which are mainly driven by T-cell activation and proliferation, including:

- diseases and disorders which are not linked to alloreactivity including:
    - Alopecia areata, atopic dermatitis, eczema, psoriasis, lichen planus, psoriatic arthritis, vitiligo;
    - Uveitis;
    - Ankylosing spondylitis, Reiter's syndrome/reactive arthritis;
    - Aplastic anemia, autoimmune lymphoproliferative syndrome/disorders, hemophagocytic lymphohistiocytosis;
    - Type 1 diabetes; and
    - Refractory celiac disease;
- Acute rejection of grafted tissues and transplanted organs; acute graft versus host disease (GVHD) after transplantation of bone marrow cells or any other source of allogenic cells including hematopoietic precursors cells and/or stem cells.

Also of interest are diseases and disorders which are driven by both T- and B-cell activation and proliferation, with an important involvement of B-cells, including:

- diseases and disorders for which the involvement of pathogenic auto-antibodies is well characterized, including:
    - Allergy;
    - Cicatricial pemphigoid, bullous pemphigoid, epidermolysis bullosa acquisita, pemphigus foliaceus, pemphigus vulgaris, dermatitis herpetiformis;
    - ANCA-associated vasculitis and microscopic polyangitis, vasculitis, Wegener's granulomatosis; Churg-Strauss syndrome (CSS), polyarteritis nodosa, cryoglobulin syndromes and essential mixed cryglobulinemia;
    - Systemic lupus erythematosus (SLE), antiphospholipid syndrome (Hughes' syndrome), cutaneous lupus, lupus nephritis, mixed connective tissue disease;
    - Thyroiditis, Hashimoto thyroiditis, Grave's disease, exophthalmos;
    - Autoimmune hemolytic anemia, autoimmune neutropenia, ITP, pernicious anaemia, pure red cell anaemia, micro-angiopathic hemolytic anemia;
    - Primary glomerulonephritis, Berger's disease, Goodpasture's syndrome, IgA nephropathy; and
    - Chronic idiopathic polyneuritis, chronic inflammatory demyelinating polyneuropathy (CIPD), chronic relapsing polyneuropathy (Guillain-Barré syndrome), Miller Fischer syndrome, Stiff man syndrome, Lambert-Eaton myasthenic syndrome, myasthenia gravis.
- diseases and disorders for which the involvement of B-cells is less clearly characterized (although sometimes illustrated by the efficacy of anti-CD20 monoclonal antibodies or intravenous immunoglobulin infusions) and may not correspond or be limited to the production of pathogenic antibodies (nevertheless, non-pathogenic antibodies are sometimes described or even often present and used as a diagnosis biomarker), including:
    - Addison's disease, autoimmune oophoritis and azoospermia, polyglandular syndromes (Whitaker's syndrome), Schmidt's syndrome;

- Autoimmune myocarditis, cardiomyopathy, Kawasaki's disease;
- Rheumatoid arthritis, Sjögren's syndrome, mixed connective tissue disease, polymyositis and dermatomyositis; polychondritis;
- Primary glomerulonephritis;
- Multiple sclerosis;
- Autoimmune hepatitis, primary biliary cirrhosis/ autoimmune cholangiopathy,
- Hyper acute rejection of transplanted organs;
- Chronic rejection of graft or transplants;
- Chronic Graft versus Host reaction / disease after transplantation of bone marrow cells or hematopoietic precursor cells.

Additionally of interest are diseases and disorders for which the mechanism is shared between activation/proliferation of T-cells and activation/proliferation of innate immune cells and other inflammatory cellular subpopulations (including myeloid cells such as macrophages or granulocytes) and resident cells (such as fibroblasts and endothelial cells), including:

- ▪ COPD, idiopathic pulmonary fibrosis, interstitial lung disease, sarcoidosis;
- ▪ Adult onset Still's disease, juvenile idiopathic arthritis, Systemic sclerosis, CREST syndrome where B cells and pathogen antibodies may also play a role; the Sweet syndrome; Takayasu arteritis, temporal arteritis/ giant cell arteritis;
- ▪ Ulcerative cholangitis, inflammatory bowel disease (IBD) including Crohn's disease and ulcerative colitis, primary sclerosing cholangitis.

Also of interest are diseases and disorders for which the mechanism remains poorly characterized but involves the activation and proliferation of T-cells, including:

- ▪ Alzheimer's disease, cardiovascular syndrome, type 2 diabetes, restenosis, chronic fatigue immune dysfunction syndrome (CFIDS).

- Autoimmune Lymphoproliferative disorders, including:
  - ▪ Autoimmune Lymphoproliferative Syndrome and X-linked lymphoproliferative disease.

Suitably the disease or disorder is selected from: inflammatory skin diseases such as psoriasis or lichen planus; acute and/or chronic GVHD such as steroid resistant acute GVHD; acute lymphoproliferative syndrome; systemic lupus erythematosus, lupus nephritis or cutaneous lupus; or transplantation. In addition, the disease or disorder may be selected from myasthenia gravis, multiple sclerosis, and scleroderma/systemic sclerosis.

The compounds of formula (I) may be used in the treatment of cancer.

Thus, in one embodiment there is provided a compound of formula (I), or a pharmaceutically acceptable salt and/or solvate thereof, for use in the treatment of cancer.

Suitably the cancer is a haematological cancer, such as Acute myeloid leukemia, Angioimmunoblastic T-cell lymphoma, B-cell acute lymphoblastic leukemia, Sweet Syndrome, T-cell Non-Hodgkins lymphoma (including natural killer/T-cell lymphoma, adult T-cell leukaemia/lymphoma, enteropathy type T-cell lymphoma, hepatosplenic T-cell lymphoma and cutaneous T-cell lymphoma), T-cell acute lymphoblastic leukemia, B-cell Non-Hodgkins lymphoma (including Burkitt lymphoma, diffuse large B-cell lymphoma, Follicular lymphoma, Mantle cell lymphoma, Marginal Zone lymphoma), Hairy Cell Leukemia, Hodgkin lymphoma, Lymphoblastic lymphoma, Lymphoplasmacytic lymphoma, Mucosa-associated lymphoid tissue lymphoma, Multiple myeloma, Myelodysplastic syndrome, Plasma cell myeloma, Primary mediastinal large B-cell lymphoma, chronic myeloproliferative disorders (such as chronic myeloid leukemia, primary myelofibrosis, essential thrombocytemia, polycytemia vera) or chronic lymphocytic leukemia.

Alternatively, the cancer is a non-haematological cancer, such as selected from the group consisting of bladder cancer, breast, melanoma, neuroblastoma, malignant pleural mesothelioma, and sarcoma.
In addition, compounds of formula (I) may be used in enhancing recovery from vascular injury or surgery and reducing morbidity and mortality associated with neointima and restenosis in a subject. For example, the compounds of formula (I) may be used in preventing, reducing, or inhibiting neointima formation. A medical device may be treated prior to insertion or implantation with an effective amount of a composition comprising a compound of formula (I) in order to prevent, reduce, or inhibit neointima formation following insertion or implantation of the device or graft into the subject. The device can be a device that is inserted into the subject transiently, or a device that is implanted permanently. In some embodiments, the device is a surgical device. Examples of medical devices include, but are not limited to, needles, cannulas, catheters, shunts, balloons, and implants such as stents and valves.
Suitably the subject is a mammal, in particular the subject is a human.

**Pharmaceutical Compositions**

For use in therapy the compounds of the invention are usually administered as a pharmaceutical composition. The invention also provides a pharmaceutical composition comprising a compound of formula (I), or a pharmaceutically acceptable salt and/or solvate (e.g. salt) thereof, and a pharmaceutically acceptable carrier or excipient.
In one embodiment, there is provided a pharmaceutical composition comprising a compound of formula (I), or a pharmaceutically acceptable salt and/or solvate (e.g. salt) thereof, for use in the treatment or prophylaxis of a disease or disorder as described herein.
The compounds of formula (I) or their pharmaceutically acceptable salts and/or solvates thereof may be administered by any convenient method, e.g. by oral, parenteral, buccal, sublingual, nasal, rectal or transdermal administration, and the pharmaceutical compositions adapted accordingly.
The compounds of formula (I) or their pharmaceutically acceptable salts and/or solvates thereof may be administered topically, for example to the eye, gut or skin. Thus, in an embodiment there is provided a pharmaceutical composition comprising a compound of the invention optionally in combination with one or more topically acceptable diluents or carriers.
A pharmaceutical composition of the invention may be delivered topically to the skin. Compositions suitable for transdermal administration include ointments, gels and patches. Such a pharmaceutical composition may also suitably be in the form of a cream, lotion, foam, powder, paste or tincture.
The pharmaceutical composition may suitably include vitamin D3 analogues (e.g. calcipotriol and maxacalcitol), steroids (e.g. fluticasone propionate, betamethasone valerate and clobetasol propionate), retinoids (e.g. tazarotene), coal tar and dithranol. Topical medicaments are often used in combination with each other (e.g. a vitamin D3 and a steroid) or with further agents such as salicylic acid.
A pharmaceutical composition of the invention may be delivered topically to the eye. Such a pharmaceutical composition may suitably be in the form of eye drops or an ointment.
A pharmaceutical composition of the invention may be delivered topically to the gut. Such a pharmaceutical composition may suitably be delivered orally, such as in the form of a tablet or a capsule, or rectally, such as in the form of a suppository.
Suitably, delayed release formulations are in the form of a capsule.
The compounds of formula (I) or their pharmaceutically acceptable salts and/or solvates thereof which are active when given orally can be formulated as liquids or solids, e.g. as syrups, suspensions, emulsions, tablets, capsules or lozenges.
A liquid formulation will generally consist of a suspension or solution of the active ingredient (such as a compound of formula (I) or a pharmaceutically acceptable salt and/or solvate (e.g. salt) thereof) in a suitable liquid carrier(s) e.g. an aqueous solvent such as water, ethanol or glycerine, or a non-aqueous solvent, such as polyethylene glycol or an oil. The formulation may also contain a suspending agent, preservative, flavouring and/or colouring agent.
A composition in the form of a tablet can be prepared using any suitable pharmaceutical carrier(s) routinely used for preparing solid formulations, such as magnesium stearate, starch, lactose, sucrose and cellulose.
A composition in the form of a capsule can be prepared using routine encapsulation procedures, e.g. pellets containing the active ingredient (such as a compound of formula (I) or a pharmaceutically acceptable salt and/or solvate (e.g. salt) thereof) can be prepared using standard carriers and then filled into a hard gelatin capsule; alternatively a dispersion or suspension can be prepared using any suitable pharmaceutical carrier(s), e.g. aqueous gums, celluloses, silicates or oils and the dispersion or suspension then filled into a soft gelatin capsule.
Typical parenteral compositions consist of a solution or suspension of the active ingredient (such as a compound of formula (I) or a pharmaceutically acceptable salt and/or solvate (e.g. salt) thereof) in a sterile aqueous carrier or parenterally acceptable oil, e.g. polyethylene glycol, polyvinyl pyrrolidone, lecithin, arachis oil or sesame oil. Alternatively, the solution can be lyophilised and then reconstituted with a suitable solvent just prior to administration.

Compositions for nasal administration may conveniently be formulated as aerosols, drops, gels and powders. Aerosol formulations typically comprise a solution or fine suspension of the active ingredient in a pharmaceutically acceptable aqueous or non-aqueous solvent and are usually presented in single or multidose quantities in sterile form in a sealed container which can take the form of a cartridge or refill for use with an atomising device. Alternatively the sealed container may be a disposable dispensing device such as a single dose nasal inhaler or an aerosol dispenser fitted with a metering valve. Where the dosage form comprises an aerosol dispenser, it will contain a propellant which can be a compressed gas e.g. air, or an organic propellant such as a fluoro-chloro-hydrocarbon or hydrofluorocarbon. Aerosol dosage forms can also take the form of pump-atomisers.

Compositions suitable for buccal or sublingual administration include tablets, lozenges and pastilles where the active ingredient is formulated with a carrier such as sugar and acacia, tragacanth, or gelatin and glycerin.

Compositions for rectal administration are conveniently in the form of suppositories containing a conventional suppository base such as cocoa butter.

Suitably, the composition is in unit dose form such as a tablet, capsule or ampoule.

The composition may for example contain from 0.1% to 100% by weight, for example from 10 to 60% by weight, of the active material, depending on the method of administration. The composition may contain from 0% to 99% by weight, for example 40% to 90% by weight, of the carrier, depending on the method of administration. The composition may contain from 0.05 mg to 2000 mg, for example from 1.0 mg to 500 mg, of the active material, depending on the method of administration. The composition may contain from 50 mg to 1000 mg, for example from 100 mg to 400 mg of the carrier, depending on the method of administration. The dose of the compound used in the treatment or prophylaxis of the aforementioned disorders will vary in the usual way with the seriousness of the disorders, the weight of the sufferer, and other similar factors. However, as a general guide suitable unit doses may be 0.05 mg to 1000 mg, more suitably 1.0 mg to 500 mg, and such unit doses may be administered more than once a day, for example two or three a day. Such therapy may extend for a number of weeks or months.

The invention provides, in a further aspect, a combination comprising a compound of formula (I) or a pharmaceutically acceptable, salt and/or solvate thereof together with a further pharmaceutically acceptable active ingredient or ingredients.

The invention provides a compound of formula (I), for use in combination with a further pharmaceutically acceptable active ingredient or ingredients.

When the compounds are used in combination with other therapeutic agents, the compounds may be administered separately, sequentially or simultaneously by any convenient route.

Optimal combinations may depend on the disease or disorder. Possible combinations include those with one or more active agents selected from the list consisting of: 5-aminosalicylic acid, or a prodrug thereof (such as sulfasalazine, olsalazine or bisalazide); corticosteroids (e.g. prednisolone, methylprednisolone, or budesonide); immunosuppressants (e.g. cyclosporin, tacrolimus, sirolimus, methotrexate, azathioprine mycophenolate mofetil, leflunomide, cyclophosphamide, 6-mercaptopurine or anti-lymphocyte (or thymocyte) globulins); anti-TNF-alpha antibodies (e.g., infliximab, adalimumab, certolizumab pegol or golimumab); anti-IL12/IL23 antibodies (e.g., ustekinumab); anti-IL6 or anti-IL6R antibodies, anti-IL17 antibodies or small molecule IL12/IL23 inhibitors (e.g., apilimod); Anti-alpha-4-beta-7 antibodies (e.g., vedolizumab); MAdCAM-1 blockers (e.g., PF-00547659); antibodies against the cell adhesion molecule alpha-4-integrin (e.g., natalizumab); antibodies against the IL2 receptor alpha subunit (e.g., daclizumab or basiliximab); JAK inhibitors including JAK1 and JAK3 inhibitors (e.g., tofacitinib, baricitinib, R348); Syk inhibitors and prodrugs thereof (e.g., fostamatinib and R-406); Phosphodiesterase-4 inhibitors (e.g., tetomilast); HMPL-004; probiotics; Dersalazine; semapimod/CPSI-2364; and protein kinase C inhibitors (e.g. AEB-071).

For cancer, the further pharmaceutically acceptable active ingredient may be selected from anti-mitotic agents such as vinblastine, paclitaxel and docetaxel; alkylating agents, for example cisplatin, carboplatin, dacarbazine and cyclophosphamide; antimetabolites, for example 5-fluorouracil, cytosine arabinoside and hydroxyurea; intercalating agents for example adriamycin and bleomycin; topoisomerase inhibitors for example etoposide, topotecan and irinotecan; thymidylate synthase inhibitors for example raltitrexed; PI3 kinase inhibitors for example idelalisib; mTor inhibitors for example everolimus and temsirolimus; proteasome inhibitors for example bortezomib; histone deacetylase inhibitors for example panobinostat or vorinostat; and hedgehog pathway blockers such as vismodegib.

The further pharmaceutically acceptable active ingredient may be selected from tyrosine kinase inhibitors such as, for example, axitinib, dasatinib, erlotinib, imatinib, nilotinib, pazopanib and sunitinib.

Anticancer antibodies may be included in a combination therapy and may be selected from the group consisting of olaratumab, daratumumab, necitumumab, dinutuximab, traztuzumab emtansine, pertuzumab, obinutuzumab, brentuximab, ofatumumab, panitumumab, catumaxomab, bevacizumab, cetuximab, tositumomab, traztuzumab, gentuzumab ozogamycin and rituximab.

Compounds or pharmaceutical compositions of the invention may also be used in combination with radiotherapy.

Some of the combinations referred to above may conveniently be presented for use in the form of a pharmaceutical formulation and thus pharmaceutical formulations comprising a combination as defined above together with a

pharmaceutically acceptable carrier or excipient comprise a further aspect of the invention. The individual components of such combinations may be administered either sequentially or simultaneously in separate or combined pharmaceutical formulations. The individual components of combinations may also be administered separately, through the same or different routes.

When a compound of formula (I) is used in combination with a second therapeutic agent active against the same disease state the dose of each compound may differ from that when the compound is used alone. Appropriate doses will be readily appreciated by those skilled in the art.

**Medical Devices**

In an embodiment, compounds of the invention or pharmaceutical compositions comprising said compounds may be formulated to permit incorporation into the medical device, thus providing application of the compound or composition directly to the site to prevent or treat conditions disclosed herein.

In an embodiment, the compounds of the invention or pharmaceutical composition thereof is formulated by including it within a coating onto the medical device. There are various coatings that can be utilized such as, for example, polymer coatings that can release the compound over a prescribed time period. The compound, or a pharmaceutical composition thereof, can be embedded directly within the medical device. In some embodiments, the compound is coated onto or within the device in a delivery vehicle such as a microparticle or liposome that facilitates its release and delivery. In some embodiments, the compound or pharmaceutical composition is miscible in the coating.

In some embodiments, the medical device is a vascular implant such as a stent. Stents are utilized in medicine to prevent or eliminate vascular restrictions. The implants may be inserted into a restricted vessel whereby the restricted vessel is widened. Excessive growth of the adjacent cells following vascular implantation results in a restriction of the vessel particularly at the ends of the implants which results in reduced effectiveness of the implants. If a vascular implant is inserted into a human artery for the elimination of for example an arteriosclerotic stenosis, intima hyperplasia can occur within a year at the ends of the vascular implant and results in renewed stenosis ("restenosis").

Accordingly, in some embodiments, the stents are coated or loaded with a composition including a compound of the invention or pharmaceutical composition thereof and optionally a targeting signal, a delivery vehicle, or a combination thereof. Many stents are commercially available or otherwise know in the art.

In some embodiments, the stent is a drug-eluting stent. Various drug eluting stents that simultaneously deliver a therapeutic substance to the treatment site while providing artificial radial support to the wall tissue are known in the art. Endoluminal devices including stents are sometimes coated on their outer surfaces with a substance such as a drug releasing agent, growth factor, or the like. Stents have also been developed having a hollow tubular structure with holes or ports cut through the sidewall to allow drug elution from a central lumen. Although the hollow nature of the stent allows the central lumen to be loaded with a drug solution that is delivered via the ports or holes in the sidewall of the stent, the hollow tubular structure may not have suitable mechanical strength to provide adequate scaffolding in the vessel.

In some embodiments, the devices are also coated or impregnated with a compound of the invention, or pharmaceutical composition thereof and one or more additional therapeutic agents, including, but not limited to, antiplatelet agents, anticoagulant agents, anti-inflammatory agents, antimicrobial agents, antimetabolic agents, additional anti-neointima agents, additional antiproliferative agents, immunomodulators, antiproliferative agents, agents that affect migration and extracellular matrix production, agents that affect platelet deposition or formation of thrombis, and agents that promote vascular healing and re-endothelialization, such as those and others described in Sousa *et al.* (2003) and Salu *et al.* (2004).

Examples of antithrombin agents include, but are not limited to, Heparin (including low molecular heparin), R-Hirudin, Hirulog, Argatroban, Efegatran, Tick anticoagulant peptide, and Ppack.

Examples of antiproliferative agents include, but are not limited to, Paclitaxel (Taxol), QP-2 Vincristin, Methotrexat, Angiopeptin, Mitomycin, BCP 678, Antisense c-myc, ABT 578, Actinomycin-D, RestenASE, 1 -Chlor- deoxyadenosin, PCNA Ribozym, and Celecoxib.

Examples of anti-restenosis agents include, but are not limited to, immunomodulators such as Sirolimus (Rapamycin), Tacrolimus, Biorest, Mizoribin, Cyclosporin, Interferon-γ lb, Leflunomid, Tranilast, Corticosteroide, Mycophenolic acid and Biphosphonate.

Examples of anti-migratory agents and extracellular matrix modulators include, but are not limited to Halofuginone, Propyl-hydroxylase-Inhibitors, C- Proteinase-Inhibitors, MMP-Inhibitors, Batimastat, Probucol.

Examples of antiplatelet agents include, but are not limited to, heparin.

Examples of wound healing agents and endothelialization promoters include vascular epithelial growth factor ("VEGF"), 17 -Estradiol, Tkase- Inhibitors, BCP 671, Statins, nitric oxide ("NO")-Donors, and endothelial progenitor cell ("EPC")-antibodies.

Besides coronary applications, drugs and active agents may be incorporated into the stent or stent coating for other indications. For example, in urological applications, antibiotic agents may be incorporated into the stent or stent

coating for the prevention of infection. In gastroenterological and urological applications, active agents may be incorporated into the stent or stent coating for the local treatment of carcinoma. It may also be advantageous to incorporate in or on the stent a contrast agent, radiopaque markers, or other additives to allow the stent to be imaged in vivo for tracking, positioning, and other purposes. Such additives could be added to the absorbable composition used to make the stent or stent coating, or absorbed into, melted onto, or sprayed onto the surface of part or all of the stent. Preferred additives for this purpose include silver, iodine and iodine labelled compounds, barium sulfate, gadolinium oxide, bismuth derivatives, zirconium dioxide, cadmium, tungsten, gold tantalum, bismuth, platinum, iridium, and rhodium. These additives may be, but are not limited to, micro- or nano-sized particles or nano particles. Radio-opacity may be determined by fluoroscopy or by x-ray analysis.

A compound of the invention and one or more additional agents, or pharmaceutical composition thereof, can be incorporated into the stent, either by loading the compound and one or more additional agents, or pharmaceutical composition thereof into the absorbable material prior to processing, and/or coating the surface of the stent with the agent(s). The rate of release of agent may be controlled by a number of methods including varying the following: the ratio of the absorbable material to the compound and one or more additional agents, or pharmaceutical composition, the molecular weight of the absorbable material, the composition of the compound and one or more additional agents, or pharmaceutical composition, the composition of the absorbable polymer, the coating thickness, the number of coating layers and their relative thicknesses, and/or the compound and one or more additional agents, or pharmaceutical composition concentration. Top coats of polymers and other materials, including absorbable polymers, may also be applied to active agent coatings to control the rate of release. For example, P4HB can be applied as a top coat on a metallic stent coated with P4HB including an active agent to retard the release of the active agent.

The invention is further exemplified by the following non-limiting examples.

## EXAMPLES

Abbreviations used herein are defined below. Any abbreviations not defined are intended to convey their generally accepted meaning.

### Abbreviations

| | |
|---|---|
| Ac | acetyl ($C(O)CH_3$) |
| AcOH | glacial acetic acid |
| $AlMe_3$ | trimethylaluminium |
| aq | aqueous |
| Ar | Aromatic ring |
| BEH | ethylene bridged hybrid |
| Bispin | Bis(pinacolato)diboron; 4,4,4',4',5,5,5',5'-Octamethyl-2,2'-bi-1,3,2-dioxaborolane |
| Bz | benzyl ($CH_2$-phenyl) |
| Boc | *tert*-butyloxycarbonyl protecting group |
| $Cs_2CO_3$ | Cesium carbonate |
| CSH | charged surface hybrid |
| d | doublet |
| DABAL-$Me_3$ | adduct of trimethylaluminum and 1,4-diazabicyclo[2.2.2]octane |
| DCM | dichloromethane |
| DIPEA | N,N-diisopropylethylamine |
| dioxane | 1,4-dioxane |
| DMAP | 4-dimethylaminopyridine |
| DME | dimethoxyethane |
| DMF | N,N-dimethylformamide |
| DMSO | dimethyl sulfoxide |
| DPPA | diphenylphosphoryl azide |
| dppf | 1,1'-bis(diphenylphosphino)ferrocene |
| ($ES^+$) | electrospray ionisation, positive mode |
| ($ES^-$) | electrospray ionisation, negative mode |
| ESI | electrospray ionisation |
| Et | ethyl |
| EtI | Ethyl iodide |

| | |
|---|---|
| EtOAc | ethyl acetate |
| EtOH | ethanol |
| g | grams |
| Hal | halogen |
| HATU | 1-[bis(dimethylamino)methylene]-1*H*-1,2,3-triazolo[4,5-*b*]pyridinium 3-oxid hexafluorophosphate |
| HPLC | high performance liquid chromatography |
| hr(s) | hour(s) |
| $IC_{50}$ | 50% inhibitory concentration |
| iPr | *iso*-propyl |
| $K_2CO_3$ | potassium carbonate |
| LCMS | liquid chromatography-mass spectrometry |
| LHMDS | lithium hexamethyldisilazide |
| LiOH | lithium hydroxide |
| $(M+H)^+$ | protonated molecular ion |
| $(M-H)^-$ | unprotonated molecular ion |
| M | molar concentration |
| mL | millilitre |
| mm | millimiter |
| mmol | millimole |
| Me | methyl |
| MeCN | acetonitrile |
| MeI | iodomethane |
| MeOH | methanol |
| MHz | megahertz |
| min(s) | minute(s) |
| MSD | mass selective detector |
| m/z | mass-to-charge ratio |
| $N_2$ | nitrogen gas |
| $NH_3$ | ammonia |
| $NH_4Cl$ | ammonium chloride |
| NaH | sodium hydride |
| $NaHCO_3$ | sodium bicarbonate |
| nm | nanometre |
| NMR | nuclear magnetic resonance (spectroscopy) |
| NSFI | *N*-fluorobenzenesulfonimide |
| P4HB | poly-4-hydroxybutyrate |
| PDA | photodiode array |
| Pd 170 | chloro(crotyl)(2-dicyclohexylphosphino-2',4',6'-triisopropybiphenyl)palladium(II) or XPhos Pd(crotyl)Cl |
| Pd 174 | allyl(2-di-*tert*-butylphosphino-2',4',6'-triisopropyl-1,1'-biphenyl)palladium(II) triflate or [*t*BuXPhosPd(allyl)]OTf |
| $[Pd(allyl)Cl_2]_2$ | bis(allyl)dichlorodipalladium |
| $PdCl_2$(dppf) | [1,1'-bis(diphenylphosphino)ferrocene]dichloropalladium(II) |
| $Pd(PPh_3)_4$ | tetrakis(triphenylphosphine)palladium(0) |
| PMB | 4-methoxybenzyl |
| prep HPLC | preparative high performance liquid chromatography |
| Ph | phenyl |
| pos/neg | positive/negative |
| q | quartet |
| RF/MS | RapidFire Mass Spectrometry |
| RT | room temperature |
| Rt | retention time |
| RP | reverse phase |
| s | singlet |
| $S_NAr$ | nucleophilic aromatic substitution |
| sat | saturated |
| SCX | solid supported cation exchange (resin) |

| | |
|---|---|
| Selectfluor | *N*-chloromethyl-*N'*-fluorotriethylenediammonium bis(tetrafluoroborate) |
| t | triplet |
| tBu | *tert*-butyl |
| T3P | 2,4,6-tripropyl-1,3,5,2,4,6-trioxatriphosphorinane-2,4,6-trioxide |
| TBME | *tert*-butyl methyl ether |
| TFA | Trifluoroacetic acid |
| [t-BuXPhos Pd(allyl)]OTf | allyl(2-di-*tert* -butylphosphino-2',4',6'-triisopropyl-1,1'-biphenyl)palladium(II) triflate |
| THF | tetrahydrofuran |
| TMP | 2,2,6,6-tetramethylpiperidinyl |
| TMSOK | potassium trimethylsilanolate |
| TTIP | titanium tetraisopropoxide |
| UPLC | ultra performance liquid chromatography |
| UV | ultraviolet |
| v/v | volume/volume |
| VWD | variable wave detector |
| wt | weight |
| um | micrometre |
| uL | microlitre |
| °C | degrees Celsius |

**General Procedures**

All starting materials and solvents were obtained either from commercial sources or prepared according to the literature. Unless otherwise stated all reactions were stirred. Organic solutions were routinely dried over anhydrous magnesium sulfate. Hydrogenations were performed on a Thales H-cube flow reactor under the conditions stated.

Column chromatography was performed on pre-packed silica (230-400 mesh, 40-63 um) cartridges using the amount indicated. SCX was purchased from Supelco and treated with 1M hydrochloric acid prior to use. Unless stated otherwise the reaction mixture to be purified was first diluted with MeOH and made acidic with a few drops of AcOH. This solution was loaded directly onto the SCX and washed with MeOH. The desired material was then eluted by washing with 0.7 M $NH_3$ in MeOH.

*Preparative Reverse Phase High Performance Liquid Chromatography*

**Prep HPLC**

***Acidic prep***

Waters X-Select CSH column C18, 5 um (19 x 50 mm), flow rate 28 mL $min^{-1}$ eluting with a $H_2O$-MeCN gradient containing 0.1 % v/v formic acid over 6.5 min using UV detection at 254 nm.

***Basic prep***

Waters X-Bridge Prep column C18, 5 um (19 x 50 mm), flow rate 28 mL $min^{-1}$ eluting with a 10 mM $NH_4HCO_3$-MeCN gradient over 6.5 min using UV detection at 254 nm.

**Analytical Methods**

*Reverse Phase HPLC Conditions for the LCMS Analytical Methods*

**HPLC acidic:** Acidic LCMS 4 minute (5-95%)

Analytical LCMS was carried out using a Waters X-Select CSH C18, 2.5 um, 4.6x30 mm column eluting with a gradient of 0.1 % Formic acid in MeCN in 0.1 % Formic acid in water. The gradient from 5-95 % 0.1 % Formic acid in MeCN occurs between 0.00-3.00 minutes at 2.5 mL/min with a flush from 3.01-3.5 minutes at 4.5 mL/min. A column re-equilibration to 5% MeCN is from 3.60-4.00 minutes at 2.5 mL/min. UV spectra of the eluted peaks were measured using an Agilent 1260 Infinity VWD at 254 nm. Mass spectra were measured using an Agilent 6120 MSD running with positive/negative switching.

**HPLC basic:** Basic LCMS 4 minute (5-95%)

Analytical LCMS was carried out using a Waters X-Select BEH C18, 2.5 um, 4.6x30 mm column eluting with a gradient of MeCN in aqueous 10mM ammonium bicarbonate. The gradient from 5-95% MeCN occurs between 0.00-3.00 minutes at 2.5mL/min with a flush from 3.01-3.5 minutes at 4.5 mL/min. A column re-equilibration to 5% MeCN is from 3.60-4.00 minutes at 2.5mL/min. UV spectra of the eluted peaks were measured using an Agilent 1260 Infinity VWD at 254nm. Mass spectra were measured using an Agilent 6120 MSD running with positive/negative switching.

*Reverse Phase HPLC Conditions for the UPLC Analytical Methods*

**UPLC acidic:** Acidic UPLC 3 minute

Analytical UPLC/MS was carried out using a Waters Acquity CSH C18, 1.7 um, 2.1x30 mm column eluting with a gradient of 0.1% Formic acid in MeCN in 0.1% Formic acid in water. The gradient is structured with a starting point of 5% MeCN held from 0.0-0.11 minutes. The gradient from 5-95% occurs between 0.11-2.15 minutes with a flush from 2.15-2.56 minutes. A column re-equilibration to 5% MeCN is from 2.56-2.83 minutes. UV spectra of the eluted peaks were measured using an Acquity PDA and mass spectra were recorded using an Acquity QDa detector with ESI pos/neg switching.

**Acidic UPLC 2** Acidic UPLC 1 minute

Analytical UPLC/MS was carried out using a Waters Acquity CSH C18, 1.7 um, 2.1x30 mm column eluting with a gradient of 0.1% Formic acid in MeCN in 0.1% Formic acid in water. The gradient is structured with a starting point of 5% MeCN held from 0.0-0.08 minutes. The gradient from 5-95% occurs between 0.08-0.70 minutes with a flush from 0.7-0.8 minutes. A column re-equilibration to 5% MeCN is from 0.8-0.9 minutes. UV spectra of the eluted peaks were measured using an Acquity PDA and mass spectra were recorded using an Acquity QDa detector with ESI pos/neg switching.

**UPLC basic:** Basic UPLC 3 minute

Analytical UPLC/MS was carried out using a Waters Acquity BEH C18, 1.7 um, 2.1x30 mm column eluting with a gradient of MeCN in aqueous 10 mM Ammonium Bicarbonate. The gradient is structured with a starting point of 5% MeCN held from 0.0-0.11 minutes. The gradient from 5-95% occurs between 0.11-2.15 minutes with a flush from 2.15-2.56 minutes. A column re-equilibration to 5% MeCN is from 2.56-2.83 minutes. UV spectra of the eluted peaks were measured using an Acquity PDA and mass spectra were recorded using an Acquity QDa detector with ESI pos/neg switching.

**Basic UPLC 2** Basic UPLC 1 minute

Analytical UPLC/MS was carried out using a WatersAcquity BEH C18, 1.7 um, 2.1x30 mm column eluting with a gradient of MeCN in aqueous 10 mM Ammonium Bicarbonate. The gradient is structured with a starting point of 5% MeCN held from 0.0-0.08 minutes. The gradient from 5-95% occurs between 0.08-0.70 minutes with a flush from 0.7-0.8 minutes. A column re-equilibration to 5% MeCN is from 0.8-0.9 minutes. UV spectra of the eluted peaks were measured using an Acquity PDA and mass spectra were recorded using an Acquity QDa detector with ESI pos/neg switching.

Column temperature is 40 °C in all runs. Injection volume is 3 uL and the flow rate is 0.77 mL/min. PDA scan from 210-400 nm on all runs.

*Normal Phase HPLC Conditions for the Chiral Analytical Methods*

**Chiral IC3 method:** Chiral HPLC (Diacel Chiralpak IC, 5 um, 4.6x250 mm, 1.0 mL/min, 25-70% EtOH (0.2% TFA) in iso-hexane (0.2% TFA)

**Chiral IC4 method:** Chiral HPLC (Diacel Chiralpak IC, 5 um, 4.6x250 mm, 1.0 mL/min, 40% EtOH (0.2% TFA) in 4:1 heptane/chloroform (0.2 % TFA).

**Chiral IC5 method:** Chiral HPLC (Diacel Chiralpak IC, 5 um, 4.6x250 mm, 1.0 mL/min, 20% EtOH (0.2% TFA) in iso-hexane (0.2% TFA).

*Reverse Phase HPLC Conditions for the Chiral Analytical Methods*

**Chiral IC6 method:** Chiral HPLC (Diacel Chiralpak IC, 5 um, 4.6x250 mm, 1.0 mL/min, 50% MeCN (0.1 % formic acid) in water (0.1 % formic acid).

**Chiral IC7 method:** Chiral HPLC (Diacel Chiralpak IC, 5 um, 4.6x250 mm, 1.0 mL/min, 5-95% MeCN (0.1 % formic acid) in water (0.1 % formic acid).

*$^1$H NMR Spectroscopy*
$^1$H NMR spectra were acquired on a Bruker Avance III spectrometer at400 MHz or Bruker Avance III HD spectrometer at 500 MHz using residual undeuterated solvent as reference and unless specified otherwise were run in DMSO-d6.

**Preparation of Intermediates**

Known synthetic intermediates were procured from commercial sources or were obtained using published literature procedures. Additional intermediates were prepared by the representative synthetic processes described herein.

Any one of Methods 1-10 (referred to later herein) or A-N may be used in the synthesis of the compounds of formula (I). For example, a scheme which is shown using a compound wherein X = N, Y = $CR_2$ and Z = $CR_3$ may also be used in the synthesis of compounds wherein X, Y and Z are as defined in the claims.

**Preparation of bi-ester intermediates**

1-(*tert*-Butyl) 3-methyl 2-(2-chloropyrimidin-4-yl)malonate **INTC1**

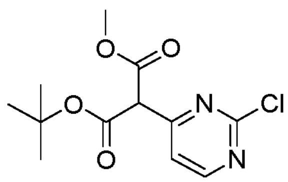

NaH (60 wt% in mineral oil, 5.10 g, 128 mmol) was added portionwise to an ice-cooled, stirred solution of *tert*-butyl methyl malonate (20.5 mL, 121 mmol) in THF (160 mL). The reaction was stirred at 0 °C for 20 mins then at RT for 60 mins until evolution of hydrogen ceased. 2,4-Dichloropyrimidine (10 g, 67.1 mmol) was then added and the resulting mixture was stirred at 70 °C for 3 hrs. The reaction was allowed to cool, partitioned between $NH_4Cl$ (sat. aq, 500 mL) and EtOAc (500 mL), the two phases were separated and the organic layer was passed through a phase separator. The crude product was purified by chromatography on silica gel (220 g column, 0-30% EtOAc/*iso*-hexane) to afford 1-*tert*-butyl 3-methyl 2-(2-chloropyrimidin-4-yl)malonate (13.1 g, 44.3 mmol, 66% yield) as a clear pale yellow oil; Rt 2.09 mins (HPLC acidic); m/z 230 (M+H-*t*Bu)$^+$ (ES$^+$) and 287 (M+H)$^+$ (ES$^+$); $^1$H NMR (400 MHz, DMSO-d6) δ 8.83 (d, J = 5.1 Hz, 1H), 7.65 (d, J = 5.1 Hz, 1H), 5.21 (s, 1H), 3.73 (s, 3H), 1.42 (s, 9H).

1-(*tert*-Butyl) 3-methyl 2-(2-chloro-5-fluoropyrimidin-4-yl)malonate **INTC2**

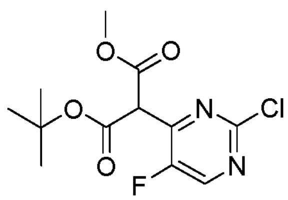

NaH (60 wt% in mineral oil, 0.575 g, 14.4 mmol) was added portionwise to a stirred solution of *tert*-butyl methyl malonate (2.23 mL, 13.2 mmol) in DMF (20 mL). The reaction was stirred at RT under $N_2$ for 10 mins until evolution of hydrogen ceased. The reaction was cooled to 0 °C and 2,4-dichloro-5-fluoropyrimidine (2.0 g, 12.0 mmol) was added. The resulting mixture was slowly stirred at RT for 18 hours. The reaction mixture was concentrated in *vacuo.* The crude product was purified by chromatography on silica gel (40 g column, 0-50% EtOAc/*iso*-hexane) to afford 1-*tert*-butyl 3-methyl 2-(2-chloro-5-fluoropyrimidin-4-yl)malonate (1.96 g, 5.47 mmol, 46% yield) as a clear colourless oil; Rt 1.42 mins (HPLC acidic); m/z 305 $(M+H)^+$ $(ES^+)$; $^1H$ NMR (400 MHz, DMSO-d6) δ 9.08 - 8.90 (m, 1H), 5.47 - 5.38 (m, 1H), 3.75 (s, 3H), 1.43 (s, 9H).

1-(*tert*-Butyl) 3-methyl 2-(2-chloro-5-methylpyrimidin-4-yl)malonate **INTC3**

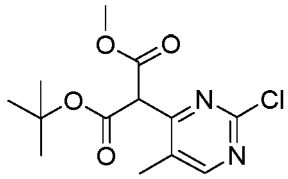

NaH (60 wt% dispersion in mineral oil, 0.466 g, 11.7 mmol) was added portionwise to an ice-cooled, stirred solution of *tert*-butyl methyl malonate (1.97 mL, 11.7 mmol) in THF (10 mL). The reaction was stirred at RT for 10 mins. 2,4-Dichloro-5-methylpyrimidine (1.0 g, 6.13 mmol) was then added and the resulting mixture was stirred at 70 °C for 2 hrs. The reaction was allowed to cool, partitioned between saturated $NH_4Cl$ (aq, 10 mL) and EtOAc (10 mL), the two phases were separated and the organic layer was passed through a phase separator. The crude product was purified by chromatography on silica gel (12 g column, 0-30% EtOAc/*iso*-hexane) to afford 1-*tert*-butyl 3-methyl 2-(2-chloro-5-methylpyrimidin-4-yl)malonate (1.40 g, 4.41 mmol, 72% yield) as a colourless oil; Rt 1.39 mins (HPLC acidic); m/z 201 $(M-Boc+H)^+$ $(ES^+)$; $^1H$ NMR (500 MHz, DMSO-d6) δ 8.66 (d, J = 0.8 Hz, 1H), 5.38 (s, 1H), 3.74 (s, 3H), 2.19 (d, J = 0.7 Hz, 3H), 1.44 (s, 9H).

1-*tert*-Butyl 3-methyl 2-(2-chloro-6-methylpyrimidin-4-yl)malonate **INTC54**

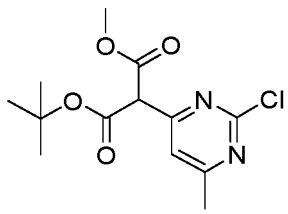

Prepared as for **INTC1** using commercial 2,4-dichloro-6-methylpyrimidine and *tert*-butyl methyl malonate to afford 1-*tert*-butyl 3-methyl 2-(2-chloro-6-methylpyrimidin-4-yl)malonate (61% yield) as a clear oil. Rt 1.39 (UPLC basic); *m*/*z* 301 $(^{35}Cl\ M+H)^+$ $(ES^+)$; $^1H$ NMR (500 MHz, DMSO-d6) δ 7.48 (s, 1H), 5.11 (s, 1H), 3.70 (s, 3H), 2.52 (s, 3H), 1.40 (s, 9H).

1-*tert*-Butyl 3-methyl 2-(2-chloro-6-(trifluoromethyl)pyrimidin-4-yl)malonate **INTC55**

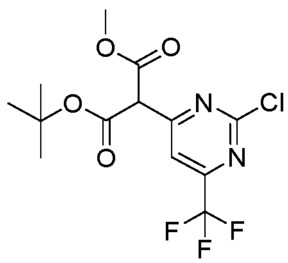

Prepared as for **INTC1** using commercial 2,4-dichloro-6-(trifluoromethyl)pyrimidine and *tert*-butyl methyl malonate to afford 1-*tert*-butyl 3-methyl 2-(2-chloro-6-(trifluoromethyl)pyrimidin-4-yl)malonate (67% yield) as a clear oil. Rt 1.34 (UPLC basic); *m/z* none observed; $^1$H NMR (500 MHz, DMSO-d6) δ 8.21 (s, 1H), 5.39 (s, 1H), 3.74 (s, 3H), 1.42 (s, 9H).

Dimethyl 2-(2-chloropyrimidin-4-yl)-2-methoxymalonate **INTC56**

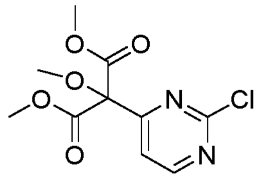

Prepared as for **INTC1** using 2,4-dichloropyrimidine and dimethyl 2-methoxymalonate to afford dimethyl 2-(2-chloropyrimidin-4-yl)-2-methoxymalonate (79% yield) as a clear colourless oil. Rt 1.55 (HPLC acidic); *m/z* 275 ($^{35}$Cl M+H)$^+$ (ES$^+$); $^1$H NMR not recorded. Material used directly in the next step with no further characterisation.

Dimethyl 2-(2-chloropyrimidin-4-yl)-2-isopropylmalonate **INTC57**

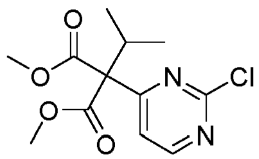

Prepared as for **INTC1** using 2,4-dichloropyrimidine and dimethyl 2-isopropylmalonate to afford dimethyl 2-(2-chloropyrimidin-4-yl)-2-isopropylmalonate (98% yield) as a red gum. Rt 0.64 (UPLC acidic 2); *m/z* 286 ($^{35}$Cl M+H)$^+$ (ES$^+$); $^1$H NMR not recorded. Material used directly in the next step with no further characterisation.

**Decarboxylation of chloro-pyrimidines**

Methyl 2-(2-chloropyrimidin-4-yl)acetate **INTC4**

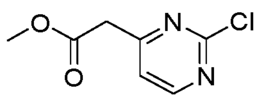

TFA (55.3 mL, 717 mmol) was added dropwise to an ice-cooled, stirred solution of 1-*tert*-butyl 3-methyl 2-(2-chloropyrimidin-4-yl)malonate **INTC1** (12.1 g, 42.2 mmol) in DCM (50 mL). The reaction was stirred at 25°C for 1 hr and then concentrated *in vacuo.* The residue was dissolved in EtOAc (200 mL), and basified with NaHCOs (200 mL), the organic layer was isolated and passed through a phase separator, the solvent was removed *in vacuo.* The crude product

was purified by chromatography on silica gel (220 g cartridge, 0-50% EtOAc/*iso*-hexane) to afford methyl 2-(2-chloropyrimidin-4-yl)acetate (7.12 g, 37.8 mmol, 90% yield) as a pale yellow oil. Rt 1.16 mins (HPLC acidic); m/z 187 $(M+H)^+$ $(ES^+)$; 1H NMR (500 MHz, DMSO-d6) δ 8.76 (d, J = 5.0 Hz, 1H), 7.60 (d, J = 5.0 Hz, 1H), 3.96 (s, 2H), 3.66 (s, 3H).

**Method A: Decarboxylation of chloro-heterocycles such as chloro-pyrimidines**

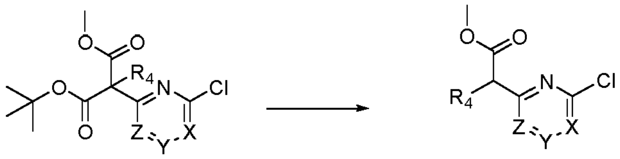

X = CH, N
Y = $CR_2$, N
Z = $CR_3$, N

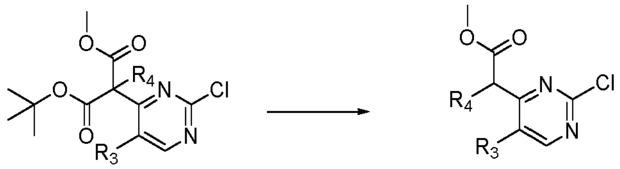

TFA (10 eq) was added dropwise to an ice-cooled, stirred solution of malonate derivative (1 eq) in DCM (15 volumes). The reaction vessel was stirred at RT for 18 hrs and then concentrated. The crude product was purified by normal phase chromatography.

**Table 1:** The following intermediates were made according to Method A.

| INTC | Name/Structure (All examples containing chiral centres are racemates unless stated) | Synthesis Method, [LCMS Method], m/z $(M+H)^+$, (Rt/min) | $^1$H NMR Chemical Shift Data (DMSO-d6 unless stated) |
|---|---|---|---|
| **INTC5** | methyl 2-(2-chloro-5-fluoropyrimidin-4-yl)acetate 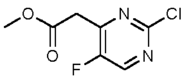 | Method A using INTC2, [HPLC acidic], 205 (0.85). | 8.91 (d, J = 1.4 Hz, 1H), 4.04 (d, J = 1.9 Hz, 2H), 3.68 (s, 3H). |
| **INTC6** | methyl 2-(2-chloro-5-methylpyrimidin-4-yl)acetate 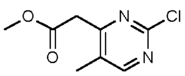 | Method A using INTC3, [HPLC acidic], 201 (0.82). | 8.60 (s, 1H), 3.96 (s, 2H), 3.66 (s, 3H), 2.24 (s, 3H). |
| **INTC58** | methyl 2-(2-chloro-6-methylpyrimidin-4-yl)acetate 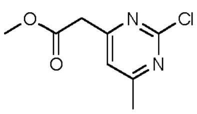 | Method A using INTC54, [UPLC basic], no m/z (0.78). | 7.45 (s, 1H), 3.88 (s, 2H), 3.65 (s, 3H), 2.47 (s, 3H). |
| **INTC59** | methyl 2-(2-chloro-6-(trifluoromethyl)pyrimidin-4-yl) acetate 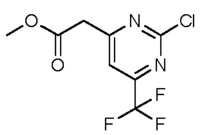 | Method A using INTC55, [UPLC basic]], no m/z (1.22). | 8.19 (s, 1H), 4.12 (s, 2H), 3.67 (s, 3H). |

(continued)

| INTC | Name/Structure (All examples containing chiral centres are racemates unless stated) | Synthesis Method, [LCMS Method], m/z $(M+H)^+$, (Rt/min) | $^1$H NMR Chemical Shift Data (DMSO-d6 unless stated) |
|---|---|---|---|
| **INTC60** | methyl 2-(2-chloropyrimidin-4-yl)-4-methoxybutanoate 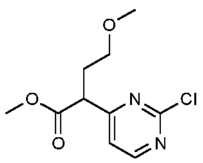 | Method A using INTC16, [UPLC acidic], M+Na $^{35}$Cl isotope 267 (0.94). | 8.76 (d, J = 5.0 Hz, 1H), 7.60 (d, J = 5.0 Hz, 1H), 3.98 - 3.94 (m, 1H), 3.63 (s, 3H), 3.37 - 3.20 (m, 2H), 3.16 (s, 3H), 2.31 - 2.21 (m, 1H), 2.14 - 2.03 (m, 1H). |
| **INTC61** | methyl 2-(2-chloropyrimidin-4-yl) butanoate 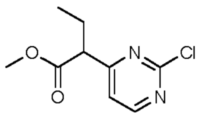 | Method A using INTC15, [HPLC acidic], M+H $^{35}$Cl isotope 215 (1.68). | 8.76 (d, J = 5.1 Hz, 1H), 7.60 (d, J = 5.1 Hz, 1H), 3.87 (t, J = 7.5 Hz, 1H), 3.63 (s, 3H), 2.08 - 1.98 (m, 1H), 1.93 - 1.83 (m, 1H), 0.83 (t, J = 7.4 Hz, 3H). |

**Alkylation**

Methyl 2-(2-chloropyrimidin-4-yl)-2-methylpropanoate **INTC7**

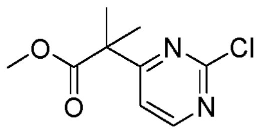

MeI (0.24 mL, 3.89 mmol) was added to a stirred suspension of methyl 2-(2-chloropyrimidin-4-yl)acetate **INTC4** (0.29 g, 1.55 mmol) and $K_2CO_3$ (0.644 g, 4.66 mmol) in acetone (5 mL). The reaction vessel was sealed and stirred at 60 °C for 18 hrs. The reaction mixture was concentrated *in vacuo,* water (40 mL) was added and extracted with DCM (2 x 40 mL). The organic phase was dried (phase separator) and concentrated *in vacuo.* The crude product was purified by chromatography on silica gel (24 g column, 0-50% EtOAc/iso-hexane) to afford methyl 2-(2-chloropyrimidin-4-yl)-2-methylpropanoate (0.25 g, 1.11 mmol, 71% yield) as a clear, pale yellow liquid; Rt 1.70 mins (HPLC acidic); m/z 215 $(M+H)^+$ $(ES^+)$; $^1$H NMR (400 MHz, DMSO-d6) δ 8.78 (d, J = 5.2 Hz, 1H), 7.66 (d, J = 5.2 Hz, 1H), 3.63 (s, 3H), 1.53 (s, 6H).

Methyl 2-(2-chloropyrimidin-4-yl)propanoate **INTC8**

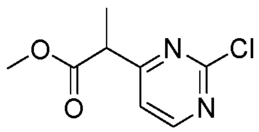

MeI (14.1 mL, 225 mmol) was added to a stirred suspension of methyl 2-(2-chloropyrimidin-4-yl)acetate **INTC4** (10.37 g, 45.0 mmol) and $K_2CO_3$ (31.1 g, 225 mmol) in acetone (150 mL). The reaction mixture was stirred at 60 °C for 40 hrs under $N_2$. The reaction mixture was concentrated *in vacuo,* the resulting mixture diluted in EtOAc and filtered. The inorganic phases were washed with EtOAc and the filtrate concentrated *in vacuo.* The crude product was purified by chromatography on silica gel (220 g column, 0-30% EtOAc/*iso*-hexane) to afford methyl 2-(2-chloropyrimidin-4-yl)propanoate (1.27 g, 5.00 mmol, 11% yield) as the by-product; Rt 0.89 mins (UPLC acidic); m/z 201 $(M+H)^+$ $(ES^+)$. No $^1$H-NMR data was recorded.

**Method B: Alkylation**

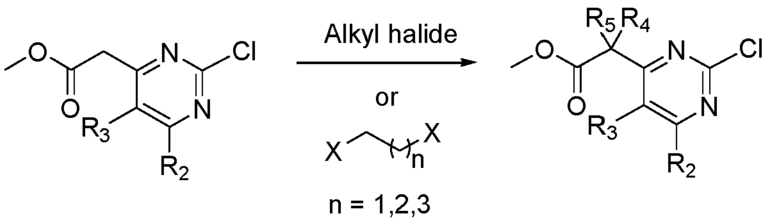


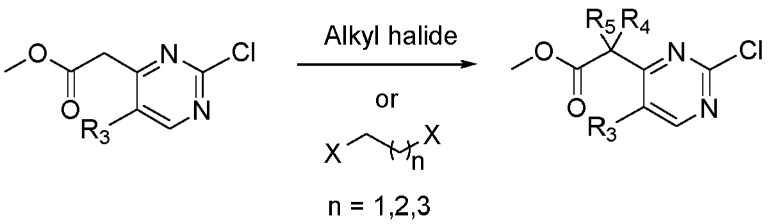


Base (2.5 - 5 eq) was added to an ice-cooled, stirred mixture of methyl 2-(2-chloropyrimidin-4-yl)acetate (1 eq) in appropriate polar aprotic solvent such as DMF or acetone (10 volumes). After 20 min, alkyl halide (1-5 eq) was added. The reaction vessel was stirred at 0 °C for 30 mins then at RT for 2 hrs. The reaction was quenched with $NH_4Cl$ (aq) or 1M HCl (aq), stirred for 20 mins then extracted with EtOAc. The organic phases were dried (phase separator) and concentrated. The crude product was purified by normal phase chromatography.

**Table 2:** The following intermediates were made according to Method B.

| INTC | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) | Base, RX, solvent |
|---|---|---|---|---|
| **INTC9** | methyl 2-(2-chloro-5-fluoropyrimidin-4-yl)-2-methylpropanoate 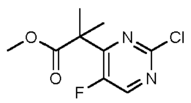 | Method B using INTC5, [UPLC acidic], 233 (1.31). | 8.88 (d, J = 2.5 Hz, 1H), 3.66 (s, 3H), 1.52 (s, 6H). | $K_2CO_3$,MeI, acetone |
| **INTC10** | methyl 2-(2-chloro-5-methylpyrimidin-4-yl) propanoate 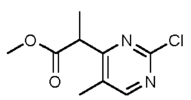 | Method B using INTC6, [UPLC acidic], 215 (1.03). | 8.60 (s, 1H), 4.25 (q, J = 7.0 Hz, 1H), 3.61 (s, 3H), 2.29 (d, J = 0.8 Hz, 3H), 1.40 (d, J = 7.0 Hz, 3H). | $K_2CO_3$,MeI, acetone |
| **INTC11** | methyl 2-(2-chloro-5-methylpyrimidin-4-yl)-2-methylpropanoate 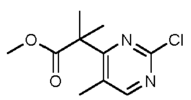 | Method B using INTC6, [UPLC acidic], 228/231 (1.26). | 8.57 (d, J = 0.8 Hz, 1H), 3.66 (s, 3H), 2.13 (d, J = 0.8 Hz, 3H), 1.50 (s, 6H). | $K_2CO_3$,MeI, acetone |

(continued)

| INTC | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) | Base, RX, solvent |
|---|---|---|---|---|
| **INTC12** | methyl 2-(2-chloropyrimidin-4-yl)-2-ethylbutanoate 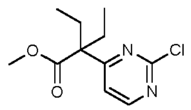 | Method B using INTC4, [UPLC acidic], 243 (1.38). | 8.83 - 8.67 (m, 1H), 7.65 - 7.52 (m, 1H), 3.63 (s, 3H), 2.07 - 1.99 (m, 4H), 0.73 - 0.59 (m, 6H). | NaOH, EtBr, DMF |
| **INTC13** | methyl 1-(2-chloropyrimidin-4-yl)cyclopropane-1-carboxylate 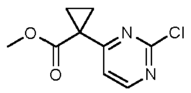 | Method B using INTC4, [UPLC acidic], 213, (1.05). | 8.78 - 8.62 (m, 1H), 7.94 - 7.81 (m, 1H), 3.68 (s, 3H), 1.70 - 1.56 (m, 4H). | NaOH, $BrCH_2CH_2Br$ DMF |
| **INTC14** | methyl 1-(2-chloropyrimidin-4-yl)cyclopentane-1-carboxylate 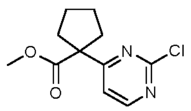 | Method B using INTC4, [UPLC acidic], 241 (1.32). | 8.79 - 8.66 (m, 1H), 7.65 - 7.55 (m, 1H), 3.62 (s, 3H), 2.41 - 2.25 (m, 2H), 2.21 - 2.06 (m, 2H), 1.81 - 1.57 (m, 4H). | NaOH, Br-(n-Bu)-Br DMF |
| **INTC15** | 1-(*tert*-butyl) 3-methyl 2-(2-chloropyrimidin-4-yl)-2-ethylmalonate 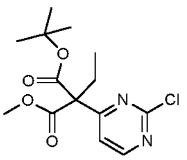 | Method B using INTC1, [UPLC acidic], 315 (1.58). | 8.83 (d, J = 5.3 Hz, 1H), 7.80 (d, J = 5.3 Hz, 1H), 3.73 (s, 3H), 2.29 - 2.14 (m, 2H), 1.40 (s, 9H), 0.82 (t, J = 7.4 Hz, 3H). | NaOH, EtBr, DMF |
| **INTC16** | 1-(*tert*-butyl) 3-methyl 2-(2-chloropyrimidin-4-yl)-2-(2-methoxyethyl)malonate 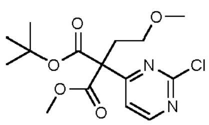 | Method B using INTC1, [UPLC acidic], 345 (1.48). | 8.83 (dd, J = 5.2, 1.0 Hz, 1H), 7.83 (d, J = 5.3 Hz, 1H), 3.72 (s, 3H), 3.31 - 3.24 (m, 2H), 3.11 (s, 3H), 2.47 - 2.40 (m, 2H), 1.39 (s, 9H). | NaOH, $BrCH_2CH_2OMe$, DMF |
| **INTC62** | methyl 2-(2-chloro-6-methylpyrimidin-4-yl)-2-methylpropanoate 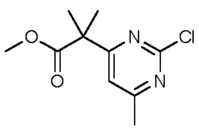 | Method B using INTC58 NO LCMS data | 7.53 (s, 1H), 3.62 (s, 3H), 2.50 (s, 3H), 1.51 (s, 6H). | $K_2CO_3$, MeI, acetone |

(continued)

| INTC | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) | Base, RX, solvent |
|---|---|---|---|---|
| **INTC63** | methyl 2-(2-chloro-6-(trifluoromethyl)pyrimidin-4-yl)-2-methylpropanoate 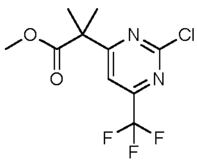 | Method B using INTC59 NO LCMS data | 8.17 (s, 1H), 3.64 (s, 3H), 1.59 (s, 6H). | $K_2CO_3$,MeI, acetone |
| **INTC64** | methyl 2-(2,6-dichloropyrimidin-4-yl)-2-methylpropanoate 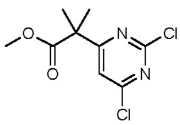 | Method B using commercial pyrimidine [UPLC acidic], $^{35}Cl$ isotope 249 (1.39). | 7.90 (s, 1H), 3.63 (s, 3H), 1.53 (s, 6H). | tBuOK, MeI, THF |

**$S_NAR$ on 2,6-dichloro pyrimidines**

Methyl 2-(2-chloro-6-methoxypyrimidin-4-yl)-2-methylpropanoate **INTC65**

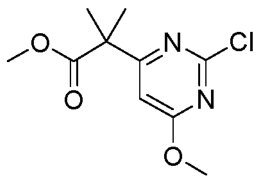

To a stirred solution of methyl 2-(2,6-dichloropyrimidin-4-yl)-2-methylpropanoate **INTC64** (0.77 g, 2.78 mmol) in MeOH (10 mL) under $N_2$ at 0 °C was added 5.4 M sodium methanolate (MeOH) (0.6 mL, 3.24 mmol). The mixture was stirred at 0 °C for 30 min then at RT for a further 30 min. The reaction was then concentrated *in vacuo.* The crude product was purified by chromatography on silica gel (40 g column, 0-50% EtOAc/*iso*-hexane) to afford methyl 2-(2-chloro-6-methoxypyrimidin-4-yl)-2-methylpropanoate (0.54 g, 1.72 mmol, 62% yield) as a white solid. Rt 1.35 min (UPLC, acidic); *m/z* 245 ($^{35}$Cl M+H)$^+$ (ES$^+$); 1H NMR (400 MHz, DMSO-d6) δ 6.99 (s, 1H), 3.96 (s, 3H), 3.61 (s, 3H), 1.48 (s, 6H).

**Heterocycle formation via alkylation**

Methyl 4-(2-chloropyrimidin-4-yl)tetrahydro-2*H*-pyran-4-carboxylate **INTC52**

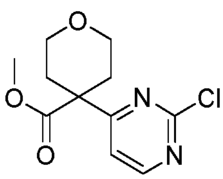

To a solution of methyl 2-(2-chloropyrimidin-4-yl)acetate **INTC4** (2.0 g, 10.7 mmol) in DMF (10 mL, 10.7 mmol) at 0 °C

was added NaOH (0.986 g, 24.6 mmol). The reaction mixture was stirred at 0 °C for 20 mins then 1-bromo-2-(2-bromoethoxy)ethane (1.8 mL, 12.9 mmol) was added. The reaction was stirred at RT for 23 hrs. The reaction mixture was acidified using 1M HCl (*aq*, 53.6 mL, 53.6 mmol) before extracting with DCM (70 mL). The phases were separated using a phase separator cartridge and the aqueous was extracted with further DCM (2 x 50 mL). The combined organics were concentrated *in vacuo*. The crude product was purified by chromatography on silica gel (80 g column, 0-50% EtOAc/*iso*-hexane) to afford methyl 4-(2-chloropyrimidin-4-yl)tetrahydro-2*H*-pyran-4-carboxylate (1.83 g, 5.57 mmol, 52% yield) as a yellow oil. Rt 1.56 min (HPLC, acidic); m/z 257 ($^{35}$Cl M+H)$^+$ (ES$^+$); $^1$H NMR (500 MHz, DMSO-d6) δ 8.80 (d, J = 5.3 Hz, 1H), 7.69 (d, J = 5.3 Hz, 1H), 3.72-3.67 (m, 2H), 3.66 (s, 3H), 3.55-3.50 (m, 2H), 2.33 - 2.22 (m, 2H), 2.16 - 2.06 (m, 2H).

**Heterocycle formation via enolate $S_N$AR**

1-*tert*-Butyl 4-methyl 4-(2-chloropyrimidin-4-yl)piperidine-1,4-dicarboxylate **INTC66**

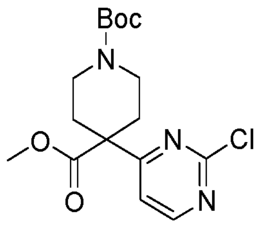

LiHMDS (1.61 mL, 1.61 mmol) was added in one portion to an ice-cooled, stirred solution of 1-*tert*-butyl 4-methyl piperidine-1,4-dicarboxylate (340 mg, 1.40 mmol) and 2,4-dichloropyrimidine (200 mg, 1.34 mmol) in THF (10 mL). The reaction mixture was allowed to warm up to RT and stirred for 2 hrs. The reaction was quenched by addition of $NaH_2PO_4$ (*aq*, 1M, 3 mL). The product was extracted with DCM (2 x 10 mL). The combined organic extracts were dried *via* a hydrophobic phase separator and concentrated *in vacuo.* The crude product was purified by chromatography on silica gel (24 g column, 0-50% EtOAc/*iso*-hexane) to afford 1-*tert*-butyl 4-methyl 4-(2-chloropyrimidin-4-yl)piperidine-1,4-dicarboxylate (315 mg, 0.66 mmol, 49% yield) as a colourless oil. Rt 2.29 min (HPLC, acidic); *m*/*z* 255 ($^{35}$Cl M-Boc+H)$^+$ (ES$^+$); $^1$H NMR (500 MHz, DMSO-d6) δ 8.79 (d, J = 5.3 Hz, 1H), 7.68 (d, J = 5.3 Hz, 1H), 3.69 - 3.59 (m, 5H), 3.13 (s, 2H), 2.26 - 2.22 (m, 2H), 2.06 - 2.00 (m, 2H), 1.40 (s, 9H).

1-*tert*-Butyl 3-methyl 3-(2-chloropyrimidin-4-yl)azetidine-1,3-dicarboxylate **INTC67**

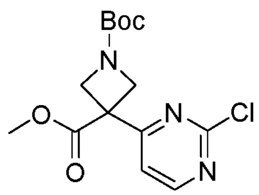

Prepared as for **INTC66** using 1-*tert*-butyl 3-methyl azetidine-1,3-dicarboxylate and 2,4-dichloropyrimidine in toluene to afford 1-*tert*-butyl 3-methyl 3-(2-chloropyrimidin-4-yl)azetidine-1,3-dicarboxylate (7% yield) as a pale yellow oil. Rt 2.12 min (HPLC, basic); *m*/*z* 272 ($^{35}$Cl M-*t*Bu+H)$^+$ (ES$^+$); $^1$H NMR (500 MHz, DMSO-d6) δ 8.83 (d, J = 5.2 Hz, 1H), 7.72 (d, J = 5.2 Hz, 1H), 4.39 - 4.35 (m, 2H), 4.34 - 4.28 (m, 2H), 3.71 (s, 3H), 1.39 (s, 9H).

**Hydrolysis of chloro-pyrimidines**

Lithium 2-(2-chloropyrimidin-4-yl)-4-methoxybutanoate **INTC68**

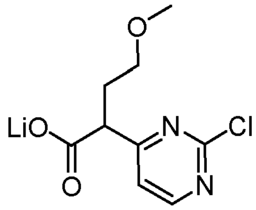

To a solution of methyl 2-(2-chloropyrimidin-4-yl)-4-methoxybutanoate **INTC60** (479 mg, 1.96 mmol) in THF (5 mL) and MeOH (2.5 mL) was added a solution of LiOH (56 mg, 2.35 mmol) in water (3 mL). The reaction mixture was stirred at RT for 72 hrs. The reaction mixture was concentrated *in vacuo* to give lithium 2-(2-chloropyrimidin-4-yl)-4-methoxybutanoate (441 mg, 1.49 mmol, 76% yield) as a colourless solid. Rt 1.34 min (HPLC acidic); *m/z* 231 (as free acid $^{35}Cl$ M+H)$^+$ (ES$^+$); $^1H$ NMR (500 MHz, DMSO-d6) δ 8.64 (d, J = 5.1 Hz, 1H), 7.48 (d, J = 5.1 Hz, 1H), 3.39 - 3.33 (m, 1H), 3.22 (s, 3H), 2.82 - 2.75 (m, 2H), 1.96 - 1.85 (m, 2H).

**Coupling (Sulfonamidation)**

1-(*tert*-Butyl) 3-methyl 2-(2-(cyclopropanesulfonamido)pyrimidin-4-yl)malonate **INTC17**

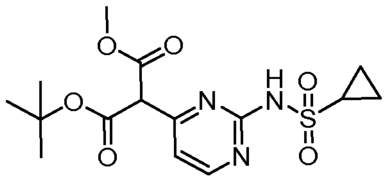

A 20 mL vial was charged with cyclopropanesulfonamide (0.254 g, 2.09 mmol), $Cs_2CO_3$ (1.14 g, 3.49 mmol), 1-*tert*-butyl 3-methyl 2-(2-chloropyrimidin-4-yl)malonate **INTC1** (0.50 g, 1.74 mmol) and dioxane (2 mL). The mixture was degassed ($N_2$, 5 mins). In a separate 20 mL vial, $[Pd(allyl)Cl]_2$ (16 mg, 0.044 mmol), tBuXPhos (74 mg, 0.174 mmol) and dioxane (1 mL) were stirred under $N_2$ for 5 mins then added to the first vial. The resulting reaction mixture was heated under $N_2$ at 60 °C for 2.5 hrs. The mixture was allowed to cool to RT, diluted with $H_2O$ (2 mL) and then carefully acidified with 1M HCl (aq, 5 mL) until pH 4. The residue was extracted with EtOAc (2 x 20 mL), the organic phase was filtered through a phase separator and the solvent was removed *in vacuo.* The yellow residue was triturated with TBME (10 mL), filtered and washed with TBME (10 mL) to give 1-*tert*-butyl 3-methyl 2-(2-(cyclopropanesulfonamido)pyrimidin-4-yl)malonate (0.394 g, 1.05 mmol, 60% yield) as a white solid.; Rt 1.87 mins (HPLC acidic); m/z 372 (M+H)$^+$ (ES$^+$); $^1H$ NMR (400 MHz, DMSO-d6) δ 12.57 (s, 1H), 11.61 (s, 1H), 7.28 - 7.21 (m, 1H), 6.10 (s, 1H), 3.67 (s, 3H), 2.75 - 2.65 (m, 1H), 1.44 (s, 9H), 1.18 - 0.83 (m, 4H). 8:2 mixture of tautomers.

**Method C: Formation of sulfonamides from aromatic halides**

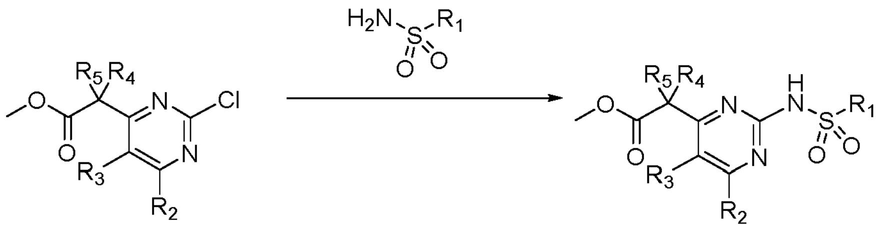

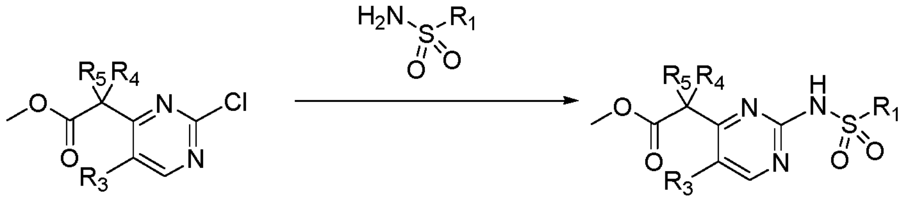

2-Chloropyrimidine intermediate (1 eq), sulfonamide (1.2 eq) and base (2 eq) were dissolved in dioxane (40 volumes). The mixture was degassed ($N_2$, 5 mins) then catalyst (5 mol%) was added. The resulting mixture was heated under nitrogen at 90 °C for 2 hrs. The mixture was filtered, washing with EtOAc or DCM and the resulting filtrate was concentrated. The crude product was purified by normal phase chromatography or trituration using a suitable solvent.

**Table 3:** The following intermediates were made according to Method C.

| INTC | Name/Structure (All examples containing chiral centres are racemates unless stated) | Synthesis Method, [LCMS Method], m/z $(M+H)^+$, (Rt/min) | $^1$H NMR Chemical Shift Data (DMSO-d6 unless stated) | Catalyst, Base, Solvent |
|---|---|---|---|---|
| **INTC18** | methyl 2-(2-(cyclopropanesulfonamido) pyrimidin-4-yl)propanoate 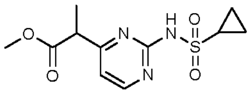 | Method C using INTC8, [UPLC acidic], 286 (0.86). | None recorded. | tBuXPhos Pd G3, $K_2CO_3$, dioxane |
| **INTC19** | methyl 2-methyl-2-(2-(methylsulfonamido) pyrimidin-4-yl)propanoate 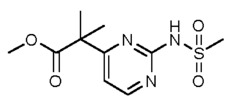 | Method C using INTC7, [HPLC acidic], 274 (1.35). | 11.30 (s, 1H), 8.59 (d, J = 5.3 Hz, 1H), 7.18 (d, J = 5.3 Hz, 1H), 3.62 (s, 3H), 3.35 (s, 3H), 1.50 (s, 6H). | [Pd(allyl) Cl]$_2$ tBuXPhos, $Cs_2CO_3$, dioxane |
| **INTC20** | methyl 2-(2-(ethylsulfonamido) pyrimidin-4-yl)-2-methylpropanoate 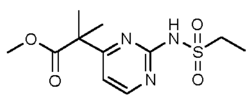 | Method C using INTC7, [UPLC acidic], 288 (0.92). | 11.20 (s, 1H), 8.58 (d, J = 5.3 Hz, 1H), 7.17 (d, J = 5.2 Hz, 1H), 3.62 (s, 3H), 3.53 (q, J = 7.4 Hz, 2H), 1.49 (s, 6H), 1.21 (t, J = 7.3 Hz, 3H). | Pd 174, $Cs_2CO_3$, dioxane |
| **INTC21** | methyl 2-(2-(cyclopropanesulfonamido) pyrimidin-4-yl)-2-methylpropanoate 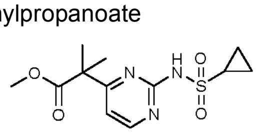 | Method C using INTC7, [HPLC acidic], 300 (1.55). | 11.28 (s, 1H), 8.55 (d, J = 5.3 Hz, 1H), 7.11 (d, J = 5.3 Hz, 1H), 3.61 (s, 3H), 3.22 - 3.11 (m, 1H), 1.49 (s, 6H), 1.14 - 0.93 (m, 4H). | Pd 174, $K_2CO_3$, dioxane |

(continued)

| INTC | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) | Catalyst, Base, Solvent |
|---|---|---|---|---|
| **INTC22** | methyl 2-(2-(cyclopropanesulfonamido)-5-fluoropyrimidin-4-yl)-2-methylpropanoate 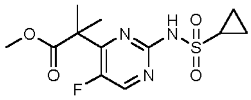 | Method C using INTC9, [UPLC acidic], 318 (1.12). | 11.38 (s, 1H), 8.64 (d, J = 2.8 Hz, 1H), 3.66 (s, 3H), 3.19 (tt, J = 7.9, 4.9 Hz, 1H), 1.52 (s, 6H), 1.14 - 1.03 (m, 4H). | $[Pd(allyl)Cl]_2$ tBuXPhos, $Cs_2CO_3$, dioxane |
| **INTC23** | methyl 2-(2-(cyclopropanesulfonamido)-5-methylpyrimidin-4-yl)-2-methylpropanoate 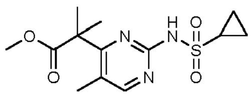 | Method C using INTC11, [UPLC acidic], 313 (1.07). | 11.07 (s, 1H), 8.36 (s, 1H), 3.66 (s, 3H), 3.26 - 3.19 (m, 1H), 2.05 (s, 3H), 1.50 (s, 6H), 1.13 - 1.00 (m, 4H). | $[Pd(allyl)Cl]_2$ tBuXPhos, $Cs_2CO_3$, dioxane |
| **INTC24** | methyl 2-(2-(cyclobutanesulfonamido) pyrimidin-4-yl)-2-methylpropanoate 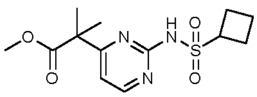 | Method C using INTC7, [HPLC acidic], 314 (1.74). | 11.11 (s, 1H), 8.56 (d, J = 5.3 Hz, 1H), 7.16 (d, J = 5.3 Hz, 1H), 4.55 (p, J = 8.4 Hz, 1H), 3.63 (s, 3H), 2.45 - 2.31 (m, 2H), 2.30 - 2.15 (m, 2H), 2.01 - 1.84 (m, 2H), 1.49 (s, 6H). | $[Pd(allyl)Cl]_2$ tBuXPhos, $Cs_2CO_3$, dioxane |
| **INTC25** | methyl 2-(2-(1,1-dimethylethylsulfonamido) pyrimidin-4-yl)-2-methylpropanoate 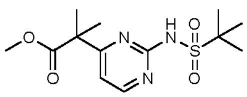 | Method C using INTC7, [UPLC acidic], 316 (1.09). | 10.73 (s, 1H), 8.55 (d, J = 5.3 Hz, 1H), 7.14 (d, J = 5.2 Hz, 1H), 3.59 (s, 3H), 1.48 (s, 6H), 1.37 (s, 9H). | Pd 174, $Cs_2CO_3$, dioxane |
| **INTC26** | methyl 2-methyl-2-(2-(1-methylcyclopropanesulfonamido) pyrimidin-4-yl)propanoate 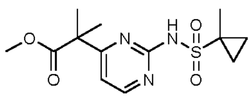 | Method C using INTC7, [HPLC acidic], 314 (1.71). | 11.04 (s, 1H), 8.57 (d, J = 5.2 Hz, 1H), 7.15 (d, J = 5.2 Hz, 1H), 3.60 (s, 3H), 1.55 - 1.45 (m, 8H), 1.43 (s, 3H), 0.89 - 0.83 (m, 2H). | Pd 174, $Cs_2CO_3$, dioxane |
| **INTC27** | methyl 2-(2-(cyclopropanesulfonamido) pyrimidin-4-yl)-2-ethylbutanoate 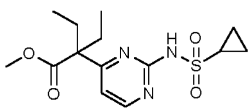 | Method C using INTC12, [UPLC acidic], 328 (1.22). | 11.24 (s, 1H), 8.66 - 8.43 (m, 1H), 7.17 - 7.01 (m, 1H), 3.60 (s, 3H), 3.23 - 3.06 (m, 1H), 2.11 - 1.84 (m, 4H), 1.15 - 0.96 (m, 4H), 0.79 - 0.57 (m, 6H). | Pd 174, $Cs_2CO_3$, dioxane |

(continued)

| INTC | Name/Structure (All examples containing chiral centres are racemates unless stated) | Synthesis Method, [LCMS Method], m/z $(M+H)^+$, (Rt/min) | $^1$H NMR Chemical Shift Data (DMSO-d6 unless stated) | Catalyst, Base, Solvent |
|---|---|---|---|---|
| **INTC28** | methyl 1-(2-(cyclopropanesulfonamido) pyrimidin-4-yl)cyclopropane-1-carboxylate 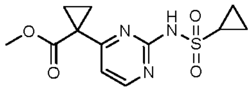 | Method C using INTC13, [UPLC acidic], 298 (0.93). | 11.19 (s, 1H), 8.57 - 8.43 (m, 1H), 7.52 - 7.32 (m, 1H), 3.67 (s, 3H), 3.20 - 3.08 (m, 1H), 1.68 - 1.52 (m, 4H), 1.15 - 0.98 (m, 4H). | Pd 174, $Cs_2CO_3$, dioxane |
| **INTC29** | methyl 1-(2-(cyclopropanesulfonamido) pyrimidin-4-yl)cyclopentane-1-carboxylate 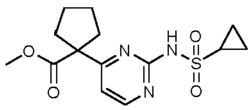 | Method C using INTC14, [UPLC acidic], 326 (1.17). | 11.23 (s, 1H), 8.59 - 8.45 (m, 1H), 7.17 - 7.05 (m, 1H), 3.61 (s, 3H), 3.25 - 3.12 (m, 1H), 2.40 - 2.24 (m, 2H), 2.21 - 2.08 (m, 2H), 1.73 - 1.59 (m, 4H), 1.18 - 0.96 (m, 4H). | Pd 174, $Cs_2CO_3$, dioxane |
| **INTC30** | 1-(*tert*-butyl) 3-methyl 2-(2-(cyclopropanesulfonamido) pyrimidin-4-yl)-2-ethylmalonate 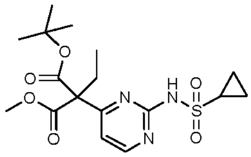 | Method C using INTC15, [UPLC acidic], 400 (1.40). | 11.30 (s, 1H), 8.62 (d, J = 5.3 Hz, 1H), 7.35 (d, J = 5.3 Hz, 1H), 3.71 (s, 3H), 3.21 - 3.10 (m, 1H), 2.30 - 2.10 (m, 2H), 1.41 (s, 9H), 1.18 - 0.97 (m, 4H), 0.83 (t, J = 7.4 Hz, 3H). | Pd 174, $Cs_2CO_3$, dioxane |
| **INTC31** | 1-(*tert*-butyl) 3-methyl 2-(2-(cyclopropanesulfonamido) pyrimidin-4-yl)-2-(2-methoxyethyl) malonate 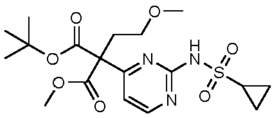 | Method C using INTC16, [UPLC acidic], 430 (1.31). | 11.31 (s, 1H), 8.63 (d, J = 5.3 Hz, 1H), 7.38 (d, J = 5.3 Hz, 1H), 3.70 (s, 3H), 3.32 - 3.24 (m, 2H), 3.20 - 3.14 (m, 1H), 3.13 (s, 3H), 2.49 - 2.32 (m, 2H), 1.39 (s, 9H), 1.15 - 0.98 (m, 4H). | Pd 174, $Cs_2CO_3$, dioxane |
| **INTC32** | 1-(*tert*-butyl) 3-methyl 2-(2-(cyclopropanesulfonamido)-5-fluoropyrimidin-4-yl)malonate 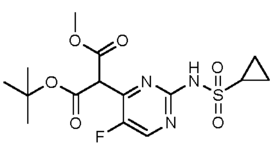 | Method C using INTC2, [UPLC acidic], 390 (1.27). | 11.47 (s, 1H), 8.81 - 8.72 (m, 1H), 5.31 - 5.20 (m, 1H), 3.75 (s, 3H), 3.20 - 3.12 (m, 1H), 1.43 (s, 9H), 1.17 - 0.99 (m, 4H). | Pd 174, $K_2CO_3$, dioxane |

(continued)

| INTC | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) | Catalyst, Base, Solvent |
|---|---|---|---|---|
| **INTC53** | methyl 4-(2-(cyclopropanesulfonamido) pyrimidin-4-yl)tetrahydro-2H-pyran-4-carboxylate 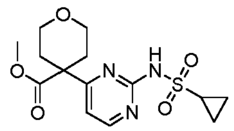 | Method C using INTC52 [UPLC, acidic], 342 (0.88). | 11.30 (s, 1H), 8.61 (d, J = 5.3 Hz, 1H), 7.20 (d, J = 5.3 Hz, 1H), 3.79 - 3.71 (m, 2H), 3.67 (s, 3H), 3.52-3.48 (m, 2H), 3.25 - 3.15 (m, 1H), 2.24-2.21 (m, 2H), 2.13 - 2.03 (m, 2H), 1.08 - 1.01 (m, 2H), 0.91 - 0.87 (m, 2H). | Pd 174, $Cs_2CO_3$, dioxane |
| **INTC69** | methyl 2-(2-(cyclopropanesulfonamido)-6-methylpyrimidin-4-yl)-2-methylpropanoate 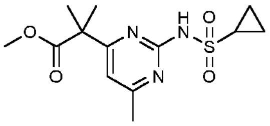 | Method C using INTC62 , [UPLC acidic], 314 (1.06). | 11.09 (s, 1H), 7.05 (s, 1H), 3.59 (s, 3H), 3.21 - 3.11 (m, 1H), 2.40 (s, 3H), 1.48 (s, 6H), 1.15 - 1.07 (m, 2H), 1.07 - 0.96 (m, 2H). | $[Pd(allyl)Cl]_2$ tBuXPhos, $Cs_2CO_3$, dioxane |
| **INTC70** | methyl 2-(2-(cyclopropanesulfonamido)-6-(trifluoromethyl)pyrimidin-4-yl)-2-methylpropanoate 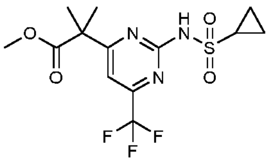 | Method C using INTC63 , [UPLC acidic], 368 (1.37). | 11.87 (s, 1H), 7.64 (s, 1H), 3.63 (s, 3H), 3.15 - 3.05 (m, 1H), 1.56 (s, 6H), 1.20 - 1.04 (m, 4H). | $[Pd(allyl)Cl]_2$ tBuXPhos, $Cs_2CO_3$, dioxane |
| **INTC71** | methyl 2-(2-(cyclopropanesulfonamido)-6-methoxypyrimidin-4-yl)-2-methylpropanoate 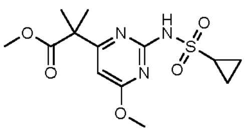 | Method C using INTC65, [HPLC acidic], 330 (1.86). | 11.11 (s, 1H), 6.53 (s, 1H), 3.91 (s, 3H), 3.59 (s, 3H), 3.25 - 3.17 (m, 1H), 1.46 (s, 6H), 1.17 - 0.90 (m, 4H). | Pd-174 $Cs_2CO_3$, dioxane |
| **INTC72** | 1-*tert*-butyl 3-methyl 3-(2-(cyclopropanesulfonamido) pyrimidin-4-yl)azetidine-1,3-dicarboxylate 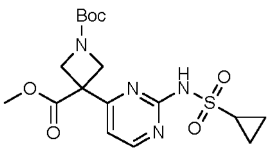 | Method C using INTC67 , [UPLC acidic], 313 (M-Boc+H)+ (1.22). | 11.40 (s, 1H), 8.64 (d, J = 5.2 Hz, 1H), 7.24 (d, J = 5.2 Hz, 1H), 4.38 - 4.34 (m, 2H), 4.32 - 4.26 (m, 2H), 3.71 (s, 3H), 3.20 - 3.11 (m, 1H), 1.39 (s, 9H), 1.16 - 1.05 (m, 2H), 1.08 - 1.00 (m, 2H). | Pd-174 $Cs_2CO_3$, dioxane |

(continued)

| INTC | Name/Structure (All examples containing chiral centres are racemates unless stated) | Synthesis Method, [LCMS Method], m/z $(M+H)^+$, (Rt/min) | $^1$H NMR Chemical Shift Data (DMSO-d6 unless stated) | Catalyst, Base, Solvent |
|---|---|---|---|---|
| **INTC73** | 1-*tert*-butyl 3-methyl 2-(2-methoxyethyl)-2-(2-(methylsulfonamido) pyrimidin-4-yl)malonate 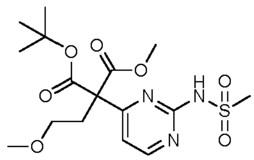 | Method C using INTC16, [HPLC acidic], 404 (1.91). | 11.38 (s, 1H), 8.63 (d, J = 5.3 Hz, 1H), 7.38 (d, J = 5.3 Hz, 1H), 3.71 (s, 3H), 3.35 - 3.31 (m, 4H), 3.29-3.23 (m, 1H), 3.12 (s, 3H), 2.49 - 2.43 (m, 1H), 2.41 - 2.34 (m, 1H), 1.40 (s, 9H). | Pd-174 $Cs_2CO_3$, dioxane |
| **INTC74** | methyl 2-fluoro-2-(2-(methylsulfonamido) pyrimidin-4-yl)butanoate 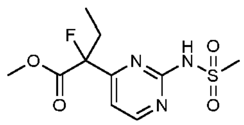 | Method C using INTC85, [HPLC acidic], 292 (1.52). | 11.56 (s, 1H), 8.74 (d, J = 5.1 Hz, 1H), 7.31 (d, J = 5.1 Hz, 1H), 3.74 (s, 3H), 3.37 (s, 3H), 2.43 - 2.17 (m, 2H), 0.88 (t, J = 7.3 Hz, 3H). | Pd-174 $Cs_2CO_3$, dioxane |
| **INTC75** | dimethyl 2-(2-(cyclopropanesulfonamido) pyrimidin-4-yl)-2-methoxymalonate 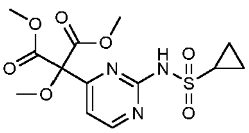 | Method C using INTC56, [HPLC acidic], 360 (1.49). | 11.48 (s, 1H), 8.70 (d, J = 5.2 Hz, 1H), 7.26 (d, J = 5.2 Hz, 1H), 3.77 (s, 6H), 3.49 (s, 3H), 3.23 - 3.10 (m, 1H), 1.16 - 1.01 (m, 4H). | Pd-174 $Cs_2CO_3$, dioxane |
| **INTC76** | 1-*tert*-butyl 4-methyl 4-(2-(methylsulfonamido) pyrimidin-4-yl)piperidine-1,4-dicarboxylate 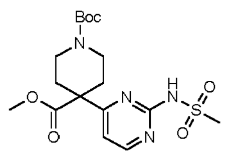 | Method C using INTC66, [UPLC acidic], 315 (M-Boc+H) (1.23). | 11.36 (s, 1H), 8.60 (d, J = 5.3 Hz, 1H), 7.20 (d, J = 5.3 Hz, 1H), 3.72 - 3.62 (m, 7H), 3.35 (s, 3H), 2.25 - 2.19 (m, 2H), 1.98 - 1.92 (m, 2H), 1.40 (s, 9H). | Pd-174 $Cs_2CO_3$, dioxane |
| **INTC77** | 1-*tert*-butyl 4-methyl 4-(2-(cyclopropanesulfonamido) pyrimidin-4-yl)piperidine-1,4-dicarboxylate 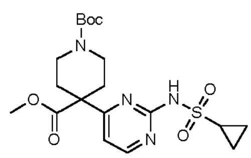 | Method C using INTC66, [HPLC acidic], 385(M-tBu+H) (2.08). | 11.30 (s, 1H), 8.59 (d, J = 5.3 Hz, 1H), 7.19 (d, J = 5.3 Hz, 1H), 3.74 - 3.67 (m, 1H), 3.67 (s, 3H), 3.24 - 3.15 (m, 1H), 2.53 - 2.48 (m, 2H), 2.26 - 2.19 (m, 3H), 2.03 - 1.92 (m, 2H), 1.40 (s, 9H), 1.15 - 1.08 (m, 2H), 1.08 - 1.00 (m, 2H). | Pd-174 $Cs_2CO_3$, dioxane |

(continued)

| INTC | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) | Catalyst, Base, Solvent |
|---|---|---|---|---|
| **INTC78** | dimethyl 2-(2-(cyclopropanesulfonamido) pyrimidin-4-yl)-2-isopropylmalonate 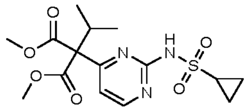 | Method C using INTC57, [UPLC acidic 2], 372 (0.58). | $^1H$ NMR not recorded. | $[Pd(allyl)Cl]_2$ tBuXPhos, $Cs_2CO_3$, dioxane |

**Decarboxylation**

Methyl 2-(2-(cyclopropanesulfonamido)pyrimidin-4-yl)acetate 2,2,2-trifluoroacetate **INTC33**

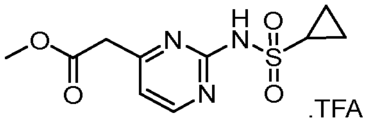

.TFA

TFA (1 mL, 13.0 mmol) was added dropwise to a stirred, ice-cooled solution of 1-*tert*-butyl 3-methyl 2-(2-(cyclopropanesulfonamido)pyrimidin-4-yl)malonate **INTC17** (0.27 g, 0.73 mmol) in DCM (2 mL). The reaction vessel was stirred at RT for 2 hrs and concentrated *in vacuo* to afford methyl 2-(2-(cyclopropanesulfonamido)pyrimidin-4-yl)acetate 2,2,2-trifluoroacetate (0.29 g, 0.68 mmol, 93% yield) as a yellow solid; Rt 0.79 mins (HPLC basic); m/z 272 $(M+H)^+$ $(ES^+)$; $^1H$ NMR (400 MHz, DMSO-$d_6$) $\delta$ 12.19 (s, 1H), 11.39 - 10.93 (m, 2H), 8.57 (d, J = 5.1 Hz, 1H), 7.13 (d, J = 5.1 Hz, 1H), 7.00 (dd, J = 7.6, 5.1 Hz, 1H), 5.95 - 5.87 (m, 1H), 4.92 (s, 1H), 3.84 (s, 2H), 3.65 (s, 3H), 3.61 (s, 3H), 3.29 - 3.10 (m, 1H), 2.72 - 2.60 (m, 1H), 1.17 - 0.85 (m, 8H).1:1 mixture of tautomers.

**Method D: Decarboxylation of pyrimidines bearing sulfonamides**

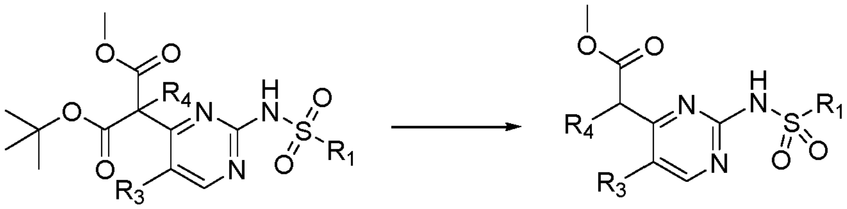

TFA (10 eq) was added dropwise to an ice-cooled, stirred solution of malonate derivative (1 eq) in DCM (15 volumes). The reaction vessel was stirred at RT for 18 hrs and then concentrated. The crude product was purified by normal phase chromatography.

**Table 4:** The following intermediates were made according to Method D.

| INTC | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| **INTC34** | methyl 2-(2-(cyclopropanesulfonamido)-5-fluoropyrimidin-4-yl)acetate 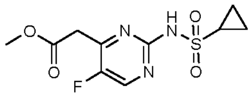 | Method D using INTC32, [HPLC acidic], 290 (0.83). | 11.37 (s, 1H), 8.76 - 8.63 (m, 1H), 3.98 - 3.89 (m, 2H), 3.67 (s, 3H), 3.26 - 3.12 (m, 1H), 1.16 - 0.98 (m, 4H). |
| **INTC35** | methyl 2-(2-(cyclopropanesulfonamido) pyrimidin-4-yl)butanoate 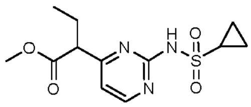 | Method D using INTC30, [UPLC acidic], 300 (0.99). | 11.26 (s, 1H), 8.57 (d, J = 5.1 Hz, 1H), 7.13 (d, J = 5.1 Hz, 1H), 3.74 (t, J = 7.5 Hz, 1H), 3.62 (s, 3H), 3.26 - 3.15 (m, 1H), 2.06 - 1.93 (m, 1H), 1.92 - 1.77 (m, 1H), 1.19 - 0.96 (m, 4H), 0.85 (t, J = 7.4 Hz, 3H). |
| **INTC36** | methyl 2-(2-(cyclopropanesulfonamido) pyrimidin-4-yl)-4-methoxybutanoate 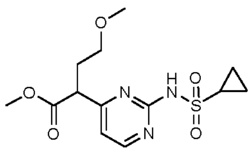 | Method D using INTC31, [UPLC basic], 330 (0.60). | 11.27 (s, 1H), 8.57 (d, J = 5.1 Hz, 1H), 7.13 (d, J = 5.1 Hz, 1H), 3.91 (t, J = 7.4 Hz, 1H), 3.62 (s, 3H), 3.33 - 3.20 (m, 3H), 3.19 (s, 3H), 2.28 - 2.18 (m, 1H), 2.11 - 2.01 (m, 1H), 1.16 - 0.99 (m, 4H). |

**Hydrolysis**

Potassium 2-(2-(cyclopropanesulfonamido)pyrimidin-4-yl)butanoate **INTC37**

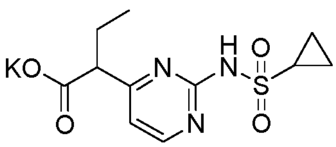

A solution of methyl 2-(2-(cyclopropanesulfonamido)pyrimidin-4-yl)butanoate **INTC35** (2.4 g, 7.22 mmol) in THF (80 mL) was treated with TMSOK (2.26 g, 15.9 mmol). The reaction mixture was allowed to stir at RT for 18 hrs. The resulting suspension was concentrated in *vacuo* to afford potassium 2-(2-(cyclopropanesulfonamido)pyrimidin-4-yl)butanoate (3 g, 6.49 mmol, 90% yield) as a pale yellow solid; Rt 0.19 mins (UPLC basic); m/z 286 $(M+H)^+$ $(ES^+)$, ionises as free acid; $^1H$ NMR (500 MHz, DMSO-$d_6$) δ 7.97 (d, *J* = 5.0 Hz, 1H), 6.35 (d, *J* = 5.0 Hz, 1H), 3.01 (tt, *J* = 8.2, 5.0 Hz, 1H), 2.88 (dd, *J* = 8.0, 6.9 Hz, 1H), 1.88 - 1.79 (m, 1H), 1.61 - 1.50 (m, 1H), 0.84 - 0.74 (m, 5H), 0.65 - 0.55 (m, 2H), NH proton not observed.

Potassium 2-(2-(cyclopropanesulfonamido)pyrimidin-4-yl)-4-methoxybutanoate **INTC38**

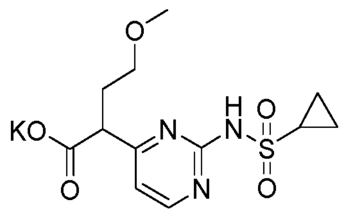

A solution of methyl 2-(2-(cyclopropanesulfonamido)pyrimidin-4-yl)-4-methoxybutanoate **INTC36** (0.23 g, 0.69 mmol) in THF (5 mL) was treated with TMSOK (0.22 g, 1.54 mmol). The reaction mixture was allowed to stir at RT for 18 hrs. The resulting suspension was quenched with MeOH (2 mL) then concentrated *in vacuo* to afford potassium 2-(2-(cyclopropanesulfonamido)pyrimidin-4-yl)-4-methoxybutanoate (0.33 g, 0.65 mmol, 94% yield) as a pale red solid; Rt 0.14 mins (UPLC basic); m/z 316 $(M+H)^+$ $(ES^+)$, ionises as free acid; $^1H$ NMR (500 MHz, DMSO-$d_6$) δ 8.03 (dd, *J* = 5.0, 2.9 Hz, 1H, minor), 7.97 (d, *J* = 5.0 Hz, 1H, major), 6.59 (d, *J* = 5.1 Hz, 1H, minor), 6.33 (d, *J* = 5.0 Hz, 1H, major), 3.28 - 3.13 (m, 7H), 3.12 - 2.96 (m, 1H), 2.22 - 1.99 (m, 1H), 1.83 - 1.77 (m, 1H), 0.84 - 0.75 (m, 2H), 0.63 - 0.57 (m, 2H). Mixture of tautomers.

Potassium 2-(2-(cyclopropanesulfonamido)pyrimidin-4-yl)acetate **INTC39**

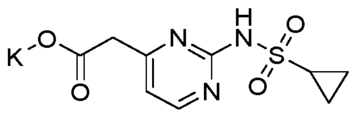

A solution of methyl 2-(2-(cyclopropanesulfonamido)pyrimidin-4-yl)acetate (600 mg, 2.212 mmol) **INTC33** in THF (12 mL) was treated with TMSOK (624 mg, 4.87 mmol). The reaction mixture was allowed to stir at RT for 18 hrs. The resulting suspension was concentrated in *vacuo* to afford potassium 2-(2-(cyclopropanesulfonamido)pyrimidin-4-yl)acetate (1.069 g, 2.208 mmol, quantitative yield) as a pale yellow solid. Rt 0.14 min (UPLC acidic); m/z 258 $(M+H)^+$ $(ES^+)$, ionises as free acid. $^1H$ NMR (500 MHz, DMSO-d6) δ 7.97 (d, J = 4.9 Hz, 1H), 6.33 (d, J = 4.9 Hz, 1H), 2.99 - 2.92 (m, 1H), 0.82 - 0.76 (m, 2H), 0.66 - 0.57 (m, 2H). $CH_2$ and NH signals not observed.

## 2-(2-(Cyclopropanesulfonamido)-5-fluoropyrimidin-4-yl)acetic acid **INTC40**

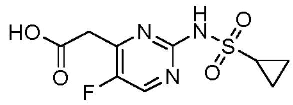

LiOH (0.105 g, 4.37 mmol) was added to a solution of methyl 2-(2-(cyclopropanesulfonamido)-5-fluoropyrimidin-4-yl)acetate (0.49 g, 1.457 mmol) **INTC34** in MeOH (5 mL) and water (2 mL) and stirred at RT for 18 hrs. The reaction mixture was concentrated in *vacuo* and the crude product was purified by chromatography by RP Flash C18 (40 g column, 0-50% MeCN/Water 0.1% Formic Acid) to afford 2-(2-(cyclopropanesulfonamido)-5-fluoropyrimidin-4-yl)acetic acid (0.44 g, 0.991 mmol, 68% yield) as a yellow solid. Rt 0.65 mins (UPLC acidic); m/z 276 $(M+H)^+$ $(ES^+)$; $^1H$ NMR (400 MHz, DMSO-d6) δ 8.49 (s, 1H), 4.15 (s, 1H), 2.97 - 2.85 (m, 1H), 2.22 - 2.15 (m, 2H), 0.86 - 0.69 (m, 2H), 0.69 - 0.52 (m, 2H), $CO_2H$ not observed.

4-(6-Ethoxypyrazin-2-yl)-N-(4-methoxybenzyl)aniline **INTC41**

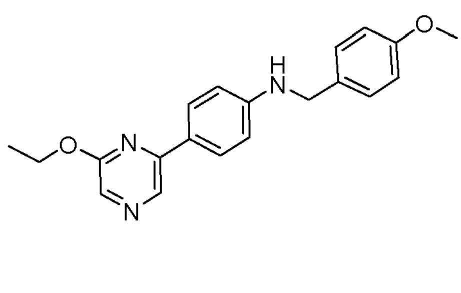

Sodium triacetoxyborohydride (0.148 g, 0.697 mmol) was added to a solution of 4-(6-ethoxypyrazin-2-yl)aniline **INTD18** (0.1 g, 0.465 mmol) and 4-methoxybenzaldehyde (0.085 mL, 0.697 mmol) in DCM (3 mL). The reaction was stirred at RT for 16 hrs. To the reaction was added saturated NaHCOs (aq) (20 mL), the aqueous extracted with DCM (3 x 20 mL) and the combined organic layers were concentrated *in vacuo.* The crude product was purified by chromatography on silica gel (24 g cartridge, 0-50% EtOAc/*iso*-hexane) to afford 4-(6-ethoxypyrazin-2-yl)-N-(4-methoxybenzyl)aniline (0.174 g, 0.467 mmol, quantitative yield) as a white solid. Rt 1.68 min (UPLC, basic); m/z 336 $(M+H)^+$ $(ES^+)$. $^1H$ NMR (500 MHz, DMSO-d6) δ 8.59 (s, 1H), 8.00 (s, 1H), 7.88 - 7.83 (m, 2H), 7.32 - 7.26 (m, 2H), 6.93 - 6.86 (m, 2H), 6.71 (t, J = 6.0 Hz, 1H), 6.70 - 6.65 (m, 2H), 4.43 (q, J = 7.0 Hz, 2H), 4.27 (d, J = 5.8 Hz, 2H), 3.73 (s, 3H), 1.37 (t, J = 7.0 Hz, 3H).

*tert*-Butyl 3-((4-(6-ethoxypyrazin-2-yl)phenyl)(4-methoxybenzyl)amino)-3-oxopropanoate **INTC42**

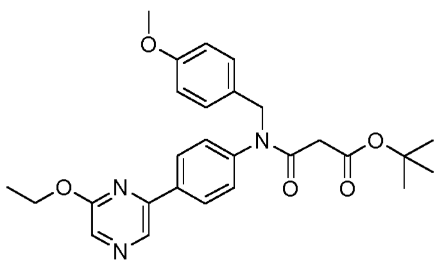

HATU (0.289 g, 0.760 mmol) was added to a stirred solution of 4-(6-ethoxypyrazin-2-yl)-N-(4-methoxybenzyl)aniline **INTC41** (0.17 g, 0.507 mmol), 3-(tert-butoxy)-3-oxopropanoic acid (0.1 mL, 0.649 mmol) and TEA (0.21 mL, 1.507 mmol) in DCM (5 mL). The resulting reaction was stirred at RT for 1 h. The reaction mixture was diluted with water (20 mL) and extracted with DCM (3 x 20 mL). The combined organic extracts were dried (phase separator) and the solvent removed under reduced pressure. The crude product was purified by chromatography on silica gel (24 g column, 0-100% EtOAc/*iso*-hexane) to afford *tert*-butyl 3-((4-(6-ethoxypyrazin-2-yl)phenyl)(4-methoxybenzyl)amino)-3-oxopropanoate (0.2 g, 0.377 mmol, 74% yield) as a clear, colourless gum. Rt 1.74 min (UPLC, basic); m/z 478 $(M+H)^+$ $(ES^+)$; $^1H$ NMR (500 MHz, DMSO-d6) δ 8.81 (s, 1H), 8.25 (s, 1H), 8.15 - 8.09 (m, 2H), 7.34 - 7.27 (m, 2H), 7.18 - 7.11 (m, 2H), 6.88 - 6.82 (m, 2H), 4.87 (s, 2H), 4.47 (q, J = 7.0 Hz, 2H), 3.72 (s, 3H), 3.20 (s, 2H), 1.39 (t, J = 7.1 Hz, 3H), 1.35 (s, 9H).

*tert*-Butyl 2-(2-chloropyrimidin-4-yl)-3-((4-(6-ethoxypyrazin-2-yl)phenyl)(4-methoxybenzyl)amino)-3-oxopropanoate **INTC43**

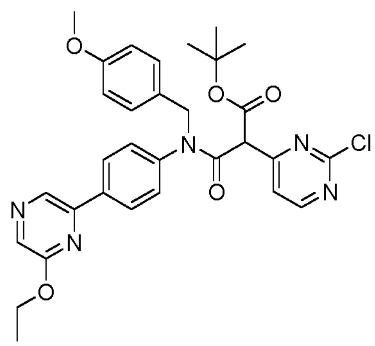

NaH (60% dispersion in mineral oil) (0.034 g, 0.838 mmol) was added to a stirred solution of *tert*-butyl 3-((4-(6-ethoxypyrazin-2-yl)phenyl)(4-methoxybenzyl)amino)-3-oxopropanoate **INTC42** (0.2 g, 0.419 mmol) in THF (4 mL, 0.419 mmol). The reaction was stirred at RT for 10 min then 2,4-dichloropyrimidine (0.087 g, 0.586 mmol) was added. The resulting mixture was stirred at 70°C for 2 hrs under $N_2$. The reaction mixture was quenched with brine (20 mL). The aqueous phase was extracted with DCM (3 x 50 mL), dried (phase separator) and concentrated *in vacuo.* The crude product was purified by chromatography on silica gel (24 g column, 0-100% EtOAc/*iso*-hexane) to afford *tert*-butyl 2-(2-chloropyrimidin-4-yl)-3-((4-(6-ethoxypyrazin-2-yl)phenyl)(4-methoxybenzyl)amino)-3-oxopropanoate (0.13 g, 0.189

mmol, 45% yield) as a clear, colourless gum. Rt 1.86 min (UPLC, basic); m/z 591 $(M+H)^+$ $(ES^+)$; $^1H$ NMR (500 MHz, DMSO-d6) δ 8.81 (s, 1H), 8.77 (d, J = 5.1 Hz, 1H), 8.26 (s, 1H), 8.15 - 8.12 (m, 2H), 7.66 - 7.63 (m, 1H), 7.22 - 7.16 (m, 4H), 6.90 - 6.85 (m, 2H), 5.02 (d, J = 14.7 Hz, 1H), 4.84 (s, 1H), 4.74 (d, J = 14.6 Hz, 1H), 4.47 (q, J = 7.0 Hz, 2H), 3.73 (s, 3H), 1.39 (t, J = 7.1 Hz, 3H), 1.37 (s, 9H).

2-(2-Chloropyrimidin-4-yl)-N-(4-(6-ethoxypyrazin-2-yl)phenyl)-*N*-(4-methoxybenzyl)acetamide **INTC44**

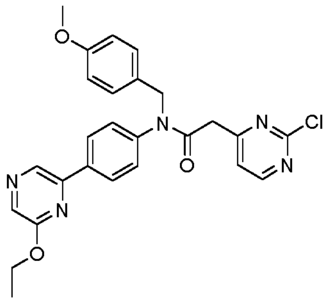

TFA (0.234 mL, 3.03 mmol) was added dropwise to an ice-cooled, stirred solution of *tert*-butyl 2-(2-chloropyrimidin-4-yl)-3-((4-(6-ethoxypyrazin-2-yl)phenyl)(4-methoxybenzyl)amino)-3-oxopropanoate **INTC43** (0.13 g, 0.189 mmol) in DCM (20 mL). The reaction vessel was stirred at 25°C for 7 hrs and then carefully basified with NaHCO3 (20 mL). The aqueous phase was extracted with DCM (2 x 20 mL), dried (phase separator) and the solvent was removed under reduced pressure. The crude product was purified by chromatography on silica gel (24 g column, 0-100% EtOAc/*iso*-hexane) to afford 2-(2-chloropyrimidin-4-yl)-N-(4-(6-ethoxypyrazin-2-yl)phenyl)-N-(4-methoxybenzyl)acetamide (0.05 g, 0.092 mmol, 49% yield) as a clear, colourless gum. Rt 1.62 min (UPLC, acidic); m/z 490 $(M+H)^+$ $(ES^+)$; $^1H$ NMR (500 MHz, DMSO-d6) δ 8.81 (s, 1H), 8.66 (d, J = 5.0 Hz, 1H), 8.25 (s, 1H), 8.14 - 8.10 (m, 2H), 7.45 (d, J = 5.1 Hz, 1H), 7.39 - 7.34 (m, 2H), 7.17 (d, J = 8.2 Hz, 2H), 6.89 - 6.82 (m, 2H), 4.89 (s, 2H), 4.46 (q, J = 7.1 Hz, 2H), 3.76 (s, 2H), 3.72 (s, 3H), 1.39 (t, J = 7.0 Hz, 3H).

2-(2-Chloropyrimidin-4-yl)-N-(4-(6-ethoxypyrazin-2-yl)phenyl)-N-(4-methoxybenzyl)butanamide **INTC45**

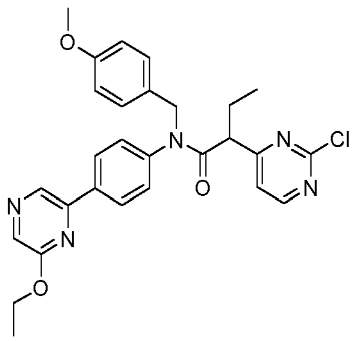

Iodoethane (0.410 mL, 5.10 mmol) was added to a stirred mixture of 2-(2-chloropyrimidin-4-yl)-N-(4-(6-ethoxypyrazin-2-yl)phenyl)-N-(4-methoxybenzyl)acetamide **INTC44** (0.5 g, 1.021 mmol) and potassium carbonate (0.705 g, 5.10 mmol) in acetone (5 mL). The reaction vessel was heated to 60°C and stirred for 18 hrs under $N_2$. The reaction mixture was concentrated, diluted in water (40 mL) and extracted into DCM (3 x 40 mL). The organics were combined, dried (phase separator) and concentrated *in vacuo.* The crude product was purified by chromatography on silica gel (40 g column, 0-100% EtOAc/*iso*-hexane) to afford 2-(2-chloropyrimidin-4-yl)-N-(4-(6-ethoxypyrazin-2-yl)phenyl)-N-(4-methoxybenzyl)butanamide (0.31 g, 0.539 mmol, 53% yield) as a clear pale yellow gum. Rt 2.73 min (HPLC, acidic); m/z 519 $(M+H)^+$ $(ES^+)$; $^1H$ NMR (500 MHz, DMSO-d6) δ 8.80 (s, 1H), 8.63 (d, J = 5.1 Hz, 1H), 8.25 (s, 1H), 8.07 (d, J = 8.3 Hz, 2H), 7.32 (d, J = 5.2 Hz, 1H), 7.17 - 7.08 (m, 4H), 6.88 - 6.81 (m, 2H), 4.92 (d, J = 14.7 Hz, 1H), 4.82 (d, J = 14.6 Hz, 1H), 4.50 - 4.43 (m, 2H), 3.73 - 3.69 (m, 4H), 2.09 - 2.00 (m, 1H), 1.83 - 1.74 (m, 1H), 1.39 (t, J = 7.0 Hz, 3H), 0.82 (t, J = 7.3 Hz, 3H).

2-(2-(Cyclopropanesulfonamido)pyrimidin-4-yl)-N-(4-(6-ethoxypyrazin-2-yl)phenyl)-N-(4-methoxybenzyl)butanamide **INTC46**

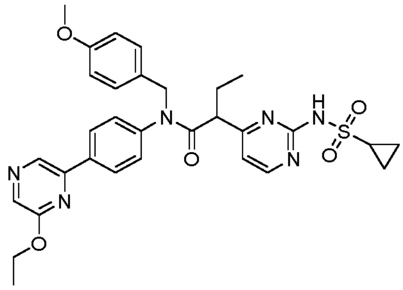

A 20 mL vial was charged with cyclopropanesulfonamide (0.078 g, 0.646 mmol), $Cs_2CO_3$ (0.351 g, 1.08 mmol), 2-(2-chloropyrimidin-4-yl)-N-(4-(6-ethoxypyrazin-2-yl)phenyl)-N-(4-methoxybenzyl)butanamide **INTC45** (0.31 g, 0.539 mmol) and dioxane (10 mL). The mixture was sparged with $N_2$ for 5 min. Pd-174 (0.012 g, 0.016 mmol) was added and the mixture was then heated at 80°C for 1 hr and then at 100°C for 7 hrs. Further cyclopropanesulfonamide (0.078 g, 0.646 mmol) and Pd-174 (0.012 g, 0.016 mmol) was added and the mixture heated at 100°C for a further 3 h. The reaction mixture was quenched with saturated $NH_4Cl$ (aq, 40 mL), extracted into DCM (3 x 40 mL), dried (phase separator) and concentrated *in vacuo.* The crude product was purified by chromatography on silica gel (40 g cartridge, 0-100% EtOAc/*iso*-hexane) to afford 2-(2-(cyclopropanesulfonamido)pyrimidin-4-yl)-N-(4-(6-ethoxypyrazin-2-yl)phenyl)-N-(4-methoxybenzyl)butanamide (0.18 g, 0.269 mmol, 50% yield) as a thick yellow gum. Rt 1.65 min (UPLC, acidic); 603.6 $(M+H)^+$ $(ES^+)$; $^1H$ NMR (500 MHz, DMSO-d6) $\delta$ 11.22 (s, 1H), 8.80 (s, 1H), 8.49 - 8.42 (m, 1H), 8.25 (s, 1H), 8.08 (d, J = 8.1 Hz, 2H), 7.22 (d, J = 8.1 Hz, 2H), 7.14 - 7.08 (m, 2H), 6.96 - 6.89 (m, 1H), 6.87 - 6.81 (m, 2H), 4.99 (d, J = 14.6 Hz, 1H), 4.76 (d, J = 14.7 Hz, 1H), 4.47 (q, J = 7.0 Hz, 2H), 3.71 (s, 3H), 3.65 (t, J = 7.3 Hz, 1H), 3.22 - 3.13 (m, 1H), 2.10 - 1.96 (m, 1H), 1.83 - 1.72 (m, 1H), 1.39 (t, J = 7.0 Hz, 3H), 1.12 - 1.06 (m, 2H), 0.93 - 0.87 (m, 2H), 0.83 (t, J = 7.3 Hz, 3H).

*tert*-Butyl 2-(2-(cyclopropanesulfonamido)pyrimidin-4-yl)-3-((4-(6-ethoxypyrazin-2-yl)phenyl)(4-methoxybenzyl)amino)-3-oxopropanoate **INTC47**

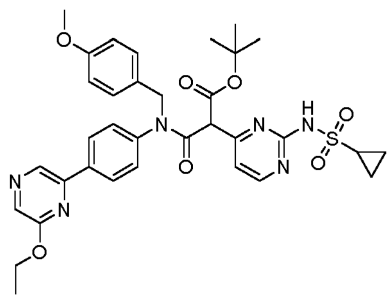

A 20 mL vial was charged with cyclopropanesulfonamide (0.074 g, 0.610 mmol), $Cs_2CO_3$ (0.331 g, 1.017 mmol), *tert*-butyl 2-(2-chloropyrimidin-4-yl)-3-((4-(6-ethoxypyrazin-2-yl)phenyl)(4-methoxybenzyl)amino)-3-oxopropanoate **INTC43** (0.3 g, 0.508 mmol) and dioxane (10 mL). The mixture was sparged with $N_2$ for 5 min. In a separate 20 mL vial was added $[Pd(allyl)Cl]_2$ (4.68 mg, 0.013 mmol), tBuXPhos (0.022 g, 0.051 mmol) and dioxane (2 mL). The mixture was stirred under $N_2$ for 5 min then added to the first mixture. The resulting mixture was heated under $N_2$ at 60°C for 4 hrs. Further cyclopropanesulfonamide (0.074 g, 0.610 mmol) was added followed by Pd-174 (11.00 mg, 0.015 mmol). The mixture was then heated at 80°C for 1 hr. The reaction mixture was quenched with saturated $NH_4Cl$ (aq, 40 mL), extracted into DCM (3 x 20 mL), dried (phase separator) and concentrated *in vacuo.* The crude product was purified by chromatography on silica gel (40 g column, 0-100% EtOAc/iso-hexane) to afford *tert*-butyl 2-(2-(cyclopropanesulfonamido)pyrimidin-4-yl)-3-((4-(6-ethoxypyrazin-2-yl)phenyl)(4-methoxybenzyl)amino)-3-oxopropanoate (0.1 g, 0.130 mmol, 26% yield) as a white solid. Rt 2.51 min (HPLC, basic); m/z 675 $(M+H)^+$ $(ES^+)$.

**PMB protection**

Methyl 2-(2-(*N*-(4-methoxybenzyl)cyclopropanesulfonamido)pyrimidin-4-yl)butanoate **INTC48**

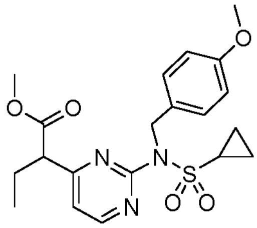

1-(Bromomethyl)-4-methoxybenzene (0.470 mL, 3.34 mmol) was added into a stirring heterogeneous mixture of methyl 2-(2-(cyclopropanesulfonamido)pyrimidin-4-yl)butanoate (1 g, 3.34 mmol) **INTC35** and $K_2CO_3$ (0.46 g, 3.34 mmol) in DMF (20 mL). The resulting reaction mixture was stirred at RT for 18 hrs and was then poured into water (200 mL) and extracted with EtOAc (3 x 50 mL). The organic extract was washed with water (100 mL) and brine (100 mL), dried over $MgSO_4$, filtered and solvent removed *in vacuo*. The crude product was purified by chromatography on silica gel (40 g column, 0-50% EtOAc/iso-hexane) to afford methyl 2-(2-(*N*-(4-methoxybenzyl) cyclopropanesulfonamido)pyrimidin-4-yl)butanoate (844 mg, 1.95 mmol, 58% yield) as a colourless oil. Rt 2.43 min (HPLC, acidic); m/z 420 $(M+H)^+$ $(ES^+)$; $^1H$ NMR (500 MHz, DMSO-d6) δ 8.64 (d, J = 5.1 Hz, 1H), 7.25 (d, J = 8.3 Hz, 2H), 7.19 (d, J = 5.1 Hz, 1H), 6.86 (d, J = 8.3 Hz, 2H), 5.17 - 5.02 (m, 2H), 3.71 (s, 3H), 3.64-3.55 (m, 4H), 2.05 - 1.93 (m, 2H), 1.89-1.76 (m, 1H), 1.10 - 0.96 (m, 4H), 0.82 (t, J = 7.3 Hz, 3H).

1-tert-Butyl 3-methyl 2-(2-(*N*-(4-methoxybenzyl)methylsulfonamido)pyrimidin-4-yl)-2-(2-methoxyethyl)malonate **INTC79**

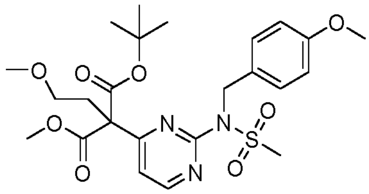

Prepared as for **INTC48** using 1-*tert*-butyl 3-methyl 2-(2-methoxyethyl)-2-(2-(methylsulfonamido)pyrimidin-4-yl)malonate **INTC73** and 1-(chloromethyl)-4-methoxybenzene to afford 1-tert-butyl 3-methyl 2-(2-(*N*-(4-methoxybenzyl)methylsulfonamido)pyrimidin-4-yl)-2-(2-methoxyethyl)malonate (65% yield) as a colourless oil. Rt 2.55 min (HPLC, acidic); *m/z* 524 $(M+H)^+$ $(ES^+)$; $^1H$ NMR (500 MHz, DMSO-d6) δ 8.72 (d, J = 5.3 Hz, 1H), 7.47 (d, J = 5.3 Hz, 1H), 7.27 - 7.21 (m, 2H), 6.92 - 6.82 (m, 2H), 5.17 - 5.10 (m, 2H), 3.71 (s, 3H), 3.67 (s, 3H), 3.44 (s, 3H), 3.29-3.22 (m, 1H), 3.22 - 3.15 (m, 1H), 3.09 (s, 3H), 2.48 - 2.29 (m, 2H), 1.37 (s, 9H).

Dimethyl 2-isopropyl-2-(2-(*N*-(4-methoxybenzyl)cyclopropanesulfonamido)pyrimidin-4-yl)malonate **INTC80**

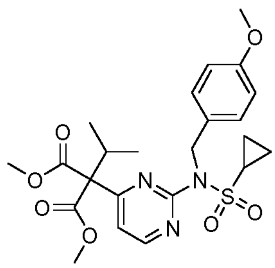

Prepared as for **INTC48** using dimethyl 2-(2-(cyclopropanesulfonamido)pyrimidin-4-yl)-2-isopropylmalonate **INTC78** and 1-(chloromethyl)-4-methoxybenzene to afford dimethyl 2-isopropyl-2-(2-(*N*-(4-methoxybenzyl)cyclopropanesulfonamido)pyrimidin-4-yl)malonate (28% yield) as a colourless oil. Rt 0.72 min (UPLC, acidic 2); m/z 492 (M+H)+ (ES+); $^{1}$H NMR not recorded.

**Decarboxylation of PMB protected Sulfonamides**

Methyl 4-methoxy-2-(2-(*N*-(4-methoxybenzyl)methylsulfonamido)pyrimidin-4-yl)butanoate **INTC81**

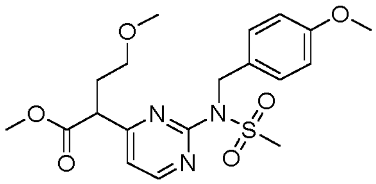

HCl (4M in dioxane) (0.44 mL, 14.51 mmol) was added into a stirring solution of 1-tert-butyl 3-methyl 2-(2-(*N*-(4-methoxybenzyl)methylsulfonamido)pyrimidin-4-yl)-2-(2-methoxyethyl)-malonate **INTC79** (8.0 g, 14.5 mmol) in DCM (100 mL) and the resulting reaction mixture was stirred at 50 °C for 4 hrs. The reaction mixture was concentrated *in vacuo* and the crude product was purified by chromatography on silica gel (220 g column, 0-100% EtOAc/iso-hexane) to afford methyl 4-methoxy-2-(2-(*N*-(4-methoxybenzyl)methylsulfonamido)pyrimidin-4-yl)butanoate (2.47 g, 5.54 mmol, 38% yield) as a colourless oil. Rt 2.13 min (HPLC, acidic); *m/z* 424 $(M+H)^+$ $(ES^+)$; $^{1}$H NMR (500 MHz, DMSO-d6) δ 8.64 (d, J = 5.1 Hz, 1H), 7.32 - 7.25 (m, 2H), 7.19 (d, J = 5.1 Hz, 1H), 6.90 - 6.84 (m, 2H), 5.20 - 5.07 (m, 2H), 3.97 (t, J = 7.4 Hz, 1H), 3.72 (s, 3H), 3.59 (s, 3H), 3.50 (s, 3H), 3.37 - 3.26 (m, 2H), 3.16 (s, 3H), 2.29 - 2.19 (m, 1H), 2.09 - 2.00 (m, 1H).

Methyl 2-(2-(*N*-(4-methoxybenzyl)cyclopropanesulfonamido)pyrimidin-4-yl)-3-methylbutanoate **INTC82**

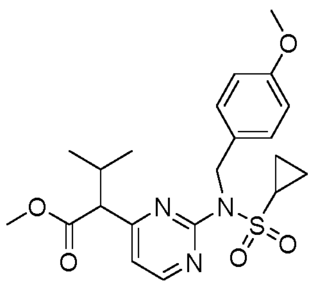

To a solution of dimethyl 2-isopropyl-2-(2-(N-(4-methoxybenzyl)cyclopropanesulfonamido)pyrimidin-4-yl)malonate **INTC80** (2.79 g, 5.68 mmol), in water (0.11 mL, 6.11 mmol) in DMSO (7 mL) was added lithium chloride (0.29 g, 6.81 mmol). The reaction mixture was heated at 140 °C for 1 hr. The reaction mixture was cooled to RT and diluted with EtOAc (100 mL) and water (100 mL). The phases were partitioned and the organic phase was further washed with water (100 mL), water/brine (1:1, 50 mL) and sat. brine (50 mL). The organic phase was dried over $MgSO_4$, filtered and concentrated onto silica (10 g). The crude product was purified by chromatography on silica gel (40 g cartridge, 0-30% EtOAc/*iso*-hexane) to afford methyl 2-(2-(*N*-(4-methoxybenzyl)cyclopropanesulfonamido)pyrimidin-4-yl)-3-methylbutanoate (2.00 g, 3.69 mmol, 65% yield) as a colourless gum. Rt 0.70 min (UPLC, acidic 2); *m/z* 434 $(M+H)^+$ $(ES^+)$; $^{1}$H NMR not recorded.

**Alkylation of PMB protected sulfonamides**

Methyl 4-methoxy-2-(2-(*N*-(4-methoxybenzyl)methylsulfonamido)pyrimidin-4-yl)-2-methylbutanoate **INTC83**

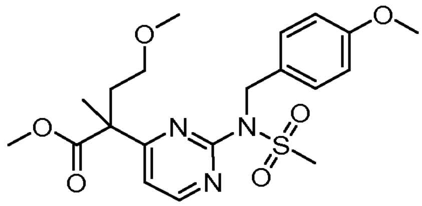

Prepared using **Method B** using methyl 4-methoxy-2-(2-(N-(4-methoxybenzyl)methylsulfonamido)pyrimidin-4-yl)butanoate **INTC81** with NaH and MeI in DMF to afford methyl 4-methoxy-2-(2-(N-(4-methoxybenzyl)methylsulfonamido)pyrimidin-4-yl)-2-methylbutanoate (89% yield) as a colourless oil. Rt 2.20 min (HPLC, acidic); *m/z* 438 $(M+H)^+$ $(ES^+)$; $^1H$ NMR (500 MHz, DMSO-d6) δ 8.66 (d, J = 5.2 Hz, 1H), 7.29 - 7.19 (m, 3H), 6.91 - 6.81 (m, 2H), 5.13 (s, 2H), 3.72 (s, 3H), 3.57 (s, 3H), 3.47 (s, 3H), 3.33 - 3.25 (m, 2H), 3.12 (s, 3H), 2.29 - 2.13 (m, 2H), 1.48 (s, 3H).

Methyl 2-(2-(*N*-(4-methoxybenzyl)cyclopropanesulfonamido)pyrimidin-4-yl)-2-methylbutanoate **INTC84**

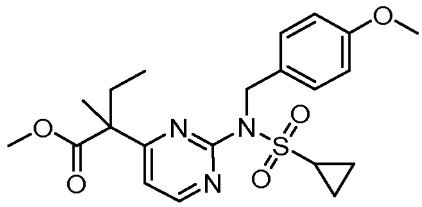

Prepared using **Method B** using methyl 2-(2-(*N*-(4-methoxybenzyl)cyclopropanesulfonamido)pyrimidin-4-yl)butanoate **INTC48** with $K_2CO_3$ and MeI in DMF to afford methyl 2-(2-(*N*-(4-methoxybenzyl)cyclopropanesulfonamido)pyrimidin-4-yl)-2-methylbutanoate (39% yield) as a colourless gum. Rt 2.57 min (HPLC, acidic); *m/z* 434 $(M+H)^+$ $(ES^+)$; $^1H$ NMR (500 MHz, DMSO-d6) δ 8.66 (d, J = 5.3 Hz, 1H), 7.26 - 7.17 (m, 3H), 6.90 - 6.82 (m, 2H), 5.10 (s, 2H), 3.71 (s, 3H), 3.64 (s, 3H), 2.05 - 1.88 (m, 2H), 1.44 (s, 3H), 1.04 - 0.97 (m, 4H), 0.76 (t, J = 7.4 Hz, 3H). (1H obscured by DMSO).

**Fluorination**

Methyl 2-fluoro-2-(2-(*N*-(4-methoxybenzyl)cyclopropanesulfonamido)pyrimidin-4-yl)butanoate **INTC49**

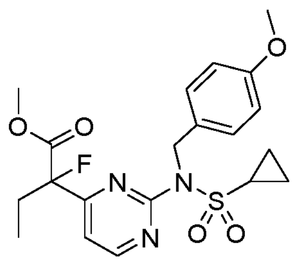

To a solution of methyl 2-(2-(*N*-(4-methoxybenzyl)cyclopropanesulfonamido)pyrimidin-4-yl)butanoate **INTC48** (400 mg, 0.95 mmol) in THF (10 mL) at -78 °C was added LHMDS (1.19 mL, 1.19 mmol, 1 M in THF) dropwise over 5 min. The resulting mixture was warmed to RT and stirred for 1 hr. The solution was cooled down to -78 °C again and a solution of *N*-fluoro-*N*-(phenylsulfonyl)benzenesulfonamide (376 mg, 1.19 mmol) in THF (3 mL) was added dropwise over 5 min. The resulting mixture was warmed to RT and stirred for 1 hr. The solution was diluted with sat. NaHCOs (*aq*, 100 mL) and EtOAc (100 mL) and the phases were separated. The aqueous phase was extracted with EtOAc (2 x 50 mL). The combined organic layers were dried over $Na_2SO_4$, filtered and the solvent was removed *in vacuo.* The crude product was purified by chromatography on silica gel (24 g column, 0-50% EtOAc/*iso*-hexane) to afford methyl 2-fluoro-2-(2-(*N*-(4-methoxybenzyl)cyclopropanesulfonamido)pyrimidin-4-yl)butanoate (390 mg, 0.865 mmol, 91% yield)

as a clear oil. Rt 2.48 min (HPLC, acidic); m/z 438 $(M+H)^+$ $(ES^+)$; $^1$H NMR (500 MHz, DMSO-d6) δ 8.81 (d, J = 5.1 Hz, 1H), 7.37 (dd, J = 5.1, 1.5 Hz, 1H), 7.29 - 7.19 (m, 2H), 6.90 - 6.83 (m, 2H), 5.17 - 5.03 (m, 2H), 3.72 (s, 3H), 3.69 (s, 3H) 3.65-3.57 (m, 1H), 2.40 - 2.14 (m, 2H), 1.11 - 0.97 (m, 4H), 0.84 (t, J = 7.4 Hz, 3H).

**INTC49** which is enantio-enriched can be made using the following method:

To a solution of methyl 2-(2-(*N*-(4-methoxybenzyl)cyclopropanesulfonamido)pyrimidin-4-yl)butanoate **INTC48** (0.066 g, 0.157 mmol) in THF (2.5 mL) at-40 °C was added LHMDS (0.189 mL, 0.189 mmol) dropwise over 5 mins. The resulting mixture was warmed to RT and stirred for 1 hr. A second solution was prepared of of (-)-Cinchonidine (0.069 g, 0.236 mmol) and Selectfluor (0.072 g, 0.205 mmol) in MeCN (2.5 mL), which was stirred at RT for 30 mins. The solution of fluorinating agent was then cooled to -40 °C and the solution of deprotonated ester was added dropwise over 5 mins. The reaction mixture was stirred at -40 °C for 1 h and warmed to RT as the cooling bath expired over 2 h. The reaction mixture was stirred at RT for 20 h. The reaction mixture was diluted with sat. NaHCOs (aq, 10 mL) and EtOAc (20 mL). The phases were separated and the organics were washed with further sat. NaHCOs (*aq*, 10 mL) then 1 M HCl (*aq*, 10 mL). The combined organics were dried ($MgSO_4$), filtered and concentrated *in vacuo.* The crude product was purified by chromatography on silica gel (4 g cartridge, 0-50% EtOAc/*iso*-hexane) to afford methyl 2-fluoro-2-(2-(*N*-(4-methoxybenzyl)cyclopropanesulfonamido)pyrimidin-4-yl)butanoate (0.024 g, 0.052 mmol, 33% yield) as a colourless oil. Rt 0.70 min (UPLC 2, acidic); m/z 438 $(M+H)^+$ $(ES^+)$; $^1$H NMR (500 MHz, DMSO-d6) δ 8.81 (d, J = 5.1 Hz, 1H), 7.37 (dd, J = 5.1, 1.5 Hz, 1H), 7.29 - 7.19 (m, 2H), 6.90 - 6.83 (m, 2H), 5.17 - 5.03 (m, 2H), 3.72 (s, 3H), 3.69 (s, 3H) 3.65-3.57 (m, 1H), 2.40 - 2.14 (m, 2H), 1.11 - 0.97 (m, 4H), 0.84 (t, J = 7.4 Hz, 3H).

Methyl 2-(2-(cyclopropanesulfonamido)pyrimidin-4-yl)-2-fluorobutanoate **INTC177**

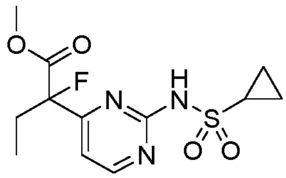

To a solution of methyl 2-fluoro-2-(2-(*N*-(4-methoxybenzyl)cyclopropane-sulfonamido)pyrimidin-4-yl)butanoate **INTC49** (24 mg, 0.055 mmol) in DCM (5 mL) was added TFA (0.5 mL, 6.49 mmol). The reaction mixture was stirred at RT for 3 h. The reaction mixture was concentrated *in vacuo* and the resulting brown residue was purified by chromatography on silica gel (4 g cartridge, 0-50% EtOAc/*iso*-hexane) to afford methyl 2-(2-(cyclopropanesulfonamido)pyrimidin-4-yl)-2-fluorobutanoate (18 mg, 0.053 mmol, 97% yield) as a colourless oil. Rt 0.55 min (UPLC 2, acidic); m/z 318 $(M+H)^+$. $^1$H NMR (500 MHz, DMSO-d6) δ 11.50 (s, 1H), 8.73 (d, J = 5.2 Hz, 1H), 7.31 (d, J = 5.2 Hz, 1H), 3.73 (s, 3H), 3.25 - 3.16 (m, 1H), 2.45 - 2.18 (m, 2H), 1.22 - 1.01 (m, 4H), 0.89 (t, J = 7.4 Hz, 3H).

The product as a mixture of enantiomers was analysed by Chiral IC7 method HPLC; Rt = 29.08 mins (10%) and 29.75 mins (90%).

**Lithium salt formation**

Lithium 2-fluoro-2-(2-(*N*-(4-methoxybenzyl)cyclopropanesulfonamido)pyrimidin-4-yl)butanoate **INTC50**

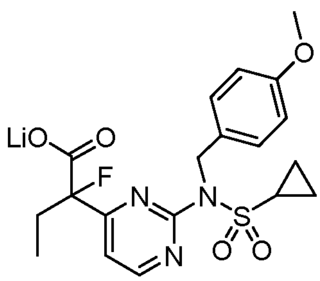

To a solution of methyl 2-fluoro-2-(2-(*N*-(4-methoxybenzyl)cyclopropanesulfonamido) pyrimidin-4-yl)butanoate **INTC49** (1.45 g, 3.31 mmol) in THF (15 mL) and MeOH (7.5 mL) was added a solution LiOH (0.091 g, 3.81 mmol) in

water (5 mL). The reaction mixture was stirred at RT for 3 hrs. The reaction mixture was concentrated *in vacuo* and the resulting yellow oil was taken up into in MeCN (10 mL) and concentrated *in vacuo* to give lithium 2-fluoro-2-(2-(*N*-(4-methoxybenzyl)cyclopropanesulfonamido)pyrimidin-4-yl)butanoate (1.46 g, 3.30 mmol, quant. yield) as a pale yellow foam which was used without further purification. Rt 0.95 min (UPLC, basic); m/z 424 (ionizes as COOH, $M+H)^+$ ($ES^+$); $^1$H NMR (500 MHz, DMSO-d6) $\delta$ 8.57 - 8.52 (m, 1H), 7.34 - 7.28 (m, 2H), 7.20 - 7.14 (m, 1H), 6.90 - 6.83 (m, 2H), 5.19 - 5.04 (m, 2H), 4.14 - 4.10 (m, 1H), 3.71 (s, 3H), 2.33-2.20 (m, 1H), 2.17-2.08 (m, 1H), 1.15-1.04 (m, 1H), 1.06-0.97 (m, 1H), 0.93 - 0.80 (m, 2H), 0.80 - 0.73 (m, 3H).

**Method H: Benzylic fluorination of hetero-aromatic esters**

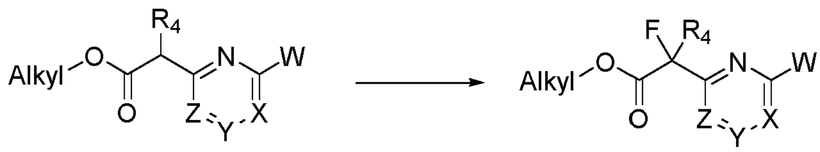

X = CH, N
Y = $CR_2$, N
Z = $CR_2$, N
W = Hal, N(PMB)$SO_2$Alkyl

A solution of hetero-aromatic ester (1 eq) in THF (10 volumes) was cooled to -78 °C to which was added LiHMDS (1.25 eq 1M in THF). The reaction mixture was then warmed to RT for 1 hr. The solution was cooled to -78 °C and a solution (in THF) of, or solid, NSFI (1.25 eq) was added dropwise then warmed to RT for 2 hrs. The solution was diluted with sat. NaHCOs (aq) and the product was extracted into EtOAc. The crude product was purified by normal phase chromatography.

**Table 5:** The following intermediates were made according to Method H.

| INTC | Name/Structure (All examples containing chiral centres are racemates unless stated) | Synthesis Method, [LCMS Method], m/z $(M+H)^+$, (Rt/min) | $^1$H NMR Chemical Shift Data (DMSO-d6 unless stated) |
|---|---|---|---|
| **INTC85** | methyl 2-(2-chloropyrimidin-4-yl)-2-fluorobutanoate 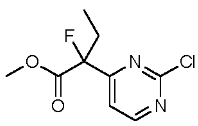 | Method H using INTC61, [HPLC basic], 233 $^{35}$Cl isotope (1.85). | 8.92 (d, J = 5.1 Hz, 1H), 7.78 (d, J = 5.1 Hz, 1H), 3.75 (s, 3H), 2.45 - 2.18 (m, 2H), 0.87 (t, J = 7.4 Hz, 3H). |

Methyl 2-fluoro-2-(2-(*N*-(4-methoxybenzyl)cyclopropanesulfonamido)pyrimidin-4-yl)-3-methylbutanoate **INTC86**

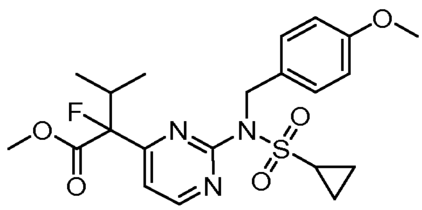

To a solution of methyl 2-(2-(*N*-(4-methoxybenzyl)cyclopropanesulfonamido)pyrimidin-4-yl)-3-methylbutanoate **INTC82** (1.50 g, 3.46 mmol) in anhydrous THF (30 mL) at - 78 °C was added LiHMDS (1 M in THF) (4.15 mL, 4.15 mmol) dropwise. The reaction mixture was stirred at - 78 °C for 5 mins then warmed to RT for 1 hr before recooling to - 78 °C. A solution of Selectfluor (1.90 g, 5.10 mmol) in MeCN (30 mL) was then added dropwise to the reaction mixture

over 5 mins. The reaction mixture was warmed to RT and stirred for 1 hr before sat. NaHCOs (*aq*, 5 mL) was added. The reaction mixture was then part concentrated *in vacuo* (to approx 10 mL) then EtOAc (100 mL) and sat. NaHCOs (*aq*, 100 mL) were added. The phases were separated and the organic phase was washed with sat. brine (50 mL). The organic layer was dried over $MgSO_4$, filtered and concentrated onto silica (15 g). The crude product was purified by chromatography on silica gel (40 g cartridge, 0-60% EtOAc/*iso*-hexane) to afford methyl 2-fluoro-2-(2-(*N*-(4-methoxybenzyl)cyclopropane-sulfonamido)pyrimidin-4-yl)-3-methylbutanoate (680 mg, 1.48 mmol, 43% yield) as a yellow gum. Rt 0.72 min (UPLC, acidic 2); *m/z* 452 $(M+H)^+$ $(ES^+)$; $^1H$ NMR (500 MHz, DMSO-d6) $\delta$ 8.79 (d, J = 5.1 Hz, 1H), 7.33 (dd, J = 5.1, 2.2 Hz, 1H), 7.29 - 7.22 (m, 2H), 6.91 - 6.84 (m, 2H), 5.19 - 5.07 (m, 2H), 3.74 - 3.66 (m, 7H), 2.93 - 2.77 (m, 1H), 1.17-1.05 (m, 2H), 1.06 - 0.97 (m, 5H), 0.67 (d, J = 6.8 Hz, 3H).

**Potassium salt formation**

Potassium 2-(2-(*N*-(4-methoxybenzyl)cyclopropanesulfonamido)pyrimidin-4-yl)-3-methylbutanoate **INTC87**

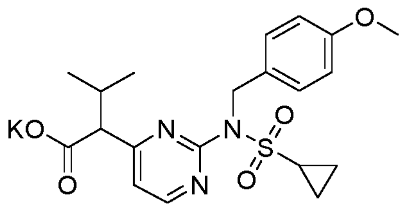

Prepared as for **INTC37** using methyl 2-(2-(*N*-(4-methoxybenzyl)cyclopropanesulfonamido)pyrimidin-4-yl)-3-methylbutanoate **INTC82** and potassium trimethylsilanolate to afford potassium 2-(2-(*N*-(4-methoxybenzyl)cyclopropanesulfonamido)pyrimidin-4-yl)-3-methylbutanoate (99% yield) as a pale yellow solid. Rt 1.59 min (HPLC, basic); m/z 420 $(M+H)^+$ (ES+); $^1H$ NMR not recorded.

**Difluoro-derivative *via* thioether**

Ethyl 2,2-difluoro-2-(2-(methylsulfonyl)pyrimidin-4-yl)acetate **INTC100**

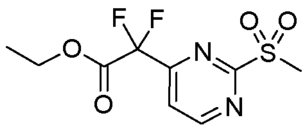

A suspension of ethyl 2,2-difluoro-2-(2-(methylthio)pyrimidin-4-yl)acetate (240 mg, 0.97 mmol) in MeOH (8 mL) and water (5 mL) was treated with Oxone (1.19 g, 1.93 mmol) and stirred vigorously for 3 hrs. DCM (10 mL) was added and the phases were partitioned with a phase separator, further extracting with DCM (2 x 5 mL). The combined organic phases were concentrated onto silica (1 g) and the crude product was purified by chromatography on silica gel (12 g column, 0-50% EtOAc/*iso*-hexane) to afford ethyl 2,2-difluoro-2-(2-(methylsulfonyl)pyrimidin-4-yl)acetate (80 mg, 0.28 mmol, 29% yield) as a colourless gum which set on standing. Rt 0.52 (UPLC acidic); *m/z* 281 $(M+H)^+$ $(ES^+)$; $^1H$ NMR (500 MHz, DMSO-d6) $\delta$ 9.42 (d, J = 5.1 Hz, 1H), 8.34 (d, J = 5.1 Hz, 1H), 4.39 (q, J = 7.1 Hz, 2H), 3.46 (s, 3H), 1.25 (t, J = 7.1 Hz, 3H).

2-(2-(Cyclopropanesulfonamido)pyrimidin-4-yl)-2,2-difluoroacetic acid **INTC101**

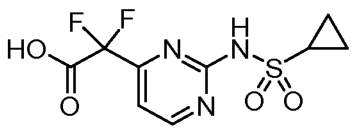

To a solution of cyclopropanesulfonamide (40 mg, 0.33 mmol) and ethyl 2,2-difluoro-2-(2-(methylsulfonyl)pyrimidin-4-yl)acetate **INTC100** (80 mg, 0.29 mmol) in DMF (1 mL) was treated with NaH (60% wt. on mineral oil) (14 mg, 0.35 mmol) and stirred at RT for 5 mins before being warmed to 60 °C for 6 hrs. 1M HCl (10 mL) was added and the reaction mixture was extracted with EtOAc (4 x 10 mL). The organic phases were combined, dried over $Na_2SO_4$, filtered and concentrated onto silica (500 mg). The crude product was purified by chromatography on silica gel (4 g column, 0-5% MeOH/DCM) to afford 2-(2-(cyclopropanesulfonamido)pyrimidin-4-yl)-2,2-difluoroacetic acid (80 mg, 0.136 mmol, 48% yield) as a brown solid. Rt 0.49 (UPLC acidic); *m/z* 294 $(M+H)^+$ $(ES^+)$; No NMR data collected.

**Tetrahydropyran-derivative *via* thioether**

Methyl 4-(2-(methylthio)pyrimidin-4-yl)tetrahydro-2*H*-pyran-4-carboxylate **INTC178**

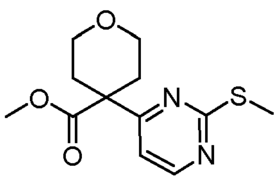

To a solution of 4-chloro-2-(methylthio)pyrimidine (0.55 g, 3.42 mmol) and methyl tetrahydro-2*H*-pyran-4-carboxylate (494 mg, 3.42 mmol) in THF (5 mL) at 30 °C was added LHMDS (1 M in THF) (4.11 mL, 4.11 mmol) dropwise. The reaction mixture was stirred at 30 °C for 5 min then was poured into water (100 mL) and extracted with EtOAc (2 x 200 mL). The organic extract was washed with brine (1 x 100 mL), dried ($MgSO_4$), filtered and solvent removed in vacuo to afford methyl 4-(2-(methylthio)pyrimidin-4-yl)tetrahydro-2*H*-pyran-4-carboxylate (915 mg, 3.24 mmol, 95% yield) as a pale yellow oil. Rt 1.74 min (HPLC acidic); *m/z* 269 $(M+H)^+$ $(ES^+)$; $^1H$ NMR (500 MHz, DMSO-d6) $\delta$ 8.62 (d, J = 5.3 Hz, 1H), 7.27 (d, J = 5.3 Hz, 1H), 3.76-3.70 (m, 2H), 3.67 (s, 3H), 3.54-3.46 (m, 2H), 2.49 (s, 3H), 2.27-2.20 (m, 2H), 2.14-2.04 (m, 2H).

Methyl 4-(2-(methylsulfonyl)pyrimidin-4-yl)tetrahydro-2*H*-pyran-4-carboxylate **INTC179**

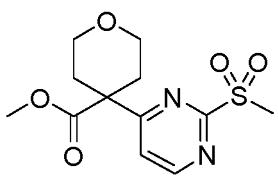

*m*CPBA (1.60 g, 7.13 mmol) was added portionwise into a stirring solution of methyl 4-(2-(methylthio)pyrimidin-4-yl)tetrahydro-2*H*-pyran-4-carboxylate **INTC178** (915 mg, 3.24 mmol) in DCM (50 mL) and the resulting reaction mixture was stirred at RT for 3 hrs. The reaction mixture was poured into sat. NaHCOs (*aq*, 200 mL) and extacted with DCM (3 x 100 mL). The organic extract was sequentially washed with sat. NaHCOs (*aq*, 100 mL) and brine (100 mL), dried ($MgSO_4$), filtered and solvent removed *in vacuo* to afford methyl 4-(2-(methylsulfonyl)pyrimidin-4-yl)tetrahydro-2H-pyran-4-carboxylate (1.10 g, 3.30 mmol, quant. yield) as thick gum. Rt 1.20 min (HPLC acidic); *m/z* 301 $(M+H)^+$ $(ES^+)$; $^1H$ NMR (500 MHz, DMSO-d6) $\delta$ 9.09 (d, J = 5.3 Hz, 1H), 7.95 (d, J = 5.3 Hz, 1H), 3.77-3.70 (m, 2H), 3.68 (s, 3H), 3.60-3.49 (m, 2H), 3.42 (s, 3H), 2.34-2.24 (m, 2H), 2.23 -2.13 (m, 2H).

Methyl 4-(2-(cyclopropanesulfonamido)pyrimidin-4-yl)tetrahydro-2*H*-pyran-4-carboxylate **INTC53**

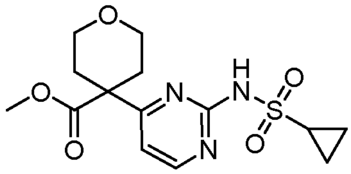

To a solution of methyl 4-(2-(methylsulfonyl)pyrimidin-4-yl)tetrahydro-2*H*-pyran-4-carboxylate **INTC179** (1.0 g, 3.33 mmol) and cyclopropanesulfonamide (0.52 g, 4.33 mmol) in NMP (100 mL) was added cesium carbonate (3.25 g, 9.99 mmol) and heated to 90 °C for 1 hr. The reaction mixture was cooled to RT and diluted with water (100 mL) and the mixture was washed with MTBE (2 x 100 mL) and the aqueous was slowly acidified to pH 3 using dilute HCl (20 mL). The resulting precepitate was filtered to afford methyl 4-(2-(cyclopropanesulfonamido)pyrimidin-4-yl)tetrahydro-2*H*-pyran-4-carboxylate (755 mg, 2.21 mmol, 66% yield) as a colourless solid. Rt. 0.88 (UPLC, acidic), *m/z* 342 $(M+H)^+$ $(ES^+)$; $^1H$ NMR (500 MHz, DMSO-d6) δ 11.30 (s, 1H), 8.60 (d, J = 5.3 Hz, 1H), 7.20 (d, J = 5.3 Hz, 1H), 3.79-3.72 (m, 2H), 3.67 (s, 3H), 3.52-3.44 (m, 2H), 3.25 - 3.14 (m, 1H), 2.30 - 2.17 (m, 2H), 2.12-2.04 (m, 2H), 1.14 - 1.01 (m, 4H).

**Amide formation of selected building blocks**

**Table 6:** The following intermediates were made according to Methods 1-4 which are described below for the synthesis of compound of formula (I).

| INTC | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| **INTC88** | 2-(2-chloropyrimidin-4-yl)-*N*-(5-(6-ethoxypyrazin-2-yl)-3-fluoropyridin-2-yl)-4-methoxybutanamide 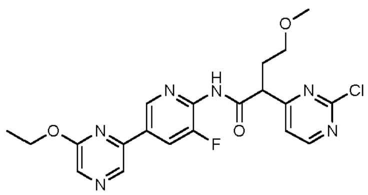 | Method 4 using INTC68 and INTD31 [HPLC basic], 447 $^{35}Cl$ isotope (2.05). | 10.89 (s, 1H), 9.03 - 8.98 (m, 1H), 8.92 (s, 1H), 8.78 (d, J = 5.1 Hz, 1H), 8.44 (dd, J = 11.1, 1.9 Hz, 1H), 8.32 (s, 1H), 7.65 (d, J = 5.1 Hz, 1H), 4.50 (q, J = 7.0 Hz, 2H), 4.26 - 4.19 (m, 1H), 3.44 - 3.33 (m, 2H), 3.23 (s, 3H), 2.36 - 2.25 (m, 1H), 2.21 - 2.11 (m, 1H), 1.40 (t, J = 7.0 Hz, 3H). |
| **INTC89** | 2-(2-chloropyrimidin-4-yl)-*N*-(5-(6-ethoxypyrazin-2-yl)pyridin-2-yl)-4-methoxybutanamide 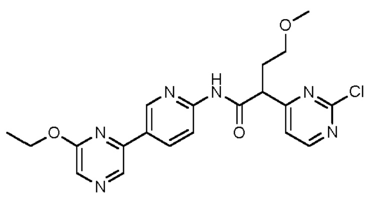 | Method 4 using INTC68 and INTD33 [UPLC acidic], 429 $^{35}Cl$ isotope (1.38). | 11.12 (s, 1H), 9.10 - 9.05 (m, 1H), 8.84 (s, 1H), 8.75 (d, J = 5.2 Hz, 1H), 8.53 - 8.47 (m, 1H), 8.25 (s, 1H), 8.20 (d, J = 8.7 Hz, 1H), 7.68 (d, J = 5.2 Hz, 1H), 4.52 - 4.44 (m, 2H), 4.34 - 4.27 (m, 1H), 3.42 - 3.32 (m, 2H), 3.20 (s, 3H), 2.37 - 2.26 (m, 1H), 2.20 - 2.09 (m, 1H), 1.43 - 1.37 (m, 3H). |

(continued)

| INTC | Name/Structure (All examples containing chiral centres are racemates unless stated) | Synthesis Method, [LCMS Method], m/z (M+H)$^+$, (Rt/min) | $^1$H NMR Chemical Shift Data (DMSO-d6 unless stated) |
|---|---|---|---|
| **INTC90** | 2-(2-chloropyrimidin-4-yl)-*N*-(4-(6-ethoxypyrazin-2-yl)-2-fluorophenyl)-4-methoxybutanamide 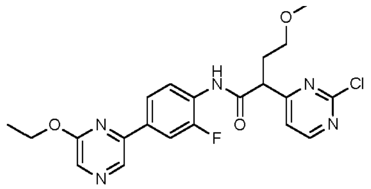 | Method 4 using INTC68 and INTD24 [UPLC acidic], 446 $^{35}$Cl isotope (1.54). | 10.34 (s, 1H), 8.83 (s, 1H), 8.76 (d, J = 4.9 Hz, 1H), 8.24 (s, 1H), 8.07 - 7.95 (m, 3H), 7.67 (d, J = 5.1 Hz, 1H), 4.54 - 4.45 (m, 2H), 4.34 - 4.27 (m, 1H), 3.42 - 3.36 (m, 1H), 3.33 (s, 3H), 2.89 - 2.86 (m, 1H), 2.34 - 2.25 (m, 1H), 2.19 - 2.09 (m, 1H), 1.41 (t, J = 6.8 Hz, 3H). |
| **INTC91** | *N*-(5-(6-ethoxypyrazin-2-yl)pyridin-2-yl)-4-methoxy-2-(2-(*N*-(4-methoxybenzyl) methylsulfonamid o)pyrimidin-4-yl)-2-methylbutanamide 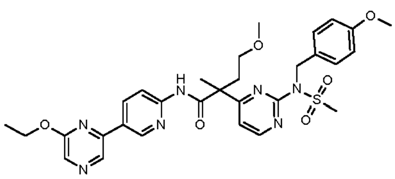 | Method 2 using INTC83 and INTD33, No LCMS data | 10.23 (s, 1H), 9.03 (dd, J = 2.4, 0.8 Hz, 1H), 8.84 (s, 1H), 8.67 (d, J = 5.3 Hz, 1H), 8.48 (dd, J = 8.8, 2.4 Hz, 1H), 8.25 (s, 1H), 8.19 (dd, J = 8.8, 0.8 Hz, 1H), 7.28 - 7.18 (m, 3H), 6.76 - 6.67 (m, 2H), 5.12 (s, 2H), 4.48 (q, J = 7.0 Hz, 2H), 3.62 (s, 3H), 3.43 (s, 3H), 3.12 (s, 3H), 2.45-2.36 (m, 2H), 2.30-2.21 (m, 2H), 1.60 (s, 3H), 1.41 (t, J = 7.0 Hz, 3H). |
| **INTC92** | *N*-(5'-chloro-[3,3'-bipyridin]-6-yl)-2-fluoro-2-(2-(*N*-(4-methoxybenzyl) cyclopropanesulfo namido)pyrimidin-4-yl) butanamide 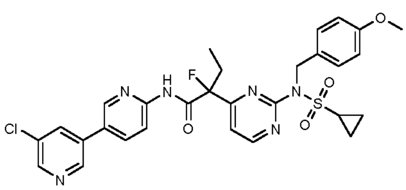 | Method 4 using INTC50 and INTD57 HPLC acidic], 612 $^{35}$Cl isotope (2.70). | 10.67 (s, 1H), 8.94 (d, J = 2.0 Hz, 1H), 8.88 - 8.81 (m, 2H), 8.66 (d, J = 2.3 Hz, 1H), 8.36 (t, J = 2.2 Hz, 1H), 8.29 (dd, J = 8.7, 2.6 Hz, 1H), 8.07 (d, J = 8.7 Hz, 1H), 7.52 (dd, J = 5.2, 1.3 Hz, 1H), 7.32 - 7.24 (m, 2H), 6.82 - 6.75 (m, 2H), 5.20 - 5.07 (m, 2H), 3.78 - 3.70 (m, 1H), 3.66 (s, 3H), 2.47 - 2.26 (m, 2H), 1.13 - 0.85 (m, 7H). |
| **INTC93** | *N*=(5-(6-ethoxypyrazin-2-yl)pyridin-2-yl)-2-(2-(N-(4-methoxybenzyl) cyclopropanesulfo namido)pyrimidin-4-yl)-2-methylbutanamide 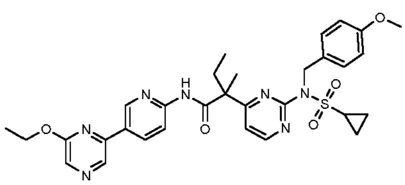 | Method 2 using INTC84 and INTD33, [HPLC acidic], 618, (2.79). | 10.20 (s, 1H), 9.03 (dd, J = 2.5, 0.6 Hz, 1H), 8.84 (s, 1H), 8.68 (d, J = 5.3 Hz, 1H), 8.48 (dd, J = 8.8, 2.5 Hz, 1H), 8.25 (s, 1H), 8.20 (dd, J = 8.8, 0.6 Hz, 1H), 7.24 (d, J = 5.3 Hz, 1H), 7.21 - 7.18 (m 2H), 6.76 - 6.68 (m, 2H), 5.09 (s, 2H), 4.48 (q, J = 7.0 Hz, 2H), 3.62 (s, 3H), 3.61 - 3.52 (m, 1H), 3.17 (d, J = 5.2 Hz, 2H), 1.55 (s, 3H), 1.40 (t, J = 7.0 Hz, 3H), 0.96-0.91 (m, 2H), 0.83-0.74 (m, 5H). |
| **INTC94** | *N*-(5-bromo-3-fluoropyridin-2-yl)-2-(2-(cyclopropanesulfonamido)pyrimid in-4-yl)butanamide 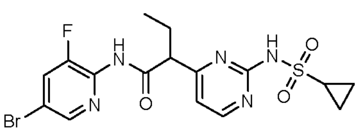 | Method 3 using INTC37 No LCMS data | 11.25 (s, 1H), 10.70 (s, 1H), 8.57 (d, J = 5.2 Hz, 1H), 8.42 (d, J = 2.0 Hz, 1H), 8.23 (dd, J = 9.4, 2.0 Hz, 1H), 7.17 (d, J = 5.2 Hz, 1H), 3.84 (dd, J = 8.7, 6.3 Hz, 1H), 2.10 - 1.98 (m, 1H), 1.98 - 1.87 (m, 1H), 1.15 - 1.10 (m, 2H), 1.07 - 0.99 (m, 2H), 0.99 - 0.89 (m, 3H). 1H obscured by $H_2O$ |

(continued)

| INTC | Name/Structure (All examples containing chiral centres are racemates unless stated) | Synthesis Method, [LCMS Method], m/z $(M+H)^+$, (Rt/min) | $^1$H NMR Chemical Shift Data (DMSO-d6 unless stated) |
|---|---|---|---|
| **INTC95** | *N*-(5-bromopyridin-2-yl)-2-(2-(cyclopropanesulfonamido)pyrimidin-4-yl)butanamide 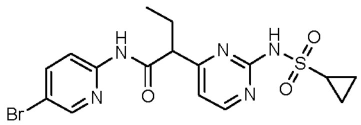 | Method 3 using INTC37 [UPLC acidic], 440 $^{79}$Br isotope, (1.26). | 11.23 (s, 1H), 10.97 (s, 1H), 8.55 (d, J = 5.2 Hz, 1H), 8.45 (d, J = 2.5 Hz, 1H), 8.06 (d, J = 8.9 Hz, 1H), 8.01 (dd, J = 8.9, 2.5 Hz, 1H), 7.19 (d, J = 5.2 Hz, 1H), 3.98 - 3.93 (m, 1H), 3.30 - 3.26 (m, 1H), 2.09 - 2.00 (m, 1H), 1.97 - 1.87 (m, 1H), 1.15 - 1.02 (m, 2H), 1.02 - 0.86 (m, 5H). |
| **INTC96** | *N*-(5-(6-ethoxypyrazin-2-yl)pyridin-2-yl)-2-fluoro-2-(2-(*N*-(4-methoxybenzyl) cyclopropanesulfo namido)pyrimidin-4-yl)-3-methylbutanamide 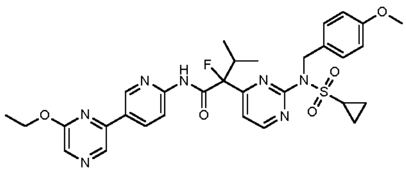 | Method 2 using INTC86 and INTD33, [UPLC acidic, 2], 636, (0.79). | $^1$H NMR not recorded. |
| **INTC97** | *N*-(4-(6-ethoxypyrazin-2-yl)-2-fluorophenyl)-2-(2-(*N*-(4-methoxybenzyl) cyclopropanesulfo namido)pyrimidin-4-yl)-3-methylbutanamide 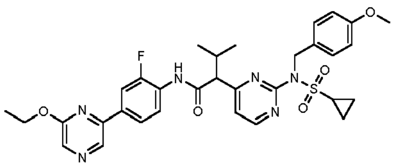 | Method 3 using INTC87 and INTD24 [UPLC acidic, 2], 635, (0.80). | $^1$H NMR not recorded. |
| **INTC98** | *N*-(5-(6-ethoxypyrazin-2-yl)pyridin-2-yl)-2-(2-(*N*-(4-methoxybenzyl) cyclopropanesulfo namido)pyrimidin-4-yl)-3-methylbutanamide 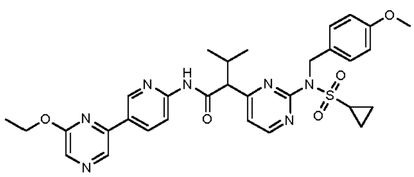 | Method 3 using INTC87 and INTD33 [UPLC acidic, 2], 618, (0.77). | $^1$H NMR not recorded. |

(continued)

| INTC | Name/Structure (All examples containing chiral centres are racemates unless stated) | Synthesis Method, [LCMS Method], m/z $(M+H)^+$, (Rt/min) | $^1$H NMR Chemical Shift Data (DMSO-d6 unless stated) |
|---|---|---|---|
| **INTC176** | N-(5-(6-cyclopropylpyrazin-2-yl)pyridin-2-yl)-2-fluoro-2-(2-(N-(4-methoxybenzyl) cyclopropanesulfo namido)pyrimidin-4-yl) butanamide 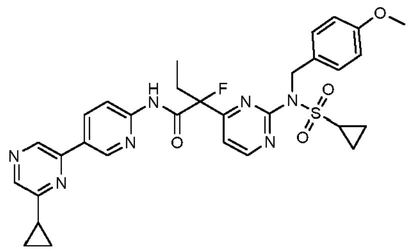 | Method 2 using INTC49 and INTD54, [HPLC acidic], 618, (2.80). | 10.67 (s, 1H), 9.06 (d, J = 2.4 Hz, 1H), 9.03 (s, 1H), 8.83 (d, J = 5.2 Hz, 1H), 8.61 (s, 1H), 8.49 (dd, J = 8.7, 2.5 Hz, 1H), 8.09 (d, J = 8.8 Hz, 1H), 7.52 (dd, J = 5.2, 1.3 Hz, 1H), 7.29 - 7.23 (m, 2H), 6.81 - 6.75 (m, 2H), 5.20 - 5.08 (m, 2H), 3.77 - 3.69 (m, 1H), 3.65 (s, 3H), 2.39 - 2.24 (m, 3H), 1.14 - 1.06 (m, 5H), 1.05 - 0.98 (m, 2H), 0.97 - 0.85 (m, 4H). |

**Lithium salt formation**

Lithium (4-(carboxylato(methoxy)methyl)pyrimidin-2-yl)(cyclopropylsulfonyl)amide **INTC99**

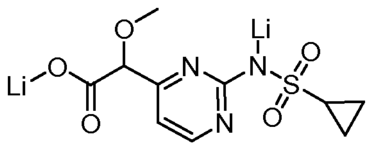

A stirred mixture of dimethyl 2-(2-(cyclopropanesulfonamido)pyrimidin-4-yl)-2-methoxymalonate (0.50 g, 1.25 mmol) **INTC75** in MeOH (15 mL) was treated with a solution of LiOH (0.10 g, 4.18 mmol) in water (5 mL). The reaction mixture was allowed to stir at RT for 2 hrs. The reaction mixture was concentrated *in vacuo* to afford lithium (4-(carboxylato(methoxy)methyl)pyrimidin-2-yl)(cyclopropylsulfonyl)amide (0.4 g, 1.19 mmol, 95% yield) as a brown solid. Rt 0.18 mins (UPLC basic); *m/z* 288 as free acid $(M+H)^+$ $(ES^+)$; $^1$H NMR not collected.

**Pyridine Core section**

**Sulfonation via Sulfonyl Chlorides**

Ethyl 2-(6-(cyclopropanesulfonamido)pyridin-2-yl)acetate **INTC102**

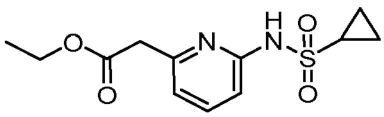

A solution of ethyl 2-(6-aminopyridin-2-yl)acetate (2 g, 11.10 mmol) and DMAP (0.136 g, 1.11 mmol) in pyridine (9.0 mL) was cooled to 0 °C. Cyclopropanesulfonyl chloride (1.12 mL, 11.10 mmol) was then added dropwise. The solution was allowed to slowly warm to RT and stirred for 18 hrs. The reaction mixture was quenched with MeOH (10

mL) and concentrated *in vacuo.* The crude product was purified by chromatography on the C18-RP silica gel (80 g column, 0-50% MeCN/Water 0.1% formic acid) to afford ethyl 2-(6-(cyclopropanesulfonamido)pyridin-2-yl)acetate (1.35 g, 4.70 mmol, 42% yield) as a pale brown oil; Rt 0.95 mins (UPLC acidic); *m/z* 285 $(M+H)^+$ $(ES^+)$. No NMR data collected.

**Alkylation**

Ethyl 2-(6-bromopyridin-2-yl)-2-methylpropanoate **INTC103**

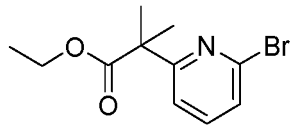

*t*-BuOK (0.115 g, 1.02 mmol) was added to a stirred, ice-cooled solution of ethyl 2-(6-bromopyridin-2-yl)acetate (0.100 g, 0.41 mmol) in THF (1.5 mL). After 30 min, MeI (2M in TBME, 0.82 mL, 1.64 mmol) was added dropwise. The reaction vessel was warmed to RT and stirred for 18 hrs. The reaction mixture was quenched with MeOH (1 mL) and concentrated *in vacuo.* The crude product was purified by chromatography on silica gel (24 g column, 0-50% EtOAc/iso-hexane) to afford ethyl 2-(6-bromopyridin-2-yl)-2-methylpropanoate (0.06 g, 0.21 mmol, 51% yield) as a clear, colourless liquid; Rt 1.54 mins (UPLC acidic); *m/z* 273 $(^{79}Br\ M+H)^+$ $(ES^+)$; $^1H$ NMR (400 MHz, DMSO-d6) δ 7.79 - 7.71 (m, 1H), 7.56 - 7.51 (m, 1H), 7.49 - 7.43 (m, 1H), 4.15 - 3.99 (m, 2H), 1.50 (s, 6H), 1.19 - 1.05 (m, 3H).

Ethyl 2-(6-bromopyridin-2-yl)butanoate **INTC104**

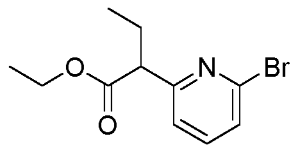

LiHMDS (1M in THF) (2.25 mL, 2.25 mmol) was added to a stirred solution of ethyl 2-(6-bromopyridin-2-yl)acetate (0.5 g, 2.05 mmol) in THF (10 mL) at -78°C. After 1 hr EtI (0.182 mL, 2.25 mmol) was added dropwise at the same temperature and the reaction was warmed to RT and stirred for 18 hrs. The reaction was partitioned between EtOAc (20 mL) and sat. $NH_4Cl$ (*aq*, 20 mL), the organic phase passed through a phase separator and the solvent was removed *in vacuo.* The crude product was purified by chromatography on silica gel (40 g cartridge, 0-50% EtOAc/*iso*-hexane) to afford ethyl 2-(6-bromopyridin-2-yl)butanoate (0.35 g, 1.27 mmol, 62% yield) as a pale yellow liquid. Rt 2.30 mins (HPLC acidic); *m/z* 272 $(^{79}Br\ M+H)^+$ $(ES^+)$; $^1H$ NMR (400 MHz, DMSO-d6) δ 7.76 - 7.72 (m, 1H), 7.55 (dd, J = 7.9, 0.9 Hz, 1H), 7.42 (dd, J = 7.6, 0.9 Hz, 1H), 4.12 - 4.05 (m, 2H), 3.74 (t, J = 7.5 Hz, 1H), 2.05 - 1.94 (m, 1H), 1.89 - 1.76 (m, 1H), 1.13 (t, J = 7.1 Hz, 3H), 0.83 (t, J = 7.4 Hz, 3H).

**Heterocycle formation via alkylation**

Ethyl 4-(6-bromopyridin-2-yl)tetrahydro-2*H*-pyran-4-carboxylate **INTC105**

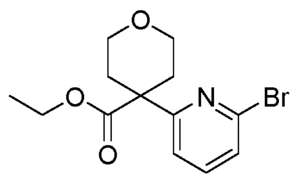

Prepared as for **INTC52** using commercial ethyl 2-(6-bromopyridin-2-yl)acetate (2.51 g, 10.28 mmol) and 1-bromo-2-(2-bromoethoxy)ethane to afford ethyl 4-(6-bromopyridin-2-yl)tetrahydro-2H-pyran-4-carboxylate (52% yield) as a clear oil. Rt 1.42 mins (UPLC basic); *m/z* 314 ($^{79}$Br M+H)$^+$ (ES$^+$); $^1$H NMR (400 MHz, DMSO-d6) δ 7.80 - 7.76 (m, 1H), 7.57 (d, J = 7.9 Hz, 1H), 7.49 (d, J = 7.7 Hz, 1H), 4.12 (q, J = 7.1 Hz, 2H), 3.77 - 3.70 (m, 2H), 3.52 - 3.45 (m, 2H), 2.30 - 2.23 (m, 2H), 2.07 - 2.01 (m, 2H), 1.12 (t, J = 7.1 Hz, 3H).

**Method I: Buchwald coupling - sulfonylation**

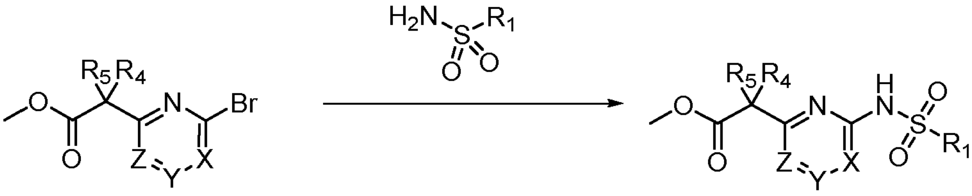

X = CH, N
Y = $CR_2$, N
Z = $CR_2$, N

2-Bromopyridine intermediate (1 eq), sulfonamide (1.2 eq) and base (2 eq) were dissolved in dioxane (40 volumes). The mixture was degassed ($N_2$, 5 mins) then catalyst (5 mol%) was added. The resulting mixture was heated under nitrogen at 90 °C for 2 hrs. The mixture was filtered, washing with EtOAc or DCM and the resulting filtrate was concentrated. The crude product was purified by normal phase chromatography.

**Table 7:** The following intermediates were made according to Method !.

| INTC | Name/Structure (All examples containing chiral centres are racemates unless stated) | Synthesis Method, [LCMS Method], m/z (M+H)$^+$, (Rt/min) | $^1$H NMR Chemical Shift Data (DMSO-d6 unless stated) | Catalyst , Base, Solvent |
|---|---|---|---|---|
| **INTC106** | ethyl 2-(6-(cyclopropanesulfonamido) pyridin -2-yl)-2-methylpropanoate 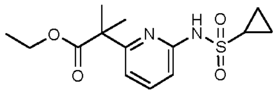 | Method I using INTC103, [UPLC acidic], 313 (1.27). | 10.48 (s, 1H), 7.75 - 7.61 (m, 1H), 7.08 - 6.95 (m, 1H), 6.85 - 6.74 (m, 1H), 4.12 - 3.97 (m, 2H), 3.19 - 3.07 (m, 1H), 1.49 (s, 6H), 1.14 - 0.95 (m, 7H). | Pd 174, $K_2CO_3$, dioxane |
| **INTC107** | ethyl 2-(6-(cyclopropanesulfonamido) pyridin -2-yl)butanoate 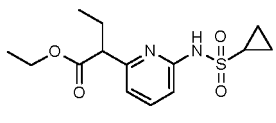 | Method I using INTC104, [HPLC acidic], 313 (1.97). | 10.50 (s, 1H), 7.69 (dd, J = 8.2, 7.5 Hz, 1H), 6.99 (d, J = 7.5 Hz, 1H), 6.85 (d, J = 8.2 Hz, 1H), 4.12 - 3.97 (m, 2H), 3.68 - 3.59 (m, 1H), 3.18 - 3.08 (m, 1H), 1.99 - 1.93 (m, 1H), 1.88 - 1.77 (m, 1H), 1.13 (t, J = 7.1 Hz, 3H), 1.10 - 1.05 (m, 2H), 1.03 - 0.94 (m, 2H), 0.84 (t, J = 7.4 Hz, 3H). | Pd 174, $Cs_2CO_3$, dioxane |
| **INTC108** | ethyl 4-(6-(cyclopropanesulfonamido) pyridin -2-yl)tetrahydro-2*H*-pyran-4-carboxylate 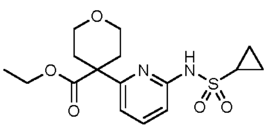 | Method I using INTC105, [HPLC acidic], 355 (1.78). | 10.55 (s, 1H), 7.74 - 7.70 (m, 1H), 7.05 (d, J = 7.7 Hz, 1H), 6.82 (d, J = 8.1 Hz, 1H), 4.10 (q, J = 7.1 Hz, 2H), 3.82-3.72 (m, 2H), 3.53-3.43 (m, 2H), 3.24-3.16 (m, 1H), 2.33-2.25 (m, 2H), 2.11-2.00 (m, 2H), 1.15 - 1.06 (m, 5H), 1.05 - 0.98 (m, 2H). | Pd 174, $Cs_2CO_3$, dioxane |

**Method J: Hydrolysis**

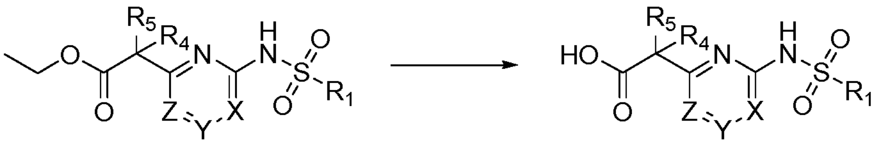

X = CH, N
Y = $CR_2$, N
Z = $CR_2$, N

2M LiOH (*aq*, 2 eq) was added into a solution of ester (1 eq) in MeOH (3 volumes) and THF (3 volumes) and the resulting reaction mixture was stirred at 50 °C for 2 hrs. The solvent was removed under reduced pressure and then was acidified with 1M HCl (aq) until pH 3. The solution was extracted with EtOAc, the organic phase was passed through a phase separator and the solvent was removed. The compound was used crude or purified by reverse phase chromatography.

**Table 8:** The following intermediates were made according to Method J.

| INT | Name/Structure (All examples containing chiral centres are racemates unless stated) | Synthesis Method, [LCMS Method], m/z $(M+H)^+$, (Rt/min) | $^1$H NMR Chemical Shift Data (DMSO-d6 unless stated) |
|---|---|---|---|
| **INTC109** | 2-(6-(cyclopropanesulfonamido)-pyridin-2-yl)-2-methylpropanoic acid 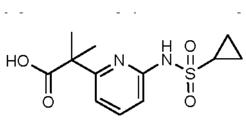 | Method J using INTC106, [UPLC acidic], 285 (0.94). | 12.25 (s, 1H), 10.46 (s, 1H), 7.71 - 7.66 (m, 1H), 7.03 - 6.98 (m 1H), 6.82 - 6.77 (m, 1H), 3.20 - 3.17 (m, 1H), 1.48 (s, 6H), 1.14 - 0.94 (m, 4H). |
| **INTC110** | 2-(6-(cyclopropanesulfonamido)-pyridin-2-yl)acetic acid 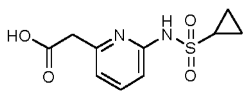 | Method J using INTC102, [UPLC acidic], 257 (0.61). | None recorded. |
| **INTC111** | 2-(6-(cyclopropanesulfonamido)-pyridin-2-yl)butanoic acid 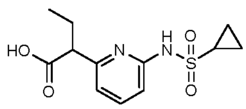 | Method J using INTC107, [HPLC acidic], 285 (0.90). | 12.34 (s, 1H), 10.49 (s, 1H), 7.72 - 7.65 (m, 1H), 6.99 - 6.95 (m, 1H), 6.89 - 6.83 (m, 1H), 3.56 - 3.52 (m, 1H), 3.17 - 3.07 (m, 1H), 1.99 - 1.93 (m, 1H), 1.86 - 1.75 (m, 1H), 1.13 - 0.91 (m, 4H), 0.86 - 0.81 (m 3H). |

**Pyrazine Core Section**

**Ester formation**

Methyl 2-(6-chloropyrazin-2-yl)acetate **INTC112**

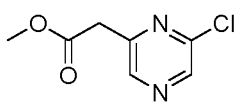

Thionyl chloride (1.15 mL, 15.65 mmol) was added dropwise into a stirring cold solution of 2-(6-chloropyrazin-2-yl)acetic acid (2.70 g, 15.65 mmol) in MeOH (50 mL) at 0°C. After addition the reaction mixture was stirred at RT for 1 hr. The reaction mixture was concentrated *in vacuo* and the crude residue was diluted with DCM (100 mL) and sequentially washed with sat. NaHCOs (aq, 2 × 100 mL), and brine (100 mL). The organic extract was dried ($MgSO_4$), filtered and solvent removed *in vacuo* to afford methyl 2-(6-chloropyrazin-2-yl)acetate (2.63 g, 13.67 mmol, 87% yield) as brown oil. Rt 1.25 min (HPLC, acidic); *m*/*z* 187 ($^{35}$Cl M+H)$^+$ (ES$^+$); $^1$H NMR (500 MHz, DMSO-d6) δ 8.74 (s, 1H), 8.68 (s, 1H), 4.00 (s, 2H), 3.66 (s, 3H).

Methyl 2-(6-chloropyrazin-2-yl)-2-methoxyacetate **INTC121**

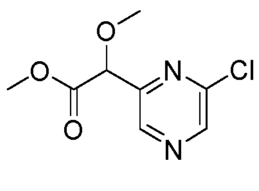

Prepared as for **INTC112** using 2-(6-chloropyrazin-2-yl)-2-methoxyacetic acid **INTC120** to afford methyl 2-(6-chloropyrazin-2-yl)-2-methoxyacetate (3.35 g, 15.31 mmol, 96% yield) as a clear yellow oil. Rt 1.33 min (HPLC, basic); *m*/*z* 217 ($^{35}$Cl M+H)$^+$ (ES$^+$), No NMR data recorded.

**Preparation of bi-ester intermediates**

1-*tert*-Butyl 3-methyl 2-(6-chloropyrazin-2-yl)malonate **INTC113**

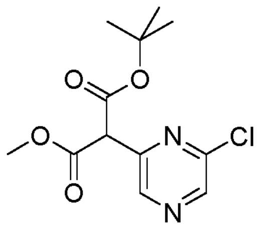

Prepared as for **INTC1** using commercial 2,6-dichloropyrazine to afford 1-*tert*-butyl 3-methyl 2-(6-chloropyrazin-2-yl)malonate (78% yield) as a clear colourless oil. Rt 2.18 min (HPLC, acidic); *m*/*z* 286 ($^{35}$Cl M+H)$^+$ (ES$^+$); $^1$H NMR (500 MHz, DMSO-d6) δ 8.82 (s, 1H), 8.72 (s, 1H), 5.28 (s, 1H), 3.73 (s, 3H), 1.44-1.37 (m, 9H).

Dimethyl 2-(6-chloropyrazin-2-yl)-2-methoxymalonate **INTC114**

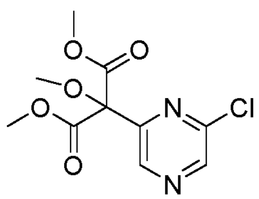

Prepared as for **INTC1** using dimethyl 2-methoxymalonate and 2,6-dichloropyrazine to afford dimethyl 2-(6-chloropyrazin-2-yl)-2-methoxymalonate (33% yield) as a clear colourless oil. Rt 1.65 min (HPLC, acidic); *m*/*z* 275 ($^{35}$Cl M+H)$^+$ (ES$^+$); $^1$H NMR (500 MHz, DMSO-d6) δ 8.89 (d, J = 0.6 Hz, 1H), 8.86 (d, J = 0.6 Hz, 1H), 3.79 (s, 6H), 3.46 (s, 3H).

**Alkylation of pyrazine intermediates**

**Table 9:** The following intermediates were made according to Method B which is described above.

| INTC | Name/Structure (All examples containing chiral centres are racemates unless stated) | Synthesis Method, [LCMS Method], m/z $(M+H)^+$, (Rt/min) | $^1$H NMR Chemical Shift Data (DMSO-d6 unless stated) | Base, RX, solvent |
|---|---|---|---|---|
| **INTC115** | 1-*tert*-Butyl 3-ethyl 2-(6-chloropyrazin-2-yl)-2-(2-methoxyethyl)malonate 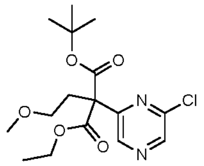 | Method B using INTC113 [UPLC Basic 2], 359 $^{35}$Cl isotope (0.72). | 8.88 (s, 1H), 8.77 (s, 1H), 4.28 - 4.13 (m, 2H), 3.29 (td, J = 6.4, 2.2 Hz, 2H), 3.10 (s, 3H), 2.46 (td, J = 6.3, 2.1 Hz, 2H), 1.40 (s, 9H), 1.20 (t, J = 7.1 Hz, 3H). | $K_2CO_3$, $MeOCH_2CH_2Br$, DMF |
| **INTC116** | Methyl 2-(6-chloropyrazin-2-yl)-2-methylpropanoate 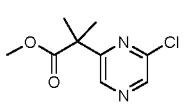 | Method B using INTC112 [HPLC acidic], 215 $^{35}$Cl isotope (1.88). | 8.79 (s, 1H), 8.73 (s, 1H), 3.63 (s, 3H), 1.58 (s, 6H). | $K_2CO_3$, MeI, acetone |
| **INTC117** | Methyl 2-(6-chloropyrazin-2-yl)butanoate 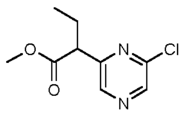 | Method B using commercial methyl 2-(6-chloropyrazin-2-yl) acetate [HPLC acidic], 215 $^{35}$Cl isotope (1.84). | 8.73 (s, 1H), 8.70 (s, 1H), 3.95 (dd, J = 8.1, 7.0 Hz, 1H), 3.62 (s, 3H), 2.13 - 2.01 (m, 1H), 1.96-1.84 (m, 1H), 0.83 (t, J = 7.4 Hz, 3H). | $K_2CO_3$, EtBr, acetone |
| **INTC119** | Ethyl 2-(6-chloropyrazin-2-yl)-4-methoxy-2-methylbutanoate 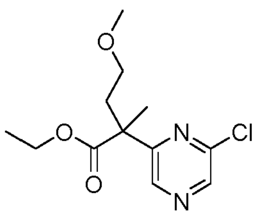 | Method B using INTC118, [UPLC, basic], 273 $^{35}$Cl isotope (1.32). | 8.74 (s, 1H), 8.71 (s, 1H), 4.08 (q, J = 7.1 Hz, 2H), 3.36 - 3.29 (m, 2H), 3.11 (s, 3H), 2.32 - 2.25 (m, 2H), 1.56 (s, 3H), 1.12 (t, J = 7.1 Hz, 3H). | NaH, MeI, THF |
| **INTC122** | Methyl 2-(6-chloropyrazin-2-yl)-2-methoxypropanoate 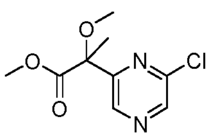 | Method B using INTC121, [HPLC, acidic], 230 $^{35}$Cl isotope (1.67). | 8.84 (s, 1H), 8.81 (s, 1H), 3.68 (s, 3H), 3.30 (s, 3H), 1.72 (s, 3H). | NaH, MeI, DMF |

**TFA decarboxylation**

Ethyl 2-(6-chloropyrazin-2-yl)-4-methoxybutanoate INTC118

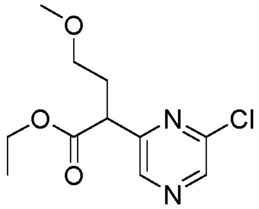

Prepared by **Method A** using *1-tert*-butyl 3-ethyl 2-(6-chloropyrazin-2-yl)-2-(2-methoxyethyl)malonate **INTC115** to afford ethyl 2-(6-chloropyrazin-2-yl)-4-methoxybutanoate (2.49 g, 8.65 mmol, 71% yield) as a pale purple oil. Rt 0.59 min (UPLC, basic 2); m/z 259 ($^{35}$Cl M+H)$^+$ (ES$^+$); $^1$H NMR (500 MHz, DMSO-d6) δ 8.73 (s, 1H), 8.69 (s, 1H), 4.16 - 4.05 (m, 3H), 3.37 - 3.29 (m, 1H), 3.28 - 3.20 (m, 1H), 3.16 (s, 3H), 2.35 - 2.24 (m, 1H), 2.16 - 2.07 (m, 1H), 1.13 (t, J = 7.1 Hz, 3H).

**Hydrolysis**

2-(6-chloropyrazin-2-yl)-2-methoxyacetic acid **INTC120**

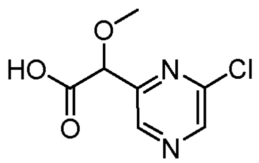

A stirred mixture of dimethyl 2-(6-chloropyrazin-2-yl)-2-methoxymalonate **INTC114** (4.54 g, 16.53 mmol) in THF (40 mL) and water (10 mL) was treated with 2M NaOH (*aq,* 4 mL, 8.00 mmol). The reaction mixture was allowed to stir at RT for 66 hrs. Further 2M NaOH (aq, 5 eq) was added and the mixture stirred for 2 hrs. The reaction mixture was concentrated *in vacuo* and the crude product was purified by chromatography on RP Flash C18 (80 g column, 5-50% MeCN/10 mM ammonium bicarbonate) to afford 2-(6-chloropyrazin-2-yl)-2-methoxyacetic acid (3.67 g, 16.30 mmol, 99% yield) as a white solid. Rt 1.06 min (HPLC acidic); *m/z* 203 ($^{35}$Cl M+H)$^+$ (ES$^+$); $^1$H NMR (500 MHz, DMSO-d6) δ 8.63 (s, 2H), 4.45 (s, 1H), 3.26 (s, 3H). One exchangeable proton not observed.

2-(6-Chloropyrazin-2-yl)-4-methoxy-2-methylbutanoic acid **INTC131**

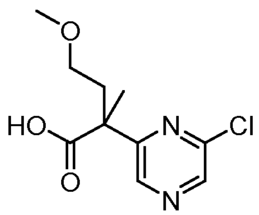

To a solution of ethyl 2-(6-chloropyrazin-2-yl)-4-methoxy-2-methylbutanoate **INTC119** (1.93 g, 7.08 mmol) in EtOH (2 mL) and THF (15 mL) was added a solution of LiOH (0.203 g, 8.49 mmol) in water (5 mL). The reaction was stirred at RT for 18 hrs. Further LiOH (0.068 g, 2.83 mmol) in water (5 mL) was added and the reaction mixture was stirred at RT for 4 hrs. The reaction mixture was concentrated *in vacuo* and the resulting residue was acidified using 1M HCl (50 mL). The product was extracted using EtOAc (3 × 50 mL), the combined organics were dried ($MgSO_4$) and concentrated *in vacuo* to give 2-(6-chloropyrazin-2-yl)-4-methoxy-2-methylbutanoic acid (1.92 g, 5.98 mmol, 84% yield) as a dark brown oil which was used without further purification. Rt 0.95 min (UPLC, acidic); *m/z* 245 ($^{35}$Cl M+H)$^+$ (ES$^+$); $^1$H NMR (500 MHz, DMSO-d6) δ 12.59 (s, 1H), 8.74 (s, 1H), 8.70 (s, 1H), 3.36 - 3.27 (m, 2H), 3.11 (s, 3H), 2.34 - 2.18 (m, 2H), 1.56 (s, 3H).

2-(6-(Cyclopropanesulfonamido)pyrazin-2-yl)butanoic acid **INTC132**

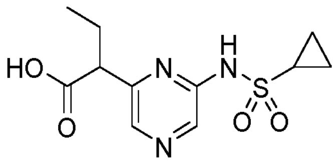

Prepared as for **INTC131** using methyl 2-(6-(cyclopropanesulfonamido)pyrazin-2-yl)butanoate **INTC126** to afford 2-(6-(cyclopropanesulfonamido)pyrazin-2-yl)butanoic acid (59% yield) as a colourless gum. Rt 1.35 min (HPLC, acidic); *m/z* 286 $(M+H)^+$ $(ES^+)$; $^1H$ NMR (500 MHz, DMSO-d6) δ 12.54 (s, 1H), 11.00 (s, 1H), 8.27 (s, 1H), 8.21 (s, 1H), 3.70-3.65 (m, 1H), 3.10-3.03 (m, 1H), 2.09 - 1.98 (m, 1H), 1.91 - 1.79 (m, 1H), 1.21 - 0.98 (m, 4H), 0.84 (t, J = 7.4 Hz, 3H).

2-(6-(Cyclopropanesulfonamido)pyrazin-2-yl)-2-fluorobutanoic acid **INTC133**

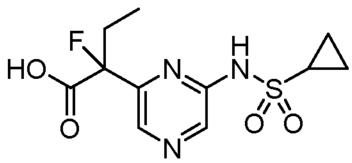

Prepared as for **INTC131** using methyl 2-(6-(cyclopropanesulfonamido)pyrazin-2-yl)-2-fluorobutanoate **INTC130** to afford 2-(6-(cyclopropanesulfonamido)pyrazin-2-yl)-2-fluorobutanoic acid (95% yield) as a thick red paste. Rt 0.89 min (UPLC, acidic); *m/z* 304 $(M+H)^+$ $(ES^+)$; $^1H$ NMR (500 MHz, DMSO-d6) δ 13.74 (s, 1H), 11.23 (s, 1H), 8.45 (s, 1H), 8.33 (s, 1H), 3.15 3.09 (m, 1H), 2.45 - 2.21 (m, 2H), 1.21 - 1.15 (m, 1H), 1.15 - 0.97 (m, 3H), 0.92 (t, J = 7.4 Hz, 3H).

2-(6-(Cyclopropanesulfonamido)pyrazin-2-yl)-2-methoxyacetic acid **INTC134**

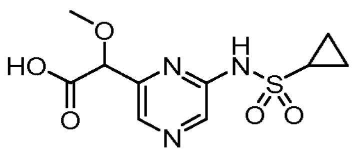

Prepared as for **INTC131** using methyl 2-(6-(cyclopropanesulfonamido)pyrazin-2-yl)-2-methoxyacetate **INTC128** to afford 2-(6-(cyclopropanesulfonamido)pyrazin-2-yl)-2-methoxyacetic acid (24% yield) as a tan solid. Rt 1.06 min (HPLC, acidic); *m/z* 288 $(M+H)^+$ $(ES^+)$; $^1H$ NMR (500 MHz, DMSO-d6) δ 11.13 (s, 1H), 8.36 (s, 1H), 8.32 (s, 1H), 6.78 (s, 1H), 4.94 (s, 1H), 3.40 (s, 3H), 3.12-3.02 (m, 1H), 1.16 -1.10 (m, 2H), 1.07-0.98 (m, 2H).

**Heterocycle formation via alkylation**

Methyl 4-(6-chloropyrazin-2-yl)tetrahydro-2*H*-pyran-4-carboxylate I**NTC123**

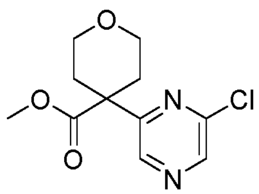

Prepared as for **INTC52** using methyl 2-(6-chloropyrazin-2-yl)acetate **INTC112** to afford methyl 4-(6-chloropyrazin-2-yl)tetrahydro-2*H*-pyran-4-carboxylate (12% yield) as a yellow oil. Rt 1.05 min (UPLC, acidic); *m/z* 257 ($^{35}$Cl M+H)$^+$ (ES$^+$); $^1$H NMR (500 MHz, DMSO-d6) δ 8.81 (s, 1H), 8.76 (s, 1H), 3.77 - 3.62 (m, 5H), 3.58 - 3.49 (m, 2H), 2.38 - 2.26 (m, 2H), 2.21 - 2.10 (m, 2H).

**Fluorination of pyrazine intermediates**

**Table 10:** The following intermediates were made according to Method H which is described above.

| INTC | Name/Structure (All examples containing chiral centres are racemates unless stated) | Synthesis Method, [LCMS Method], m/z (M+H)$^+$, (Rt/min) | $^1$H NMR Chemical Shift Data (DMSO-d6 unless stated) |
|---|---|---|---|
| **INTC124** | methyl 2-(6-chloropyrazin-2-yl)-2-fluorobutanoate 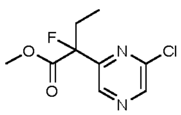 | Method H using INTC117, [UPLC basic], no m/z collected (1.22). | 8.91 (s, 1H), 8.90 (s, 1H), 3.76 (s, 3H), 2.48 - 2.24 (m, 2H), 0.91 (t, J = 7.4 Hz, 3H). |

**Amide formation of selected building blocks**

**Table 11:** The following intermediates were made using methods analogous to Methods 1-10 which are described below for the synthesis of compound of formula (I).

| INTC | Name/Structure (All examples containing chiral centres are racemates unless stated) | Synthesis Method, [LCMS Method], m/z (M+H)$^+$, (Rt/min) | $^1$H NMR Chemical Shift Data (DMSO-d6 unless stated) |
|---|---|---|---|
| **INTC135** | 2-(6-chloropyrazin-2-yl)-N-(4-(pyridin-3-yl)phenyl) acetamide 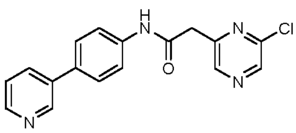 | Method 1 using commercial starting materials, [HPLC acidic],325 $^{35}$Cl isotope (1.11). | 10.48 (s, 1H), 8.89 (dd, J = 2.5, 0.9 Hz, 1H), 8.73 (s, 1H), 8.71 (s, 1H), 8.54 (dd, J = 4.8, 1.6 Hz, 1H), 8.06 (ddd, J = 8.1, 2.5, 1.6 Hz, 1H), 7.79 - 7.60 (m, 4H), 7.47 (ddd, J = 8.1, 4.8, 0.9 Hz, 1H), 4.01 (s, 2H). |
| **INTC136** | 2-(6-chloropyrazin-2-yl)-N-(5-(6-ethoxypyrazin-2-yl)pyridin-2-yl)-4-methoxy-2-methylbutanamide 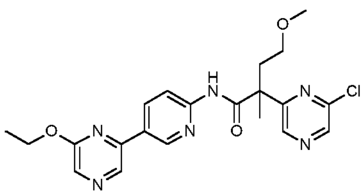 | Method 8 using INTC131 and INTD33, [UPLC acidic 2],443 $^{35}$Cl isotope (0.69). | 10.32 (s, 1H), 9.02 (dd, J = 2.5, 0.8 Hz, 1H), 8.84 (s, 1H), 8.74 (s, 1H), 8.72 (s, 1H), 8.50 (dd, J = 8.8, 2.5 Hz, 1H), 8.25 (s, 1H), 8.22 (dd, J = 8.8, 0.8 Hz, 1H), 4.48 (q, J = 7.0 Hz, 2H), 3.40 - 3.33 (m, 2H), 3.10 (s, 3H), 2.47 - 2.32 (m, 2H), 1.68 (s, 3H), 1.40 (t, J = 7.0 Hz, 3H). |

(continued)

| INTC | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| **INTC137** | 2-(6-chloropyrazin-2-yl)-N-(5-(6-ethoxypyrazin-2-yl)pyridin-2-yl)-2-methoxyacetamide 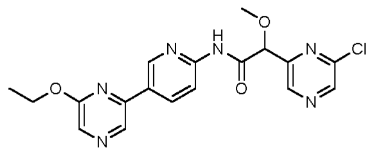 | Method 3 using INTC120 and INTD33, [HPLC acidic], 401 $^{35}Cl$ isotope (2.20). | 10.85 (s, 1H), 9.12 (dd, J = 2.4, 0.8 Hz, 1H), 8.91 - 8.81 (m, 3H), 8.54 (dd, J = 8.7, 2.5 Hz, 1H), 8.27 (s, 1H), 8.24 - 8.16 (m, 1H), 5.33 (s, 1H), 4.49 (q, J = 7.1 Hz, 2H), 3.48 (s, 3H), 1.40 (t, J = 7.0 Hz, 3H). |

**2,6-Pyrimidine core**

N-(2-Chloropyrimidin-4-yl)cyclopropanesulfonamide **INTC138**

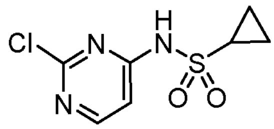

To a suspension of 2,4-dichloropyrimidine (15 g, 101 mmol) in acetonitrile (250 mL) was added cyclopropanesulfonamide (14.64 g, 121 mmol) and $K_2CO_3$ (27.8 g, 201 mmol). The resulting mixture was allowed to stir under reflux for 18 hrs. The mixture was poured slowly into ice cold 6M HCl (*aq,* 100 mL) under vigorous stirring, then diluted with EtOAc (100 mL). The bi-phasic mixture was filtered, the phases were separated and the aqueous phase was extracted with EtOAc (3 × 50 mL). The combined organic layers were dried over $Na_2SO_4$, filtered, and concentrated onto silica then purified by chromatography on silica gel (330 g column, 50-100% EtOAc/iso-hexane) to afford a white solid. The solid was triturated from water (50 mL) then azeotroped with MeCN (30 mL) *in vacuo* to afford N-(2-chloropyrimidin-4-yl)cyclopropanesulfonamide (11.68 g, 49.5 mmol, 49% yield) as a white solid. Rt 0.79 min (UPLC, acidic); m/z 234 ($^{35}Cl$ $M+H)^+$ ($ES^+$); $^1H$ NMR (500 MHz, DMSO-d6) $\delta$ 11.68 (s, 1H), 8.60 (d, J = 5.3 Hz, 1H), 7.32 (d, J = 5.3 Hz, 1H), 3.11-3.06 (m, 1H), 1.30 - 0.94 (m, 4H).

N-(2-Chloropyrimidin-4-yl)-*N*-(4-methoxybenzyl)cyclopropanesulfonamide **INTC139**

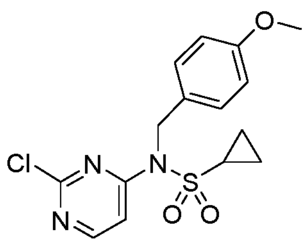

To a stirred solution of *N*-(2-chloropyrimidin-4-yl)cyclopropanesulfonamide **INTC138** (11.68 g, 50.0 mmol) in DMF (50 mL) was successively added $K_2CO_3$ (13.82 g, 100 mmol) and 1-(chloromethyl)-4-methoxybenzene (8.13 mL, 60.0 mmol) at RT. The reaction mixture was stirred at RT for 3 hrs then heated at 40°C for 18 hrs. Additional 1-(chloromethyl)-4-methoxybenzene (2.03 mL, 15.0 mmol) was added and the resulting mixture was stirred at 40°C for 18 hrs. The mixture was cooled down to RT and poured into water (200 mL) and diluted with EtOAc (100 mL). The phases were separated and the aqueous layer was extracted with EtOAc (3 × 50 mL). The combined organic layers were dried over

$Na_2SO_4$, filtered, and the solvent was removed *in vacuo.* The crude product was purified by chromatography on silica gel (330 g column, 0-50% EtOAc/isohexane) to afford a white solid. The solid was triturated from MeCN (20 mL) to afford N-(2-chloropyrimidin-4-yl)-*N*-(4-methoxybenzyl)cyclopropanesulfonamide (8.4 g, 23.50 mmol, 47% yield) as a white powder. Rt 1.41 min (UPLC, basic); m/z 354 ($^{35}$Cl M+H)$^+$ (ES$^+$); $^1$H NMR (500 MHz, DMSO-d6) $\delta$ 8.54 (d, J = 5.9 Hz, 1H), 7.42 (d, J = 5.9 Hz, 1H), 7.28 (d, J = 8.7 Hz, 2H), 6.91 (d, J = 8.7 Hz, 2H), 5.11 (s, 2H), 3.73 (s, 3H), 3.32 - 3.26 (m, 1H), 1.23 - 1.08 (m, 4H).

1-*tert*-Butyl 3-ethyl 2-(4-(*N*-(4-methoxybenzyl)cyclopropanesulfonamido)pyrimidin-2-yl)malonate **INTC140**

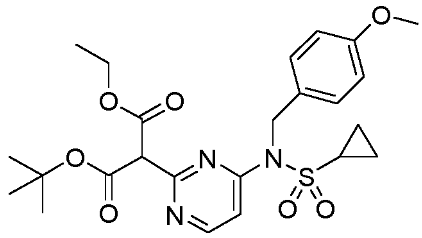

To a solution of *tert*-butyl ethyl malonate (1.41 mL, 7.46 mmol) in DME (25 mL) was successively added $Cs_2CO_3$ (4.86 g, 14.92 mmol) and *N*-(2-chloropyrimidin-4-yl)-*N*-(4-methoxybenzyl)cyclopropanesulfonamide **INTC139** (2.4 g, 6.78 mmol) and the resulting mixture was heated at 90°C for 24 hrs. The reaction mixture was cooled to RT and poured into sat. $NH_4Cl$ (*aq*, 100 mL) and diluted with EtOAc (50 mL). The phases were separated and the aqueous layer was extracted with EtOAc (3 $\times$ 30 mL). The combined organic layers were dried over $Na_2SO_4$, filtered, and the solvent was removed *in vacuo.* The crude product was purified by chromatography on silica gel (220 g column, 0-70% EtOAc/isohexane) to afford 1-*tert*-butyl 3-ethyl 2-(4-(*N*-(4-methoxybenzyl)cyclopropanesulfonamido)pyrimidin-2-yl)malonate (1.67 g, 3.14 mmol, 46% yield) as a yellow oil. Rt 1.67 min (UPLC, acidic); m/z 507 (M+H)$^+$ (ES$^+$); $^1$H NMR (500 MHz, DMSO-d6) $\delta$ 8.59 (d, J = 6.0 Hz, 1H), 7.33 (d, J = 6.0 Hz, 1H), 7.29 - 7.22 m, 2H), 6.89 - 6.84 (m, 2H), 5.17 - 5.07 (m, 2H), 4.99 (s, 1H), 4.14 (q, J = 7.2 Hz, 2H), 3.71 (s, 3H), 3.31 - 3.26 (m, 1H), 1.39 (s, 9H), 1.19 - 1.14 (m, 3H), 1.14 - 0.98 (m, 4H).

1-tert-Butyl 3-ethyl 2-ethyl-2-(4-(*N*-(4-methoxybenzyl)cyclopropanesulfonamido)pyrimidin-2-yl)malonate **INTC141**

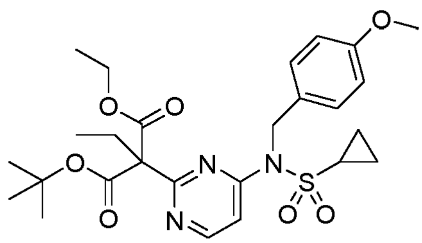

To a solution of 1-*tert*-butyl 3-ethyl 2-(4-(*N*-(4-methoxybenzyl) cyclopropanesulfonamido)pyrimidin-2-yl)malonate **INTC140** (2.96 g, 5.85 mmol) in DMF (40 mL) was successively added $K_2CO_3$ (1.780 g, 12.88 mmol) and EtI (0.52 mL, 6.44 mmol). The resulting mixture was vigorously stirred at 60°C for 2 hrs. The reaction mixture was cooled to RT and poured into sat. $NH_4Cl$ (*aq*, 150 mL) and diluted with EtOAc (50 mL). The phases were separated and the aqueous layer was extracted with EtOAc (2 $\times$ 30 mL). The combined organic layers were washed with half saturated brine (50 mL), dried over $Na_2SO_4$, filtered, and the solvent was removed *in vacuo.* The crude product was purified by chromatography on silica gel (120 g column, 0-50% EtOAc/iso-hexane) to afford 1-*tert*-butyl 3-ethyl 2-ethyl-2-(4-(*N*-(4-methoxybenzyl)cyclopropanesulfonamido)pyrimidin-2-yl)malonate (2.63 g, 4.39 mmol, 75% yield) as a light yellow oil. Rt 1.82 min (UPLC, acidic); m/z 534 (M+H)$^+$ (ES$^+$); $^1$H NMR (500 MHz, DMSO-d6) $\delta$ 8.59 (d, J = 5.9 Hz, 1H), 7.30 (d, J = 5.9 Hz, 1H), 7.25-7.00 (m, 2H), 6.89-6.84 (m, 2H), 5.16-5.07 (m, 2H), 4.18 - 4.11 (m, 1H), 4.11 -4.01 (m, 1H), 3.71 (s, 3H), 3.30 - 3.25 (m, 1H), 2.27 - 2.13 (m, 2H), 1.35 (s, 9H), 1.13 (t, J = 7.1 Hz, 3H), 1.10 - 0.97 (m, 4H), 0.92 (t, J = 7.3 Hz, 3H).

Ethyl 2-(4-(N-(4-methoxybenzyl)cyclopropanesulfonamido)pyrimidin-2-yl)butanoate **INTC142**

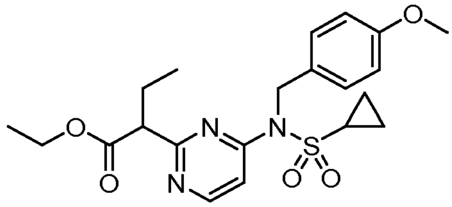

To a solution of 1-*tert*-butyl 3-ethyl 2-ethyl-2-(4-(*N*-(4-methoxybenzyl)cyclopropanesulfonamido)pyrimidin-2-yl)malonate **INTC141** (3.0 g, 5.06 mmol) in DCM (40 mL) was added TFA (15.59 mL, 202 mmol). The resulting solution was allowed to stir at RT for 18 hrs. The solution was poured into sat. NaHCOs (aq, 200 mL) and diluted with DCM (50 mL). The phases were separated and the aqueous layer was extracted with DCM (2 × 50 mL). The pH was readjusted to 4 with 12 M HCl and the aqueous layer was extracted with DCM (3 × 50 mL). The combined organic layers were dried over $Na_2SO_4$, filtered, and the solvent was removed *in vacuo.* The crude product was purified by chromatography on silica gel (120 g column, 0-100% EtOAc/*iso*-hexane) to afford ethyl 2-(4-(*N*-(4-methoxybenzyl)cyclopropanesulfonamido)pyrimidin-2-yl)butanoate (1.74 g, 3.85 mmol, 76% yield) as a colourless oil. Rt 1.56 min (UPLC, acidic); m/z 434 $(M+H)^+$ $(ES^+)$; $^1H$ NMR (500 MHz, DMSO-d6) δ 8.55 (d, J = 5.9 Hz, 1H), 7.34 - 7.21 (m, 3H), 6.90-6.86 (m, 2H), 5.15-5.06 (m, 2H), 4.05 - 4.00 (m, 2H), 3.78 (t, J = 7.4 Hz, 1H), 3.71 (s, 3H), 1.98 - 1.87 (m, 2H), 1.13 - 1.01 (m, 7H), 0.82 (t, J = 7.4 Hz, 3H). One CH proton obscured by DMSO peak.

*N*-(5-(6-ethoxypyrazin-2-yl)pyridin-2-yl)-2-(4-(*N*-(4-methoxybenzyl)cyclopropanesulfonamido)pyrimidin-2-yl)butanamide **INTC143**

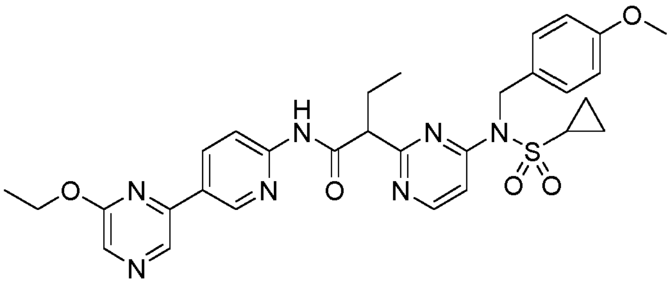

Prepared using **Method 2** using 5-(6-ethoxypyrazin-2-yl)pyridin-2-amine **INTD33** (449 mg, 2.08 mmol) and ethyl 2-(4-(*N*-(4-methoxybenzyl)cyclopropanesulfonamido)pyrimidin-2-yl)butanoate **INTC142** to afford *N*-(5-(6-ethoxypyrazin-2-yl)pyridin-2-yl)-2-(4-(N-(4-methoxybenzyl)cyclopropanesulfonamido) pyrimidin-2-yl)butanamide (11% yield) as a colourless oil. Rt 1.70 min (UPLC, acidic); *m/z* 604 $(M+H)^+$ $(ES^+)$; $^1H$ NMR (500 MHz, DMSO-d6) δ 10.99 (s, 1H), 9.07 (d, J = 2.5 Hz, 1H), 8.85 (s, 1H), 8.58 (d, J = 5.9 Hz, 1H), 8.49 (dd, J = 8.8, 2.5 Hz, 1H), 8.25 (s, 1H), 8.24 (d, J = 8.8 Hz, 1H), 7.26 - 7.21 (m, 3H), 6.79 - 6.74 (m, 2H), 5.12 (d, J = 16.3 Hz, 1H), 5.08 (d, J = 16.3 Hz, 1H), 4.49 (q, J = 7.0 Hz, 2H), 4.24 - 4.15 (m, 1H), 3.64 (s, 3H), 3.42 - 3.37 (m, 1H), 2.15 - 2.02 (m, 2H), 1.41 (t, J = 7.0 Hz, 3H), 1.10 - 1.05 (m, 2H), 1.00 - 0.96 (m, 2H), 0.94 (t, J = 7.4 Hz, 3H).

**Benzamide Pyrimidine Intermediates**

**Sulfonylation**

*N*-(4-cyanopyrimidin-2-yl)cyclopropanesulfonamide **INTC144**

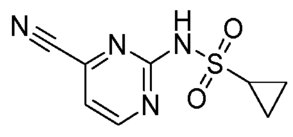

Prepared following **Method C** using 2-chloropyrimidine-4-carbonitrile, cyclopropanesulfonamide with $Cs_2CO_3$, tBuXPhos and $[Pd(allyl)Cl]_2$ in dioxane to afford *N*-(4-cyanopyrimidin-2-yl)cyclopropanesulfonamide (88% yield) as a pale orange solid. Rt 0.70 mins (UPLC acidic); *m/z* 225 $(M)^+$ $(ES^+)$; $^1H$ NMR (500 MHz, DMSO-d6) $\delta$ 11.87 (s, 1H), 8.95 (d, J = 4.9 Hz, 1H), 7.75 (d, J = 4.9 Hz, 1H), 3.23 - 3.14 (m, 1H), 1.19 - 1.04 (m, 4H).

**Nitrile Reduction**

*tert*-Butyl ((2-(cyclopropanesulfonamido)pyrimidin-4-yl)methyl)carbamate **INTC145**

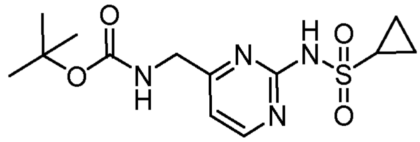

To a suspension of *N*-(4-cyanopyrimidin-2-yl)cyclopropanesulfonamide **INTC144** (0.5 g, 2.23 mmol) in MeOH (20 mL) at 0 °C was added di-*tert*-butyl dicarbonate (0.973 g, 4.46 mmol) followed by nickel (II) chloride hexahydrate (0.029 g, 0.223 mmol). $NaBH_4$ (0.675 g, 17.8 mmol) was then added portionwise over 30 mins, each portion only added once the previous had stopped effervescing. The reaction mixture was stirred at RT for 18 hrs. The reaction was quenched by addition of *N*-(2-aminoethyl)-1,2-ethanediamine (0.5 mL, 4.50 mmol) and stirred for 1.5 hrs at RT. The reaction mixture was concentrated to dryness and the resulting orange residue was dissolved in EtOAc (50 mL) and water (50 mL). The phases were separated, the aqueous (pH 8) was neutralised using sat. $NH_4Cl$ (aq, 50 mL) and the product was extracted using EtOAc (50 mL). The aqueous was further acidified to pH 4 by portion wise addition of 1M HCl (aq). The product was extracted using EtOAc (50 mL). The combined organics were dried (phase separator) and concentrated *in vacuo.* The crude product was concentrated onto silica and was purified by chromatography on silica gel (24 g column, 0-100% EtOAc/iso-hexane) to afford tert-butyl ((2-(cyclopropanesulfonamido)pyrimidin-4-yl)methyl)carbamate (85 mg, 0.207 mmol, 9% yield) as a clear colourless glass; Rt 0.98 mins (UPLC acidic); *m/z* 350 $(M+Na)^+$ $(ES^+)$; $^1H$ NMR (500 MHz, DMSO-d6) $\delta$ 8.62 (d, J = 4.9 Hz, 1H), 8.55 (d, J = 5.2 Hz, 1H), 7.52 -7.48 (m, 1H), 6.96 (d, J = 5.2 Hz, 1H), 4.14 (d, J = 6.1 Hz, 2H), 3.30 - 3.19 (m, 1H), 1.41 (s, 9H), 1.15 - 1.05 (m, 2H), 1.08 - 1.00 (m, 2H).

**Alkylation**

Methyl 1-(2-chloropyrimidin-4-yl)cyclopropanecarboxylate **INTC146**

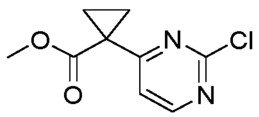

To a solution of methyl 2-(2-chloropyrimidin-4-yl)acetate (3 g, 16.08 mmol) in DMF (40 mL) was added NaOH (1.93 g, 48.2 mmol). The resulting mixture was allowed to stir for 15 min at RT before 1,2-dibromoethane (2.77 mL, 32.2 mmol) was added dropwise and allowed to stir at RT for 3 hrs. The mixture was poured into sat. $NH_4Cl$ (aq, 100 mL) and diluted with EtOAc (40 mL). The phases were separated and the aqueous phase was extracted with EtOAc (2 × 40 mL). The combined organic layers were dried ($Na_2SO_4$), filtered, and the solvent was removed *in vacuo.*

The crude product was purified by chromatography on silica gel (120 g column, 0-50% EtOAc/isohexane) to afford methyl 1-(2-chloropyrimidin-4-yl)cyclopropanecarboxylate (1.78 g, 8.12 mmol, 51% yield) as a colourless oil. Rt 1.05 min (UPLC, basic); *m/z* 213 $(M+H)^+$ $(ES^+)$; $^1H$ NMR (500 MHz, DMSO-d6) $\delta$ 8.70 (d, J = 5.2 Hz, 1H), 7.88 (d, J = 5.2 Hz, 1H), 3.67 (s, 3H), 1.68 - 1.63 (m, 2H), 1.59 (dt, J = 5.1, 2.9 Hz, 2H).

**Hydrolysis**

1-(2-Chloropyrimidin-4-yl)cyclopropanecarboxylic acid **INTC147**

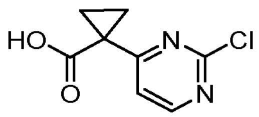

Prepared by **Method J** using methyl 1-(2-chloropyrimidin-4-yl)cyclopropanecarboxylate **INTC146** to afford 1-(2-chloropyrimidin-4-yl)cyclopropanecarboxylic acid (quantitative yield) as a colourless solid. Rt 0.83 min (UPLC acidic); m/z 199 $(M+H)^+$ $(ES^+)$. No NMR data recorded.

**Curtius**

*tert*-Butyl (1-(2-chloropyrimidin-4-yl)cyclopropyl)carbamate **INTC148**

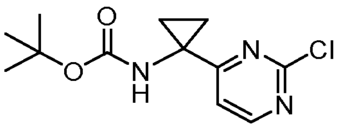

To a solution of 1-(2-chloropyrimidin-4-yl)cyclopropanecarboxylic acid **INTC147** (1.85 g, 9.31 mmol) in *tert*-butanol (15 mL) and toluene (15 mL) were successively added $Et_3N$ (1.49 mL, 10.3 mmol) and DPPA (2.23 mL, 9.78 mmol). The resulting mixture was allowed to stir at 90°C for 4 hrs. The mixture was cooled to RT and diluted with sat. NaHCOs (aq, 50 mL) and EtOAc (30 mL). The phases were separated and the aqueous layer was extracted with EtOAc (3 × 20 mL). The combined organic layers were dried ($Na_2SO_4$), filtered, and the solvent was removed *in vacuo.* The crude product was purified by chromatography on silica gel (120 g column, 0-50% EtOAc/isohexane) to afford *tert*-butyl (1-(2-chloropyrimidin-4-yl)cyclopropyl)carbamate (1.02 g, 3.33 mmol, 36% yield) as a colourless solid. Rt 1.26 min (UPLC acidic); *m/z* 270 $(M+H)^+$ $(ES^+)$. $^1H$ NMR (500 MHz, DMSO-d6) δ 8.63 (d, J = 5.3 Hz, 1H), 7.91 (s, 1H), 7.38 (d, J = 5.3 Hz, 1H), 1.42 (s, 9H), 1.35 - 1.21 (m, 4H).

1-(2-Bromopyrimidin-4-yl)propan-1-one **INTC149**

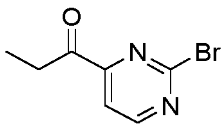

A solution of 2-bromopyrimidine (17.91 g, 113 mmol) in anhydrous THF (150 mL) was cooled to -60 °C. 1M Lithium magnesium 2,2,6,6-tetramethylpiperidin-1-ide dichloride (in THF/Toluene) (180 mL, 169 mmol) was added dropwise over 1 hr. The resulting solution was stirred at -55 °C for 3 hrs then a solution of *N*-methoxy-*N*-methylpropionamide (11 g, 94 mmol) in anhydrous THF (20 mL) was added dropwise to the resulting suspension. The reaction mixture was warmed to - 40 °C then after 30 min allowed to warm slowly from -40 °C to RT over 18 hrs. The reaction mixture was cooled in an ice bath then quenched carefully with dropwise addition of 5% citric acid (aq, 80 mL). The mixture was diluted with brine (150 mL) and the organic phase separated. The aqueous phase was further extracted with DCM (3 × 100 mL), the combined organic phases dried ($MgSO_4$), filtered then concentrated *in vacuo.* The crude product was purified by chromatography on silica gel (330 g column, 0-10% EtOAc/iso-hexane) to afford 1-(2-bromopyrimidin-4-yl)propan-1-one (11.39 g, 50.8 mmol, 54% yield) as a yellow solid. Rt 1.74 min (HPLC basic); m/z 215 $(^{79}Br\ M+H)^+$ $(ES^+)$. $^1H$ NMR (500 MHz, DMSO-d6) δ 8.97 (d, J = 4.9 Hz, 1H), 7.95 (d, J = 4.9 Hz, 1H), 3.12 (q, J = 7.1 Hz, 2H), 1.09

(t, J = 7.1 Hz, 3H).

*N*-(1-(2-Bromopyrimidin-4-yl)propylidene)-2-methylpropane-2-sulfinamide **INTC150**

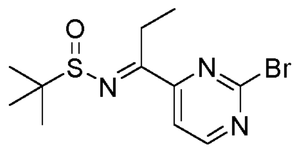

Ti(O-*i*-Pr)$_4$ (30.1 ml, 103 mmol) was added to a mixture of (R)-2-methylpropane-2-sulfinamide (7.3 g, 60.2 mmol), (S)-2-methylpropane-2-sulfinamide (5.9 g, 48.7 mmol) and 1-(2-bromopyrimidin-4-yl)propan-1-one **INTC149** (11.5 g, 51.3 mmol). The reaction mixture was then heated to 70 °C for 10 hrs. The reaction mixture was cooled in an ice-bath, diluted in THF (200 mL) and treated dropwise with brine (50 mL). The mixture was stirred for 15 min then filtered over celite (80 g) eluting with THF (1 L). The filtrate was concentrated *in vacuo.* The crude product was purified by chromatography on silica gel (330 g column, 0-100% EtOAc/iso-hexane) to afford N-(1-(2-bromopyrimidin-4-yl)propylidene)-2-methylpropane-2-sulfinamide (12.87 g, 39.6 mmol, 77% yield) (a mixture of E and Z isomers) as a pale yellow solid. Rt 1.79 and 2.12 min (HPLC basic); *m/z* 318 ($^{79}$Br M+H)$^+$ (ES$^+$). No NMR data collected.

**Reduction of sulfoximines**

*N*-(1-(2-Bromopyrimidin-4-yl)propyl)-2-methylpropane-2-sulfinamide **INTC151**

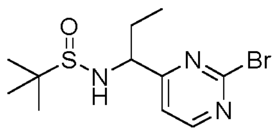

A solution of *N*-(1-(2-bromopyrimidin-4-yl)propylidene)-2-methylpropane-2-sulfinamide **INTC150** (10 g, 30.8 mmol) in THF (200 mL) and water (2 mL) was cooled to - 50 °C (external bath temp) then treated with sodium borohydride (1.2 g, 31.7 mmol). The reaction mixture was stirred for 10 min then allowed to warm to RT. After 1 h sat. NaHCOs (aq, 20 mL) was added and the reaction mixture was stirred for 20 min. The mixture was acidified to pH 5 with 1 N HCl (aq) then concentrated *in vacuo.* The aqueous phase was extracted with DCM (3 × 80 mL), the combined organic phases dried (phase separator) and concentrated *in vacuo* to afford N-(1-(2-bromopyrimidin-4-yl)propyl)-2-methylpropane-2-sulfinamide (7.2 g, 20.9 mmol, 68% yield) as an orange gum, as a 1:3 mixture of diastereomers. Rt 1.63 and 1.77 mins (HPLC basic); *m/z* 320 (M+H)$^+$ (ES$^+$). No NMR data collected.

**Grignard to add in second $R_4$/$R_5$ group**

*N*-(2-(2-Bromopyrimidin-4-yl)butan-2-yl)-2-methylpropane-2-sulfinamide **INTC152**

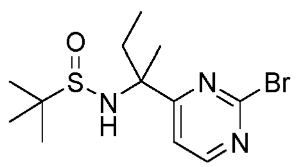

To a solution of *N*-(1-(2-bromopyrimidin-4-yl)propylidene)-2-methylpropane-2-sulfinamide **INTC150** (100 mg, 0.314 mmol) in THF (100 mL) at -78 °C was added MeMgBr (0.13 mL, 0.38 mmol) dropwise over 5 min. The resulting mixture was allowed to warm up to room temperature and stirred for 1 h then quenched by addition of sat. $NH_4Cl$ (aq,

50 mL). The product was extracted into EtOAc (2 × 100 mL), dried ($MgSO_4$), filtered and the solvent removed *in vacuo.* The crude product was purified by chromatography on silica gel (40 g column, 0-100% EtOAc/isohexane) to afford *N*-(2-(2-bromopyrimidin-4-yl)butan-2-yl)-2-methylpropane-2-sulfinamide (0.107 g, 0.321 mmol, quantitative yield) as a clear colourless gum, as a single diastereomer. Rt 1.95 min (HPLC acidic); *m/z* 332 ($^{79}$Br M +H)$^+$ (ES$^+$). $^1$H NMR (500 MHz, DMSO-d6) δ 8.70 (d, J = 5.2 Hz, 1H), 7.85 (d, J = 5.2 Hz, 1H), 5.63 (s, 1H), 1.97 - 1.82 (m, 2H), 1.54 (s, 3H), 1.17 (s, 9H), 0.75 (t, J = 7.4 Hz, 3H).

**Formation of sulfonamides from aromatic halides**

**Table 12:** The following intermediates were made according to Method C which is described above.

| INTC | Name/Structure (All examples containing chiral centres are racemates unless stated) | Synthesis Method, [LCMS Method], m/z (M+H)$^+$, (Rt/min) | $^1$H NMR Chemical Shift Data (DMSO-d6 unless stated) | Catalyst, Base, Solvent |
|---|---|---|---|---|
| **INTC153** | *tert*-Butyl (1-(2-(cyclopropanesulfonamido) pyrimidin-4-yl)cyclopropyl) carbamate 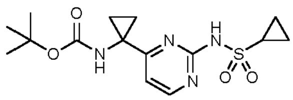 | Method C using INTC148, [UPLC acidic], 355 (1.11). | 11.07 (s, 1H), 8.45 (s, 1H), 7.83 (s, 1H), 6.99 (s, 1H), 3.16 - 3.03 (m, 1H), 1.42 (s, 9H), 1.12 - 1.01 (m, 4H), 0.95 - 0.84 (m, 4H). | Pd 174, $Cs_2CO_3$, dioxane |
| **INTC154** | *N*-(4-(2-(1,1-dimethylethylsulfinamido)butan-2-yl)pyrimidin-2-yl) cyclopropanesulfonamide 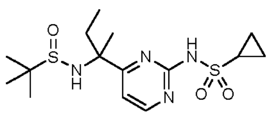 | Method C using INTC152, [UPLC acidic], 375 (1.10). | 11.26 (s, 1H), 8.58 (d, J = 5.3 Hz, 1H), 7.36 (d, J = 5.3 Hz, 1H), 5.54 (s, 1H), 3.30 - 3.20 (m, 1H), 1.95 - 1.85 (m, 2H), 1.56 (s, 3H), 1.19 (s, 9H), 1.16 - 0.99 (m, 4H), 0.71 (t, J = 7.3 Hz, 3H). | Pd 174, $Cs_2CO_3$, dioxane |
| **INTC155** | *N*-(4-propionylpyrimidin-2-yl) cyclopropanesulfonamide 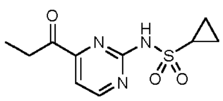 | Method C using INTC149, [UPLC acidic 2], 256 (0.50). | No data recorded | [Pd(allyl) Cl]$_2$ tBuXPhos, $Cs_2CO_3$, dioxane |
| **INTC125** | Methyl 2-(6-(cyclopropanesulfonamido) pyrazin-2-yl)-2-methylpropanoate 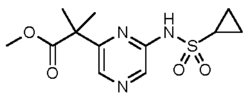 | Method C using INTC116, [HPLC acidic], 300 (1.65). | 11.04 (s, 1H), 8.36 (s, 1H), 8.19 (s, 1H), 3.60 (s, 3H), 3.08-2.98 (m, 1H), 1.55 (s, 6H), 1.23 - 1.00 (m, 4H). | Pd-174, $Cs_2CO_3$, dioxane |
| **INTC126** | Methyl 2-(6-(cyclopropanesulfonamido) pyrazin-2-yl)butanoate | Method C using INTC117, [HPLC acidic], 300 (1.62). | 11.04 (s, 1H), 8.27 (s, 1H), 8.21 (s, 1H), 3.82 (t, J = 7.5 Hz, 1H), 3.60 (s, 3H), 3.08-3.01 (m, 1H), 2.09 - 2.01 (m, 1H), 1.91 - 1.82 (m, 1H), 1.18 (t, J = 7.1 Hz, 3H), 1.07 - 1.02 (m, 2H), 0.92 - 0.86 (m, 2H). | Pd-174, $Cs_2CO_3$, dioxane |

(continued)

| INTC | Name/Structure (All examples containing chiral centres are racemates unless stated) | Synthesis Method, [LCMS Method], m/z $(M+H)^+$, (Rt/min) | 1H NMR Chemical Shift Data (DMSO-d6 unless stated) | Catalyst, Base, Solvent |
|---|---|---|---|---|
| | 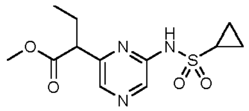 | | | |
| **INTC127** | Methyl 4-(6-(cyclopropanesulfonamido) pyrazin-2-yl)tetrahydro-2H-pyran-4-carboxylate 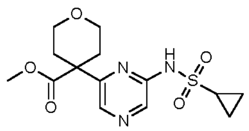 | Method C using INTC123, [HPLC acidic], 342 (1.45). | 11.11 (s, 1H), 8.34 - 7.68 (m, 2H), 3.78 - 3.67 (m, 2H), 3.63 (s, 3H), 3.52 - 3.44 (m, 2H), 3.02 - 2.98 (m, 1H), 2.25 (s, 2H), 2.09 (s, 2H), 1.09 - 0.85 (m, 4H). | Pd-174, $Cs_2CO_3$, dioxane |
| **INTC128** | Methyl 2-(6-(cyclopropanesulfonamido) pyrazin-2-yl)-2-methoxyacetate 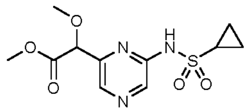 | Method C using INTC121, [HPLC acidic], 302 (1.25). | 11.17 (s, 1H), 8.38 (s, 1H), 8.31 (s, 1H), 5.12 (s, 1H), 3.68 (s, 3H), 3.41 (s, 3H), 3.08-2.99 (m, 1H), 1.17 - 1.01 (m, 4H). | Pd-174, $Cs_2CO_3$, dioxane |
| **INTC129** | methyl 2-(6-(cyclopropanesulfonamido) pyrazin-2-yl)-2-methoxypropanoate 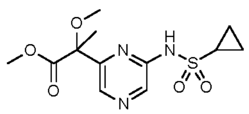 | Method C using INTC122, [HPLC acidic], 316 (1.46). | 11.14 (s, 1H), 8.44 (s, 1H), 8.25 (s, 1H), 3.66 (s, 3H), 3.29 (s, 3H), 3.06-2.96 (m, 1H), 1.70 (s, 3H), 1.18 - 0.99 (m, 4H). | Pd-174, $Cs_2CO_3$, dioxane |
| **INTC130** | Methyl 2-(6-(cyclopropanesulfonamido) pyrazin-2-yl)-2-fluorobutanoate 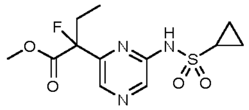 | Method C using INTC124, [UPLC acidic], 318 (1.08). | 11.29 (s, 1H), 8.48 (s, 1H), 8.33 (s, 1H), 3.73 (s, 3H), 3.10-3.04 (m, 1H), 2.45 - 2.27 (m, 2H), 1.24 - 1.00 (m, 4H), 0.91 (t, J = 7.4 Hz, 3H). | Pd-174, $Cs_2CO_3$, dioxane |
| **INTC170** | Methyl 6-(cyclopropanesulfonamido) pyrazin e-2-carboxylate 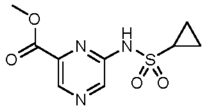 | Method C using commercia l SM, [HPLC acidic], 258 (1.25). | 11.40 (s, 1H), 8.85 (s, 1H), 8.58 (s, 1H), 3.92 (s, 3H), 3.14 - 3.03 (m, 1H), 1.26 - 1.18 (m, 2H), 1.12 - 1.03 (m, 2H). | Pd 174, $Cs_2CO_3$, dioxane |

(continued)

| INTC | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) | Catalyst, Base, Solvent |
|---|---|---|---|---|
| **INTC171** | *tert*-Butyl (1-(6-(cyclopropanesulfonamido) pyrazin-2-yl)propyl)carbamate 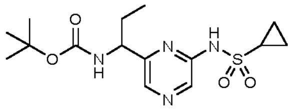 | Method C using INTC169, [HPLC acidic], 357 (1.89). | 10.98 (s, 1H), 8.22 (s, 1H), 8.19 (s, 1H), 7.39 (d, J = 8.2 Hz, 1H), 4.49 - 4.39 (m, 1H), 3.20 - 3.06 (m, 1H), 1.85 - 1.74 (m, 1H), 1.74 - 1.63 (m, 1H), 1.38 (s, 9H), 1.15 - 1.08 (m, 2H), 1.08 - 1.02 (m, 2H), 0.87 (t, J = 7.3 Hz, 3H). | [Pd(allyl) Cl] 2 tBuXPhos, $Cs_2CO_3$, dioxane |

**Deprotection: Boc**

*N*-(4-(1-Aminocyclopropyl)pyrimidin-2-yl)cyclopropanesulfonamide hydrochloride **INTC156**

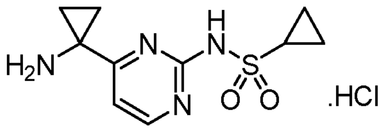

To a solution of *tert*-butyl (1-(2-(cyclopropanesulfonamido)pyrimidin-4-yl)cyclopropyl)carbamate **INTC153** (200 mg, 0.564 mmol) in dioxane (2 mL) was added HCl (4 M in dioxane) (1.41 mL, 5.64 mmol) and the resulting solution was stirred at RT for 18 hrs. The solvent was removed *in vacuo* to afford *N*-(4-(1-aminocyclopropyl)pyrimidin-2-yl)cyclopropanesulfonamide hydrochloride (164 mg, 0.564 mmol, quantitative yield) as a slightly yellow solid which was used without any further purification. Rt 0.39 min (UPLC acidic); m/z 255 $(M+H)^+$ $(ES^+)$. No NMR data collected.

*N*-(4-(Aminomethyl)pyrimidin-2-yl)cyclopropanesulfonamide hydrochloride INTC157

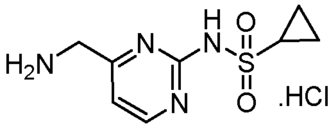

Prepared as for **INTC156** using *tert*-butyl ((2-(cyclopropanesulfonamido)pyrimidin-4-yl)methyl)carbamate **INTC145** to afford *N*-(4-(aminomethyl)pyrimidin-2-yl)cyclopropanesulfonamide, HCl (70 mg, 0.225 mmol, 88% yield) as a pale yellow solid; Rt 0.13 mins (UPLC acidic); *m/z* 229 $(M+H)^+$ $(ES^+)$. No NMR data collected.

**Sulfoximine deprotection**

1-(2-Chloropyrimidin-4-yl)propan-1-amine **INTC158**

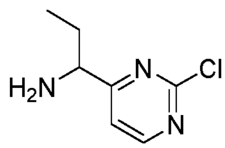

A solution of *N*-(1-(2-bromopyrimidin-4-yl)propyl)-2-methylpropane-2-sulfinamide **INTC151** (7.2 g, 22.48 mmol) in THF (30 mL) was treated with 4 N HCl in dioxane (2.9 mL, 95 mmol) and MeOH (1.0 mL) then stirred at RT for 3 hrs. The reaction mixture was concentrated *in vacuo* then basified with sat. NaHCOs (aq, 100 mL). The product was extracted into DCM (3 × 80 mL), the combined organic phases were dried (phase separator) and concentrated *in vacuo.* The crude product was purified by chromatography on silica gel (80 g column, 0-10% (0.7 M Ammonia/MeOH)/DCM) to afford 1-(2-chloropyrimidin-4-yl)propan-1-amine (1.34 g, 6.01 mmol, 27% yield) as a clear brown oil. Rt 0.75 mins (UPLC basic); m/z 172 ($^{35}$Cl M +H)$^+$ (ES$^+$). $^1$H NMR (500 MHz, DMSO-d6) δ 8.71 (d, J = 5.1 Hz, 1H), 7.64 (d, J = 5.1 Hz, 1H), 3.77 - 3.64 (m, 1H), 2.03 (s, 2H), 1.74 - 1.63 (m, 1H), 1.62 - 1.49 (m, 1H), 0.84 (t, J = 7.4 Hz, 3H). Br-C! exchange observed in reaction.

*N*-(4-(2-Aminobutan-2-yl)pyrimidin-2-yl)cyclopropanesulfonamide hydrochloride **INTC159**

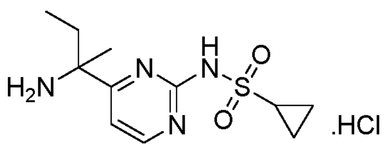

Prepared as for **INTC158** using *N*-(4-(2-(1,1-dimethylethylsulfinamido)butan-2-yl)pyrimidin-2-yl)cyclopropanesulfonamide **INTC154** to afford *N*-(4-(2-aminobutan-2-yl)pyrimidin-2-yl)cyclopropanesulfonamide hydrochloride (92% yield) as a yellow solid. Rt 0.41 min (UPLC basic); *m/z* 271 (M+H)$^+$ (ES$^+$). $^1$H NMR (500 MHz, DMSO-d6) δ 11.39 (s, 1H), 8.79 - 8.51 (m, 4H), 7.37 (d, J = 5.0 Hz, 1H), 3.38 (s, 1H), 2.05 - 1.85 (m, 2H), 1.61 (s, 3H), 1.19 - 0.99 (m, 4H), 0.84 - 0.68 (m, 3H).

**Reduction amination**

1-(2-bromopyrimidin-4-yl)propan-1-amine **INTC160**

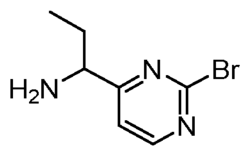

A suspension of 2,2,2-trifluoroacetic acid, ammonia salt (6.09 g, 46.5 mmol) and 1-(2-bromopyrimidin-4-yl)propan-1-one **INTC149** (500 mg, 2.32 mmol) in THF (20 mL, 244 mmol) was stirred at 45 °C for 15 min to give a clear yellow solution, which was cooled to RT. Additional 2,2,2-trifluoroacetic acid, ammonia salt (1.8 g, 13.7 mmol) was added. $NaHB(OAc)_3$ (985 mg, 4.65 mmol) was added and the reaction mixture was stirred at RT for 3 hrs. The reaction mixture was reduced *in vacuo* to ca. 10 mL and diluted with EtOAc (50 mL) and washed with 2 M $Na_2CO_3$ (aq, 2 × 50 mL). The organic layer was then stirred with Si-Tosic acid (10 g), filtered washed with EtOAc (2 × 50 mL) and MeOH (2 × 50 mL), and eluted with 0.7 M $NH_3$ in MeOH to afford 1-(2-bromopyrimidin-4-yl)propan-1-amine (227 mg, 0.99 mmol, 43% yield) as an orange oil. Rt 0.69 mins (UPLC basic); *m/z* 216 ($^{79}$Br M +H)$^+$ (ES$^+$). $^1$H NMR (500 MHz, DMSO-d6) δ 8.64 (d, J = 5.1 Hz, 1H), 7.67 (d, J = 5.1 Hz, 1H), 3.72 (t, J = 6.6 Hz, 1H), 1.73 - 1.62 (m, 1H), 1.62 - 1.51 (m, 1H), 0.84 (t, J = 7.4 Hz, 3H), $NH_2$ not observed.

**Oxime formation**

*N*-(4-(1-(Methoxyimino)propyl)pyrimidin-2-yl)cyclopropanesulfonamide **INTC161**

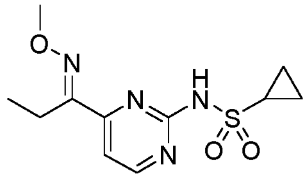

A suspension of O-methylhydroxylamine hydrochloride (170 mg, 2.04 mmol), N-(4-propionylpyrimidin-2-yl)cyclopropanesulfonamide **INTC155** (500 mg, 1.96 mmol) and pyridine (0.35 mL, 4.33 mmol) in EtOH (4 mL) was heated to reflux for 18 hrs. The reaction mixture was concentrated then taken up in EtOAc (20 mL) and washed with 1 M HCl (15 mL) and brine (15 mL). The organic phase was dried ($Na_2SO_4$), filtered and concentrated onto silica (3 g). The crude product was purified by chromatography on silica gel (12 g column, 0-100% EtOAc/*iso*-hexane) to afford *N*-(4-(1-(methoxyimino)propyl)pyrimidin-2-yl)cyclopropanesulfonamide (428 mg, 1.43 mmol, 70% yield) as a yellow gum. Rt 0.60 mins (UPLC, basic 2); *m/z* 285 $(M+H)^+$ $(ES^+)$. $^1H$ NMR (500 MHz, DMSO-d6) δ 11.35 (s, 1H), 8.60 (d, J = 5.2 Hz, 1H), 7.45 (d, J = 5.2 Hz, 1H), 4.02 (s, 3H), 3.25 - 3.15 (m, 1H), 2.76 (q, J = 7.5 Hz, 2H), 1.15 - 1.01 (m, 7H).

*N*-(4-(1-Aminopropyl)pyrimidin-2-yl)cyclopropanesulfonamide **INTC162**

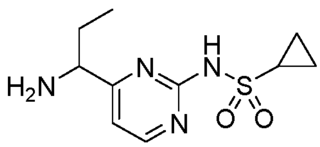

A solution of *N*-(4-(1-(methoxyimino)propyl)pyrimidin-2-yl)cyclopropanesulfonamide (428 mg, 1.51 mmol) **INTC161** in 7M $NH_3$ in MeOH (4 mL) was treated with Pd-C 10% on charcoal (10 mg) and hydrogenated at 5 bar for 1 hr. The reaction mixture was filtered over celite eluting with DCM (30 mL) then concentrated *in vacuo* to afford *N*-(4-(1-aminopropyl)pyrimidin-2-yl)cyclopropanesulfonamide (380 mg, 1.19 mmol, 79% yield) as an off-white solid. Rt 0.44 mins (HPLC basic); *m/z* 257 $(M+H)^+$ $(ES^+)$. No NMR data collected.

**HATU Amide Coupling**

**Table 13:** The following intermediates were made according to Method 1 which is described below for the synthesis of compound of formula (I).

| INTC | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| **INTC163** | *N*-(1-(2-chloropyrimidin-4-yl)propyl)-5-(6-ethoxypyrazin-2-yl)picolinamide 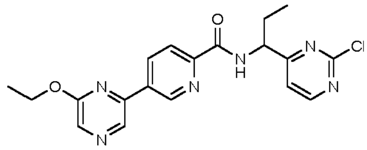 | Method 1 using INTC158 and INTD86, [UPLC acidic], 398 $^{35}Cl$ isotope (1.52). | 9.44 - 9.39 (m, 1H), 9.29 (d, J = 8.2 Hz, 1H), 9.01 (s, 1H), 8.75 (d, J = 5.1 Hz, 1H), 8.72 - 8.67 (m, 1H), 8.38 (s, 1H), 8.17 (d, J = 8.2 Hz, 1H), 7.66 (d, J = 5.1 Hz, 1H), 5.10 - 4.96 (m, 1H), 4.52 (q, J = 7.1 Hz, 2H), 2.06 - 1.90 (m, 2H), 1.42 (t, J = 7.1 Hz, 3H), 0.94 (t, J = 7.3 Hz, 3H). |

(continued)

| INTC | Name/Structure (All examples containing chiral centres are racemates unless stated) | Synthesis Method, [LCMS Method], m/z $(M+H)^+$, (Rt/min) | $^1H$ NMR Chemical Shift Data (DMSO-d6 unless stated) |
|---|---|---|---|
| **INTC164** | *N*-(1-(2-chloropyrimidin-4-yl)propyl)-4-(6-ethoxypyrazin-2-yl)-2-(trifluoromethyl)benzamide 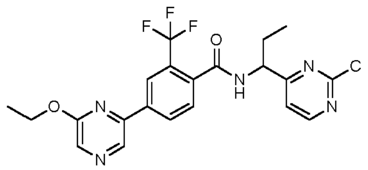 | Method 1 using INTC158 and INTD80, [UPLC acidic], 466 $^{35}Cl$ isotope (1.55). | 9.25 (d, J = 7.7 Hz, 1H), 9.00 (s, 1H), 8.80 (d, J = 5.1 Hz, 1H), 8.53 - 8.46 (m, 2H), 8.36 (s, 1H), 7.77 (d, J = 8.0 Hz, 1H), 7.61 (d, J = 5.1 Hz, 1H), 4.93 - 4.82 (m, 1H), 4.51 (q, J = 7.0 Hz, 2H), 1.96 - 1.85 (m, 1H), 1.85 - 1.73 (m, 1H), 1.42 (t, J = 7.0 Hz, 3H), 0.99 (t, J = 7.3 Hz, 3H). |
| **INTC165** | *N*-(1-(2-bromopyrimidin-4-yl)propyl)-4-(6-ethoxypyrazin-2-yl)-2-fluorobenzamide 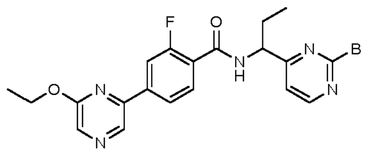 | Method 1 using INTC160 and INTD74, [HPLC acidic], 460 $^{79}Br$ isotope (2.44). | 9.02 (d, J = 7.5 Hz, 1H), 8.93 (s, 1H), 8.70 (d, J = 5.1 Hz, 1H), 8.33 (s, 1H), 8.09 - 8.05 (m, 2H), 7.77-7.73 (m, 1H), 7.62 (d, J = 5.1 Hz, 1H), 4.93 - 4.83 (m, 1H), 4.50 (q, J = 7.0 Hz, 2H), 1.96 - 1.86 (m, 1H), 1.86 - 1.74 (m, 1H), 1.41 (t, J = 7.0 Hz, 3H), 0.99 (t, J = 7.3 Hz, 3H). |
| **INTC166** | *N*-(1-(2-chloropyrimidin-4-yl)propyl)-4-(6-(trifluoromethyl)pyrazin-2-yl) benzamide 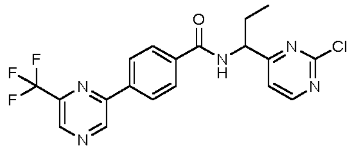 | Method 1 using INTC158 and INTD75, [UPLC acidic], 422 $^{35}Cl$ isotope (1.47). | 9.71 (s, 1H), 9.21 (s, 1H), 9.13 (d, J = 7.4 Hz, 1H), 8.75 (d, J = 5.1 Hz, 1H), 8.33 (d, J = 8.3 Hz, 2H), 8.13 (d, J = 8.3 Hz, 2H), 7.61 (d, J = 5.1 Hz, 1H), 4.98 - 4.87 (m, 1H), 1.98 - 1.84 (m, 2H), 1.00 (t, J = 7.3 Hz, 3H). |
| **INTC167** | *N*-(1-(2-chloropyrimidin-4-yl)propyl)-4-(6-isopropoxypyrazin-2-yl) benzamide 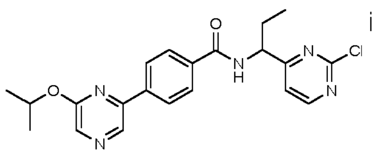 | Method 1 using INTC158 and INTD82, [UPLC acidic], 412 $^{35}Cl$ isotope (1.54). | 9.05 (d, J = 7.5 Hz, 1H), 8.88 (s, 1H), 8.74 (d, J = 5.1 Hz, 1H), 8.26 - 8.20 (m, 3H), 8.06 (d, J = 8.2 Hz, 2H), 7.60 (d, J = 5.1 Hz, 1H), 5.48 - 5.34 (m, 1H), 4.98 - 4.88 (m, 1H), 1.99 - 1.83 (m, 2H), 1.40 (d, J = 6.1 Hz, 6H), 1.00 (t, J = 7.3 Hz, 3H). |
| **INTC168** | *N*-(1-(2-chloropyrimidin-4-yl)propyl)-4-(6-ethoxypyrazin-2-yl)benzamide 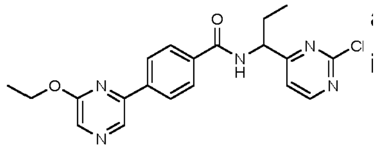 | Method 1 using INTC158 and INTD83, [UPLC acidic], 398 $^{35}Cl$ isotope (1.45). | 9.06 (d, J = 7.6 Hz, 1H), 8.90 (s, 1H), 8.74 (d, J = 5.1 Hz, 1H), 8.30 (s, 1H), 8.25 (d, J = 8.2 Hz, 2H), 8.06 (d, J = 8.2 Hz, 2H), 7.60 (d, J = 5.1 Hz, 1H), 4.98 - 4.82 (m, 1H), 4.50 (q, J = 7.0 Hz, 2H), 1.98 - 1.83 (m, 2H), 1.42 (t, J = 7.0 Hz, 3H), 1.00 (t, J = 7.3 Hz, 3H). |

**Benzamide Pyrazines**

**Curtius**

*tert*-Butyl (1-(6-chloropyrazin-2-yl)propyl)carbamate **INTC169**

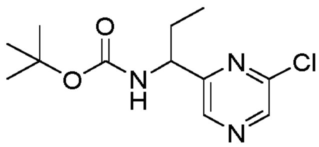

Prepared as for **INTC148** using commercial 2-(6-chloropyrazin-2-yl)butanoic acid to afford *tert*-butyl (1-(6-chloropyrazin-2-yl)propyl)carbamate (6% yield) as a colourless solid. Rt 2.15 min (HPLC acidic); *m/z* 272 ($^{35}$Cl M+H)$^+$ (ES$^+$). $^1$H NMR (4:1 mixture of rotamers) (500 MHz, DMSO-d6) δ 8.68 (s, 1H), 8.61 (s, 1H), 7.52 (d, J = 7.9 Hz, 1H), 4.55 - 4.44 (m, 1H), 1.85 - 1.57 (m, 2H), 1.37 (s, 9H, major), 1.22 (s, 9H, minor), 0.87 (t, J = 7.3 Hz, 3H).

**Grignard**

N-(6-(2-Hydroxypropan-2-yl)pyrazin-2-yl)cyclopropanesulfonamide **INTC172**

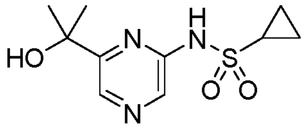

A solution of methyl 6-(cyclopropanesulfonamido)pyrazine-2-carboxylate **INTC170** (3.00 g, 11.7 mmol) in THF (30 mL) was cooled to 0 °C then MeMgBr (3.0 M in $Et_2O$) (18 mL, 54.0 mmol) was added dropwise over 15 mins then the reaction mixture was warmed to RT. The reaction mixture was stirred at RT for 18 hrs. The reaction mixture was heated to 40 °C for a further 24 hrs. The reaction mixture was cooled with an ice bath and 1M HCl (aq, 60 mL) was added cautiously. The aqueous was extracted with EtOAc (4 × 500 mL). The organic phases were combined, dried ($Na_2SO_4$), filtered and concentrated onto silica (10 g). The crude product was purified by chromatography on silica gel (40 g column, 0-100% EtOAc/iso-hexane) to afford *N*-(6-(2-hydroxypropan-2-yl)pyrazin-2-yl)cyclopropanesulfonamide (340 mg, 1.30 mmol, 11% yield) as a brown gum. Rt 0.24 min (UPLC, acidic 2); *m/z* 258 (M+H)$^+$ (ES$^+$). 1H NMR (500 MHz, DMSO-d6) δ 10.92 (s, 1H), 8.51 (s, 1H), 8.16 (s, 1H), 5.40 (s, 1H), 3.11 - 3.01 (m, 1H), 1.45 (s, 6H), 1.14 - 1.02 (m, 4H).

**Ritter**

2-Chloro-N-(2-(6-(cyclopropanesulfonamido)pyrazin-2-yl)propan-2-yl)acetamide **INTC173**

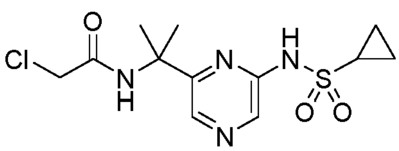

A mixture of *N*-(6-(2-hydroxypropan-2-yl)pyrazin-2-yl)cyclopropanesulfonamide **INTC172** (330 mg, 1.28 mmol) and 2-chloroacetonitrile (0.65 mL, 10.3 mmol) in AcOH (0.75 mL, 13.1 mmol) was cooled in an ice bath before $H_2SO_4$ (0.82 mL, 15.4 mmol) was added dropwise. The reaction was then warmed to 50 °C and stirred for 18 hrs. The solution was poured onto ice water (30 mL) and extracted with EtOAc (3 × 30 mL), the organic phases were combined, dried ($Na_2SO_4$), filtered and concentrated onto silica (2 g). The crude product was purified by chromatography on silica gel

(12 g column, 0-100% EtOAc/*iso*-hexane) to afford 2-chloro-*N*-(2-(6-(cyclopropanesulfonamido)pyrazin-2-yl)propan-2-yl)acetamide (100 mg, 0.294 mmol, 19% yield) as a yellow gum. Rt 0.98 min (HPLC acidic); *m/z* 333 ($^{35}$Cl M+H)$^+$ (ES$^+$). $^1$H NMR (500 MHz, DMSO-d6) δ 10.94 (s, 1H), 8.63 (s, 1H), 8.29 (s, 1H), 8.12 (s, 1H), 4.08 (s, 2H), 3.16 - 3.08 (m, 1H), 1.58 (s, 6H), 1.14 - 1.03 (m, 4H).

**Boc removal**

*N*-(6-(1-Aminopropyl)pyrazin-2-yl)cyclopropanesulfonamide.HCl **INTC174**

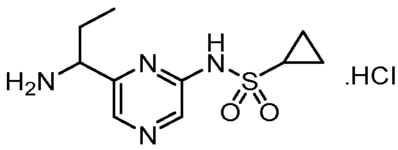

Prepared as for **INTC156** using *tert*-butyl (1-(6-(cyclopropanesulfonamido)pyrazin-2-yl)propyl)carbamate **INTC171** to afford *N*-(6-(1-aminopropyl)pyrazin-2-yl)cyclopropanesulfonamide, HCl (85 mg, 0.276 mmol, 42% yield) as a colourless solid. Rt 0.58 min (HPLC basic); *m/z* 257 (M+H)$^+$ (ES$^+$). $^1$H NMR (500 MHz, DMSO-d6) δ11.25 (s, 1H), 8.77 - 8.66 (m, 3H), 8.45 (s, 1H), 8.31 (s, 1H), 4.37 - 4.23 (m, 1H), 3.53 - 3.45 (m, 1H), 2.09 - 1.83 (m, 2H), 1.20 - 0.99 (m, 4H), 0.83 (t, J = 7.4 Hz, 3H).

**Thiourea Deprotection**

N-(6-(2-Aminopropan-2-yl)pyrazin-2-yl)cyclopropanesulfonamide **INTC175**

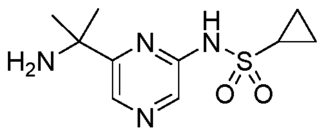

A suspension of 2-chloro-*N*-(2-(6-(cyclopropanesulfonamido)pyrazin-2-yl)propan-2-yl)acetamide (100 mg, 0.30 mmol) **INTC173** in EtOH (1.3 mL) was treated with thiourea (23 mg, 0.302 mmol) followed by AcOH (0.35 mL, 6.11 mmol) then heated to reflux for 1 hr. The reaction mixture was allowed to cool to RT then concentrated *in vacuo.* This was then treated cautiously with 0.7M $NH_3$ in MeOH (5 mL) and concentrated. The crude product was purified by chromatography on RP Flash C18 (12 g column, 0-25% MeCN/10 mM Ammonium Bicarbonate) to afford *N*-(6-(2-aminopropan-2-yl)pyrazin-2-yl)cyclopropanesulfonamide (63 mg, 0.23 mmol, 78% yield) as a colourless solid. Rt 0.37 min (HPLC basic); *m/z* 257 (M+H)$^+$ (ES$^+$). $^1$H NMR (500 MHz, DMSO-d6) δ 8.03 (s, 1H), 7.82 (s, 1H), 2.77 - 2.64 (m, 1H), 1.53 (s, 6H), 0.91 - 0.77 (m, 2H), 0.77 - 0.66 (m, 2H), 3 × exchangeable Hs not observed.

**Amine intermediate preparation**

**Method E: Suzuki coupling of halo anilines with heteroaromatic boronates**

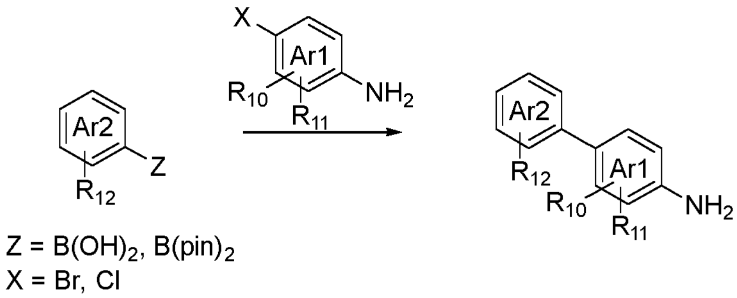


A solution ofAr1-X (1 eq) and Ar2-Z (1 eq) in solvent (3 volumes) and base (2.5 eq) was degassed ($N_2$, 5 min) and heated to 40 °C whereupon Pd catalyst (3 mol%) was added and the reaction mixture further degassed ($N_2$, 5 min) before being heated to 90 °C for 90 mins. The reaction mixture was allowed to cool to RT. In general, the desired compound was purified by column chromatography.

**Method F: Suzuki coupling of heteroaromatic halides with aniline boronates**

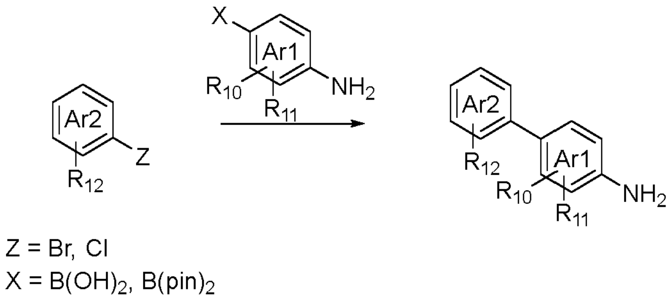


Pd catalyst (5 mol%) was added to a degassed ($N_2$, 5 mins) solution of Ar1-X (1 eq), Ar2-Z (1 eq) and base (3 eq, 6.85 mmol) in solvent (3 volumes). The solution was then degassed further ($N_2$, 5 mins) and then heated to 90 °C for 2 hrs then allowed to cool to RT. In general, the desired compound was purified by column chromatography.

**Method G: Telescoped boronate formation and Suzuki coupling**

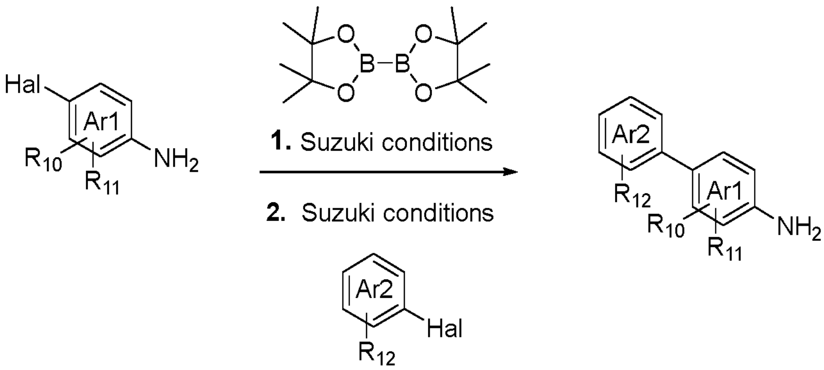


Bispin (1.1 eq) and KOAc (4 eq) were added to Ar1-Hal (1 eq) in dioxane (5 volumes). The reaction was heated to 60 °C and degassed ($N_2$, 5 mins). $PdCl_2$(dppf) (5 mol%) was added to the reaction mixture and the temperature was increased to 90 °C for 1 hr. The reaction mixture was then cooled to RT and a solution of Ar2-Hal (1 eq) in dioxane (3 volumes) was added, followed by a solution of $K_2CO_3$ (4 eq) in water (2 volumes). The temperature was then increased to 90 °C for 18 hrs. The reaction was cooled to RT, an aqueous work up was performed and the crude compound was purified by normal phase chromatography.

## Anilines

**Table 14:** The following intermediates were made according to Methods E, F or G.

| INTD | Name/Structure | Synthesis Method, [LCMS Method], m/z (M+H)$^+$, (Rt/min) | $^1$H NMR Chemical Shift Data (DMSO-d6 unless stated) | Catalyst, Base, solvent |
|---|---|---|---|---|
| **INTD1** | 4-(6-methoxypyrazin-2-yl) aniline 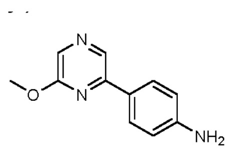 | Method F, [HPLC basic], 202, (1.63). | 8.61 (s, 1H), 8.04 (s, 1H), 7.94 - 7.75 (m, 2H), 6.75 - 6.54 (m, 2H), 5.59 (s, 2H), 3.98 (s, 3H). | $Pd(PPh_3)_4$, NaHCOs, MeCN |
| **INTD2** | 5-(6-(trifluoromethyl) pyrazin-2-yl)pyridin-2-amine 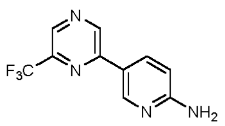 | Method F, [UPLC acidic], 241, (0.52). | 9.52 - 9.41 (m, 1H), 8.95 (t, J = 0.6 Hz, 1H), 8.81 (dd, J = 2.5, 0.8 Hz, 1H), 8.16 (dd, J = 8.8, 2.5 Hz, 1H), 6.66 (s, 2H), 6.59 (dd, J = 8.8, 0.8 Hz, 1H). | $Pd(PPh_3)_4$, $NaHCO_3$, EtOH, toluene |
| **INTD3** | 3-(4-aminophenyl)-5-(difluoro-l3-methoxy) pyridine 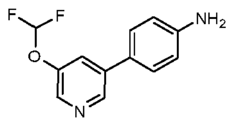 | Method E, [UPLC basic], 237, (1.05). | 8.74 - 8.66 (m, 1H), 8.36 - 8.24 (m, 1H), 7.81 - 7.74 (m, 1H), 7.53 - 7.43 (m, 2H), 7.61 - 7.15 (m, 1H), 6.72 - 6.58 (m, 2H), 5.44 (s, 2H). | $PdCl_2$(dppf), $K_2CO_3$, dioxane |
| **INTD4** | 4-(5-ethoxypyridin-3-yl) aniline 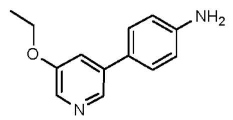 | Method F, [UPLC basic], 215, (1.05). | 8.36 (d, J = 1.9 Hz, 1H), 8.11 (d, J = 2.7 Hz, 1H), 7.48 - 7.39 (m, 3H), 6.70 - 6.62 (m, 2H), 5.34 (s, 2H), 4.17 (q, J = 6.9 Hz, 2H), 1.36 (t, J = 7.0 Hz, 3H). | $PdCl_2$(dppf), $K_2CO_3$, dioxane |
| **INTD5** | 5-(4-aminophenyl) nicotinonitrile 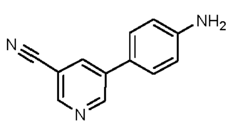 | Method E, [UPLC basic], 196, (0.89). | 9.08 (d, J = 2.4 Hz, 1H), 8.91 - 8.79 (m, 1H), 8.49 - 8.43 (m, 1H), 7.58 - 7.48 (m, 2H), 6.73 - 6.57 (m, 2H), 5.49 (d, J = 7.0 Hz, 2H). | $PdCl_2$(dppf), $K_2CO_3$, dioxane |
| **INTD6** | 4-(5-fluoropyridin-3-yl) aniline 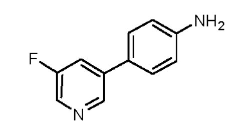 | Method E, [HPLC basic], 189, (1.53). | 8.69 (t, J = 2.0 Hz, 1H), 8.39 (d, J = 2.7 Hz, 1H), 7.87 (ddd, J = 10.9, 2.7, 1.9 Hz, 1H), 7.53 - 7.45 (m, 2H), 6.71 - 6.63 (m, 2H), 5.44 (s, 2H). | $PdCl_2$(dppf), $K_2CO_3$, dioxane |

(continued)

| INTD | Name/Structure | Synthesis Method, [LCMS Method], m/z $(M+H)^+$, (Rt/min) | $^1$H NMR Chemical Shift Data (DMSO-d6 unless stated) | Catalyst, Base, solvent |
|---|---|---|---|---|
| **INTD7** | 4-(5-(trifluoromethyl) pyridin-3-yl)aniline 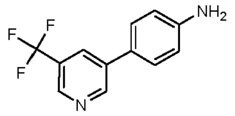 | Method E, [UPLC basic], 239, (1.21). | 9.15 - 9.06 (m, 1H), 8.82 - 8.72 (m, 1H), 8.30 - 8.23 (m, 1H), 7.60 - 7.51 (m, 2H), 6.74 - 6.64 (m, 2H), 5.50 (s, 2H). | $PdCl_2$(dppf), $K_2CO_3$, dioxane |
| **INTD8** | 4-(5-chloropyridin-3-yl) aniline 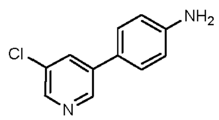 | Method E, [UPLC basic], 205, (1.11). | 8.79 - 8.74 (m, 1H), 8.49 - 8.39 (m, 1H), 8.10 - 7.98 (m, 1H), 7.54 - 7.42 (m, 2H), 6.72 - 6.61 (m, 2H), 5.45 (s, 2H). | $PdCl_2$(dppf), $K_2CO_3$, dioxane |
| **INTD9** | 4-(6-methylpyridin-3-yl) aniline 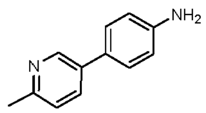 | Method E, [HPLC basic], 185, (1.43). | 8.63 (dd, J = 2.5, 0.8 Hz, 1H), 7.80 (dd, J = 8.1, 2.5 Hz, 1H), 7.40 - 7.32 (m, 2H), 7.23 (dt, J = 8.1, 0.7 Hz, 1H), 6.70 - 6.58 (m, 2H), 5.27 (s, 2H), 2.46 (s, 3H). | Pd 170, $K_3PO_4$, dioxane |
| **INTD10** | 4-(5-methylpyridin-3-yl) aniline 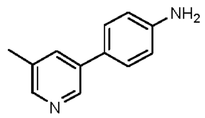 | Method E, [HPLC basic], 185, (1.47). | 8.57 (d, J = 2.3 Hz, 1H), 8.25 (dd, J = 2.0, 0.8 Hz, 1H), 7.74 (td, J = 2.2, 0.9 Hz, 1H), 7.43 - 7.30 (m, 2H), 6.72 - 6.52 (m, 2H), 5.31 (s, 2H), 2.33 (d, J = 0.8 Hz, 3H). | Pd 170, $K_3PO_4$, dioxane |
| **INTD11** | 4-(5-isopropoxypyridin-3-yl) aniline 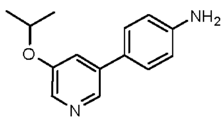 | Method F, [UPLC basic], 229, (1.15). | 8.41 - 8.30 (m, 1H), 8.13 - 8.05 (m, 1H), 7.48 - 7.35 (m, 3H), 6.72 - 6.59 (m, 2H), 5.34 (s, 2H), 4.86 - 4.69 (m, 1H), 1.37 - 1.23 (m, 6H). | $PdCl_2$(dppf), $K_2CO_3$, dioxane |
| **INTD12** | 2-fluoro-4-(5-isopropoxypyridin-3-yl) aniline 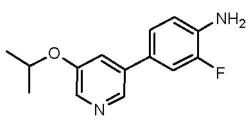 | Method F, [UPLC basic], 247, (1.25). | 8.42 - 8.34 (m, 1H), 8.17 - 8.06 (m, 1H), 7.53 - 7.42 (m, 2H), 7.35 - 7.28 (m, 1H), 6.89 - 6.80 (m, 1H), 5.39 (s, 2H), 4.90 - 4.75 (m, 1H), 1.35 - 1.26 (m, 6H). | $PdCl_2$(dppf), $K_2CO_3$, dioxane |
| **INTD13** | 4-(5-chloropyridin-3-yl)-2-fluoroaniline 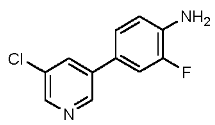 | Method E, [HPLC basic], 223, (1.9). | 8.80 (d, J = 2.0 Hz, 1H), 8.48 (d, J = 2.2 Hz, 1H), 8.14 (t, J = 2.2 Hz, 1H), 7.54 (dd, J = 13.0, 2.1 Hz, 1H), 7.38 (dd, J = 8.3, 2.1 Hz, 1H), 6.86 (dd, J = 9.5, 8.3 Hz, 1H), 5.51 (s, 2H). | $PdCl_2$(dppf), $K_2CO_3$, dioxane |

(continued)

| INTD | Name/Structure | Synthesis Method, [LCMS Method], m/z $(M+H)^+$, (Rt/min) | $^1H$ NMR Chemical Shift Data (DMSO-d6 unless stated) | Catalyst, Base, solvent |
|---|---|---|---|---|
| **INTD14** | 2-fluoro-4-(5-(2,2,2-trifluoro ethoxy)pyridin-3-yl)aniline 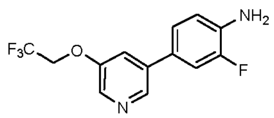 | Method F, [UPLC basic], 287, (1.26). | 8.61 - 8.50 (m, 1H), 8.31 - 8.22 (m, 1H), 7.77 - 7.64 (m, 1H), 7.59 - 7.47 (m, 1H), 7.42 - 7.33 (m, 1H), 6.93 - 6.75 (m, 1H), 5.44 (s, 2H), 5.01 - 4.88 (m, 2H). | $PdCl_2$(dppf), $K_3PO_4$, dioxane |
| **INTD15** | 4-(5-(2,2,2-trifluoro ethoxy) pyridin-3-yl)aniline 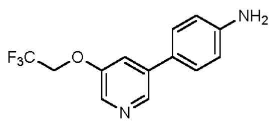 | Method F, [UPLC basic], 269, (1.18). | 8.55 - 8.41 (m, 1H), 8.29 - 8.13 (m, 1H), 7.69 - 7.61 (m, 1H), 7.53 - 7.43 (m, 2H), 6.73 - 6.61 (m, 2H), 5.38 (s, 2H), 5.02 - 4.82 (m, 2H). | $PdCl_2$(dppf), $K_3PO_4$, dioxane |
| **INTD16** | 5'-ethoxy-[3,3'-bipyridin]-6-amine 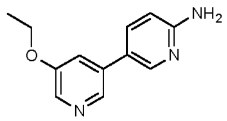 | Method F, [UPLC basic], 216, (0.88). | 8.39 (d, J = 1.9 Hz, 1H), 8.32 (d, J = 2.5 Hz, 1H), 8.17 (d, J = 2.7 Hz, 1H), 7.78 (dd, J = 8.6, 2.6 Hz, 1H), 7.51 (dd, J = 2.7, 1.9 Hz, 1H), 6.54 (dd, J = 8.6, 0.8 Hz, 1H), 6.18 (s, 2H), 4.18 (q, J = 7.0 Hz, 2H), 1.37 (t, J = 7.0 Hz, 3H). | $PdCl_2$(dppf), $K_2CO_3$, dioxane |
| **INTD17** | 5'-(2,2,2-trifluoroethoxy)-[3,3'-bipyridin]-6-amine 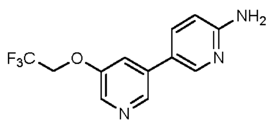 | Method F, [UPLC basic], 270, (1.00). | 8.57 - 8.46 (m, 1H), 8.41 - 8.34 (m, 1H), 8.32 - 8.23 (m, 1H), 7.88 - 7.78 (m, 1H), 7.76 - 7.68 (m, 1H), 6.60 - 6.50 (m, 1H), 6.22 (s, 2H), 5.03 - 4.87 (m, 2H). | $PdCl_2$(dppf), $K_3PO_4$, dioxane |
| **INTD18** | 4-(6-ethoxypyrazin-2-yl) aniline 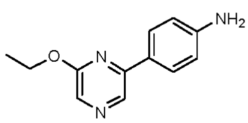 | Method F, [HPLC basic], 216, (1.78). | 8.59 (s, 1H), 8.00 (s, 1H), 7.86 - 7.75 (m, 2H), 6.69 - 6.59 (m, 2H), 5.59 (s, 2H), 4.43 (q, J = 7.0 Hz, 2H), 1.38 (t, J = 7.0 Hz, 3H). | $Pd(PPh_3)_4$, NaHCOs, MeCN |
| **INTD19** | 4-(6-(trifluoromethyl) pyrazin-2-yl)aniline 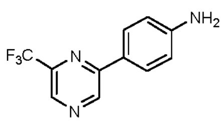 | Method F, [HPLC acidic], 240, (1.92). | 9.40 (s, 1H), 8.86 (s, 1H), 7.99 - 7.89 (m, 2H), 6.74 - 6.66 (m, 2H), 5.83 (s, 2H). | $Pd(PPh_3)_4$, NaHCOs, MeCN |
| **INTD20** | 4-(6-isopropoxypyrazin-2-yl)aniline 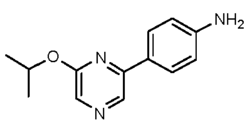 | Method F, [UPLC basic], 230, (1.31). | 8.57 (s, 1H), 7.95 (s, 1H), 7.87 - 7.73 (m, 2H), 6.73 - 6.56 (m, 2H), 5.59 (s, 2H), 5.45 - 5.27 (m, 1H), 1.47 - 1.25 (m, 6H). | $PdCl_2$(dppf), $K_3PO_4$, dioxane |

(continued)

| INTD | Name/Structure | Synthesis Method, [LCMS Method], m/z $(M+H)^+$, (Rt/min) | $^1H$ NMR Chemical Shift Data (DMSO-d6 unless stated) | Catalyst, Base, solvent |
|---|---|---|---|---|
| **INTD21** | 4-(6-cyclopropoxypyrazin-2-yl)aniline 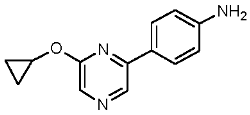 | Method F, [HPLC basic], 228, (1.83). | 8.19 - 8.02 (m, 1H), 7.58 - 7.41 (m, 1H), 7.40 - 7.22 (m, 2H), 6.21 - 5.99 (m, 2H), 5.09-4.99 (m, 2H), 3.86-3.73 (m, 1H), 0.37 - 0.09 (m, 4H). | $PdCl_2$(dppf), $K_3PO_4$, dioxane |
| **INTD22** | 4-(6-chloropyrazin-2-yl) aniline 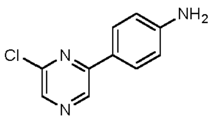 | Method F, [HPLC basic], 206, (1.75). | 9.06 (d, J = 0.6 Hz, 1H), 8.47 (d, J = 0.6 Hz, 1H), 7.89 - 7.81 (m, 2H), 6.73 - 6.62 (m, 2H), 5.79 (s, 2H). | $PdCl_2$(dppf), $K_3PO_4$, dioxane |
| **INTD23** | 2-fluoro-4-(pyrazin-2-yl) aniline 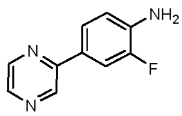 | Method F, [UPLC basic], 190, (0.84). | 9.12 (d, J = 1.6 Hz, 1H), 8.58 (dd, J = 2.6, 1.5 Hz, 1H), 8.44 (d, J = 2.5 Hz, 1H), 7.86 - 7.71 (m, 2H), 6.87 (dd, J = 9.3, 8.4 Hz, 1H), 5.67 (s, 2H). | $PdCl_2$(dppf), $K_2CO_3$, dioxane |
| **INTD24** | 4-(6-ethoxypyrazin-2-yl)-2-fluoroaniline 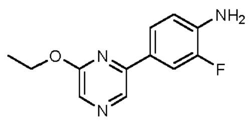 | Method F, [UPLC basic], 234, (1.31). | 8.66 (s, 1H), 8.06 (s, 1H), 7.84 - 7.63 (m, 2H), 6.93 - 6.75 (m, 1H), 5.65 (s, 2H), 4.54 - 4.34 (m, 2H), 1.47 - 1.29 (m, 3H). | $PdCl_2$(dppf), $K_2CO_3$, dioxane |
| **INTD25** | 2-fluoro-4-(6-(trifluoromethyl) pyrazin-2-yl)aniline 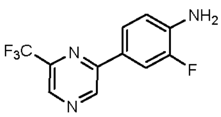 | Method F, [HPLC basic], 258, (2.13). | 9.46 (s, 1H), 8.92 (s, 1H), 7.88 (dd, J = 13.1, 2.1 Hz, 1H), 7.83 (dd, J = 8.4, 2.1 Hz, 1H), 6.90 (t, J = 8.8 Hz, 1H), 5.90 (s, 2H). | $PdCl_2$(dppf), $K_3PO_4$, dioxane |
| **INTD26** | 4-(6-chloropyrazin-2-yl)-2-methylaniline 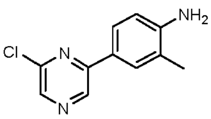 | Method F, [UPLC basic], 220, (1.24). | 9.06 (s, 1H), 8.46 (s, 1H), 7.84 - 7.65 (m, 2H), 6.78 - 6.63 (m, 1H), 5.55 (s, 2H), 2.14 (s, 3H). | $PdCl_2$(dppf), $K_3PO_4$, dioxane |
| **INTD27** | 4-(6-ethoxypyrazin-2-yl)-2-methylaniline 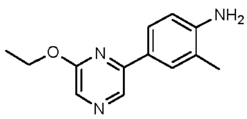 | Method F, [UPLC basic], 230, (1.27). | 8.60 (s, 1H), 8.00 (s, 1H), 7.80 - 7.63 (m, 2H), 6.75 - 6.61 (m, 1H), 5.35 (s, 2H), 4.51 - 4.33 (m, 2H), 2.13 (s, 3H), 1.45 - 1.31 (m, 3H). | $PdCl_2$(dppf), $K_3PO_4$, dioxane |

(continued)

| INTD | Name/Structure | Synthesis Method, [LCMS Method], m/z $(M+H)^+$, (Rt/min) | $^1H$ NMR Chemical Shift Data (DMSO-d6 unless stated) | Catalyst, Base, solvent |
|---|---|---|---|---|
| **INTD28** | 2-fluoro-4-(6-(2,2,2-trifluoroethoxy)pyrazin-2-yl) aniline 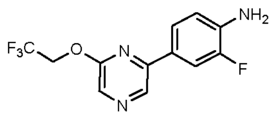 | Method F, [UPLC basic], 288, (1.38). | 8.82 (s, 1H), 8.24 (s, 1H), 7.94 - 7.85 (m, 1H), 7.81 - 7.74 (m, 1H), 6.92 - 6.82 (m, 1H), 5.72 (s, 2H), 5.23 - 5.09 (m, 2H). | $PdCl_2$(dppf), $K_3PO_4$, dioxane |
| **INTD29** | 4-(6-(2,2,2-trifluoroethoxy) pyrazin-2-yl)aniline 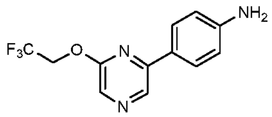 | Method F, [UPLC basic], 270, (1.31). | 8.76 (s, 1H), 8.18 (s, 1H), 7.95 - 7.84 (m, 2H), 6.73 - 6.58 (m, 2H), 5.67 (s, 2H), 5.22 - 5.04 (m, 2H). | $PdCl_2$(dppf), $K_3PO_4$, dioxane |
| **INTD30** | 5-(6-cyclopropoxypyrazin-2-yl)pyridin-2-amine 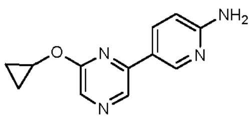 | Method F, [UPLC basic], 229, (0.98). | 8.81 - 8.64 (m, 2H), 8.18 - 8.00 (m, 2H), 6.55 (dd, J = 8.8, 0.8 Hz, 1H), 6.44 (s, 2H), 4.37 (tt, J = 6.2, 3.0 Hz, 1H), 0.94 - 0.69 (m, 4H). | $Pd(PPh_3)_4$, $Na_2CO_3$, Toluene, EtOH |
| **INTD31** | 5-(6-ethoxypyrazin-2-yl)-3-fluoropyridin-2-amine 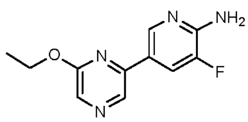 | Method F, [HPLC basic], 235, (1.73). | 8.72 (s, 1H), 8.62 - 8.57 (m, 1H), 8.12 (s, 1H), 8.04 (dd, J = 12.6, 1.9 Hz, 1H), 6.73 (s, 2H), 4.46 (q, J = 7.0 Hz, 2H), 1.39 (t, J = 7.0 Hz, 3H). | $PdCl_2$(dppf), $K_2CO_3$, dioxane |
| **INTD32** | 5-(6-isopropoxypyrazin-2-yl)pyridin-2-amine 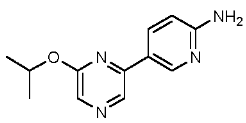 | Method F, [UPLC basic], 232, (1.09). | 8.69 (d, J = 2.4 Hz, 1H), 8.62 (s, 1H), 8.07 (dd, J = 8.7, 2.5 Hz, 1H), 8.02 (s, 1H), 6.55 (dd, J = 8.7, 0.8 Hz, 1H), 6.42 (s, 2H), 5.44 - 5.28 (m, 1H), 1.37 (d, J = 6.1 Hz, 6H). | $Pd(PPh_3)_4$, $Na_2CO_3$, dioxane |
| **INTD33** | 5-(6-ethoxypyrazin-2-yl) pyridin-2-amine 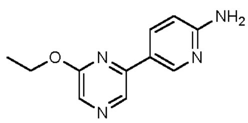 | Method F, [UPLC, basic], 217, (0.98). | 8.70 (dd, J = 2.5, 0.8 Hz, 1H), 8.64 (s, 1H), 8.10 - 8.06 (m, 2H), 6.54 (dd, J = 8.7, 0.8 Hz, 1H), 6.41 (s, 2H), 4.43 (q, J = 7.0 Hz, 2H), 1.38 (t, J = 7.0 Hz, 3H). | $PdCl_2$(dppf), $Cs_2CO_3$, dioxane |
| **INTD34** | 2-methyl-4-(6-(trifluoromethyl) pyrazin-2-yl)aniline 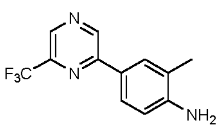 | Method F, [HPLC acidic], 254 (2.14). | 9.40 (s, 1H), 8.85 (s, 1H), 7.89 - 7.78 (m, 2H), 6.73 (d, J = 8.3 Hz, 1H), 5.60 (s, 2H), 2.15 (s, 3H). | $PdCl_2$(dppf), $K_3PO_4$, dioxane |

(continued)

| INTD | Name/Structure | Synthesis Method, [LCMS Method], m/z $(M+H)^+$, (Rt/min) | $^1H$ NMR Chemical Shift Data (DMSO-d6 unless stated) | Catalyst, Base, solvent |
|---|---|---|---|---|
| **INTD35** | 2-fluoro-4-(6-isopropoxypyrazin-2-yl) aniline 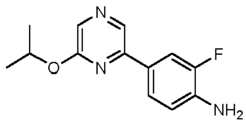 | Method F, [UPLC basic], 248 (1.41). | 8.64 (s, 1H), 8.01 (s, 1H), 7.82 - 7.63 (m, 2H), 6.92 - 6.80 (m, 1H), 5.67 (s, 2H), 5.45 - 5.28 (m, 1H), 1.46 - 1.24 (m, 6H). | $PdCl_2$(dppf), $K_2CO_3$, dioxane |
| **INTD36** | 4-(6-ethoxypyrazin-2-yl)-5-fluoro-2-methylaniline 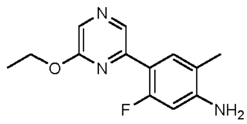 | Method G, [UPLC basic], 248 (1.36). | 8.45 (d, J = 2.2 Hz, 1H), 8.06 (s, 1H), 7.63 (d, J = 8.8 Hz, 1H), 6.47 (d, J = 14.2 Hz, 1H), 5.68 (s, 2H), 4.43 (q, J = 7.0 Hz, 2H), 2.09 (s, 3H), 1.39 (t, J = 7.0 Hz, 3H). | (i)$PdCl_2$(dppf) , KOAc, dioxane, then (ii)$PdCl_2$ (dppf ), $K_2CO_3$, dioxane |
| **INTD37** | 4-(6-ethoxypyrazin-2-yl)-5-fluoro-2-methoxyaniline 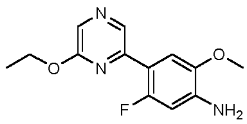 | Method G, [UPLC basic], 264 (1.37). | 8.50 (d, J = 2.0 Hz, 1H), 8.08 (s, 1H), 7.43 (d, J = 7.2 Hz, 1H), 6.51 (d, J = 13.5 Hz, 1H), 5.61 (s, 2H), 4.45 (q, J = 7.1 Hz, 2H), 3.83 (s, 3H), 1.40 (t, J = 7.0 Hz, 3H). | (i)$PdCl_2$(dppf) , KOAc, dioxane, then (ii)$PdCl_2$ (dppf ), $K_2CO_3$, dioxane |
| **INTD38** | 4-(6-ethoxypyrazin-2-yl)-2,3-dimethylaniline 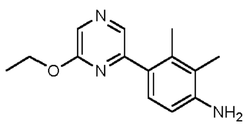 | Method G, [UPLC basic], 244 (1.28). | 8.18 (s, 1H), 8.10 (s, 1H), 7.02 (d, J = 8.3 Hz, 1H), 6.60 (d, J = 8.2 Hz, 1H), 5.11 (s, 2H), 4.36 (q, J = 7.0 Hz, 2H), 2.22 (s, 3H), 2.04 (s, 3H), 1.35 (t, J = 7.0 Hz, 3H). | (i)$PdCl_2$(dppf) , KOAc, dioxane, then (ii)$PdCl_2$ (dppf ), $K_2CO_3$, dioxane |
| **INTD39** | 4-(6-ethoxypyrazin-2-yl)-2-fluoro-5-methylaniline 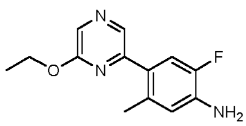 | Method G, [HPLC acidic], 248 (2.41). | 8.31 (s, 1H), 8.13 (s, 1H), 7.24 (d, J = 12.5 Hz, 1H), 6.68 (d, J = 9.1 Hz, 1H), 5.44 (s, 2H), 4.38 (q, J = 7.0 Hz, 2H), 2.32 (s, 3H), 1.36 (t, J = 7.0 Hz, 3H). | (i)$PdCl_2$(dppf) , KOAc, dioxane, then (ii)$PdCl_2$ (dppf ), $K_2CO_3$, dioxane |
| **INTD40** | 4-(6-ethoxypyrazin-2-yl)-2,5-dimethylaniline 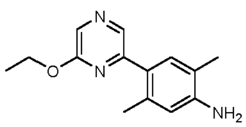 | Method G, [HPLC acidic], 244 (2.22). | 8.26 (s, 1H), 8.07 (s, 1H), 7.15 (s, 1H), 6.53 (s, 1H), 5.13 (s, 2H), 4.36 (q, J = 7.0 Hz, 2H), 2.31 (s, 3H), 2.07 (s, 3H), 1.36 (t, J = 7.1 Hz, 3H). | (i)$PdCl_2$(dppf) , KOAc, dioxane, then (ii)$PdCl_2$ (dppf ), $K_2CO_3$, dioxane |
| **INTD41** | 4-(6-ethoxypyrazin-2-yl)-2,6-difluoroaniline 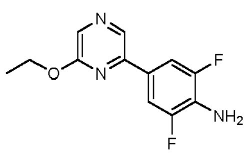 | Method G, [HPLC acidic], 252 (2.41). | 8.73 (s, 1H), 8.12 (s, 1H), 7.74 (dd, J = 8.0, 2.5 Hz, 2H), 5.73 (s, 2H), 4.46 (q, J = 7.0 Hz, 2H), 1.39 (t, J = 7.1 Hz, 3H). | (i)$PdCl_2$(dppf) , KOAc, dioxane, then (ii)$PdCl_2$ (dppf ), $K_2CO_3$, dioxane |

(continued)

| INTD | Name/Structure | Synthesis Method, [LCMS Method], m/z $(M+H)^+$, (Rt/min) | $^1H$ NMR Chemical Shift Data (DMSO-d6 unless stated) | Catalyst, Base, solvent |
|---|---|---|---|---|
| **INTD42** | 3'-ethoxy-[1,1'-biphenyl]-4-amine 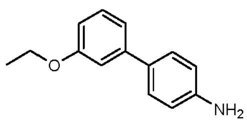 | Method F, [UPLC basic], 214 (1.39). | 7.37 - 7.32 (m, 2H), 7.29 - 7.24 (m, 1H), 7.09 (ddd, J = 7.7, 1.7, 0.9 Hz, 1H), 7.06 - 7.01 (m, 1H), 6.77 (ddd, J = 8.1, 2.5, 1.0 Hz, 1H), 6.63 (d, J = 8.3 Hz, 2H), 5.22 (s, 2H), 4.07 (q, J = 7.0 Hz, 2H), 1.34 (t, J = 6.9 Hz, 3H) | $PdCl_2$(dppf), $K_2CO_3$, dioxane |
| **INTD43** | 4-(pyrazin-2-yl)aniline 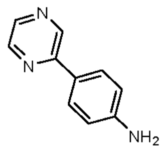 | Method F, [UPLC acidic], 172 (0.56). | 9.05 (d, J = 1.6 Hz, 1H), 8.54 (dd, J = 2.5, 1.6 Hz, 1H), 8.38 (d, J = 2.5 Hz, 1H), 7.93 - 7.77 (m, 2H), 6.72 - 6.62 (m, 2H), 5.61 (s, 2H). | $PdCl_2$(dppf), $K_2CO_3$, dioxane |
| **INTD44** | 5'-(trifluoromethyl)-[3,3'-bipyridin]-6-amine 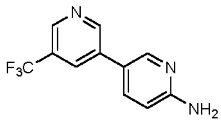 | Method F, [HPLC basic], 240 (1.59). | 9.13 (d, J = 2.2 Hz, 1H), 8.84 (dd, J = 2.1, 1.0 Hz, 1H), 8.44 (dd, J = 2.6, 0.8 Hz, 1H), 8.40 - 8.33 (m, 1H), 7.90 (dd, J = 8.7, 2.6 Hz, 1H), 6.57 (dd, J = 8.7, 0.8 Hz, 1H), 6.32 (s, 2H). | $PdCl_2$(dppf), $K_3PO_4$, dioxane |
| **INTD45** | 4-(5-methoxypyridin-3-yl) aniline 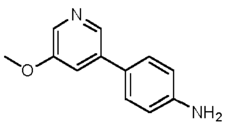 | Method E [UPLC acidic], 201 (0.91). | 8.38 (d, J = 1.9 Hz, 1H), 8.13 (d, J = 2.8 Hz, 1H), 7.49 - 7.40 (m, 3H), 6.71 - 6.62 (m, 2H), 5.35 (s, 2H), 3.88 (s, 3H). | $PdCl_2$(dppf), $K_2CO_3$, dioxane |
| **INTD46** | 2-fluoro-4-(6-methoxypyrazin-2-yl)aniline 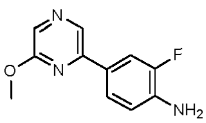 | Method F, [UPLC basic], 220 (1.20). | 8.68 (s, 1H), 8.09 (s, 1H), 7.81 (dd, J = 13.1, 2.0 Hz, 1H), 7.74 (dd, J = 8.4, 2.0 Hz, 1H), 6.90 - 6.82 (m, 1H), 5.66 (s, 2H), 3.99 (s, 3H). | $PdCl_2$(dppf), $K_2CO_3$, dioxane |
| **INTD47** | 4-(6-ethoxypyrazin-2-yl)-2-(trifluoromethoxy)aniline 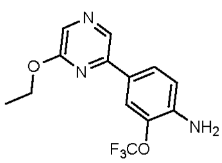 | Method F, [HPLC basic], 246 (1.99). | 8.67 (s, 1H), 8.08 (s, 1H), 7.93 - 7.78 (m, 2H), 6.91 (d, J = 8.5 Hz, 1H), 5.91 (s, 2H), 4.43 (q, J = 7.0 Hz, 2H), 1.39 (t, J = 7.0 Hz, 3H). | $PdCl_2$(dppf), $K_3PO_4$, dioxane |

(continued)

| INTD | Name/Structure | Synthesis Method, [LCMS Method], m/z $(M+H)^+$, (Rt/min) | $^1H$ NMR Chemical Shift Data (DMSO-d6 unless stated) | Catalyst, Base, solvent |
|---|---|---|---|---|
| **INTD48** | 4-(6-ethoxypyrazin-2-yl)-2-methoxyaniline 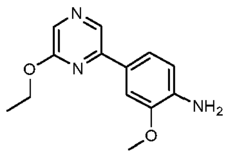 | Method F, [HPLC basic], 300 (2.38). | 8.67 (s, 1H), 8.02 (s, 1H), 7.62 - 7.46 (m, 2H), 6.72 (d, J = 8.0 Hz, 1H), 5.24 (s, 2H), 4.45 (q, J = 7.0 Hz, 2H), 3.87 (s, 3H), 1.39 (t, J = 7.0 Hz, 3H). | $PdCl_2$(dppf), $K_3PO_4$, dioxane |
| **INTD49** | 2-chloro-4-(6-ethoxypyrazin-2-yl)aniline 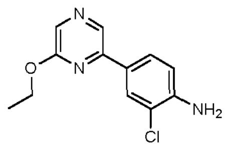 | Method F, [HPLC basic], 250 (2.26). | 8.66 (d, J = 0.5 Hz, 1H), 8.07 (d, J = 0.5 Hz, 1H), 7.99 (d, J = 2.1 Hz, 1H), 7.83 (dd, J = 8.5, 2.1 Hz, 1H), 6.89 (d, J = 8.5 Hz, 1H), 5.85 (s, 2H), 4.44 (q, J = 7.0 Hz, 2H), 1.39 (t, J = 7.0 Hz, 3H). | $PdCl_2$(dppf), $K_3PO_4$, dioxane |
| **INTD50** | 2-fluoro-4-(pyridin-3-yl) aniline 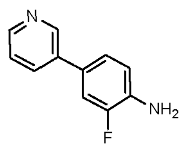 | Method E, [UPLC basic], 189 (0.90). | 8.82 (dd, J = 2.5, 0.9 Hz, 1H), 8.45 (dd, J = 4.7, 1.6 Hz, 1H), 7.97 (ddd, J = 8.0, 2.5, 1.6 Hz, 1H), 7.45 (dd, J = 13.0, 2.1 Hz, 1H), 7.40 (ddd, J = 8.0, 4.7, 0.9 Hz, 1H), 7.31 (dd, J = 8.2, 2.1 Hz, 1H), 6.86 (dd, J = 9.5, 8.3 Hz, 1H), 5.39 (s, 2H). | $PdCl_2$(dppf), $K_2CO_3$, dioxane |
| **INTD51** | 2-amino-5-(6-ethoxypyrazin-2-yl) benzonitrile 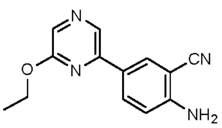 | Method G, [HPLC basic], 241 (1.99). | 8.70 (s, 1H), 8.19 (d, J = 2.2 Hz, 1H), 8.13 - 8.06 (m, 2H), 6.91 (d, J = 8.9 Hz, 1H), 6.52 (s, 2H), 4.45 (q, J = 7.0 Hz, 2H), 1.39 (t, J = 7.0 Hz, 3H). | (i) $PdCl_2$(dppf), KOAc, dioxane, then (ii)$PdCl_2$ (dppf ), $K_2CO_3$, dioxane |
| **INTD52** | 4-(6-(prop-1-en-2-yl) pyrazin-2-yl)aniline 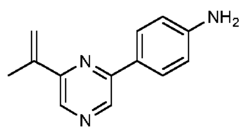 | Method F, Using INTD60 [HPLC basic], 212, (1.93). | 8.93 (s, 1H), 8.65 (s, 1H), 7.91 - 7.88 (m, 2H), 6.69 - 6.65 (m, 2H), 6.09 - 6.07 (m, 1H), 5.59 (s, 2H), 5.45 - 5.43 (m, 1H), 2.22 - 2.21 (m, 3H). | $PdCl_2$(dppf), $K_3PO_4$, dioxane |
| **INTD53** | 6-(6-ethoxypyrazin-2-yl) pyridin-3-amine 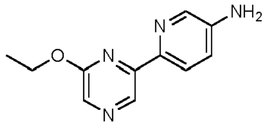 | Method E, [HPLC basic], 217, (1.60). | 8.88 (s, 1H), 8.12 (s, 1H), 8.06 - 7.97 (m, 2H), 7.05 (dd, J = 8.5, 2.7 Hz, 1H), 5.84 (s, 2H), 4.45 (q, J = 7.0 Hz, 2H), 1.39 (t, J = 7.0 Hz, 3H). | $PdCl_2$(dppf), $K_2CO_3$, dioxane |

(continued)

| INTD | Name/Structure | Synthesis Method, [LCMS Method], m/z $(M+H)^+$, (Rt/min) | $^1H$ NMR Chemical Shift Data (DMSO-d6 unless stated) | Catalyst, Base, solvent |
|---|---|---|---|---|
| **INTD54** | 5-(6-cyclopropylpyrazin-2-yl)pyridin-2-amine 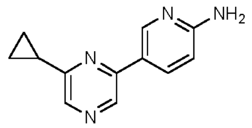 | Method F, No LCMS data | 8.82 (s, 1H), 8.66 (d, J = 2.5 Hz, 1H), 8.41 (s, 1H), 8.05 (dd, J = 8.7, 2.5 Hz, 1H), 6.53 (d, J = 8.7 Hz, 1H), 6.38 (s, 2H), 2.22 - 2.15 (m, 1H), 1.07 - 1.01 (m, 4H). | $PdCl_2$(dppf), $K_2CO_3$, dioxane |
| **INTD55** | 4-(6-cyclopropylpyrazin-2-yl)-2-fluoroaniline 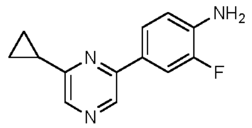 | Method F, No LCMS data | 8.82 (s, 1H), 8.39 (s, 1H), 7.74 (dd, J = 13.2, 1.9 Hz, 1H), 7.68 (dd, J = 8.3, 1.9 Hz, 1H), 6.87 6.82 (m, 1H), 5.62 (s, 2H), 2.23 - 2.14 (m, 1H), 1.07 - 0.94 (m, 4H). | $PdCl_2$(dppf), $K_2CO_3$, dioxane |
| **INTD56** | 6-(4-aminophenyl)-N, N-dimethylpyrazin-2-amine 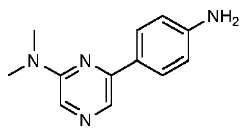 | Method F, [UPLC basic], 215, (1.02). | 8.22 (s, 1H), 7.91 (s, 1H), 7.83 - 7.76 (m, 2H), 6.67 - 6.60 (m, 2H), 5.47 (s, 2H), 3.11 (s, 6H). | $PdCl_2$(dppf), $K_2CO_3$, dioxane |
| **INTD57** | 5'-chloro-[3,3'-bipyridin]-6-amine 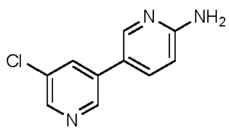 | Method E, [HPLC acidic], 205, (0.48). | 8.79 (d, J = 2.2 Hz, 1H), 8.49 (d, J = 2.2 Hz, 1H), 8.37 (d, J = 2.5 Hz, 1H), 8.15 - 8.13 (m, 1H), 7.82 (dd, J = 8.7, 2.5 Hz, 1H), 6.54 (dd, J = 8.7, 0.7 Hz, 1H), 6.28 (s, 2H). | $PdCl_2$(dppf), $K_3PO_4$, dioxane |
| **INTD58** | 5-(6-ethoxypyrazin-2-yl)-3-methylpyridin-2-amine 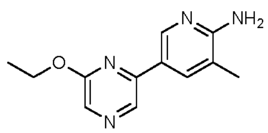 | Method G, [UPLC acidic], 231, (1.07). | 8.64 (s, 1H), 8.59 (d, J = 2.3 Hz, 1H), 8.06 (s, 1H), 7.96 - 7.94 (m, 1H), 6.21 (s, 2H), 4.44 (q, J = 7.1 Hz, 2H), 2.12 (s, 3H), 1.38 (t, J = 7.1 Hz, 3H). | (i)$PdCl_2$(dppf) , KOAc, dioxane, then (ii)$PdCl_2$ (dppf ), $K_2CO_3$, dioxane |
| **INTD59** | 5-(6-(2,2,2-trifluoroethoxy) pyrazin-2-yl)pyridin-2-amine 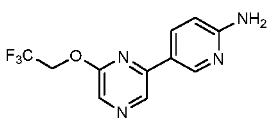 | Method F, [UPLC basic], 271, (1.11). | 8.84 - 8.80 (m, 1H), 8.79 - 8.75 (m, 1H), 8.30 - 8.23 (m, 1H), 8.19 - 8.12 (m, 1H), 6.62 - 6.52 (m, 1H), 6.49 (s, 2H), 5.23 - 5.06 (m, 2H). | $PdCl_2$(dppf), $K_3PO_4$, dioxane |

2-Chloro-6-(prop-1-en-2-yl)pyrazine **INTD60**

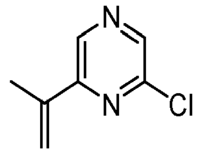

A solution of 2,6-dichloropyrazine (1.0 g, 6.71 mmol) and 4,4,5,5-tetramethyl-2-(prop-1-en-2-yl)-1,3,2-dioxaborolane (1.13 g, 6.72 mmol) in dioxane (60 mL) was treated with 2M $K_2CO_3$ (aq, 8.4 mL, 16.8 mmol) then degassed ($N_2$, 5 mins) and heated to 40 °C. $PdCl_2$(dppf)-DCM adduct (274 mg, 0.336 mmol) was added and the mixture further degassed ($N_2$, 5 mins) before the reaction was heated to 70 °C for 1 hr. The reaction was allowed to cool to RT then treated with 1M HCl (aq, 40 mL) and EtOAc (40 mL). This was passed through celite, the phases were separated and the aqueous phase was further extracted with EtOAc (2 × 20 mL). The organic phases were combined, dried ($MgSO_4$), filtered and concentrated onto silica (4 g). The crude product was purified by chromatography on silica gel (24 g column, 0-15% EtOAc/iso-hexane) to afford 2-chloro-6-(prop-1-en-2-yl)pyrazine (1.0 g, 3.75 mmol, 56% yield) as a brown gum; Rt 1.96 mins (HPLC acidic); m/z none observed. $^1H$ NMR (500 MHz, DMSO-d6) δ 8.94 (s, 1H), 8.69 (s, 1H), 6.11 - 6.08 (m, 1H), 5.55 - 5.52 (m, 1H), 2.16 - 2.14 (m, 3H).

5-(6-(Prop-1-en-2-yl)pyrazin-2-yl)pyridin-2-amine **INTD61**

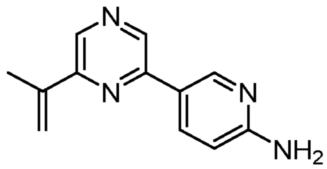

A solution of 2-chloro-6-(prop-1-en-2-yl)pyrazine **INTD60** (1 g, 3.75 mmol) and 5-(4,4,5,5-tetramethyl-1,3,2-dioxaborolan-2-yl)pyridin-2-amine (1.65 g, 7.50 mmol) in dioxane (60 mL) was treated with 2M $K_2CO_3$ (aq, 7.5 mL, 15.00 mmol) then degassed ($N_2$, 5 mins) and heated to 40 °C. $PdCl_2$(dppf)-DCM adduct (0.306 g, 0.375 mmol) was added and the mixture degassed further ($N_2$, 5 mins) and the reaction was heated to 70 °C for 1 hr. The reaction was allowed to cool to RT then concentrated (to approx. 10 mL). This was then treated with 1M HCl (aq, 37.5 mL) and EtOAc (40 mL) and filtered over celite eluting with EtOAc (50 mL). The phases were partitioned and the organic phase was discarded. The aqueous phase was then brought to pH 10 by addition of solid $Na_2CO_3$ and then extracted with EtOAc (3 × 50 mL). The organic phases were combined, dried ($MgSO_4$), filtered and concentrated onto silica (5 g) and the crude product was purified by chromatography on silica gel (24 g column, 30-100% EtOAc/iso-hexane) to afford 5-(6-(prop-1-en-2-yl)pyrazin-2-yl)pyridin-2-amine (320 mg, 1.43 mmol, 38% yield) as an off-white solid; Rt 0.98mins (HPLC acidic); m/z 213 $(M+H)^+$ $(ES^+)$; $^1H$ NMR (500 MHz, DMSO-d6) δ 9.00 (s, 1H), 8.78 - 8.75 (m, 1H), 8.74 (s, 1H), 8.16 (dd, J = 8.7, 2.5 Hz, 1H), 6.57 (dd, J = 8.7, 0.7 Hz, 1H), 6.43 (s, 2H), 6.12 - 6.09 (m, 1H), 5.48 - 5.45 (m, 1H), 2.23 (s, 3H).

4-(6-Isopropylpyrazin-2-yl)aniline **INTD62**

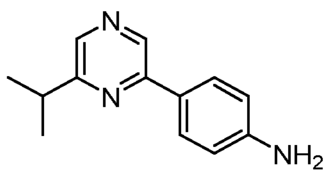

A solution of 4-(6-(prop-1-en-2-yl)pyrazin-2-yl)aniline **INTD52** (380 mg, 1.44 mmol) in MeOH (10 mL) was hydrogenated using the H-Cube flow hydrogenation apparatus (10% Pd/C, 30x4 mm, Full hydrogen, 25 °C, 1 mL/min). The reaction mixture was concentrated to afford 4-(6-isopropylpyrazin-2-yl)aniline (296 mg, 1.37 mmol, 95% yield) as an orange oil; Rt 1.74 mins (HPLC basic); m/z 214 $(M+H)^+$ $(ES^+)$; $^1H$ NMR (500 MHz, DMSO-d6) δ 8.85 (s, 1H), 8.31 (s, 1H), 7.89 - 7.82 (m, 2H), 6.68 - 6.63 (m, 2H), 5.56 (s, 2H), 3.08 (hept, J = 6.9 Hz, 1H), 1.29 (d, J = 6.9 Hz, 6H).

2-Methoxy-6-(4,4,5,5-tetramethyl-1,3,2-dioxaborolan-2-yl)pyrazine **INTD63**

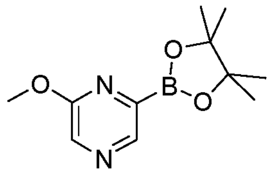

To a solution of 2-bromo-6-methoxypyrazine (500 mg, 2.65 mmol) in 1,4-dioxane (15 mL) was successively added bispin (739 mg, 2.91 mmol) and KOAc (1.04 g, 10.58 mmol). The resulting mixture was degassed ($N_2$), and $PdCl_2$(dppf)-$CH_2Cl_2$ adduct (108 mg, 0.132 mmol) was added. The resulting mixture was heated at 110 °C for 2.5 hrs. The mixture was cooled to RT, filtered through celite and the solvent was removed *in vacuo.* The crude product was purified by chromatography on silica gel (24 g cartridge, 0-50% EtOAc/isohexane). The residue was dissolved in EtOAc (20 mL) and washed with water (3 × 10 mL). The organic layer was dried over $Na_2SO_4$ filtered and concentrated *in vacuo* to afford 2-methoxy-6-(4,4,5,5-tetramethyl-1,3,2-dioxaborolan-2-yl)pyrazine (281 mg, 0.845 mmol, 32% yield) as a pale tan solid. $^1$H NMR (500 MHz, DMSO-d6) δ 8.41 (s, 1H), 8.34 (s, 1H), 3.93 (s, 3H), 1.33 (s, 12H).

**Method K: Suzuki coupling**

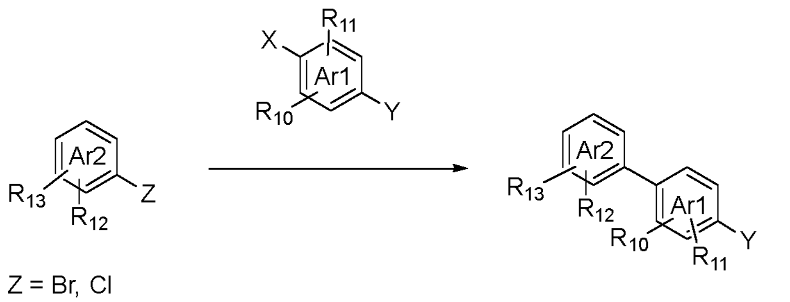

Z = Br, Cl
X = $B(OH)_2$, $B(pin)_2$

Y = $CO_2$t-Bu, $CO_2$Me, CN

A solution of boronic acid (1 eq), aryl halide (1.05 eq.) and $Cs_2CO_3$ (3 eq.) in a mixture of dioxane (40 volumes) and water (6 volumes) was degassed ($N_2$, 5 mins). $PdCl_2$(dppf).$CH_2Cl_2$ (5 mol%) was added and the reaction was further degassed ($N_2$) before being heated to 90 °C for 18 hrs. The reaction mixture was filtered through celite before an aqueous workup was undertaken, followed by purification by normal phase chromatography.

**Table 15:** The following intermediates were made according to Method K. All aryl-halides are commercially available.

| INTD | Name/Structure | Synthesis Method, [LCMS Method], m/z $(M+H)^+$, (Rt/min) | $^1$H NMR Chemical Shift Data (DMSO-$d_6$ unless stated) |
|---|---|---|---|
| **INTD64** | *tert*-butyl 4-(5-(trifluoromethyl) pyridin-3-yl)benzoate 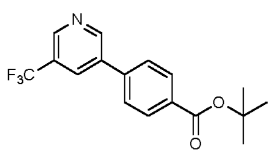 | Using Ar2Br, [HPLC acidic], 324 (2.78). | 9.27 (d, J = 2.2 Hz, 1H), 9.03 (dd, J = 2.2, 1.0 Hz, 1H), 8.61 - 8.41 (m, 1H), 8.22 - 7.83 (m, 4H), 1.58 (s, 9H). |

(continued)

| INTD | Name/Structure | Synthesis Method, [LCMS Method], m/z $(M+H)^+$, (Rt/min) | $^1H$ NMR Chemical Shift Data (DMSO-$d_6$ unless stated) |
|---|---|---|---|
| **INTD65** | *tert*-butyl 4-(6-(trifluoromethyl) pyrazin-2-yl)benzoate 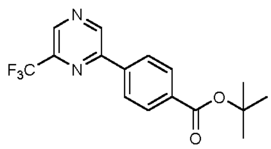 | Using Ar2Cl, [HPLC acidic], no ionisation (2.83). | 9.70 (s, 1H), 9.23 (s, 1H), 8.40 - 8.30 (m, 2H), 8.12 - 8.03 (m, 2H), 1.59 (s, 9H). |
| **INTD66** | methyl 4-(5-chloropyridin-3-yl) benzoate 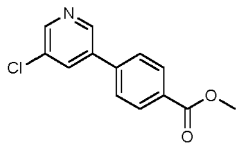 | Using Ar2Cl, [HPLC acidic], 247 $^{35}Cl$ isotope, (2.20). | 8.94 (d, J = 2.0 Hz, 1H), 8.69 (d, J = 2.2 Hz, 1H), 8.35 (t, J = 2.2 Hz, 1H), 8.10 - 8.01 (m, 2H), 8.00 - 7.89 (m, 2H), 3.89 (s, 3H). |
| **INTD67** | methyl 2-fluoro-4-(5-(trifluoromethyl)pyridin-3-yl)benzoate 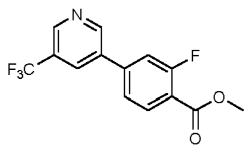 | Using Ar2Cl [HPLC acidic], 300 (2.30). | No $^1H$ NMR recorded. |
| **INTD68** | methyl 4-(5-chloropyridin-3-yl)-2-fluorobenzoate 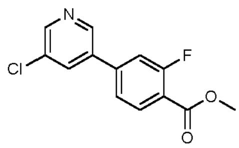 | Using Ar2Br [HPLC acidic], 266 $^{35}Cl$ isotope (2.20). | No $^1H$ NMR recorded. |
| **INTD69** | methyl 4-(6-ethoxypyrazin-2-yl)-2-fluorobenzoate 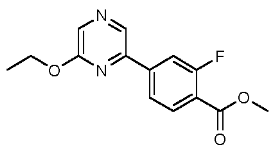 | Using Ar2Cl, [UPLC acidic], 277 (1.53). | 8.94 (s, 1H), 8.34 (s, 1H), 8.12 - 8.08 (m, 2H), 8.04 - 8.00 (m, 1H), 4.50 (q, J = 7.0 Hz, 2H), 3.89 (s, 3H), 1.41 (t, J = 7.0 Hz, 3H). |
| **INTD70** | methyl 2-fluoro-4-(6-isopropoxypyrazin-2-yl) benzoate 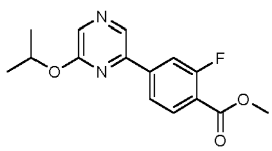 | Using Ar2Cl, [UPLC acidic], 291 (1.63). | 8.91 (s, 1H), 8.28 (s, 1H), 8.13 - 7.94 (m, 3H), 5.43 (hept, J = 6.1 Hz, 1H), 3.89 (s, 3H), 1.39 (d, J = 6.2 Hz, 6H). |

(continued)

| INTD | Name/Structure | Synthesis Method, [LCMS Method], m/z $(M+H)^+$, (Rt/min) | $^1H$ NMR Chemical Shift Data (DMSO-$d_6$ unless stated) |
|---|---|---|---|
| **INTD71** | methyl 4-(6-ethoxypyrazin-2-yl)-2-(trifluoromethyl)benzoate 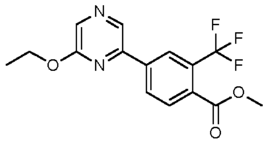 | Using Ar2Cl, [UPLC acidic], 327 (2.59) | 9.01 (d, J = 1.6 Hz, 1H), 8.56 - 8.52 (m, 2H), 8.37 (d, J = 1.6 Hz, 1H), 8.01 (d, J = 8.4 Hz, 1H), 4.58 - 4.41 (m, 2H), 3.91 (s, 3H), 1.42 (t, J = 7.0 Hz, 3H). |
| **INTD72** | *tert*-butyl 4-(6-isopropoxypyrazin-2-yl) benzoate 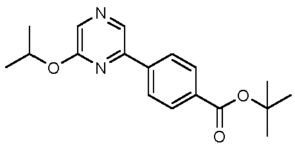 | Using Ar2Cl, [UPLC acidic], 315 (1.97). | 8.86 (s, 1H), 8.30 - 8.21 (m, 3H), 8.06 - 8.02 (m, 2H), 5.41 (hept, J = 6.2 Hz, 1H), 1.58 (s, 9H), 1.40 (d, J = 6.2 Hz, 6H). |
| **INTD73** | *tert*-butyl 4-(6-ethoxypyrazin-2-yl)benzoate 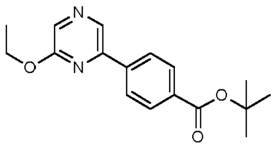 | Using Ar2Cl, [HPLC acidic], 301 (2.89). | 8.87 (s, 1H), 8.30 (s, 1H), 8.26 - 8.22 (m, 2H), 8.06 - 7.98 (m, 2H), 4.48 (q, J = 7.1 Hz, 2H), 1.57 (s, 9H), 1.40 (t, J = 7.0 Hz, 3H). |

**Method L: Ester deprotection with TFA**

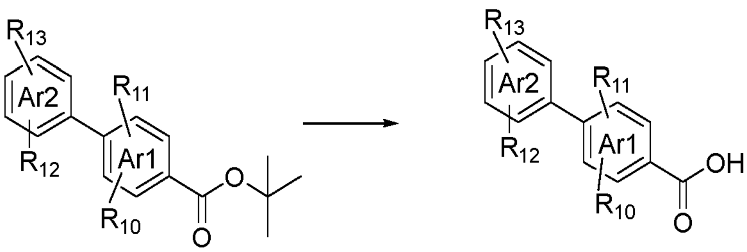

A solution of the ester (1 eq) in DCM (20 volumes) was treated with TFA (10 eq.) and stirred at RT for 3 hrs. The reaction mixture was then concentrated and azeotroped with MeOH and MeCN. No further purification was undertaken.

**Method M: Ester deprotection with base**

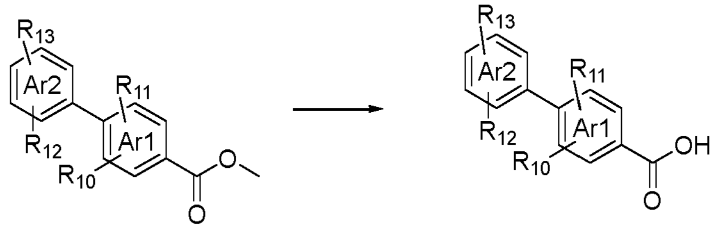

A solution of the ester (1 eq) in a mixture of THF/MeOH (4/1 volumes) was treated with LiOH (2.2-6 eq.) and stirred between RT and 50 °C for between 3 hrs and 18 hrs. The organic solvents were removed *in vacuo* then acidified with 1 M HCl and extracted with EtOAc. The organic phases were combined, dried ($Na_2SO_4$), filtered and concentrated. The products were used directly in the next step with no further purification undertaken.

**Method N: Potassium salt formation**

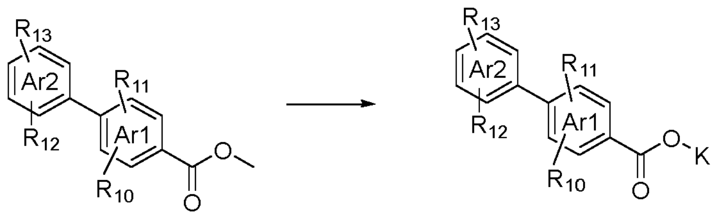

A solution of the ester (1 eq.) in THF (4 volumes) was treated with TMSOK (1 eq.) and stirred at RT for 2 hrs before the reaction mixtures were filtered and washed with iso-hexanes. The products were used directly in the next step with no further purification undertaken.

**Table 16:** The following intermediates were made according to Method L-N.

| INTD | Name/Structure | Synthesis Method, [LCMS Method], m/z $(M+H)^+$, (Rt/min) | $^1H$ NMR Chemical Shift Data (DMSO-$d_6$ unless stated) |
|---|---|---|---|
| **INTD74** | 4-(6-ethoxypyrazin-2-yl)-2-fluorobenzoic acid 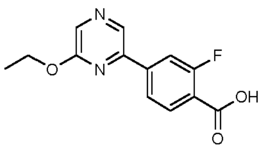 | Method M, Using **INTD69,** [HPLC acidic], 263 (2.07). | 13.40 (s, 1H), 8.94 (s, 1H), 8.34 (s, 1H), 8.12 - 8.03 (m, 2H), 8.03 - 7.92 (m, 1H), 4.50 (q, J = 7.0 Hz, 2H), 1.41 (t, J = 7.0 Hz, 3H). |
| **INTD75** | 4-(6-(trifluoromethyl)pyrazin-2-yl)benzoic acid 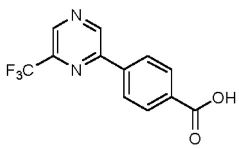 | Method L, Using **INTD65,** [UPLC acidic], 269 (1.33). | 13.25 (s, 1H), 9.70 (s, 1H), 9.23 (s, 1H), 8.42 - 8.20 (m, 2H), 8.20 - 8.00 (m, 2H). |
| **INTD76** | potassium 4-(5-chloropyridin-3-yl)-2-fluorobenzoate 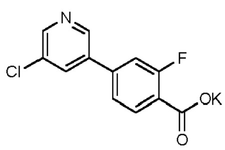 | Method N, Using **INTD68,** [HPLC acidic], 251 $^{35}Cl$ isotope, ionises as free acid, (1.88). | 8.91 - 8.85 (m, 1H), 8.63 - 8.54 (m, 1H), 8.30 - 8.20 (m, 1H), 7.59 - 7.49 (m, 1H), 7.49 - 7.34 (m, 2H). |

(continued)

| INTD | Name/Structure | Synthesis Method, [LCMS Method], m/z (M+H)+, (Rt/min) | $^{1}$H NMR Chemical Shift Data (DMSO-$d_6$ unless stated) |
|---|---|---|---|
| **INTD77** | 4-(5-(trifluoromethyl)pyridin-3-yl)benzoic acid 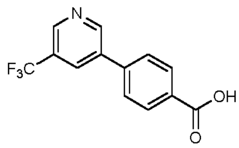 | Method L, Using **INTD64,** [HPLC acidic], 268 (2.01). | 13.12 (s, 1H), 9.28 (d, J = 2.2 Hz, 1H), 9.03 (dd, J = 2.2, 1.0 Hz, 1H), 8.56 (d, J = 2.2 Hz, 1H), 8.13 - 8.04 (m, 2H), 8.04 - 7.86 (m, 2H). |
| **INTD78** | potassium 2-fluoro-4-(5-(trifluoromethyl)pyridin-3-yl)benzoate 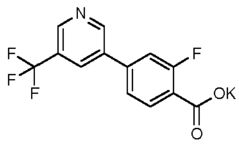 | Method N, Using **INTD67** [HPLC acidic], 286 ionises as free acid, (2.01). | 9.22 (d, J = 2.2 Hz, 1H), 8.95 (d, J = 2.2 Hz, 1H), 8.48 (d, J = 2.3 Hz, 1H), 7.64 -7.45 (m, 3H). |
| **INTD79** | potassium 4-(5-chloropyridin-3-yl)benzoate 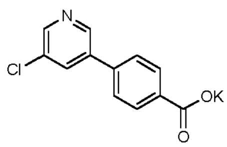 | Method N, Using **INTD66,** [UPLC acidic], 234 $^{35}$Cl isotope, ionises as free acid, (1.18). | 8.87 (d, J = 2.0 Hz, 1H), 8.59 (d, J = 2.2 Hz, 1H), 8.23 (t, J = 2.2 Hz, 1H), 7.95 - 7.86 (m, 2H), 7.72 - 7.55 (m, 2H). |
| **INTD80** | 4-(6-ethoxypyrazin-2-yl)-2-(trifluoromethyl)benzoic acid 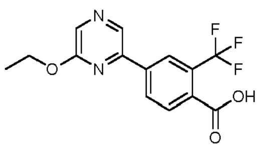 | Method M, Using **INTD71,** [UPLC acidic], 313 (2.30) | 13.75 (s, 1H), 8.98 (s, 1H), 8.52 - 8.46 (m, 2H), 8.35 (s, 1H), 7.97 (d, J = 7.9 Hz, 1H), 4.49 (q, J = 7.0 Hz, 2H), 1.42 (t, J = 7.0 Hz, 3H). |
| **INTD81** | 2-fluoro-4-(6-isopropoxypyrazin-2-yl)benzoic acid 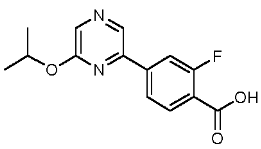 | Method M, Using **INTD70,** [HPLC acidic], 277 (2.24). | 13.53 (s, 1H), 8.90 (s, 1H), 8.27 (s, 1H), 8.08 - 7.87 (m, 3H), 5.43 (hept, J = 6.2 Hz, 1H), 1.39 (d, J = 6.2 Hz, 6H). |
| **INTD82** | 4-(6-isopropoxypyrazin-2-yl) benzoic acid 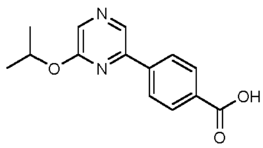 | Method L, Using **INTD72,** [UPLC acidic], 259 (1.40). | 13.13 (s, 1H) 8.87 (s, 1H), 8.27 - 8.20 (m, 3H), 8.09 - 8.05 (m, 2H), 5.43 (p, J = 6.2 Hz, 1H), 1.40 (d, J = 6.2 Hz, 6H). |

(continued)

| INTD | Name/Structure | Synthesis Method, [LCMS Method], m/z $(M+H)^+$, (Rt/min) | $^1$H NMR Chemical Shift Data (DMSO-$d_6$ unless stated) |
|---|---|---|---|
| **INTD83** | 4-(6-ethoxypyrazin-2-yl)benzoic acid 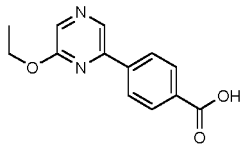 | Method L, Using **INTD73,** [UPLC acidic], 245 (1.29) | 13.15 (v. br. s, 1H), 8.89 (s, 1H), 8.31 (s, 1H), 8.29 - 8.22 (m, 2H), 8.11 - 8.01 (m, 2H), 4.51 (q, J = 7.0 Hz, 2H), 1.42 (t, J = 7.0 Hz, 3H). |

(5-(6-Ethoxypyrazin-2-yl)pyridin-2-yl)methanol **INTD84**

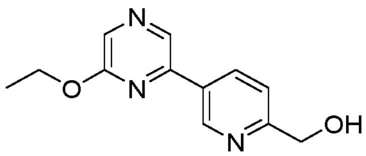

A suspension of (5-bromopyridin-2-yl)methanol (1.00 g, 5.32 mmol), Bispin (1.5 g, 5.91 mmol) and KOAc (1.6 g, 16.0 mmol) in dioxane (20 mL) was heated to 30 °C then degassed ($N_2$). $PdCl_2$(dppf)-$CH_2Cl_2$ (0.217 g, 0.266 mmol) was added and the reaction mixture was heated to 90 °C for 2 hrs. The reaction mixture was cooled to 40 °C whereupon 2-chloro-6-ethoxypyrazine (900 mg, 5.68 mmol), $Cs_2CO_3$ (3.47 g, 10.6 mmol) and water (5 mL) were added. The mixture was degassed ($N_2$), then $PdCl_2$(dppf)-$CH_2Cl_2$ (0.217 g, 0.266 mmol) was added and the mixture was again degassed ($N_2$). The reaction mixture was then heated to 90 °C for 18 hrs. The reaction mixture was part concentrated (to approx. 5 mL) then taken up with water (20 mL) and EtOAc (50 mL) and passed through celite, eluting with EtOAc (20 mL). The phases were then diluted with water (20 mL) and partitioned. The organic phase was washed with brine (30 mL), dried ($Na_2SO_4$), filtered and concentrated onto silica (5 g). The crude product was purified by chromatography on silica (40 g cartridge, 0-100% EtOAc/*iso*-hexanes) to afford (5-(6-ethoxypyrazin-2-yl)pyridin-2-yl)methanol (675 mg, 2.86 mmol, 54% yield) as a brown solid. Rt 1.24 min (HPLC, acidic); m/z 232 $(M+H)^+$ $(ES^+)$; $^1$H NMR (500 MHz, DMSO-d6) δ 9.27 - 9.09 (m, 1H), 8.87 (s, 1H), 8.49 (dd, J = 8.2, 2.3 Hz, 1H), 8.29 (s, 1H), 7.62 (d, J = 8.2 Hz, 1H), 5.53 (t, J = 5.9 Hz, 1H), 4.64 (d, J = 5.9 Hz, 2H), 4.50 (q, J = 7.1 Hz, 2H), 1.41 (t, J = 7.1 Hz, 3H).

5-(6-Ethoxypyrazin-2-yl)picolinaldehyde **INTD85**

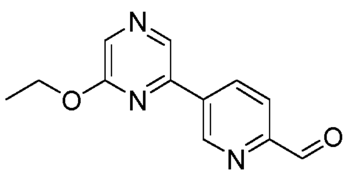

A solution of (5-(6-ethoxypyrazin-2-yl)pyridin-2-yl)methanol **INTD84** (375 mg, 3.18 mmol) in $CH_2Cl_2$ (15 mL) was treated with manganese dioxide (3 g, 34.5 mmol). The reaction was stirred for 4 hrs at RT then filtered through celite and concentrated onto silica (4 g). The crude product was purified by chromatography on silica (24 g cartridge, 0-100% EtOAc/iso-hexanes) to afford 5-(6-ethoxypyrazin-2-yl)picolinaldehyde (309 mg, 1.32 mmol, 42% yield) as a colourless solid. Rt 1.85 min (HPLC, acidic); m/z 230 $(M+H)^+$ $(ES^+)$; $^1$H NMR (500 MHz, OMSO-$d_6$) δ 10.07 (d, J = 0.8 Hz, 1H), 9.55 (dd, J = 2.2, 0.9 Hz, 1H), 9.03 (s, 1H), 8.73 (ddd, J = 8.1, 2.2, 0.8 Hz, 1H), 8.39 (s, 1H), 8.08 (dd, J = 8.1, 0.9 Hz, 1H), 4.53 (q, J = 7.0 Hz, 2H), 1.42 (t, J = 7.0 Hz, 3H).

5-(6-ethoxypyrazin-2-yl)picolinic acid **INTD86**

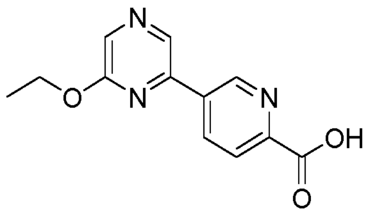

A solution of 5-(6-ethoxypyrazin-2-yl)picolinaldehyde **INTD85** (302 mg, 1.32 mmol) in DMF (5 mL) was treated with oxone (1.02 g, 1.66 mmol). The reaction mixture was stirred at RT for 4 days. The reaction mixture was diluted with water (10 mL) and filtered. The filtrate was then taken up in EtOAc (10 mL) and heated to 40 °C to afford a free flowing suspension. This was then treated dropwise with iso-hexanes (10 mL), cooled to RT and filtered to afford 5-(6-ethoxypyrazin-2-yl)picolinic acid (240 mg, 0.93 mmol, 71% yield) as a colourless solid. Rt 1.45 min (HPLC, acidic); m/z 246 $(M+H)^+$ $(ES^+)$; $^1H$ NMR (500 MHz, OMSO-$d_6$) δ 13.31 (s, 1H), 9.46 - 9.38 (m, 1H), 8.98 (s, 1H), 8.64 (dd, J = 8.1, 2.3 Hz, 1H), 8.36 (s, 1H), 8.17 (dd, J = 8.1, 0.8 Hz, 1H), 4.51 (q, J = 7.0 Hz, 2H), 1.42 (t, J = 7.0 Hz, 3H).

**Preparation of Examples**

**Amide formation**

**Method 1: Amide coupling using HATU**

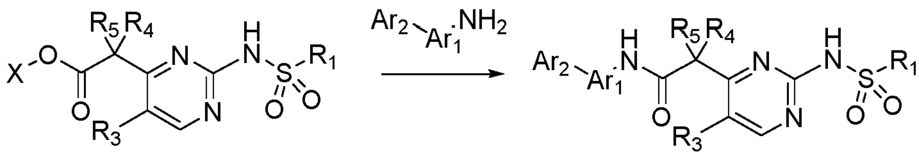

To a stirred suspension of the acid or the potassium salt (1 eq, X= H or K) and DIPEA (6 eq) in DMF (15 vol) the aniline (1 eq) and HATU (1.5 eq) were added. The reaction was stirred at RT for 18 hrs then concentrated *in vacuo.* MeOH and 2M NaOH (aq) were added. The mixture was stirred for 30 min then concentrated *in vacuo.* The aqueous phase acidified to pH 6 with 1M HCl (aq) and the product extracted into DCM. The organics were combined, dried (phase separator) and concentrated *in vacuo.*

The crude product was purified by reverse or normal phase chromatography or a combination of both.

N-(4-(5-Chloropyridin-3-yl)phenyl)-2-(2-(cyclopropanesulfonamido)pyrimidin-4-yl)butanamide **P1**

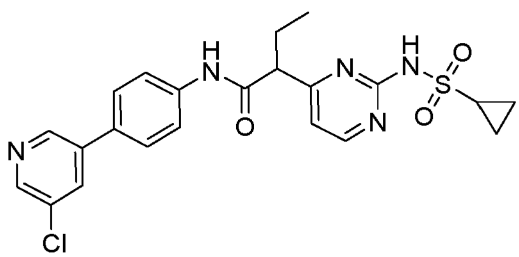

4-(5-chloropyridin-3-yl)aniline **INTD8** (0.117 g, 0.573 mmol) and HATU (0.327 g, 0.859 mmol) were added to a stirred suspension of potassium 2-(2-(cyclopropanesulfonamido)pyrimidin-4-yl)butanoate **INTC37** (0.265 g, 0.573 mmol) and DIPEA (0.60 mL, 3.44 mmol) in DMF (6 mL).

The reaction was stirred at RT 18 hrs then concentrated *in vacuo.* The crude material was dissolved in MeOH (20 mL) and 2M NaOH (aq) (20 mL) was added. The mixture was stirred for 30 min then concentrated *in vacuo.* The aqueous phase acidified to pH 6 with 1M HCl (aq) (40 mL) and the product extracted into DCM (3 × 20 mL). The organics were combined, dried (phase separator) and concentrated *in vacuo.* The crude product was purified by chromatography on silica gel (12 g column, 0-100% EtOAc/iso-hexane) followed by chromatography on RP Flash C18 (5-75% MeCN/Water 0.1% formic acid) to afford N-(4-(5-chloropyridin-3-yl)phenyl)-2-(2 (cyclopropanesulfonamido)pyrimidin-4-yl)butanamide (0.158 g, 0.318 mmol, 56% yield) as a white solid. Rt 1.36 min; m/z 472 $(M+H)^+$ $(ES^+)$; $^1H$ NMR (500 MHz, DMSO-d6) δ 11.28 (s, 1H), 10.39 (s, 1H), 8.86 (d, J = 2.0 Hz, 1H), 8.63 - 8.48 (m, 2H), 8.22 (t, J = 2.2 Hz, 1H), 7.81 - 7.71 (m, 4H), 7.19 (d, J = 5.2 Hz, 1H), 3.80 - 3.71 (m, 1H), 3.31 - 3.24 (m, 1H), 2.14 - 2.01 (m, 1H), 2.00 - 1.88 (m, 1H), 1.16 - 1.04 (m, 2H), 1.03 - 0.84 (m, 5H).

**Method 2: $AlMe_3$ mediated amide coupling from ester**

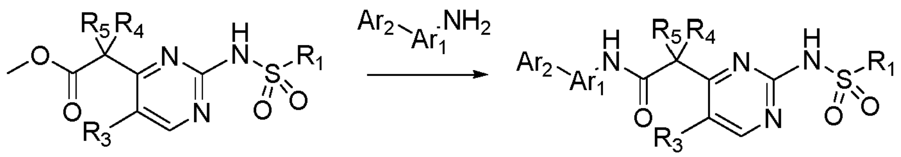

To an ice cooled solution of aniline (2 eq) in toluene (40 volumes) was added $AlMe_3$ (2.0 M in heptane, 2 eq). The mixture was stirred at this temperature for 5 mins then at RT for 10 mins. To this solution was added ester (1 eq) in one portion and the resultant mixture heated and stirred at 80 °C for 2 hrs. The reaction mixture was cooled in an ice bath and carefully quenched with MeOH (10 volumes). After stirring for 20 mins the mixture was diluted in a mixture of DCM/MeOH (10 volumes), filtered through celite and the filtrate concentrated. The crude product was purified by reverse or normal phase chromatography.

1-(2-(Cyclopropanesulfonamido)pyrimidin-4-yl)-*N*-(4-(6-ethoxypyrazin-2-yl)phenyl)cyclopentanecarboxamide **P2**

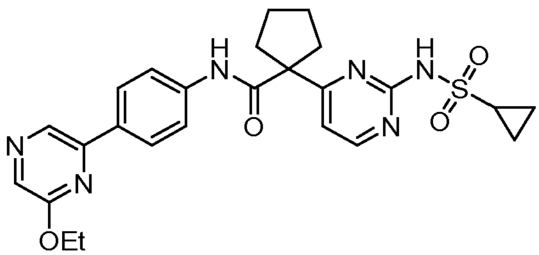

To an ice cooled solution of 4-(6-ethoxypyrazin-2-yl)aniline **INTD18** (0.099 g, 0.461 mmol) in toluene (4 mL) was added $AlMe_3$ (2.0 M in toluene) (0.307 mL, 0.615 mmol). The mixture was stirred at this temperature for 5 mins then at RT for 20 mins. To this solution was added methyl 1-(2-(cyclopropanesulfonamido)pyrimidin-4-yl)cyclopentanecarboxylate **INTC29** (0.1 g, 0.307 mmol) in one portion and the resultant mixture heated and stirred at 100°C for 3 h under $N_2$. The reaction mixture was carefully quenched with MeOH (2 mL). After stirring for 20 mins the mixture was diluted in MeOH (50 mL), filtered through celite (5 g) and the filtrate was concentrated in *vacuo.* The crude product was purified by chromatography on RP Flash C18 (25-75% MeCN/Water 0.1% formic acid) to afford 1-(2-(cyclopropanesulfonamido)pyrimidin-4-yl)-N-(4-(6-ethoxypyrazin-2-yl)phenyl)cyclopentanecarboxamide (0.053 g, 0.099 mmol, 32% yield) as a white solid. Rt 1.59 min (UPLC, acidic); m/z 509 $(M+H)^+$ $(ES^+)$; $^1H$ NMR (500 MHz, DMSO-d6) δ 11.33 (s, 1H), 9.58 (s, 1H), 8.76 (s, 1H), 8.62 - 8.46 (m, 1H), 8.18 (s, 1H), 8.11 - 8.00 (m, 2H), 7.83 - 7.70 (m, 2H), 7.17 - 6.96 (m, 1H), 4.56 - 4.37 (m, 2H), 3.28 - 3.16 (m, 1H), 2.51 - 2.40 (m, 2H), 2.25 - 2.09 (m, 2H), 1.82 - 1.60 (m, 4H), 1.46 - 1.34 (m, 3H), 1.12 - 0.99 (m, 2H), 0.95 - 0.80 (m, 2H).

2-(2-(cyclopropanesulfonamido)pyrimidin-4-yl)-*N*-(4-(6-methoxypyrazin-2-yl)phenyl)-2-methylpropanamide **P3**

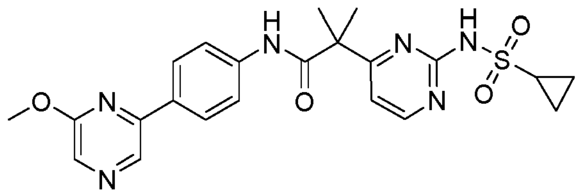

4-(6-Methoxypyrazin-2-yl)aniline **INTD1** (101 mg, 0.501 mmol) was added to an ice cooled solution of $AlMe_3$ (2M in heptane) (0.33 mL, 0.668 mmol) in toluene (4 mL). The mixture was stirred at this temperature for 5 mins then at RT for 10 mins. Methyl 2-(2-(cyclopropanesulfonamido)pyrimidin-4-yl)-2-methylpropanoate **INTC21** (100 mg, 0.334 mmol) was added in one portion and the resultant mixture heated at 100°C for 2 hrs. The reaction mixture was cooled in an ice bath and carefully quenched with MeOH (10 mL). After stirring for 20 mins the mixture was diluted with a mixture of DCM/MeOH (10 mL, 1:1), filtered through celite and the solvent removed to give an orange oil. The crude product was purified by chromatography on silica gel (24 g column, 0-100% EtOAc/iso-hexane) to afford 2-(2-(cyclopropanesulfonamido)pyrimidin-4-yl)-N-(4-(6-methoxypyrazin-2-yl)phenyl)-2-methylpropanamide (37 mg, 0.077 mmol, 23% yield) as a pale beige solid. Rt 2.03 min (HPLC acidic); m/z 469 $(M+H)^+$ $(ES^+)$; $^1H$ NMR (400 MHz, DMSO-d6) $\delta$ 11.27 (s, 1H), 9.51 (s, 1H), 8.78 (s, 1H), 8.61 (d, J = 5.3 Hz, 1H), 8.21 (s, 1H), 8.14 - 8.04 (m, 2H), 7.84 - 7.74 (m, 2H), 7.20 (d, J = 5.3 Hz, 1H), 4.02 (s, 3H), 3.25 - 3.18 (m, 1H), 1.60 (s, 6H), 1.08 - 0.99 (m, 2H), 0.85 - 0.74 (m, 2H).

2-(2-(Cyclopropanesulfonamido)pyrimidin-4-yl)-2-methyl-*N*-(4-(5-(trifluoromethyl)pyridin-3-yl)phenyl)propanamide **P4**

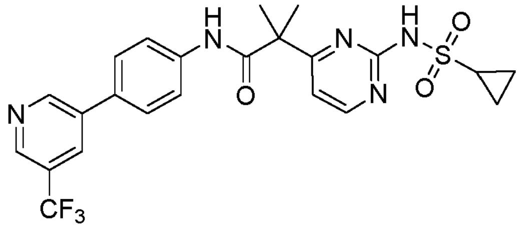

To an ice cooled solution of 4-(5-(trifluoromethyl)pyridin-3-yl)aniline **INTD7** (0.119 g, 0.501 mmol) in toluene (4 mL) and THF (2 mL) was added $AlMe_3$ (2.0 M in heptane) (0.334 mL, 0.668 mmol). The mixture was stirred at this temperature for 5 mins then at RT for 10 min. To this solution was added methyl 2-(2-(cyclopropanesulfonamido)pyrimidin-4-yl)-2-methylpropanoate **INTC21** (0.1 g, 0.334 mmol) in one portion and the resultant mixture stirred and heated at 80°C for 2 hrs in a sealed vessel. The reaction mixture was cooled in an ice bath and carefully quenched with MeOH. After stirring for 20 min the mixture was diluted in a mixture of DCM/MeOH, filtered through celite and the filtrate concentrated in *vacuo.* The crude product was purified by chromatography on RP Flash C18 (5-75% MeCN/Water 0.1% formic acid) to afford 2-(2-(cyclopropanesulfonamido)pyrimidin-4-yl)-2-methyl-N-(4-(5-(trifluoromethyl)-pyridin-3-yl)phenyl)propanamide (0.109 g, 0.205 mmol, 61% yield) as a white solid. Rt 2.17 (HPLC acidic); m/z 506 $(M+H)^+$ $(ES^+)$; $^1H$ NMR (400 MHz, DMSO-d6) $\delta$ 11.28 (s, 1H), 9.49 (s, 1H), 9.28 - 9.11 (m, 1H), 8.98 - 8.84 (m, 1H), 8.68 - 8.54 (m, 1H), 8.50 - 8.37 (m, 1H), 7.95 - 7.71 (m, 4H), 7.28 - 7.12 (m, 1H), 3.27 - 3.13 (m, 1H), 1.60 (s, 6H), 1.13 - 0.95 (m, 2H), 0.91 - 0.69 (m, 2H).

2-Methyl-N-(2-methyl-4-(6-methylpyrazin-2-yl)phenyl)-2-(2-(methylsulfonamido)pyrimidin-4-yl)propanamide **P5**

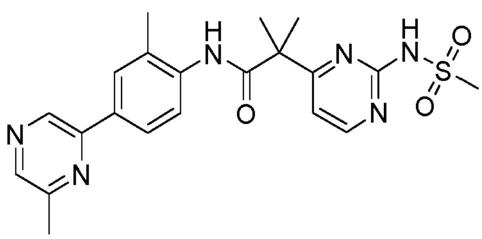

To an ice cooled solution of 4-(6-chloropyrazin-2-yl)-2-methylaniline **INTD26** (0.549 mmol, 121 mg) in toluene (2 mL) was added $AlMe_3$ (0.55 mL, 1.098 mmol, 2.0 M in heptane). The mixture was stirred at this temperature for 5 min then at RT for 10 min. To this solution was added methyl 2-methyl-2-(2-(methylsulfonamido)pyrimidin-4-yl)propanoate **INTC19** (100 mg, 0.366 mmol) in one portion and the resultant mixture stirred and heated at 90 °C for 2 hrs. The reactions were cooled to 0 °C, 1M HCl (5 mL) was added and the residues were extracted with EtOAc (2 × 20 mL). The combined organic extract was passed through a phase separator and the solvent was removed under reduced pressure. The crude product was purified by chromatography on RP Flash C18 (0-100% MeCN/Water 0.1% formic acid) to afford 2-methyl-N-(2-methyl-4-(6-methylpyrazin-2-yl)phenyl)-2-(2-(methylsulfonamido)pyrimidin-4-yl)propanamide (78.9 mg, 0.170 mmol, 47% yield) as an off-white solid. Rt 1.74 (HPLC, acidic); m/z 441 $(M+H)^+$ $(ES^+)$; $^1H$ NMR (500 MHz, DMSO-d6) δ 11.35 (s, 1H), 9.07 - 8.99 (m, 2H), 8.62 (d, J = 5.3 Hz, 1H), 8.48 (s, 1H), 7.99 (d, J = 2.1 Hz, 1H), 7.93 (dd, J = 8.3, 2.2 Hz, 1H), 7.42 (d, J = 8.3 Hz, 1H), 7.23 (d, J = 5.3 Hz, 1H), 3.39 (s, 3H), 2.56 (s, 3H), 2.19 (s, 3H), 1.62 (s, 6H).

4-(2-(Cyclopropanesulfonamido)pyrimidin-4-yl)-*N*-(5-(6-ethoxypyrazin-2-yl)pyridin-2-yl)tetrahydro-2*H*-pyran-4-carboxamide **P115**

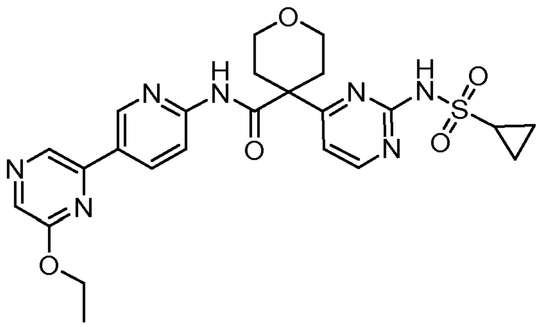

To a solution of 5-(6-ethoxypyrazin-2-yl)pyridin-2-amine **INTD33** (0.14 g, 0.66 mmol) in toulene (3.0 mL, 28.2 mmol) at 0 °C was added $AlMe_3$ (0.66 mL, 1.32 mmol, 2.0 M in heptane). The reaction mixture was stirred for 5 mins at 0 °C then 10 mins at RT. Methyl 4-(2-(cyclopropanesulfonamido)pyrimidin-4-yl)tetrahydro-2/7-pyran-4-carboxylate **INTC53** (0.15 g, 0.44 mmol) was added in one portion and the reaction mixture was heated to 95 °C for 1 h, then cooled to 0 °C. The reaction mixture was quenched with 1 M HCl (5 mL) and diluted with EtOAc (10 mL). The phases were separated and the aqueous was extracted using further EtOAc (2 × 10 mL). The combined organics were dried over $MgSO_4$, filtered and concentrated *in vacuo.* The crude product was purified by chromatography on silica gel (12 g column, 0-100% EtOAc/*iso*hexane) to afford 4-(2-(cyclopropanesulfonamido)pyrimidin-4-yl)-N-(5-(6-ethoxypyrazin-2-yl)pyridin-2-yl)tetrahydro-2*H*-pyran-4-carboxamide (0.022 g, 0.040 mmol, 9% yield) as a white solid. Rt 1.31 min (UPLC, acidic); m/z 526 $(M+H)^+$ $(ES^+)$; $^1H$ NMR (500 MHz, DMSO-d6) δ 11.31 (s, 1H), 10.13 (s, 1H), 9.03 (d, J = 2.5 Hz, 1H), 8.84 (s, 1H), 8.63 (d, J = 5.3 Hz, 1H), 8.50 (dd, J = 8.8, 2.5 Hz, 1H), 8.26 (s, 1H), 8.20 (d, J = 8.8 Hz, 1H), 7.26 (d, J = 5.3 Hz, 1H), 4.48 (q, J = 7.0 Hz, 2H), 3.81 - 3.69 (m, 2H), 3.67 - 3.56 (m, 2H), 3.31 - 3.20 (m, 1H), 2.49-2.41 (m, 2H), 2.25-2.17 (m, 2H), 1.40 (t, J = 7.0 Hz, 3H), 1.09-1.03 (m, 2H), 0.95-0.84 (m , 2H).

**Method 2b: $DABALMe_3$ mediated amide coupling from ester**

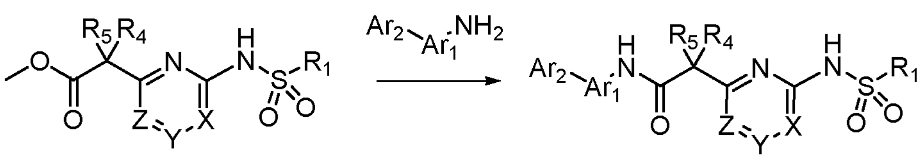

X = CH, N
Y = $CR_2$, N
Z = $CR_2$, N

To a solution of ester (1 eq) and aniline (1.5 eq) in toluene (30 volumes) was added DABAL-Mes (1.5 eq) and

the resulting mixture was heated at 100 °C for 4 h. The reaction mixture was cooled to 0 °C and quenched by careful addition of 1 M HCl (aq, 20 volumes). The aqueous phase was extracted with EtOAc (3 × 20 volumes). The combined organics were washed with 1 M HCl (aq, 2 × 10 volumes), dried over $Na_2SO_4$, filtered and concentrated *in vacuo.* The crude product was purified by reverse or normal phase chromatography.

**Method 3: Amide coupling from potassium salt using T3P**

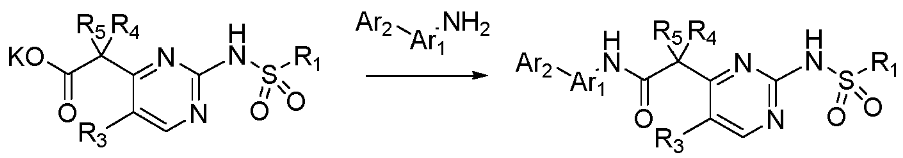

Pyridine (10 eq) followed by T3P (50 wt% in DMF, 2 eq) was added to a stirring solution of amine (1.1 eq) and potassium 2-(2-(cyclopropanesulfonamido)pyrimidin-4-yl)butanoate (1 eq) in DMF (16 volumes). The resulting reaction was stirred at RT for 24 hrs. The crude reaction mixture was concentrated *in vacuo* then diluted with $NH_4Cl$ (sat. aq) and extracted with DCM. The combined organic extracts were dried (phase separator) and the solvent removed. The crude product was purified by reverse or normal phase chromatography.

2-(2-(Cyclopropanesulfonamido)pyrimidin-4-yl)-*N*-(2-fluoro-4-(pyrazin-2-yl)phenyl)butanamide **P6**

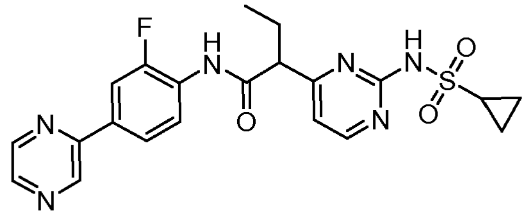

T3P (50 wt% in DMF) (1.120 mL, 1.546 mmol) was added to a stirred suspension of 2-fluoro-4-(pyrazin-2-yl)aniline **INTD23** (154 mg, 0.773 mmol), potassium 2-(2-(cyclopropanesulfonamido)pyrimidin-4-yl)butanoate **INTC37** (250 mg, 0.773 mmol) and pyridine (0.313 mL, 3.87 mmol) in DMF (1 mL). The resulting reaction was stirred at RT for 18 hrs. Water (5 mL) was added and the newly formed precipitate filtered. The product was recovered by dissolving in DCM (10 mL) and concentrated *in vacuo.* The crude product was purified by preparative HPLC (20-50% MeCN/Water 0.1% formic acid) to afford 2-(2-(cyclopropanesulfonamido)pyrimidin-4-yl)-N-(2-fluoro-4-(pyrazin-2-yl)phenyl)butanamide (32 mg, 0.069 mmol, 9% yield) as a colourless powder. Rt 1.15 min (UPLC acidic); m/z 457 $(M+H)^+$ $(ES^+)$; $^1H$ NMR (500 MHz, DMSO-d6) δ 11.26 (s, 1H), 10.25 (s, 1H), 9.29 (d, J = 1.6 Hz, 1H), 8.72 (dd, J = 2.5, 1.5 Hz, 1H), 8.62 (d, J = 2.5 Hz, 1H), 8.57 (d, J = 5.2 Hz, 1H), 8.12 - 8.03 (m, 2H), 8.03 - 7.97 (m, 1H), 7.20 (d, J = 5.2 Hz, 1H), 4.00 (dd, J = 7.5 Hz, 1H), 3.31 - 3.28 (m, 1H), 2.12 - 2.02 (m, 1H), 2.00 - 1.92 (m, 1H), 1.16 - 1.07 (m, 2H), 1.03 - 0.93 (m, 5H).

2-(2-(Cyclopropanesulfonamido)pyrimidin-4-yl)-N-(4-(5-(trifluoromethyl)pyridin-3-yl)phenyl)butanamide **P7**

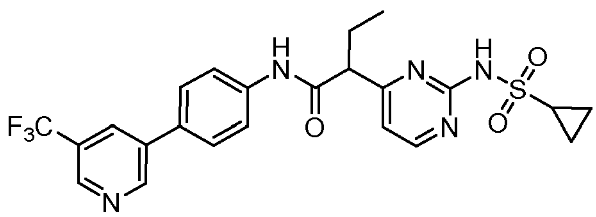

T3P (50 wt% in DMF) (0.78 mL, 1.082 mmol) was added to a stirred suspension of potassium 2-(2-(cyclopropanesulfonamido)pyrimidin-4-yl)butanoate **INTC37** (250 mg, 0.541 mmol) and 4-(5-(trifluoromethyl)pyridin-3-yl)aniline

**INTD7** (129 mg, 0.541 mmol) in pyridine (0.13 mL, 1.623 mmol) and DMF (3 mL). The resulting reaction was stirred at RT for 18 hrs. The crude reaction mixture was diluted with saturated $NH_4Cl$ (aq) (10 mL) and extracted with DCM (3 × 10 mL). The combined organic extracts were dried (phase separator) and the solvent removed under reduced pressure. The crude product was purified by chromatography on silica gel (0-10% MeOH in DCM), followed by chromatography on RP Flash C18 (15-75% MeCN/Water 0.1% formic acid) to afford 2-(2-(cyclopropanesulfonamido)pyrimidin-4-yl)-N-(4-(5-(trifluoromethyl)pyridin-3-yl)phenyl)butanamide (19 mg; 0.036 mmol; 7% yield). Rt 1.44 (UPLC, acidic); m/z 506 $(M+H)^+$ $(ES^+)$; $^1H$ NMR (500 MHz, DMSO-d6) δ 11.25 (s, 1H), 10.41 (s, 1H), 9.20 (d, J = 2.2 Hz, 1H), 8.94 - 8.92 (m, 1H), 8.57 (d, J = 5.2 Hz, 1H), 8.45 - 8.42 (m, 1H), 7.87 - 7.83 (m, 2H), 7.79 - 7.75 (m, 2H), 7.21 (d, J = 5.2 Hz, 1H), 3.77 (dd, J = 8.7, 6.3 Hz, 1H), 3.31 - 3.26 (m, 1H), 2.13 - 2.03 (m, 1H), 1.98 - 1.89 (m, 1H), 1.13 - 1.06 (m, 2H), 1.01 - 0.89 (m, 5H).

2-(2-(Cyclopropanesulfonamido)pyrimidin-4-yl)-N-(4-(6-(trifluoromethyl)pyrazin-2-yl)phenyl)acetamide **P8**

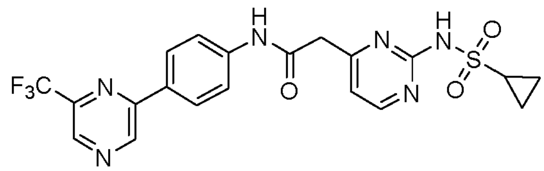

T3P (50 wt% in DMF) (0.343 mL, 0.474 mmol) was added to a stirred suspension of potassium 2-(2-(cyclopropanesulfonamido)pyrimidin-4-yl)acetate **INTC39** (100 mg, 0.237 mmol), 4-(6-(trifluoromethyl)pyrazin-2-yl)aniline **INTD19** (56.7 mg, 0.237 mmol) and pyridine (0.096 mL, 1.185 mmol) in DMF (1 mL). The resulting reaction was stirred at RT for 18 hrs. Water (5 mL) was added and the newly formed precipitate was filtered to afford the crude product. The crude product was purified by chromatography on silica gel (0-10% MeOH in DCM) followed by preparative HPLC (5-95% MeCN/Water 0.1% formic acid) to afford 2-(2-(cyclopropanesulfonamido)pyrimidin-4-yl)-N-(4-(6-(trifluoromethyl)pyrazin-2-yl)phenyl)acetamide (10 mg, 0.021 mmol, 9% yield) as a yellow powder. Rt 1.31 min (UPLC, acidic); m/z 479 $(M+H)^+$ $(ES^+)$; $^1H$ NMR (500 MHz, DMSO-d6) observed as mixture of tautomers δ 12.81 (s, 1H, minor), 11.24 (s, 1H, major), 10.95 (s, 1H, minor), 10.58 (s, 1H, major), 10.09 (s, 1H, minor), 9.58 (s, 1H, major), 9.57 (s, 1H, minor), 9.09 (s, 1H, major), 9.06 (s, 1H, minor), 8.57 (d, J = 5.1 Hz, 1H, major), 8.24 - 8.13 (m, 2 × 2H, major and minor), 7.85 - 7.79 (m, 2 × 2H, major and minor), 7.18 (d, J = 5.0 Hz, 1H, major), 6.95 (d, J = 7.5 Hz, 1H, minor), 5.89 (d, J = 7.5 Hz, 1H, minor), 5.06 (s, 1H, minor), 3.89 (s, 2H, major), 3.28 - 3.22 (m, 1H, major), 2.73 - 2.65 (m, 1H, minor), 1.13 - 0.90 (m, 2 × 4H, major and minor).

**Method 4: Amide coupling from lithium salt using T3P**

*N*-(5-(6-Ethoxypyrazin-2-yl)pyridin-2-yl)-2-fluoro-2-(2-(*N*-(4-methoxybenzyl)cyclopropanesulfonamido)pyrimidin-4-yl)butanamide **INTC51**

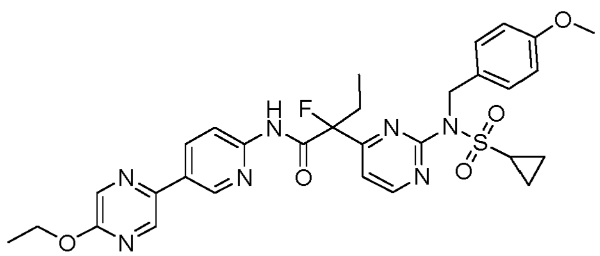

To a solution of lithium 2-fluoro-2-(2-(*N*-(4-methoxybenzyl)cyclopropane-sulfonamido)pyrimidin-4-yl)butanoate **INTC50** (0.50 g, 1.17 mmol) in DMF (5 mL) at 0 °C was added 5-(6-ethoxypyrazin-2-yl)pyridin-2-amine **INTD33** (0.30 g, 1.40 mmol) followed by pyridine (0.57 mL, 7.01 mmol) and T3P (50 wt% in DMF) (1.69 mL, 2.34 mmol). The reaction mixture was stirred at 0 °C for 2 hrs then warmed to RT for 20 hrs. The reaction mixture was cooled to 0 °C and further T3P (50 wt% in DMF) (0.5 mL, 0.69 mmol) was added. The reaction mixture was stirred at 0 °C for 1 hr, then RT for 3 hrs. The reaction mixture was diluted with sat. $NH_4Cl$ (aq, 45 mL) and the resultant precipitate was isolated by filtration,

washing with water (2 × 20 mL). The resultant yellow precipitate was dissolved in DCM (30 mL) and MeOH (30 mL) and concentrated onto silica. The crude product was purified by chromatography on silica gel (24 g column, 0-60% EtOAc/isohexane) to afford *N*-(5-(6-ethoxypyrazin-2-yl)pyridin-2-yl)-2-fluoro-2-(2-(*N*-(4-methoxybenzyl) cyclopropanesulfonamido)pyrimidin-4-yl)butanamide (0.274 g, 0.433 mmol, 37% yield) as a colourless oil. Rt 1.84 min (UPLC, acidic); m/z 622 $(M+H)^+$ $(ES^+)$; $^1H$ NMR (500 MHz, DMSO-d6) δ 10.69 (s, 1H), 9.10 (d, J = 2.5 Hz, 1H), 8.88 - 8.81 (m, 2H), 8.52 (dd, J = 8.7, 2.5 Hz, 1H), 8.27 (s, 1H), 8.10 (d, J = 8.7 Hz, 1H), 7.52 (dd, J = 5.2, 1.3 Hz, 1H), 7.30 - 7.23 (m, 2H), 6.81 - 6.74 (m, 2H), 5.20 - 5.08 (m, 2H), 4.48 (q, J = 7.0 Hz, 2H), 3.76 - 3.70 (m, 1H), 3.65 (s, 3H), 2.50 - 2.39 (m, 1H), 2.38 - 2.24 (m, 1H), 1.40 (t, J = 7.0 Hz, 3H), 1.14 - 1.06 (m, 1H), 1.10 - 0.97 (m, 2H), 0.96 - 0.92 (m, 1H), 0.89 (t, J = 7.3 Hz, 3H).

**Method 5: NH-Amide formation *via* amide deprotection and/or decarboxylation**

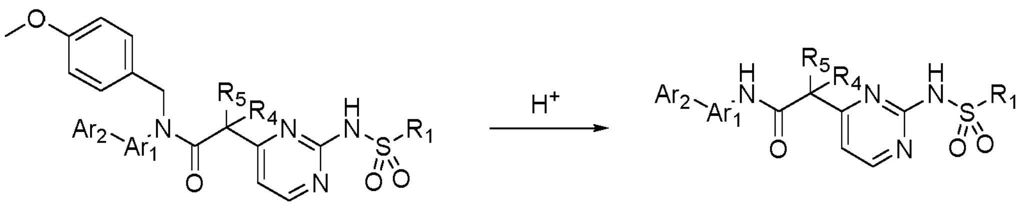


$R_4$ = Alkyl, $R_5$ = Alkyl or H
or
$R_4$ = *t*Bu-ester, $R_5$ = H

$R_4$ = Alkyl, $R_5$ = Alkyl or H
or
$R_4$ = $R_5$ = H

To a solution of the protected amide in DCM a mixture of TFA (88 eq) and triflic acid (1-6 eq) was added and the mixture left stirring at RT for 18-36 hrs and then concentrated *in vacuo.* The crude product was purified by column chromatography on silica gel or by RP chromatography.

2-(2-(Cyclopropanesulfonamido)pyrimidin-4-yl)-*N*-(4-(6-ethoxypyrazin-2-yl)phenyl)butanamide **P105**

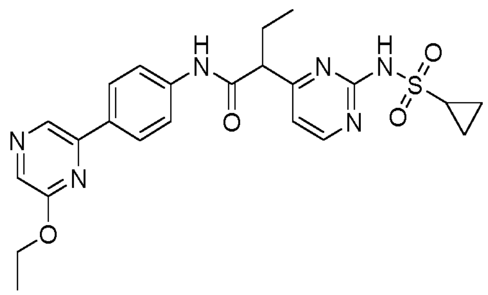

A solution of 2-(2-(cyclopropanesulfonamido)pyrimidin-4-yl)-*N*-(4-(6-ethoxypyrazin-2-yl)phenyl)-*N*-(4-methoxybenzyl)butanamide **INTC46** (0.18 g, 0.299 mmol) in a mixture of TFA (2 mL, 26.0 mmol) and DCM (2 mL) was stirred at 25°C for 18 hrs. The reaction was heated at 50°C for 2 hrs. To the reaction was added triflic acid (0.027 mL, 0.299 mmol) and the mixture stirred at 25°C for 2 hrs. The reaction mixture was concentrated and then diluted in 1 N HCl (aq) (20 mL). The aqueous phase was extracted with DCM (3 × 20 mL), dried (phase separator) and the solvent was removed under reduced pressure. The crude product was purified by chromatography on RP Flash C18 (24 g column, 5-75% MeCN/Water 0.1% formic acid) to afford 2-(2-(cyclopropanesulfonamido)pyrimidin-4-yl)-*N*-(4-(6-ethoxypyrazin-2-yl)phenyl)butanamide (0.02 g, 0.041 mmol, 14% yield) as a white solid. Rt 2.23 min (HPLC acidic); 483 $(M+H)^+$ $(ES^+)$.

2-(2-(Cyclopropanesulfonamido)pyrimidin-4-yl)-*N*-(4-(6-ethoxypyrazin-2-yl)phenyl)acetamide **P18**

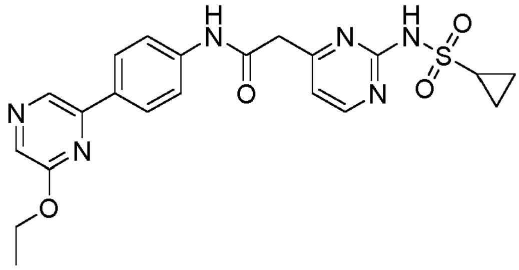

To a solution of *tert*-butyl 2-(2-(cyclopropanesulfonamido)pyrimidin-4-yl)-3-((4-(6-ethoxypyrazin-2-yl)phenyl)(4-methoxybenzyl)amino)-3-oxopropanoate **INTC47** (0.1 g, 0.148 mmol) in a mixture of TFA (1 mL, 12.98 mmol) and DCM (20 mL) was added triflic acid (0.039 mL, 0.445 mmol). The mixture was stirred at 25°C for 18 hrs. Further triflic acid (0.039 mL, 0.445 mmol) was added and the mixture stirred at 25°C for a further 18 hrs. The reaction mixture was concentrated under reduced pressure. The crude product was purified by chromatography on silica gel (12 g column, 0-10% MeOH/DCM,) to afford 2-(2-(cyclopropanesulfonamido)pyrimidin-4-yl)-*N*-(4-(6-ethoxypyrazin-2-yl)phenyl)acetamide (0.03 g, 0.063 mmol, 42% yield) as a pale yellow solid. Rt 1.98 min (HPLC, acidic); m/z 455 $(M+H)^+$ $(ES^+)$.

**Method 6: Deprotection of Sulfonamide**

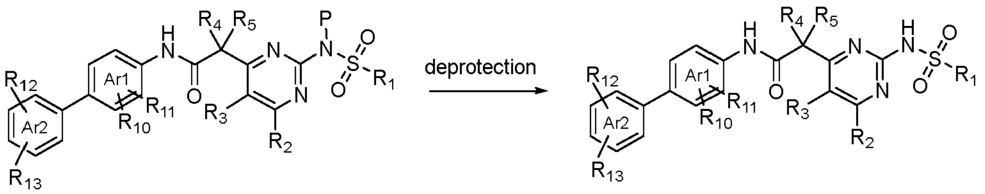


2-(2-(Cyclopropanesulfonamido)pyrimidin-4-yl)-*N*-(5-(6-ethoxypyrazin-2-yl)pyridin-2-yl)-2-fluorobutanamide **P112**

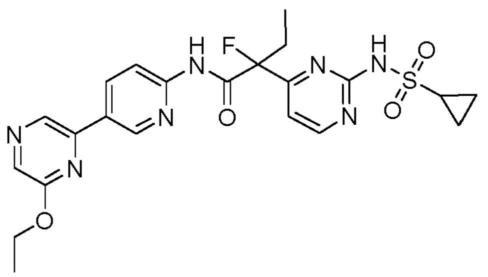

TFA (0.28 mL, 3.70 mmol) was added into a stirring solution of *N*-(5-(6-ethoxypyrazin-2-yl)pyridin-2-yl)-2-fluoro-2-(2-(N-(4-methoxybenzyl)cyclopropanesulfonamido)pyrimidin-4-yl)butanamide **INTC51** (115mg, 0.185 mmol) in DCM (10 mL) and the resulting reaction mixture was stirred at RT for 4 hrs. The reaction mixture was concentrated *in vacuo* and the crude product was purified by chromatography on silica gel (12 g column, 0-100% EtOAc/iso-hexane) to afford 2-(2-(cyclopropanesulfonamido)pyrimidin-4-yl)-*N*-(5-(6-ethoxypyrazin-2-yl)pyridin-2-yl)-2-fluorobutanamide (77 mg, 0.15 mmol, 81% yield) as a white solid. Rt 2.28 min (HPLC, acidic); m/z 502 $(M+H)^+$ (ES+); $^1H$ NMR (500 MHz, DMSO-d6) δ 11.50 (s, 1H), 10.60 (d, J = 2.3 Hz, 1H), 9.10 (d, J = 2.5 Hz, 1H), 8.87 (s, 1H), 8.76 (d, J = 5.1 Hz, 1H), 8.53 (dd, J = 8.8, 2.5 Hz, 1H), 8.27 (s, 1H), 8.10 (d, J = 8.8 Hz, 1H), 7.48 (d, J = 5.1 Hz, 1H), 4.49 (q, J = 7.0 Hz, 2H), 3.38-3.27

(m, 1H), 2.44 - 2.29 (m, 2H), 1.40 (t, J = 7.0 Hz, 3H), 1.20 - 0.92 (m, 7H).

The racemate **P112** was separated by chiral preparative HPLC using a Diacel Chiralpak IC column (20% EtOH in [4:1 heptane:chloroform (0.2% TFA):]) to afford:

**P112 Enantiomer 1 Stereochemistry of product was not assigned (P113)**

2-(2-(cyclopropanesulfonamido)pyrimidin-4-yl)-*N*-(5-(6-ethoxypyrazin-2-yl)pyridin-2-yl)-2-fluorobutanamide; Rt 2.28 mins (HPLC acidic); m/z 502 $(M+H)^+$ (ES+); $^1H$ NMR (500 MHz, DMSO-d6) $\delta$ 11.50 (s, 1H), 10.60 (d, J = 2.2 Hz, 1H), 9.11 (d, J = 2.5 Hz, 1H), 8.87 (s, 1H), 8.76 (d, J = 5.1 Hz, 1H), 8.53 (dd, J = 8.8, 2.5 Hz, 1H), 8.27 (s, 1H), 8.10 (d, J = 8.8 Hz, 1H), 7.48 (d, J = 5.1 Hz, 1H), 4.49 (q, J = 7.0 Hz, 2H), 3.39 - 3.26 (m, 1H), 2.54 - 2.43 (m, 1H), 2.41 - 2.28 (m, 1H), 1.40 (t, J = 7.0 Hz, 3H), 1.22 - 0.89 (m, 7H).

The product was analysed by Chiral IC3 method HPLC; Rt = 10.47 mins, 100% ee at 254 nm.

**P112 Enantiomer 2 Stereochemistry of product was not assigned (P114)**

2-(2-(cyclopropanesulfonamido)pyrimidin-4-yl)-*N*-(5-(6-ethoxypyrazin-2-yl)pyridin-2-yl)-2-fluorobutanamide; Rt 2.28 min (HPLC acidic); m/z 502 $(M+H)^+$ (ES+); $^1H$ NMR (500 MHz, DMSO-d6) $\delta$ 11.50 (s, 1H), 10.60 (d, J = 2.3 Hz, 1H), 9.11 (d, J = 2.5 Hz, 1H), 8.87 (s, 1H), 8.76 (d, J = 5.1 Hz, 1H), 8.53 (dd, J = 8.7, 2.5 Hz, 1H), 8.27 (s, 1H), 8.10 (d, J = 8.7 Hz, 1H), 7.48 (d, J = 5.1 Hz, 1H), 4.49 (q, J = 7.0 Hz, 2H), 3.39 - 3.25 (m, 1H), 2.55 - 2.42 (m, 1H), 2.42 - 2.27 (m, 1H), 1.40 (t, J = 7.0 Hz, 3H), 1.25 - 0.88 (m, 7H).

The product was analysed by Chiral IC3 method HPLC Rt = 14.24 mins, 100% ee at 254 nm.

**Method 7: Sulfonylation from aromatic chloride**

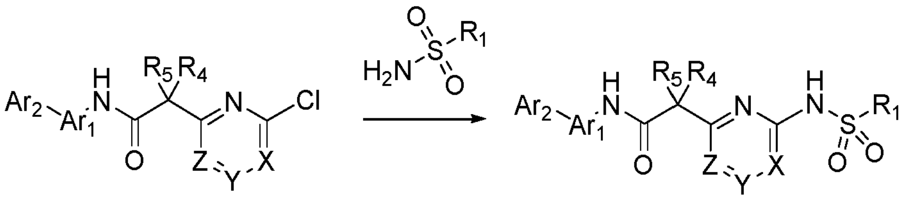

X = CH, N
Y = $CR_2$, N
Z = $CR_2$, N

2-Chloro-heteroaromatic intermediate (1 eq), sulfonamide (1.2 eq) and base (2 eq) were dissolved in dioxane (40 volumes). The mixture was degassed (evacuated and backfilled with $N_2$ x 3) then catalyst (10 mol%) was added. The resulting mixture was heated under nitrogen at 90 °C for 2 hrs. The mixture was cooled to RT, diluted with sat. $NH_4Cl$ (aq, 80 volumes) and DCM (80 volumes). The phases were separated and the aqueous was extracted with further DCM (2 × 80 volumes). The combined organics were dried ($MgSO_4$), filtered and concentrated *in vacuo*. The crude product was purified by normal phase chromatography or trituration using a suitable solvent.

**Method 8: Amide coupling using 1-chloro-*N,N,2*-trimethylprop-1-en-1-amine**

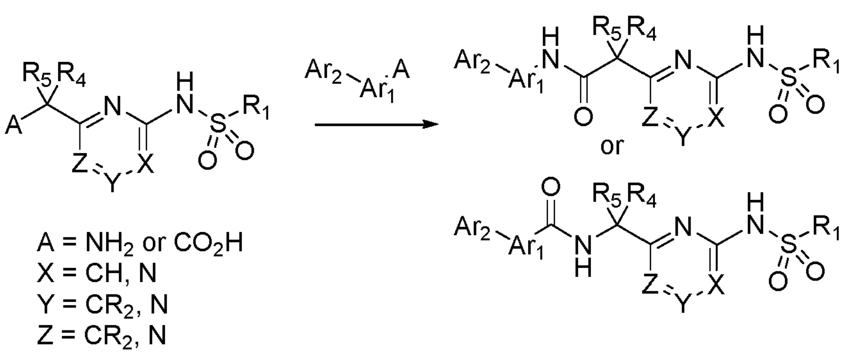

or

A = $NH_2$ or $CO_2H$
X = CH, N
Y = $CR_2$, N
Z = $CR_2$, N

1-Chloro-*N,N*,2-trimethylprop-1-en-1-amine (2 eq) was added to a solution of carboxylic acid (1 eq) in DCM

(20 volumes). The reaction mixture was stirred at RT for 2 hrs. The reaction mixture was concentrated *in vacuo* and the residue redissolved in DCM (20 volumes) before addition of pyridine (2 mL) followed by addition of the appropriate amine (1.1 eq). The reaction mixture was stirred at RT for 2 hrs. An aqueous work up was performed and the crude product was purified by normal phase chromatography, reverse phase chromatography or trituration from an appropriate solvent.

**Method 9: Suzuki ArBr**

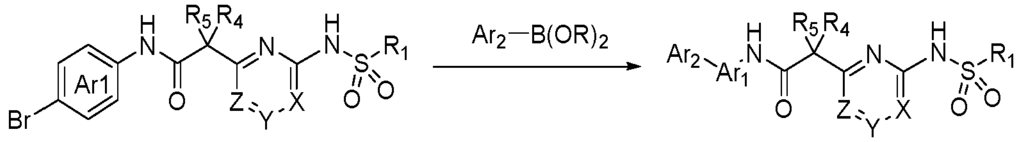


X = CH, N
Y = $CR_2$, N
Z = $CR_2$, N

To a suspension of Ar1-Br (1 eq) in dioxane (10 volumes) was added arylboronic acid or ester (1 eq) and a solution of $K_2CO_3$ (2 eq) in water (5 volumes). The resulting suspension was degassed ($N_2$, 5 mins). $PdCl_2$(dppf)-$CH_2Cl_2$ adduct or other appropriate catalyst (10 mol%) was added and the reaction mixture was stirred at 80 °C for 2 hrs. The reaction mixture was then cooled to RT. An aqueous work up was performed and the crude product was purified by normal phase chromatography, reverse phase chromatography or trituration from an appropriate solvent.

**Method 10: T3P with free acid**

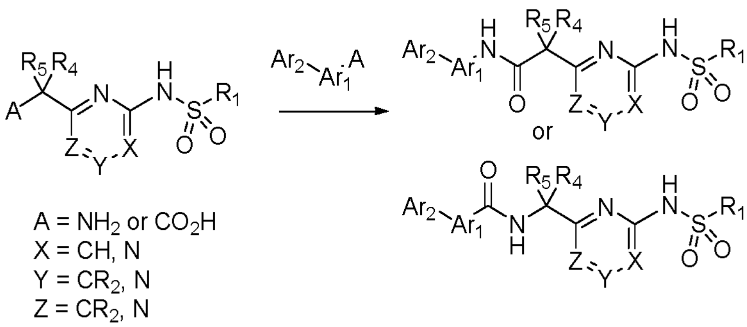


or

Pyridine (10 eq) followed by T3P (50 wt% in DMF, 2 eq) was added to a stirring solution of amine (1.1 eq) and carboxylic acid (1 eq) in DMF (16 volumes). The resulting reaction was stirred at RT for 24 hrs. The crude reaction mixture was concentrated *in vacuo* then diluted with $NH_4Cl$ (sat. aq) and extracted with DCM. The combined organic extracts were dried (phase separator) and the solvent removed. The crude product was purified by reverse or normal phase chromatography.

**Table 17**: Preparation methods and characterisation data of examples **P9-P115, P117-P225**

| P | Name/Structure (All examples containing chiral centres are racemates unless stated) | Synthesis Method, [LCMS Method], m/z $(M+H)^+$, (RT/Min) | $^1H$ NMR Chemical Shift Data (DMSO-d6 unless stated) |
|---|---|---|---|
| **P9** | 2-(2-(cyclopropanesulfonamido)pyrimidin-4-yl)-N-(5-(6-isopropoxypyrazin-2-yl)pyridin-2-yl)-2-methylpropanamide 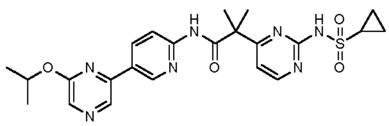 | Method 2: using INTC21 and INTD32 [HPLC acidic], 498, (2.28) | 11.23 (s, 1H), 10.14 (s, 1H), 9.03 - 8.97 (m, 1H), 8.82 (s, 1H), 8.60 (d, J = 5.3 Hz, 1H), 8.53 - 8.42 (m, 1H), 8.25 - 8.16 (m, 2H), 7.21 (d, J = 5.3 Hz, 1H), 5.46 - 5.34 (m, 1H), 3.23 - 3.10 (m, 1H), 1.61 (s, 6H), 1.40 - 1.37 (m, 6H), 1.04 - 0.98 (m, 2H), 0.81 - 0.74 (m, 2H). |
| **P10** | 2-(2-(cyclopropanesulfonamido)pyrimidin-4-yl)-N-(4-(6-ethoxypyrazin-2-yl)phenyl)-2-ethylbutanamide 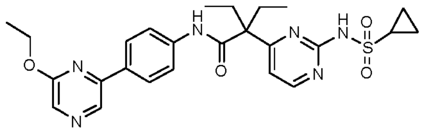 | Method 2 using INTC27 and INTD18, [UPLC acidic], 511, (1.57) | 11.27 (s, 1H), 9.47 (s, 1H), 8.75 (s, 1H), 8.59 (d, J = 5.3 Hz, 1H), 8.18 (s, 1H), 8.09 - 8.01 (m, 2H), 7.79 - 7.73 (m, 2H), 7.14 (d, J = 5.3 Hz, 1H), 4.47 (q, J = 7.1 Hz, 2H), 3.23 - 3.11 (m, 1H), 2.12 (q, J = 7.6 Hz, 4H), 1.40 (t, J = 7.1 Hz, 3H), 1.05 - 0.96 (m, 2H), 0.80 - 0.66 (m, 8H). |
| **P11** | 2-(2-(cyclopropanesulfonamido)pyrimidin-4-yl)-N-(2-fluoro-4-(6-(trifluoromethyl)pyrazin-2-yl) phenyl)acetamide 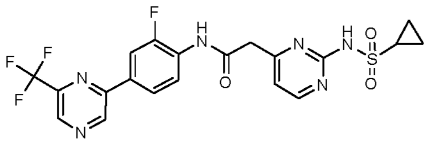 | Method 2 using INTC33 and INTD25, [HPLC Acidic], 497, (2.09) | 12.77 (s, 1H, minor), 11.24 (s, 1H, major), 10.98 (s, 1H, minor), 10.37 (s, 1H, major), 9.81 (s, 1H, minor), 9.64 (s, 1H, major), 9.63 (s, 1H, minor), 9.14 (s, 1H, major), 9.12 (s, 1H, minor), 8.57 (d, J = 5.1 Hz, 1H, major), 8.34 (t, J = 8.3 Hz, 1H, minor), 8.24 (t, J = 8.3 Hz, 1H, major), 8.17 - 8.04 (m, 2 x 2H, major and minor), 7.18 (d, J = 5.1 Hz, 1H, major), 6.96 (d, J = 7.6 Hz, 1H, minor), 5.85 (dd, J = 7.7, 1.6 Hz, 1H, minor), 5.34 (s, 1H, minor), 4.00 (s, 2H, major), 3.30 - 3.23 (m, 1H, major), 2.71 - 2.61 (m, 1H, minor), 1.15 - 0.88 (m, 2x4H, major and minor). |

(continued)

| P | Name/Structure (All examples containing chiral centres are racemates unless stated) | Synthesis Method, [LCMS Method], m/z $(M+H)^+$, (RT/Min) | $^1H$ NMR Chemical Shift Data (DMSO-d6 unless stated) |
|---|---|---|---|
| **P12** | 2-(2-(cyclopropanesulfonamido)pyrimidin-4-yl)-N-(2-fluoro-4-(6-isopropoxypyrazin-2-yl) phenyl)acetamide 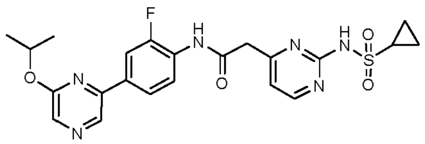 | Method 2 using INTC33 and INTD35, [HPLC Acidic], 487, (2.17) | 12.79 (s, 1H, minor), 11.23 (s, 1H, major), 10.95 (s, 1H, minor), 10.29 (s, 1H, major), 9.74 (s, 1H, minor), 8.81 (s, 1H, major), 8.80 (s, 1H, minor), 8.57 (d, J = 5.0 Hz, 1H, major), 8.23 (t, J = 8.5 Hz, 1H, minor), 8.19 (s, 1H, major), 8.17 (s, 1H, minor), 8.13 (t, J = 8.3 Hz, 1H, major), 8.06 - 7.93 (m, 2 × 2H, major and minor), 7.17 (d, J = 5.2 Hz, 1H, major), 6.94 (d, J = 7.5 Hz, 1H, minor), 5.84 (dd, J = 7.6, 1.7 Hz, 1H, minor), 5.47 - 5.38 (m, 2 × 1H, major and minor), 5.31 (s, 1H, minor), 3.98 (s, 2H, major), 3.26 (s, 1H, major), 2.69 - 2.62 (m, 1H, minor), 1.39 (dd, J = 6.2, 1.9 Hz, 2 × 6H, major and minor), 1.13 - 0.90 (m, 2 × 4H, major and minor). |
| **P13** | 2-(2-(cyclopropanesulfonamido)pyrimidin-4-yl)-N-(4-(5-(trifluoromethyl)pyridin-3-yl)phenyl) acetamide 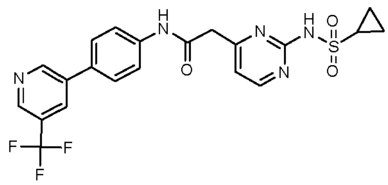 | Method 3 using INTC39 and INTD7, [UPLC Acidic], 478, (1.27) | 12.82 (s, 1H, minor), 11.25 (s, 1H, major), 10.92 (s, 1H, minor), 10.47 (s, 1H, major), 9.98 (s, 1H, minor), 9.23 - 9.19 (m, 2 × 1H, major and minor), 8.95 - 8.90 (m, 2 × 1H, major and minor), 8.56 (d, J = 5.0 Hz, 1H, major), 8.46 - 8.41 (m, 2 × 1H, major and minor), 7.89 - 7.81 (m, 2 × 2H, major and minor), 7.80 - 7.74 (m, 2 × 2H, major and minor), 7.17 (d, J = 5.0 Hz, 1H, major), 6.93 (d, J = 7.5 Hz, 1H, minor), 5.88 (d, J = 7.6 Hz, 1H, minor), 5.04 (s, 1H, minor), 3.87 (s, 2H, major), 3.28 - 3.22 (m, 1H, major), 2.70 - 2.65 (m, 1H, minor), 1.13 - 0.90 (m, 2 x 4H, major and minor). |

(continued)

| P | Name/Structure (All examples containing chiral centres are racemates unless stated) | Synthesis Method, [LCMS Method], m/z $(M+H)^+$, (RT/Min) | $^1$H NMR Chemical Shift Data (DMSO-d6 unless stated) |
|---|---|---|---|
| **P14** | 2-(2-(cyclopropanesulfonamido)pyrimidin-4-yl)-N-(4-(5-(2,2,2-trifluoroethoxy)pyridin-3-yl) phenyl)acetamide 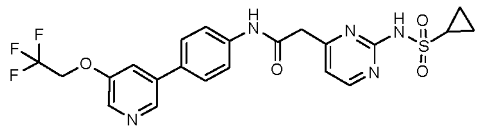 | Method 2 using INTC33 and INTD15, [HPLC Acidic], 508, (1.87) | 12.83 (s, 1H, minor), 11.26 (s, 1H, major), 10.89 (s, 1H, minor), 10.43 (s, 1H, major), 9.96 (s, 1H, minor), 8.59 (t, J = 2.2 Hz, 2 × 1H, major and minor), 8.57 (d, J = 5.1 Hz, 1H, major), 8.35 (dd, J = 4.6, 2.8 Hz, 2 × 1H, major and minor), 7.83 - 7.68 (m, 2 × 5H, major and minor), 7.17 (d, J = 5.1 Hz, 1H, major), 6.92 (d, J = 7.6 Hz, 1H, minor), 5.88 (dd, J = 7.6, 1.6 Hz, 1H, minor), 5.05 (s, 1H, minor), 5.01 - 4.94 (m, 2 × 2H, major and minor), 3.87 (s, 2H, major), 3.30 - 3.23 (m, 1H, major), 2.71 - 2.62 (m, 1H, minor), 1.13 - 0.89 (m, 2 × 4H, major and minor. |
| **P15** | 2-(2-(cyclopropanesulfonamido)-5-fluoropyrimidin-4-yl)-N-(4-(pyridin-3-yl)phenyl) acetamide 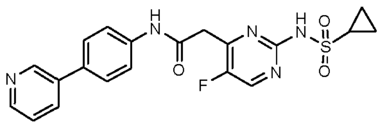 | Method 1 using INTC40 and a commercial aniline, [UPLC acidic], 428, (0.68) | 11.36 (s, 1H), 10.51 (s, 1H), 8.89 (s, 1H), 8.74 - 8.61 (m, 1H), 8.60 - 8.42 (m, 1H), 8.11 - 7.99 (m, 1H), 7.78 - 7.59 (m, 4H), 7.55 - 7.38 (m, 1H), 4.03 - 3.85 (m, 2H), 3.24 - 3.11 (m, 1H), 1.15 - 1.01 (m, 2H), 0.99 - 0.88 (m, 2H). |
| **P16** | 2-(2-(cyclopropanesulfonamido)pyrimidin-4-yl)-N-(4-(pyridin-3-yl)phenyl)acetamide 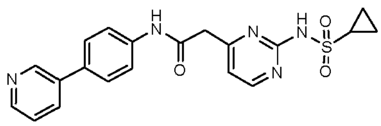 | Method 2 using INTC33 and a commercial aniline, [HPLC basic], 410, (1.20) | 12.82 (s, 1H, minor), 11.22 (s, 2 × 1H, major and minor), 10.42 (s, 2 × 1H, major and minor), 9.92 (s, 1H, minor), 8.89 (d, *J* = 2.2 Hz, 3H), 8.60 - 8.50 (m, 4H), 8.11 - 8.01 (m, 3H), 7.80 - 7.67 (m, 11H), 7.46 (ddd, *J* = 7.3, 4.8, 1.7 Hz, 2H), 7.16 (d, *J* = 5.1 Hz, 1H, major), 6.92 (d, *J* = 7.5 Hz, 1H, minor), 5.87 (dd, *J* = 7.6, 1.6 Hz, 1H, minor), 5.03 (s, 1H, minor), 3.86 (s, 2H, major), 3.29 - 3.22 (m, 1H, major), 3.18 (s, 3H), 2.73 - 2.61 (m, 1H, minor), 1.13 - 1.06 (m, 2H, major), 1.05 - 0.98 (m, 2H, minor), 0.98 - 0.88 (m, 2 x 2H, major and minor). |

(continued)

| P | Name/Structure (All examples containing chiral centres are racemates unless stated) | Synthesis Method, [LCMS Method], m/z $(M+H)^+$, (RT/Min) | $^1H$ NMR Chemical Shift Data (DMSO-d6 unless stated) |
|---|---|---|---|
| **P17** | N-([1,1'-biphenyl]-4-yl)-2-(2-(cyclopropanesulfonamido)pyrimidin-4-yl) acetamide 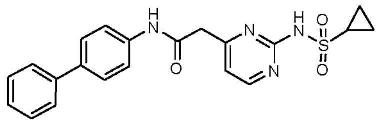 | Method 2, using INTC33 and a commercial aniline, [HPLC basic], 409, (1.84) | 12.84 (s, 1H, minor), 11.19 (s, 2H, major), 10.37 (s, 1H, major), 9.89 (s, 1H, minor), 8.59 - 8.53 (m, 1H, major), 7.71 - 7.40 (m, 2 × 8H, major and minor) 7.38 - 7.28 (m, 2 × 1H, major and minor), 7.19 - 7.13 (m, 1H, major), 6.94 - 6.87 (m, 1H, minor), 5.90 - 5.82 (m, 1H, minor), 5.04 (s, 1H, minor), 3.86 (s, 2H, major), 3.30 - 3.14 (m, 1H, major), 2.69 - 2.61 (m, 1H, minor), 1.14 - 0.88 (m, 2 × 4H, major and minor). |
| **P18** | 2-(2-(cyclopropanesulfonamido)pyrimidin-4-yl)-N-(4-(6-ethoxypyrazin-2-yl)phenyl)acetamide 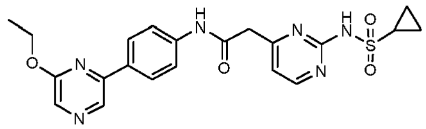 | Method 3 using INTC39 and INTD18, or Method 5 using INTC47, [UPLC Acidic], 455, (1.26) | 12.83 (s, 1H, minor), 11.23 (s, 1H, major), 10.91 (s, 1H, minor), 10.49 (s, 1H, major), 10.01 (s, 1H, minor), 8.77 (s, 1H, major), 8.76 (s, 1H, minor), 8.57 (d, J = 5.2 Hz, 1H, major), 8.19 (s, 1H, major), 8.17 (s, 1H, minor), 8.13 - 8.06 (m, 2 × 2H, major and minor), 7.79 - 7.73 (m, 2 × 2H, major and minor), 7.17 (d, J = 5.0 Hz, 1H, major), 6.93 (d, J = 7.6 Hz, 1H, minor), 5.88 (d, J = 7.6 Hz, 1H, minor), 5.05 (s, 1H, minor), 4.48 (q, J = 7.0 Hz, 2 x 2H, major and minor), 3.87 (s, 2H, major), 3.28 - 3.22 (m, 1H, major), 2.71 - 2.65 (m, 1H, minor), 1.40 (t, J = 7.0 Hz, 2 x 3H, major and minor), 1.13 - 0.90 (m, 2 x 4H, major and minor). |
| **P19** | 2-(2-(cyclopropanesulfonamido)pyrimidin-4-yl)-N-(4-(6-methoxypyrazin-2-yl)phenyl) acetamide 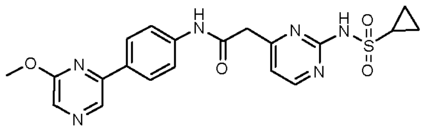 | Method 2, using INTC33 and INTD1, [HPLC Acidic], 441, (1.83) | 12.83 (s, 1H, minor), 11.24 (s, 1H, major), 10.91 (s, 1H, minor), 10.50 (s, 1H, major), 10.02 (s, 1H, minor), 8.79 (s, 1H, major), 8.78 (s, 1H, minor), 8.57 (d, J = 5.1 Hz, 1H, major), 8.22 (s, 1H, major), 8.20 (s, 1H, minor), 8.16 - 8.09 (m, 2 × 2H, major and minor), 7.79 - 7.73 (m, 2 × 2H, major and minor), 7.18 (d, J = 5.1 Hz, 1H, major), 6.93 (d, J = 7.6 Hz, 1H, minor), 5.88 (dd, J = 7.7, 1.6 Hz, 1H, minor), 5.06 (s, 1H, minor), 4.02 (s, 2 × 3H, major and minor), 3.88 (s, 2H, major), 3.30 - 3.21 (m, 1H, major), 2.70 - 2.62 (m, 1H, minor), 1.14 - 0.88 (m, 2 x 4H, major and minor). |

(continued)

| P | Name/Structure (All examples containing chiral centres are racemates unless stated) | Synthesis Method, [LCMS Method], m/z $(M+H)^+$, (RT/Min) | $^1H$ NMR Chemical Shift Data (DMSO-d6 unless stated) |
|---|---|---|---|
| P20 | 2-(2-(cyclopropanesulfonamido)pyrimidin-4-yl)-N-(4-(6-(2,2,2-trifluoroethoxy)pyrazin-2-yl) phenyl)acetamide 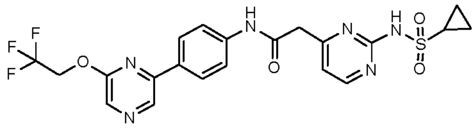 | Method 2, using INTC33 and INTD29, [HPLC Acidic], 509, (2.12) | 12.84 (s, 1H, minor), 11.24 (s, 1H, major), 10.92 (s, 1H, minor), 10.51 (s, 1H, major), 10.04 (s, 1H, minor), 8.94 (s, 1H, major), 8.92 (s, 1H, minor), 8.57 (d, J = 5.1 Hz, 1H, major), 8.38 (s, 1H, major), 8.36 (s, 1H, minor), 8.20 - 8.14 (m, 2 × 2H, major and minor), 7.79 - 7.75 (m, 2 × 2H, major and minor), 7.18 (d, J = 5.1 Hz, 1H, major), 6.93 (d, J = 7.6 Hz, 1H, minor), 5.89 (dd, J = 7.7, 1.6 Hz, 1H, minor), 5.24 - 5.15 (m, 2 × 2H, major and minor), 5.06 (s, 1H, minor), 3.88 (s, 2H, major), 3.29 - 3.22 (m, 1H, major), 2.70 - 2.63 (m, 1H, minor), 1.12 - 0.91 (m, 2 x 4H, major and minor). |
| P21 | 2-(2-(cyclopropanesulfonamido)pyrimidin-4-yl)-N-(4-(6-isopropoxypyrazin-2-yl)phenyl) acetamide 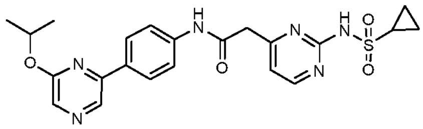 | Method 2, using INTC33 and INTD20, [HPLC Acidic], 469, (2.12) | 12.84 (s, 1H, minor), 11.24 (s, 1H, major), 10.91 (s, 1H, minor), 10.49 (s, 1H, major), 10.02 (s, 1H, minor), 8.74 (s, 1H, major), 8.73 (s, 1H, minor), 8.57 (d, J = 5.1 Hz, 1H, major), 8.13 (s, 1H, major), 8.11 (s, 1H, minor), 8.11 - 8.05 (m, 2 × 2H, major and minor), 7.78 - 7.72 (m, 2 × 2H, major and minor), 7.18 (d, J = 5.1 Hz, 1H, major), 6.93 (d, J = 7.6 Hz, 1H, minor), 5.88 (dd, J = 7.6, 1.6 Hz, 1H, minor), 5.46 - 5.36 (m, 2 × 1H, major and minor), 5.06 (s, 1H, minor), 3.88 (s, 2H, major), 3.29 - 3.21 (m, 1H, major), 2.70 - 2.61 (m, 1H, minor), 1.42 - 1.35 (m, 2 x 6H, major and minor), 1.13 - 0.90 (m, 2 x 4H, major and minor). |
| P22 | 2-(2-(cyclobutanesulfonamido)pyrimidin-4-yl)-N-(5-(6-ethoxypyrazin-2-yl)pyridin-2-yl)-2-methylpropanamide 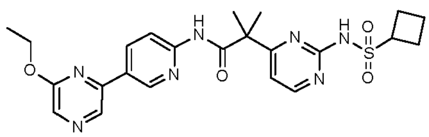 | Method 2, using INTC24 and INTD33, [HPLC Acidic], 498, (2.24) | 11.15 (s, 1H), 10.19 (s, 1H), 9.05 (d, J = 2.4 Hz, 1H), 8.85 (s, 1H), 8.60 - 8.51 (m, 2H), 8.29 (d, J = 8.8 Hz, 1H), 8.25 (s, 1H), 7.17 (d, J = 5.3 Hz, 1H), 4.55 - 4.43 (m, 3H), 2.32 - 2.22 (m, 2H), 2.04 - 1.92 (m, 2H), 1.80 - 1.70 (m, 1H), 1.59 (s, 6H), 1.57 - 1.52 (m, 1H), 1.40 (t, J = 7.0 Hz, 3H) |

(continued)

| P | Name/Structure (All examples containing chiral centres are racemates unless stated) | Synthesis Method, [LCMS Method], m/z (M+H)+, (RT/Min) | $^1$H NMR Chemical Shift Data (DMSO-d6 unless stated) |
|---|---|---|---|
| **P23** | 2-(2-(cyclobutanesulfonamido)pyrimidin-4-yl)-N-(2-fluoro-4-(6-isopropoxypyrazin-2-yl) phenyl)-2-methylpropanamide 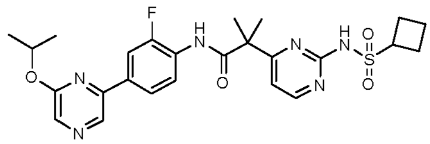 | Method 2, using INTC24 and INTD35, [HPLC Acidic], 529, (2.48) | 11.15 (s, 1H), 9.36 (s, 1H), 8.82 (s, 1H), 8.59 (d, J = 5.2 Hz, 1H), 8.19 (s, 1H), 8.02 - 7.94 (m, 2H), 7.79 - 7.73 (m, 1H), 7.20 (d, J = 5.3 Hz, 1H), 5.46 - 5.38 (m, 1H), 4.64 - 4.55 (m, 1H), 2.44 - 2.33 (m, 2H), 2.23 - 2.12 (m, 2H), 1.90 - 1.76 (m, 2H), 1.60 (s, 6H), 1.38 (d, J = 6.3 Hz, 6H); |
| **P24** | 2-(2-(cyclobutanesulfonamido)pyrimidin-4-yl)-N-(4-(6-ethoxypyrazin-2-yl)-2-methylphenyl)-2-methylpropanamide 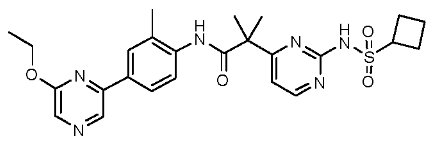 | Method 2, using INTC24 and INTD27, [HPLC Acidic], 511, (2.31) | 11.18 (s, 1H), 9.03 (s, 1H), 8.78 (s, 1H), 8.59 (d, J = 5.2 Hz, 1H), 8.20 (s, 1H), 7.98 (d, J = 2.0 Hz, 1H), 7.93 (dd, J = 8.3, 2.1 Hz, 1H), 7.46 (d, J = 8.3 Hz, 1H), 7.21 (d, J = 5.3 Hz, 1H), 4.66 - 4.56 (m, 1H), 4.48 (q, J = 7.0 Hz, 2H), 2.45 - 2.34 (m, 2H), 2.26 - 2.12 (m, 5H), 1.94 - 1.83 (m, 2H), 1.61 (s, 6H), 1.40 (t, J = 7.0 Hz, 3H). |
| **P25** | 2-(2-(cyclobutanesulfonamido)pyrimidin-4-yl)-N-(4-(6-methoxypyrazin-2-yl)phenyl)-2-methylpropanamide 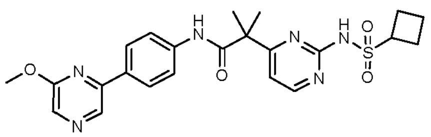 | Method 2, using INTC24 and INTD1, [HPLC Acidic], 483, (2.18) | 11.11 (s, 1H), 9.50 (s, 1H), 8.79 (s, 1H), 8.59 (d, J = 5.3 Hz, 1H), 8.21 (s, 1H), 8.15 - 8.09 (m, 2H), 7.90 - 7.81 (m, 2H), 7.21 (d, J = 5.3 Hz, 1H), 4.54 (p, J = 8.4 Hz, 1H), 4.01 (s, 3H), 2.37 - 2.22 (m, 2H), 2.07 - 1.97 (m, 2H), 1.64 - 1.54 (m, 6H), 0.89 - 0.82 (m, 2H), |
| **P26** | 2-(2-(cyclobutanesulfonamido)pyrimidin-4-yl)-N-(4-(6-ethoxypyrazin-2-yl)phenyl)-2-methylpropanamide 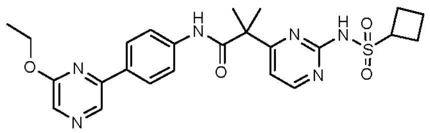 | Method 2, using INTC24 and INTD18, [HPLC Basic], 497, (2.33) | 11.14 (s, 1H), 9.51 (s, 1H), 8.77 (s, 1H), 8.58 (d, J = 5.3 Hz, 1H), 8.18 (s, 1H), 8.13 - 8.06 (m, 2H), 7.88 - 7.77 (m, 2H), 7.19 (d, J = 5.4 Hz, 1H), 4.54 (q, J = 8.3 Hz, 1H), 4.47 (q, J = 7.1 Hz, 2H), 2.36 - 2.24 (m, 2H), 2.06 - 1.95 (m, 2H), 1.81 - 1.69 (m, 1H), 1.66 - 1.60 (m, 1H), 1.58 (s, 6H), 1.40 (t, J = 7.0 Hz, 3H). |
| **P27** | 2-(2-(cyclopropanesulfonamido)pyrimidin-4-yl)-N-(5-(6-ethoxypyrazin-2-yl)-3-fluoropyridin-2-yl)-2-methylpropanamide 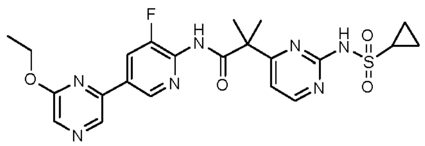 | Method 2, using INTC21 and INTD31, [UPLC Acidic], 502, (1.27) | 11.28 (s, 1H), 10.08 (s, 1H), 8.99 - 8.97 (m, 1H), 8.93 (s, 1H), 8.62 (d, J = 5.3 Hz, 1H), 8.45 - 8.40 (m, 1H), 8.32 (s, 1H), 7.19 (d, J = 5.3 Hz, 1H), 4.50 (q, J = 7.0 Hz, 2H), 3.33 - 3.27 (m, 1H), 1.62 (s, 6H), 1.41 (t, J = 7.0 Hz, 3H), 1.15 - 1.07 (m, 2H), 1.06 - 0.98 (m, 2H). |

(continued)

| P | Name/Structure (All examples containing chiral centres are racemates unless stated) | Synthesis Method, [LCMS Method], m/z (M+H)+, (RT/Min) | $^1$H NMR Chemical Shift Data (DMSO-d6 unless stated) |
|---|---|---|---|
| **P28** | 2-(2-(cyclopropanesulfonamido)pyrimidin-4-yl)-N-(5'-ethoxy-[3,3'-bipyridin]-6-yl)-2-methylpropanamide 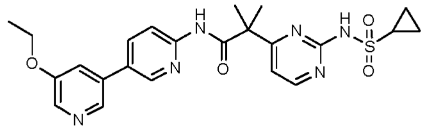 | Method 2, using INTC21 and INTD16, [UPLC acidic], 483, (1.09) | 11.25 (s, 1H), 10.06 (s, 1H), 8.72 - 8.68 (m, 1H), 8.60 (d, J = 5.3 Hz, 1H), 8.52 (d, J = 1.9 Hz, 1H), 8.29 (d, J = 2.7 Hz, 1H), 8.24 - 8.12 (m, 2H), 7.70 - 7.65 (m, 1H), 7.20 (d, J = 5.3 Hz, 1H), 4.21 (q, J = 7.0 Hz, 2H), 3.23 - 3.13 (m, 1H), 1.61 (s, 6H), 1.38 (t, J = 7.0 Hz, 3H), 1.07 - 0.97 (m, 2H), 0.83 - 0.73 (m, 2H). |
| **P29** | N-([3,3'-bipyridin]-6-yl)-2-(2-(cyclopropanesulfonamido)pyrimidin-4-yl)-2-methylpropanamide 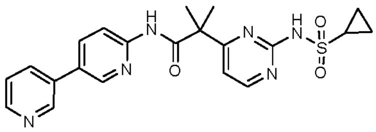 | Method 2, using INTC21 and a commercial aniline, [HPLC acidic], 439, (1.29) | 11.25 (s, 1H), 10.06 (s, 1H), 8.94 (d, J = 2.3 Hz, 1H), 8.70 - 8.67 (m, 1H), 8.61 - 8.56 (m, 2H), 8.22 - 8.08 (m, 3H), 7.54 - 7.46 (m, 1H), 7.18 (d, J = 5.3 Hz, 1H), 3.23 - 3.13 (m, 1H), 1.61 (s, 6H), 1.07 - 0.96 (m, 2H), 0.83 - 0.71 (m, 2H). |
| **P30** | 2-(2-(cyclopropanesulfonamido)pyrimidin-4-yl)-2-methyl-N-(5-(6-(trifluoromethyl)pyrazin-2-yl) pyridin-2-yl)propanamide 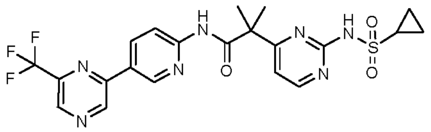 | Method 2 using INTC21 and INTD2, [UPLC Acidic], 508, (1.41) | 11.24 (s, 1H), 10.29 (s, 1H), 9.66 (s, 1H), 9.21 - 9.02 (m, 2H), 8.69 - 8.49 (m, 2H), 8.36 - 8.19 (m, 1H), 7.21 (d, J = 5.3 Hz, 1H), 3.23 - 3.11 (m, 1H), 1.61 (s, 6H), 1.07 - 0.91 (m, 2H), 0.83 - 0.71 (m, 2H). |
| **P31** | 2-(2-(cyclopropanesulfonamido)pyrimidin-4-yl)-N-(5-(6-ethoxypyrazin-2-yl)pyridin-2-yl)-2-methylpropanamide 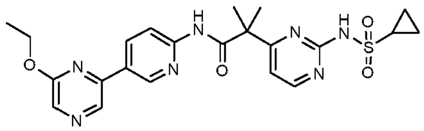 | Method 2, using INTC21 and INTD33, [UPLC Acidic], 484, (1.37) | 11.25 (s, 1H), 10.16 (s, 1H), 9.09 - 8.98 (m, 1H), 8.84 (s, 1H), 8.59 (d, J = 5.3 Hz, 1H), 8.52 - 8.46 (m, 1H), 8.25 (s, 1H), 8.23 - 8.18 (m, 1H), 7.20 (d, J = 5.3 Hz, 1H), 4.48 (q, J = 7.0 Hz, 2H), 3.24 - 3.11 (m, 1H), 1.61 (s, 6H), 1.40 (t, J = 7.0 Hz, 3H), 1.06 - 0.95 (m, 2H), 0.83 - 0.70 (m, 2H). |
| **P32** | 2-(2-(cyclopropanesulfonamido)pyrimidin-4-yl)-N-(5-(6-cyclopropoxypyrazin-2-yl)pyridin-2-yl)-2-methylpropanamide 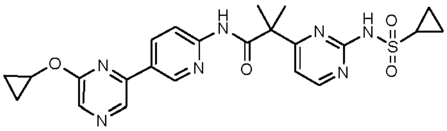 | Method 2, using INTC21 and INTD30, [UPLC acidic], 496, (1.39) | 11.30 (s, 1H), 10.20 (s, 1H), 9.05 (d, J = 2.4 Hz, 1H), 8.92 (s, 1H), 8.58 (d, J = 5.3 Hz, 1H), 8.55 - 8.49 (m, 1H), 8.31 (s, 1H), 8.21 (d, J = 8.8 Hz, 1H), 7.18 (d, J = 5.3 Hz, 1H), 4.48 - 4.35 (m, 1H), 3.23 - 3.10 (m, 1H), 1.61 (s, 6H), 1.06 - 0.94 (m, 2H), 0.92 - 0.68 (m, 6H). |

(continued)

| P | Name/Structure (All examples containing chiral centres are racemates unless stated) | Synthesis Method, [LCMS Method], m/z $(M+H)^+$, (RT/Min) | $^1$H NMR Chemical Shift Data (DMSO-d6 unless stated) |
|---|---|---|---|
| P33 | N-(2-chloro-4-(6-ethoxypyrazin-2-yl)phenyl)-2-(2-(cyclopropanesulfonamido)pyrimidin-4-yl)-2-methylpropanamide 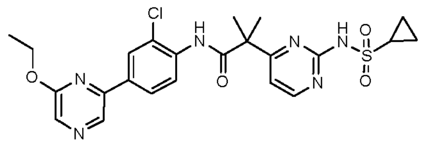 | Method 2, using INTC21 and INTD49, [UPLC Acidic], 517, (1.53) | 11.30 (s, 1H), 9.24 (s, 1H), 8.85 (s, 1H), 8.63 (d, J = 5.3 Hz, 1H), 8.27 - 8.20 (m, 2H), 8.10 (dd, J = 8.5, 2.1 Hz, 1H), 7.78 (d, J = 8.4 Hz, 1H), 7.25 (d, J = 5.3 Hz, 1H), 4.48 (q, J = 7.0 Hz, 2H), 3.31 - 3.20 (m, 1H), 1.63 (s, 6H), 1.40 (t, J = 7.0 Hz, 3H), 1.17 - 1.07 (m, 2H), 1.03 - 0.95 (m, 2H). |
| P34 | N-(2-cyano-4-(6-ethoxypyrazin-2-yl)phenyl)-2-(2-(cyclopropanesulfonamido)pyrimidin-4-yl)-2-methylpropanamide 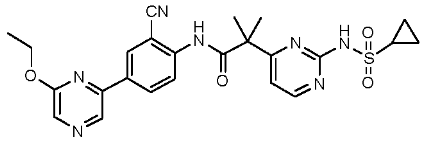 | Method 2, using INTC21 and INTD51, [UPLC Acidic], 508, (1.45) | 11.28 (s, 1H), 9.85 (s, 1H), 8.90 (s, 1H), 8.62 (d, J = 5.3 Hz, 1H), 8.55 (d, J = 2.1 Hz, 1H), 8.43 (dd, J = 8.6, 2.1 Hz, 1H), 8.29 (s, 1H), 7.69 - 7.62 (m, 1H), 7.25 (d, J = 5.3 Hz, 1H), 4.50 (q, J = 7.0 Hz, 2H), 3.28 - 3.19 (m, 1H), 1.63 (s, 6H), 1.40 (t, J = 7.0 Hz, 3H), 1.17 - 1.08 (m, 2H), 1.04 - 0.96 (m, 2H). |
| P35 | 2-(2-(cyclopropanesulfonamido)pyrimidin-4-yl)-N-(2-fluoro-4-(5-isopropoxypyridin-3-yl) phenyl)-2-methylpropanamide 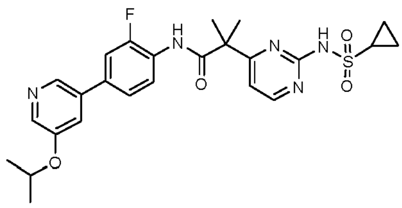 | Method 2, using INTC21 and INTD12, [UPLC acidic], 514, (1.25) | 11.52 (s, 1H), 9.48 (s, 1H), 8.57 (d, J = 5.3 Hz, 1H), 8.53 - 8.46 (m, 1H), 8.25 (d, J = 2.7 Hz, 1H), 7.75 - 7.53 (m, 4H), 7.12 (d, J = 5.3 Hz, 1H), 4.99 - 4.78 (m, 1H), 3.26 - 3.16 (m, 1H), 1.60 (s, 6H), 1.32 (d, J = 6.0 Hz, 6H), 1.12 - 1.03 (m, 2H), 1.00 - 0.89 (m, 2H). |
| P36 | 2-(2-(cyclopropanesulfonamido)pyrimidin-4-yl)-N-(2-fluoro-4-(pyridin-3-yl)phenyl)-2-methylpropanamide 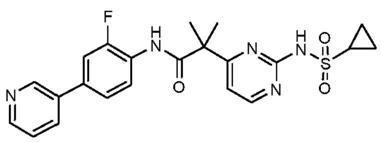 | Method 2, using INTC21 and INTD50, [HPLC Acidic], 456, (1.37) | 11.41 (s, 1H), 9.41 (s, 1H), 8.97 - 8.90 (m, 1H), 8.62 - 8.55 (m, 2H), 8.15 - 8.09 (m, 1H), 7.72 - 7.54 (m, 3H), 7.52 - 7.46 (m, 1H), 7.16 (d, J = 5.3 Hz, 1H), 3.27 - 3.19 (m, 1H), 1.61 (s, 6H), 1.15 -1.05 (m, 2H), 1.02 - 0.90 (m, 2H). |
| P37 | 2-(2-(cyclopropanesulfonamido)pyrimidin-4-yl)-N-(2-fluoro-4-(6-(trifluoromethyl)pyrazin-2-yl) phenyl)-2-methylpropanamide 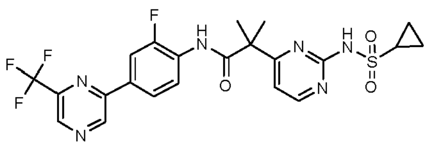 | Method 2, using INTC21 and INTD25, [UPLC Acidic], 525, (1.47) | 11.30 (s, 1H), 9.65 (s, 1H), 9.47 (s, 1H), 9.15 (s, 1H), 8.61 (d, J = 5.3 Hz, 1H), 8.13 - 8.02 (m, 2H), 7.85 - 7.77 (m, 1H), 7.21 (d, J = 5.3 Hz, 1H), 3.28 - 3.21 (m, 1H), 1.62 (s, 6H), 1.16 - 1.05 (m, 2H), 1.03 - 0.92 (m, 2H). |

(continued)

| P | Name/Structure (All examples containing chiral centres are racemates unless stated) | Synthesis Method, [LCMS Method], m/z $(M+H)^+$, (RT/Min) | $^1H$ NMR Chemical Shift Data (DMSO-d6 unless stated) |
|---|---|---|---|
| P38 | 2-(2-(cyclopropanesulfonamido)pyrimidin-4-yl)-N-(4-(6-ethoxypyrazin-2-yl)-2-fluorophenyl)-2-methylpropanamide 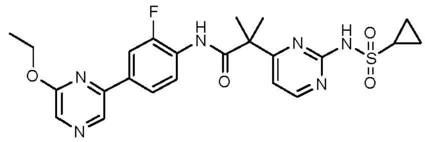 | Method 2, using INTC21 and INTD24; [UPLC acidic], 501, (1.46) | 11.32 (s, 1H), 9.41 (s, 1H), 8.84 (s, 1H), 8.70 - 8.51 (m, 1H), 8.25 (s, 1H), 8.06 - 7.90 (m, 2H), 7.77 - 7.63 (m, 1H), 7.28 - 7.13 (m, 1H), 4.56 - 4.42 (m, 2H), 3.28 - 3.19 (m, 1H), 1.61 (s, 6H), 1.47 - 1.32 (m, 3H), 1.18 - 1.05 (m, 2H), 1.03 - 0.91 (m, 2H). |
| P39 | 2-(2-(cyclopropanesulfonamido)pyrimidin-4-yl)-N-(2-fluoro-4-(6-isopropoxypyrazin-2-yl) phenyl)-2-methylpropanamide 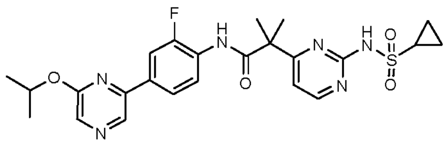 | Method 2, using INTC21 and INTD35, [UPLC acidic], 515, (1.54) | 11.33 (s, 1H), 9.41 (s, 1H), 8.82 (s, 1H), 8.61 (d, J = 5.3 Hz, 1H), 8.19 (s, 1H), 8.04 - 7.90 (m, 2H), 7.79 - 7.63 (m, 1H), 7.19 (d, J = 5.3 Hz, 1H), 5.51 - 5.33 (m, 1H), 3.29 - 3.17 (m, 1H), 1.61 (s, 6H), 1.39 (d, J = 6.1 Hz, 6H), 1.15 - 1.06 (m, 2H), 1.02 - 0.92 (m, 2H). |
| P40 | 2-(2-(cyclopropanesulfonamido)pyrimidin-4-yl)-N-(4-(6-ethoxypyrazin-2-yl)-2-fluoro-5-methylphenyl)-2-methylpropanamide 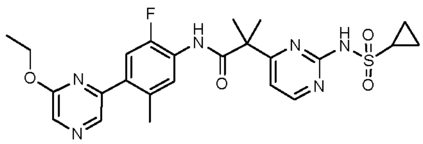 | Method 2, using INTC21 and INTD39, [UPLC Acidic], 515, (1.46) | 11.29 (s, 1H), 9.34 (s, 1H), 8.61 (d, J = 5.3 Hz, 1H), 8.41 (s, 1H), 8.27 (s, 1H), 7.48 (d, J = 7.7 Hz, 1H), 7.42 (d, J = 11.2 Hz, 1H), 7.19 (d, J = 5.3 Hz, 1H), 4.39 (q, J = 7.1 Hz, 2H), 3.30 - 3.21 (m, 1H), 2.37 (s, 3H), 1.61 (s, 6H), 1.37 (t, J = 7.0 Hz, 3H), 1.14 - 1.08 (m, 2H), 1.03 - 0.96 (m, 2H). |
| P41 | 2-(2-(cyclopropanesulfonamido)pyrimidin-4-yl)-N-(4-(6-ethoxypyrazin-2-yl)-2,6-difluorophenyl)-2-methylpropanamide 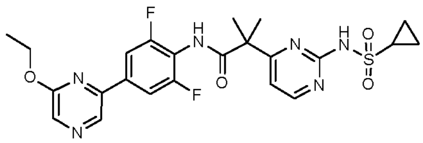 | Method 2, using INTC21 and INTD41, [UPLC Acidic], 519, (1.42) | 11.29 (s, 1H), 9.47 (s, 1H), 8.90 (s, 1H), 8.62 (d, J = 5.3 Hz, 1H), 8.30 (s, 1H), 7.92 (d, J = 8.9 Hz, 2H), 7.16 (d, J = 5.3 Hz, 1H), 4.49 (q, J = 7.0 Hz, 2H), 3.31 - 3.22 (m, 1H), 1.61 (s, 6H), 1.39 (t, J = 7.0 Hz, 3H), 1.15 - 1.08 (m, 2H), 1.10 - 0.99 (m, 2H). |
| P42 | 2-(2-(cyclopropanesulfonamido)pyrimidin-4-yl)-N-(2-fluoro-4-(pyrazin-2-yl)phenyl)-2-methylpropanamide 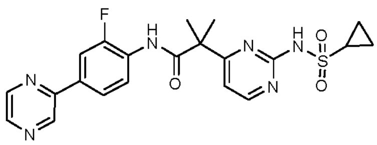 | Method 2, using INTC21 and INTD23, [UPLC Acidic], 457, (1.14) | 11.30 (s, 1H), 9.40 (s, 1H), 9.30 (d, J = 1.5 Hz, 1H), 8.74 - 8.69 (m, 1H), 8.67 - 8.56 (m, 2H), 8.07 - 7.95 (m, 2H), 7.73 (t, J = 8.1 Hz, 1H), 7.20 (d, J = 5.3 Hz, 1H), 3.28 - 3.19 (m, 1H), 1.61 (s, 6H), 1.16 - 1.03 (m, 2H), 1.02 - 0.92 (m, 2H). |

(continued)

| P | Name/Structure (All examples containing chiral centres are racemates unless stated) | Synthesis Method, [LCMS Method], m/z $(M+H)^+$, (RT/Min) | $^1H$ NMR Chemical Shift Data (DMSO-d6 unless stated) |
|---|---|---|---|
| P43 | 2-(2-(cyclopropanesulfonamido)pyrimidin-4-yl)-2-methyl-N-(2-methyl-4-(6-(trifluoromethyl) pyrazin-2-yl)phenyl)propanamide 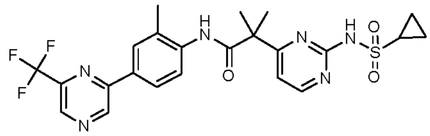 | Method 2, using INTC21 and INTD34; [UPLC Acidic], 521, (1.45) | 11.28 (s, 1H), 9.60 (s, 1H), 9.12 - 9.05 (m, 2H), 8.62 (d, J = 5.3 Hz, 1H), 8.09 - 7.99 (m, 2H), 7.52 (d, J = 8.3 Hz, 1H), 7.23 (d, J = 5.3 Hz, 1H), 3.31 - 3.21 (m, 1H), 2.21 (s, 3H), 1.62 (s, 6H), 1.17-1.06 (m, 2H), 1.04 - 0.96 (m, 2H). |
| P44 | 2-(2-(cyclopropanesulfonamido)pyrimidin-4-yl)-N-(4-(6-ethoxypyrazin-2-yl)-2,3-dimethylphenyl)-2-methylpropanamide 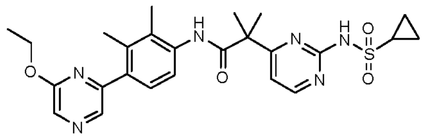 | Method 2, using INTC21 and INTD38, [UPLC Acidic], 512, (1.39) | 11.28 (s, 1H), 9.16 (s, 1H), 8.62 (d, J = 5.3 Hz, 1H), 8.26 (d, J = 11.0 Hz, 2H), 7.26 - 7.19 (m, 2H), 7.15 (d, J = 8.2 Hz, 1H), 4.37 (q, J = 7.1 Hz, 2H), 3.31 - 3.23 (m, 1H), 2.23 (s, 3H), 2.05 (s, 3H), 1.63 (s, 6H), 1.36 (t, J = 7.0 Hz, 3H), 1.16-1.09 (m, 2H), 1.08 - 0.99 (m, 2H). |
| P45 | 2-(2-(cyclopropanesulfonamido)pyrimidin-4-yl)-N-(4-(6-ethoxypyrazin-2-yl)-5-fluoro-2-methylphenyl)-2-methylpropanamide 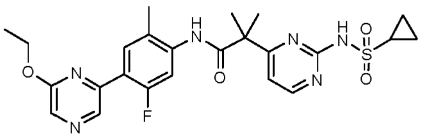 | Method 2, using INTC21 and INTD36; [UPLC Acidic], 515, (1.50) | 11.29 (s, 1H), 9.08 (s, 1H), 8.65 - 8.55 (m, 2H), 8.25 (d, J = 2.9 Hz, 1H), 7.82 (dd, J = 8.6, 2.5 Hz, 1H), 7.42 (dd, J = 12.9, 2.6 Hz, 1H), 7.27 - 7.21 (m, 1H), 4.50 - 4.41 (m, 2H), 3.26 - 3.20 (m, 1H), 2.16 (s, 3H), 1.61 (s, 6H), 1.42 - 1.35 (m, 3H), 1.14 - 1.05 (m, 2H), 1.03 - 0.97 (m, 2H). |
| P46 | 2-(2-(cyclopropanesulfonamido)pyrimidin-4-yl)-N-(4-(6-ethoxypyrazin-2-yl)-2,5-dimethylphenyl)-2-methylpropanamide 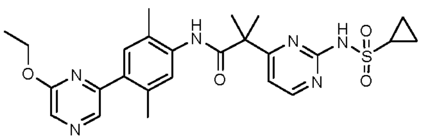 | Method 2, using INTC21 and INTD40, [UPLC Acidic], 511, (1.44) | 11.28 (s, 1H), 9.01 (s, 1H), 8.62 (d, J = 5.3 Hz, 1H), 8.35 (s, 1H), 8.23 (s, 1H), 7.34 (s, 1H), 7.25 - 7.20 (m, 2H), 4.38 (q, J = 7.0 Hz, 2H), 3.31 - 3.22 (m, 1H), 2.34 (s, 3H), 2.10 (s, 3H), 1.62 (s, 6H), 1.36 (t, J = 7.0 Hz, 3H), 1.16 - 1.09 (m, 2H), 1.05 - 0.98 (m, 2H). |
| P47 | 2-(2-(cyclopropanesulfonamido)pyrimidin-4-yl)-N-(4-(6-ethoxypyrazin-2-yl)-2-(trifluoromethoxy)phenyl)-2-methylpropanamide 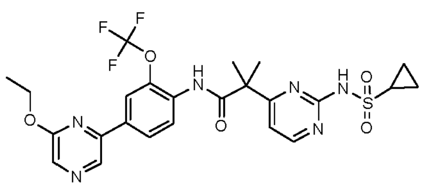 | Method 2, using INTC21 and INTD47, [UPLC Acidic], 567, (1.59) | 11.28 (s, 1H), 9.36 (s, 1H), 8.87 (s, 1H), 8.61 (d, J = 5.3 Hz, 1H), 8.27 (s, 1H), 8.16 (dd, J = 8.5, 2.0 Hz, 1H), 8.12 - 8.07 (m, 1H), 7.78 (d, J = 8.4 Hz, 1H), 7.21 (d, J = 5.2 Hz, 1H), 4.48 (q, J = 7.0 Hz, 2H), 3.27 - 3.21 (m, 1H), 1.61 (s, 6H), 1.40 (t, J = 7.0 Hz, 3H), 1.15 - 1.08 (m, 2H), 1.03 - 0.96 (m, 2H). |

(continued)

| P | Name/Structure (All examples containing chiral centres are racemates unless stated) | Synthesis Method, [LCMS Method], m/z $(M+H)^+$, (RT/Min) | $^1$H NMR Chemical Shift Data (DMSO-d6 unless stated) |
|---|---|---|---|
| P48 | 2-(2-(cyclopropanesulfonamido)pyrimidin-4-yl)-N-(4-(6-ethoxypyrazin-2-yl)-5-fluoro-2-methoxyphenyl)-2-methylpropanamide 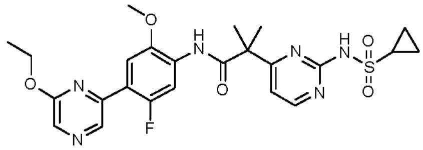 | Method 2, using INTC21 and INTD37, [UPLC Acidic], 531, (1.59) | 11.36 (s, 1H), 9.01 (s, 1H), 8.66 - 8.58 (m, 2H), 8.25 (s, 1H), 8.00 (d, J = 13.1 Hz, 1H), 7.59 (d, J = 6.9 Hz, 1H), 7.26 (d, J = 5.3 Hz, 1H), 4.51 - 4.42 (m, 2H), 3.89 (s, 3H), 3.23 - 3.14 (m, 1H), 1.63 (s, 6H), 1.43 - 1.36 (m, 3H), 1.13 - 1.08 (m, 2H), 1.00 - 0.94 (m, 2H). |
| P49 | 2-(2-(cyclopropanesulfonamido)pyrimidin-4-yl)-N-(4-(6-ethoxypyrazin-2-yl)-2-methoxyphenyl)-2-methylpropanamide 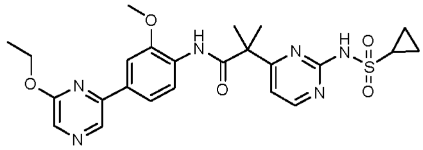 | Method 2, using INTC21 and INTD48; [UPLC Acidic], 513, (1.48) | 11.34 (s, 1H), 8.86 - 8.81 (m, 2H), 8.63 (d, J = 5.3 Hz, 1H), 8.21 - 8.17 (m, 1H), 8.05 (d, J = 8.7 Hz, 1H), 7.74 - 7.69 (m, 2H), 7.26 (d, J = 5.3 Hz, 1H), 4.47 (q, 2H), 3.91 (s, 3H), 3.26 - 3.17 (m, 1H), 1.63 (s, 6H), 1.39 (t, J = 7.0 Hz, 3H), 1.13 - 1.06 (m, 2H), 0.99 - 0.91 (m, 2H). |
| P50 | 2-(2-(cyclopropanesulfonamido)pyrimidin-4-yl)-2-methyl-N-(4-(pyrimidin-5-yl)phenyl) propanamide 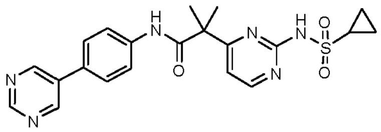 | Method 2, using INTC21 and a commercial aniline, [UPLC Acidic], 439, (1.04) | 11.25 (s, 1H), 9.46 (s, 1H), 9.17 - 9.09 (m, 3H), 8.59 (d, J = 5.3 Hz, 1H), 7.81 - 7.73 (m, 4H), 3.24 - 3.14 (m, 1H), 1.59 (s, 6H), 1.07 - 0.99 (m, 2H), 0.85 - 0.77 (m, 2H). 1 exchangeable proton not observed. |
| P51 | N-(4-(5-chloropyridin-3-yl)phenyl)-2-(2-(cyclopropanesulfonamido)pyrimidin-4-yl)-2-methylpropanamide 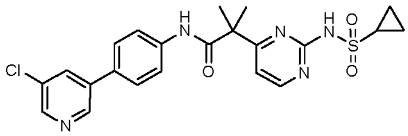 | Method 2, using INTC21 and INTD8; [UPLC acidic], 472, (1.36) | 11.30 (s, 1H), 9.48 (s, 1H), 8.86 (d, J = 2.0 Hz, 1H), 8.63 - 8.55 (m, 2H), 8.23 (t, J = 2.2 Hz, 1H), 7.76 (s, 4H), 7.18 (d, J = 5.3 Hz, 1H), 3.25 - 3.13 (m, 1H), 1.59 (s, 6H), 1.09 - 0.98 (m, 2H), 0.85 - 0.75 (m, 2H). |
| P52 | N-(4-(5-cyanopyridin-3-yl)phenyl)-2-(2-(cyclopropanesulfonamido)pyrimidin-4-yl)-2-methylpropanamide 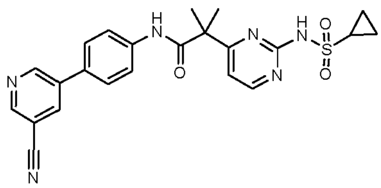 | Method 2, using INTC21 and INTD5, [HPLC acidic], 463, (2.46) | 11.34 (s, 1H), 9.52 (s, 1H), 9.19 (d, J = 2.3 Hz, 1H), 8.96 (d, J = 1.9 Hz, 1H), 8.64 (t, J = 2.1 Hz, 1H), 8.59 (d, J = 5.3 Hz, 1H), 7.83 - 7.73 (m, 4H), 7.17 (d, J = 5.3 Hz, 1H), 3.26 - 3.13 (m, 1H), 1.59 (s, 6H), 1.08 - 0.98 (m, 2H), 0.86 - 0.74 (m, 2H). |

(continued)

| P | Name/Structure (All examples containing chiral centres are racemates unless stated) | Synthesis Method, [LCMS Method], m/z $(M+H)^+$, (RT/Min) | $^1H$ NMR Chemical Shift Data (DMSO-d6 unless stated) |
|---|---|---|---|
| P53 | 2-(2-(cyclopropanesulfonamido)pyrimidin-4-yl)-N-(4-(5-fluoropyridin-3-yl)phenyl)-2-methylpropanamide 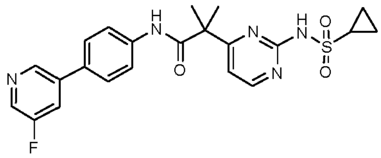 | Method 2, using INTC21 and INTD6, [UPLC acidic], 456, (1.25) | 11.30 (s, 1H), 9.48 (s, 1H), 8.88 - 8.76 (m, 1H), 8.60 (d, J = 5.3 Hz, 1H), 8.53 (d, J = 2.7 Hz, 1H), 8.09 - 8.00 (m, 1H), 7.76 (s, 4H), 7.19 (d, J = 5.3 Hz, 1H), 3.25 - 3.15 (m, 1H), 1.60 (s, 6H), 1.08 - 0.99 (m, 2H), 0.87 - 0.74 (m, 2H). |
| P54 | 2-(2-(cyclopropanesulfonamido)pyrimidin-4-yl)-2-methyl-N-(4-(5-methylpyridin-3-yl)phenyl) propanamide 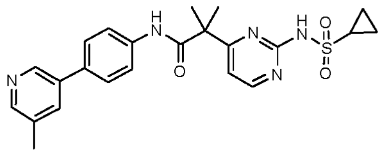 | Method 2, using INTC21 and INTD10, [HPLC acidic], 452, (1.26) | 11.29 (s, 1H), 9.43 (s, 1H), 8.67 (d, J = 2.2 Hz, 1H), 8.60 (d, J = 5.3 Hz, 1H), 8.39 - 8.35 (m, 1H), 7.90 - 7.86 (m, 1H), 7.77 - 7.62 (m, 4H), 7.18 (d, J = 5.3 Hz, 1H), 3.28 - 3.11 (m, 1H), 2.36 (s, 3H), 1.60 (s, 6H), 1.09 - 0.97 (m, 2H), 0.86 - 0.74 (m, 2H). |
| P55 | 2-(2-(cyclopropanesulfonamido)pyrimidin-4-yl)-N-(4-(5-(difluoromethoxy)pyridin-3-yl) phenyl)-2-methylpropanamide 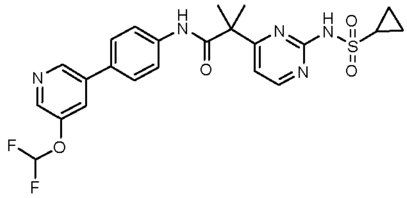 | Method 2, using INTC21 and INTD3, [UPLC acidic], 504, (1.32) | 11.29 (s, 1H), 9.51 (s, 1H), 8.80 (d, J = 1.9 Hz, 1H), 8.59 (d, J = 5.3 Hz, 1H), 8.44 (d, J = 2.6 Hz, 1H), 7.95 - 7.89 (m, 1H), 7.81 - 7.71 (m, 4H), 7.34 (t, J = 73.5 Hz, 1H), 7.16 (d, J = 5.3 Hz, 1H), 3.25 - 3.13 (m, 1H), 1.60 (s, 6H), 1.08 - 0.98 (m, 2H), 0.87 - 0.74 (m, 2H). |
| P56 | 2-(2-(cyclopropanesulfonamido)pyrimidin-4-yl)-N-(4-(5-methoxypyridin-3-yl)phenyl)-2-methylpropanamide 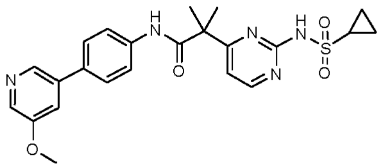 | Method 2, using INTC21 and INTD45, [HPLC acidic], 468, (1.89) | 11.34 (s, 1H), 9.46 (s, 1H), 8.60 (d, J = 5.3 Hz, 1H), 8.47 (d, J = 1.9 Hz, 1H), 8.25 (d, J = 2.8 Hz, 1H), 7.79 - 7.67 (m, 4H), 7.63 - 7.56 (m, 1H), 7.17 (d, J = 5.3 Hz, 1H), 3.91 (s, 3H), 3.26 - 3.14 (m, 1H), 1.60 (s, 6H), 1.09 - 0.96 (m, 2H), 0.88 - 0.75 (m, 2H). |
| P57 | 2-(2-(cyclopropanesulfonamido)pyrimidin-4-yl)-N-(4-(5-ethoxypyridin-3-yl)phenyl)-2-methylpropanamide 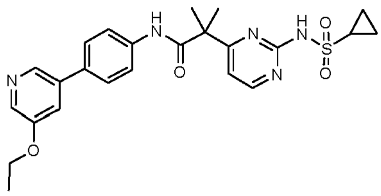 | Method 2, using INTC21 and INTD4, [UPLC acidic], 482, (1.06) | 11.29 (s, 1H), 9.43 (s, 1H), 8.60 (d, J = 5.3 Hz, 1H), 8.46 (d, J = 1.9 Hz, 1H), 8.23 (d, J = 2.7 Hz, 1H), 7.80 - 7.64 (m, 4H), 7.60 - 7.54 (m, 1H), 7.18 (d, J = 5.3 Hz, 1H), 4.20 (q, J = 7.0 Hz, 2H), 3.26 - 3.13 (m, 1H), 1.60 (s, 6H), 1.38 (t, J = 7.0 Hz, 3H), 1.10 - 0.96 (m, 2H), 0.88 - 0.75 (m, 2H). |

(continued)

| P | Name/Structure (All examples containing chiral centres are racemates unless stated) | Synthesis Method, [LCMS Method], m/z $(M+H)^+$, (RT/Min) | $^1H$ NMR Chemical Shift Data (DMSO-d6 unless stated) |
|---|---|---|---|
| **P58** | 2-(2-(cyclopropanesulfonamido)pyrimidin-4-yl)-N-(4-(5-isopropoxypyridin-3-yl)phenyl)-2-methylpropanamide 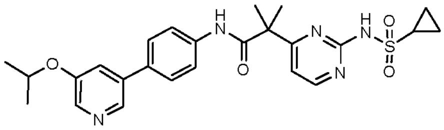 | Method 2, using INTC21 and INTD11, [UPLC acidic], 496, (1.16) | 11.34 (s, 1H), 9.45 (s, 1H), 8.59 (d, J = 5.2 Hz, 1H), 8.44 (d, J = 1.9 Hz, 1H), 8.21 (d, J = 2.7 Hz, 1H), 7.78 - 7.65 (m, 4H), 7.59 - 7.53 (m, 1H), 7.16 (d, J = 5.3 Hz, 1H), 4.92 - 4.76 (m, 1H), 3.27 - 3.13 (m, 1H), 1.60 (s, 6H), 1.32 (d, J = 6.0 Hz, 6H), 1.08 - 0.98 (m, 2H), 0.87 - 0.73 (m, 2H). |
| **P59** | 2-(2-(cyclopropanesulfonamido)pyrimidin-4-yl)-2-methyl-N-(4-(pyridin-3-yl)phenyl)propanamide 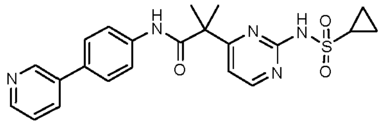 | Method 2, using INTC21 and acommercial aniline, [HPLC acidic], 438, (1.28) | 9.44 (s, 1H), 8.89 - 8.85 (m, 1H), 8.60 (d, J = 5.3 Hz, 1H), 8.53 (dd, J = 4.7, 1.6 Hz, 1H), 8.15 (s, 1H), 8.07 - 8.03 (m, 1H), 7.80 - 7.61 (m, 4H), 7.49 - 7.41 (m, 1H), 7.18 (d, J = 5.3 Hz, 1H), 3.26 - 3.15 (m, 1H), 1.60 (s, 6H), 1.08 - 0.99 (m, 2H), 0.89 - 0.73 (m, 2H). |
| **P60** | 2-(2-(cyclopropanesulfonamido)pyrimidin-4-yl)-2-methyl-N-(3'-(trifluoromethyl)-[1,1'-biphenyl]-4-yl)propanamide 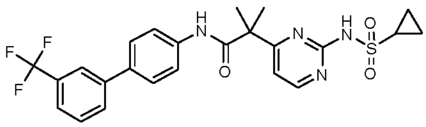 | Method 2, using INTC21 and acommercial aniline; UPLC Acidic], 505, (1.65) | 11.25 (s, 1H), 9.42 (s, 1H), 8.59 (d, J = 5.3 Hz, 1H), 7.99 - 7.91 (m, 2H), 7.77 - 7.63 (m, 6H), 7.18 (d, J = 5.3 Hz, 1H), 3.24 - 3.15 (m, 1H), 1.58 (s, 6H), 1.06 - 0.98 (m, 2H), 0.82 - 0.75 (m, 2H). |
| **P61** | N-(3'-chloro-[1, 1 '-biphenyl]-4-yl)-2-(2-(cyclopropanesulfonamido)pyrimidin-4-yl)-2-methylpropanamide 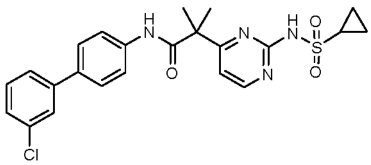 | Method 2, using INTC21 and acommercial aniline, [UPLC Acidic], 471, (1.63) | 11.26 (s, 1H), 9.41 (s, 1H), 8.59 (d, J = 5.3 Hz, 1H), 7.75 - 7.69 (m, 3H), 7.67 - 7.60 (m, 3H), 7.46 (td, J = 7.8, 1.6 Hz, 1H), 7.38 (d, J = 8.0 Hz, 1H), 7.18 (d, J = 5.3 Hz, 1H), 3.24 - 3.15 (m, 1H), 1.59 (s, 6H), 1.06 - 0.99 (m, 2H), 0.83 - 0.75 (m, 2H). |
| **P62** | N-(3'-cyano-[1, 1 '-biphenyl]-4-yl)-2-(2-(cyclopropanesulfonamido)pyrimidin-4-yl)-2-methylpropanamide 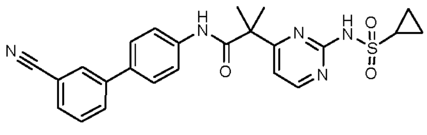 | Method 2, using INTC21 and acommercial aniline, [UPLC Acidic], 463, (1.42) | 11.27 (s, 1H), 9.42 (s, 1H), 8.59 (d, J = 5.3 Hz, 1H), 8.13 (d, J = 1.7 Hz, 1H), 8.03 - 7.97 (m, 1H), 7.80 - 7.69 (m, 5H), 7.63 (t, J = 7.8 Hz, 1H), 7.18 (d, J = 5.3 Hz, 1H), 3.24 - 3.15 (m, 1H), 1.59 (s, 6H), 1.06 - 0.99 (m, 2H), 0.83 - 0.75 (m, 2H). |

(continued)

| P | Name/Structure (All examples containing chiral centres are racemates unless stated) | Synthesis Method, [LCMS Method], m/z $(M+H)^+$, (RT/Min) | $^1H$ NMR Chemical Shift Data (DMSO-d6 unless stated) |
|---|---|---|---|
| **P63** | 2-(2-(cyclopropanesulfonamido)pyrimidin-4-yl)-N-(3'-ethoxy-[1,1'-biphenyl]-4-yl)-2-methylpropanamide 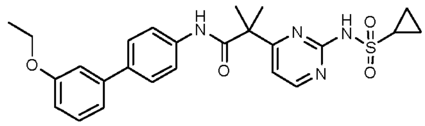 | Method 2, using INTC21 and INTD42, [UPLC Acidic], 481, (1.58) | 11.26 (s, 1H), 9.38 (s, 1H), 8.60 (d, J = 5.3 Hz, 1H), 7.72 - 7.66 (m, 2H), 7.63 - 7.57 (m, 2H), 7.37 - 7.30 (m, 1H), 7.22 - 7.12 (m, 3H), 6.88 (dd, J = 8.2, 2.5 Hz, 1H), 4.09 (q, J = 6.9 Hz, 2H), 3.25 - 3.16 (m, 1H), 1.59 (s, 6H), 1.35 (t, J = 6.9 Hz, 3H), 1.07 - 1.00 (m, 2H), 0.84 - 0.76 (m, 2H). |
| **P64** | 2-(2-(cyclopropanesulfonamido)pyrimidin-4-yl)-2-methyl-N-(4-(6-(trifluoromethyl)pyrazin-2-yl)phenyl)propanamide 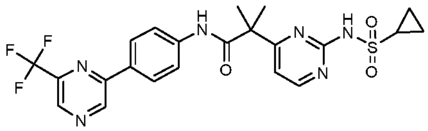 | Method 2, using INTC21 and INTD19, [HPLC acidic], 507, (2.25) | 11.27 (s, 1H), 9.59 (d, J = 5.0 Hz, 2H), 9.07 (s, 1H), 8.61 (d, J = 5.3 Hz, 1H), 8.22 - 8.13 (m, 2H), 7.90 - 7.78 (m, 2H), 7.20 (d, J = 5.3 Hz, 1H), 3.25 - 3.15 (m, 1H), 1.60 (s, 6H), 1.06 - 0.98 (m, 2H), 0.85 - 0.75 (m, 2H). |
| **P65** | 2-(2-(cyclopropanesulfonamido)pyrimidin-4-yl)-N-(4-(6-ethoxypyrazin-2-yl)phenyl)-2-methylpropanamide 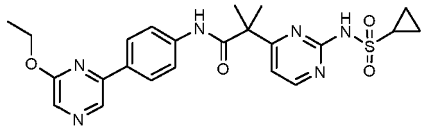 | Method 2, using INTC21 and INTD18, [HPLC acidic], 483, (2.19) | 11.27 (s, 1H), 9.50 (s, 1H), 8.76 (d, J = 0.5 Hz, 1H), 8.61 (d, J = 5.3 Hz, 1H), 8.18 (d, J = 0.5 Hz, 1H), 8.12 - 8.02 (m, 2H), 7.83 - 7.73 (m, 2H), 7.19 (d, J = 5.3 Hz, 1H), 4.48 (q, J = 7.0 Hz, 2H), 3.26 - 3.15 (m, 1H), 1.60 (s, 6H), 1.40 (t, J = 7.0 Hz, 3H), 1.08 - 1.00 (m, 2H), 0.87 - 0.75 (m, 2H). |
| **P66** | 2-(2-(cyclopropanesulfonamido)pyrimidin-4-yl)-N-(4-(6-cyclopropoxypyrazin-2-yl)phenyl)-2-methylpropanamide 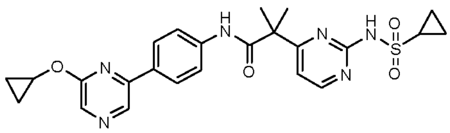 | Method 2, using INTC21 and INTD21, [UPLC acidic], 495, (1.44) | 11.32 (s, 1H), 9.53 (s, 1H), 8.84 (s, 1H), 8.60 (d, J = 5.3 Hz, 1H), 8.23 (s, 1H), 8.14 - 8.05 (m, 2H), 7.83 - 7.74 (m, 2H), 7.19 (d, J = 5.3 Hz, 1H), 4.47 - 4.33 (m, 1H), 3.27 - 3.12 (m, 1H), 1.60 (s, 6H), 1.09 - 0.96 (m, 2H), 0.93 - 0.69 (m, 6H). |
| **P67** | 2-(2-(cyclopropanesulfonamido)pyrimidin-4-yl)-N-(4-(6-isopropoxypyrazin-2-yl)phenyl)-2-methylpropanamide 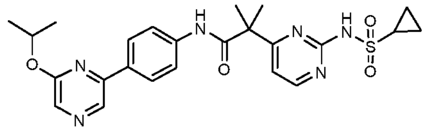 | Method 2, using INTC21 and INTD20, [UPLC acidic], 497, (1.53) | 11.34 (s, 1H), 9.52 (s, 1H), 8.73 (s, 1H), 8.60 (d, J = 5.2 Hz, 1H), 8.12 (s, 1H), 8.08 - 8.03 (m, 2H), 7.80 - 7.75 (m, 2H), 7.18 (d, J = 5.3 Hz, 1H), 5.47 - 5.35 (m, 1H), 3.25 - 3.12 (m, 1H), 1.60 (s, 6H), 1.38 (d, J = 6.2 Hz, 6H), 1.08 - 0.97 (m, 2H), 0.87 - 0.74 (m, 2H). |
| **P68** | 2-(2-(cyclopropanesulfonamido)-5-fluoropyrimidin-4-yl)-N-(4-(6-ethoxypyrazin-2-yl)phenyl)-2-methylpropanamide 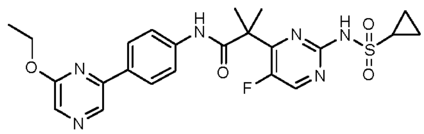 | Method 2, using INTC22 and INTD18, [UPLC Acidic], 501, (1.48) | 11.36 (s, 1H), 9.70 (s, 1H), 8.76 (s, 1H), 8.61 (s, 1H), 8.19 (s, 1H), 8.12 - 8.05 (m, 2H), 7.80 - 7.72 (m, 2H), 4.48 (q, J = 7.0 Hz, 2H), 3.27 - 3.16 (m, 1H), 1.61 (s, 6H), 1.40 (t, J = 7.0 Hz, 3H), 1.14 - 1.07 (m, 2H), 1.02 - 0.97 (m, 2H). |

(continued)

| P | Name/Structure (All examples containing chiral centres are racemates unless stated) | Synthesis Method, [LCMS Method], m/z $(M+H)^+$, (RT/Min) | $^1$H NMR Chemical Shift Data (DMSO-d6 unless stated) |
|---|---|---|---|
| **P69** | N-(4-(6-ethoxypyrazin-2-yl)phenyl)-2-methyl-2-(2-((1-methylcyclopropane)-1-sulfonamido) pyrimidin-4-yl)propanamide 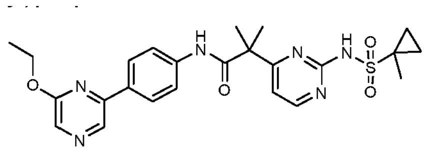 | Method 2, using INTC26 and INTD18, [UPLC Acidic], 497, (1.49) | 11.12 - 11.08 (m, 1H), 9.48 (s, 1H), 8.77 (s, 1H), 8.59 (d, J = 5.2 Hz, 1H), 8.18 (s, 1H), 8.10 - 8.04 (m, 2H), 7.79 - 7.74 (m, 2H), 7.16 (d, J = 5.2 Hz, 1H), 4.47 (q, J = 7.0 Hz, 2H), 1.58 (s, 6H), 1.50 - 1.46 (m, 2H), 1.43 - 1.36 (m, 6H), 0.84 - 0.80 (m, 2H). |
| **P70** | 2-(2-(cyclopropanesulfonamido)-5-methylpyrimidin-4-yl)-N-(4-(6-ethoxypyrazin-2-yl)phenyl)-2-methylpropanamide 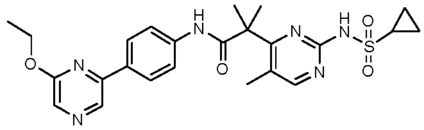 | Method 2, using INTC23 and INTD18; [UPLC Basic], 497, (1.27) | 11.07 (s, 1H), 9.69 (s, 1H), 8.76 (s, 1H), 8.34 (s, 1H), 8.18 (s, 1H), 8.10 - 8.05 (m, 2H), 7.79 - 7.74 (m, 2H), 4.47 (q, J = 7.1 Hz, 2H), 3.30 - 3.25 (m, 1H), 2.09 (s, 3H), 1.56 (s, 6H), 1.40 (t, J = 7.0 Hz, 3H), 1.14 - 1.09 (m, 2H), 1.06 - 1.01 (m, 2H). |
| **P71** | 2-(2-(cyclopropanesulfonamido)pyrimidin-4-yl)-2-methyl-N-(4-(pyrazin-2-yl)phenyl) propanamide 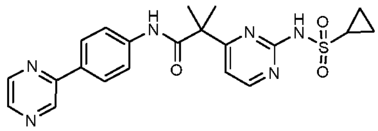 | Method 2, using INTC21 and INTD43, [HPLC acidic], 439, (1.72) | 11.26 (s, 1H), 9.50 (s, 1H), 9.22 (d, J = 1.5 Hz, 1H), 8.68 (dd, J = 2.5, 1.5 Hz, 1H), 8.61 (d, J = 5.3 Hz, 1H), 8.56 (d, J = 2.5 Hz, 1H), 8.15 - 8.04 (m, 2H), 7.85 - 7.74 (m, 2H), 7.20 (d, J = 5.3 Hz, 1H), 3.26 - 3.15 (m, 1H), 1.60 (s, 6H), 1.06 - 0.99 (m, 2H), 0.85 - 0.75 (m, 2H). |
| **P72** | N-(4-(6-ethoxypyrazin-2-yl)-2-fluorophenyl)-2-(2-(ethylsulfonamido)pyrimidin-4-yl)-2-methylpropanamide 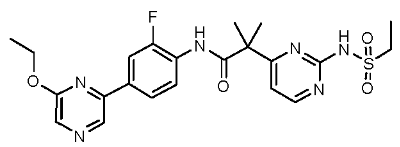 | Method 2 using INTC20 and INTD24, [UPLC Acidic], 489, (1.41) | 11.23 (s, 1H), 9.35 (s, 1H), 8.85 (s, 1H), 8.61 (d, J = 5.3 Hz, 1H), 8.25 (s, 1H), 8.03 - 7.93 (m, 2H), 7.70 (t, J = 8.1 Hz, 1H), 7.21 (d, J = 5.3 Hz, 1H), 4.49 (q, J = 7.0 Hz, 2H), 3.55 (q, J = 7.3 Hz, 2H), 1.60 (s, 6H), 1.40 (t, J = 7.0 Hz, 3H), 1.19 (t, J = 7.3 Hz, 3H). |
| **P73** | 2-(2-(ethylsulfonamido)pyrimidin-4-yl)-2-methyl-N-(4-(6-(trifluoromethyl)pyrazin-2-yl)phenyl) propanamide 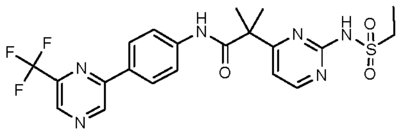 | Method 2, using INTC20 and INTD19, [UPLC Acidic], 495, (1.44) | 11.21 (s, 1H), 9.57 (d, J = 9.3 Hz, 2H), 9.07 (s, 1H), 8.61 (d, J = 5.3 Hz, 1H), 8.22 - 8.13 (m, 2H), 7.91 - 7.79 (m, 2H), 7.20 (d, J = 5.3 Hz, 1H), 3.49 (q, J = 7.3 Hz, 2H), 1.59 (s, 6H), 1.09 (t, J = 7.3 Hz, 3H). |

(continued)

| P | Name/Structure (All examples containing chiral centres are racemates unless stated) | Synthesis Method, [LCMS Method], m/z $(M+H)^+$, (RT/Min) | $^1H$ NMR Chemical Shift Data (DMSO-d6 unless stated) |
|---|---|---|---|
| P74 | N-(4-(6-ethoxypyrazin-2-yl)phenyl)-2-(2-(ethylsulfonamido)pyrimidin-4-yl)-2-methylpropanamide 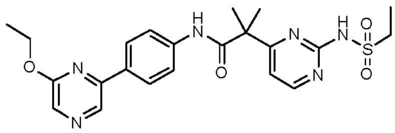 | Method 2, using INTC20 and INTD18, [UPLC Acidic], 471, (1.4) | 11.21 (s, 1H), 9.47 (s, 1H), 8.77 (s, 1H), 8.60 (d, J = 5.3 Hz, 1H), 8.18 (s, 1H), 8.11 - 8.04 (m, 2H), 7.82 - 7.73 (m, 2H), 7.19 (d, J = 5.3 Hz, 1H), 4.48 (q, J = 7.0 Hz, 2H), 3.50 (q, J = 7.3 Hz, 2H), 1.59 (s, 6H), 1.40 (t, J = 7.0 Hz, 3H), 1.10 (t, J = 7.3 Hz, 3H). |
| P75 | N-(5-(6-ethoxypyrazin-2-yl)-3-fluoropyridin-2-yl)-2-methyl-2-(2-(methylsulfonamido)pyrimidin-4-yl)propanamide 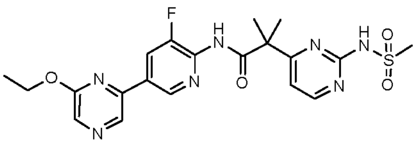 | Method 2, using INTC19 and INTD31, [HPLC Acidic], 476, (1.88) | 11.37 (s, 1H), 10.13 - 10.05 (m, 1H), 9.00 (s, 1H), 8.93 (d, J = 2.6 Hz, 1H), 8.62 (dd, J = 5.3, 2.3 Hz, 1H), 8.43 (dq, J = 10.9, 1.8 Hz, 1H), 8.35 - 8.30 (m, 1H), 7.17 (d, J = 5.1 Hz, 1H), 4.55-4.46 (m, 2H), 3.32 (s, 3H), 1.61 (d, J = 2.6 Hz, 6H), 1.45 - 1.37 (m, 3H) |
| P76 | N-(5-(6-ethoxypyrazin-2-yl)pyridin-2-yl)-2-methyl-2-(2-(methylsulfonamido)pyrimidin-4-yl) propanamide 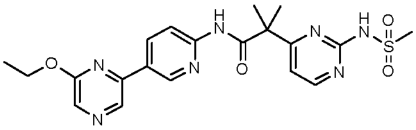 | Method 2, using INTC19 and INTD33, [HPLC Acidic], 458, (2.03) | 11.34 (s, 1H), 10.14 (s, 1H), 9.08 - 8.99 (m, 1H), 8.84 (s, 1H), 8.59 (d, J = 5.3 Hz, 1H), 8.49 (dd, J = 8.8, 2.5 Hz, 1H), 8.25 (s, 1H), 8.23 - 8.18 (m, 1H), 7.17 (d, J = 5.3 Hz, 1H), 4.48 (q, J = 7.0 Hz, 2H), 3.32 (s, 3H), 1.61 (s, 6H), 1.40 (t, J = 7.0 Hz, 3H) |
| P77 | N-(2-fluoro-4-(5-isopropoxypyridin-3-yl)phenyl)-2-methyl-2-(2-(methylsulfonamido)pyrimidin-4-yl)propanamide 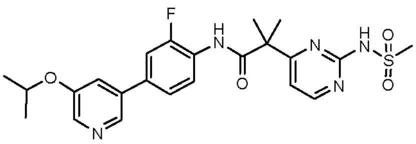 | Method 2, using INTC19 and INTD12, [HPLC Acidic], 488, (1.79) | 11.39 (s, 1H), 9.35 (s, 1H), 8.61 (d, J = 5.3 Hz, 1H), 8.49 (d, J = 1.9 Hz, 1H), 8.25 (d, J = 2.7 Hz, 1H), 7.72 - 7.68 (m, 1H), 7.66 (dd, J = 2.7, 1.9 Hz, 1H), 7.62 - 7.55 (m, 2H), 7.18 (d, J = 5.3 Hz, 1H), 4.91 - 4.82 (m, 1H), 3.37 (s, 3H), 1.60 (s, 6H), 1.32 (d, J = 6.0 Hz, 6H). |
| P78 | N-(2-fluoro-4-(6-isopropoxypyrazin-2-yl)phenyl)-2-methyl-2-(2-(methylsulfonamido)pyrimidin-4-yl)propanamide 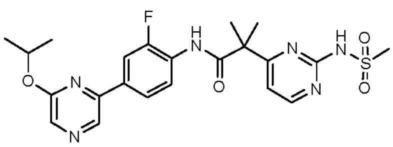 | Method 2, using INTC19 and INTD35, [HPLC Acidic], 489, (2.26) | 11.39 (s, 1H), 9.42 (s, 1H), 8.84 - 8.81 (m, 1H), 8.61 (d, J = 5.3 Hz, 1H), 8.19 (s, 1H), 8.01-7.94 (m, 2H), 7.67 (t, J = 8.1 Hz, 1H), 7.18 (d, J = 5.3 Hz, 1H), 5.45 - 5.39 (m, 1H), 3.36 (s, 3H), 1.61 (s, 6H), 1.39 (d, J = 6.2 Hz, 6H) |
| P79 | 2-methyl-N-(2-methyl-4-(6-(trifluoromethyl) pyrazin-2-yl)phenyl)-2-(2-(methylsulfonamido) pyrimidin-4-yl)propanamide 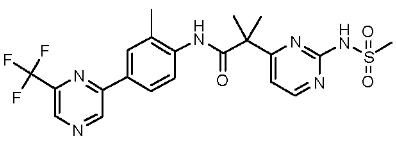 | Method 2 using INTC19 and INTD34; [HPLC Acidic], 495, (2.16) | 11.37 (s, 1H), 9.60 (s, 1H), 9.11 (d, J = 4.4 Hz, 2H), 8.63 (d, J = 5.3 Hz, 1H), 8.07 (d, J = 2.1 Hz, 1H), 8.02 (dd, J = 8.3, 2.2 Hz, 1H), 7.51 (d, J = 8.3 Hz, 1H), 7.23 (d, J = 5.3 Hz, 1H), 3.38 (s, 3H), 2.22 (s, 3H), 1.63 (s, 6H). |

(continued)

| P | Name/Structure (All examples containing chiral centres are racemates unless stated) | Synthesis Method, [LCMS Method], m/z $(M+H)^+$, (RT/Min) | $^1$H NMR Chemical Shift Data (DMSO-d6 unless stated) |
|---|---|---|---|
| **P80** | 2-methyl-2-(2-(methylsulfonamido)pyrimidin-4-yl)-N-(4-(6-(trifluoromethyl)pyrazin-2-yl)phenyl) propanamide 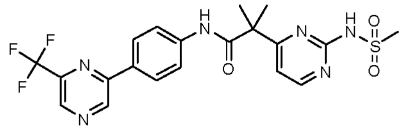 | Method 2, using INTC19 and INTD19, [HPLC Acidic], 481, (2.18) | 11.35 (s, 1H), 9.58 (s, 2H), 9.08 (s, 1H), 8.61 (d, J = 5.3 Hz, 1H), 8.24 - 8.12 (m, 2H), 7.93 - 7.77 (m, 2H), 7.18 (d, J = 5.3 Hz, 1H), 3.33 (s, 3H), 1.61 (s, 6H). |
| **P81** | N-(4-(6-ethoxypyrazin-2-yl)phenyl)-2-methyl-2-(2-(methylsulfonamido)pyrimidin-4-yl) propanamide 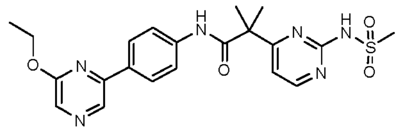 | Method 2, using INTC19 and INTD18, [HPLC Acidic], 457, (2.11) | 11.39 (s, 1H), 9.51 (s, 1H), 8.76 (s, 1H), 8.60 (d, J = 5.3 Hz, 1H), 8.18 (s, 1H), 8.11 - 8.04 (m, 2H), 7.82 - 7.70 (m, 2H), 7.16 (d, J = 5.3 Hz, 1H), 4.48 (q, J = 7.0 Hz, 2H), 3.32 (s, 3H), 1.60 (s, 6H), 1.40 (t, J = 7.1 Hz, 3H). |
| **P82** | 2-(2-((1,1-dimethylethyl)sulfonamido)pyrimidin-4-yl)-N-(4-(6-ethoxypyrazin-2-yl)phenyl)-2-methylpropanamide 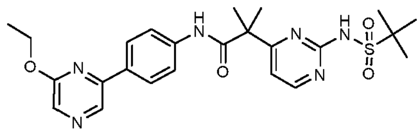 | Method 2, using INTC25 and INTD18, [UPLC Acidic], 499, (1.56) | 10.85 (s, 1H), 9.52 (s, 1H), 8.77 (s, 1H), 8.61 - 8.56 (m, 1H), 8.18 (s, 1H), 8.11 - 8.05 (m, 2H), 7.81 - 7.74 (m, 2H), 7.17 (s, 1H), 4.48 (q, J = 7.0 Hz, 2H), 1.58 (s, 6H), 1.43 - 1.36 (m, 12H). |
| **P83** | 1-(2-(cyclopropanesulfonamido)pyrimidin-4-yl)-N-(4-(6-ethoxypyrazin-2-yl)phenyl) cyclopropanecarboxamide 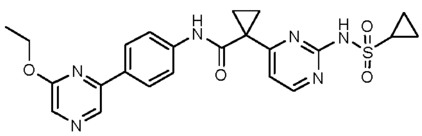 | Method 2, using INTC28 and INTD18, [UPLC acidic], 481, (1.39) | 11.45 (s, 1H), 10.70 (s, 1H), 8.78 (s, 1H), 8.44 (d, J = 5.3 Hz, 1H), 8.19 (s, 1H), 8.13 - 8.07 (m, 2H), 7.87 - 7.80 (m, 2H), 6.92 (s, 1H), 4.48 (q, J = 7.1 Hz, 2H), 3.11 (s, 1H), 1.66 - 1.59 (m, 2H), 1.53 - 1.46 (m, 2H), 1.40 (t, J = 7.1 Hz, 3H), 1.07 - 0.99 (m, 2H), 0.95 - 0.85 (m, 2H). |
| **P84** | 2-(2-(cyclopropanesulfonamido)pyrimidin-4-yl)-N-(5'-(trifluoromethyl)-[3,3'-bipyridin]-6-yl) butanamide 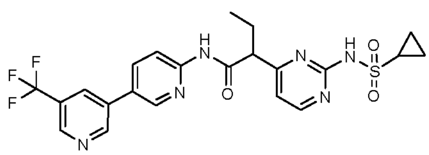 | Method 3 using INTC37 and INTD44, [UPLC Acidic], 507, (1.36) | 11.24 (s, 1H), 11.04 (s, 1H), 9.27 (d, J = 2.2 Hz, 1H), 8.98 (dd, J = 2.2, 1.0 Hz, 1H), 8.86 (dd, J = 2.6, 0.8 Hz, 1H), 8.59 - 8.54 (m, 2H), 8.33 (dd, J = 8.7, 2.6 Hz, 1H), 8.21 (d, J = 8.7 Hz, 1H), 7.21 (d, J = 5.2 Hz, 1H), 4.00 (dd, J = 8.6, 6.3 Hz, 1H), 3.32 - 3.28 (m, 1H), 2.11 - 2.04 (m, 1H), 2.00 - 1.90 (m, 1H), 1.16 - 1.06 (m, 2H), 1.00 - 0.88 (m, 5H). |

(continued)

| P | Name/Structure (All examples containing chiral centres are racemates unless stated) | Synthesis Method, [LCMS Method], m/z $(M+H)^+$, (RT/Min) | $^1H$ NMR Chemical Shift Data (DMSO-d6 unless stated) |
|---|---|---|---|
| **P85** | 2-(2-(cyclopropanesulfonamido)pyrimidin-4-yl)-N-(5'-(2,2,2-trifluoroethoxy)-[3,3'-bipyridin]-6-yl)butanamide 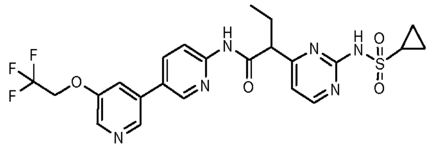 | Method 3 using INTC37 and INTD17, [UPLC Acidic], 537, (1.3) | 11.23 (s, 1H), 11.00 (s, 1H), 8.79 (d, J = 2.5 Hz, 1H), 8.66 (d, J = 1.8 Hz, 1H), 8.56 (d, J = 5.2 Hz, 1H), 8.41 (d, J = 2.8 Hz, 1H), 8.24 (dd, J = 8.7, 2.5 Hz, 1H), 8.19 (d, J = 8.7 Hz, 1H), 7.92 - 7.89 (m, 1H), 7.21 (d, J = 5.2 Hz, 1H), 4.99 (q, J = 8.8 Hz, 2H), 4.00 (dd, J = 7.5 Hz, 1H), 2.12 - 2.04 (m, 1H), 1.98 - 1.90 (m, 1H), 1.12 - 1.08 (m, 2H), 0.98 - 0.90 (m, 5H), 1H obscured by $H_2O$. |
| **P86** | N-([3,3'-bipyridin]-6-yl)-2-(2-(cyclopropanesulfonamido)pyrimidin-4-yl) butanamide 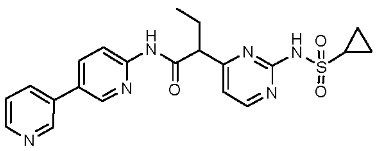 | Method 3 using INTC37 and a commercial aniline; [UPLC Acidic], 439, (0.81) | 11.25 (s, 1H), 10.96 (s, 1H), 8.94 (d, J = 2.4 Hz, 1H), 8.73 (dd, J = 1.7 Hz, 1H), 8.59 (dd, J = 4.7, 1.6 Hz, 1H), 8.56 (d, J = 5.1 Hz, 1H), 8.19 (d, J = 1.7 Hz, 2H), 8.14 (ddd, J = 8.0, 1.8 Hz, 1H), 7.51 (dd, J = 7.9, 4.8 Hz, 1H), 7.21 (d, J = 5.2 Hz, 1H), 4.04 - 3.96 (m, 1H), 3.31 - 3.28 (m, 1H), 2.13 - 2.02 (m, 1H), 1.99 - 1.89 (m, 1H), 1.17 - 1.02 (m, 2H), 0.99 - 0.86 (m, 5H). |
| **P87** | 2-(2-(cyclopropanesulfonamido)pyrimidin-4-yl)-N-(5-(6-(trifluoromethyl)pyrazin-2-yl)pyridin-2-yl)butanamide 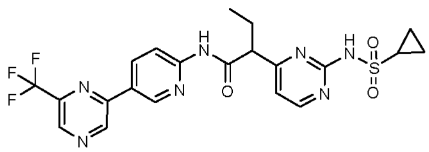 | Method 3 using INTC37 and INTD2, [UPLC Acidic], 508, (1.41) | 11.24 (s, 1H), 11.16 (s, 1H), 9.66 (s, 1H), 9.16 (dd, J = 2.5, 0.8 Hz, 2H), 8.61 - 8.53 (m, 2H), 8.31 - 8.24 (m, 1H), 7.22 (d, J = 5.2 Hz, 1H), 4.06 - 3.97 (m, 1H), 3.32 - 3.26 (m, 1H), 2.15 - 2.02 (m, 1H), 2.01 - 1.88 (m, 1H), 1.17 - 1.03 (m, 2H), 1.03 - 0.85 (m, 5H). |
| **P88** | 2-(2-(cyclopropanesulfonamido)pyrimidin-4-yl)-N-(5-(6-ethoxypyrazin-2-yl)pyridin-2-yl) butanamide 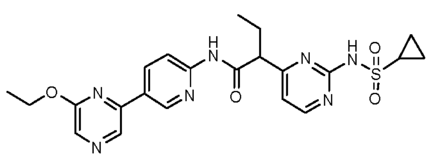 | Method 3 using INTC37 and INTD33, [UPLC Acidic], 484, (1.38) | 11.24 (s, 1H), 11.04 (s, 1H), 9.11 - 9.02 (m, 1H), 8.84 (s, 1H), 8.56 (d, J = 5.2 Hz, 1H), 8.53 - 8.46 (m, 1H), 8.25 (s, 1H), 8.21 (d, J = 8.8 Hz, 1H), 7.21 (d, J = 5.2 Hz, 1H), 4.48 (q, J = 7.0 Hz, 2H), 4.06 - 3.96 (m, 1H), 3.32 - 3.26 (m, 1H), 2.15 - 2.01 (m, 1H), 2.00 - 1.88 (m, 1H), 1.40 (t, J = 7.0 Hz, 3H), 1.17 - 1.03 (m, 2H), 1.03 - 0.87 (m, 5H). |

(continued)

| P | Name/Structure (All examples containing chiral centres are racemates unless stated) | Synthesis Method, [LCMS Method], m/z $(M+H)^+$, (RT/Min) | $^1$H NMR Chemical Shift Data (DMSO-d6 unless stated) |
|---|---|---|---|
| **P89** | 2-(2-(cyclopropanesulfonamido)pyrimidin-4-yl)-N-(5-(6-isopropoxypyrazin-2-yl)pyridin-2-yl) butanamide 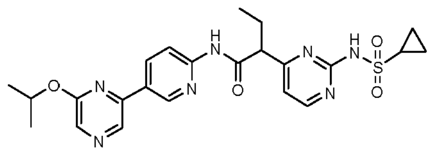 | Method 3 using INTC37 and INTD32, [UPLC Acidic], 498, (1.48) | 11.23 (s, 1H), 11.03 (s, 1H), 9.05 (dd, J = 2.5, 0.8 Hz, 1H), 8.82 (s, 1H), 8.56 (d, J = 5.2 Hz, 1H), 8.48 (dd, J = 8.8, 2.5 Hz, 1H), 8.25 - 8.17 (m, 2H), 7.21 (d, J = 5.2 Hz, 1H), 5.41 (hept, J = 6.1 Hz, 1H), 4.01 (dd, J = 8.6, 6.4 Hz, 1H), 3.32 - 3.28 (m, 1H), 2.13 - 2.03 (m, 1H), 2.01 - 1.89 (m, 1H), 1.38 (d, J = 6.2 Hz, 6H), 1.15 - 1.05 (m, 2H), 1.01 - 0.88 (m, 5H). |
| **P90** | N-(4-(5-chloropyridin-3-yl)-2-fluorophenyl)-2-(2-(cyclopropanesulfonamido)pyrimidin-4-yl) butanamide 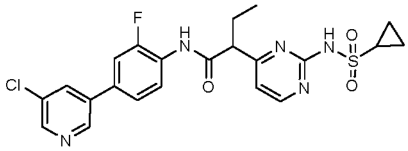 | Method 3 using INTC37 and INTD13, [UPLC Acidic], 490, (1.37) | 11.27 (s, 1H), 10.21 (s, 1H), 8.91 (d, J = 2.0 Hz, 1H), 8.63 (d, J = 2.3 Hz, 1H), 8.56 (d, J = 5.2 Hz, 1H), 8.31 (dd, J = 2.3 Hz, 1H), 8.00 (dd, J = 8.3 Hz, 1H), 7.81 (dd, J = 12.2, 2.1 Hz, 1H), 7.65 (dd, J = 8.4, 2.1 Hz, 1H), 7.19 (d, J = 5.2 Hz, 1H), 3.97 (dd, J = 8.7, 6.3 Hz, 1H), 3.31 - 3.27 (m, 1H), 2.11 - 2.01 (m, 1H), 1.99 - 1.91 (m, 1H), 1.16 - 1.08 (m, 2H), 1.05 - 0.91 (m, 5H). |
| **P91** | 2-(2-(cyclopropanesulfonamido)pyrimidin-4-yl)-N-(2-fluoro-4-(5-(2,2,2-trifluoroethoxy) pyridin-3-yl)phenyl)butanamide 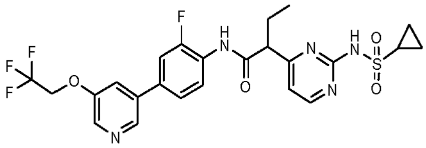 | Method 3 using INTC37 and INTD14; [UPLC Acidic], 554, (1.38) | 11.27 (s, 1H), 10.20 (s, 1H), 8.65 (d, J = 1.8 Hz, 1H), 8.57 (d, J = 5.2 Hz, 1H), 8.39 (d, J = 2.8 Hz, 1H), 7.99 (dd, J = 8.3 Hz, 1H), 7.87 (dd, J = 2.3 Hz, 1H), 7.80 (dd, J = 12.2, 2.1 Hz, 1H), 7.64 (dd, J = 8.5, 2.0 Hz, 1H), 7.20 (d, J = 5.2 Hz, 1H), 4.99 (q, J = 8.9 Hz, 2H), 3.97 (dd, J = 8.8, 6.2 Hz, 1H), 2.10 - 2.01 (m, 1H), 1.99 - 1.89 (m, 1H), 1.15 - 1.08 (m, 2H), 1.03 - 0.92 (m, 5H), 1H obscured by $H_2O$. |
| **P92** | 2-(2-(cyclopropanesulfonamido)pyrimidin-4-yl)-N-(2-fluoro-4-(5-isopropoxypyridin-3-yl) phenyl)butanamide 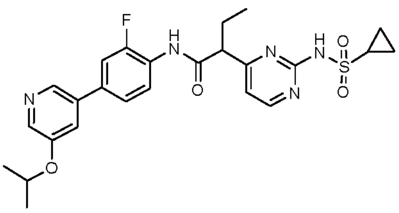 | Method 3 using INTC37 and INTD12, [UPLC Acidic], 514, (1.22) | 11.25 (s, 1H), 10.17 (s, 1H), 8.57 (d, J = 5.1 Hz, 1H), 8.49 (d, J = 1.9 Hz, 1H), 8.25 (d, J = 2.7 Hz, 1H), 7.96 (dd, J = 8.3 Hz, 1H), 7.74 (dd, J = 12.2, 2.1 Hz, 1H), 7.69 - 7.62 (m, 1H), 7.59 (dd, J = 8.4, 2.0 Hz, 1H), 7.20 (s, 1H), 4.92 - 4.82 (m, 1H), 4.01 - 3.93 (m, 1H), 3.31 - 3.27 (m, 1H), 2.13 - 2.01 (m, 1H), 1.99 - 1.90 (m, 1H), 1.32 (d, J = 6.0 Hz, 6H), 1.17 - 1.09 (m, 2H), 1.05 - 0.92 (m, 5H). |

(continued)

| P | Name/Structure (All examples containing chiral centres are racemates unless stated) | Synthesis Method, [LCMS Method], m/z $(M+H)^+$, (RT/Min) | $^1H$ NMR Chemical Shift Data (DMSO-d6 unless stated) |
|---|---|---|---|
| **P93** | 2-(2-(cyclopropanesulfonamido)pyrimidin-4-yl)-N-(2-fluoro-4-(pyridin-3-yl)phenyl) butanamide 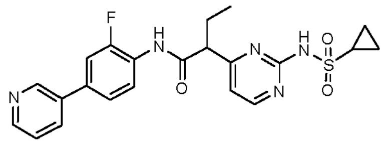 | Method 3 using INTC37 and INTD50; [UPLC Acidic], 456, (0.85) | 11.27 (s, 1H), 10.17 (s, 1H), 8.93 (d, J = 2.4 Hz, 1H), 8.62 - 8.54 (m, 2H), 8.11 (dt, J = 8.1, 1.9 Hz, 1H), 7.98 (dd, J = 8.3 Hz, 1H), 7.72 (dd, J = 12.2, 2.1 Hz, 1H), 7.58 (dd, J = 8.4, 2.1 Hz, 1H), 7.52 - 7.45 (m, 1H), 7.20 (d, J = 5.2 Hz, 1H), 4.00 - 3.93 (m, 1H), 2.12 - 2.01 (m, 1H), 2.00 - 1.91 (m, 1H), 1.16 - 1.08 (m, 2H), 1.03 - 0.91 (m, 5H), 1H obscured by $H_2O$. |
| **P94** | 2-(2-(cyclopropanesulfonamido)pyrimidin-4-yl)-N-(2-fluoro-4-(6-(trifluoromethyl)pyrazin-2-yl) phenyl)butanamide 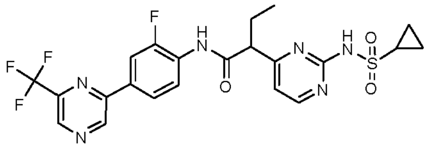 | Method 3 using INTC37 and INTD25, [UPLC Acidic], 525, (1.49) | 11.25 (s, 1H), 10.32 (s, 1H), 9.64 (s, 1H), 9.15 (s, 1H), 8.57 (d, J = 5.2 Hz, 1H), 8.21 - 8.09 (m, 2H), 8.06 (dd, J = 8.6, 2.0 Hz, 1H), 7.21 (d, J = 5.3 Hz, 1H), 4.02 (t, J = 7.5 Hz, 1H), 3.32 - 3.26 (m, 1H), 2.12 - 2.02 (m, 1H), 2.01 - 1.88 (m, 1H), 1.17 - 1.05 (m, 2H), 1.05 - 0.92 (m, 5H). |
| **P95** | 2-(2-(cyclopropanesulfonamido)pyrimidin-4-yl)-N-(2-fluoro-4-(6-methoxypyrazin-2-yl)phenyl) butanamide 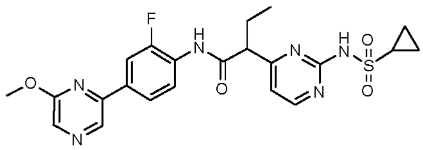 | Method 3 using INTC37 and INTD46, [UPLC Acidic], 487, (1.35) | 11.25 (s, 1H), 10.24 (s, 1H), 8.85 (s, 1H), 8.57 (d, J = 5.2 Hz, 1H), 8.28 (s, 1H), 8.09 - 8.03 (m, 2H), 8.00 - 7.97 (m, 1H), 7.20 (d, J = 4.7 Hz, 1H), 4.03 (s, 3H), 3.99 (dd, J = 10.2, 5.0 Hz, 1H), 3.31 - 3.27 (m, 1H), 2.12 - 2.02 (m, 1H), 2.00 - 1.91 (m, 1H), 1.16 - 1.08 (m, 2H), 1.02 - 0.93 (m, 5H). |
| **P96** | 2-(2-(cyclopropanesulfonamido)pyrimidin-4-yl)-N-(4-(6-ethoxypyrazin-2-yl)-2-fluorophenyl) butanamide 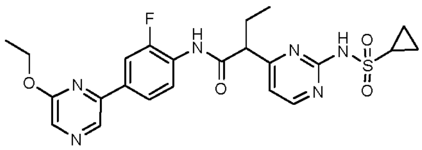 | Method 3 using INTC37 and INTD24, [UPLC acidic], 501, (1.45) | 11.27 (s, 1H), 10.24 (s, 1H), 8.83 (s, 1H), 8.56 (d, J = 5.2 Hz, 1H), 8.24 (s, 1H), 8.09 - 8.00 (m, 2H), 7.99 - 7.94 (m, 1H), 7.19 (d, J = 5.2 Hz, 1H), 4.49 (q, J = 7.1 Hz, 2H), 4.05 - 3.92 (m, 1H), 3.33 - 3.28 (m, 1H), 2.12 - 2.01 (m, 1H), 2.00 - 1.89 (m, 1H), 1.40 (t, J = 7.0 Hz, 3H), 1.17 - 1.06 (m, 2H), 1.05 - 0.89 (m, 5H). |
| **P97** | 2-(2-(cyclopropanesulfonamido)pyrimidin-4-yl)-N-(2-fluoro-4-(6-isopropoxypyrazin-2-yl) phenyl)butanamide 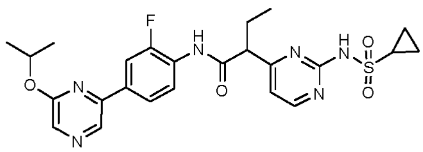 | Method 3 using INTC37 and INTD35, [UPLC Acidic], 515, (1.55) | 11.25 (s, 1H), 10.24 (s, 1H), 8.81 (s, 1H), 8.57 (d, J = 5.2 Hz, 1H), 8.19 (s, 1H), 8.06 (d, J = 8.3 Hz, 1H), 8.00 (dd, J = 12.2, 1.9 Hz, 1H), 7.95 (dd, J = 8.5, 2.0 Hz, 1H), 7.20 (d, J = 5.1 Hz, 1H), 5.45 - 5.39 (m, 1H), 4.00 (t, J = 7.7 Hz, 1H), 3.31 - 3.27 (m, 1H), 2.13 - 2.02 (m, 1H), 1.99 - 1.90 (m, 1H), 1.39 (d, J = 6.2 Hz, 6H), 1.16 - 1.08 (m, 2H), 1.03 - 0.93 (m, 5H). |

(continued)

| P | Name/Structure (All examples containing chiral centres are racemates unless stated) | Synthesis Method, [LCMS Method], m/z $(M+H)^+$, (RT/Min) | $^1H$ NMR Chemical Shift Data (DMSO-d6 unless stated) |
|---|---|---|---|
| P98 | 2-(2-(cyclopropanesulfonamido)pyrimidin-4-yl)-N-(2-fluoro-4-(6-(2,2,2-trifluoroethoxy) pyrazin-2-yl)phenyl)butanamide 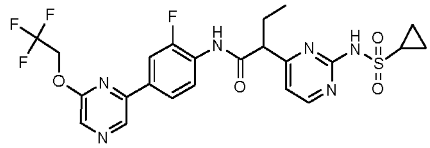 | Method 3 using INTC37 and INTD28, [UPLC Acidic], 555, (1.49) | 11.27 (s, 1H), 10.28 (s, 1H), 9.00 (s, 1H), 8.57 (d, J = 5.2 Hz, 1H), 8.44 (s, 1H), 8.15 (dd, J = 12.3, 1.9 Hz, 1H), 8.10 (dd, J = 8.1 Hz, 1H), 8.04 (dd, J = 8.5, 1.9 Hz, 1H), 7.21 (d, J = 5.2 Hz, 1H), 5.22 (q, J = 9.0 Hz, 2H), 4.01 (dd, J = 8.6, 6.2 Hz, 1H), 2.12 - 2.02 (m, 1H), 1.99 - 1.90 (m, 1H), 1.17 - 1.06 (m, 2H), 1.03 - 0.90 (m, 5H), 1H obscured by $H_2O$. |
| P99 | N-(4-(5-cyanopyridin-3-yl)phenyl)-2-(2-(cyclopropanesulfonamido)pyrimidin-4-yl) butanamide 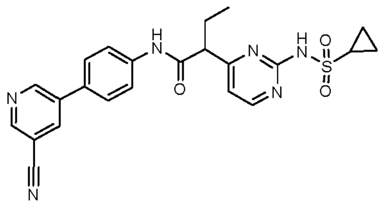 | Method 3 using INTC37 and INTD5, [UPLC Acidic], 463, (1.23) | 11.24 (s, 1H), 10.41 (s, 1H), 9.18 (d, J = 2.3 Hz, 1H), 8.97 (d, J = 1.9 Hz, 1H), 8.63 (dd, J = 2.1 Hz, 1H), 8.57 (d, J = 5.2 Hz, 1H), 7.83 - 7.80 (m, 2H), 7.78 - 7.75 (m, 2H), 7.21 (d, J = 5.2 Hz, 1H), 3.77 (dd, J = 8.7, 6.3 Hz, 1H), 3.31 - 3.26 (m, 1H), 2.13 - 2.02 (m, 1H), 1.98 - 1.89 (m, 1H), 1.13 - 1.07 (m, 2H), 1.00 - 0.90 (m, 5H). |
| P100 | 2-(2-(cyclopropanesulfonamido)pyrimidin-4-yl)-N-(4-(5-(2,2,2-trifluoroethoxy)pyridin-3-yl) phenyl)butanamide 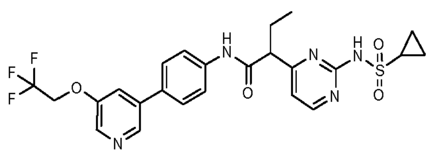 | Method 3 using INTC37 and INTD15, [UPLC Acidic], 536, (1.32) | 11.27 (s, 1H), 10.39 (s, 1H), 8.61 - 8.53 (m, 2H), 8.36 (d, J = 2.8 Hz, 1H), 7.82 - 7.70 (m, 5H), 7.21 (d, J = 5.2 Hz, 1H), 4.98 (q, J = 8.8 Hz, 2H), 3.76 (dd, J = 8.7, 6.3 Hz, 1H), 3.31 - 3.26 (m, 1H), 2.11 - 2.02 (m, 1H), 1.98 - 1.90 (m, 1H), 1.13 - 1.05 (m, 2H), 1.00 - 0.88 (m, 5H). |
| P101 | 2-(2-(cyclopropanesulfonamido)pyrimidin-4-yl)-N-(4-(5-isopropoxypyridin-3-yl)phenyl) butanamide 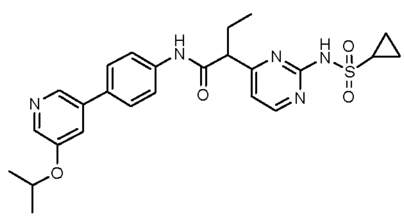 | Method 3 using INTC37 and INTD11, [UPLC Acidic], 496, (1.13) | 11.26 (s, 1H), 10.36 (s, 1H), 8.57 (d, J = 5.2 Hz, 1H), 8.44 (d, J = 1.9 Hz, 1H), 8.21 (d, J = 2.7 Hz, 1H), 7.75 - 7.69 (m, 4H), 7.58 - 7.55 (m, 1H), 7.20 (d, J = 5.2 Hz, 1H), 4.84 (hept, J = 6.0 Hz, 1H), 3.76 (dd, J = 8.8, 6.3 Hz, 1H), 3.33 - 3.27 (m, 1H), 2.13 - 2.02 (m, 1H), 1.99 - 1.90 (m, 1H), 1.32 (d, J = 6.0 Hz, 6H), 1.14 - 1.05 (m, 2H), 1.01 - 0.88 (m, 5H). |
| P102 | 2-(2-(cyclopropanesulfonamido)pyrimidin-4-yl)-N-(4-(pyridin-3-yl)phenyl)butanamide 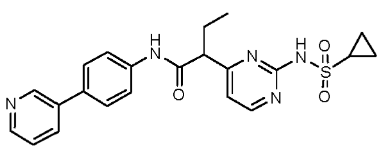 | Method 2, using INTC35 and a commercial aniline, [HPLC acidic], 438, (1.31) | 11.27 (s, 1H), 10.36 (s, 1H), 8.94 - 8.80 (m, 1H), 8.61 - 8.47 (m, 2H), 8.09 - 8.01 (m, 1H), 7.78 - 7.61 (m, 4H), 7.50 - 7.42 (m, 1H), 7.20 (d, J = 5.2 Hz, 1H), 3.79 - 3.72 (m, 1H), 3.31 - 3.25 (m, 1H), 2.14 - 2.01 (m, 1H), 2.00 - 1.87 (m, 1H), 1.15 - 1.04 (m, 2H), 1.02 - 0.86 (m, 5H). |

(continued)

| P | Name/Structure (All examples containing chiral centres are racemates unless stated) | Synthesis Method, [LCMS Method], m/z (M+H)$^+$, (RT/Min) | $^1$H NMR Chemical Shift Data (DMSO-d6 unless stated) |
|---|---|---|---|
| **P103** | 2-(2-(cyclopropanesulfonamido)pyrimidin-4-yl)-N-(4-(6-(trifluoromethyl)pyrazin-2-yl)phenyl) butanamide 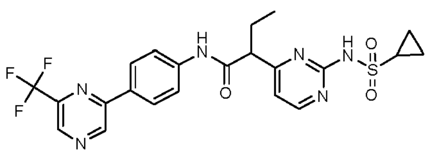 | Method 1 using INTC37 and INTD19, [UPLC Acidic], 507, (1.47) | 11.27 (s, 1H), 10.52 (s, 1H), 9.58 (s, 1H), 9.08 (s, 1H), 8.57 (d, J = 5.2 Hz, 1H), 8.23 - 8.15 (m, 2H), 7.86 - 7.78 (m, 2H), 7.20 (d, J = 5.2 Hz, 1H), 3.85 - 3.72 (m, 1H), 3.32 - 3.24 (m, 1H), 2.14 - 2.01 (m, 1H), 2.00 - 1.90 (m, 1H), 1.14 - 1.03 (m, 2H), 1.02 - 0.85 (m, 5H). |
| **P104** | N-(4-(6-chloropyrazin-2-yl)phenyl)-2-(2-(cyclopropanesulfonamido)pyrimidin-4-yl) butanamide 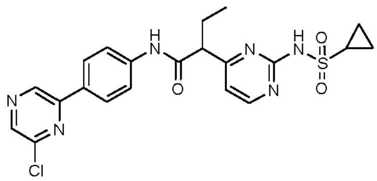 | Method 3 using INTC37 and INTD22, [UPLC Acidic], 473, (1.4) | 11.25 (s, 1H), 10.49 (s, 1H), 9.24 (s, 1H), 8.69 (s, 1H), 8.57 (d, J = 5.2 Hz, 1H), 8.15 - 8.09 (m, 2H), 7.83 - 7.75 (m, 2H), 7.20 (d, J = 5.2 Hz, 1H), 3.78 (dd, J = 8.6, 6.3 Hz, 1H), 3.30 - 3.26 (m, 1H), 2.12 - 2.03 (m, 1H), 1.96 - 1.92 (m, 1H), 1.13 - 1.05 (m, 2H), 1.01 - 0.88 (m, 5H). |
| **P105** | 2-(2-(cyclopropanesulfonamido)pyrimidin-4-yl)-N-(4-(6-ethoxypyrazin-2-yl)phenyl) butanamide 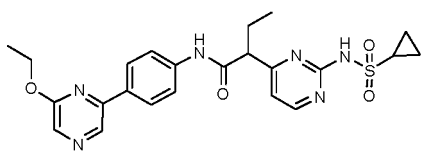 | Method 1 using INTC37 and INTD18, or Method 5 using INTC46 [UPLC acidic], 483, (1.43) | 11.28 (s, 1H), 10.43 (s, 1H), 8.76 (s, 1H), 8.56 (d, J = 5.2 Hz, 1H), 8.18 (s, 1H), 8.15 - 8.04 (m, 2H), 7.80 - 7.71 (m, 2H), 7.19 (d, J = 5.2 Hz, 1H), 4.48 (q, J = 7.0 Hz, 2H), 3.81 - 3.73 (m, 1H), 3.32 - 3.26 (m, 1H), 2.13 - 2.01 (m, 1H), 2.01 - 1.87 (m, 1H), 1.40 (t, J = 7.0 Hz, 3H), 1.13-1.04 (m, 2H), 1.03 - 0.85 (m, 5H). |
| **P106** | 2-(2-(cyclopropanesulfonamido)pyrimidin-4-yl)-N-(4-(6-methoxypyrazin-2-yl)phenyl) butanamide 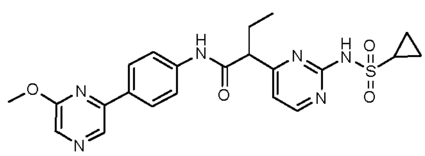 | Method 3 using INTC37 and INTD1, [UPLC Acidic], 469, (1.33) | 11.25 (s, 1H), 10.44 (s, 1H), 8.78 (s, 1H), 8.57 (d, J = 5.2 Hz, 1H), 8.22 (s, 1H), 8.15 - 8.10 (m, 2H), 7.80 - 7.73 (m, 2H), 7.21 (d, J = 5.3 Hz, 1H), 4.02 (s, 3H), 3.78 (dd, J = 8.7, 6.3 Hz, 1H), 3.32 - 3.27 (m, 1H), 2.13 - 2.03 (m, 1H), 1.99 - 1.90 (m, 1H), 1.14 - 1.06 (m, 2H), 1.00 - 0.89 (m, 5H). |
| **P107** | 2-(2-(cyclopropanesulfonamido)pyrimidin-4-yl)-N-(4-(6-isopropoxypyrazin-2-yl)phenyl) butanamide 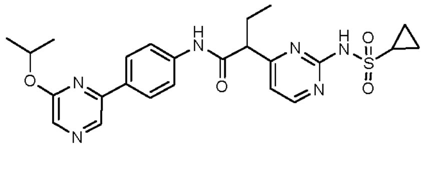 | Method 3 using INTC37 and INTD20, [UPLC Acidic], 497, (1.53) | 11.25 (s, 1H), 10.44 (s, 1H), 8.74 (s, 1H), 8.57 (d, J = 5.2 Hz, 1H), 8.13 (s, 1H), 8.11 - 8.05 (m, 2H), 7.79 - 7.73 (m, 2H), 7.21 (d, J = 5.2 Hz, 1H), 5.41 (hept, J = 6.1 Hz, 1H), 3.78 (dd, J = 8.7, 6.4 Hz, 1H), 3.32 - 3.26 (m, 1H), 2.12 - 2.03 (m, 1H), 1.99 - 1.90 (m, 1H), 1.38 (d, J = 6.2 Hz, 6H), 1.14 - 1.06 (m, 2H), 1.00 - 0.87 (m, 5H). |

(continued)

| P | Name/Structure (All examples containing chiral centres are racemates unless stated) | Synthesis Method, [LCMS Method], m/z $(M+H)^+$, (RT/Min) | $^1$H NMR Chemical Shift Data (DMSO-d6 unless stated) |
|---|---|---|---|
| **P108** | 2-(2-(cyclopropanesulfonamido)pyrimidin-4-yl)-N-(4-(6-(2,2,2-trifluoroethoxy)pyrazin-2-yl) phenyl)butanamide 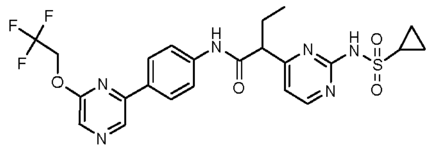 | Method 3 using INTC37 and INTD29, [UPLC Acidic], 537, (1.49) | 11.25 (s, 1H), 10.46 (s, 1H), 8.93 (s, 1H), 8.57 (d, J = 5.2 Hz, 1H), 8.38 (s, 1H), 8.20 - 8.14 (m, 2H), 7.81 - 7.76 (m, 2H), 7.21 (d, J = 5.3 Hz, 1H), 5.19 (q, J = 9.0 Hz, 2H), 3.78 (dd, J = 8.5, 6.4 Hz, 1H), 2.13 - 2.03 (m, 1H), 2.01 - 1.90 (m, 1H), 1.14 - 1.07 (m, 2H), 0.99 - 0.89 (m, 5H), 1H obscured by $H_2O$. |
| **P109** | 2-(2-(cyclopropanesulfonamido)pyrimidin-4-yl)-N-(4-(pyrazin-2-yl)phenyl)butanamide 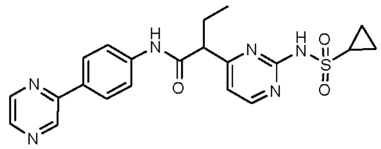 | Method 3 using INTC37 and INTD43, [UPLC Basic], 439, (0.87) | 11.25 (s, 1H), 10.44 (s, 1H), 9.22 (d, J = 1.6 Hz, 1H), 8.68 (dd, J = 2.5, 1.5 Hz, 1H), 8.60 - 8.53 (m, 2H), 8.15 - 8.10 (m, 2H), 7.82 - 7.75 (m, 2H), 7.20 (d, J = 5.2 Hz, 1H), 3.78 (dd, J = 8.7, 6.3 Hz, 1H), 3.31 - 3.26 (m, 1H), 2.13 - 2.02 (m, 1H), 2.00 - 1.89 (m, 1H), 1.12 - 1.04 (m, 2H), 1.01 - 0.88 (m, 5H). |
| **P110** | 2-(2-(cyclopropanesulfonamido)pyrimidin-4-yl)-N-(4-(6-ethoxypyrazin-2-yl)phenyl)-4-methoxybutanamide 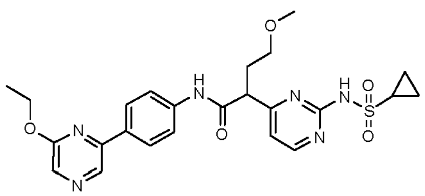 | Method 3 using INTC38 and INTD18, [UPLC Acidic], 513, (1.34) | 11.28 (s, 1H), 10.47 (s, 1H), 8.76 (s, 1H), 8.56 (d, J = 5.2 Hz, 1H), 8.18 (s, 1H), 8.11 - 8.06 (m, 2H), 7.78 - 7.67 (m, 2H), 7.20 (d, J = 5.2 Hz, 1H), 4.47 (q, J = 7.1 Hz, 2H), 4.06 - 3.95 (m, 1H), 3.41 - 3.35 (m, 3H), 3.23 (s, 3H), 2.33 - 2.25 (m, 1H), 2.20 - 2.11 (m, 1H), 1.40 (t, J = 7.0 Hz, 3H), 1.18 - 1.03 (m, 2H), 1.01 -0.88 (m, 2H). |
| **P111** | 2-(2-(cyclopropanesulfonamido)pyrimidin-4-yl)-N-(4-(pyridin-3-yl)phenyl)propanamide 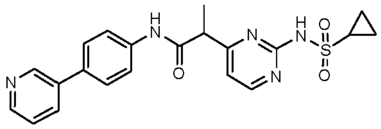 | Method 2, using INTC18 and a commercial aniline, [UPLC acidic], 424, (0.70) | (Methanol-d4) 8.81 (s, 1H), 8.59 - 8.46 (m, 2H), 8.21 - 8.02 (m, 2H), 7.81 - 7.73 (m, 2H), 7.70 - 7.61 (m, 2H), 7.56 - 7.48 (m, 1H), 7.17 (d, J = 5.2 Hz, 1H), 4.08 - 3.92 (m, 1H), 3.32 - 3.25 (m, 1H), 1.63 (d, J = 6.9 Hz, 3H), 1.29 - 1.18 (m, 2H), 1.03 - 0.88 (m, 2H), 1 exchangeable proton not observed. |
| **P112** | 2-(2-(Cyclopropanesulfonamido)pyrimidin-4-yl)-N-(5-(6-ethoxypyrazin-2-yl)pyridin-2-yl)-2-fluorobutanamide 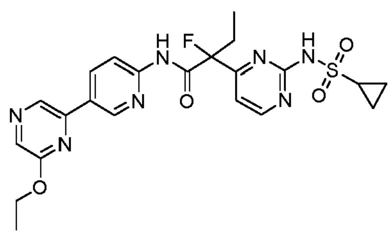 | Method 4 using INTC50 and INTD33 then Method 6 using INTC51 [UPLC acidic], 502 (2.28). | 11.50 (s, 1H), 10.60 (d, J = 2.3 Hz, 1H), 9.10 (d, J = 2.4 Hz, 1H), 8.87 (s, 1H), 8.76 (d, J = 5.2 Hz, 1H), 8.53 (dd, J = 8.7, 2.5 Hz, 1H), 8.27 (s, 1H), 8.10 (d, J = 8.8 Hz, 1H), 7.48 (d, J = 5.1 Hz, 1H), 4.49 (q, J = 7.1 Hz, 2H), 3.38-3.27 (m, 1H), 2.44 - 2.29 (m, 2H), 1.40 (t, J = 7.0 Hz, 3H), 1.20 - 0.92 (m, 7H). |

(continued)

| P | Name/Structure (All examples containing chiral centres are racemates unless stated) | Synthesis Method, [LCMS Method], m/z (M+H)$^+$, (RT/Min) | $^1$H NMR Chemical Shift Data (DMSO-d6 unless stated) |
|---|---|---|---|
| **P113** | Single enantiomer - stereochemistry not assigned 2-(2-(Cyclopropanesulfonamido) pyrimidin-4-yl)-N-(5-(6-ethoxypyrazin-2-yl) pyridin-2-yl)-2-fluorobutanamide 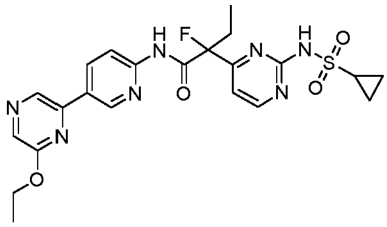 | Method 4 using INTC50 and INTD33 then Method 6 using INTC51 [UPLC acidic], 502, (2.28); [Chiral IC3 HPLC], 10.47 | 11.50 (s, 1H), 10.60 (d, J = 2.2 Hz, 1H), 9.11 (d, J = 2.4 Hz, 1H), 8.87 (s, 1H), 8.76 (d, J = 5.2 Hz, 1H), 8.53 (dd, J = 8.8, 2.5 Hz, 1H), 8.27 (s, 1H), 8.10 (d, J = 8.8 Hz, 1H), 7.48 (d, J = 5.1 Hz, 1H), 4.49 (q, J = 7.0 Hz, 2H), 3.39-3.26 (m, 1H), 2.54 - 2.43 (m, 1H), 2.41 - 2.28 (m, 1H), 1.40 (t, J = 7.0 Hz, 3H), 1.22 - 0.89 (m, 7H). |
| **P114** | Single enantiomer - stereochemistry not assigned 2-(2-(Cyclopropanesulfonamido) pyrimidin-4-yl)-N-(5-(6-ethoxypyrazin-2-yl) pyridin-2-yl)-2-fluorobutanamide 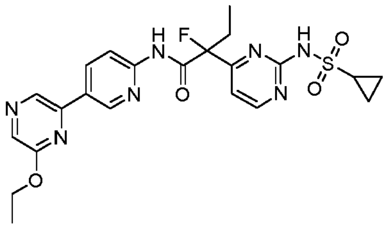 | Method 4 using INTC50 and INTD33 then Method 6 using INTC51 [UPLC acidic], 502, (2.28); [Chiral IC3 HPLC], 14.24 | 11.50 (s, 1H), 10.60 (d, J = 2.3 Hz, 1H), 9.11 (d, J = 2.4 Hz, 1H), 8.87 (s, 1H), 8.76 (d, J = 5.2 Hz, 1H), 8.53 (dd, J = 8.7, 2.5 Hz, 1H), 8.27 (s, 1H), 8.10 (d, J = 8.7 Hz, 1H), 7.48 (d, J = 5.1 Hz, 1H), 4.49 (q, J = 7.0 Hz, 2H), 3.39 - 3.25 (m, 1H), 2.55 - 2.42 (m, 1H), 2.42 - 2.27 (m, 1H), 1.40 (t, J = 7.0 Hz, 3H), 1.25 - 0.88 (m, 7H). |
| **P115** | 4-(2-(Cyclopropanesulfonamido)pyrimidin-4-yl)-N-(5-(6-ethoxypyrazin-2-yl)pyridin-2-yl) tetrahydro-2H-pyran-4-carboxamide 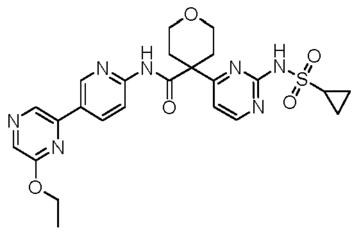 | Method 2 using INTC53 and INTD33, [UPLC, acidic], 528, (1.31) | 11.31 (s, 1H), 10.13 (s, 1H), 9.03 (d, J = 2.5 Hz, 1H), 8.84 (s, 1H), 8.63 (d, J = 5.3 Hz, 1H), 8.50 (dd, J = 8.8, 2.5 Hz, 1H), 8.26 (s, 1H), 8.20 (d, J = 8.8 Hz, 1H), 7.26 (d, J = 5.3 Hz, 1H), 4.48 (q, J = 7.0 Hz, 2H), 3.81 - 3.69 (m, 2H), 3.67 - 3.56 (m, 2H), 3.31 - 3.20 (m, 1H), 2.49-2.41 (m, 2H), 2.25-2.17 (m, 2H), 1.40 (t, J = 7.0 Hz, 3H), 1.09-1.03 (m, 2H), 0.95-0.84 (m , 2H). |
| **P117** | 2-(2-(cyclopropanesulfonamido)pyrimidin-4-yl)-N-(4-(6-ethoxypyrazin-2-yl)-2-fluorophenyl)-2,2-difluoroacetamide | Method 1 using INTC101 and INTD24, [UPLC acidic], 509, (1.45) | 11.77 (s, 1H), 10.98 (s, 1H), 8.92 - 8.82 (m, 2H), 8.28 (s, 1H), 8.11 - 8.00 (m, 2H), 7.72 - 7.64 (m, 1H), 7.57 - 7.46 (m, 1H), 4.50 (q, J = 7.1 Hz, 2H), 3.22 - 3.14 (m, 1H), 1.41 (t, J = 7.0 Hz, 3H), 1.16-1.05 (m, 2H), 1.02 - 0.89 (m, 2H). |

(continued)

| P | Name/Structure (All examples containing chiral centres are racemates unless stated) | Synthesis Method, [LCMS Method], m/z $(M+H)^+$, (RT/Min) | $^1$H NMR Chemical Shift Data (DMSO-d6 unless stated) |
|---|---|---|---|
| | 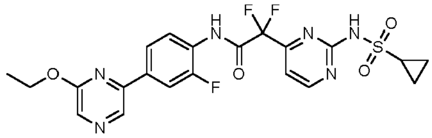 | | |
| **P118** | N-((2-(cyclopropanesulfonamido)pyrimidin-4-yl) methyl)-4-(6-ethoxypyrazin-2-yl)benzamide 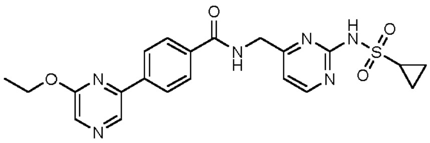 | Method 8 using INTC157 and INTD83, [UPLC acidic], 455, (1.17) Reverse Amide | 11.23 (s, 1H), 9.28 (d, J = 6.1 Hz, 1H), 8.91 (s, 1H), 8.53 (d, J = 5.1 Hz, 1H), 8.32 - 8.23 (m, 3H), 8.06 (dd, J = 8.4, 1.8 Hz, 2H), 7.07 (d, J = 5.0 Hz, 1H), 4.54 - 4.47 (m, 4H), 3.29 - 3.19 (m, 1H), 1.41 (t, J = 7.0 Hz, 3H), 1.10 - 1.06 (m, 2H), 0.96 - 0.91 (m, 2H). |
| **P122** | 2-(2-(cyclopropanesulfonamido)pyrimidin-4-yl)-2-methyl-N-(5-(6-(prop-1-en-2-yl)pyrazin-2-yl) pyridin-2-yl)propanamide 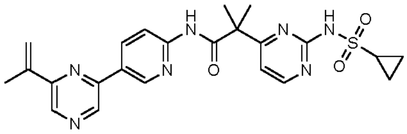 | Method 2b using INTC21 and INTD61, [HPLC acidic], 480, (2.22) | 11.21 (s, 1H), 10.16 (s, 1H), 9.18 (s, 1H), 9.08 (d, J = 2.5 Hz, 1H), 8.90 (s, 1H), 8.59 (d, J = 5.3 Hz, 1H), 8.56 (dd, J = 8.7, 2.5 Hz, 1H), 8.22 (d, J = 8.7 Hz, 1H), 7.20 (d, J = 5.3 Hz, 1H), 6.17 - 6.14 (m, 1H), 5.53 - 5.51 (m, 1H), 3.21 - 3.15 (m, 1H), 2.25 (s, 3H), 1.61 (s, 6H), 1.04 - 0.99 (m, 2H), 0.80 - 0.74 (m, 2H). |
| **P123** | 2-(2-(cyclopropanesulfonamido)-6-methylpyrimidin-4-yl)-N-(5-(6-ethoxypyrazin-2-yl)pyridin-2-yl)-2-methylpropanamide 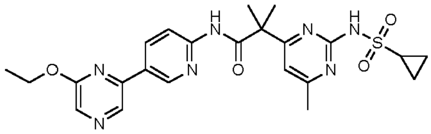 | Method 2b using INTC69 and INTD33, [UPLC acidic], 498, (1.45) | 11.08 (s, 1H), 10.04 (s, 1H), 9.00 (d, J = 2.5 Hz, 1H), 8.83 (s, 1H), 8.48 (dd, J = 8.8, 2.5 Hz, 1H), 8.24 (s, 1H), 8.23 - 8.16 (m, 1H), 7.11 (s, 1H), 4.47 (q, J = 7.0 Hz, 2H), 3.22 - 3.14 (m, 1H), 2.42 (s, 3H), 1.59 (s, 6H), 1.39 (t, J = 7.0 Hz, 3H), 1.04 - 0.97 (m, 2H), 0.79 - 0.70 (m, 2H). |
| **P124** | 2-(2-(cyclopropanesulfonamido)-6-(trifluoromethyl)pyrimidin-4-yl)-N-(5-(6-ethoxypyrazin-2-yl)pyridin-2-yl)-2-methylpropanamide 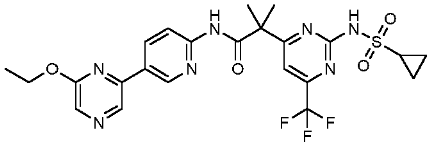 | Method 2b using INTC70 and INTD33, [UPLC acidic], 552, (1.65) | 11.80 (s, 1H), 10.32 (s, 1H), 9.01 (d, J = 2.5 Hz, 1H), 8.84 (s, 1H), 8.50 (dd, J = 8.8, 2.5 Hz, 1H), 8.24 (s, 1H), 8.22 (d, J = 8.7 Hz, 1H), 7.65 (s, 1H), 4.47 (q, J = 7.0 Hz, 2H), 3.10 - 3.02 (m, 1H), 1.64 (s, 6H), 1.39 (t, J = 7.0 Hz, 3H), 1.07 - 0.99 (m, 2H), 0.79 - 0.71 (m, 2H). |

(continued)

| P | Name/Structure (All examples containing chiral centres are racemates unless stated) | Synthesis Method, [LCMS Method], m/z $(M+H)^+$, (RT/Min) | $^1H$ NMR Chemical Shift Data (DMSO-d6 unless stated) |
|---|---|---|---|
| P125 | 2-(2-(cyclopropanesulfonamido)pyrimidin-4-yl)-N-(5-(6-cyclopropylpyrazin-2-yl)pyridin-2-yl)-2-methylpropanamide 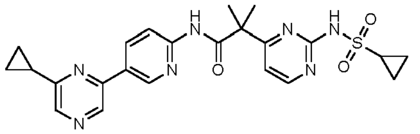 | Method 2b using INTC21 and INTD54, [UPLC acidic], 480, (1.37) | 11.21 (s, 1H), 10.13 (s, 1H), 9.00 (s, 1H), 8.97 (d, J = 2.5 Hz, 1H), 8.59 (d, J = 5.3 Hz, 1H), 8.57 (s, 1H), 8.45 (dd, J = 8.8, 2.5 Hz, 1H), 8.19 (d, J = 8.8 Hz, 1H), 7.19 (d, J = 5.3 Hz, 1H), 3.21 -3.14 (m, 1H), 2.29 - 2.22 (m, 1H), 1.60 (s, 6H), 1.13 - 1.04 (m, 4H), 1.04 - 0.96 (m, 2H), 0.84 - 0.70 (m, 2H) |
| P126 | 2-(2-(cyclopropanesulfonamido)pyrimidin-4-yl)-N-(6-(6-ethoxypyrazin-2-yl)pyridin-3-yl)-2-methylpropanamide 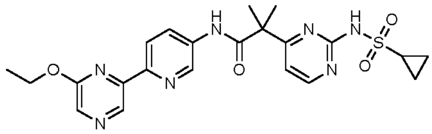 | Method 2 using INTC21 and INTD53, [UPLC acidic], 484, (1.34) | 11.26 (s, 1H), 9.74 (s, 1H), 9.00 (s, 1H), 8.91 (d, J = 2.4 Hz, 1H), 8.61 (d, J = 5.3 Hz, 1H), 8.30 - 8.18 (m, 3H), 7.22 (d, J = 5.3 Hz, 1H), 4.49 (q, J = 7.0 Hz, 2H), 3.22 - 3.15 (m, 1H), 1.60 (s, 6H), 1.40 (t, J = 7.0 Hz, 3H), 1.08 - 1.01 (m, 2H), 0.87 - 0.81 (m, 2H). |
| P128 | 2-(2-(cyclopropanesulfonamido)pyrimidin-4-yl)-N-(4-(6-cyclopropylpyrazin-2-yl)-2-fluorophenyl)-2-methylpropanamide 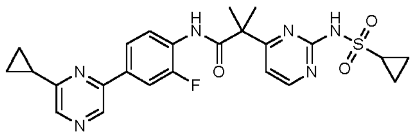 | Method 2b using INTC21 and INTD55, [UPLC acidic], 497, (1.45) | 11.28 (s, 1H), 9.37 (s, 1H), 9.00 (s, 1H), 8.60 (d, J = 5.3 Hz, 1H), 8.58 (s, 1H), 7.97 - 7.90 (m, 2H), 7.71 - 7.67 (m, 1H), 7.19 (d, J = 5.3 Hz, 1H), 3.28 - 3.20 (m, 1H), 2.30 - 2.23 (m, 1H), 1.60 (s, 6H), 1.12 - 1.05 (m, 6H), 1.00 - 0.95 (m, 2H). |
| P129 | 2-(2-(cyclopropanesulfonamido)-6-methylpyrimidin-4-yl)-N-(4-(6-ethoxypyrazin-2-yl)-2-fluorophenyl)-2-methylpropanamide 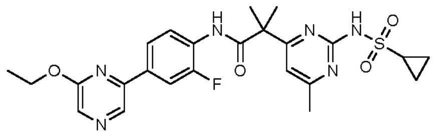 | Method 2b using INTC69 and INTD24, [UPLC acidic], 515, (1.51) | 11.15 (s, 1H), 9.34 (s, 1H), 8.84 (s, 1H), 8.24 (s, 1H), 8.04 - 7.90 (m, 2H), 7.76 - 7.64 (m, 1H), 7.11 (s, 1H), 4.48 (q, J = 7.0 Hz, 2H), 3.28 - 3.22 (m, 1H), 2.43 (s, 3H), 1.59 (s, 6H), 1.40 (t, J = 7.0 Hz, 3H), 1.15 - 1.04 (m, 2H), 1.01 - 0.91 (m, 2H). |
| P130 | 2-(2-(cyclopropanesulfonamido)-6-(trifluoromethyl)pyrimidin-4-yl)-N-(4-(6-ethoxypyrazin-2-yl)-2-fluorophenyl)-2-methylpropanamide 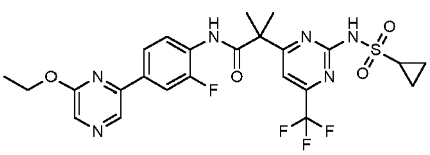 | Method 2b using INTC70 and INTD24, [UPLC acidic], 569, (1.7) | 11.87 (s, 1H), 9.39 (s, 1H), 8.84 (s, 1H), 8.25 (s, 1H), 8.04 - 7.93 (m, 2H), 7.74 - 7.67 (m, 1H), 7.61 (s, 1H), 4.48 (q, J = 7.0 Hz, 2H), 3.22 - 3.13 (m, 1H), 1.65 (s, 6H), 1.40 (t, J = 7.0 Hz, 3H), 1.21 - 1.09 (m, 2H), 1.06 - 0.96 (m, 2H). |

(continued)

| P | Name/Structure (All examples containing chiral centres are racemates unless stated) | Synthesis Method, [LCMS Method], m/z $(M+H)^+$, (RT/Min) | $^1H$ NMR Chemical Shift Data (DMSO-d6 unless stated) |
|---|---|---|---|
| **P131** | 2-(2-(cyclopropanesulfonamido)pyrimidin-4-yl)-2-methyl-N-(4-(6-(prop-1-en-2-yl)pyrazin-2-yl) phenyl)propanamide 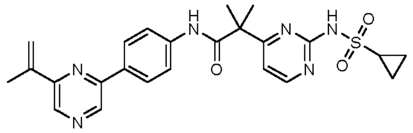 | Method 2b using INTC21 and INTD52, [HPLC acidic], 479, (2.29) | 11.26 (s, 1H), 9.51 (s, 1H), 9.10 (s, 1H), 8.83 (s, 1H), 8.60 (d, J = 5.3 Hz, 1H), 8.17 - 8.09 (m, 2H), 7.83 -7.74 (m, 2H), 7.19 (d, J = 5.3 Hz, 1H), 6.16 - 6.12 (m, 1H), 5.51 - 5.49 (m, 1H), 3.25 - 3.15 (m, 1H), 2.24 (s, 3H), 1.60 (s, 6H), 1.06 - 0.97 (m, 2H), 0.84 - 0.76 (m, 2H). |
| **P132** | 2-(2-(cyclopropanesulfonamido)pyrimidin-4-yl)-N-(4-(6-isopropylpyrazin-2-yl)phenyl)-2-methylpropanamide 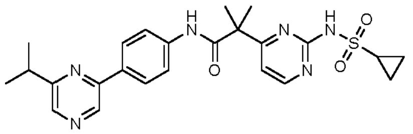 | Method 2b using INTC21 and INTD62, [HPLC acidic], 481, (2.29) | 11.26 (s, 1H), 9.50 (s, 1H), 9.02 (s, 1H), 8.61 (d, J = 5.3 Hz, 1H), 8.50 (s, 1H), 8.15 - 8.06 (m, 2H), 7.82 -7.74 (m, 2H), 7.20 (d, J = 5.3 Hz, 1H), 3.25 - 3.11 (m, 2H), 1.60 (s, 6H), 1.33 (d, J = 6.9 Hz, 6H), 1.07 - 0.99 (m, 2H), 0.86 - 0.76 (m, 2H). |
| **P133** | 2-(2-(cyclopropanesulfonamido)pyrimidin-4-yl)-N-(4-(6-(dimethylamino)pyrazin-2-yl)phenyl)-2-methylpropanamide 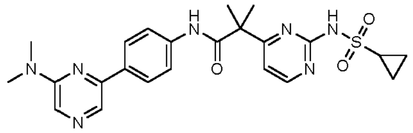 | Method 2 using INTC21 and INTD56, [UPLC acidic], 482, (1.28) | 11.25 (s, 1H), 9.45 (s, 1H), 8.60 (d, J = 5.3 Hz, 1H), 8.35 (s, 1H), 8.08 - 7.99 (m, 3H), 7.77 - 7.70 (m, 2H), 7.19 (d, J = 5.3 Hz, 1H), 3.25 - 3.16 (m, 1H), 3.14 (s, 6H), 1.59 (s, 6H), 1.05 - 0.99 (m, 2H), 0.82 - 0.76 (m, 2H). |
| **P134** | 2-(2-(cyclopropanesulfonamido)-6-methylpyrimidin-4-yl)-N-(4-(6-ethoxypyrazin-2-yl)phenyl)-2-methylpropanamide 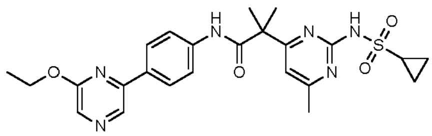 | Method 2b using INTC69 and INTD18, [UPLC acidic], 497, (1.5) | 11.13 (s, 1H), 9.46 (s, 1H), 8.75 (s, 1H), 8.17 (s, 1H), 8.10 - 8.01 (m, 2H), 7.81 - 7.72 (m, 2H), 7.10 (s, 1H), 4.47 (q, J = 7.1 Hz, 2H), 3.24 - 3.16 (m, 1H), 2.42 (s, 3H), 1.58 (s, 6H), 1.39 (t, J = 7.0 Hz, 3H), 1.05 - 0.96 (m, 2H), 0.82 - 0.71 (m, 2H). |
| **P135** | 2-(2-(cyclopropanesulfonamido)-6-(trifluoromethyl)pyrimidin-4-yl)-N-(4-(6-ethoxypyrazin-2-yl)phenyl)-2-methylpropanamide 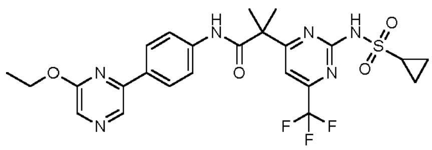 | Method 2b using INTC70 and INTD18, [UPLC acidic], 551, (1.7) | 11.85 (s, 1H), 9.49 (s, 1H), 8.75 (s, 1H), 8.17 (s, 1H), 8.11 - 8.04 (m, 2H), 7.77 - 7.71 (m, 2H), 7.65 (s, 1H), 4.47 (q, J = 7.0 Hz, 2H), 3.15 - 3.04 (m, 1H), 1.65 (s, 6H), 1.39 (t, J = 7.0 Hz, 3H), 1.09 - 1.00 (m, 2H), 0.86 - 0.74 (m, 2H). |

(continued)

| P | Name/Structure (All examples containing chiral centres are racemates unless stated) | Synthesis Method, [LCMS Method], m/z (M+H)$^+$, (RT/Min) | $^1$H NMR Chemical Shift Data (DMSO-d6 unless stated) |
|---|---|---|---|
| **P127** | 2-(2-(Cyclopropanesulfonamido)-6-methoxypyrimidin-4-yl)-2-methyl-N-(4-(pyridin-3-yl)phenyl)propanamide 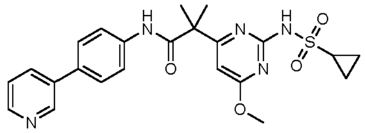 | Method 2 Using INTC71 and commercial aniline, [UPLC acidic], 468, (0.98) | 11.19 (s, 1H), 9.37 (s, 1H), 8.94 - 8.82 (m, 1H), 8.58 - 8.47 (m, 1H), 8.10 - 7.96 (m, 1H), 7.82 - 7.61 (m, 4H), 7.52 - 7.39 (m, 1H), 6.56 (s, 1H), 3.93 (s, 3H), 3.28 - 3.10 (m, 1H), 1.56 (s, 6H), 1.12 - 0.94 (m, 2H), 0.90 - 0.71 (m, 2H). |
| **P136** | 1-(2-(cyclopropanesulfonamido)pyrimidin-4-yl)-N-(5-(6-ethoxypyrazin-2-yl)pyridin-2-yl) cyclopentane-1-carboxamide 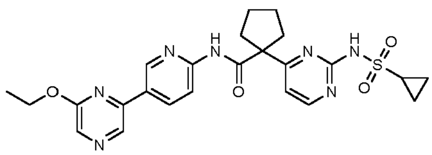 | Method 2 using INTC29 and INTD33, [HPLC acidic], 510, (2.36) | 11.24 (s, 1H), 10.15 (s, 1H), 9.01 (d, J = 2.5 Hz, 1H), 8.84 (s, 1H), 8.60 - 8.46 (m, 2H), 8.32 - 8.15 (m, 2H), 7.15 (s, 1H), 4.48 (q, J = 7.0 Hz, 2H), 2.62-2.42 (m, 3H, oscured by DMSO), 2.28 - 2.13 (m, 2H), 1.80 - 1.61 (m, 4H), 1.40 (t, J = 7.0 Hz, 3H), 1.09-1.00 (m, 2H), 0.90-0.79 (m, 2H). |
| **P137** | 4-(2-(cyclopropanesulfonamido)pyrimidin-4-yl)-N-(4-(6-ethoxypyrazin-2-yl)phenyl) tetrahydro-2H-pyran-4-carboxamide 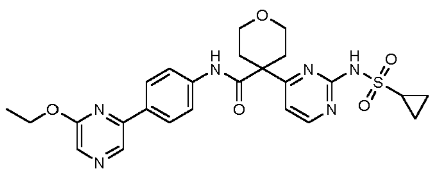 | Method 2 using INTC53 and INTD18, [UPLC acidic], 525, (1.38) | 11.33 (s, 1H), 9.54 (s, 1H), 8.76 (s, 1H), 8.64 - 8.57 (m, 1H), 8.18 (s, 1H), 8.10 - 8.05 (m, 2H), 7.76 (d, J = 8.6 Hz, 2H), 7.20 (s, 1H), 4.47 (q, J = 7.0 Hz, 2H), 3.78 - 3.71 (m, 2H), 3.65 - 3.57 (m, 2H), 3.28 - 3.22 (m, 1H), 2.45 - 2.38 (m, 2H), 2.25 - 2.16 (m, 2H), 1.39 (t, J = 7.0 Hz, 3H), 1.10 - 1.04 (m, 2H), 0.95 - 0.88 (m, 2H). |
| **P138** | N-(5-(6-ethoxypyrazin-2-yl)pyridin-2-yl)-4-(2-(methylsulfonamido)pyrimidin-4-yl) piperidine-4-carboxamide 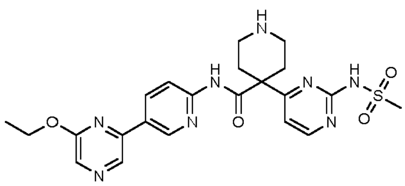 | Method 2 using INTC76 and INTD33, [UPLC acidic], 499, (0.75) (both Boc-protected and free amine isolated) | 10.35 (s, 1H), 9.04 (d, J = 2.3 Hz, 1H), 8.84 (s, 1H), 8.62 (d, J = 5.3 Hz, 1H), 8.54 - 8.48 (m, 1H), 8.26 (s, 1H), 8.18 (d, J = 8.8 Hz, 1H), 7.16 - 7.12 (m, 1H), 4.47 (q, J = 7.1 Hz, 2H), 3.34 (s, 3H), 3.26 - 3.20 (m, 2H), 3.17 - 3.09 (m, 2H), 2.61 - 2.52 (m, 2H), 2.37 (s, 3H), 1.39 (t, J = 7.1 Hz, 3H). 1 x exchangeable NH not observed |

(continued)

| P | Name/Structure (All examples containing chiral centres are racemates unless stated) | Synthesis Method, [LCMS Method], m/z $(M+H)^+$, (RT/Min) | $^1H$ NMR Chemical Shift Data (DMSO-d6 unless stated) |
|---|---|---|---|
| **P139** | *tert*-butyl 4-(2-(cyclopropanesulfonamido) pyrimidin-4-yl)-4-((5-(6-ethoxypyrazin-2-yl) pyridin-2-yl)carbamoyl)piperidine-1-carboxylate 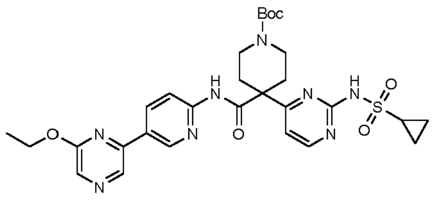 | Method 2 using INTC77 and INTD33, [UPLC acidic], 625, (1.62) (both Boc-protected and free amine isolated) | 9.00 (d, J = 2.4 Hz, 1H), 8.66 (s, 1H), 8.60 (d, J = 5.3 Hz, 1H), 8.48 (dd, J = 8.7, 2.4 Hz, 1H), 8.39 - 8.35 (m, 2H), 8.24 (d, J = 8.7 Hz, 1H), 8.14 (s, 1H), 7.24 (d, J = 5.3 Hz, 1H), 4.54 (q, J = 7.0 Hz, 2H), 3.77 - 3.70 (m, 2H), 3.48 - 3.44 (m, 2H), 3.32 - 3.26 (m, 1H), 2.56 - 2.50 (m, 2H), 2.31 - 2.23 (m, 2H), 1.51 - 1.43 (m, 12H), 1.33 - 1.21 (m, 2H), 1.07 - 0.99 (m, 2H). |
| **P140** | 4-(2-(cyclopropanesulfonamido)pyrimidin-4-yl)-N-(5-(6-ethoxypyrazin-2-yl)pyridin-2-yl) piperidine-4-carboxamide 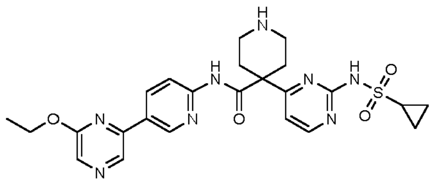 | Method 2 using INTC77 and INTD33, followed by Boc deprotection with HCl, [UPLC acidic], 525, (0.86) | 11.38 (s, 1H), 10.37 (s, 1H), 9.03 (dd, J = 2.4, 0.8 Hz, 1H), 8.91 - 8.76 (m, 2H), 8.65 (d, J = 5.3 Hz, 1H), 8.51 (dd, J = 8.8, 2.4 Hz, 1H), 8.25 (s, 1H), 8.18 (dd, J = 8.8, 0.8 Hz, 1H), 7.21 (d, J = 5.3 Hz, 1H), 4.47 (q, J = 7.0 Hz, 2H), 3.32 - 3.23 (m, 3H), 3.14 - 3.08 (m, 2H), 2.67 - 2.60 (m, 2H), 2.43 - 2.32 (m, 2H), 1.39 (t, J = 7.0 Hz, 3H), 1.10 - 1.03 (m, 2H), 0.96 - 0.87 (m, 2H). |
| **P141** | *tert*-butyl 3-(2-(cyclopropanesulfonamido) pyrimidin-4-yl)-3-((5-(6-ethoxypyrazin-2-yl) pyridin-2-yl)carbamoyl)azetidine-1-carboxylate 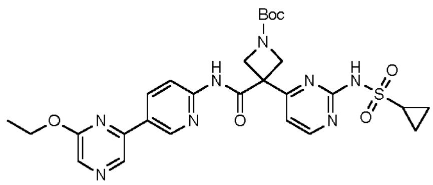 | Method 2 using INTC72 and INTD33, [UPLC acidic], 597, (1.55) | 11.37 (s, 1H), 10.99 (s, 1H), 9.05 (s, 1H), 8.85 (s, 1H), 8.64 (d, J = 5.2 Hz, 1H), 8.57 - 8.51 (m, 1H), 8.26 (s, 4H), 7.27 (d, J = 5.2 Hz, 1H), 4.53 - 4.40 (m, 3H), 4.27 (s, 1H), 3.32 - 3.15 (m, 1H), 1.42 - 1.37 (m, 12H), 1.10 - 1.06 (m, 2H), 0.93 - 0.88 (m, 2H). |
| **P142** | *tert*-butyl 4-((5-(6-ethoxypyrazin-2-yl)pyridin-2-yl)carbamoyl)-4-(2-(methylsulfonamido) pyrimidin-4-yl)piperidine-1-carboxylate 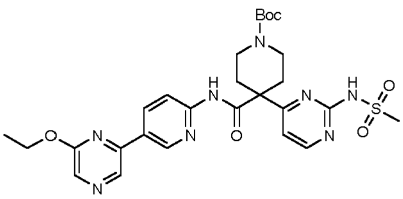 | Method 2 using INTC76 and INTD33, [UPLC acidic], 599, (1.54) (both Boc-protected and free amine isolated) | 11.37 (s, 1H), 10.17 (s, 1H), 9.04 (d, J = 2.3 Hz, 1H), 8.88 - 8.83 (m, 1H), 8.61 (d, J = 5.2 Hz, 1H), 8.52 - 8.47 (m, 1H), 8.28 - 8.24 (m, 1H), 8.19 (d, J = 8.8 Hz, 1H), 7.24 - 7.20 (m, 1H), 4.48 (q, J = 7.0 Hz, 2H), 3.65 - 3.58 (m, 2H), 3.35 (s, 3H), 3.30 - 3.18 (m, 2H), 2.47 - 2.40 (m, 2H), 2.17 - 2.09 (m, 2H), 1.45 - 1.37 (m, 12H). |

(continued)

| P | Name/Structure (All examples containing chiral centres are racemates unless stated) | Synthesis Method, [LCMS Method], m/z $(M+H)^+$, (RT/Min) | $^1H$ NMR Chemical Shift Data (DMSO-d6 unless stated) |
|---|---|---|---|
| **P143** | 4-(2-(cyclopropanesulfonamido)pyrimidin-4-yl)-N-(4-(6-ethoxypyrazin-2-yl)-2-fluorophenyl) tetrahydro-2H-pyran-4-carboxamide 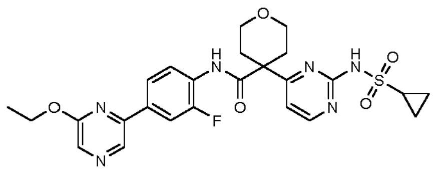 | Method 2 using INTC53 and INTD24, [UPLC acidic], 543, (1.37) | 11.33 (s, 1H), 9.47 (s, 1H), 8.84 (s, 1H), 8.63 (d, J = 5.3 Hz, 1H), 8.25 (s, 1H), 8.03 - 7.93 (m, 2H), 7.64 - 7.57 (m, 1H), 7.22 (d, J = 5.3 Hz, 1H), 4.48 (q, J = 7.0 Hz, 2H), 3.79 - 3.71 (m, 2H), 3.67 - 3.59 (m, 2H), 3.31 - 3.27 (m, 1H), 2.44 - 2.37 (m, 2H), 2.24 - 2.15 (m, 2H), 1.39 (t, J = 7.0 Hz, 3H), 1.15 - 1.08 (m, 2H), 1.05 - 0.98 (m, 2H). |
| **P144** | 2-(2-(cyclopropanesulfonamido)pyrimidin-4-yl)-N-(5-(6-ethoxypyrazin-2-yl)-3-fluoropyridin-2-yl)-4-methoxybutanamide 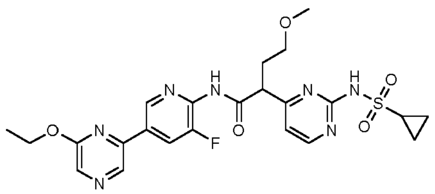 | Method 7 using INTC88, [UPLC acidic], 532, (1.22) | 11.30 (s, 1H), 10.82 (s, 1H), 9.00 (d, J = 1.9 Hz, 1H), 8.92 (s, 1H), 8.56 (s, 1H), 8.43 (dd, J = 11.0, 1.9 Hz, 1H), 8.32 (s, 1H), 7.17 (s, 1H), 4.50 (q, J = 7.0 Hz, 2H), 4.11 (s, 1H), 3.42 - 3.36 (m, 2H), 3.35 - 3.29 (m, 1H), 3.24 (s, 3H), 2.32 - 2.23 (m, 1H), 2.21 - 2.11 (m, 1H), 1.40 (t, J = 7.0 Hz, 3H), 1.13 - 1.09 (m, 2H), 1.03 - 0.99 (m, 2H). |
| **P145** | 2-(2-(cyclopropanesulfonamido)pyrimidin-4-yl)-N-(5-(6-ethoxypyrazin-2-yl)pyridin-2-yl)-4-methoxybutanamide 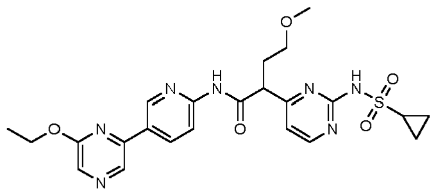 | Method 7 using INTC89, [UPLC acidic], 514, (1.3) | 11.24 (s, 1H), 11.03 (s, 1H), 9.06 (d, J = 2.4 Hz, 1H), 8.84 (s, 1H), 8.56 (d, J = 5.2 Hz, 1H), 8.49 (dd, J = 8.7, 2.4 Hz, 1H), 8.25 (s, 1H), 8.19 (d, J = 8.7 Hz, 1H), 7.21 (d, J = 5.2 Hz, 1H), 4.48 (q, J = 7.0 Hz, 2H), 4.22 (dd, J = 8.4, 6.1 Hz, 1H), 3.41 - 3.32 (m, 3H), 3.21 (s, 3H), 2.36 - 2.25 (m, 1H), 2.20 - 2.06 (m, 1H), 1.40 (t, J = 7.0 Hz, 3H), 1.16 - 1.03 (m, 2H), 1.02 - 0.89 (m, 2H). |
| **P146** | 2-(2-(cyclopropanesulfonamido)pyrimidin-4-yl)-N-(4-(6-ethoxypyrazin-2-yl)-2-fluorophenyl)-4-methoxybutanamide 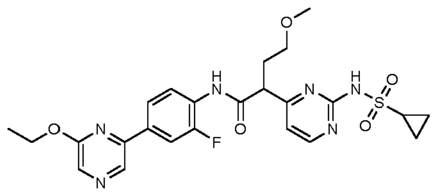 | Method 7 using INTC90, [UPLC acidic], 531, (1.38) | 11.25 (s, 1H), 10.24 (s, 1H), 8.83 (s, 1H), 8.56 (d, J = 5.1 Hz, 1H), 8.23 (s, 1H), 8.07 - 7.98 (m, 2H), 7.99 - 7.93 (m, 1H), 7.19 (d, J = 5.1 Hz, 1H), 4.48 (q, J = 7.0 Hz, 2H), 4.25 - 4.18 (m, 1H), 3.43 - 3.34 (m, 2H), 3.35 - 3.32 (m, 1H), 3.23 (s, 3H), 2.32 - 2.23 (m, 1H), 2.21 - 2.10 (m, 1H), 1.39 (t, J = 7.0 Hz, 3H), 1.13 - 1.08 (m, 2H), 1.02 - 0.94 (m, 2H). |

(continued)

| P | Name/Structure (All examples containing chiral centres are racemates unless stated) | Synthesis Method, [LCMS Method], m/z $(M+H)^+$, (RT/Min) | $^1H$ NMR Chemical Shift Data (DMSO-d6 unless stated) |
|---|---|---|---|
| P147 | N-(5-(6-ethoxypyrazin-2-yl)pyridin-2-yl)-4-methoxy-2-methyl-2-(2-(methylsulfonamido) pyrimidin-4-yl)butanamide 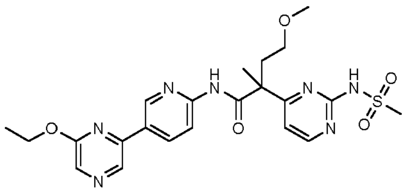 | Method 6 using INTC91, [HPLC acidic], 502, (2.03) | 11.32 (s, 1H), 10.18 (s, 1H), 9.02 (dd, J = 2.5, 0.8 Hz, 1H), 8.84 (s, 1H), 8.59 (d, J = 5.3 Hz, 1H), 8.49 (dd, J = 8.8, 2.5 Hz, 1H), 8.25 (s, 1H), 8.20 (dd, J = 8.8, 0.8 Hz, 1H), 7.16 (d, J = 5.3 Hz, 1H), 4.48 (q, J = 7.1 Hz, 2H), 3.40 - 3.32 (m, 5H), 3.15 (s, 3H), 2.47-2.39 (m, 1H), 2.33-2.26 (m, 1H), 1.61 (s, 3H), 1.40 (t, J = 7.1 Hz, 3H). |
| P148 | N-(5'-chloro-[3,3'-bipyridin]-6-yl)-2-(2-(cyclopropanesulfonamido)pyrimidin-4-yl) butanamide 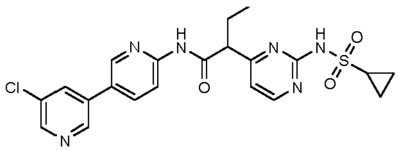 | Method 3 using INTC37 and NTD57, HPLC acidic], 473 $^{35}Cl$ isotope, (2.02) | 11.23 (s, 1H), 11.01 (s, 1H), 8.93 (d, J = 2.0 Hz, 1H), 8.81 - 8.79 (m, 1H), 8.36 - 8.34 (m, 1H), 8.56 (d, J = 5.2 Hz, 1H), 8.35 (t, J = 2.2 Hz, 1H), 8.27-8.23 (m, 1H), 8.19 (d, J = 8.7 Hz, 1H), 7.21 (d, J = 5.2 Hz, 1H), 4.07 - 3.97 (m, 1H), 3.32 - 3.28 (m, 1H), 2.13 - 2.02 (m, 1H), 1.99-1.89 (m, 1H), 1.15-1.05 (m, 2H), 1.01 - 0.90 (m, 5H). |
| P149 | N-(5'-chloro-[3,3'-bipyridin]-6-yl)-2-(2-(cyclopropanesulfonamido)pyrimidin-4-yl)-2-fluorobutanamide 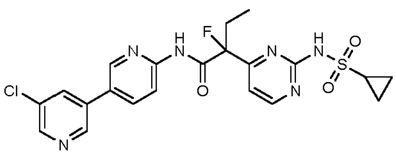 | Method 6 using INTC92 [HPLC acidic], 491 $^{35}Cl$ isotope, (2.14) | 11.50 (s, 1H), 10.58 (d, J = 2.0 Hz, 1H), 8.94 (d, J = 2.0 Hz, 1H), 8.85 - 8.82 (m, 1H), 8.76 (d, J = 5.2 Hz, 1H), 8.66 (d, J = 2.3 Hz, 1H), 8.38 - 8.36 (m, 1H), 8.29 (dd, J = 8.7, 2.6 Hz, 1H), 8.07 (d, J = 8.7 Hz, 1H), 7.49 - 7.45 (m, 1H), 3.50-3.25 (m, 1H), 2.48 - 2.43 (m, 1H), 2.39-2.38 (m, 1H), 1.20 - 1.14 (m, 1H), 1.12 - 1.02 (m, 2H), 1.00 - 0.90 (m, 4H). |
| P150 | 2-(2-(cyclopropanesulfonamido)pyrimidin-4-yl)-N-(5-(6-cyclopropylpyrazin-2-yl)pyridin-2-yl)-2-fluorobutanamide 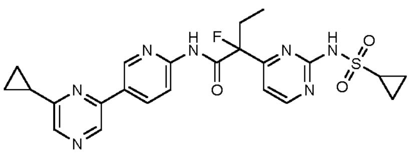 | Method 6 using INTC176, [HPLC acidic], 498, (2.27) | 11.50 (s, 1H), 10.59 (d, J = 2.3 Hz, 1H), 9.06 (d, J = 2.4 Hz, 1H), 9.03 (s, 1H), 8.76 (d, J = 5.2 Hz, 1H), 8.60 (s, 1H), 8.50 (dd, J = 8.7, 2.4 Hz, 1H), 8.09 (d, J = 8.7 Hz, 1H), 7.48 (dd, J = 5.2, 1.2 Hz, 1H), 3.26-3.32 (m, 1H), 2.49-2.44 (m, 1H), 2.40 - 2.24 (m, 2H), 1.19 - 1.13 (m, 1H), 1.13 - 1.01 (m, 6H), 1.00 - 0.90 (m, 4H). |

(continued)

| P | Name/Structure (All examples containing chiral centres are racemates unless stated) | Synthesis Method, [LCMS Method], m/z (M+H)+, (RT/Min) | $^1$H NMR Chemical Shift Data (DMSO-d6 unless stated) |
|---|---|---|---|
| **P151** | N-(5-(6-ethoxypyrazin-2-yl)pyridin-2-yl)-2-fluoro-2-(2-(methylsulfonamido)pyrimidin-4-vl) butanamide 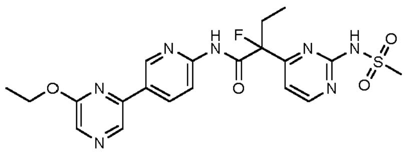 | Method 2 using INTC74 and INTD33, [UPLC acidic], 476, (1.38) | 11.56 (s, 1H), 10.61 (s, 1H), 9.10 (d, J = 2.5 Hz, 1H), 8.86 (s, 1H), 8.77 - 8.72 (m, 1H), 8.52 (dd, J = 8.7, 2.5 Hz, 1H), 8.26 (s, 1H), 8.10 (d, J = 8.7 Hz, 1H), 7.47 - 7.43 (m, 1H), 4.48 (q, J = 7.0 Hz, 2H), 3.31 (s, 3H), 2.51 - 2.42 (m, 1H), 2.36 - 2.26 (m, 1H), 1.39 (t, J = 7.0 Hz, 3H), 0.92 (t, J = 7.3 Hz, 3H). |
| **P155** | 2-(2-(cyclopropanesulfonamido)pyrimidin-4-yl)-N-(5-(6-ethoxypyrazin-2-yl)-3-methylpyridin-2-yl)butanamide 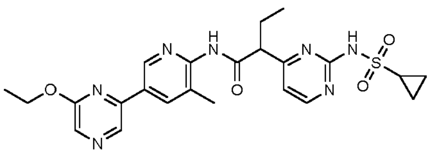 | Method 3 using INTC37 and INTD58, [UPLC acidic], 498, (1.22) | 11.25 (s, 1H), 10.51 (s, 1H), 8.96 (d, J = 2.2 Hz, 1H), 8.86 (s, 1H), 8.58 (d, J = 5.2 Hz, 1H), 8.35 (d, J = 2.2 Hz, 1H), 8.28 (s, 1H), 7.21 (d, J = 5.2 Hz, 1H), 4.49 (q, J = 7.0 Hz, 2H), 3.90 - 3.85 (m, 1H), 3.41 - 3.34 (m, 1H), 2.18 (s, 3H), 2.14 - 2.05 (m, 1H), 2.00 - 1.90 (m, 1H), 1.41 (t, J = 7.0 Hz, 3H), 1.18 -1.10 (m, 2H), 1.07 - 1.01 (m, 2H), 0.98 (t, J = 7.3 Hz, 3H). |
| **P156** | 2-(2-(cyclopropanesulfonamido)pyrimidin-4-yl)-N-(5-(6-cyclopropylpyrazin-2-yl)pyridin-2-yl) butanamide 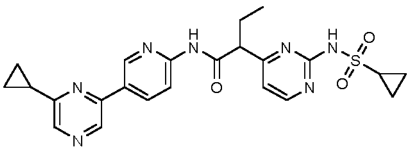 | Method 3 using INTC37 and INTD54, [UPLC acidic], 480, (1.36) | 11.22 (s, 1H), 11.03 (s, 1H), 9.02 (d, J = 2.4 Hz, 1H), 9.00 (s, 1H), 8.58 (s, 1H), 8.55 (d, J = 5.2 Hz, 1H), 8.45 (dd, J = 8.7, 2.4 Hz, 1H), 8.19 (d, J = 8.7 Hz, 1H), 7.20 (d, J = 5.2 Hz, 1H), 4.00 (dd, J = 8.6, 6.4 Hz, 1H), 3.31 - 3.26 (m, 1H), 2.30 - 2.22 (m, 1H), 2.13 - 2.00 (m, 1H), 1.94 (dq, J = 13.7, 6.9 Hz, 1H), 1.13-1.04 (m, 6H), 1.00 - 0.88 (m, 5H) |
| **P157** | 2-(2-(cyclopropanesulfonamido)pyrimidin-4-yl)-N-(5-(6-(2,2,2-trifluoroethoxy)pyrazin-2-yl) pyridin-2-yl)butanamide 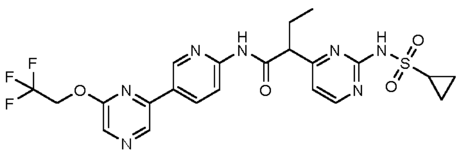 | Method 3 using INTC37 and INTD59, [UPLC acidic], 538, (1.44) | 11.23 (s, 1H), 11.07 (s, 1H), 9.15 (d, J = 2.4 Hz, 1H), 9.01 (s, 1H), 8.62 - 8.54 (m, 2H), 8.45 (s, 1H), 8.23 (d, J = 8.8 Hz, 1H), 7.22 (d, J = 5.2 Hz, 1H), 5.23 (d, J = 9.0 Hz, 1H), 5.20 (d, J = 9.1 Hz, 1H), 4.02 (dd, J = 8.5, 6.4 Hz, 1H), 2.12 - 2.02 (m, 1H), 1.99 - 1.90 (m, 1H), 1.14 - 1.06 (m, 2H), 1.01 - 0.90 (m, 5H). 1H obscured by $H_2O$ |
| **P158** | 2-(2-(cyclopropanesulfonamido)pyrimidin-4-yl)-N-(3-fluoro-5-(6-methoxypyrazin-2-yl)pyridin-2-yl)butanamide 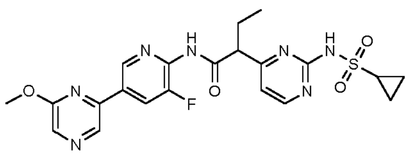 | Method 9 using INTC94 and INTD63, [UPLC acidic], 488, (1.17) | 11.26 (s, 1H), 10.80 (s, 1H), 9.03 (d, J = 1.9 Hz, 1H), 8.95 (s, 1H), 8.59 (d, J = 5.2 Hz, 1H), 8.46 (dd, J = 11.0, 1.9 Hz, 1H), 8.36 (s, 1H), 7.22 - 7.18 (m, 1H), 4.04 (s, 3H), 3.93 - 3.86 (m, 1H), 2.13 - 2.01 (m, 1H), 2.00 - 1.90 (m, 1H), 1.15 - 1.11 (m, 2H), 1.07 - 1.01 (m, 2H), 0.97 (t, 3H). 1H obscured by $H_2O$ |

(continued)

| P | Name/Structure (All examples containing chiral centres are racemates unless stated) | Synthesis Method, [LCMS Method], m/z $(M+H)^+$, (RT/Min) | $^1H$ NMR Chemical Shift Data (DMSO-d6 unless stated) |
|---|---|---|---|
| **P159** | 2-(2-(cyclopropanesulfonamido)pyrimidin-4-yl)-N-(5-(6-methoxypyrazin-2-yl)pyridin-2-yl) butanamide 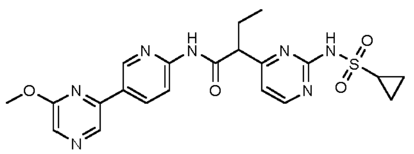 | Method 9 using INTC95 and INTD63, [UPLC acidic], 470, (1.25) | 11.23 (s, 1H), 11.06 (s, 1H), 9.10 (d, J = 2.6 Hz, 1H), 8.87 (s, 1H), 8.60 - 8.50 (m, 2H), 8.29 (s, 1H), 8.22 (d, J = 8.8 Hz, 1H), 7.21 (d, J = 5.2 Hz, 1H), 4.03 (s, 3H), 4.03 - 3.99 (m, 1H), 3.32 - 3.28 (m, 1H), 2.13 - 2.03 (m, 1H), 2.00 - 1.91 (m, 1H), 1.14 - 1.07 (m, 2H), 0.93 (t, J = 7.3 Hz, 5H). |
| **P160** | 2-(2-(cyclopropanesulfonamido)pyrimidin-4-yl)-N-(4-(6-cyclopropylpyrazin-2-yl)-2-fluorophenyl)butanamide 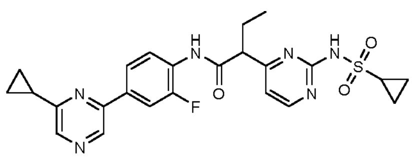 | Method 3 using INTC37 and INTD55, [UPLC acidic], 497, (1.43) | 11.23 (s, 1H), 10.21 (s, 1H), 8.99 (s, 1H), 8.59 - 8.53 (m, 2H), 8.07 - 7.99 (m, 1H), 7.97 (dd, J = 12.2, 2.0 Hz, 1H), 7.92 (dd, J = 8.5, 2.0 Hz, 1H), 7.19 (d, J = 5.2 Hz, 1H), 3.98 (dd, J = 8.6, 6.3 Hz, 1H), 3.32 - 3.26 (m, 1H), 2.29 - 2.20 (m, 1H), 2.10 - 2.00 (m, 1H), 2.00 - 1.88 (m, 1H), 1.13 - 1.05 (m, 6H), 1.03 - 0.91 (m, 5H). |
| **P161** | 2-(2-(cyclopropanesulfonamido)pyrimidin-4-yl)-N-(4-(6-ethoxypyrazin-2-yl)-2-methylphenyl) butanamide 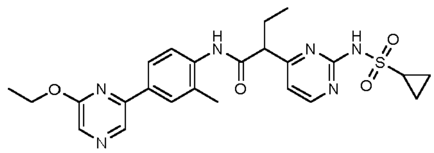 | Method 3 using INTC37 and INTD27, [UPLC acidic], 497, (1.4) | 11.26 (s, 1H), 9.73 (s, 1H), 8.78 (s, 1H), 8.58 (d, J = 5.2 Hz, 1H), 8.21 (s, 1H), 7.99 (d, J = 2.2 Hz, 1H), 7.92 (dd, J = 8.3, 2.2 Hz, 1H), 7.56 (d, J = 8.3 Hz, 1H), 7.23 (d, J = 5.2 Hz, 1H), 4.48 (q, J = 7.0 Hz, 2H), 3.87 (dd, J = 8.7, 6.4 Hz, 1H), 2.27 (s, 3H), 2.13 - 2.04 (m, 1H), 2.00 - 1.92 (m, 1H), 1.40 (t, J = 7.1 Hz, 3H), 1.17 - 1.09 (m, 2H), 1.06 - 0.94 (m, 5H). 1H obscured by $H_2O$ |
| **P162** | 2-(2-(cyclopropanesulfonamido)pyrimidin-4-yl)-N-(5-(6-ethoxypyrazin-2-yl)-3-fluoropyridin-2-yl)butanamide 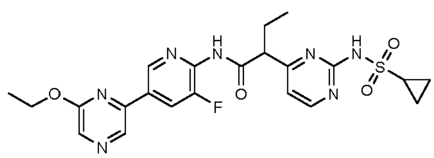 | Method 3 using INTC37 and INTD31, [UPLC acidic], 502, (1.27) | 11.27 (s, 1H), 10.80 (s, 1H), 9.04 - 8.98 (m, 1H), 8.92 (s, 1H), 8.59 (d, J = 5.2 Hz, 1H), 8.43 (dd, J = 11.1, 1.9 Hz, 1H), 8.32 (s, 1H), 7.20 (d, J = 5.2 Hz, 1H), 4.50 (q, J = 7.0 Hz, 2H), 3.90 (dd, J = 8.7, 6.4 Hz, 1H), 3.38 - 3.34 (m, 1H), 2.13 - 2.03 (m, 1H), 2.01 - 1.89 (m, 1H), 1.41 (t, J = 7.0 Hz, 3H), 1.19 - 1.09 (m, 2H), 1.08 - 1.00 (m, 2H), 0.97 (t, J = 7.3 Hz, 3H). |

(continued)

| P | Name/Structure (All examples containing chiral centres are racemates unless stated) | Synthesis Method, [LCMS Method], m/z $(M+H)^+$, (RT/Min) | $^1H$ NMR Chemical Shift Data (DMSO-d6 unless stated) |
|---|---|---|---|
| **P163** | 2-(2-(cyclopropanesulfonamido)pyrimidin-4-yl)-N-(5-(6-ethoxypyrazin-2-yl)pyridin-2-yl)-2-methylbutanamide 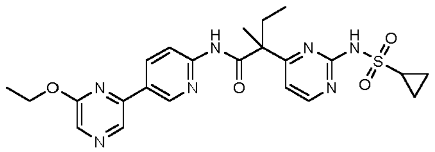 | Method 6 using INTC93, [HPLC acidic], 498, (2.30) | 11.22 (s, 1H), 10.11 (s, 1H), 9.01 (dd, J = 2.5, 0.8 Hz, 1H), 8.84 (s, 1H), 8.59 (d, J = 5.3 Hz, 1H), 8.49 (dd, J = 8.8, 2.5 Hz, 1H), 8.25 (s, 1H), 8.21 (dd, J = 8.8, 0.8 Hz, 1H), 7.17 (d, J = 5.3 Hz, 1H), 4.48 (q, J = 7.0 Hz, 2H), 3.23-3.15 (m, 1H), 2.27-2.17 (m, 1H), 2.07-2.00 (m, 1H), 1.55 (s, 3H), 1.40 (t, J = 7.1 Hz, 3H), 1.06-0.96 (m, 2H), 0.83 (t, J = 7.4 Hz, 3H), 0.80 - 0.74 (m, 2H). |
| **P152** | 2-(2-(cyclopropanesulfonamido)pyrimidin-4-yl)-N-(5-(6-ethoxypyrazin-2-yl)pyridin-2-yl)-2-fluoro-3-methylbutanamide 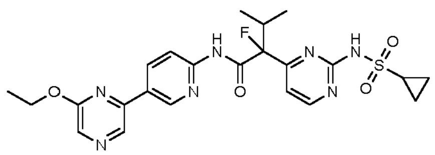 | Method 6 using INTC96, [UPLC acidic], 516, (1.53) | 11.53 (s, 1H), 10.50 (d, J = 2.5 Hz, 1H), 9.10 (d, J = 2.4 Hz, 1H), 8.86 (s, 1H), 8.75 (d, J = 5.2 Hz, 1H), 8.52 (dd, J = 8.7, 2.4 Hz, 1H), 8.27 (s, 1H), 8.08 (d, J = 8.7 Hz, 1H), 7.52 - 7.39 (m, 1H), 4.48 (q, J = 7.1 Hz, 2H), 3.44 - 3.36 (m, 1H), 3.15 - 2.96 (m, 1H), 1.40 (t, J = 7.1 Hz, 3H), 1.27 - 0.96 (m, 7H), 0.79 (d, J = 6.9 Hz, 3H). |
| **P153** | 2-(2-(cyclopropanesulfonamido)pyrimidin-4-yl)-N-(4-(6-ethoxypyrazin-2-yl)-2-fluorophenyl)-3-methylbutanamide 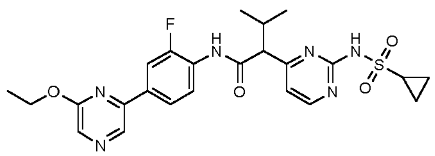 | Method 6 using INTC97, [HPLC acidic], 515, (2.38) | 11.25 (s, 1H), 10.21 (s, 1H), 8.83 (d, J = 0.6 Hz, 1H), 8.58 (d, J = 5.2 Hz, 1H), 8.24 (s, 1H), 8.04 - 7.93 (m, 3H), 7.28 (d, J = 5.2 Hz, 1H), 4.48 (q, J = 7.0 Hz, 2H), 3.74 (d, J = 10.2 Hz, 1H), 3.39 - 3.30 (m, 1H), 2.54 - 2.47 (m, 1H), 1.40 (t, J = 7.0 Hz, 3H), 1.21 - 0.92 (m, 7H), 0.81 (d, J = 6.6 Hz, 3H). |
| **P154** | 2-(2-(cyclopropanesulfonamido)pyrimidin-4-yl)-N-(5-(6-ethoxypyrazin-2-yl)pyridin-2-yl)-3-methylbutanamide 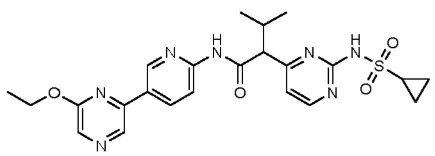 | Method 6 using INTC98, [HPLC acidic], 498, (2.30) | 11.24 (s, 1H), 11.04 (s, 1H), 9.07 (dd, J = 2.4, 0.8 Hz, 1H), 8.84 (s, 1H), 8.57 (d, J = 5.2 Hz, 1H), 8.49 (dd, J = 8.8, 2.4 Hz, 1H), 8.25 (s, 1H), 8.19 (d, J = 8.8 Hz, 1H), 7.28 (d, J = 5.2 Hz, 1H), 4.48 (q, J = 7.0 Hz, 2H), 3.78 (d, J = 10.2 Hz, 1H), 3.42 - 3.25 (m, 1H), 2.57 - 2.44 (m, 1H), 1.40 (t, J = 7.0 Hz, 3H), 1.22 - 0.94 (m, 7H), 0.79 (d, J = 6.6 Hz, 3H). |

(continued)

| P | Name/Structure (All examples containing chiral centres are racemates unless stated) | Synthesis Method, [LCMS Method], m/z (M+H)$^+$, (RT/Min) | $^1$H NMR Chemical Shift Data (DMSO-d6 unless stated) |
|---|---|---|---|
| **P164** | 2-(2-(cyclopropanesulfonamido)pyrimidin-4-yl)-N-(4-(6-ethoxypyrazin-2-yl)phenyl)-2-methoxyacetamide 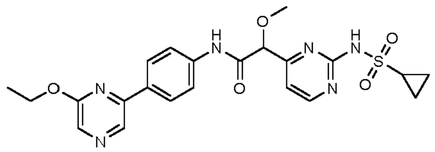 | Method 4 using INTC99 and INTD18, [HPLC acidic], 485, (2.05) | 11.38 (s, 1H), 10.45 (s, 1H), 8.78 (s, 1H), 8.66 (d, J = 5.1 Hz, 1H), 8.19 (s, 1H), 8.13 - 8.09 (m, 2H), 7.85 - 7.81 (m, 2H), 7.27 (d, J = 5.1 Hz, 1H), 4.98 (s, 1H), 4.54 - 4.43 (m, 2H), 3.48 (s, 3H), 3.28 - 3.19 (m, 1H), 1.40 (t, J = 7.0 Hz, 3H), 1.13 - 0.95 (m, 2H), 0.89 - 0.78 (m, 2H). |
| **P165** | Single enantiomer - stereochemistry unassigned N-(5-(6-ethoxypyrazin-2-yl)pyridin-2-yl)-2-fluoro-2-(2-(methylsulfonamido)pyrimidin-4-yl) butanamide 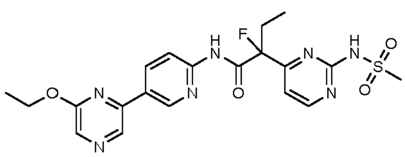 | Method 2 using INTC74 and INTD33, Chiral IC6 (14.67), [UPLC acidic], 476, (1.36) | 11.55 (s, 1H), 10.60 (s, 1H), 9.10 (d, J = 2.4 Hz, 1H), 8.85 (s, 1H), 8.76 - 8.71 (m, 1H), 8.52 (dd, J = 8.7, 2.4 Hz, 1H), 8.26 (s, 1H), 8.10 (d, J = 8.7 Hz, 1H), 7.47 - 7.43 (m, 1H), 4.47 (q, J = 7.0 Hz, 2H), 3.39 (s, 3H), 2.48 - 2.29 (m, 2H), 1.39 (t, J = 7.0 Hz, 3H), 0.92 (t, J = 7.3 Hz, 3H). |
| **P166** | Single enantiomer - stereochemistry unassigned N-(5-(6-ethoxypyrazin-2-yl)pyridin-2-yl)-2-fluoro-2-(2-(methylsulfonamido)pyrimidin-4-yl) butanamide 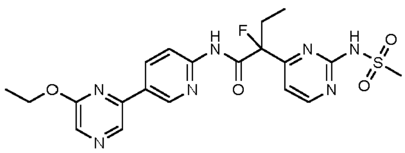 | Method 2 using INTC74 and INTD33, Chiral IC6 (17.03), [UPLC acidic], 476, (1.36) | 11.56 (s, 1H), 10.62 (s, 1H), 9.11 (d, J = 2.4 Hz, 1H), 8.87 (s, 1H), 8.77 - 8.72 (m, 1H), 8.53 (dd, J = 8.7, 2.4 Hz, 1H), 8.27 (s, 1H), 8.11 (d, J = 8.7 Hz, 1H), 7.46 - 7.42 (m, 1H), 4.49 (q, J = 7.0 Hz, 2H), 3.38 (s, 3H), 2.44 - 2.27 (m, 2H), 1.40 (t, J = 7.0 Hz, 3H), 0.93 (t, J = 7.3 Hz, 3H). |
| **P167** | N-(4-(5-chloropyridin-3-yl)phenyl)-2-(6-(cyclopropanesulfonamido)pyridin-2-yl) acetamide 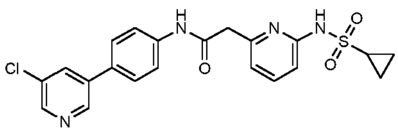 | Method 1 using INTC110 and INTD8, [UPLC acidic], 443 $^{35}$Cl isotope, (1.26) | 10.62 (s, 1H), 10.38 (s, 1H), 8.87 (d, J = 2.0 Hz, 1H), 8.58 (d, J = 2.3 Hz, 1H), 8.26 - 8.20 (m, 1H), 7.82 - 7.66 (m, 5H), 7.03 (d, J = 7.4 Hz, 1H), 6.91 (d, J = 8.3 Hz, 1H), 3.82 (s, 2H), 3.13 - 3.08 (m, 1H), 1.08 - 0.99 (m, 2H), 0.92 - 0.80 (m, 2H). |
| **P168** | N-(4-(5-cyanopyridin-3-yl)phenyl)-2-(6-(cyclopropanesulfonamido)pyridin-2-yl) acetamide 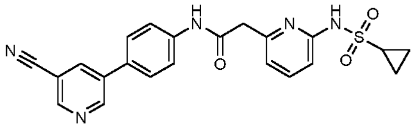 | Method 1 using INTC110 and INTD5, [UPLC acidic], 434, (1.14) | 10.75 (s, 1H), 10.38 (s, 1H), 9.19 (d, J = 2.3 Hz, 1H), 8.97 (d, J = 1.9 Hz, 1H), 8.69 - 8.57 (m, 1H), 7.88 - 7.62 (m, 5H), 7.02 (d, J = 7.5 Hz, 1H), 6.91 (d, J = 8.3 Hz, 1H), 3.82 (s, 2H), 3.18 - 3.00 (m, 1H), 1.11 - 0.94 (m, 2H), 0.93 - 0.75 (m, 2H). |

(continued)

| P | Name/Structure (All examples containing chiral centres are racemates unless stated) | Synthesis Method, [LCMS Method], m/z $(M+H)^+$, (RT/Min) | $^1H$ NMR Chemical Shift Data (DMSO-d6 unless stated) |
|---|---|---|---|
| P169 | 2-(6-(cyclopropanesulfonamido)pyridin-2-yl)-N-(4-(5-fluoropyridin-3-yl)phenyl)acetamide 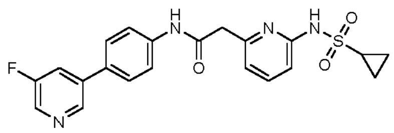 | Method 1 using INTC110 and INTD6, [UPLC acidic], 427, (1.16) | 10.75 (s, 1H), 10.37 (s, 1H), 8.87 - 8.66 (m, 1H), 8.54 (d, J = 2.8 Hz, 1H), 8.14 - 7.95 (m, 1H), 7.85 - 7.58 (m, 5H), 7.01 (d, J = 7.4 Hz, 1H), 6.91 (d, J = 8.3 Hz, 1H), 3.82 (s, 2H), 3.14 - 3.01 (m, 1H), 1.07 - 0.95 (m, 2H), 0.93 - 0.79 (m, 2H). |
| P170 | 2-(6-(cyclopropanesulfonamido)pyridin-2-yl)-N-(4-(5-methoxypyridin-3-yl)phenyl) acetamide 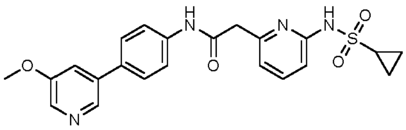 | Method 1 using INTC110 and INTD45, [UPLC acidic], 439, (0.88) | 10.35 (s, 1H), 8.48 (d, J = 1.8 Hz, 1H), 8.25 (d, J = 2.7 Hz, 1H), 7.79 - 7.65 (m, 6H), 7.60 (dd, J = 2.7, 1.8 Hz, 1H), 7.01 (d, J = 7.4 Hz, 1H), 6.91 (d, J = 8.1 Hz, 1H), 3.91 (s, 3H), 3.81 (s, 2H), 3.20 - 3.00 (m, 1H), 1.08 - 0.96 (m, 2H), 0.92 - 0.80 (m, 2H). |
| P171 | 2-(6-(cyclopropanesulfonamido)pyridin-2-yl)-N-(4-(pyridin-3-yl)phenyl)acetamide 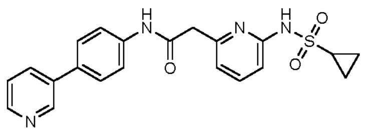 | Method 1 using INTC110 and commercial aniline, [UPLC acidic], 409, (0.7) | 10.72 (s, 1H), 10.33 (s, 1H), 8.96 - 8.79 (m, 1H), 8.54 (dd, J = 4.8, 1.6 Hz, 1H), 8.12 - 7.97 (m, 1H), 7.83 - 7.60 (m, 5H), 7.51 - 7.38 (m, 1H), 7.01 (d, J = 7.4 Hz, 1H), 6.91 (d, J = 8.2 Hz, 1H), 3.82 (s, 2H), 3.09 (s, 1H), 1.09 - 0.97 (m, 2H), 0.92 - 0.79 (m, 2H). |
| P172 | 2-(6-(cyclopropanesulfonamido)pyridin-2-yl)-N-(4-(6-(trifluoromethyl)pyrazin-2-yl)phenyl) acetamide 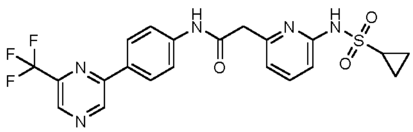 | Method 10 using INTC110 and INTD19, [UPLC acidic], 478, (1.4) | 10.51 (d, J = 20.2 Hz, 2H), 9.58 (s, 1H), 9.08 (s, 1H), 8.26 - 8.13 (m, 2H), 7.88 - 7.78 (m, 2H), 7.71 (dd, J = 8.3, 7.5 Hz, 1H), 7.07 (d, J = 7.3 Hz, 1H), 6.90 (d, J = 8.3 Hz, 1H), 3.84 (s, 2H), 3.20 3.08 (m, 1H), 1.08 - 1.01 (m, 2H), 0.94 - 0.82 (m, 2H). |
| P173 | 2-(6-(cyclopropanesulfonamido)pyridin-2-yl)-N-(4-(6-methoxypyrazin-2-yl)phenyl) acetamide 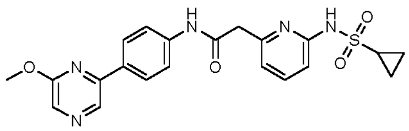 | Method 10 using INTC110 and INTD1, [UPLC acidic], 440, (1.25) | 10.54 (s, 1H), 10.40 (s, 1H), 8.79 (s, 1H), 8.22 (s, 1H), 8.20 - 8.09 (m, 2H), 7.85 - 7.68 (m, 3H), 7.07 (d, J = 7.6 Hz, 1H), 6.89 (d, J = 8.3 Hz, 1H), 4.02 (s, 3H), 3.83 (s, 2H), 3.22 - 3.07 (m, 1H), 1.04 (s, 2H), 0.96 - 0.75 (m, 2H). |
| P174 | 2-(6-(cyclopropanesulfonamido)pyridin-2-yl)-N-(4-(pyrazin-2-yl)phenyl)acetamide 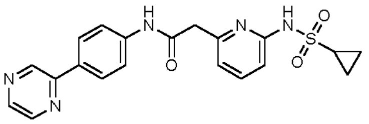 | Method 10 using INTC110 and commercial aniline, [HPLC acidic], 410, (1.64) | 10.54 (s, 1H), 10.40 (s, 1H), 9.22 (d, J = 1.6 Hz, 1H), 8.68 (dd, J = 2.5, 1.6 Hz, 1H), 8.56 (d, J = 2.5 Hz, 1H), 8.18 - 8.07 (m, 2H), 7.84 - 7.64 (m, 3H), 7.07 (d, J = 7.4 Hz, 1H), 6.90 (d, J = 8.1 Hz, 1H), 3.83 (s, 2H), 3.15 (s, 1H), 1.03 (s, 2H), 0.93 - 0.79 (m, 2H). |

(continued)

| P | Name/Structure (All examples containing chiral centres are racemates unless stated) | Synthesis Method, [LCMS Method], m/z $(M+H)^+$, (RT/Min) | $^1H$ NMR Chemical Shift Data (DMSO-d6 unless stated) |
|---|---|---|---|
| **P175** | N-([3,3'-bipyridin]-6-yl)-2-(6-(cyclopropanesulfonamido)pyridin-2-yl)-2-methylpropanamide 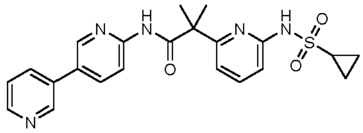 | Method 2 using INTC106 and commercial aniline, [UPLC acidic], 438, (1.52) | 10.63 (s, 1H), 9.68 (s, 1H), 8.96 - 8.90 (m, 1H), 8.68 - 8.62 (m, 1H), 8.61 - 8.55 (m, 1H), 8.19 - 8.09 (m, 3H), 7.79 - 7.69 (m, 1H), 7.54 - 7.46 (m, 1H), 7.17 - 7.10 (m, 1H), 6.87 - 6.79 (m, 1H), 3.17 - 3.08 (m, 1H), 1.62 (s, 6H), 1.02 - 0.93 (m, 2H), 0.76 - 0.67 (m, 2H). |
| **P176** | N-(4-(5-chloropyridin-3-yl)phenyl)-2-(6-(cyclopropanesulfonamido)pyridin-2-yl)-2-methylpropanamide 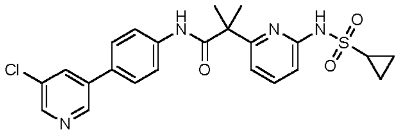 | Method 8 using INTC109 and INTD8, [HPLC basic], 471 $^{35}Cl$ isotope, (2.2) | 10.61 (s, 1H), 9.40 (s, 1H), 8.87 (d, J = 2.0 Hz, 1H), 8.58 (d, J = 2.3 Hz, 1H), 8.23 - 8.21 (m, 1H), 7.82 - 7.71 (m, 5H), 7.13 (d, J = 7.6 Hz, 1H), 6.82 (d, J = 8.1 Hz, 1H), 3.23 - 3.11 (m, 1H), 1.62 (s, 6H), 1.06 - 0.96 (m, 2H), 0.83 - 0.73 (m, 2H). |
| **P177** | 2-(6-(cyclopropanesulfonamido)pyridin-2-yl)-N-(4-(5-fluoropyridin-3-yl)phenyl)-2-methylpropanamide 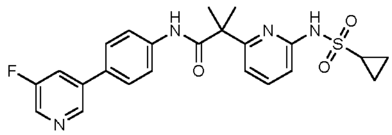 | Method 2 using INTC106 and INTD6, [HPLC acidic], 455, (2.15) | 10.61 (s, 1H), 9.39 (s, 1H), 8.84 - 8.74 (m, 1H), 8.59 - 8.45 (m, 1H), 8.09 - 7.97 (m, 1H), 7.83 - 7.68 (m, 5H), 7.18 - 7.06 (m, 1H), 6.87 - 6.74 (m, 1H), 3.21 - 3.04 (m, 1H), 1.61 (s, 6H), 1.07 - 0.92 (m, 2H), 0.84 - 0.68 (m, 2H). |
| **P178** | 2-(6-(cyclopropanesulfonamido)pyridin-2-yl)-N-(4-(5-ethoxypyridin-3-yl)phenyl)-2-methylpropanamide 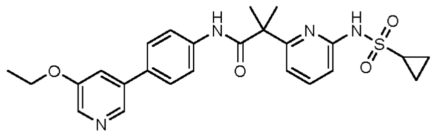 | Method 2 using INTC106 and INTD4, [HPLC acidic], 481, (1.83) | 10.61 (s, 1H), 9.37 (s, 1H), 8.52 - 8.37 (m, 1H), 8.30 - 8.16 (m, 1H), 7.78 - 7.65 (m, 5H), 7.59 - 7.55 (m, 1H), 7.15 - 7.07 (m, 1H), 6.84 - 6.78 (m, 1H), 4.24 - 4.14 (m, 2H), 3.22 - 3.08 (m, 1H), 1.61 (s, 6H), 1.43 - 1.32 (m, 3H), 1.05 - 0.95 (m, 2H), 0.84 - 0.71 (m, 2H). |
| **P179** | 2-(6-(cyclopropanesulfonamido)pyridin-2-yl)-2-methyl-N-(4-(pyridin-3-yl)phenyl)propanamide 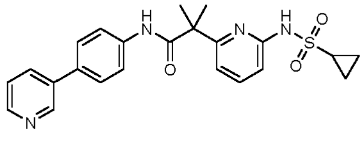 | Method 2 using INTC106 and commercial aniline, [UPLC acidic], 437, (0.92) | 10.62 (s, 1H), 9.36 (s, 1H), 8.92 - 8.81 (m, 1H), 8.53 (dd, J = 4.7, 1.6 Hz, 1H), 8.09 - 8.00 (m, 1H), 7.81 - 7.61 (m, 5H), 7.51 - 7.39 (m, 1H), 7.12 (d, J = 7.6 Hz, 1H), 6.82 (d, J = 8.0 Hz, 1H), 3.22 - 3.10 (m, 1H), 1.61 (s, 6H), 1.06 - 0.93 (m, 2H), 0.84 - 0.70 (m, 2H). |
| **P180** | 2-(6-(cyclopropanesulfonamido)pyridin-2-yl)-N-(2-fluoro-4-(pyrazin-2-yl)phenyl)-2-methylpropanamide 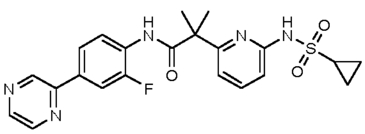 | Method 8 using INTC109 and INTD23, [HPLC basic], 456, (1.90) | 10.61 (s, 1H), 9.31 - 9.25 (m, 2H), 8.73 - 8.67 (m, 1H), 8.61 (d, J = 2.5 Hz, 1H), 8.03 - 7.93 (m, 2H), 7.79 - 7.69 (m, 2H), 7.13 (d, J = 7.6 Hz, 1H), 6.86 (d, J = 8.1 Hz, 1H), 3.20 - 3.08 (m, 1H), 1.61 (s, 6H), 1.10 - 0.96 (m, 2H), 0.99 - 0.87 (m, 2H). |

(continued)

| P | Name/Structure (All examples containing chiral centres are racemates unless stated) | Synthesis Method, [LCMS Method], m/z $(M+H)^+$, (RT/Min) | $^1$H NMR Chemical Shift Data (DMSO-d6 unless stated) |
|---|---|---|---|
| **P181** | 2-(6-(cyclopropanesulfonamido)pyridin-2-yl)-2-methyl-N-(4-(6-(trifluoromethyl)pyrazin-2-yl) phenyl)propanamide 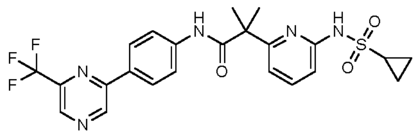 | Method 8 using INTC1 09 and INTD19, [UPLC basic], 506, (1.60) | 10.58 (s, 1H), 9.56 (s, 1H), 9.49 (s, 1H), 9.05 (s, 1H), 8.19 - 8.10 (m, 2H), 7.88 - 7.80 (m, 2H), 7.74 (t, J = 7.9 Hz, 1H), 7.12 (d, J = 7.7 Hz, 1H), 6.80 (d, J = 8.1 Hz, 1H), 3.21 - 3.10 (m, 1H), 1.60 (s, 6H), 1.09 - 0.91 (m, 2H), 0.79 - 0.69 (m, 2H). |
| **P182** | N-(4-(6-chloropyrazin-2-yl)phenyl)-2-(6-(cyclopropanesulfonamido)pyridin-2-yl)-2-methylpropanamide 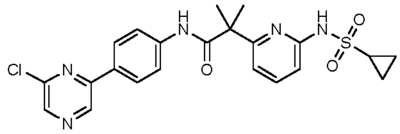 | Method 8 using INTC1 09 and INTD22, [UPLC basic], 472 35Cl isotope, (1.46) | 10.59 (s, 1H), 9.47 (s, 1H), 9.23 (s, 1H), 8.68 (s, 1H), 8.12 - 8.04 (m, 2H), 7.86 - 7.78 (m, 2H), 7.78 - 7.73 (m, 1H), 7.13 (d, J = 7.7 Hz, 1H), 6.81 (d, J = 8.1 Hz, 1H), 3.22 - 3.10 (m, 1H), 1.61 (s, 6H), 1.08 - 0.95 (m, 2H), 0.79 - 0.69 (m, 2H). |
| **P183** | 2-(6-(cyclopropanesulfonamido)pyridin-2-yl)-N-(4-(6-ethoxypyrazin-2-yl)phenyl)-2-methylpropanamide 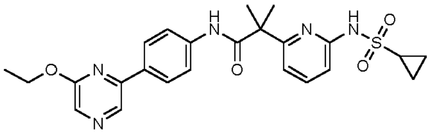 | Method 2 using INTC106 and INTD18, [HPLC acidic], 482, (2.36) | 10.60 (s, 1H), 9.43 (s, 1H), 8.76 (s, 1H), 8.17 (s, 1H), 8.12 - 8.00 (m, 2H), 7.84 - 7.66 (m, 3H), 7.13 (d, J = 7.7 Hz, 1H), 6.81 (d, J = 8.1 Hz, 1H), 4.54 - 4.39 (m, 2H), 3.20 - 3.12 (m, 1H), 1.61 (s, 6H), 1.47 - 1.32 (m, 3H), 1.04 - 0.95 (m, 2H), 0.81 - 0.71 (m, 2H). |
| **P184** | 2-(6-(cyclopropanesulfonamido)pyridin-2-yl)-N-(4-(6-methoxypyrazin-2-yl)phenyl)-2-methylpropanamide 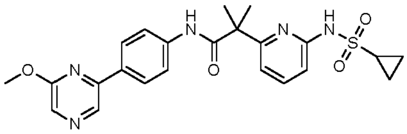 | Method 2 using INTC106 andINTD1, [HPLC acidic], 468, (2.24) | 10.60 (s, 1H), 9.43 (s, 1H), 8.78 (s, 1H), 8.20 (s, 1H), 8.11 - 8.04 (m, 2H), 7.83 - 7.70 (m, 3H), 7.13 (d, J = 7.7 Hz, 1H), 6.81 (d, J = 8.1 Hz, 1H), 4.01 (s, 3H), 3.23 - 3.11 (m, 1H), 1.61 (s, 6H), 1.05 - 0.95 (m, 2H), 0.83 - 0.72 (m, 2H). |
| **P185** | 2-(6-(cyclopropanesulfonamido)pyridin-2-yl)-2-methyl-N-(4-(pyrazin-2-yl)phenyl)propanamide 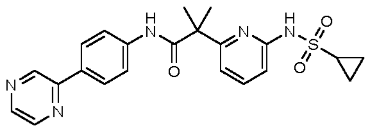 | Method 8 using INTC1 09 and commercial aniline, [UPLC acidic], 438, (1.97) | 10.59 (s, 1H), 9.45 (s, 1H), 9.21 (d, J = 1.6 Hz, 1H), 8.68 - 8.66 (m, 1H), 8.55 (d, J = 2.5 Hz, 1H), 8.10 - 8.06 (m, 2H), 7.83 - 7.78 (m, 2H), 7.78 - 7.66 (m, 1H), 7.18 - 7.05 (m, 1H), 6.87 - 6.69 (m, 1H), 3.20 - 3.12 (m, 1H), 1.61 (s, 6H), 1.04 - 0.92 (m, 2H), 0.78 - 0.62 (m, 2H). |

(continued)

| P | Name/Structure (All examples containing chiral centres are racemates unless stated) | Synthesis Method, [LCMS Method], m/z $(M+H)^+$, (RT/Min) | $^1H$ NMR Chemical Shift Data (DMSO-d6 unless stated) |
|---|---|---|---|
| **P186** | 4-(6-(cyclopropanesulfonamido)pyridin-2-yl)-N-(5-(6-ethoxypyrazin-2-yl)pyridin-2-yl) tetrahydro-2H-pyran-4-carboxamide 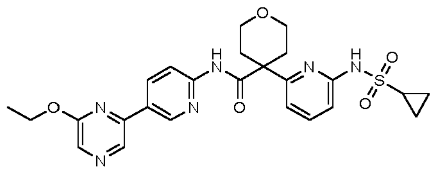 | Method 2 using INTC1 08 and INTD33, [HPLC acidic], 525, (2.23) | 10.62 (s, 1H), 9.75 (s, 1H), 9.00 (d, J = 2.4 Hz, 1H), 8.83 (s, 1H), 8.49 (dd, J = 8.8, 2.4 Hz, 1H), 8.25 (s, 1H), 8.17 (d, J = 8.8 Hz, 1H), 7.79 - 7.76 (m, 1H), 7.19 (d, J = 7.7 Hz, 1H), 6.85 (d, J = 8.1 Hz, 1H), 4.47 (q, J = 7.1 Hz, 2H), 3.74 - 3.60 (m, 4H), 3.24-3.16 (m, 1H), 2.53-2.46 (m, 2H, obscured by DMSO), 2.27-2.19 (m, 2H), 1.40 (t, J = 7.1 Hz, 3H), 1.06-1.01 (m, 2H), 0.94 - 0.84 (m, 2H). |
| **P187** | 2-(6-(cyclopropanesulfonamido)pyridin-2-yl)-N-(5-(6-(trifluoromethyl)pyrazin-2-yl)pyridin-2-yl)butanamide 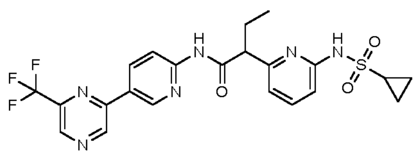 | Method 10 using INTC111 and INTD2, [HPLC acidic], 507, (2.37) | 10.98 (s, 1H), 10.58 (s, 1H), 9.65 (s, 1H), 9.17 - 9.10 (m, 2H), 8.56 (dd, J = 8.8, 2.5 Hz, 1H), 8.27 (d, J = 8.8 Hz, 1H), 7.70 - 7.67 (m, 1H), 7.09 (d, J = 7.6 Hz, 1H), 6.84 (d, J = 8.1 Hz, 1H), 3.98 (dd, J = 8.5, 6.5 Hz, 1H), 3.29 - 3.24 (m, 1H), 2.14 - 2.00 (m, 1H), 1.99 - 1.85 (m, 1H), 1.11 - 0.98 (m, 2H), 0.97 - 0.78 (m, 5H). |
| **P188** | 2-(6-(cyclopropanesulfonamido)pyridin-2-yl)-N-(5-(6-ethoxypyrazin-2-yl)pyridin-2-yl) butanamide 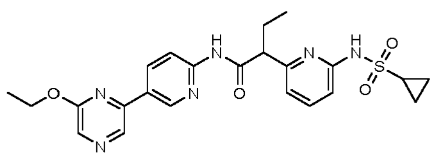 | Method 10 using INTC111 and INTD33, [HPLC acidic], 483, (2.32) | 10.86 (s, 1H), 10.48 (s, 1H), 9.05 (d, J = 2.5 Hz, 1H), 8.84 (s, 1H), 8.48 (dd, J = 8.8, 2.5 Hz, 1H), 8.25 (s, 1H), 8.21 (d, J = 8.8 Hz, 1H), 7.72 - 7.68 (m, 1H), 7.12 (d, J = 7.5 Hz, 1H), 6.83 (d, J = 8.1 Hz, 1H), 4.48 (q, J = 7.0 Hz, 2H), 4.00 - 3.96 (m, 1H), 3.31 - 3.26 (m, 1H), 2.12 - 2.02 (m, 1H), 1.97 - 1.86 (m, 1H), 1.40 (t, J = 7.0 Hz, 3H), 1.12 - 1.00 (m, 2H), 0.98 - 0.82 (m, 5H). |
| **P189** | N-(4-(5-chloropyridin-3-yl)phenyl)-2-(6-(cyclopropanesulfonamido)pyridin-2-yl) butanamide 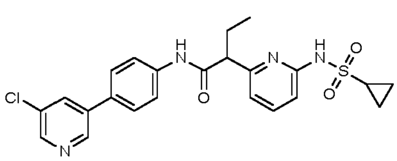 | Method 10 using INTC111 and INTD8, [HPLC acidic], 471 35Cl isotope, (2.26) | 10.55 (s, 1H), 10.23 (s, 1H), 8.86 (d, J = 2.0 Hz, 1H), 8.58 (d, J = 2.3 Hz, 1H), 8.22 - 8.21 (m, 1H), 7.80 - 7.65 (m, 5H), 7.10 (d, J = 7.5 Hz, 1H), 6.84 (d, J = 8.1 Hz, 1H), 3.73 (dd, J = 8.7, 6.4 Hz, 1H), 3.29 - 3.20 (m, 1H), 2.13 - 2.00 (m, 1H), 1.97 - 1.85 (m, 1H), 1.12 - 1.01 (m, 2H), 0.98 - 0.80 (m, 5H). |

(continued)

| P | Name/Structure (All examples containing chiral centres are racemates unless stated) | Synthesis Method, [LCMS Method], m/z $(M+H)^+$, (RT/Min) | $^1H$ NMR Chemical Shift Data (DMSO-d6 unless stated) |
|---|---|---|---|
| **P190** | 2-(6-(cyclopropanesulfonamido)pyridin-2-yl)-N-(4-(6-ethoxypyrazin-2-yl)-2-fluorophenyl) butanamide 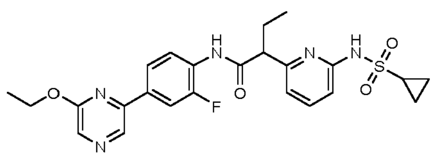 | Method 10 using INTC111 and INTD24, [HPLC acidic], 500, (2.39) | 10.53 (s, 1H), 10.08 (s, 1H), 8.83 (s, 1H), 8.24 (s, 1H), 8.07 - 7.98 (m, 2H), 7.95 (dd, J = 8.6, 2.0 Hz, 1H), 7.73 - 7.70 (m, 1H), 7.11 (d, J = 7.6 Hz, 1H), 6.85 (d, J = 8.1 Hz, 1H), 4.48 (q, J = 7.0 Hz, 2H), 3.96 - 3.93 (m, 1H), 3.30 - 3.22 (m, 1H), 2.11 - 2.01 (m, 1H), 1.97 - 1.89 (m, 1H), 1.40 (t, J = 7.0 Hz, 3H), 1.11 - 1.05 (m, 2H), 1.01 - 0.87 (m, 5H). |
| **P191** | 2-(6-(cyclopropanesulfonamido)pyridin-2-yl)-N-(4-(6-ethoxypyrazin-2-yl)phenyl) butanamide 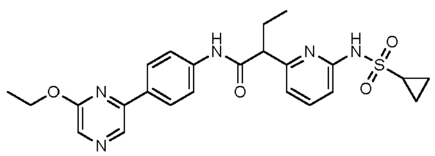 | Method 10 using INTC111 and INTD18, [HPLC acidic], 482, (2.36) | 10.54 (s, 1H), 10.27 (s, 1H), 8.76 (s, 1H), 8.18 (s, 1H), 8.12 - 8.05 (m, 2H), 7.79 - 7.74 (m, 2H), 7.74 - 7.68 (m, 1H), 7.10 (d, J = 7.6 Hz, 1H), 6.84 (d, J = 8.2 Hz, 1H), 4.47 (q, J = 7.0 Hz, 2H), 3.75 (dd, J = 8.6, 6.5 Hz, 1H), 3.29 - 3.22 (m, 1H), 2.11 - 2.03 (m, 1H), 1.96 - 1.87 (m, 1H), 1.40 (t, J = 7.0 Hz, 3H), 1.10 - 1.02 (m, 2H), 0.96 - 0.85 (m, 5H). |
| **P192** | 2-(6-(cyclopropanesulfonamido)pyrazin-2-yl)-N-(4-(pyridin-3-yl)phenyl)acetamide 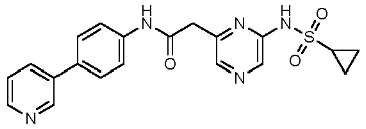 | Method 7 using INTC135, [UPLC acidic], 410, (0.64) | 11.01 (s, 1H), 10.40 (s, 1H), 8.88 (dd, J = 2.5, 0.9 Hz, 1H), 8.53 (dd, J = 4.8, 1.6 Hz, 1H), 8.27 (s, 1H), 8.20 (s, 1H), 8.05 (ddd, J = 8.0, 2.5, 1.6 Hz, 1H), 7.76 - 7.67 (m, 4H), 7.46 (ddd, J = 8.0, 4.8, 0.9 Hz, 1H), 3.88 (s, 2H), 3.12 - 2.99 (m, 1H), 1.11 -1.03 (m, 2H), 0.96 - 0.74 (m, 2H). |
| **P193** | 2-(6-(ethylsulfonamido)pyrazin-2-yl)-N-(4-(pyridin-3-yl)phenyl)acetamide 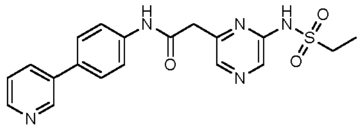 | Method 7 using INTC135, [UPLC acidic], 398, (0.55) | 10.92 (v. br. s, 1H), 10.38 (s, 1H), 8.88 (dd, J = 2.5, 0.9 Hz, 1H), 8.53 (dd, J = 4.7, 1.6 Hz, 1H), 8.20 (s, 1H), 8.13 (s, 1H), 8.05 (ddd, J = 8.0, 2.5, 1.6 Hz, 1H), 7.79 - 7.63 (m, 4H), 7.46 (ddd, J = 8.0, 4.8, 0.9 Hz, 1H), 3.84 (s, 2H), 3.42 (q, J = 7.3 Hz, 2H), 1.14 (t, J = 7.3 Hz, 3H). |
| **P194** | 2-(6-(methylsulfonamido)pyrazin-2-yl)-N-(4-(pyridin-3-yl)phenyl)acetamide 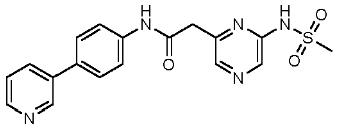 | Method 7 using INTC135, [UPLC acidic], 384, (0.55) | 11.05 (s, 1H), 10.40 (s, 1H), 8.89 (d, J = 2.4 Hz, 1H), 8.54 (dd, J = 4.7, 1.6 Hz, 1H), 8.29 (s, 1H), 8.19 (s, 1H), 8.05 (ddd, J = 8.0, 2.4, 1.6 Hz, 1H), 7.78 - 7.64 (m, 4H), 7.47 (ddd, J = 8.0, 4.7, 0.9 Hz, 1H), 3.90 (s, 2H), 3.33 (s, 3H). |

(continued)

| P | Name/Structure (All examples containing chiral centres are racemates unless stated) | Synthesis Method, [LCMS Method], m/z (M+H)+, (RT/Min) | 1H NMR Chemical Shift Data (DMSO-d6 unless stated) |
|---|---|---|---|
| **P195** | 2-(6-(cyclopropanesulfonamido)pyrazin-2-yl)-N-(5-(6-ethoxypyrazin-2-yl)pyridin-2-yl)-2-methylpropanamide 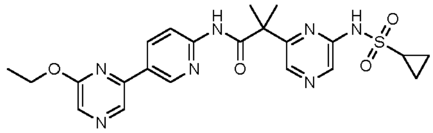 | Method 2 using INTC125 and INTD33, [HPLC acidic], 484, (2.15) | 10.97 (s, 1H), 10.13 (s, 1H), 9.00 (d, J = 2.5 Hz, 1H), 8.84 (s, 1H), 8.49 (dd, J = 8.8, 2.5 Hz, 1H), 8.41 (s, 1H), 8.25 (s, 1H), 8.23 - 8.17 (m, 2H), 4.48 (q, J = 7.0 Hz, 2H), 3.02-2.95 (m, 1H), 1.66 (s, 6H), 1.40 (t, J = 7.0 Hz, 3H), 1.04-0.95 (m, 2H), 0.75 - 0.66 (m, 2H). |
| **P196** | 2-(6-(cyclopropanesulfonamido)pyrazin-2-yl)-N-(4-(6-ethoxypyrazin-2-yl)phenyl)-2-methylpropanamide 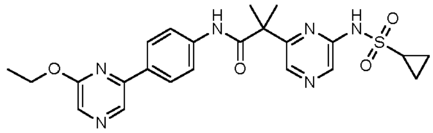 | Method 2 using INTC125 and INTD18, [HPLC acidic], 483, (2.2) | 11.03 (s, 1H), 9.43 (s, 1H), 8.76 (s, 1H), 8.42 (s, 1H), 8.18 (d, J = 7.7 Hz, 2H), 8.11 - 8.02 (m, 2H), 7.81 - 7.71 (m, 2H), 4.47 (q, J = 7.0 Hz, 2H), 3.00-3.00 (m, 1H), 1.65 (s, 6H), 1.40 (t, J = 7.0 Hz, 3H), 1.06-0.97 (m, 2H), 0.81 - 0.66 (m, 2H). |
| **P197** | 4-(6-(cyclopropanesulfonamido)pyrazin-2-yl)-N-(5-(6-ethoxypyrazin-2-yl)pyridin-2-yl) tetrahydro-2H-pyran-4-carboxamide 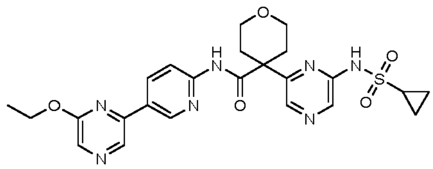 | Method 2 using INTC127 and INTD33, [UPLC acidic], 526, (1.31) | 11.06 (s, 1H), 10.14 (s, 1H), 9.01 (d, J = 2.5 Hz, 1H), 8.84 (s, 1H), 8.49 (dd, J = 8.7, 2.5 Hz, 1H), 8.45 (s, 1H), 8.25 (s, 1H), 8.22 - 8.16 (m, 2H), 4.47 (q, J = 7.0 Hz, 2H), 3.79 - 3.72 (m, 2H), 3.68 - 3.60 (m, 2H), 3.15 - 3.05 (m, 1H), 2.56 - 2.52 (m, 2H), 2.27 - 2.17 (m, 2H), 1.39 (t, J = 7.0 Hz, 3H), 1.08 - 1.02 (m, 2H), 0.88 - 0.80 (m, 2H). |
| **P198** | 2-(6-(cyclopropanesulfonamido)pyrazin-2-yl)-N-(5-(6-ethoxypyrazin-2-yl)pyridin-2-yl)-4-methoxy-2-methylbutanamide 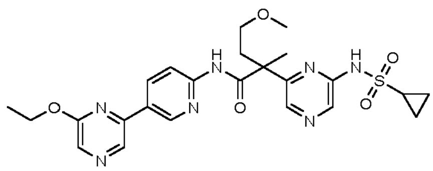 | Method 7 using INTC136, [UPLC acidic], 528, (1.36) | 10.98 (s, 1H), 10.18 (s, 1H), 9.00 (d, J = 2.4 Hz, 1H), 8.84 (s, 1H), 8.49 (dd, J = 8.8, 2.4 Hz, 1H), 8.38 (s, 1H), 8.25 (s, 1H), 8.23 - 8.16 (m, 2H), 4.48 (q, J = 7.0 Hz, 2H), 3.43 - 3.27 (m, 2H), 3.14 (s, 3H), 3.03 - 2.94 (m, 1H), 2.48 - 2.38 (m, 1H), 2.39 - 2.29 (m, 1H), 1.67 (s, 3H), 1.40 (t, J = 7.0 Hz, 3H), 1.04 - 0.94 (m, 2H), 0.73 - 0.64 (m, 2H). |
| **P199** | N-(5-(6-ethoxypyrazin-2-yl)pyridin-2-yl)-4-methoxy-2-methyl-2-(6-(methylsulfonamido) pyrazin-2-yl)butanamide 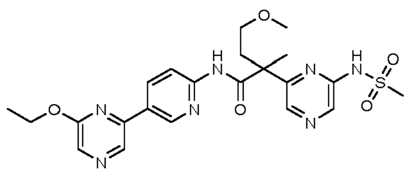 | Method 7 using INTC136, [HPLC acidic], 502, (1.98) | 11.03 (s, 1H), 10.16 (s, 1H), 9.06 - 8.99 (m, 1H), 8.86 - 8.82 (m, 1H), 8.52 - 8.45 (m, 1H), 8.35 (s, 1H), 8.27 - 8.23 (m, 1H), 8.22 - 8.14 (m, 2H), 4.48 (q, J = 7.0 Hz, 2H), 3.42 - 3.27 (m, 5H), 3.17 - 3.12 (m, 3H), 2.49 - 2.40 (m, 1H), 2.37 - 2.29 (m, 1H), 1.68 - 1.62 (m, 3H), 1.40 (t, J = 7.0 Hz, 3H). |

(continued)

| P | Name/Structure (All examples containing chiral centres are racemates unless stated) | Synthesis Method, [LCMS Method], m/z (M+H)+, (RT/Min) | $^1$H NMR Chemical Shift Data (DMSO-d6 unless stated) |
|---|---|---|---|
| **P200** | 2-(6-(cyclopropanesulfonamido)pyrazin-2-yl)-N-(5-(6-ethoxypyrazin-2-yl)pyridin-2-yl)-2-fluorobutanamide 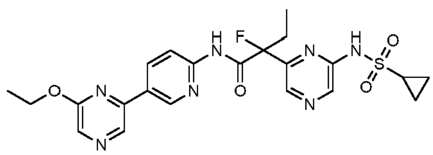 | Method 10 using INTC133 and INTD33, [UPLC acidic], 502, (1.47) | 11.25 (s, 1H), 10.62 (d, J = 2.4 Hz, 1H), 9.10 (d, J = 2.4 Hz, 1H), 8.87 (s, 1H), 8.65 (s, 1H), 8.53 (dd, J = 8.7, 2.4 Hz, 1H), 8.34 (s, 1H), 8.27 (s, 1H), 8.12 (d, J = 8.7 Hz, 1H), 4.49 (q, J = 7.0 Hz, 2H), 3.20-3.12 (m, 1H), 2.48 - 2.36 (m, 2H), 1.40 (t, J = 7.0 Hz, 3H), 1.23 - 0.89 (m, 7H). |
| **P201** | 2-(6-(cyclopropanesulfonamido)pyrazin-2-yl)-N-(5-(6-ethoxypyrazin-2-yl)pyridin-2-yl) butanamide 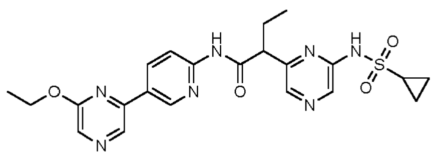 | Method 10 using INTC132 and INTD33, [HPLC acidic], 484, (2.15) | 11.00 (s, 2H), 9.06 (d, J = 2.4 Hz, 1H), 8.84 (s, 1H), 8.49 (dd, J = 8.8, 2.4 Hz, 1H), 8.38 (s, 1H), 8.25 (s, 1H), 8.22 - 8.19 (m, 2H), 4.48 (q, J = 7.0 Hz, 2H), 4.07 (t, J = 7.5 Hz, 1H), 3.23-3.13 (m, 1H), 2.19-2.08 (m, 1H), 2.01-1.95 (m, 1H), 1.40 (t, J = 7.0 Hz, 3H), 1.18 -1.05 (m, 2H), 1.02 - 0.87 (m, 5H). |
| **P202** | 2-(6-(cyclopropanesulfonamido)pyrazin-2-yl)-N-(4-(6-ethoxypyrazin-2-yl)phenyl) butanamide 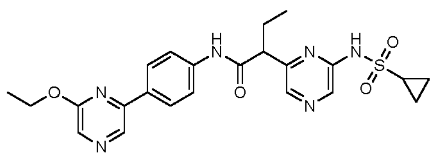 | Method 10 using INTC132 and INTD18, [HPLC acidic], 483, (2.22) | 11.02 (s, 1H), 10.37 (s, 1H), 8.76 (s, 1H), 8.37 (s, 1H), 8.19 (d, J = 11.0 Hz, 2H), 8.15 - 8.04 (m, 2H), 7.81 - 7.72 (m, 2H), 4.47 (q, J = 7.0 Hz, 2H), 3.85 (dd, J = 8.4, 6.6 Hz, 1H), 3.21-3.12 (m, 1H), 2.20 - 2.07 (m, 1H), 2.03 - 1.90 (m, 1H), 1.40 (t, J = 7.0 Hz, 3H), 1.15 - 1.04 (m, 2H), 1.02 - 0.84 (m, 5H). |
| **P203** | 2-(6-(cyclopropanesulfonamido)pyrazin-2-yl)-N-(5-(6-ethoxypyrazin-2-yl)pyridin-2-yl)-2-methoxyacetamide 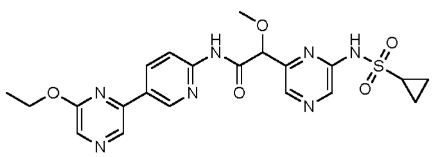 | Method 7 using INTC137 [HPLC acidic], 486, (1.98) | 11.12 (s, 1H), 10.71 (s, 1H), 9.10 (dd, J = 2.4, 0.8 Hz, 1H), 8.86 (s, 1H), 8.53 (dd, J = 8.7, 2.4 Hz, 1H), 8.45 (s, 1H), 8.30 (s, 1H), 8.27 (s, 1H), 8.18 (d, J = 8.7 Hz, 1H), 5.27 (s, 1H), 4.49 (q, J = 7.0 Hz, 2H), 3.48 (s, 3H), 3.12-3.02 (m, 1H), 1.40 (t, J = 7.0 Hz, 3H), 1.12 - 0.96 (m, 2H), 0.93 - 0.73 (m, 2H). |
| **P204** | 2-(6-(cyclopropanesulfonamido)pyrazin-2-yl)-N-(4-(6-ethoxypyrazin-2-yl)phenyl)-2-methoxyacetamide 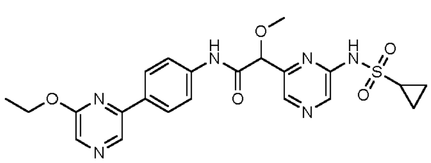 | Method 10 using INTC134 and INTD18, HPLC acidic], 485, 2.05) | 11.13 (s, 1H), 10.38 (s, 1H), 8.78 (s, 1H), 8.46 (s, 1H), 8.29 (s, 1H), 8.19 (s, 1H), 8.14 - 8.07 (m, 2H), 7.89 - 7.81 (m, 2H), 5.08 (s, 1H), 4.48 (q, J = 7.0 Hz, 2H), 3.48 (s, 3H), 3.13-3.03 (m, 1H), 1.40 (t, J = 7.0 Hz, 3H), 1.11-1.00 (m, 2H), 0.87 - 0.73 (m, 2H). |

(continued)

| P | Name/Structure (All examples containing chiral centres are racemates unless stated) | Synthesis Method, [LCMS Method], m/z $(M+H)^+$, (RT/Min) | $^1$H NMR Chemical Shift Data (DMSO-d6 unless stated) |
|---|---|---|---|
| **P205** | 2-(6-(cyclopropanesulfonamido)pyrazin-2-yl)-N-(5-(6-ethoxypyrazin-2-yl)pyridin-2-yl)-2-methoxypropanamide 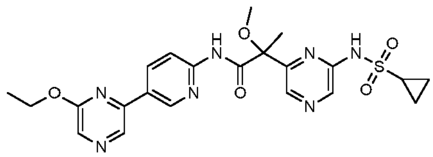 | Method 2 using INTC129 and INTD33, [HPLC acidic], 500, (2.16) | 11.13 (s, 1H), 9.90 (s, 1H), 9.08 (dd, J = 2.4, 0.8 Hz, 1H), 8.86 (s, 1H), 8.58 (s, 1H), 8.53 (dd, J = 8.8, 2.4 Hz, 1H), 8.27 (s, 1H), 8.26 (s, 1H), 8.13 (dd, J = 8.8, 0.8 Hz, 1H), 4.49 (q, J = 7.1 Hz, 2H), 3.31 (s, 3H), 3.12-3.05 (m, 1H), 1.85 (s, 3H), 1.40 (t, J = 7.0 Hz, 3H), 1.15-1.04 (m, 2H), 0.98 - 0.83 (m, 2H). |
| **P205a** | Single enantiomer - stereochemistry unassigned 2-(6-(cyclopropanesulfonamido)pyrazin-2-yl)-N-(5-(6-ethoxypyrazin-2-yl)pyridin-2-yl)-2-methoxypropanamide 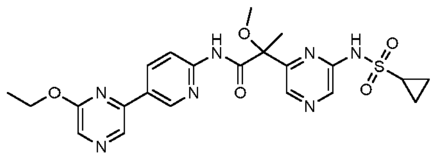 | Method 2 using INTC129 and INTD33, Chiral IC4 (4.40), [HPLC acidic], 500, (2.16) | 11.13 (s, 1H), 9.90 (s, 1H), 9.08 (dd, J = 2.4, 0.8 Hz, 1H), 8.86 (s, 1H), 8.58 (s, 1H), 8.53 (dd, J = 8.8, 2.4 Hz, 1H), 8.27 (s, 1H), 8.26 (s, 1H), 8.13 (dd, J = 8.8, 0.8 Hz, 1H), 4.49 (q, J = 7.1 Hz, 2H), 3.31 (s, 3H), 3.12-3.05 (m, 1H), 1.85 (s, 3H), 1.40 (t, J = 7.0 Hz, 3H), 1.15-1.04 (m, 2H), 0.98 - 0.83 (m, 2H). |
| **P205b** | Single enantiomer - stereochemistry unassigned 2-(6-(cyclopropanesulfonamido)pyrazin-2-yl)-N-(5-(6-ethoxypyrazin-2-yl)pyridin-2-yl)-2-methoxypropanamide 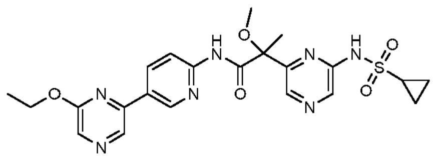 | Method 2 using INTC129 and INTD33, Chiral IC4 (5.04), [HPLC acidic], 500, (2.16) | 11.13 (s, 1H), 9.90 (s, 1H), 9.08 (dd, J = 2.4, 0.8 Hz, 1H), 8.86 (s, 1H), 8.58 (s, 1H), 8.53 (dd, J = 8.8, 2.4 Hz, 1H), 8.27 (s, 1H), 8.26 (s, 1H), 8.13 (dd, J = 8.8, 0.8 Hz, 1H), 4.49 (q, J = 7.1 Hz, 2H), 3.31 (s, 3H), 3.12-3.05 (m, 1H), 1.85 (s, 3H), 1.40 (t, J = 7.0 Hz, 3H), 1.15-1.04 (m, 2H), 0.98 - 0.83 (m, 2H). |
| **P206** | Single enantiomer - stereochemistry unassigned 2-(6-(cyclopropanesulfonamido)pyrazin-2-yl)-N-(5-(6-ethoxypyrazin-2-yl)pyridin-2-yl)-2-fluorobutanamide 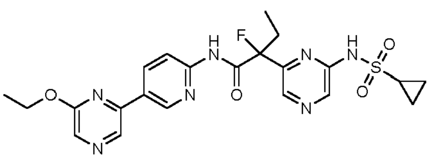 | Method 10 using INTC133 and INTD33, Chiral IC5 (12.55), [HPLC acidic], 502, (2.27) | 11.25 (s, 1H), 10.61 (d, J = 2.4 Hz, 1H), 9.10 (d, J = 2.4 Hz, 1H), 8.87 (s, 1H), 8.64 (s, 1H), 8.53 (dd, J = 8.7, 2.4 Hz, 1H), 8.34 (s, 1H), 8.27 (s, 1H), 8.12 (d, J = 8.7 Hz, 1H), 4.49 (q, J = 7.0 Hz, 2H), 3.19-3.10 (m, 1H), 2.58 - 2.34 (m, 2H), 1.40 (t, J = 7.1 Hz, 3H), 1.23 - 0.91 (m, 7H). |

(continued)

| P | Name/Structure (All examples containing chiral centres are racemates unless stated) | Synthesis Method, [LCMS Method], m/z $(M+H)^+$, (RT/Min) | $^1H$ NMR Chemical Shift Data (DMSO-d6 unless stated) |
|---|---|---|---|
| **P207** | Single enantiomer - stereochemistry unassigned 2-(6-(cyclopropanesulfonamido)pyrazin-2-yl)-N-(5-(6-ethoxypyrazin-2-yl)pyridin-2-yl)-2-fluorobutanamide 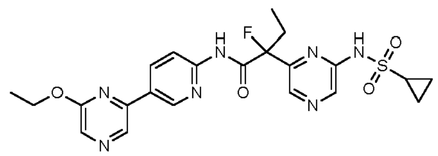 | Method 10 using INTC133 and INTD33, Chiral IC5 (19.98), [HPLC acidic], 502, (2.27) | 11.25 (s, 1H), 10.61 (d, J = 2.4 Hz, 1H), 9.10 (d, J = 2.4 Hz, 1H), 8.87 (s, 1H), 8.64 (s, 1H), 8.53 (dd, J = 8.7, 2.4 Hz, 1H), 8.33 (s, 1H), 8.27 (s, 1H), 8.12 (d, J = 8.7 Hz, 1H), 4.49 (q, J = 7.1 Hz, 2H), 3.19-3.10 (m, 1H), 2.58 - 2.34 (m, 2H), 1.40 (t, J = 7.0 Hz, 3H), 1.20 - 0.90 (m, 7H). |
| **P208** | 2-(4-(cyclopropanesulfonamido)pyrimidin-2-yl)-N-(5-(6-ethoxypyrazin-2-yl)pyridin-2-yl) butanamide 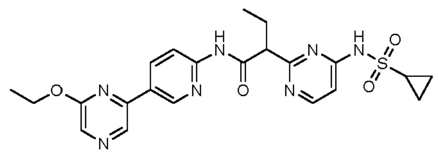 | Method 6 using INTC143, [UPLC acidic], 484, (1.32) | 11.24 (s, 1H), 10.93 (s, 1H), 9.07 (d, J = 2.4 Hz, 1H), 8.85 (s, 1H), 8.49 (dd, J = 8.7, 2.4 Hz, 2H), 8.25 (s, 1H), 8.23 (d, J = 8.7 Hz, 1H), 6.84 (s, 1H), 4.49 (q, J = 7.0 Hz, 2H), 4.15 - 4.06 (m, 1H), 3.26 - 3.13 (m, 1H), 2.17 - 2.00 (m, 2H), 1.41 (t, J = 7.0 Hz, 3H), 1.10 - 1.04 (m, 2H), 0.96 (t, J = 7.4 Hz, 3H), 0.94 - 0.88 (m, 2H). |
| **P209** | N-(1-(2-(cyclopropanesulfonamido)pyrimidin-4-yl)cyclopropyl)-4-(6-ethoxypyrazin-2-yl)-2-fluorobenzamide 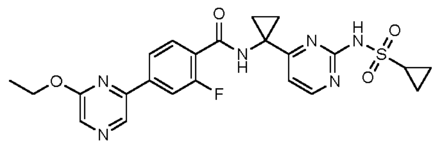 | Method 1 using INTC156 and INTD74, [UPLC acidic], 499, (1.32) | 11.16 (s, 1H), 9.29 (s, 1H), 8.94 (s, 1H), 8.47 (d, J = 4.7 Hz, 1H), 8.33 (s, 1H), 8.09 (s, 1H), 8.07 (dd, J = 4.7, 1.5 Hz, 1H), 7.85-7.81 (m, 1H), 7.16 - 7.09 (m, 1H), 4.51 (q, J = 7.0 Hz, 2H), 3.16 - 3.08 (m, 1H), 1.66 (q, J = 4.3 Hz, 2H), 1.41 (t, J = 7.0 Hz, 3H), 1.40 - 1.36 (m, 2H), 1.12 - 1.00 (m, 4H). |
| **P210** | N-(1-(2-(cyclopropanesulfonamido)pyrimidin-4-yl)propyl)-5-(6-ethoxypyrazin-2-yl)picolinamide 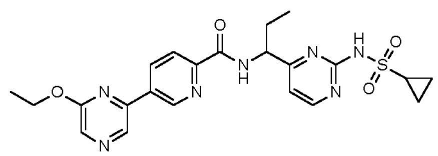 | Method 7 using INTC163, [UPLC acidic], 484, (1.36) | 11.31 (s, 1H), 9.38 (d, J = 2.2 Hz, 1H), 9.19 (d, J = 8.4 Hz, 1H), 9.00 (s, 1H), 8.74 - 8.65 (m, 1H), 8.56 (d, J = 5.1 Hz, 1H), 8.38 (s, 1H), 8.17 (d, J = 8.4 Hz, 1H), 7.20 (d, J = 5.1 Hz, 1H), 5.03 - 4.94 (m, 1H), 4.52 (q, J = 7.0 Hz, 2H), 3.32 - 3.24 (m, 1H), 2.07 - 1.87 (m, 2H), 1.43 (t, J = 7.0 Hz, 3H), 1.18 - 0.89 (m, 7H). |

(continued)

| P | Name/Structure (All examples containing chiral centres are racemates unless stated) | Synthesis Method, [LCMS Method], m/z $(M+H)^+$, (RT/Min) | $^1$H NMR Chemical Shift Data (DMSO-d6 unless stated) |
|---|---|---|---|
| **P211** | N-(1-(2-(cyclopropanesulfonamido)pyrimidin-4-yl)propyl)-2-fluoro-4-(5-(trifluoromethyl)pyridin-3-yl)benzamide 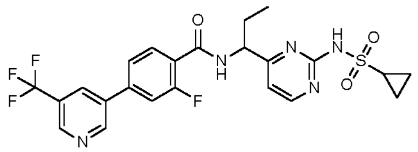 | Method 1 using INTC162 and INTD78, [HPLC Basic], 524, (1.8) | Methanol-d4, 9.22 - 9.14 (m, 1H), 8.99 - 8.92 (m, 1H), 8.51 - 8.46 (m, 2H), 7.92 - 7.89 (m, 1H), 7.76 - 7.69 (m, 2H), 7.07 (d, J = 5.2 Hz, 1H), 5.11 - 4.99 (m, 1H), 3.32 - 3.23 (m, 1H), 2.14 - 2.01 (m, 1H), 2.00 - 1.84 (m, 1H), 1.31 - 1.20 (m, 2H), 1.08 (t, J = 7.4 Hz, 3H), 1.05 - 0.93 (m, 2H), 2 x N-H not observed. |
| **P212** | 4-(5-chloropyridin-3-yl)-N-(1-(2-(cyclopropanesulfonamido)pyrimidin-4-yl)propyl)-2-fluorobenzamide 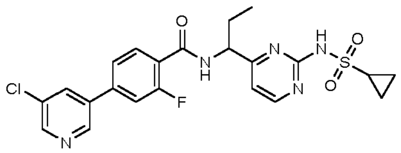 | Method 1 INTC162 and INTD76 [HPLC Basic], 490 $^{35}$Cl isotope, (1.68) | Methanol-d4, 8.90 - 8.78 (m, 1H), 8.70 - 8.60 (m, 1H), 8.55 - 8.45 (m, 1H), 8.27 - 8.23 (m, 1H), 7.91 - 7.83 (m, 1H), 7.70 - 7.63 (m, 2H), 7.14 - 7.03 (m, 1H), 5.09 -5.01 (m, 1H), 3.30 - 3.23 (m, 1H), 2.16 - 2.02 (m, 1H), 2.02 - 1.85 (m, 1H), 1.33 - 1.22 (m, 2H), 1.07 (t, J = 7.3 Hz, 3H), 1.04 - 0.95 (m, 2H), 2 x N-H not observed. |
| **P213** | N-(1-(2-(cyclopropanesulfonamido)pyrimidin-4-yl)propyl)-4-(5-(trifluoromethyl)pyridin-3-yl) benzamide 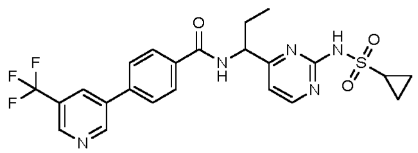 | Method 1 using INTC162 and INTD77, [HPLC Basic], 506, (1.76) | Methanol-d4, 9.17 (d, J = 2.1 Hz, 1H), 8.96 - 8.92 (m, 1H), 8.50 (d, J = 5.2 Hz, 1H), 8.47 - 8.44 (m, 1H), 8.11 - 8.07 (m, 2H), 7.93 - 7.88 (m, 2H), 7.11 (d, J = 5.2 Hz, 1H), 5.10 - 4.98 (m, 1H), 3.31 - 3.22 (m, 1H), 2.17 - 2.06 (m, 1H), 2.03 - 1.91 (m, 1H), 1.34 - 1.18 (m, 2H), 1.08 (t, J = 7.4 Hz, 3H), 1.03 - 0.88 (m, 2H), 2 x N-H not observed. |
| **P214** | 4-(5-chloropyridin-3-yl)-N-(1-(2-(cyclopropanesulfonamido)pyrimidin-4-yl)propyl)benzamide 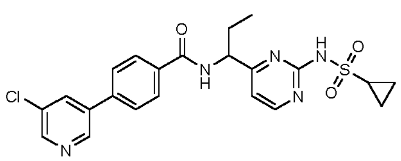 | Method 1 INTC162 and INTD79 [HPLC Basic], 472 $^{35}$Cl isotope, (1.64) | 11.25 (s, 1H), 8.98 - 8.95 (m, 1H), 8.92 - 8.86 (m, 1H), 8.68 (d, J = 2.3 Hz, 1H), 8.59 - 8.48 (m, 1H), 8.36 (t, J = 2.2 Hz, 1H), 8.10 - 8.02 (m, 2H), 7.98 -7.90 (m, 2H), 7.11 (s, 1H), 4.96 - 4.82 (m, 1H), 3.30 - 3.23 (m, 1H), 2.06 - 1.78 (m, 2H), 1.19 - 0.68 (m, 7H). |

(continued)

| P | Name/Structure (All examples containing chiral centres are racemates unless stated) | Synthesis Method, [LCMS Method], m/z $(M+H)^+$, (RT/Min) | $^1H$ NMR Chemical Shift Data (DMSO-d6 unless stated) |
|---|---|---|---|
| **P215** | N-(1-(2-(cyclopropanesulfonamido)pyrimidin-4-yl)propyl)-4-(6-ethoxypyrazin-2-yl)-2-(trifluoromethyl)benzamide 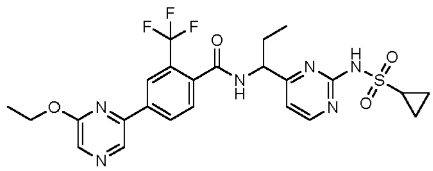 | Method 7 using INTC164, [UPLC acidic], 551, (1.44) | 11.28 (s, 1H), 9.12 (d, J = 7.9 Hz, 1H), 9.00 (s, 1H), 8.61 (d, J = 5.2 Hz, 1H), 8.53 - 8.44 (m, 2H), 8.36 (s, 1H), 7.84 (d, J = 7.9 Hz, 1H), 7.17 (d, J = 5.2 Hz, 1H), 4.88 - 4.80 (m, 1H), 4.51 (q, J = 7.1 Hz, 2H), 3.37 - 3.33 (m, 1H), 1.99 - 1.87 (m, 1H), 1.83 - 1.70 (m, 1H), 1.43 (t, J = 7.0 Hz, 3H), 1.21 - 1.03 (m, 4H), 1.00 (t, J = 7.3 Hz, 3H). |
| **P216** | N-(1-(2-(cyclopropanesulfonamido)pyrimidin-4-yl)propyl)-4-(6-ethoxypyrazin-2-yl)-2-fluorobenzamide 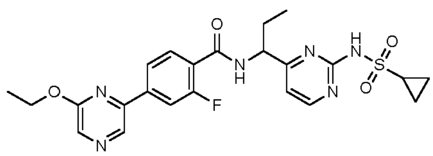 | Method 7 using INTC165, [HPLC acidic], 501, (2.18) | 11.25 (s, 1H), 8.93 (s, 1H), 8.89 (d, J = 7.8 Hz, 1H), 8.58 (d, J = 5.1 Hz, 1H), 8.32 (s, 1H), 8.09 - 8.06 (m, 2H), 7.80 - 7.77 (m, 1H), 7.16 (d, J = 5.2 Hz, 1H), 4.89 - 4.81 (m, 1H), 4.50 (q, J = 7.0 Hz, 2H), 3.30 - 3.25 (m, 1H), 1.99 - 1.90 (m, 1H), 1.84 - 1.74 (m, 1H), 1.41 (t, J = 7.0 Hz, 3H), 1.15 - 1.09 (m, 2H), 1.06 - 1.01 (m, 2H), 0.99 (t, J = 7.3 Hz, 3H). |
| **P217** | N-(1-(2-(cyclopropanesulfonamido)pyrimidin-4-yl)propyl)-4-(6-(trifluoromethyl)pyrazin-2-yl) benzamide 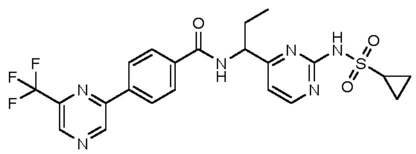 | Method 7 using INTC166, [HPLC acidic], 507, (1.37) | 11.25 (s, 1H), 9.72 (s, 1H), 9.21 (s, 1H), 8.98 (d, J = 7.6 Hz, 1H), 8.56 (d, J = 5.2 Hz, 1H), 8.35 - 8.31 (m, 2H), 8.16 - 8.11 (m, 2H), 7.16 (d, J = 5.2 Hz, 1H), 4.95 - 4.84 (m, 1H), 3.32 - 3.24 (m, 1H), 2.05 - 1.94 (m, 1H), 1.93 - 1.83 (m, 1H), 1.16 - 1.06 (m, 2H), 1.05 - 0.92 (m, 5H). |
| **P218** | N-(1-(2-(cyclopropanesulfonamido)pyrimidin-4-yl)propyl)-4-(6-isopropoxypyrazin-2-yl) benzamide 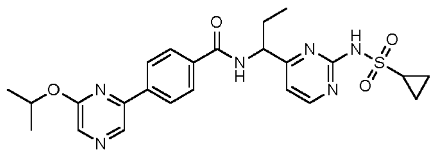 | Method 7 using INTC167, [UPLC acidic], 497, (1.43) | 11.25 (s, 1H), 8.98 - 8.82 (m, 2H), 8.55 (d, J = 5.2 Hz, 1H), 8.29 - 8.16 (m, 3H), 8.07 (d, J = 8.1 Hz, 2H), 7.15 (d, J = 5.2 Hz, 1H), 5.48 - 5.36 (m, 1H), 4.93 - 4.82 (m, 1H), 3.30 - 3.22 (m, 1H), 2.04 - 1.93 (m, 1H), 1.93 - 1.81 (m, 1H), 1.40 (d, J = 6.1 Hz, 6H), 1.15-1.05 (m, 2H), 1.05 - 0.93 (m, 5H). |
| **P219** | N-(1-(2-(cyclopropanesulfonamido)pyrimidin-4-yl)propyl)-4-(6-ethoxypyrazin-2-yl)benzamide 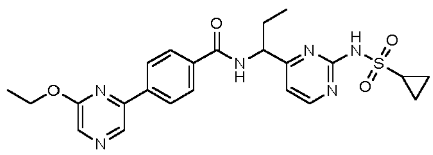 | Method 7 using INTC168, [UPLC acidic], 483, (1.34) | 11.24 (s, 1H), 8.98 - 8.86 (m, 2H), 8.56 (d, J = 5.2 Hz, 1H), 8.30 (s, 1H), 8.25 (d, J = 8.3 Hz, 2H), 8.09 - 8.04 (m, 2H), 7.16 (d, J = 5.2 Hz, 1H), 4.92 - 4.82 (m, 1H), 4.51 (q, J = 7.0 Hz, 2H), 3.32 - 3.22 (m, 1H), 2.05 - 1.93 (m, 1H), 1.93 - 1.81 (m, 1H), 1.42 (t, J = 7.0 Hz, 3H), 1.16 - 1.06 (m, 2H), 1.05 - 0.93 (m, 5H). |

(continued)

| P | Name/Structure (All examples containing chiral centres are racemates unless stated) | Synthesis Method, [LCMS Method], m/z $(M+H)^+$, (RT/Min) | $^1H$ NMR Chemical Shift Data (DMSO-d6 unless stated) |
|---|---|---|---|
| **P220** | N-(2-(2-(cyclopropanesulfonamido)pyrimidin-4-yl)butan-2-yl)-4-(6-ethoxypyrazin-2-yl)-2-fluorobenzamide 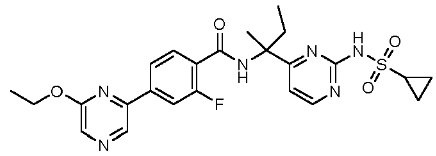 | Method 1 using INTC159 and INTD74, [UPLC acidic], 515, (1.46) | 11.21 (s, 1H), 8.93 (s, 1H), 8.67 (s, 1H), 8.54 (d, J = 5.3 Hz, 1H), 8.33 (s, 1H), 8.12 - 7.98 (m, 2H), 7.77 - 7.73 (m, 1H), 7.18 (d, J = 5.3 Hz, 1H), 4.51 (q, J = 7.0 Hz, 2H), 3.29 - 3.22 (m, 1H), 2.17 - 2.00 (m, 2H), 1.63 (s, 3H), 1.42 (t, J = 7.0 Hz, 3H), 1.18 - 0.96 (m, 4H), 0.81 (t, J = 7.4 Hz, 3H). |
| **P221** | N-(2-(6-(cyclopropanesulfonamido)pyrazin-2-yl) propan-2-yl)-2-fluoro-4-(6-isopropoxypyrazin-2-yl) benzamide 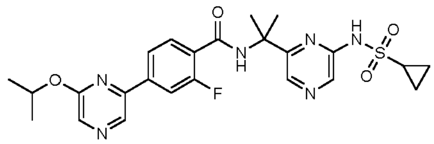 | Method 1 using INTC175 and INTD81, [HPLC acidic], 515, (2.3) | 10.96 (s, 1H), 8.90 (s, 1H), 8.83 (s, 1H), 8.40 (s, 1H), 8.27 (s, 1H), 8.15 (s, 1H), 8.06 - 8.00 (m, 2H), 7.80 - 7.63 (m, 1H), 5.50 - 5.31 (m, 1H), 3.18 - 3.09 (m, 1H), 1.69 (s, 6H), 1.40 (d, J = 6.2 Hz, 6H), 1.15 - 1.09 (m, 2H), 1.05 - 0.99 (m, 2H). |
| **P222** | N-(2-(6-(cyclopropanesulfonamido)pyrazin-2-yl) propan-2-yl)-4-(6-(trifluoromethyl)pyrazin-2-yl) benzamide 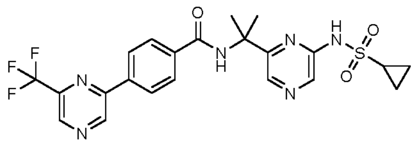 | Method 1 using INTC175 and INTD75, [HPLC acidic], 507, (2.13) | 10.94 (s, 1H), 9.71 (s, 1H), 9.20 (s, 1H), 8.85 (s, 1H), 8.37 (s, 1H), 8.33 - 8.27 (m, 2H), 8.12 (s, 1H), 8.09 - 8.04 (m, 2H), 3.13 - 3.03 (m, 1H), 1.73 (s, 6H), 1.10-1.00 (m, 2H), 0.89 - 0.77 (m, 2H). |
| **P223** | N-(1-(6-(cyclopropanesulfonamido)pyrazin-2-yl) propyl)-4-(6-ethoxypyrazin-2-yl)-2-fluorobenzamide 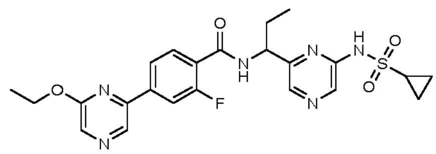 | Method 1using INTC174 and INTD74, [HPLC acidic], 501, (2.19) | 11.04 (s, 1H), 8.93 (s, 1H), 8.90 - 8.85 (m, 1H), 8.36 - 8.30 (m, 2H), 8.22 (s, 1H), 8.09 - 8.04 (m, 2H), 7.79 - 7.74 (m, 1H), 5.06 - 4.93 (m, 1H), 4.51 (q, J = 7.0 Hz, 2H), 3.25 - 3.09 (m, 1H), 2.03 - 1.77 (m, 2H), 1.42 (t, J = 7.0 Hz, 3H), 1.16 - 1.10 (m, 2H), 1.08 - 1.00 (m, 2H), 0.98 (t, J = 7.3 Hz, 3H). |
| **P224** | Single enantiomer - stereochemistry unassigned N-(1-(6-(cyclopropanesulfonamido)pyrazin-2-yl) propyl)-4-(6-ethoxypyrazin-2-yl)-2-fluorobenzamide 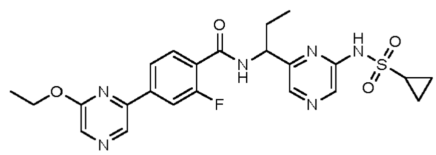 | Method 1 using INTC174 and INTD74, Chiral IC4 (9.28), [HPLC acidic], 501, (2.19) | 11.04 (s, 1H), 8.93 (s, 1H), 8.87 (d, J = 7.8 Hz, 1H), 8.41 - 8.24 (m, 2H), 8.22 (s, 1H), 8.11 - 8.03 (m, 2H), 7.82 - 7.72 (m, 1H), 5.03 - 4.93 (m, 1H), 4.51 (q, J = 7.0 Hz, 2H), 3.24 - 3.15 (m, 1H), 2.03 - 1.79 (m, 2H), 1.41 (t, J = 7.0 Hz, 3H), 1.18 - 1.09 (m, 2H), 1.09 - 1.02 (m, 2H), 0.98 (t, J = 7.3 Hz, 3H). |

(continued)

| P | Name/Structure (All examples containing chiral centres are racemates unless stated) | Synthesis Method, [LCMS Method], m/z (M+H)+, (RT/Min) | $^1$H NMR Chemical Shift Data (DMSO-d6 unless stated) |
|---|---|---|---|
| **P225** | Single enantiomer - stereochemistry unassigned N-(1-(6-(cyclopropanesulfonamido)pyrazin-2-yl) propyl)-4-(6-ethoxypyrazin-2-yl)-2-fluorobenzamide 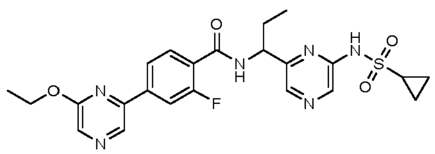 | Method 1 using INTC174 and INTD74, Chiral IC4 (19.90), [HPLC acidic], 501, (2.19) | 11.04 (s, 1H), 8.93 (s, 1H), 8.88 (d, J = 7.8 Hz, 1H), 8.36 (s, 1H), 8.33 (s, 1H), 8.22 (s, 1H), 8.09 - 8.03 (m, 2H), 7.79 - 7.74 (m, 1H), 5.02 - 4.94 (m, 1H), 4.51 (q, J = 7.0 Hz, 2H), 3.25 - 3.16 (m, 1H), 2.03 - 1.75 (m, 2H), 1.41 (t, J = 7.0 Hz, 3H), 1.18 - 1.11 (m, 2H), 1.11 - 1.02 (m, 2H), 0.98 (t, J = 7.3 Hz, 3H). |

2-(2-(Cyclopropanesulfonamido)pyrimidin-4-yl)-*N*-(5-(6-isopropylpyrazin-2-yl)pyridin-2-yl)-2-methylpropanamide **P116**

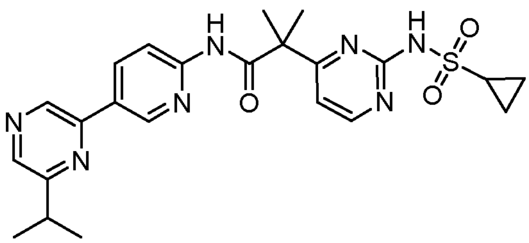

A solution of 2-(2-(cyclopropanesulfonamido)pyrimidin-4-yl)-2-methyl-*N*-(5-(6-(prop-1-en-2-yl)pyrazin-2-yl)pyridin-2-yl)propanamide **P122** (77 mg, 0.161 mmol) in MeOH/DCM (4:1, 10 mL) was hydrogenated using the H-Cube flow hydrogenation apparatus (10% Pd/C, 30x4 mm, Full hydrogen, 25 °C, 1 mL/min). The crude product was purified by chromatography on silica gel (12 g column, 50-100% EtOAc/iso-hexane) to afford 2-(2-(cyclopropanesulfonamido)pyrimidin-4-yl)-N-(5-(6-isopropylpyrazin-2-yl)pyridin-2-yl)-2-methylpropanamide (21 mg, 0.043 mmol, 27% yield) as a white solid. Rt 2.22 mins (HPLC acidic); m/z 482 $(M+H)^+$ $(ES^+)$; $^1$H NMR (500 MHz, DMSO-d6) $\delta$ 11.23 (s, 1H), 10.15 (s, 1H), 9.10 (s, 1H), 9.03 (dd, J = 2.4, 0.8 Hz, 1H), 8.59 (d, J = 5.3 Hz, 1H), 8.56 (s, 1H), 8.52 (dd, J = 8.8, 2.5 Hz, 1H), 8.21 (dd, J = 8.8, 0.8 Hz, 1H), 7.19 (d, J = 5.3 Hz, 1H), 3.23 - 3.10 (m, 2H), 1.61 (s, 6H), 1.32 (d, J = 6.9 Hz, 6H), 1.04 - 0.97 (m, 2H), 0.80 - 0.72 (m, 2H).

## Biological Examples

### Biological Example 1 - Human CTPS1 Enzyme Inhibition

The enzyme inhibitory activities of compounds invented against the target of interest were determined using the ADP-Glo™ Max assay (Promega, UK). Assays for human CTPS1 were performed in 1x assay buffer containing 50mM Tris, 10mM $MgCl_2$, 0.01% Tween-20, pH to 8.0 accordingly. Finally, immediately before use, L-cysteine was added to the 1x assay buffer to a final concentration of 2mM. All reagents are from Sigma-Aldrich unless specified otherwise. Human full length active C-terminal FLAG-Hiss-tag CTPS1 (UniProtKB - P17812, CTPS[1-591]-GGDYKDDDDKGGHHHHHHHH) was obtained from Proteros biostructures GmbH.

*Assay Procedure*

3x human CTPS1 protein was prepared in 1x assay buffer to the final working protein concentration required for the reaction. A 2uL volume per well of 3x human CTPS1 protein was mixed with 2uL per well of 3x test compound (compound prepared in 1x assay buffer to an appropriate final 3x compound concentration respective to the concentration

response curve designed for the compounds under test) for 10 minutes at 25°C. The enzymatic reaction was then initiated by addition of a 2uL per well volume of a pre-mixed substrate mix (UltraPure ATP from ADP-Glo™ Max kit (0.31mM), GTP (0.034mM), UTP (0.48mM) and L-glutamine (0.186mM)) and the mixture was incubated for an appropriate amount of time within the determined linear phase of the reaction at 25°C under sealed plate conditions with constant agitation at 500 revolutions per minute (rpm). ADP-Glo™ Max reagent was added for 60 minutes (6µL per well) and subsequently ADP-Glo™ Max development reagent was added for 60 minutes (12uL per well) prior to signal detection in a microplate reader (EnVision® Multilabel Reader, Perkin Elmer). Following each reagent addition over the course of the assay, assay plates were pulse centrifuged for 30 seconds at 500rpm.

In all cases, the enzyme converts ATP to ADP and the ADP-Glo™ Max reagent subsequently depletes any remaining endogenous ATP in the reaction system. The ADP-Glo™ Max detection reagent converts the ADP that has been enzymatically produced back into ATP and using ATP as a substrate together with luciferin for the enzyme luciferase, light is generated which produces a detectable luminescence. The luminescent signal measured is directly proportional to the amount of ADP produced by the enzyme reaction and a reduction in this signal upon compound treatment demonstrates enzyme inhibition. The percentage inhibition produced by each concentration of compound was calculated using the equation shown below:

$$\% \, Inhibition = 1 - \frac{(Mean_{Min} - Mean_{Inh})}{(Mean_{Min} - Mean_{Max})} \, x \, 100$$

Percentage inhibition was then plotted against compound concentration, and the 50% inhibitory concentration ($IC_{50}$) was determined from the resultant concentration-response curve.

The data for all compounds of formula (I) tested are presented below.

**Table 18:** *Human CTPS1 Enzyme Inhibition data grouped by potency range* (± indicates $IC_{50}$ in the range of >10 to 20 micromolar, + indicates $IC_{50}$ in the range >1 to 10 micromolar, ++ indicates $IC_{50}$ in the range >0.1 to 1 micromolar, +++ indicates $IC_{50}$ of ≤0.1 micromolar)

| P | CTPS1 |
|---|---|
| P1 | ++ |
| P2 | +++ |
| P3 | +++ |
| P4 | ++ |
| P5 | + |
| P6 | ++ |
| P7 | ++ |
| P8 | +++ |
| P9 | +++ |
| P10 | +++ |
| P11 | +++ |
| P12 | +++ |
| P13 | ++ |
| P14 | ++ |
| P15 | + |
| P16 | ++ |
| P17 | + |
| P18 | +++ |
| P19 | +++ |
| P20 | +++ |

(continued)

| P | CTPS1 |
|---|---|
| P21 | +++ |
| P22 | ++ |
| P23 | ++ |
| P24 | ++ |
| P25 | ++ |
| P26 | ++ |
| P27 | +++ |
| P28 | ++ |
| P29 | + |
| P30 | +++ |
| P31 | +++ |
| P32 | +++ |
| P33 | +++ |
| P34 | +++ |
| P35 | ++ |
| P36 | + |
| P37 | +++ |
| P38 | +++ |
| P39 | +++ |
| P40 | ++ |
| P41 | +++ |
| P42 | + |
| P43 | ++ |
| P44 | ++ |
| P45 | ++ |
| P46 | ++ |
| P47 | +++ |
| P48 | ++ |
| P49 | +++ |
| P50 | + |
| P51 | ++ |
| P52 | ++ |
| P53 | ++ |
| P54 | ++ |
| P55 | +++ |
| P56 | ++ |
| P57 | ++ |
| P58 | ++ |

(continued)

| P | CTPS1 |
|---|---|
| P59 | + |
| P60 | + |
| P61 | + |
| P62 | + |
| P63 | ± |
| P64 | +++ |
| P65 | +++ |
| P66 | ++ |
| P67 | +++ |
| P68 | ++ |
| P69 | ++ |
| P70 | + |
| P71 | + |
| P72 | ++ |
| P73 | ++ |
| P74 | ++ |
| P75 | ++ |
| P76 | ++ |
| P77 | + |
| P78 | ++ |
| P79 | ++ |
| P80 | ++ |
| P81 | +++ |
| P82 | + |
| P83 | +++ |
| P84 | ++ |
| P85 | ++ |
| P86 | + |
| P87 | +++ |
| P88 | +++ |
| P89 | +++ |
| P90 | ++ |
| P91 | ++ |
| P92 | ++ |
| P93 | + |
| P94 | +++ |
| P95 | +++ |
| P96 | +++ |

(continued)

| P | CTPS1 |
|---|---|
| P97 | +++ |
| P98 | +++ |
| P99 | ++ |
| P100 | ++ |
| P101 | ++ |
| P102 | ++ |
| P103 | ++ |
| P104 | ++ |
| P105 | +++ |
| P106 | +++ |
| P107 | +++ |
| P108 | +++ |
| P109 | ++ |
| P110 | +++ |
| P111 | ++ |
| P112 | +++ |
| P113 | +++ |
| P114 | +++ |
| P115 | +++ |
| P116 | ++ |
| P117 | +++ |
| P118 | +++ |
| P122 | ++ |
| P123 | ++ |
| P124 | ++ |
| P125 | ++ |
| P126 | +++ |
| P128 | ++ |
| P129 | ++ |
| P130 | ++ |
| P131 | ++ |
| P132 | ++ |
| P133 | + |
| P134 | ++ |
| P135 | ++ |
| P136 | +++ |
| P137 | +++ |
| P138 | ++ |

(continued)

| P | CTPS1 |
|---|---|
| P139 | ++ |
| P140 | ++ |
| P141 | ++ |
| P142 | ++ |
| P143 | +++ |
| P144 | +++ |
| P145 | +++ |
| P146 | +++ |
| P147 | ++ |
| P148 | +++ |
| P149 | +++ |
| P150 | +++ |
| P151 | +++ |
| P152 | +++ |
| P153 | +++ |
| P154 | +++ |
| P155 | +++ |
| P156 | +++ |
| P157 | +++ |
| P158 | ++ |
| P159 | +++ |
| P160 | +++ |
| P161 | +++ |
| P162 | +++ |
| P163 | +++ |
| P164 | +++ |
| P165 | +++ |
| P166 | +++ |
| P167 | ++ |
| P168 | ++ |
| P169 | ++ |
| P170 | ++ |
| P171 | + |
| P172 | ++ |
| P173 | +++ |
| P174 | + |
| P175 | + |
| P176 | + |

(continued)

| P | CTPS1 |
|---|---|
| P177 | + |
| P178 | + |
| P179 | + |
| P180 | + |
| P181 | + |
| P182 | ++ |
| P183 | ++ |
| P184 | ++ |
| P185 | + |
| P186 | +++ |
| P187 | ++ |
| P188 | +++ |
| P189 | + |
| P190 | ++ |
| P191 | ++ |
| P192 | + |
| P193 | + |
| P194 | 58.6 uM |
| P195 | +++ |
| P196 | +++ |
| P197 | +++ |
| P198 | +++ |
| P199 | ++ |
| P200 | +++ |
| P201 | +++ |
| P202 | +++ |
| P203 | +++ |
| P204 | +++ |
| P205 | +++ |
| P206 | +++ |
| P207 | +++ |
| P208 | +++ |
| P209 | ++ |
| P210 | ++ |
| P211 | ++ |
| P212 | ++ |
| P213 | ++ |
| P214 | ++ |

(continued)

| P | CTPS1 |
|---|---|
| P215 | ++ |
| P216 | +++ |
| P217 | ++ |
| P218 | +++ |
| P219 | ++ |
| P220 | ++ |
| P221 | ++ |
| P222 | ++ |
| P223 | ++ |
| P224 | ++ |
| P225 | +++ |

All compounds of the invention which have been tested were found to demonstrate inhibition of CTPS1 enzyme in this assay. Consequently, these compounds may be expected to have utility in the inhibition of CTPS1. The compounds of the invention are also expected to have utility as research tools, for example, for use in CTPS assays.

**Biological Example 2 - RapidFire/MS-based Enzyme Selectivity Assays.**

*Human CTPS1 versus CTPS2 Selectivity Assessment by RapidFire/MS Analysis.*

The enzyme inhibitory activities against each target isoform of interest may be determined for the compounds of the invention using an optimised RapidFire high-throughput mass spectrometry (RF/MS) assay format. RF/MS assays for both human CTPS1 and CTPS2 may be performed in assay buffer consisting of 50mM HEPES (Merck), 20mM $MgCl_2$, 5mM KCl, 1mM DTT, 0.01% Tween-20, pH to 8.0 accordingly. Human full-length active C-terminal FLAG-His-tag CTPS1 (UniProtKB - P17812, CTPS[1-591]-GGDYKDDDDKGGHHHHHHHH) may be obtained from Proteros biostructures GmbH. Human full length active C-terminal FLAG-His-Avi tagged CTPS2 (UniProtKB - Q9NRF8, CTPS2 [1-586]- DYKDDDDKHHHHHHGLNDIFEAQKIEWHE) may be obtained from Harker Bio.

*Assay Procedure*

Human CTPS (1 or 2) protein may be prepared in 1x assay buffer to the final working protein concentration required for the reaction. A 2uL volume per well of 2x CTPS (1 or 2) protein may be mixed with 40nL of compound using acoustic (ECHO) delivery and incubated for 10 minutes at 25°C. Each isoform enzymatic reaction may be subsequently initiated by addition of 2uL per well of a 2x substrate mix in assay buffer. For hCTPS1: ATP (0.3mM), UTP (0.2mM), GTP (0.07mM) and L-glutamine (0.1mM). For hCTPS2: ATP (0.1mM), UTP (0.04mM), GTP (0.03mM) and L-glutamine (0.1mM). Each mixture may be incubated for an appropriate amount of time per isoform within the determined linear phase of the reaction at 25°C. A 60uL volume of stop solution (1% formic acid with 0.5uM $^{13}C_9$-$^{15}N_3$-CTP in $H_20$) may be added and the plate immediately heat-sealed and centrifuged for 10 minutes at 4,000rpm. Following centrifugation, plates may be loaded onto the Agilent RapidFire microfluidic solid phase extraction system coupled to an API4000 triple quadrupole mass spectrometer (RF/MS) for analysis.

In all cases, the enzyme converts UTP to CTP. Highly specific and sensitive multiple reaction monitoring (MRM) MS methods may be optimised for the detection of the enzymatic reaction product, CTP, and the stable isotope labelled product standard $^{13}C_9$-$^{15}N_3$-CTP. Readout for data analysis may be calculated as the ratio between the peak area of the product CTP and the internal standard $^{13}C_9$-$^{15}N_3$-CTP. For data reporting, the following equation may be used:

$$R = \frac{P}{IS}$$

(R = ratio/readout, P = product signal area, IS = internal standard signal area)

For each screening plate, the means of the negative (DMSO) and positive control values were used for the calculation of the respective assay window (S/B) and Z' values. The median of the respective control values was used for calculation of percent inhibition according to the following equation:

$$I = \frac{R_{neg} - R_{sample}}{[R_{neg} - R_{pos}]} \%$$

(I = Inhibition, $R_{neg}$ = median of negative control readout values, $R_{pos}$ = median of positive control readout values, $R_{sample}$ = sample readout value)
Percentage inhibition was then plotted against compound concentration, and the 50% inhibitory concentration ($IC_{50}$) was determined from the resultant concentration-response curve.

Fold selectivity between CTPS1 and CTPS2 was subsequently calculated according to the following equation:

$$\text{Fold selectivity} = \frac{\text{CTPS2 } IC_{50}}{\text{CTPS1 } IC_{50}}$$

Certain compounds of formula (I) were tested in the assay above. The data for all compounds tested are presented below.

**Table 19:** Selectivity data split into grouping of 2-30 fold (+), >30-60 fold (++) or >60 fold (+++)

| P | Selectivity |
|---|---|
| **P1** | + |
| **P2** | +++ |
| **P9** | +++ |
| **P12** | ++ |
| **P16** | ++ |
| **P18** | ++ |
| **P21** | ++ |
| **P31** | +++ |
| **P34** | + |
| **P38** | + |
| **P39** | + |
| **P59** | + |
| **P65** | ++ |
| **P68** | ++ |
| **P70** | + |
| **P74** | ++ |
| **P76** | ++ |
| **P83** | +++ |
| **P87** | ++ |
| **P88** | +++ |
| **P89** | +++ |
| **P95** | + |
| **P96** | + |

(continued)

| P | Selectivity |
|---|---|
| P98 | +++ |
| P103 | + |
| P105 | ++ |
| P108 | +++ |
| P110 | ++ |
| P112 | ++ |
| P113 | + |
| P114 | +++ |
| P115 | +++ |
| P118 | +++ |
| P125 | ++ |
| P128 | + |
| P132 | ++ |
| P136 | +++ |
| P143 | +++ |
| P145 | +++ |
| P146 | +++ |
| P151 | +++ |
| P155 | + |
| P158 | + |
| P159 | +++ |
| P161 | + |
| P162 | ++ |
| P163 | +++ |
| P164 | + |
| P188 | ++ |
| P191 | ++ |
| P195 | +++ |
| P196 | +++ |
| P197 | +++ |
| P198 | +++ |
| P200 | ++ |
| P201 | +++ |
| P202 | +++ |
| P205a | +++ |
| P205b | ++ |
| P206 | +++ |
| P207 | + |

(continued)

| P | Selectivity |
|---|---|
| **P216** | +++ |
| **P221** | + |
| **P222** | + |

All compounds tested in the assay described in Biological Example 2 were found to have at least 2 fold selectivity for CTPS1 over CTPS2, with many compounds having a selectivity for CTPS1 of over 60 fold. In particular, these compounds may be expected to have utility in the treatment of diseases whereby a selective CTPS1 compound is beneficial.

Throughout the specification and the claims which follow, unless the context requires otherwise, the word 'comprise', and variations such as 'comprises' and 'comprising', will be understood to imply the inclusion of a stated integer, step, group of integers or group of steps but not to the exclusion of any other integer, step, group of integers or group of steps.

REFERENCES

Evans, D. R. & Guy, H. I. Mammalian pyrimidine biosynthesis: fresh insights into an ancient pathway. J. Biol. Chem. 279, 33035-33038 (2004).

Fairbanks, L. D. et al. Importance of ribonucleotide availability to proliferating T-lymphocytes from healthy humans. Disproportionate expansion of pyrimidine pools and contrasting effects of de novo synthesis inhibitors. J. Biol. Chem. 270, 29682-29689 (1995).

Higgins, M. J. et al. Regulation of human cytidine triphosphate synthetase 1 by glycogen synthase kinase 3. J. Biol. Chem. 282, 29493-29503 (2007).

Kursula, P. et al. Structure of the synthetase domain of human CTP synthetase, a target for anticancer therapy. Acta Crystallogr Sect F Struct Biol Cryst Commun. 62 (Pt7): 613-617 (2006).

Lieberman I. Enzymatic amination of uridine triphosphate to cytidine triphosphate. The J. Biol. Chem. 222 (2): 765-75 (1956).

Martin E. et al. CTP synthase 1 deficiency in humans reveals its central role in lymphocytes proliferation. Nature. Jun 12; 510(7504):288-92 (2014). Erratum in: Nature. Jul 17; 511(7509):370 (2014).

McCluskey GD et al., Exploring the Potent Inhibition of CTP Synthase by Gemcitabine-5'-Triphosphate. Chembiochem. 17, 2240-2249 (2016).

Ostrander, D. B. et al. Effect of CTP synthetase regulation by CTP on phospholipid synthesis in Saccharomyces cerevisiae. J. Biol. Chem. 273, 18992-19001 (1998).

Sakamoto K. et al. Identification of cytidine-5-triphosphate synthase1-selective inhibitory peptide from random peptide library displayed on T7 phage. Peptides. 2017; 94:56-63 (2017).

Salu et al. Drug-eluting stents: a new treatment in the prevention of restenosis Part I: experimental studies. Acta Cardiol, 59, 51-61 (2004).

Sousa J. E. et al. Drug-Eluting Stents. Circulation, 107 (2003) 2274 (Part I), 2283 (Part II).

Tang R. et al. CTP synthase 1, a smooth muscle-sensitive therapeutic target for effective vascular repair. Arterioscler Thromb Vase Biol. 33(10), 1-19, (2013).

van den Berg, A. A. et al. Cytidine triphosphate (CTP) synthetase activity during cell cycle progression in normal and malignant T-lymphocytic cells. Eur. J. Cancer 31, 108-112 (1995).

van Kuilenburg, A.B.P. et al. Identification of a cDNA encoding an isoform of human CTP synthetase. Biochimica et Biophysica Acta 1492548-552 (2000).

## Claims

1. A compound of formula (I):

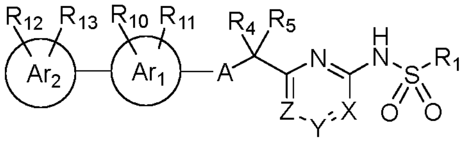

(I)

wherein

A is an amide linker having the following structure: -C(=O)NH- or -NHC(=O)-;
Xis N or CH;
Yis N or $CR_2$;
Z is N or $CR_3$;
with the proviso that when at least one of X or Z is N, Y cannot be N;
$R_1$ is $C_{1-5}$alkyl, $CF_3$, $C_{0-2}$alkylene$C_{3-5}$cycloalkyl or $C_{0-2}$alkylene$C_{3-5}$cycloalkyl which cycloalkyl is substituted by $CH_3$;
$R_2$ is H, halo, $C_{1-2}$alkyl, $OC_{1-2}$alkyl, $C_{1-2}$haloalkyl or $OC_{1-2}$haloalkyl;
$R_3$ is H, halo, $CH_3$, $OCH_3$, $CF_3$ or $OCF_3$;
wherein at least one of $R_2$ and $R_3$ is H;
$R_4$ and $R_5$ are each independently H, $C_{1-6}$alkyl, $C_{1-6}$alkylOH, $C_{1-6}$haloalkyl, $C_{0-2}$alkylene$C_{3-6}$cycloalkyl, $C_{0-2}$alkylene$C_{3-6}$heterocycloalkyl, $C_{1-3}$alkylene$OC_{1-3}$alkyl, or $R_4$ and $R_5$ together with the carbon atom to which they are attached form a $C_{3-6}$cycloalkyl or $C_{3-6}$heterocycloalkyl; and
when A is -NHC(=O)-:
$R_4$ and $R_5$ may additionally be selected from halo, $OC_{1-6}$haloalkyl, $OC_{0-2}$alkylene$C_{3-6}$cycloalkyl, $OC_{0-2}$alkylene$C_{3-6}$heterocycloalkyl, $OC_{1-6}$alkyl and $NR_{21}R_{22}$;
Ar1 is a 6-membered aryl or heteroaryl;
Ar2 is a 6-membered aryl or heteroaryl and is attached to Ar1 in the para position relative to the amide;
$R_{10}$ is H, halo, $C_{1-3}$alkyl, $C_{1-2}$haloalkyl, $OC_{1-2}$alkyl, $OC_{1-2}$haloalkyl or CN;
$R_{11}$ is H, F, Cl, $C_{1-2}$alkyl, $CF_3$, $OCH_3$ or CN;
$R_{12}$ is attached to Ar2 in the ortho or meta position relative to Ar1 and $R_{12}$ is H, halo, $C_{1-4}$alkyl, $C_{2-4}$alkenyl, $C_{0-2}$alkylene$C_{3-5}$cycloalkyl, $OC_{1-4}$alkyl, $OC_{0-2}$alkylene$C_{3-5}$cycloalkyl, $C_{1-4}$haloalkyl, $OC_{1-4}$haloalkyl, hydroxy, $C_{1-4}$alkylOH, $SO_2C_{1-2}$alkyl, $C(O)N(C_{1-2}alkyl)_2$, $NHC(O)C_{1-3}$alkyl or $NR_{23}R_{24}$; and
when A is -NHC(=O)-:
$R_{12}$ may additionally be selected from CN, $OCH_2CH_2N(CH_3)_2$ and a $C_{3-6}$heterocycloalkyl comprising one nitrogen located at the point of attachment to Ar2, or $R_{12}$ together with a nitrogen atom to which it is attached forms an N-oxide ($N^+$-$O^-$);
$R_{13}$ is H or halo;
$R_{21}$ is H, $C_{1-5}$alkyl, $C(O)C_{1-5}$alkyl, $C(O)OC_{1-5}$alkyl;
$R_{22}$ is H or $CH_3$;
$R_{23}$ is H or $C_{1-2}$alkyl; and
$R_{24}$ is H or $C_{1-2}$alkyl;

or a salt and/or solvate thereof.

2. The compound, salt and/or solvate thereof according to claim 1 wherein A is -NHC(=O)-.

3. The compound, salt and/or solvate thereof according to any one of claims 1 to 2 wherein X is N, Y is $CR_2$ and Z is $CR_3$.

4. The compound, salt and/or solvate thereof according to any one of claims 1 to 3 wherein $R_1$ is $C_{1-5}$alkyl,

5. The compound, salt and/or solvate thereof according to any one of claims 1 to 3 wherein $R_1$ is $C_{3-5}$cycloalkyl or $C_{3-5}$cycloalkyl substituted by $CH_3$.

6. The compound, salt and/or solvate thereof according to any one of claims 1 to 5 wherein $R_2$ is H and $R_3$ is H.

7. The compound, salt and/or solvate thereof according to any one of claims 1 to 6 wherein $R_4$ is selected from halo, $C_{1-6}$alkyl, $C_{1-3}$alkyleneO$C_{1-3}$alkyl or O$C_{1-6}$alkyl and $R_5$ is H, fluoro, methyl or ethyl.

8. The compound, salt and/or solvate thereof according to any one of claims 1 to 6 wherein $R_4$ and $R_5$ together with the carbon atom to which they are attached form a cyclopropyl ring or a cyclopentyl ring; or $R_4$ and $R_5$ together with the carbon atom to which they are attached form a tetrahydropyranyl ring.

9. The compound, salt and/or solvate thereof according to any one of claims 1 to 8 wherein Ar1 is phenyl or 2-pyridyl.

10. The compound, salt and/or solvate thereof according to any one of claims 1 to 9 wherein $R_{10}$ is H, F, Cl, $CH_3$, $OCH_3$, $OCF_3$ or CN and $R_{11}$ is H or F.

11. The compound, salt and/or solvate thereof according to any one of claims 1 to 10 wherein Ar2 is 3-pyridyl or 2,5-pyrazinyl.

12. The compound, salt and or solvate thereof according to any one of claims 1 to 11 wherein, $R_{12}$ is H, F, Cl, $CH_3$, methoxy, ethoxy, isopropoxy, O$C_0$alkylene$C_3$cycloalkyl, CN, $CF_3$, $OCHF_2$ or $OCH_2CF_3$ and $R_{13}$ is H.

13. The compound, pharmaceutically acceptable salt and/or solvate thereof according to any one of claims 1 to 12.

14. The compound, pharmaceutically acceptable salt and/or solvate thereof according to claim 13 for use as a medicament.

15. The compound, pharmaceutically acceptable salt and/or solvate thereof for use according to claim 14, for use in the reduction of T-cell and/or B-cell proliferation in a subject; or for use in the treatment or prophylaxis of: inflammatory skin diseases such as psoriasis or lichen planus; acute and/or chronic GVHD such as steroid resistant acute GVHD; acute lymphoproliferative syndrome (ALPS); systemic lupus erythematosus, lupus nephritis or cutaneous lupus; or transplantation; or for use in the treatment or prophylaxis of myasthenia gravis, multiple sclerosis or scleroderma/systemic sclerosis; or for use in enhancing recovery from vascular injury or surgery and reducing morbidity and mortality associated with neointima and restenosis in a subject.

16. The compound, pharmaceutically acceptable salt and/or solvate thereof according to claim 14, for use in the treatment of cancer.

17. The compound, pharmaceutically acceptable salt and/or solvate thereof for use according to claim 16, wherein the cancer is a haematological cancer.

18. The compound, pharmaceutically acceptable salt and/or solvate thereof for use according to claim 17 wherein the haematological cancer is selected from the group consisting of Acute myeloid leukemia, Angioimmunoblastic T-cell lymphoma, B-cell acute lymphoblastic leukemia, Sweet Syndrome, T-cell Non-Hodgkins lymphoma (including natural killer/T-cell lymphoma, adult T-cell leukaemia/lymphoma, enteropathy type T-cell lymphoma, hepatosplenic T-cell lymphoma and cutaneous T-cell lymphoma), T-cell acute lymphoblastic leukemia, B-cell Non-Hodgkins lymphoma (including Burkitt lymphoma, diffuse large B-cell lymphoma, Follicular lymphoma, Mantle cell lymphoma, Marginal Zone lymphoma), Hairy Cell Leukemia, Hodgkin lymphoma, Lymphoblastic lymphoma, Lymphoplasmacytic lymphoma, Mucosa-associated lymphoid tissue lymphoma, Multiple myeloma, Myelodysplastic syndrome, Plasma cell myeloma, Primary mediastinal large B-cell lymphoma, chronic myeloproliferative disorders (such as chronic myeloid leukemia, primary myelofibrosis, essential thrombocytemia, polycytemia vera) and chronic lymphocytic leukemia.

19. The compound, pharmaceutically acceptable salt and/or solvate thereof for use according to any one of claims 14 to 18, for administration to a human subject.

**20.** A pharmaceutical composition comprising a compound, pharmaceutically acceptable salt and/or solvate thereof according to claim 13.

**21.** A compound selected from the group consisting of:

- a compound of formula **(II):**

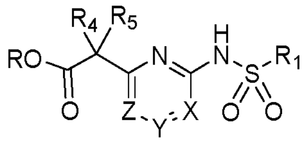

**(II)**

wherein $R_1$, X, Y, Z, $R_4$ and $R_5$ are as defined in any preceding claim and R is H, $C_{1-6}$alkyl or benzyl; or
- a compound of formula **(XXXXII):**

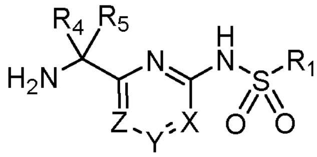

**(XXXXII)**;

wherein $R_1$, X, Y, Z, $R_4$ and $R_5$ are as defined in any preceding claim; or
- a compound of formula **(XX):**

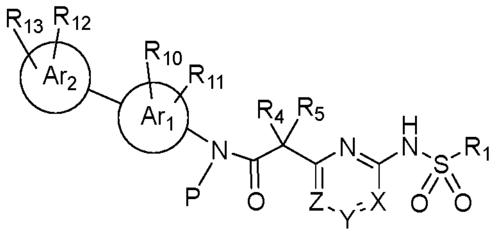

**(XX)**;

wherein Ar1, Ar2, $R_1$, X, Y, Z, $R_4$, $R_5$, $R_{10}$, $R_{11}$, $R_{12}$ and $R_{13}$ are as defined in any preceding claim and P is a nitrogen protecting group; or
- a compound of formula **(XXIV):**

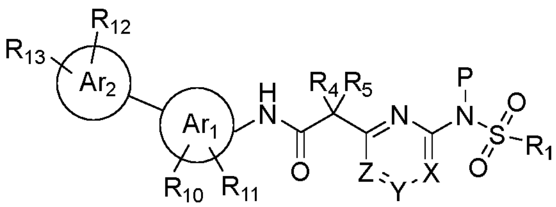

**(XXIV)**;

wherein Ar2, Ar1, $R_1$, X, Y, Z, $R_4$, $R_5$, $R_{10}$, $R_{11}$, $R_{12}$ and $R_{13}$ are as defined in any preceding claim and P is a nitrogen protecting group; or
- a compound of formula **(XXXI):**

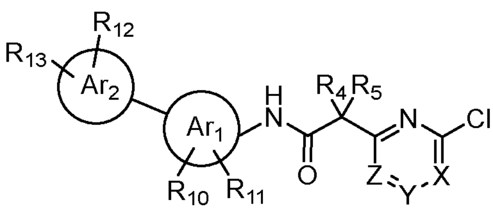

**(XXXI)**;

wherein Ar2 is 3-pyridyl or 2,5-pyrazinyl and Ar1, X, Y, Z, $R_4$, $R_5$, $R_{10}$, $R_{11}$, $R_{12}$ and $R_{13}$ are as defined in any preceding claim; or

- a compound of formula **(LI):**

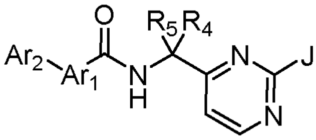

**(LI)**;

wherein J is Cl or Br and Ar1, Ar2, $R_4$ and $R_5$ are as defined in any preceding claim; and

- a compound of formula **(LVIII):**

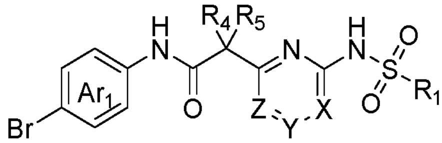

**(LVIII)**;

wherein Ar1, $R_1$, X, Y, Z, $R_4$ and $R_5$ are as defined in any preceding claim; or salts of any one thereof.

## Patentansprüche

**1.** Verbindung der Formel (I):

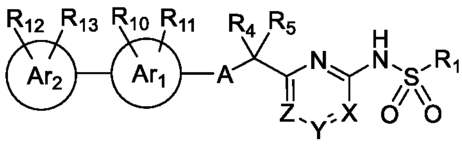

(I)

wobei

A ein Amidlinker mit der folgenden Struktur ist: -C(=O)NH- oder -NHC(=O)-;
X N oder CH ist;
Y N oder $CR_2$ ist;
Z N oder $CR_3$ ist;
mit der Maßgabe, dass, wenn mindestens eines von X oder Z N ist, Y nicht N sein kann;
$R_1$ $C_{1-5}$-Alkyl, $CF_3$, $C_{0-2}$-Alkylen-$C_{3-5}$-cycloalkyl oder $C_{0-2}$-Alkylen-$C_{3-5}$-cycloalkyl ist, wobei das Cycloalkyl mit $CH_3$ substituiert ist;
$R_2$ H, Halogen, $C_{1-2}$-Alkyl, $OC_{1-2}$-Alkyl, $C_{1-2}$-Halogenalkyl oder $OC_{1-2}$-Halogenalkyl ist;
$R_3$ H, Halogen, $CH_3$, $OCH_3$, $CF_3$ oder $OCF_3$ ist;
wobei mindestens einer von $R_2$ und $R_3$ H ist;
$R_4$ und $R_5$ jeweils unabhängig H, $C_{1-6}$-Alkyl, $C_{1-6}$-Alkyl-OH, $C_{1-6}$-Halogenalkyl, $C_{0-2}$-Alkylen-$C_{3-6}$-cycloalkyl, $C_{0-2}$-Alkylen-$C_{3-6}$-heterocycloalkyl, $C_{1-3}$-Alkylen-$OC_{1-3}$-alkyl sind oder $R_4$ und $R_5$ zusammen mit dem Kohlenstoffatom, an das sie gebunden sind, ein $C_{3-6}$-Cycloalkyl oder $C_{3-6}$-Heterocycloalkyl bilden; und
wenn A -NHC(=O)- ist:
$R_4$ und $R_5$ zusätzlich aus Halogen, $OC_{1-6}$-Halogenalkyl, $OC_{0-2}$-Alkylen-$C_{3-6}$-cycloalkyl, $OC_{0-2}$-Alkylen-$C_{3-6}$-heterocycloalkyl, $OC_{1-6}$-Alkyl and $NR_{21}R_{22}$ ausgewählt sein können;
Ar 1 ein 6-gliedriges Aryl oder Heteroaryl ist;
Ar2 ein 6-gliedriges Aryl oder Heteroaryl ist und in para-Stellung zum Amid an Ar1 gebunden ist;
$R_{10}$ H, Halogen, $C_{1-3}$-Alkyl, $C_{1-2}$-Halogenalkyl, $OC_{1-2}$-Alkyl, $OC_{1-2}$-Halogenalkyl oder CN ist;
$R_{11}$ H, F, Cl, $C_{1-2}$-Alkyl, $CF_3$, $OCH_3$ oder CN ist;
$R_{12}$ in ortho- oder meta-Stellung zu Ar1 an Ar2 gebunden ist und $R_{12}$ H, Halogen, $C_{1-4}$-Alkyl, $C_{2-4}$-Alkenyl, $C_{0-2}$-Alkylen-$C_{3-5}$-cycloalkyl, $OC_{1-4}$-Alkyl, $OC_{0-2}$-Alkylen-$C_{3-5}$-cycloalkyl, $C_{1-4}$-Halogenalkyl, $OC_{1-4}$-Halogenalkyl, Hydroxy, $C_{1-4}$-Alkyl-OH, $SO_2C_{1-2}$-Alkyl, $C(O)N(C_{1-2}\text{-Alkyl})_2$, $NHC(O)C_{1-3}$-Alkyl oder $NR_{23}R_{24}$ ist; und

wenn A -NHC(=O)- ist:
$R_{12}$ zusätzlich aus CN, $OCH_2CH_2N(CH_3)_2$ und einem $C_{3-6}$-Heterocycloalkyl, das ein Stickstoffatom umfasst, das sich an der Bindungsstelle an Ar2 befindet, ausgewählt sein kann, oder $R_{12}$ zusammen mit dem Stickstoffatom, an das es gebunden ist, ein N-Oxid ($N^+$-$O^-$) bildet;
$R_{13}$ H oder Halogen ist;
$R_{21}$ H, $C_{1-5}$-Alkyl, C(O)$C_{1-5}$-Alkyl, C(O)O$C_{1-5}$-Alkyl ist;
$R_{22}$ H oder $CH_3$ ist;
$R_{23}$ H oder $C_{1-2}$-Alkyl ist; und
$R_{24}$ H oder $C_{1-2}$-Alkyl ist;

oder ein Salz und/oder Solvat davon.

2. Verbindung, Salz und/oder Solvat davon nach Anspruch 1, wobei A -NHC(=O)- ist.

3. Verbindung, Salz und/oder Solvat davon nach einem der Ansprüche 1 bis 2, wobei X N ist, Y $CR_2$ ist und Z $CR_3$ ist.

4. Verbindung, Salz und/oder Solvat davon nach einem der Ansprüche 1 bis 3, wobei $R_1$ $C_{1-5}$-Alkyl ist,

5. Verbindung, Salz und/oder Solvat davon nach einem der Ansprüche 1 bis 3, wobei $R_1$ $C_{3-5}$-Cycloalkyl oder $C_{3-5}$-Cycloalkyl, substituiert mit $CH_3$ ist.

6. Verbindung, Salz und/oder Solvat davon nach einem der Ansprüche 1 bis 5, wobei $R_2$ H ist und $R_3$ H ist.

7. Verbindung, Salz und/oder Solvat davon nach einem der Ansprüche 1 bis 6, wobei $R_4$ aus Halogen, $C_{1-6}$-Alkyl, $C_{1-3}$-Alkylen-O$C_{1-3}$-alkyl oder O$C_{1-6}$-Alkyl ausgewählt ist oder $R_5$ H, Fluor, Methyl oder Ethyl ist.

8. Verbindung, Salz und/oder Solvat davon nach einem der Ansprüche 1 bis 6, wobei $R_4$ und $R_5$ zusammen mit dem Kohlenstoffatom, an das sie gebunden sind, einen Cyclopropylring oder einen Cyclopentylring bilden; oder $R_4$ und $R_5$ zusammen mit dem Kohlenstoffatom, an das sie gebunden sind, einen Tetrahydropyranylring bilden.

9. Verbindung, Salz und/oder Solvat davon nach einem der Ansprüche 1 bis 8, wobei Ar1 Phenyl oder 2-Pyridyl ist.

10. Verbindung, Salz und/oder Solvat davon nach einem der Ansprüche 1 bis 9, wobei $R_{10}$ H, F, Cl, $CH_3$, $OCH_3$, $OCF_3$ oder CN ist und $R_{11}$ H oder F ist.

11. Verbindung, Salz und/oder Solvat davon nach einem der Ansprüche 1 bis 10, wobei Ar2 3-Pyridyl oder 2,5-Pyrazinyl ist.

12. Verbindung, Salz und Solvat davon nach einem der Ansprüche 1 bis 11, wobei $R_{12}$ H, F, Cl, $CH_3$, Methoxy, Ethoxy, Isopropoxy, OC $_0$-Alkylen-$C_3$-Cycloalkyl, CN, $CF_3$, $OCHF_2$ oder $OCH_2CF_3$ ist und $R_{13}$ H ist.

13. Verbindung, pharmazeutisch verträgliches Salz und/oder Solvat davon nach einem der Ansprüche 1 bis 12.

14. Verbindung, pharmazeutisch verträgliches Salz und/oder Solvat davon nach Anspruch 13 zur Verwendung als Medikament.

15. Verbindung, pharmazeutisch verträgliches Salz und/oder Solvat davon zur Verwendung nach Anspruch 14, zur Verwendung bei der Reduzierung der T-Zell- und/oder B-Zell-Proliferation bei einem Subjekt; oder zur Verwendung bei der Behandlung oder Prophylaxe von Folgendem: entzündlichen Hauterkrankungen wie etwa Psoriasis oder Lichen planus; akuter und/oder chronischer GVHD wie etwa steroidresistenter akuter GVHD; akutem lymphoproliferativem Syndrom (ALPS); systemischem Lupus erythematodes, Lupusnephritis oder kutanem Lupus; oder Transplantation; oder zur Verwendung bei der Behandlung oder Prophylaxe von Myasthenia gravis, multipler Sklerose oder Sklerodermie/systemischer Sklerose; oder zur Verwendung bei der Verbesserung der Erholung von Gefäßverletzungen oder Operationen und zur Reduzierung der Morbidität und Mortalität im Zusammenhang mit Neointima und Restenose bei einem Subjekt.

16. Verbindung, pharmazeutisch verträgliches Salz und/oder Solvat davon nach Anspruch 14 zur Verwendung bei der Behandlung von Krebs.

**17.** Verbindung, pharmazeutisch verträgliches Salz und/oder Solvat davon zur Verwendung nach Anspruch 16, wobei der Krebs ein hämatologischer Krebs ist.

**18.** Verbindung, pharmazeutisch verträgliches Salz und/oder Solvat davon zur Verwendung nach Anspruch 17, wobei der hämatologische Krebs aus der Gruppe bestehend aus akuter myeloischer Leukämie, angioimmunoblastischem T-Zell-Lymphom, akuter lymphatischer B-Zell-Leukämie, Sweet-Syndrom, Non-Hodgkins-T-Zell-Lymphom (einschließlich natürlichem Killer/T-Zell-Lymphom, T-Zell-Leukämie/Lymphom bei Erwachsen, T-Zell-Lymphom vom Enteropathie-Typ, hepatosplenischem T-Zell-Lymphom und kutanem T-Zell-Lymphom), akuter T-Zell-Lymphoblastenleukämie, Non-Hodgkins-B-Zell-Lymphom (einschließlich Burkitt-Lymphom, diffusem großzelligen B-Zell-Lymphom, follikulärem Lymphom, Mantelzell-Lymphom, Marginal-Zone-Lymphom), Haarzellenleukämie, Hodgkin-Lymphom, lymphoblastischem Lymphom, lymphoplasmazytischem Lymphom, Mukosa-assoziiertem lymphatischem Gewebelymphom, multiplem Myelom, myelodysplastischem Syndrom, Plasmazellmyelom, primärem mediastinalem großzelligem B-Zell-Lymphom, chronisch myeloproliferativen Erkrankungen (wie etwa chronischer myeloischer Leukämie, primärer Myelofibrose, essenzieller Thrombozyämie, Polycytemia vera) und chronischer lymphatischer Leukämie ausgewählt ist.

**19.** Verbindung, pharmazeutisch verträgliches Salz und/oder Solvat davon zur Verwendung nach einem der Ansprüche 14 bis 18 zur Verabreichung an ein menschliches Subjekt.

**20.** Pharmazeutische Zusammensetzung, umfassend eine Verbindung, ein pharmazeutisch verträgliches Salz und/oder Solvat davon nach Anspruch 13.

**21.** Verbindung ausgewählt aus der Gruppe bestehend aus:

- einer Verbindung der Formel (II):

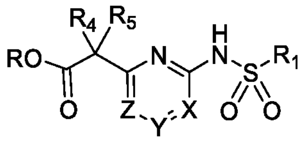

(II)

wobei $R_1$, X, Y, Z, $R_4$ und $R_5$ wie in einem vorhergehenden Anspruch definiert sind und R H, $C_{1-6}$-Alkyl oder Benzyl ist; oder
- eine Verbindung der Formel **(XXXXII)**:

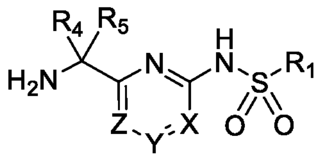

(XXXXII);

wobei $R_1$, X, Y, Z, $R_4$ und $R_5$ wie in einem beliebigen vorhergehenden Anspruch definiert sind; oder
- eine Verbindung der Formel **(XX):**

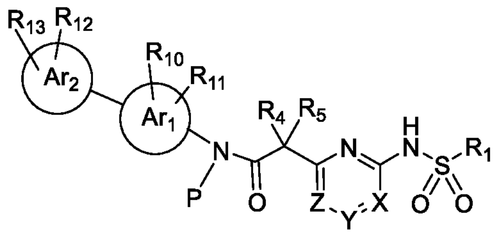

(XX);

wobei Ar1, Ar2, $R_1$, X, Y, Z, $R_4$, $R_5$, $R_{10}$, $R_{11}$, $R_{12}$ und $R_{13}$ wie in einem vorhergehenden Anspruch definiert sind und P eine Stickstoff-Schutzgruppe ist; oder
- Verbindung der Formel **(XXIV):**

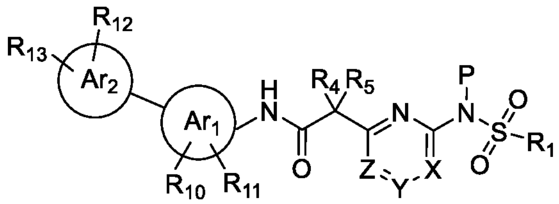

(XXIV) ;

wobei Ar2, Ar1, $R_1$, X, Y, Z, $R_4$, $R_5$, $R_{10}$, $R_{11}$, $R_{12}$ und $R_{13}$ wie in einem vorhergehenden Anspruch definiert sind und P eine Stickstoff-Schutzgruppe ist; oder

- eine Verbindung der Formel **(XXXI):**

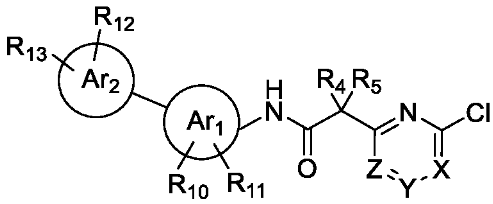

(XXXI) ;

wobei Ar2 3-Pyridyl oder 2,5-Pyrazinyl ist und Ar1, X, Y, Z, $R_4$, $R_5$, $R_{10}$, $R_{11}$, $R1_{12}$ und $R_{13}$ wie in einem der vorhergehenden Ansprüche definiert sind; oder

- eine Verbindung der Formel **(LI):**

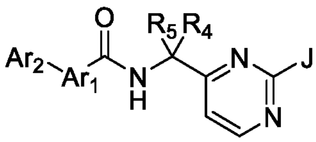

(LI) ;

wobei J Cl oder Br ist und Ar1, Ar2, $R_4$ und $R_5$ wie in einem vorhergehenden Anspruch definiert sind; und

- eine Verbindung der Formel **(LVIII):**

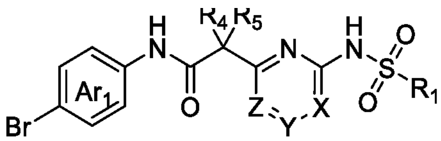

(LVIII) ;

Wobei Ar1, $R_1$, X, Y, Z, $R_4$ und $R_5$ wie in einem beliebigen vorhergehenden Anspruch definiert sind; oder Salze einer beliebigen davon.

## Revendications

**1.** Composé de formule (I) :

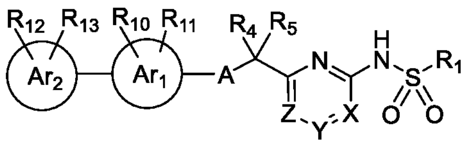

(I)

où

A est un agent de liaison amide ayant la structure suivante : -C(=O)NH- ou -NHC(=O)- ;
X représente N ou CH ;
Y représente N ou $CR_2$ ;

Z représente N ou $CR_3$;
étant entendu que lorsqu'au moins l'un parmi X ou Z représente N, Y ne peut pas représenter N ;
$R_1$ représente un $C_{1-5}$alkyle, $CF_3$, $C_{0-2}$alkylène$C_{3-5}$cycloalkyle ou $C_{0-2}$alkylène$C_{3-5}$cycloalkyle, lequel cycloalkyle est substitué par $CH_3$ ;
$R_2$ représente H, halo, un $C_{1-2}$alkyle, $OC_{1-2}$alkyle, un $C_{1-2}$haloalkyle ou un haloalkyle en $OC_{1-2}$ ;
$R_3$ est H, halo, $CH_3$, $OCH_3$, $CF_3$ ou $OCF_3$ ;
où au moins l'un parmi $R_2$ et $R_3$ représente H ;
$R_4$ et $R_5$ représentent chacun indépendamment H, un $C_{1-6}$alkyle, $C_{1-6}$alkyleOH, un $C_{1-6}$haloalkyle, $C_{0-2}$alkylène$C_{3-6}$cycloalkyle, $C_{0-2}$alkylène$C_{3-6}$hétérocycloalkyle, $C_{1-3}$alkylène$OC_{1-3}$alkyle, ou $R_4$ et $R_5$ ainsi que l'atome de carbone auquel ils sont fixés, forment un $C_{3-6}$ cycloalkyle ou un $C_{3-6}$hétérocycloalkyle ; et
lorsque A représente -NHC(=O)- :
$R_4$ et $R_5$ peuvent être en outre choisis parmi halo, $OC_{1-6}$haloalkyle, $OC_{0-2}$alkylène$C_{3-6}$cycloalkyle, $OC_{0-2}$alkylène$C_{3-6}$hétérocycloalkyle, $OC_{1-6}$alkyle et $NR_{21}R_{22}$ ;
Ar1 représente un aryle ou hétéroaryle à 6 chaînons ; Ar2 représente un aryle ou hétéroaryle à 6 chaînons et est fixé à Ar1 en position par rapport à l'amide ;
$R_{10}$ représente H, halo, un $C_{1-3}$alkyle, un $C_{1-2}$haloalkyle, $OC_{1-2}$alkyle, $OC_{1-2}$haloalkyle ou CN ;
$R_{11}$ représente H, F, Cl, un $C_{1-2}$alkyle, $CF_3$, $OCH_3$ ou CN ;
$R_{12}$ est fixé à Ar2 en position ortho ou méta par rapport à Ar1 et $R_{12}$ représente H, halo, un $C_{1-4}$alkyle, un $C_{2-4}$alcényle, $C_{0-2}$alkylène$C_{3-5}$cycloalkyle, $OC_{1-4}$alkyle, $OC_{0-2}$alkylène$C_{3-5}$cycloalkyle, un $C_{1-4}$haloalkyle, $OC_{1-4}$haloalkyle, hydroxy, $C_{1-4}$alkyleOH, $SO_2C_{1-2}$alkyle, $C(O)N(C_{1-2}alkyle)_2$, $NHC(O)C_{1-3}$alkyle ou $NR_{23}R_{24}$ ; et
lorsque A représente -NHC(=O)- :
$R_{12}$ peut en outre être choisi parmi CN, $OCH_2CH_2N(CH_3)_2$ et un $C_{3-6}$hétérocycloalkyle en comprenant un azote situé au point de fixation à Ar2, ou $R_{12}$ forme avec un atome d'azote auquel il est fixé un N-oxyde ($N^+$-$O^-$) ;
$R_{13}$ représente H ou halo ;
$R_{21}$ représente H, un $C_{1-5}$alkyle, $C(O)C_{1-5}$alkyle, $C(O)OC_{1-5}$alkyle ;
$R_{22}$ représente H ou $CH_3$ ;
$R_{23}$ représente H ou un $C_{1-2}$alkyle ; et
$R_{24}$ représente H ou un $C_{1-2}$alkyle ;

ou un sel et/ou un solvate de celui-ci.

**2.** Composé, sel et/ou solvate de celui-ci selon la revendication 1, dans lesquels A représente -NHC(=O)-.

**3.** Composé, sel et/ou solvate de celui-ci selon l'une quelconque des revendications 1 et 2, dans lesquels X représente N, Y représente $CR_2$ et Z représente $CR_3$.

**4.** Composé, sel et/ou solvate de celui-ci selon l'une quelconque des revendications 1 à 3, dans lesquels $R_1$ est un $C_{1-5}$alkyle.

**5.** Composé, sel et/ou solvate de celui-ci selon l'une quelconque des revendications 1 à 3, dans lesquels $R_1$ est un $C_{3-5}$ cycloalkyle ou un $C_{3-5}$cycloalkyle substitué par $CH_3$.

**6.** Composé, sel et/ou solvate de celui-ci selon l'une quelconque des revendications 1 à 5, dans lesquels $R_2$ représente H et $R_3$ représente H.

**7.** Composé, sel et/ou solvate de celui-ci selon l'une quelconque des revendications 1 à 6, dans lesquels $R_4$ est choisi parmi halo, un $C_{1-6}$alkyle, $C_{1-3}$ alkylène$OC_{1-3}$alkyle ou $OC_{1-6}$alkyle et $R_5$ représente H, fluoro, le méthyle ou l'éthyle.

**8.** Composé, sel et/ou solvate de celui-ci selon l'une quelconque des revendications 1 à 6, dans lesquels $R_4$ et $R_5$ forment ensemble avec l'atome de carbone auquel ils sont fixés un cycle cyclopropyle ou un cycle cyclopentyle ; ou $R_4$ et $R_5$ forment ensemble avec l'atome de carbone auquel ils sont fixés un cycle tétrahydropyranyle.

**9.** Composé, sel et/ou solvate de celui-ci selon l'une quelconque des revendications 1 à 8, dans lesquels Ar1 est le phényle ou le 2-pyridyle.

**10.** Composé, sel et/ou solvate de celui-ci selon l'une quelconque des revendications 1 à 9, dans lesquels $R_{10}$ représente H, F, Cl, $CH_3$, $OCH_3$, $OCF_3$ ou CN et $R_{11}$ représente H ou F.

**11.** Composé, sel et/ou solvate de celui-ci selon l'une quelconque des revendications 1 à 10, dans lesquels Ar2 est le 3-pyridyle ou le 2,5-pyrazinyle.

**12.** Composé, sel ou solvate de celui-ci selon l'une quelconque des revendications 1 à 11, dans lesquels $R_{12}$ représente H, F, Cl, $CH_3$, méthoxy, éthoxy, isopropoxy, $OC_0$alkylène$C_3$cycloalkyle, CN, $CF_3$, $OCHF_2$ ou $OCH_2CF_3$ et $R_{13}$ représente H.

**13.** Composé, sel pharmaceutiquement acceptable et/ou solvate de celui-ci selon l'une quelconque des revendications 1 à 12.

**14.** Composé, sel pharmaceutiquement acceptable et/ou solvate de celui-ci selon la revendication 13, destinés à être utilisés en tant que médicament.

**15.** Composé, sel pharmaceutiquement acceptable et/ou solvate de celui-ci destinés à être utilisés selon la revendication 14, pour une utilisation dans la réduction de la prolifération des lymphocytes T et/ou des lymphocytes B chez un sujet ; ou pour une utilisation dans le traitement ou la prophylaxie : des maladies inflammatoires de la peau telles que le psoriasis ou le lichen plan ; de la GVHD aiguë et/ou chronique telle que la GVHD aiguë résistante aux stéroïdes ; du syndrome lymphoprolifératif aigu (ALPS) ; du lupus érythémateux disséminé, de la néphrite lupique ou du lupus cutané ; ou une transplantation ; ou pour une utilisation dans le traitement ou la prophylaxie de la myasthénie grave, de la sclérose en plaques ou de la sclérodermie/sclérose systémique ; ou pour une utilisation pour améliorer la récupération après une lésion vasculaire ou une chirurgie et réduire la morbidité et la mortalité associées à une néointima et une resténose chez un sujet.

**16.** Composé, sel pharmaceutiquement acceptable et/ou solvate de celui-ci selon la revendication 14, destinés à être utilisés dans le traitement du cancer.

**17.** Composé, sel pharmaceutiquement acceptable et/ou solvate de celui-ci destinés à être utilisés selon la revendication 16, dans lequel le cancer est un cancer hématologique.

**18.** Composé, sel pharmaceutiquement acceptable et/ou solvate de celui-ci destinés à être utilisés selon la revendication 17, dans lequel le cancer hématologique est choisi dans le groupe constitué de la leucémie myéloïde aiguë, du lymphome angioimmunoblastique à cellules T, de la leucémie lymphoblastique aiguë à cellules B, du syndrome de Sweet, du lymphome non Hodgkinien à cellules T (y compris le lymphome tueur naturel/à cellules T, la leucémie/le lymphome à cellules T de l'adulte, le lymphome à cellules T de type entéropathique, le lymphome hépatosplénique à cellules T et le lymphome cutané à cellules T), de la leucémie lymphoblastique aiguë à cellules T, du lymphome non Hodgkinien à cellules B (y compris le lymphome de Burkitt, le lymphome diffus à grandes cellules B, le lymphome folliculaire, le lymphome à cellules du manteau, le lymphome de la zone marginale), de la leucémie à cellules pileuses, du lymphome de Hodgkin, du lymphome lymphoblastique, du lymphome lymphoplasmocytaire, du lymphome du tissu lymphoïde associé aux muqueuses, du myélome multiple, du syndrome myélodysplasique, du myélome à plasmocytes, du lymphome médiastinal primitif à grandes cellules B, des troubles myéloprolifératifs chroniques (tels que la leucémie myéloïde chronique, la myélofibrose primitive, la thrombocytémie essentielle, la polyglobulie essentielle) et de la leucémie lymphocytaire chronique.

**19.** Composé, sel pharmaceutiquement acceptable et/ou solvate de celui-ci destinés à être utilisés selon l'une quelconque des revendications 14 à 18, pour une administration à un sujet humain.

**20.** Composition pharmaceutique comprenant un composé, un sel pharmaceutiquement acceptable et/ou un solvate de celui-ci selon la revendication 13.

**21.** Composé choisi dans le groupe constitué :

- d'un composé de formule (II) :

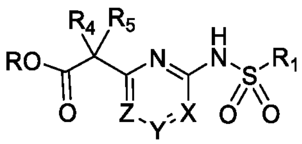

(II)

où $R_1$, X, Y, Z, $R_4$ et $R_5$ sont tels que définis dans une quelconque revendication précédente et R représente H, un $C_{1-6}$alkyle ou le benzyle ; ou
- d'un composé de formule **(XXXXII)** :

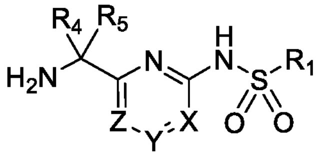

(XXXXII) ;

où $R_1$, X, Y, Z, $R_4$ et $R_5$ sont tels que définis selon une quelconque revendication précédente ; ou
- d'un composé de formule (XX) :

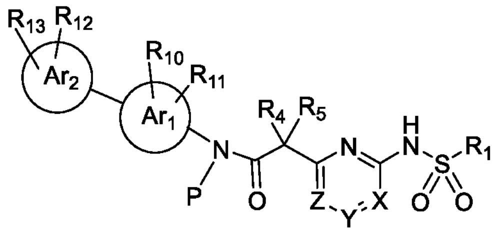

(XX) ;

où Ar1, Ar2, $R_1$, X, Y, Z, $R_4$, $R_5$, $R_{10}$, $R_{11}$, $R_{12}$ et $R_{13}$ sont tels que définis selon une quelconque revendication précédente et P est un groupe protecteur de l'azote ; ou
- d'un composé de formule **(XXIV)** :

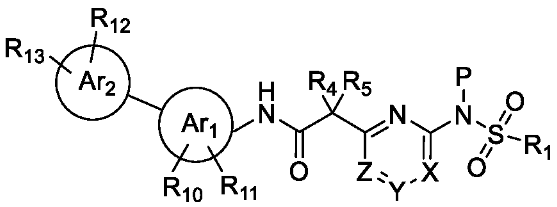

(XXIV) ;

où Ar2, Ar1, $R_1$, X, Y, Z, $R_4$, $R_5$, $R_{10}$, $R_{11}$, $R_{12}$ et $R_{13}$ sont tels que définis selon une quelconque revendication précédente et P est un groupe protecteur de l'azote ; ou
- d'un composé de formule **(XXXI)** :

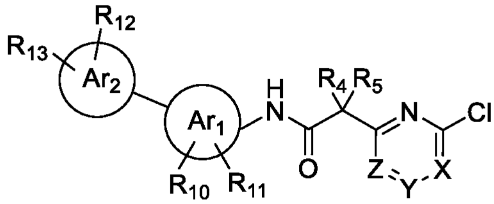

(XXXI) ;

où Ar2 est le 3-pyridyle ou le 2,5-pyrazinyle et Ar1, X, Y, Z, $R_4$, $R_5$, $R_{10}$, $R_{11}$, $R_{12}$ et $R_{13}$ sont tels que définis selon une quelconque revendication précédente ; ou
- d'un composé de formule **(LI)** :

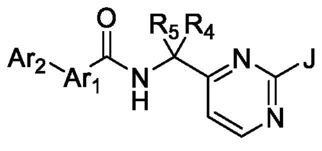

(LI) ;

où J représente Cl ou Br et Ar1, Ar2, $R_4$ et $R_5$ sont tels que définis selon une quelconque revendication

précédente ; et
- d'un composé de formule **(LVIII)** :

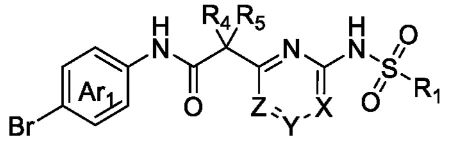

(LVIII) ;

où Ar1, $R_1$, X, Y, Z, $R_4$ et $R_5$ sont tels que définis selon une quelconque revendication précédente ; ou sels de l'un quelconque de ceux-ci.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **EVANS, D. R. ; GUY, H. I.** Mammalian pyrimidine biosynthesis: fresh insights into an ancient pathway. *J. Biol. Chem.,* 2004, vol. 279, 33035-33038
- **FAIRBANKS, L. D. et al.** Importance of ribonucleotide availability to proliferating T-lymphocytes from healthy humans. Disproportionate expansion of pyrimidine pools and contrasting effects of de novo synthesis inhibitors. *J. Biol. Chem.,* 1995, vol. 270, 29682-29689
- **HIGGINS, M. J. et al.** Regulation of human cytidine triphosphate synthetase 1 by glycogen synthase kinase 3. *J. Biol. Chem.,* 2007, vol. 282, 29493-29503
- **KURSULA, P. et al.** Structure of the synthetase domain of human CTP synthetase, a target for anticancer therapy. *Acta Crystallogr Sect F Struct Biol Cryst Commun,* 2006, vol. 62, 613-617
- **LIEBERMAN I.** Enzymatic amination of uridine triphosphate to cytidine triphosphate. *The J. Biol. Chem.,* 1956, vol. 222 (2), 765-75
- **MARTIN E. et al.** CTP synthase 1 deficiency in humans reveals its central role in lymphocytes proliferation. *Nature,* 12 June 2014, vol. 510 (7504), 288-92
- *Nature,* 17 July 2014, vol. 511 (7509), 370
- **MCCLUSKEY GD et al.** Exploring the Potent Inhibition of CTP Synthase by Gemcitabine-5'-Triphosphate. *Chembiochem.,* 2016, vol. 17, 2240-2249
- **OSTRANDER, D. B. et al.** Effect of CTP synthetase regulation by CTP on phospholipid synthesis in Saccharomyces cerevisiae. *J. Biol. Chem.,* 1998, vol. 273, 18992-19001
- **SAKAMOTO K. et al.** Identification of cytidine-5-triphosphate synthase1-selective inhibitory peptide from random peptide library displayed on T7 phage. *Peptides.,* 2017, vol. 94, 56-63
- **SALU et al.** Drug-eluting stents: a new treatment in the prevention of restenosis Part I: experimental studies. *Acta Cardiol,* 2004, vol. 59, 51-61
- **SOUSA J. E. et al.** Drug-Eluting Stents. *Circulation,* 2003, vol. 107, 2274, , 2283
- **TANG R. et al.** CTP synthase 1, a smooth muscle-sensitive therapeutic target for effective vascular repair. *Arterioscler Thromb Vase Biol.,* 2013, vol. 33 (10), 1-19
- **VAN DEN BERG, A. A. et al.** Cytidine triphosphate (CTP) synthetase activity during cell cycle progression in normal and malignant T-lymphocytic cells. *Eur. J. Cancer,* 1995, vol. 31, 108-112
- **VAN KUILENBURG, A.B.P. et al.** Identification of a cDNA encoding an isoform of human CTP synthetase. *Biochimica et Biophysica Acta,* 2000, 1492548-552